# DEMANDE DE BREVET EUROPEEN

(11) **EP 2 275 534 A1**
(43) Date de publication de la demande: **19.01.2011**
(21) Numéro de dépôt: 10005885.8
(22) Date de dépôt: 02.12.2004
(51) Int. Cl.: C12N 7/00, C12N 15/50, C12N 15/63, C07K 14/165, C07K 16/10, C12Q 1/68, C12Q 1/70, G01N 33/53, G01N 33/569, C07H 21/00

(54) **Nouvelle souche de coronavirus associé au SRAS et ses applications**

(30) Priorité: 02.12.2003 FR 0314151; 02.12.2003 FR 0314152
(62) Demande divisionnaire de: 04805625.3
(71) Demandeur: Institut Pasteur, 75015 Paris (FR); Centre National de la Recherche Scientifique, 75016 Paris Cedex 16 (FR); Université Paris Diderot - Paris 7, 75205 Paris Cedex 13 (FR)
(72) Inventeur: Van der Werf, Sylvie, 91190 GIF-Sur Yvette (FR); Escriou, Nicolas, 75014 Paris (FR); Crescenzo-Chaigné, Bernadette, 92200 Neuilly-Sur-Seine (FR); Manuguerra, Jean-Claude, 75018 Paris (FR); Kunst, Frederik, 94200 Ivry-sur-Seine (FR); Callendret, Benoît, 92000 Nanterre (FR); Betton, Jean-Michel, 75014 Paris (FR); Lorin, Valérie, 92120 Montrouge (FR); Gerbaud, Sylvie, 94100 Saint-Maur-des-Fosses (FR); Burguiere, Ana Maria, 92140 Clamart (FR); Azebi, Saliha, 94400 Vitry-sur-Seine (FR); Charneau, Pierre, 75005 Paris (FR); Tangy, Frédéric, 93260 Les Lilas (FR); Combredet, Chantal, 75017 Paris (FR); Delagneau, Jean-François, 78170 La Celle Saint Cloud (FR); Martin, Monique, 92290 Chatenay Malabry (FR)
(74) Mandataire: Rançon, Xavier Lucien Abel

(57) **Abrégé**

Nouvelle souche de coronavirus associé au syndrome respiratoire aigu sévère (SRAS), issue d'un prélèvement répertorié sous le n° 031589 et prélevé à Hanoi (Vietnam), des molécules d'acide nucléique issues de son génome, protéines et peptides codés par lesdites molécules d'acide nucléique et plus particulièrement la protéine N ainsi que leurs applications, notamment en tant que réactifs de diagnostic et/ou comme vaccin.

SEQUENCE LISTING

<110> INSTITUT PASTEUR
CENTRE NATIONAL DE LA RECHERCHE SCIENTIFIQUE
UNIVERSITE PARIS 7
VAN DER WERF, Sylvie
ESCRIOU, Nicolas
CRESCENZO-CHAIGNE, Bernadette
MANUGUERRA, Jean-Claude
KUNST, Franck
CALLENDRET, Benoît
BETTON, Jean-Michel
LORIN, Valérie
GERBAUD, Sylvie
BURGUIERE, Ana Maria
AZEBI, Saliha
CHARNEAU, Pierre
TANGY, Frédéric
COMBREDET, Chantal
DELAGNEAU, Jean-François
MARTIN, Monique

<120> NOUVELLE SOUCHE DE CORONAVIRUS ASSOCIE AU SRAS ET SES APPLICATIONS

<130> 226-108ext

<150> FR 0314152
<151> 2003-12-02

<150> FR 0314151
<151> 2003-12-02

<160> 158

<170>  PatentIn version 3.1

<210>  1

<211>  29746

<212>  DNA

<213>  CORONAVIRUS

<400>  1
atattaggtt tttacctacc caggaaaagc caaccaacct cgatctcttg tagatctgtt      60

ctctaaacga actttaaaat ctgtgtagct gtcgctcggc tgcatgccta gtgcacctac     120

gcagtataaa caataataaa ttttactgtc gttgacaaga aacgagtaac tcgtccctct     180

tctgcagact gcttacggtt tcgtccgtgt tgcagtcgat catcagcata cctaggtttc     240

gtccgggtgt gaccgaaagg taagatggag agccttgttc ttggtgtcaa cgagaaaaca     300

cacgtccaac tcagtttgcc tgtccttcag gttagagacg tgctagtgcg tggcttcggg     360

gactctgtgg aagaggccct atcggaggca cgtgaacacc tcaaaaatgg cacttgtggt     420

ctagtagagc tggaaaaagg cgtactgccc cagcttgaac agccctatgt gttcattaaa     480

cgttctgatg ccttaagcac caatcacggc cacaaggtcg ttgagctggt tgcagaaatg     540

gacggcattc agtacggtcg tagcggtata acactgggag tactcgtgcc acatgtgggc     600

gaaaccccaa ttgcataccg caatgttctt cttcgtaaga acggtaataa gggagccggt     660

ggtcatagct atggcatcga tctaaagtct tatgacttag gtgacgagct tggcactgat     720

cccattgaag attatgaaca aaactggaac actaagcatg gcagtggtgc actccgtgaa     780

ctcactcgtg agctcaatgg aggtgcagtc actcgctatg tcgacaacaa tttctgtggc     840

ccagatgggt accctcttga ttgcatcaaa gattttctcg cacgcgcggg caagtcaatg     900

tgcactcttt ccgaacaact tgattacatc gagtcgaaga gaggtgtcta ctgctgccgt     960

gaccatgagc atgaaattgc ctggttcact gagcgctctg ataagagcta cgagcaccag    1020

acacccttcg aaattaagag tgccaagaaa tttgacactt tcaaagggga atgcccaaag    1080

tttgtgtttc ctcttaactc aaaagtcaaa gtcattcaac cacgtgttga aaagaaaaag    1140

actgagggtt tcatggggcg tatacgctct gtgtaccctg ttgcatctcc acaggagtgt    1200

aacaatatgc acttgtctac cttgatgaaa tgtaatcatt gcgatgaagt ttcatggcag    1260

acgtgcgact ttctgaaagc cacttgtgaa cattgtggca ctgaaaattt agttattgaa    1320

ggacctacta catgtgggta cctacctact aatgctgtag tgaaaatgcc atgtcctgcc    1380

tgtcaagacc cagagattgg acctgagcat agtgttgcag attatcacaa ccactcaaac    1440

attgaaactc gactccgcaa gggaggtagg actagatgtt ttggaggctg tgtgtttgcc    1500

tatgttggct gctataataa gcgtgcctac tgggttcctc gtgctagtgc tgatattggc    1560

tcaggccata ctggcattac tggtgacaat gtggagacct tgaatgagga tctccttgag    1620

atactgagtc gtgaacgtgt taacattaac attgttggcg attttcattt gaatgaagag 1680
gttgccatca ttttggcatc tttctctgct tctacaagtg cctttattga cactataaag 1740
agtcttgatt acaagtcttt caaaaccatt gttgagtcct gcggtaacta taaagttacc 1800
aagggaaagc ccgtaaaagg tgcttggaac attggacaac agagatcagt tttaacacca 1860
ctgtgtggtt ttccctcaca ggctgctggt gttatcagat caatttttgc gcgcacactt 1920
gatgcagcaa accactcaat tcctgatttg caaagagcag ctgtcaccat acttgatggt 1980
atttctgaac agtcattacg tcttgtcgac gccatggttt atacttcaga cctgctcacc 2040
aacagtgtca ttattatggc atatgtaact ggtggtcttg tacaacagac ttctcagtgg 2100
ttgtctaatc ttttgggcac tactgttgaa aaactcaggc ctatctttga atggattgag 2160
gcgaaactta gtgcaggagt tgaatttctc aaggatgctt gggagattct caaatttctc 2220
attacaggtg tttttgacat cgtcaagggt caaatacagg ttgcttcaga taacatcaag 2280
gattgtgtaa aatgcttcat tgatgttgtt aacaaggcac tcgaaatgtg cattgatcaa 2340
gtcactatcg ctggcgcaaa gttgcgatca ctcaacttag gtgaagtctt catcgctcaa 2400
agcaagggac tttaccgtca gtgtatacgt ggcaaggagc agctgcaact actcatgcct 2460
cttaaggcac caaaagaagt aacctttctt gaaggtgatt cacatgacac agtacttacc 2520
tctgaggagg ttgttctcaa gaacggtgaa ctcgaagcac tcgagacgcc cgttgatagc 2580
ttcacaaatg gagctatcgt tggcacacca gtctgtgtaa atggcctcat gctcttagag 2640
attaaggaca aagaacaata ctgcgcattg tctcctggtt tactggctac aaacaatgtc 2700
tttcgcttaa aagggggtgc accaattaaa ggtgtaacct ttggagaaga tactgtttgg 2760
gaagttcaag gttacaagaa tgtgagaatc acatttgagc ttgatgaacg tgttgacaaa 2820
gtgcttaatg aaaagtgctc tgtctacact gttgaatccg gtaccgaagt tactgagttt 2880
gcatgtgttg tagcagaggc tgttgtgaag actttacaac cagtttctga tctccttacc 2940
aacatgggta ttgatcttga tgagtggagt gtagctacat tctacttatt tgatgatgct 3000
ggtgaagaaa acttttcatc acgtatgtat tgttcctttt accctccaga tgaggaagaa 3060
gaggacgatg cagagtgtga ggaagaagaa attgatgaaa cctgtgaaca tgagtacggt 3120
acagaggatg attatcaagg tctccctctg gaatttggtg cctcagctga aacagttcga 3180
gttgaggaag aagaagagga agactggctg gatgatacta ctgagcaatc agagattgag 3240
ccagaaccag aacctacacc tgaagaacca gttaatcagt ttactggtta tttaaaactt 3300
actgacaatg ttgccattaa atgtgttgac atcgttaagg aggcacaaag tgctaatcct 3360
atggtgattg taaatgctgc taacatacac ctgaaacatg gtggtggtgt agcaggtgca 3420
ctcaacaagg caaccaatgg tgccatgcaa aaggagagtg atgattacat taagctaaat 3480
ggccctctta cagtaggagg gtcttgtttg ctttctggac ataatcttgc taagaagtgt 3540
ctgcatgttg ttggacctaa cctaaatgca ggtgaggaca tccagcttct taaggcagca 3600
tatgaaaatt tcaattcaca ggacatctta cttgcaccat tgttgtcagc aggcatattt 3660

ggtgctaaac cacttcagtc tttacaagtg tgcgtgcaga cggttcgtac acaggtttat 3720
attgcagtca atgacaaagc tctttatgag caggttgtca tggattatct tgataacctg 3780
aagcctagag tggaagcacc taaacaagag gagccaccaa acacagaaga ttccaaaact 3840
gaggagaaat ctgtcgtaca gaagcctgtc gatgtgaagc caaaaattaa ggcctgcatt 3900
gatgaggtta ccacaacact ggaagaaact aagtttctta ccaataagtt actcttgttt 3960
gctgatatca atggtaagct ttaccatgat tctcagaaca tgcttagagg tgaagatatg 4020
tctttccttg agaaggatgc accttacatg gtaggtgatg ttatcactag tggtgatatc 4080
acttgtgttg taataccctc caaaaaggct ggtggcacta ctgagatgct ctcaagagct 4140
ttgaagaaag tgccagttga tgagtatata accacgtacc ctggacaagg atgtgctggt 4200
tatacacttg aggaagctaa gactgctctt aagaaatgca aatctgcatt ttatgtacta 4260
ccttcagaag cacctaatgc taaggaagag attctaggaa ctgtatcctg gaatttgaga 4320
gaaatgcttg ctcatgctga agagacaaga aaattaatgc ctatatgcat ggatgttaga 4380
gccataatgg caaccatcca acgtaagtat aaaggaatta aaattcaaga gggcatcgtt 4440
gactatggtg tccgattctt cttttatact agtaaagagc ctgtagcttc tattattacg 4500
aagctgaact ctctaaatga gccgcttgtc acaatgccaa ttggttatgt gacacatggt 4560
tttaatcttg aagaggctgc gcgctgtatg cgttctctta aagctcctgc cgtagtgtca 4620
gtatcatcac cagatgctgt tactacatat aatggatacc tcacttcgtc atcaaagaca 4680
tctgaggagc actttgtaga aacagtttct ttggctggct cttacagaga ttggtcctat 4740
tcaggacagc gtacagagtt aggtgttgaa tttcttaagc gtggtgacaa aattgtgtac 4800
cacactctgg agagccccgt cgagtttcat cttgacggtg aggttctttc acttgacaaa 4860
ctaaagagtc tcttatccct gcgggaggtt aagactataa aagtgttcac aactgtggac 4920
aacactaatc tccacacaca gcttgtggat atgtctatga catatggaca gcagtttggt 4980
ccaacatact tggatggtgc tgatgttaca aaaattaaac ctcatgtaaa tcatgagggt 5040
aagactttct ttgtactacc tagtgatgac acactacgta gtgaagcttt cgagtactac 5100
catactcttg atgagagttt tcttggtagg tacatgtctg ctttaaacca cacaaagaaa 5160
tggaaatttc ctcaagttgg tggtttaact tcaattaaat gggctgataa caattgttat 5220
ttgtctagtg ttttattagc acttcaacag cttgaagtca aattcaatgc accagcactt 5280
caagaggctt attatagagc ccgtgctggt gatgctgcta acttttgtgc actcatactc 5340
gcttacagta ataaaactgt tggcgagctt ggtgatgtca gagaaactat gacccatctt 5400
ctacagcatg ctaatttgga atctgcaaag cgagttctta atgtggtgtg taaacattgt 5460
ggtcagaaaa ctactacctt aacgggtgta gaagctgtga tgtatatggg tactctatct 5520
tatgataatc ttaagacagg tgtttccatt ccatgtgtgt gtggtcgtga tgctacacaa 5580
tatctagtac aacaagagtc ttcttttgtt atgatgtctg caccacctgc tgagtataaa 5640
ttacagcaag gtacattctt atgtgcgaat gagtacactg gtaactatca gtgtggtcat 5700

tacactcata taactgctaa ggagaccctc tatcgtattg acggagctca ccttacaaag 5760
atgtcagagt acaaaggacc agtgactgat gttttctaca aggaaacatc ttacactaca 5820
accatcaagc ctgtgtcgta taaactcgat ggagttactt acacagagat tgaaccaaaa 5880
ttggatgggt attataaaaa ggataatgct tactatacag agcagcctat agaccttgta 5940
ccaactcaac cattaccaaa tgcgagtttt gataatttca aactcacatg ttctaacaca 6000
aaatttgctg atgatttaaa tcaaatgaca ggcttcacaa agccagcttc acgagagcta 6060
tctgtcacat tcttcccaga cttgaatggc gatgtagtgg ctattgacta tagacactat 6120
tcagcgagtt tcaagaaagg tgctaaatta ctgcataagc caattgtttg gcacattaac 6180
caggctacaa ccaagacaac gttcaaacca aacacttggt gtttacgttg tctttggagt 6240
acaaagccag tagatacttc aaattcattt gaagttctgg cagtagaaga cacacaagga 6300
atggacaatc ttgcttgtga aagtcaacaa cccacctctg aagaagtagt ggaaaatcct 6360
accatacaga aggaagtcat agagtgtgac gtgaaaacta ccgaagttgt aggcaatgtc 6420
atacttaaac catcagatga aggtgttaaa gtaacacaag agttaggtca tgaggatctt 6480
atggctgctt atgtggaaaa cacaagcatt accattaaga aacctaatga gctttcacta 6540
gccttaggtt taaaaacaat tgccactcat ggtattgctg caattaatag tgttccttgg 6600
agtaaaattt tggcttatgt caaaccattc ttaggacaag cagcaattac aacatcaaat 6660
tgcgctaaga gattagcaca acgtgtgttt aacaattata tgccttatgt gtttacatta 6720
ttgttccaat tgtgtacttt tactaaaagt accaattcta gaattagagc ttcactacct 6780
acaactattg ctaaaaatag tgttaagagt gttgctaaat tatgtttgga tgccggcatt 6840
aattatgtga agtcacccaa attttctaaa ttgttcacaa tcgctatgtg gctattgttg 6900
ttaagtattt gcttaggttc tctaatctgt gtaactgctg cttttggtgt actcttatct 6960
aattttggtg ctccttctta ttgtaatggc gttagagaat tgtatcttaa ttcgtctaac 7020
gttactacta tggatttctg tgaaggttct tttccttgca gcatttgttt aagtggatta 7080
gactcccttg attcttatcc agctcttgaa accattcagg tgacgatttc atcgtacaag 7140
ctagacttga caattttagg tctggccgct gagtgggttt tggcatatat gttgttcaca 7200
aaattctttt atttattagg tctttcagct ataatgcagg tgttctttgg ctattttgct 7260
agtcatttca tcagcaattc ttggctcatg tggtttatca ttagtattgt acaaatggca 7320
cccgtttctg caatggttag gatgtacatc ttctttgctt ctttctacta catatggaag 7380
agctatgttc atatcatgga tggttgcacc tcttcgactt gcatgatgtg ctataagcgc 7440
aatcgtgcca cacgcgttga gtgtacaact attgttaatg gcatgaagag atctttctat 7500
gtctatgcaa atggaggccg tggcttctgc aagactcaca attggaattg tctcaattgt 7560
gacacatttt gcactggtag tacattcatt agtgatgaag ttgctcgtga tttgtcactc 7620
cagtttaaaa gaccaatcaa ccctactgac cagtcatcgt atattgttga tagtgttgct 7680
gtgaaaaatg gcgcgcttca cctctacttt gacaaggctg gtcaaaagac ctatgagaga 7740

catccgctct cccattttgt caatttagac aatttgagag ctaacaacac taaaggttca 7800
ctgcctatta atgtcatagt ttttgatggc aagtccaaat gcgacgagtc tgcttctaag 7860
tctgcttctg tgtactacag tcagctgatg tgccaaccta ttctgttgct tgaccaagct 7920
cttgtatcag acgttggaga tagtactgaa gtttccgtta agatgtttga tgcttatgtc 7980
gacacctttt cagcaacttt tagtgttcct atggaaaaac ttaaggcact tgttgctaca 8040
gctcacagcg agttagcaaa gggtgtagct ttagatggtg tcctttctac attcgtgtca 8100
gctgcccgac aaggtgttgt tgataccgat gttgacacaa aggatgttat tgaatgtctc 8160
aaactttcac atcactctga cttagaagtg acaggtgaca gttgtaacaa tttcatgctc 8220
acctataata aggttgaaaa catgacgccc agagatcttg gcgcatgtat tgactgtaat 8280
gcaaggcata tcaatgccca agtagcaaaa agtcacaatg tttcactcat ctggaatgta 8340
aaagactaca tgtctttatc tgaacagctg cgtaaacaaa ttcgtagtgc tgccaagaag 8400
aacaacatac cttttagact aacttgtgct acaactagac aggttgtcaa tgtcataact 8460
actaaaatct cactcaaggg tggtaagatt gttagtactt gttttaaact tatgcttaag 8520
gccacattat tgtgcgttct tgctgcattg gtttgttata tcgttatgcc agtacataca 8580
ttgtcaatcc atgatggtta cacaaatgaa atcattggtt acaaagccat tcaggatggt 8640
gtcactcgtg acatcatttc tactgatgat tgttttgcaa ataaacatgc tggttttgac 8700
gcatggttta gccagcgtgg tggttcatac aaaaatgaca aaagctgccc tgtagtagct 8760
gctatcatta caagagagat tggtttcata gtgcctggct taccgggtac tgtgctgaga 8820
gcaatcaatg gtgacttctt gcattttcta cctcgtgttt ttagtgctgt tggcaacatt 8880
tgctacacac cttccaaact cattgagtat agtgattttg ctacctctgc ttgcgttctt 8940
gctgctgagt gtacaatttt taaggatgct atgggcaaac ctgtgccata ttgttatgac 9000
actaatttgc tagagggttc tatttcttat agtgagcttc gtccagacac tcgttatgtg 9060
cttatggatg gttccatcat acagtttcct aacacttacc tggagggttc tgttagagta 9120
gtaacaactt ttgatgctga gtactgtaga catggtacat gcgaaaggtc agaagtaggt 9180
atttgcctat ctaccagtgg tagatgggtt cttaataatg agcattacag agctctatca 9240
ggagttttct gtggtgttga tgcgatgaat ctcatagcta acatctttac tcctcttgtg 9300
caacctgtgg gtgctttaga tgtgtctgct tcagtagtgg ctggtggtat tattgccata 9360
ttggtgactt gtgctgccta ctactttatg aaattcagac gtgtttttgg tgagtacaac 9420
catgttgttg ctgctaatgc acttttgttt ttgatgtctt tcactatact ctgtctggta 9480
ccagcttaca gctttctgcc gggagtctac tcagtctttt acttgtactt gacattctat 9540
ttcaccaatg atgtttcatt cttggctcac cttcaatggt ttgccatgtt ttctcctatt 9600
gtgccttttt ggataacagc aatctatgta ttctgtattt ctctgaagca ctgccattgg 9660
ttctttaaca actatcttag gaaaagagtc atgtttaatg gagttacatt tagtaccttc 9720
gaggaggctg ctttgtgtac ctttttgctc aacaaggaaa tgtacctaaa attgcgtagc 9780

gagacactgt tgccacttac acagtataac aggtatcttg ctctatataa caagtacaag 9840
tatttcagtg gagccttaga tactaccagc tatcgtgaag cagcttgctg ccacttagca 9900
aaggctctaa atgactttag caactcaggt gctgatgttc tctaccaacc accacagaca 9960
tcaatcactt ctgctgttct gcagagtggt tttaggaaaa tggcattccc gtcaggcaaa 10020
gttgaagggt gcatggtaca agtaacctgt ggaactacaa ctcttaatgg attgtggttg 10080
gatgacacag tatactgtcc aagacatgtc atttgcacag cagaagacat gcttaatcct 10140
aactatgaag atctgctcat tcgcaaatcc aaccatagct ttcttgttca ggctggcaat 10200
gttcaacttc gtgttattgg ccattctatg caaaattgtc tgcttaggct taaagttgat 10260
acttctaacc ctaagacacc caagtataaa tttgtccgta tccaacctgg tcaaacattt 10320
tcagttctag catgctacaa tggttcacca tctggtgttt atcagtgtgc catgagacct 10380
aatcatacca ttaaaggttc tttccttaat ggatcatgtg gtagtgttgg ttttaacatt 10440
gattatgatt gcgtgtcttt ctgctatatg catcatatgg agcttccaac aggagtacac 10500
gctggtactg acttagaagg taaattctat ggtccatttg ttgacagaca aactgcacag 10560
gctgcaggta cagacacaac cataacatta aatgttttgg catggctgta tgctgctgtt 10620
atcaatggtg ataggtggtt tcttaataga ttcaccacta ctttgaatga ctttaacctt 10680
gtggcaatga agtacaacta tgaacctttg acacaagatc atgttgacat attgggacct 10740
ctttctgctc aaacaggaat tgccgtctta gatatgtgtg ctgctttgaa agagctgctg 10800
cagaatggta tgaatggtcg tactatcctt ggtagcacta ttttagaaga tgagtttaca 10860
ccatttgatg ttgttagaca atgctctggt gttaccttcc aaggtaagtt caagaaaatt 10920
gttaagggca ctcatcattg gatgctttta actttcttga catcactatt gattcttgtt 10980
caaagtacac agtggtcact gtttttcttt gtttacgaga atgctttctt gccatttact 11040
cttggtatta tggcaattgc tgcatgtgct atgctgcttg ttaagcataa gcacgcattc 11100
ttgtgcttgt ttctgttacc ttctcttgca acagttgctt actttaatat ggtctacatg 11160
cctgctagct gggtgatgcg tatcatgaca tggcttgaat tggctgacac tagcttgtct 11220
ggttataggc ttaaggattg tgttatgtat gcttcagctt tagttttgct tattctcatg 11280
acagctcgca ctgtttatga tgatgctgct agacgtgttt ggacactgat gaatgtcatt 11340
acacttgttt acaaagtcta ctatggtaat gctttagatc aagctatttc catgtgggcc 11400
ttagttattt ctgtaacctc taactattct ggtgtcgtta cgactatcat gtttttagct 11460
agagctatag tgtttgtgtg tgttgagtat tacccattgt tatttattac tggcaacacc 11520
ttacagtgta tcatgcttgt ttattgtttc ttaggctatt gttgctgctg ctactttggc 11580
cttttctgtt tactcaaccg ttacttcagg cttactcttg gtgtttatga ctacttggtc 11640
tctacacaag aatttaggta tatgaactcc caggggcttt tgcctcctaa gagtagtatt 11700
gatgctttca agcttaacat taagttgttg ggtattggag gtaaaccatg tatcaaggtt 11760
gctactgtac agtctaaaat gtctgacgta aagtgcacat ctgtggtact gctctcggtt 11820

cttcaacaac ttagagtaga gtcatcttct aaattgtggg cacaatgtgt acaactccac 11880
aatgatattc ttcttgcaaa agacacaact gaagctttcg agaagatggt ttctcttttg 11940
tctgttttgc tatccatgca gggtgctgta gacattaata ggttgtgcga ggaaatgctc 12000
gataaccgtg ctactcttca ggctattgct tcagaattta gttctttacc atcatatgcc 12060
gcttatgcca ctgcccagga ggcctatgag caggctgtag ctaatggtga ttctgaagtc 12120
gttctcaaaa agttaaagaa atctttgaat gtggctaaat ctgagtttga ccgtgatgct 12180
gccatgcaac gcaagttgga aaagatggca gatcaggcta tgacccaaat gtacaaacag 12240
gcaagatctg aggacaagag ggcaaaagta actagtgcta tgcaaacaat gctcttcact 12300
atgcttagga agcttgataa tgatgcactt aacaacatta tcaacaatgc gcgtgatggt 12360
tgtgttccac tcaacatcat accattgact acagcagcca aactcatggt tgttgtccct 12420
gattatggta cctacaagaa cacttgtgat ggtaacacct ttacatatgc atctgcactc 12480
tgggaaatcc agcaagttgt tgatgcggat agcaagattg ttcaacttag tgaaattaac 12540
atggacaatt caccaaattt ggcttggcct cttattgtta cagctctaag agccaactca 12600
gctgttaaac tacagaataa tgaactgagt ccagtagcac tacgacagat gtcctgtgcg 12660
gctggtacca cacaaacagc ttgtactgat gacaatgcac ttgcctacta taacaattcg 12720
aagggaggta ggtttgtgct ggcattacta tcagaccacc aagatctcaa atgggctaga 12780
ttccctaaga gtgatggtac aggtacaatt tacacagaac tggaaccacc ttgtaggttt 12840
gttacagaca caccaaaagg gcctaaagtg aaatacttgt acttcatcaa aggcttaaac 12900
aacctaaata gaggtatggt gctgggcagt ttagctgcta cagtacgtct tcaggctgga 12960
aatgctacag aagtacctgc caattcaact gtgctttcct tctgtgcttt tgcagtagac 13020
cctgctaaag catataagga ttacctagca agtggaggac aaccaatcac caactgtgtg 13080
aagatgttgt gtacacacac tggtacagga caggcaatta ctgtaacacc agaagctaac 13140
atggaccaag agtcctttgg tggtgcttca tgttgtctgt attgtagatg ccacattgac 13200
catccaaatc ctaaaggatt ctgtgacttg aaaggtaagt acgtccaaat acctaccact 13260
tgtgctaatg acccagtggg ttttacactt agaaacacag tctgtaccgt ctgcggaatg 13320
tggaaaggtt atggctgtag ttgtgaccaa ctccgcgaac ccttgatgca gtctgcggat 13380
gcatcaacgt ttttaaacgg gtttgcggtg taagtgcagc ccgtcttaca ccgtgcggca 13440
caggcactag tactgatgtc gtctacaggg cttttgatat ttacaacgaa aaagttgctg 13500
gttttgcaaa gttcctaaaa actaattgct gtcgcttcca ggagaaggat gaggaaggca 13560
atttattaga ctcttacttt gtagttaaga ggcatactat gtctaactac caacatgaag 13620
agactattta taacttggtt aaagattgtc cagcggttgc tgtccatgac tttttcaagt 13680
ttagagtaga tggtgacatg gtaccacata tatcacgtca gcgtctaact aaatacacaa 13740
tggctgattt agtctatgct ctacgtcatt ttgatgaggg taattgtgat acattaaaag 13800
aaatactcgt cacatacaat tgctgtgatg atgattattt caataagaag gattggtatg 13860

acttcgtaga gaatcctgac atcttacgcg tatatgctaa cttaggtgag cgtgtacgcc 13920
aatcattatt aaagactgta caattctgcg atgctatgcg tgatgcaggc attgtaggcg 13980
tactgacatt agataatcag gatcttaatg ggaactggta cgatttcggt gatttcgtac 14040
aagtagcacc aggctgcgga gttcctattg tggattcata ttactcattg ctgatgccca 14100
tcctcacttt gactagggca ttggctgctg agtcccatat ggatgctgat ctcgcaaaac 14160
cacttattaa gtgggatttg ctgaaatatg attttacgga agagagactt tgtctcttcg 14220
accgttattt taaatattgg gaccagacat accatcccaa ttgtattaac tgtttggatg 14280
ataggtgtat ccttcattgt gcaaacttta atgtgttatt ttctactgtg tttccaccta 14340
caagttttgg accactagta agaaaaatat ttgtagatgg tgttcctttt gttgtttcaa 14400
ctggatacca ttttcgtgag ttaggagtcg tacataatca ggatgtaaac ttacatagct 14460
cgcgtctcag tttcaaggaa cttttagtgt atgctgctga tccagctatg catgcagctt 14520
ctggcaattt attgctagat aaacgcacta catgcttttc agtagctgca ctaacaaaca 14580
atgttgcttt tcaaactgtc aaacccggta attttaataa agacttttat gactttgctg 14640
tgtctaaagg tttctttaag gaaggaagtt ctgttgaact aaaacacttc ttctttgctc 14700
aggatggcaa cgctgctatc agtgattatg actattatcg ttataatctg ccaacaatgt 14760
gtgatatcag acaactccta ttcgtagttg aagttgttga taaatacttt gattgttacg 14820
atggtggctg tattaatgcc aaccaagtaa tcgttaacaa tctggataaa tcagctggtt 14880
tcccatttaa taaatggggt aaggctagac tttattatga ctcaatgagt tatgaggatc 14940
aagatgcact tttcgcgtat actaagcgta atgtcatccc tactataact caaatgaatc 15000
ttaagtatgc cattagtgca aagaatagag ctcgcaccgt agctggtgtc tctatctgta 15060
gtactatgac aaatagacag tttcatcaga aattattgaa gtcaatagcc gccactagag 15120
gagctactgt ggtaattgga acaagcaagt tttacggtgg ctggcataat atgttaaaaa 15180
ctgtttacag tgatgtagaa actccacacc ttatgggttg ggattatcca aaatgtgaca 15240
gagccatgcc taacatgctt aggataatgg cctctcttgt tcttgctcgc aaacataaca 15300
cttgctgtaa cttatcacac cgtttctaca ggttagctaa cgagtgtgcg caagtattaa 15360
gtgagatggt catgtgtggc ggctcactat atgttaaacc aggtggaaca tcatccggtg 15420
atgctacaac tgcttatgct aatagtgtct ttaacatttg tcaagctgtt acagccaatg 15480
taaatgcact tctttcaact gatggtaata agatagctga caagtatgtc cgcaatctac 15540
aacacaggct ctatgagtgt ctctatagaa atagggatgt tgatcatgaa ttcgtggatg 15600
agttttacgc ttacctgcgt aaacatttct ccatgatgat tctttctgat gatgccgttg 15660
tgtgctataa cagtaactat gcggctcaag gtttagtagc tagcattaag aactttaagg 15720
cagttcttta ttatcaaaat aatgtgttca tgtctgaggc aaaatgttgg actgagactg 15780
accttactaa aggacctcac gaattttgct cacagcatac aatgctagtt aaacaaggag 15840
atgattacgt gtacctgcct tacccagatc catcaagaat attaggcgca ggctgttttg 15900

tcgatgatat tgtcaaaaca gatggtacac ttatgattga aaggttcgtg tcactggcta 15960
ttgatgctta cccacttaca aaacatccta atcaggagta tgctgatgtc tttcacttgt 16020
atttacaata cattagaaag ttacatgatg agcttactgg ccacatgttg gacatgtatt 16080
ccgtaatgct aactaatgat aacacctcac ggtactggga acctgagttt tatgaggcta 16140
tgtacacacc acatacagtc ttgcaggctg taggtgcttg tgtattgtgc aattcacaga 16200
cttcacttcg ttgcggtgcc tgtattagga gaccattcct atgttgcaag tgctgctatg 16260
accatgtcat ttcaacatca cacaaattag tgttgtctgt taatccctat gtttgcaatg 16320
ccccaggttg tgatgtcact gatgtgacac aactgtatct aggaggtatg agctattatt 16380
gcaagtcaca taagcctccc attagttttc cattatgtgc taatggtcag gtttttggtt 16440
tatacaaaaa cacatgtgta ggcagtgaca atgtcactga cttcaatgcg atagcaacat 16500
gtgattggac taatgctggc gattacatac ttgccaacac ttgtactgag agactcaagc 16560
ttttcgcagc agaaacgctc aaagccactg aggaaacatt taagctgtca tatggtattg 16620
ccactgtacg cgaagtactc tctgacagag aattgcatct ttcatgggag gttggaaaac 16680
ctagaccacc attgaacaga aactatgtct ttactggtta ccgtgtaact aaaaatagta 16740
aagtacagat tggagagtac acctttgaaa aaggtgacta tggtgatgct gttgtgtaca 16800
gaggtactac gacatacaag ttgaatgttg gtgattactt tgtgttgaca tctcacactg 16860
taatgccact tagtgcacct actctagtgc cacaagagca ctatgtgaga attactggct 16920
tgtacccaac actcaacatc tcagatgagt tttctagcaa tgttgcaaat tatcaaaagg 16980
tcggcatgca aaagtactct acactccaag gaccacctgg tactggtaag agtcattttg 17040
ccatcggact tgctctctat tacccatctg ctcgcatagt gtatacggca tgctctcatg 17100
cagctgttga tgccctatgt gaaaaggcat taaaatattt gcccatagat aaatgtagta 17160
gaatcatacc tgcgcgtgcg cgcgtagagt gttttgataa attcaaagtg aattcaacac 17220
tagaacagta tgttttctgc actgtaaatg cattgccaga aacaactgct gacattgtag 17280
tctttgatga aatctctatg gctactaatt atgacttgag tgttgtcaat gctagacttc 17340
gtgcaaaaca ctacgtctat attggcgatc ctgctcaatt accagccccc cgcacattgc 17400
tgactaaagg cacactagaa ccagaatatt ttaattcagt gtgcagactt atgaaaacaa 17460
taggtccaga catgttcctt ggaacttgtc gccgttgtcc tgctgaaatt gttgacactg 17520
tgagtgcttt agtttatgac aataagctaa aagcacacaa ggataagtca gctcaatgct 17580
tcaaaatgtt ctacaaaggt gttattacac atgatgtttc atctgcaatc aacagacctc 17640
aaataggcgt tgtaagagaa tttcttacac gcaatcctgc ttggagaaaa gctgttttta 17700
tctcacctta taattcacag aacgctgtag cttcaaaaat cttaggattg cctacgcaga 17760
ctgttgattc atcacagggt tctgaatatg actatgtcat attcacacaa actactgaaa 17820
cagcacactc ttgtaatgtc aaccgcttca atgtggctat cacaagggca aaaattggca 17880
ttttgtgcat aatgtctgat agagatcttt atgacaaact gcaatttaca agtctagaaa 17940

taccacgtcg caatgtggct acattacaag cagaaaatgt aactggactt tttaaggact 18000
gtagtaagat cattactggt cttcatccta cacaggcacc tacacacctc agcgttgata 18060
taaagttcaa gactgaagga ttatgtgttg acataccagg cataccaaag gacatgacct 18120
accgtagact catctctatg atgggtttca aaatgaatta ccaagtcaat ggttacccta 18180
atatgtttat cacccgcgaa gaagctattc gtcacgttcg tgcgtggatt ggctttgatg 18240
tagagggctg tcatgcaact agagatgctg tgggtactaa cctacctctc cagctaggat 18300
tttctacagg tgttaactta gtagctgtac cgactggtta tgttgacact gaaaataaca 18360
cagaattcac cagagttaat gcaaaacctc caccaggtga ccagtttaaa catcttatac 18420
cactcatgta taaaggcttg ccctggaatg tagtgcgtat taagatagta caaatgctca 18480
gtgatacact gaaaggattg tcagacagag tcgtgttcgt cctttgggcg catggctttg 18540
agcttacatc aatgaagtac tttgtcaaga ttggacctga aagaacgtgt tgtctgtgtg 18600
acaaacgtgc aacttgcttt tctacttcat cagatactta tgcctgctgg aatcattctg 18660
tgggttttga ctatgtctat aacccattta tgattgatgt tcagcagtgg ggctttacgg 18720
gtaaccttca gagtaaccat gaccaacatt gccaggtaca tggaaatgca catgtggcta 18780
gttgtgatgc tatcatgact agatgtttag cagtccatga gtgctttgtt aagcgcgttg 18840
attggtctgt tgaataccct attataggag atgaactgag ggttaattct gcttgcagaa 18900
aagtacaaca catggttgtg aagtctgcat tgcttgctga taagtttcca gttcttcatg 18960
acattggaaa tccaaaggct atcaagtgtg tgcctcaggc tgaagtagaa tggaagttct 19020
acgatgctca gccatgtagt gacaaagctt acaaaataga ggaactcttc tattcttatg 19080
ctacacatca cgataaattc actgatggtg tttgtttgtt ttggaattgt aacgttgatc 19140
gttacccagc caatgcaatt gtgtgtaggt ttgacacaag agtcttgtca aacttgaact 19200
taccaggctg tgatggtggt agtttgtatg tgaataagca tgcattccac actccagctt 19260
tcgataaaag tgcatttact aatttaaagc aattgccttt cttttactat tctgatagtc 19320
cttgtgagtc tcatggcaaa caagtagtgt cggatattga ttatgttcca ctcaaatctg 19380
ctacgtgtat tacacgatgc aatttaggtg gtgctgtttg cagacaccat gcaaatgagt 19440
accgacagta cttggatgca tataatatga tgatttctgc tggatttagc ctatggattt 19500
acaaacaatt tgatacttat aacctgtgga atacatttac caggttacag agtttagaaa 19560
atgtggctta taatgttgtt aataaaggac actttgatgg acacgccggc gaagcacctg 19620
tttccatcat taataatgct gtttacacaa aggtagatgg tattgatgtg gagatctttg 19680
aaaataagac aacacttcct gttaatgttg catttgagct ttgggctaag cgtaacatta 19740
aaccagtgcc agagattaag atactcaata atttgggtgt tgatatcgct gctaatactg 19800
taatctggga ctacaaaaga gaagccccag cacatgtatc tacaataggt gtctgcacaa 19860
tgactgacat tgccaagaaa cctactgaga gtgcttgttc ttcacttact gtcttgtttg 19920
atggtagagt ggaaggacag gtagaccttt ttagaaacgc ccgtaatggt gttttaataa 19980

cagaaggttc agtcaaaggt ctaacacctt caaagggacc agcacaagct agcgtcaatg 20040
gagtcacatt aattggagaa tcagtaaaaa cacagtttaa ctactttaag aaagtagacg 20100
gcattattca acagttgcct gaaacctact ttactcagag cagagactta gaggatttta 20160
agcccagatc acaaatggaa actgactttc tcgagctcgc tatggatgaa ttcatacagc 20220
gatataagct cgagggctat gccttcgaac acatcgttta tggagatttc agtcatggac 20280
aacttggcgg tcttcattta atgataggct tagccaagcg ctcacaagat tcaccactta 20340
aattagagga ttttatccct atggacagca cagtgaaaaa ttacttcata acagatgcgc 20400
aaacaggttc atcaaaatgt gtgtgttctg tgattgatct tttacttgat gactttgtcg 20460
agataataaa gtcacaagat ttgtcagtga tttcaaaagt ggtcaaggtt acaattgact 20520
atgctgaaat ttcattcatg ctttggtgta aggatggaca tgttgaaacc ttctacccaa 20580
aactacaagc aagtcaagcg tggcaaccag gtgttgcgat gcctaacttg tacaagatgc 20640
aaagaatgct tcttgaaaag tgtgaccttc agaattatgg tgaaaatgct gttataccaa 20700
aaggaataat gatgaatgtc gcaaagtata ctcaactgtg tcaatactta aatacactta 20760
ctttagctgt accctacaac atgagagtta ttcactttgg tgctggctct gataaaggag 20820
ttgcaccagg tacagctgtg ctcagacaat ggttgccaac tggcacacta cttgtcgatt 20880
cagatcttaa tgacttcgtc tccgacgcag attctacttt aattggagac tgtgcaacag 20940
tacatacggc taataaatgg gaccttatta ttagcgatat gtatgaccct aggaccaaac 21000
atgtgacaaa agagaatgac tctaaagaag ggtttttcac ttatctgtgt ggatttataa 21060
agcaaaaact agccctgggt ggttctatag ctgtaaagat aacagagcat tcttggaatg 21120
ctgaccttta caagcttatg ggccatttct catggtggac agcttttgtt acaaatgtaa 21180
atgcatcatc atcggaagca tttttaattg gggctaacta tcttggcaag ccgaaggaac 21240
aaattgatgg ctataccatg catgctaact acattttctg gaggaacaca aatcctatcc 21300
agttgtcttc ctattcactc tttgacatga gcaaatttcc tcttaaatta agaggaactg 21360
ctgtaatgtc tcttaaggag aatcaaatca atgatatgat ttattctctt ctggaaaaag 21420
gtaggcttat cattagagaa aacaacagag ttgtggtttc aagtgatatt cttgttaaca 21480
actaaacgaa catgtttatt ttcttattat ttcttactct cactagtggt agtgaccttg 21540
accggtgcac cacttttgat gatgttcaag ctcctaatta cactcaacat acttcatcta 21600
tgaggggggt ttactatcct gatgaaattt ttagatcaga cactctttat ttaactcagg 21660
atttatttct tccattttat tctaatgtta cagggtttca tactattaat catacgtttg 21720
gcaaccctgt catacctttt aaggatggta tttattttgc tgccacagag aaatcaaatg 21780
ttgtccgtgg ttgggttttt ggttctacca tgaacaacaa gtcacagtcg gtgattatta 21840
ttaacaattc tactaatgtt gttatacgag catgtaactt tgaattgtgt gacaaccctt 21900
tctttgctgt ttctaaaccc atgggtacac agacacatac tatgatattc gataatgcat 21960
ttaattgcac tttcgagtac atatctgatg ccttttcgct tgatgtttca gaaaagtcag 22020

gtaattttaa acacttacga gagtttgtgt ttaaaaataa agatgggttt ctctatgttt 22080
ataagggcta tcaacctata gatgtagttc gtgatctacc ttctggtttt aacactttga 22140
aacctatttt taagttgcct cttggtatta acattacaaa ttttagagcc attcttacag 22200
ccttttcacc tgctcaagac atttggggca cgtcagctgc agcctatttt gttggctatt 22260
taaagccaac tacatttatg ctcaagtatg atgaaaatgg tacaatcaca gatgctgttg 22320
attgttctca aaatccactt gctgaactca aatgctctgt taagagcttt gagattgaca 22380
aaggaattta ccagacctct aatttcaggg ttgttccctc aggagatgtt gtgagattcc 22440
ctaatattac aaacttgtgt ccttttggag aggtttttaa tgctactaaa ttcccttctg 22500
tctatgcatg ggagagaaaa aaaatttcta attgtgttgc tgattactct gtgctctaca 22560
actcaacatt tttttcaacc tttaagtgct atggcgtttc tgccactaag ttgaatgatc 22620
tttgcttctc caatgtctat gcagattctt ttgtagtcaa gggagatgat gtaagacaaa 22680
tagcgccagg acaaactggt gttattgctg attataatta taaattgcca gatgatttca 22740
tgggttgtgt ccttgcttgg aatactagga acattgatgc tacttcaact ggtaattata 22800
attataaata taggtatctt agacatggca agcttaggcc ctttgagaga gacatatcta 22860
atgtgccttt ctcccctgat ggcaaacctt gcaccccacc tgctcttaat tgttattggc 22920
cattaaatga ttatggtttt tacaccacta ctggcattgg ctaccaacct tacagagttg 22980
tagtactttc ttttgaactt ttaaatgcac cggccacggt ttgtggacca aaattatcca 23040
ctgaccttat taagaaccag tgtgtcaatt ttaattttaa tggactcact ggtactggtg 23100
tgttaactcc ttcttcaaag agatttcaac catttcaaca atttggccgt gatgtttctg 23160
atttcactga ttccgttcga gatcctaaaa catctgaaat attagacatt tcaccttgct 23220
cttttggggg tgtaagtgta attacacctg gaacaaatgc ttcatctgaa gttgctgttc 23280
tatatcaaga tgttaactgc actgatgttt ctacagcaat tcatgcagat caactcacac 23340
cagcttggcg catatattct actggaaaca atgtattcca gactcaagca ggctgtctta 23400
taggagctga gcatgtcgac acttcttatg agtgcgacat tcctattgga gctggcattt 23460
gtgctagtta ccatacagtt tctttattac gtagtactag ccaaaaatct attgtggctt 23520
atactatgtc tttaggtgct gatagttcaa ttgcttactc taataacacc attgctatac 23580
ctactaactt ttcaattagc attactacag aagtaatgcc tgtttctatg gctaaaacct 23640
ccgtagattg taatatgtac atctgcggag attctactga atgtgctaat ttgcttctcc 23700
aatatggtag cttttgcaca caactaaatc gtgcactctc aggtattgct gctgaacagg 23760
atcgcaacac acgtgaagtg ttcgctcaag tcaaacaaat gtacaaaacc ccaactttga 23820
aatattttgg tggttttaat ttttcacaaa tattacctga ccctctaaag ccaactaaga 23880
ggtcttttat tgaggacttg ctctttaata aggtgacact cgctgatgct ggcttcatga 23940
agcaatatgg cgaatgccta ggtgatatta atgctagaga tctcatttgt gcgcagaagt 24000
tcaatggact tacagtgttg ccacctctgc tcactgatga tatgattgct gcctacactg 24060

ctgctctagt tagtggtact gccactgctg gatggacatt tggtgctggc gctgctcttc 24120
aaataccttt tgctatgcaa atggcatata ggttcaatgg cattggagtt acccaaaatg 24180
ttctctatga gaaccaaaaa caaatcgcca accaatttaa caaggcgatt agtcaaattc 24240
aagaatcact tacaacaaca tcaactgcat tgggcaagct gcaagacgtt gttaaccaga 24300
atgctcaagc attaaacaca cttgttaaac aacttagctc taattttggt gcaatttcaa 24360
gtgtgctaaa tgatatcctt tcgcgacttg ataaagtcga ggcggaggta caaattgaca 24420
ggttaattac aggcagactt caaagccttc aaacctatgt aacacaacaa ctaatcaggg 24480
ctgctgaaat cagggcttct gctaatcttg ctgctactaa aatgtctgag tgtgttcttg 24540
gacaatcaaa aagagttgac ttttgtggaa agggctacca ccttatgtcc ttcccacaag 24600
cagccccgca tggtgttgtc ttcctacatg tcacgtatgt gccatcccag gagaggaact 24660
tcaccacagc gccagcaatt tgtcatgaag gcaaagcata cttccctcgt gaaggtgttt 24720
ttgtgtttaa tggcacttct tggtttatta cacagaggaa cttcttttct ccacaaataa 24780
ttactacaga caatacattt gtctcaggaa attgtgatgt cgttattggc atcattaaca 24840
acacagttta tgatcctctg caacctgagc ttgactcatt caaagaagag ctggacaagt 24900
acttcaaaaa tcatacatca ccagatgttg atcttggcga catttcaggc attaacgctt 24960
ctgtcgtcaa cattcaaaaa gaaattgacc gcctcaatga ggtcgctaaa aatttaaatg 25020
aatcactcat tgaccttcaa gaattgggaa aatatgagca atatattaaa tggccttggt 25080
atgtttggct cggcttcatt gctggactaa ttgccatcgt catggttaca atcttgcttt 25140
gttgcatgac tagttgttgc agttgcctca agggtgcatg ctcttgtggt tcttgctgca 25200
agtttgatga ggatgactct gagccagttc tcaagggtgt caaattacat tacacataaa 25260
cgaacttatg gatttgttta tgagattttt tactcttgga tcaattactg cacagccagt 25320
aaaaattgac aatgcttctc ctgcaagtac tgttcatgct acagcaacga taccgctaca 25380
agcctcactc cctttcggat ggcttgttat tggcgttgca tttcttgctg tttttcagag 25440
cgctaccaaa ataattgcgc tcaataaaag atggcagcta gccctttata agggcttcca 25500
gttcatttgc aatttactgc tgctatttgt taccatctat tcacatcttt tgcttgtcgc 25560
tgcaggtatg gaggcgcaat ttttgtacct ctatgccttg atatattttc tacaatgcat 25620
caacgcatgt agaattatta tgagatgttg gctttgttgg aagtgcaaat ccaagaaccc 25680
attactttat gatgccaact actttgtttg ctggcacaca cataactatg actactgtat 25740
accatataac agtgtcacag atacaattgt cgttactgaa ggtgacggca tttcaacacc 25800
aaaactcaaa gaagactacc aaattggtgg ttattctgag gataggcact caggtgttaa 25860
agactatgtc gttgtacatg gctatttcac cgaagtttac taccagcttg agtctacaca 25920
aattactaca gacactggta ttgaaaatgc tacattcttc atctttaaca agcttgttaa 25980
agacccaccg aatgtgcaaa tacacacaat cgacggctct tcaggagttg ctaatccagc 26040
aatggatcca atttatgatg agccgacgac gactactagc gtgcctttgt aagcacaaga 26100

aagtgagtac gaacttatgt actcattcgt ttcggaagaa acaggtacgt taatagttaa 26160
tagcgtactt ctttttcttg ctttcgtggt attcttgcta gtcacactag ccatccttac 26220
tgcgcttcga ttgtgtgcgt actgctgcaa tattgttaac gtgagtttag taaaaccaac 26280
ggtttacgtc tactcgcgtg ttaaaaatct gaactcttct gaaggagttc ctgatcttct 26340
ggtctaaacg aactaactat tattattatt ctgtttggaa ctttaacatt gcttatcatg 26400
gcagacaacg gtactattac cgttgaggag cttaaacaac tcctggaaca atggaaccta 26460
gtaataggtt tcctattcct agcctggatt atgttactac aatttgccta ttctaatcgg 26520
aacaggtttt tgtacataat aaagcttgtt ttcctctggc tcttgtggcc agtaacactt 26580
gcttgttttg tgcttgctgc tgtctacaga attaattggg tgactggcgg gattgcgatt 26640
gcaatggctt gtattgtagg cttgatgtgg cttagctact tcgttgcttc cttcaggctg 26700
tttgctcgta cccgctcaat gtggtcattc aacccagaaa caaacattct tctcaatgtg 26760
cctctccggg ggacaattgt gaccagaccg ctcatggaaa gtgaacttgt cattggtgct 26820
gtgatcattc gtggtcactt gcgaatggcc ggacactccc tagggcgctg tgacattaag 26880
gacctgccaa aagagatcac tgtggctaca tcacgaacgc tttcttatta caaattagga 26940
gcgtcgcagc gtgtaggcac tgattcaggt tttgctgcat acaaccgcta ccgtattgga 27000
aactataaat taaatacaga ccacgccggt agcaacgaca atattgcttt gctagtacag 27060
taagtgacaa cagatgtttc atcttgttga cttccaggtt acaatagcag agatattgat 27120
tatcattatg aggactttca ggattgctat ttggaatctt gacgttataa taagttcaat 27180
agtgagacaa ttatttaagc ctctaactaa gaagaattat tcggagttag atgatgaaga 27240
acctatggag ttagattatc cataaaacga acatgaaaat tattctcttc ctgacattga 27300
ttgtatttac atcttgcgag ctatatcact atcaggagtg tgttagaggt acgactgtac 27360
tactaaaaga accttgccca tcaggaacat acgagggcaa ttcaccattt caccctcttg 27420
ctgacaataa atttgcacta acttgcacta gcacacactt tgcttttgct tgtgctgacg 27480
gtactcgaca tacctatcag ctgcgtgcaa gatcagtttc accaaaactt ttcatcagac 27540
aagaggaggt tcaacaagag ctctactcgc cactttttct cattgttgct gctctagtat 27600
ttttaatact ttgcttcacc attaagagaa agacagaatg aatgagctca ctttaattga 27660
cttctatttg tgctttttag cctttctgct attccttgtt ttaataatgc ttattatatt 27720
ttggttttca ctcgaaatcc aggatctaga agaaccttgt accaaagtct aaacgaacat 27780
gaaacttctc attgttttga cttgtatttc tctatgcagt tgcatatgca ctgtagtaca 27840
gcgctgtgca tctaataaac ctcatgtgct tgaagatcct tgtaaggtac aacactaggg 27900
gtaatactta tagcactgct tggctttgtg ctctaggaaa ggttttacct tttcatagat 27960
ggcacactat ggttcaaaca tgcacaccta atgttactat caactgtcaa gatccagctg 28020
gtggtgcgct tatagctagg tgttggtacc ttcatgaagg tcaccaaact gctgcattta 28080
gagacgtact tgttgtttta aataaacgaa caaattaaaa tgtctgataa tggaccccaa 28140

tcaaaccaac gtagtgcccc ccgcattaca tttggtggac ccacagattc aactgacaat 28200
aaccagaatg gaggacgcaa tggggcaagg ccaaaacagc gccgacccca aggtttaccc 28260
aataatactg cgtcttggtt cacagctctc actcagcatg gcaaggagga acttagattc 28320
cctcgaggcc agggcgttcc aatcaacacc aatagtggtc cagatgacca aattggctac 28380
taccgaagag ctacccgacg agttcgtggt ggtgacggca aaatgaaaga gctcagcccc 28440
agatggtact tctattacct aggaactggc ccagaagctt cacttcccta cggcgctaac 28500
aaagaaggca tcgtatgggt tgcaactgag ggagccttga atacacccaa agaccacatt 28560
ggcacccgca atcctaataa caatgctgcc accgtgctac aacttcctca aggaacaaca 28620
ttgccaaaag gcttctacgc agagggaagc agaggcggca gtcaagcctc ttctcgctcc 28680
tcatcacgta gtcgcggtaa ttcaagaaat tcaactcctg gcagcagtag gggaaattct 28740
cctgctcgaa tggctagcgg aggtggtgaa actgccctcg cgctattgct gctagacaga 28800
ttgaaccagc ttgagagcaa agtttctggt aaaggccaac aacaacaagg ccaaactgtc 28860
actaagaaat ctgctgctga ggcatctaaa aagcctcgcc aaaaacgtac tgccacaaaa 28920
cagtacaacg tcactcaagc atttgggaga cgtggtccag aacaaaccca aggaaatttc 28980
ggggaccaag acctaatcag acaaggaact gattacaaac attggccgca aattgcacaa 29040
tttgctccaa gtgcctctgc attctttgga atgtcacgca ttggcatgga agtcacacct 29100
tcgggaacat ggctgactta tcatggagcc attaaattgg atgacaaaga tccacaattc 29160
aaagacaacg tcatactgct gaacaagcac attgacgcat acaaaacatt cccaccaaca 29220
gagcctaaaa aggacaaaaa gaaaaagact gatgaagctc agcctttgcc gcagagacaa 29280
aagaagcagc ccactgtgac tcttcttcct gcggctgaca tggatgattt ctccagacaa 29340
cttcaaaatt ccatgagtgg agcttctgct gattcaactc aggcataaac actcatgatg 29400
accacacaag gcagatgggc tatgtaaacg ttttcgcaat tccgtttacg atacatagtc 29460
tactcttgtg cagaatgaat tctcgtaact aaacagcaca agtaggttta gttaacttta 29520
atctcacata gcaatcttta atcaatgtgt aacattaggg aggacttgaa agagccacca 29580
cattttcatc gaggccacgc ggagtacgat cgagggtaca gtgaataatg ctagggagag 29640
ctgcctatat ggaagagccc taatgtgtaa aattaatttt agtagtgcta tccccatgtg 29700
attttaatag cttcttagga gaatgacaaa aaaaaaaaaa aaaaaa 29746

<210> 2
<211> 3945
<212> DNA
<213> CORONAVIRUS

<220>

<221>  CDS

<222>  (89)..(3853)

<223>

<400>  2
ttctcttctg gaaaaaggta ggcttatcat tagagaaaac aacagagttg tggtttcaag      60

tgatattctt gttaacaact aaacgaac atg ttt att ttc tta tta ttt ctt      112
                               Met Phe Ile Phe Leu Leu Phe Leu
                               1               5

act ctc act agt ggt agt gac ctt gac cgg tgc acc act ttt gat gat      160
Thr Leu Thr Ser Gly Ser Asp Leu Asp Arg Cys Thr Thr Phe Asp Asp
    10                  15                  20

gtt caa gct cct aat tac act caa cat act tca tct atg agg ggg gtt      208
Val Gln Ala Pro Asn Tyr Thr Gln His Thr Ser Ser Met Arg Gly Val
25                  30                  35                  40

tac tat cct gat gaa att ttt aga tca gac act ctt tat tta act cag      256
Tyr Tyr Pro Asp Glu Ile Phe Arg Ser Asp Thr Leu Tyr Leu Thr Gln
                45                  50                  55

gat tta ttt ctt cca ttt tat tct aat gtt aca ggg ttt cat act att      304
Asp Leu Phe Leu Pro Phe Tyr Ser Asn Val Thr Gly Phe His Thr Ile
            60                  65                  70

aat cat acg ttt ggc aac cct gtc ata cct ttt aag gat ggt att tat      352
Asn His Thr Phe Gly Asn Pro Val Ile Pro Phe Lys Asp Gly Ile Tyr
        75                  80                  85

ttt gct gcc aca gag aaa tca aat gtt gtc cgt ggt tgg gtt ttt ggt      400
Phe Ala Ala Thr Glu Lys Ser Asn Val Val Arg Gly Trp Val Phe Gly
    90                  95                  100

tct acc atg aac aac aag tca cag tcg gtg att att att aac aat tct      448
Ser Thr Met Asn Asn Lys Ser Gln Ser Val Ile Ile Ile Asn Asn Ser
105                 110                 115                 120

act aat gtt gtt ata cga gca tgt aac ttt gaa ttg tgt gac aac cct      496
Thr Asn Val Val Ile Arg Ala Cys Asn Phe Glu Leu Cys Asp Asn Pro
                125                 130                 135

ttc ttt gct gtt tct aaa ccc atg ggt aca cag aca cat act atg ata      544
Phe Phe Ala Val Ser Lys Pro Met Gly Thr Gln Thr His Thr Met Ile
            140                 145                 150

ttc gat aat gca ttt aat tgc act ttc gag tac ata tct gat gcc ttt      592
Phe Asp Asn Ala Phe Asn Cys Thr Phe Glu Tyr Ile Ser Asp Ala Phe
        155                 160                 165

tcg ctt gat gtt tca gaa aag tca ggt aat ttt aaa cac tta cga gag      640
Ser Leu Asp Val Ser Glu Lys Ser Gly Asn Phe Lys His Leu Arg Glu
    170                 175                 180

ttt gtg ttt aaa aat aaa gat ggg ttt ctc tat gtt tat aag ggc tat      688
Phe Val Phe Lys Asn Lys Asp Gly Phe Leu Tyr Val Tyr Lys Gly Tyr
185                 190                 195                 200

caa cct ata gat gta gtt cgt gat cta cct tct ggt ttt aac act ttg      736
Gln Pro Ile Asp Val Val Arg Asp Leu Pro Ser Gly Phe Asn Thr Leu
                205                 210                 215

aaa cct att ttt aag ttg cct ctt ggt att aac att aca aat ttt aga      784

Lys Pro Ile Phe Lys Leu Pro Leu Gly Ile Asn Ile Thr Asn Phe Arg
            220                     225                     230

gcc att ctt aca gcc ttt tca cct gct caa gac att tgg ggc acg tca      832
Ala Ile Leu Thr Ala Phe Ser Pro Ala Gln Asp Ile Trp Gly Thr Ser
        235                     240                     245

gct gca gcc tat ttt gtt ggc tat tta aag cca act aca ttt atg ctc      880
Ala Ala Ala Tyr Phe Val Gly Tyr Leu Lys Pro Thr Thr Phe Met Leu
    250                     255                     260

aag tat gat gaa aat ggt aca atc aca gat gct gtt gat tgt tct caa      928
Lys Tyr Asp Glu Asn Gly Thr Ile Thr Asp Ala Val Asp Cys Ser Gln
265                     270                     275                     280

aat cca ctt gct gaa ctc aaa tgc tct gtt aag agc ttt gag att gac      976
Asn Pro Leu Ala Glu Leu Lys Cys Ser Val Lys Ser Phe Glu Ile Asp
                285                     290                     295

aaa gga att tac cag acc tct aat ttc agg gtt gtt ccc tca gga gat      1024
Lys Gly Ile Tyr Gln Thr Ser Asn Phe Arg Val Val Pro Ser Gly Asp
            300                     305                     310

gtt gtg aga ttc cct aat att aca aac ttg tgt cct ttt gga gag gtt      1072
Val Val Arg Phe Pro Asn Ile Thr Asn Leu Cys Pro Phe Gly Glu Val
        315                     320                     325

ttt aat gct act aaa ttc cct tct gtc tat gca tgg gag aga aaa aaa      1120
Phe Asn Ala Thr Lys Phe Pro Ser Val Tyr Ala Trp Glu Arg Lys Lys
    330                     335                     340

att tct aat tgt gtt gct gat tac tct gtg ctc tac aac tca aca ttt      1168
Ile Ser Asn Cys Val Ala Asp Tyr Ser Val Leu Tyr Asn Ser Thr Phe
345                     350                     355                     360

ttt tca acc ttt aag tgc tat ggc gtt tct gcc act aag ttg aat gat      1216
Phe Ser Thr Phe Lys Cys Tyr Gly Val Ser Ala Thr Lys Leu Asn Asp
                365                     370                     375

ctt tgc ttc tcc aat gtc tat gca gat tct ttt gta gtc aag gga gat      1264
Leu Cys Phe Ser Asn Val Tyr Ala Asp Ser Phe Val Val Lys Gly Asp
            380                     385                     390

gat gta aga caa ata gcg cca gga caa act ggt gtt att gct gat tat      1312
Asp Val Arg Gln Ile Ala Pro Gly Gln Thr Gly Val Ile Ala Asp Tyr
        395                     400                     405

aat tat aaa ttg cca gat gat ttc atg ggt tgt gtc ctt gct tgg aat      1360
Asn Tyr Lys Leu Pro Asp Asp Phe Met Gly Cys Val Leu Ala Trp Asn
    410                     415                     420

act agg aac att gat gct act tca act ggt aat tat aat tat aaa tat      1408
Thr Arg Asn Ile Asp Ala Thr Ser Thr Gly Asn Tyr Asn Tyr Lys Tyr
425                     430                     435                     440

agg tat ctt aga cat ggc aag ctt agg ccc ttt gag aga gac ata tct      1456
Arg Tyr Leu Arg His Gly Lys Leu Arg Pro Phe Glu Arg Asp Ile Ser
                445                     450                     455

aat gtg cct ttc tcc cct gat ggc aaa cct tgc acc cca cct gct ctt      1504
Asn Val Pro Phe Ser Pro Asp Gly Lys Pro Cys Thr Pro Pro Ala Leu
            460                     465                     470

aat tgt tat tgg cca tta aat gat tat ggt ttt tac acc act act ggc      1552
Asn Cys Tyr Trp Pro Leu Asn Asp Tyr Gly Phe Tyr Thr Thr Thr Gly
        475                     480                     485

att ggc tac caa cct tac aga gtt gta gta ctt tct ttt gaa ctt tta      1600

Ile Gly Tyr Gln Pro Tyr Arg Val Val Val Leu Ser Phe Glu Leu Leu
    490                 495                 500

aat gca ccg gcc acg gtt tgt gga cca aaa tta tcc act gac ctt att      1648
Asn Ala Pro Ala Thr Val Cys Gly Pro Lys Leu Ser Thr Asp Leu Ile
505                 510                 515                 520

aag aac cag tgt gtc aat ttt aat ttt aat gga ctc act ggt act ggt      1696
Lys Asn Gln Cys Val Asn Phe Asn Phe Asn Gly Leu Thr Gly Thr Gly
                525                 530                 535

gtg tta act cct tct tca aag aga ttt caa cca ttt caa caa ttt ggc      1744
Val Leu Thr Pro Ser Ser Lys Arg Phe Gln Pro Phe Gln Gln Phe Gly
            540                 545                 550

cgt gat gtt tct gat ttc act gat tcc gtt cga gat cct aaa aca tct      1792
Arg Asp Val Ser Asp Phe Thr Asp Ser Val Arg Asp Pro Lys Thr Ser
        555                 560                 565

gaa ata tta gac att tca cct tgc tct ttt ggg ggt gta agt gta att      1840
Glu Ile Leu Asp Ile Ser Pro Cys Ser Phe Gly Gly Val Ser Val Ile
    570                 575                 580

aca cct gga aca aat gct tca tct gaa gtt gct gtt cta tat caa gat      1888
Thr Pro Gly Thr Asn Ala Ser Ser Glu Val Ala Val Leu Tyr Gln Asp
585                 590                 595                 600

gtt aac tgc act gat gtt tct aca gca att cat gca gat caa ctc aca      1936
Val Asn Cys Thr Asp Val Ser Thr Ala Ile His Ala Asp Gln Leu Thr
                605                 610                 615

cca gct tgg cgc ata tat tct act gga aac aat gta ttc cag act caa      1984
Pro Ala Trp Arg Ile Tyr Ser Thr Gly Asn Asn Val Phe Gln Thr Gln
            620                 625                 630

gca ggc tgt ctt ata gga gct gag cat gtc gac act tct tat gag tgc      2032
Ala Gly Cys Leu Ile Gly Ala Glu His Val Asp Thr Ser Tyr Glu Cys
        635                 640                 645

gac att cct att gga gct ggc att tgt gct agt tac cat aca gtt tct      2080
Asp Ile Pro Ile Gly Ala Gly Ile Cys Ala Ser Tyr His Thr Val Ser
    650                 655                 660

tta tta cgt agt act agc caa aaa tct att gtg gct tat act atg tct      2128
Leu Leu Arg Ser Thr Ser Gln Lys Ser Ile Val Ala Tyr Thr Met Ser
665                 670                 675                 680

tta ggt gct gat agt tca att gct tac tct aat aac acc att gct ata      2176
Leu Gly Ala Asp Ser Ser Ile Ala Tyr Ser Asn Asn Thr Ile Ala Ile
                685                 690                 695

cct act aac ttt tca att agc att act aca gaa gta atg cct gtt tct      2224
Pro Thr Asn Phe Ser Ile Ser Ile Thr Thr Glu Val Met Pro Val Ser
            700                 705                 710

atg gct aaa acc tcc gta gat tgt aat atg tac atc tgc gga gat tct      2272
Met Ala Lys Thr Ser Val Asp Cys Asn Met Tyr Ile Cys Gly Asp Ser
        715                 720                 725

act gaa tgt gct aat ttg ctt ctc caa tat ggt agc ttt tgc aca caa      2320
Thr Glu Cys Ala Asn Leu Leu Leu Gln Tyr Gly Ser Phe Cys Thr Gln
    730                 735                 740

cta aat cgt gca ctc tca ggt att gct gct gaa cag gat cgc aac aca      2368
Leu Asn Arg Ala Leu Ser Gly Ile Ala Ala Glu Gln Asp Arg Asn Thr
745                 750                 755                 760

cgt gaa gtg ttc gct caa gtc aaa caa atg tac aaa acc cca act ttg      2416

Arg Glu Val Phe Ala Gln Val Lys Gln Met Tyr Lys Thr Pro Thr Leu
                765                 770                 775

aaa tat ttt ggt ggt ttt aat ttt tca caa ata tta cct gac cct cta      2464
Lys Tyr Phe Gly Gly Phe Asn Phe Ser Gln Ile Leu Pro Asp Pro Leu
            780                 785                 790

aag cca act aag agg tct ttt att gag gac ttg ctc ttt aat aag gtg      2512
Lys Pro Thr Lys Arg Ser Phe Ile Glu Asp Leu Leu Phe Asn Lys Val
        795                 800                 805

aca ctc gct gat gct ggc ttc atg aag caa tat ggc gaa tgc cta ggt      2560
Thr Leu Ala Asp Ala Gly Phe Met Lys Gln Tyr Gly Glu Cys Leu Gly
    810                 815                 820

gat att aat gct aga gat ctc att tgt gcg cag aag ttc aat gga ctt      2608
Asp Ile Asn Ala Arg Asp Leu Ile Cys Ala Gln Lys Phe Asn Gly Leu
825                 830                 835                 840

aca gtg ttg cca cct ctg ctc act gat gat atg att gct gcc tac act      2656
Thr Val Leu Pro Pro Leu Leu Thr Asp Asp Met Ile Ala Ala Tyr Thr
                845                 850                 855

gct gct cta gtt agt ggt act gcc act gct gga tgg aca ttt ggt gct      2704
Ala Ala Leu Val Ser Gly Thr Ala Thr Ala Gly Trp Thr Phe Gly Ala
            860                 865                 870

ggc gct gct ctt caa ata cct ttt gct atg caa atg gca tat agg ttc      2752
Gly Ala Ala Leu Gln Ile Pro Phe Ala Met Gln Met Ala Tyr Arg Phe
        875                 880                 885

aat ggc att gga gtt acc caa aat gtt ctc tat gag aac caa aaa caa      2800
Asn Gly Ile Gly Val Thr Gln Asn Val Leu Tyr Glu Asn Gln Lys Gln
    890                 895                 900

atc gcc aac caa ttt aac aag gcg att agt caa att caa gaa tca ctt      2848
Ile Ala Asn Gln Phe Asn Lys Ala Ile Ser Gln Ile Gln Glu Ser Leu
905                 910                 915                 920

aca aca aca tca act gca ttg ggc aag ctg caa gac gtt gtt aac cag      2896
Thr Thr Thr Ser Thr Ala Leu Gly Lys Leu Gln Asp Val Val Asn Gln
                925                 930                 935

aat gct caa gca tta aac aca ctt gtt aaa caa ctt agc tct aat ttt      2944
Asn Ala Gln Ala Leu Asn Thr Leu Val Lys Gln Leu Ser Ser Asn Phe
            940                 945                 950

ggt gca att tca agt gtg cta aat gat atc ctt tcg cga ctt gat aaa      2992
Gly Ala Ile Ser Ser Val Leu Asn Asp Ile Leu Ser Arg Leu Asp Lys
        955                 960                 965

gtc gag gcg gag gta caa att gac agg tta att aca ggc aga ctt caa      3040
Val Glu Ala Glu Val Gln Ile Asp Arg Leu Ile Thr Gly Arg Leu Gln
    970                 975                 980

agc ctt caa acc tat gta aca caa caa cta atc agg gct gct gaa atc      3088
Ser Leu Gln Thr Tyr Val Thr Gln Gln Leu Ile Arg Ala Ala Glu Ile
985                 990                 995                 1000

agg gct tct gct aat  ctt gct gct act aaa  atg tct gag tgt gtt      3133
Arg Ala Ser Ala Asn  Leu Ala Ala Thr Lys  Met Ser Glu Cys Val
                1005                1010                1015

ctt gga caa tca aaa  aga gtt gac ttt tgt  gga aag ggc tac cac      3178
Leu Gly Gln Ser Lys  Arg Val Asp Phe Cys  Gly Lys Gly Tyr His
                1020                1025                1030

ctt atg tcc ttc cca  caa gca gcc ccg cat  ggt gtt gtc ttc cta      3223

Leu Met Ser Phe Pro Gln Ala Ala Pro His Gly Val Val Phe Leu
                1035                1040                1045

cat gtc acg tat gtg cca tcc cag gag agg aac ttc acc aca gcg      3268
His Val Thr Tyr Val Pro Ser Gln Glu Arg Asn Phe Thr Thr Ala
                1050                1055                1060

cca gca att tgt cat gaa ggc aaa gca tac ttc cct cgt gaa ggt      3313
Pro Ala Ile Cys His Glu Gly Lys Ala Tyr Phe Pro Arg Glu Gly
                1065                1070                1075

gtt ttt gtg ttt aat ggc act tct tgg ttt att aca cag agg aac      3358
Val Phe Val Phe Asn Gly Thr Ser Trp Phe Ile Thr Gln Arg Asn
                1080                1085                1090

ttc ttt tct cca caa ata att act aca gac aat aca ttt gtc tca      3403
Phe Phe Ser Pro Gln Ile Ile Thr Thr Asp Asn Thr Phe Val Ser
                1095                1100                1105

gga aat tgt gat gtc gtt att ggc atc att aac aac aca gtt tat      3448
Gly Asn Cys Asp Val Val Ile Gly Ile Ile Asn Asn Thr Val Tyr
                1110                1115                1120

gat cct ctg caa cct gag ctt gac tca ttc aaa gaa gag ctg gac      3493
Asp Pro Leu Gln Pro Glu Leu Asp Ser Phe Lys Glu Glu Leu Asp
                1125                1130                1135

aag tac ttc aaa aat cat aca tca cca gat gtt gat ctt ggc gac      3538
Lys Tyr Phe Lys Asn His Thr Ser Pro Asp Val Asp Leu Gly Asp
                1140                1145                1150

att tca ggc att aac gct tct gtc gtc aac att caa aaa gaa att      3583
Ile Ser Gly Ile Asn Ala Ser Val Val Asn Ile Gln Lys Glu Ile
                1155                1160                1165

gac cgc ctc aat gag gtc gct aaa aat tta aat gaa tca ctc att      3628
Asp Arg Leu Asn Glu Val Ala Lys Asn Leu Asn Glu Ser Leu Ile
                1170                1175                1180

gac ctt caa gaa ttg gga aaa tat gag caa tat att aaa tgg cct      3673
Asp Leu Gln Glu Leu Gly Lys Tyr Glu Gln Tyr Ile Lys Trp Pro
                1185                1190                1195

tgg tat gtt tgg ctc ggc ttc att gct gga cta att gcc atc gtc      3718
Trp Tyr Val Trp Leu Gly Phe Ile Ala Gly Leu Ile Ala Ile Val
                1200                1205                1210

atg gtt aca atc ttg ctt tgt tgc atg act agt tgt tgc agt tgc      3763
Met Val Thr Ile Leu Leu Cys Cys Met Thr Ser Cys Cys Ser Cys
                1215                1220                1225

ctc aag ggt gca tgc tct tgt ggt tct tgc tgc aag ttt gat gag      3808
Leu Lys Gly Ala Cys Ser Cys Gly Ser Cys Cys Lys Phe Asp Glu
                1230                1235                1240

gat gac tct gag cca gtt ctc aag ggt gtc aaa tta cat tac aca      3853
Asp Asp Ser Glu Pro Val Leu Lys Gly Val Lys Leu His Tyr Thr
                1245                1250                1255

taaacgaact tatggatttg tttatgagat tttttactct tggatcaatt actgcacagc      3913

cagtaaaaat tgacaatgct tctcctgcaa gt                                    3945

<210>  3

<211>  1255

<212>  PRT

<213>  CORONAVIRUS

<400>  3

Met Phe Ile Phe Leu Leu Phe Leu Thr Leu Thr Ser Gly Ser Asp Leu
1               5                   10                  15

Asp Arg Cys Thr Thr Phe Asp Asp Val Gln Ala Pro Asn Tyr Thr Gln
            20                  25                  30

His Thr Ser Ser Met Arg Gly Val Tyr Tyr Pro Asp Glu Ile Phe Arg
        35                  40                  45

Ser Asp Thr Leu Tyr Leu Thr Gln Asp Leu Phe Leu Pro Phe Tyr Ser
    50                  55                  60

Asn Val Thr Gly Phe His Thr Ile Asn His Thr Phe Gly Asn Pro Val
65                  70                  75                  80

Ile Pro Phe Lys Asp Gly Ile Tyr Phe Ala Ala Thr Glu Lys Ser Asn
                85                  90                  95

Val Val Arg Gly Trp Val Phe Gly Ser Thr Met Asn Asn Lys Ser Gln
            100                 105                 110

Ser Val Ile Ile Ile Asn Asn Ser Thr Asn Val Val Ile Arg Ala Cys
        115                 120                 125

Asn Phe Glu Leu Cys Asp Asn Pro Phe Phe Ala Val Ser Lys Pro Met
    130                 135                 140

Gly Thr Gln Thr His Thr Met Ile Phe Asp Asn Ala Phe Asn Cys Thr
145                 150                 155                 160

Phe Glu Tyr Ile Ser Asp Ala Phe Ser Leu Asp Val Ser Glu Lys Ser
                165                 170                 175

Gly Asn Phe Lys His Leu Arg Glu Phe Val Phe Lys Asn Lys Asp Gly
            180                 185                 190

Phe Leu Tyr Val Tyr Lys Gly Tyr Gln Pro Ile Asp Val Val Arg Asp
        195                 200                 205

Leu Pro Ser Gly Phe Asn Thr Leu Lys Pro Ile Phe Lys Leu Pro Leu
    210                 215                 220

Gly Ile Asn Ile Thr Asn Phe Arg Ala Ile Leu Thr Ala Phe Ser Pro
225                 230                 235                 240

Ala Gln Asp Ile Trp Gly Thr Ser Ala Ala Ala Tyr Phe Val Gly Tyr
                245                 250                 255

Leu Lys Pro Thr Thr Phe Met Leu Lys Tyr Asp Glu Asn Gly Thr Ile
            260                 265                 270

Thr Asp Ala Val Asp Cys Ser Gln Asn Pro Leu Ala Glu Leu Lys Cys
        275                 280                 285

Ser Val Lys Ser Phe Glu Ile Asp Lys Gly Ile Tyr Gln Thr Ser Asn
    290                 295                 300

Phe Arg Val Val Pro Ser Gly Asp Val Val Arg Phe Pro Asn Ile Thr
305                 310                 315                 320

Asn Leu Cys Pro Phe Gly Glu Val Phe Asn Ala Thr Lys Phe Pro Ser
                325                 330                 335

Val Tyr Ala Trp Glu Arg Lys Lys Ile Ser Asn Cys Val Ala Asp Tyr
            340                 345                 350

Ser Val Leu Tyr Asn Ser Thr Phe Phe Ser Thr Phe Lys Cys Tyr Gly
        355                 360                 365

Val Ser Ala Thr Lys Leu Asn Asp Leu Cys Phe Ser Asn Val Tyr Ala
    370                 375                 380

Asp Ser Phe Val Val Lys Gly Asp Asp Val Arg Gln Ile Ala Pro Gly
385                 390                 395                 400

Gln Thr Gly Val Ile Ala Asp Tyr Asn Tyr Lys Leu Pro Asp Asp Phe
                405                 410                 415

Met Gly Cys Val Leu Ala Trp Asn Thr Arg Asn Ile Asp Ala Thr Ser
            420                 425                 430

Thr Gly Asn Tyr Asn Tyr Lys Tyr Arg Tyr Leu Arg His Gly Lys Leu
        435                 440                 445

Arg Pro Phe Glu Arg Asp Ile Ser Asn Val Pro Phe Ser Pro Asp Gly
    450                 455                 460

Lys Pro Cys Thr Pro Pro Ala Leu Asn Cys Tyr Trp Pro Leu Asn Asp
465                 470                 475                 480

Tyr Gly Phe Tyr Thr Thr Thr Gly Ile Gly Tyr Gln Pro Tyr Arg Val
                485                 490                 495

Val Val Leu Ser Phe Glu Leu Leu Asn Ala Pro Ala Thr Val Cys Gly
            500                 505                 510

Pro Lys Leu Ser Thr Asp Leu Ile Lys Asn Gln Cys Val Asn Phe Asn
        515                 520                 525

Phe Asn Gly Leu Thr Gly Thr Gly Val Leu Thr Pro Ser Ser Lys Arg
    530                 535                 540

Phe Gln Pro Phe Gln Gln Phe Gly Arg Asp Val Ser Asp Phe Thr Asp
545                 550                 555                 560

Ser Val Arg Asp Pro Lys Thr Ser Glu Ile Leu Asp Ile Ser Pro Cys
                565                 570                 575

Ser Phe Gly Gly Val Ser Val Ile Thr Pro Gly Thr Asn Ala Ser Ser
            580                 585                 590

Glu Val Ala Val Leu Tyr Gln Asp Val Asn Cys Thr Asp Val Ser Thr
        595                 600                 605

Ala Ile His Ala Asp Gln Leu Thr Pro Ala Trp Arg Ile Tyr Ser Thr
    610                 615                 620

Gly Asn Asn Val Phe Gln Thr Gln Ala Gly Cys Leu Ile Gly Ala Glu
625                 630                 635                 640

His Val Asp Thr Ser Tyr Glu Cys Asp Ile Pro Ile Gly Ala Gly Ile
                645                 650                 655

Cys Ala Ser Tyr His Thr Val Ser Leu Leu Arg Ser Thr Ser Gln Lys
            660                 665                 670

Ser Ile Val Ala Tyr Thr Met Ser Leu Gly Ala Asp Ser Ser Ile Ala
        675                 680                 685

Tyr Ser Asn Asn Thr Ile Ala Ile Pro Thr Asn Phe Ser Ile Ser Ile
    690                 695                 700

Thr Thr Glu Val Met Pro Val Ser Met Ala Lys Thr Ser Val Asp Cys
705                 710                 715                 720

Asn Met Tyr Ile Cys Gly Asp Ser Thr Glu Cys Ala Asn Leu Leu Leu
                725                 730                 735

Gln Tyr Gly Ser Phe Cys Thr Gln Leu Asn Arg Ala Leu Ser Gly Ile
            740                 745                 750

Ala Ala Glu Gln Asp Arg Asn Thr Arg Glu Val Phe Ala Gln Val Lys
        755                 760                 765

Gln Met Tyr Lys Thr Pro Thr Leu Lys Tyr Phe Gly Gly Phe Asn Phe
    770                 775                 780

Ser Gln Ile Leu Pro Asp Pro Leu Lys Pro Thr Lys Arg Ser Phe Ile
785                 790                 795                 800

Glu Asp Leu Leu Phe Asn Lys Val Thr Leu Ala Asp Ala Gly Phe Met
                805                 810                 815

Lys Gln Tyr Gly Glu Cys Leu Gly Asp Ile Asn Ala Arg Asp Leu Ile
            820                 825                 830

Cys Ala Gln Lys Phe Asn Gly Leu Thr Val Leu Pro Pro Leu Leu Thr
        835                 840                 845

Asp Asp Met Ile Ala Ala Tyr Thr Ala Ala Leu Val Ser Gly Thr Ala
    850                 855                 860

Thr Ala Gly Trp Thr Phe Gly Ala Gly Ala Ala Leu Gln Ile Pro Phe
865                 870                 875                 880

Ala Met Gln Met Ala Tyr Arg Phe Asn Gly Ile Gly Val Thr Gln Asn
                885                 890                 895

Val Leu Tyr Glu Asn Gln Lys Gln Ile Ala Asn Gln Phe Asn Lys Ala
            900                 905                 910

Ile Ser Gln Ile Gln Glu Ser Leu Thr Thr Thr Ser Thr Ala Leu Gly
        915                 920                 925

Lys Leu Gln Asp Val Val Asn Gln Asn Ala Gln Ala Leu Asn Thr Leu
    930                 935                 940

Val Lys Gln Leu Ser Ser Asn Phe Gly Ala Ile Ser Ser Val Leu Asn
945                 950                 955                 960

Asp Ile Leu Ser Arg Leu Asp Lys Val Glu Ala Glu Val Gln Ile Asp
                965                 970                 975

Arg Leu Ile Thr Gly Arg Leu Gln Ser Leu Gln Thr Tyr Val Thr Gln
            980                 985                 990

Gln Leu Ile Arg Ala Ala Glu Ile  Arg Ala Ser Ala Asn  Leu Ala Ala
        995                 1000                1005

Thr Lys  Met Ser Glu Cys Val  Leu Gly Gln Ser Lys  Arg Val Asp
    1010                1015                1020

Phe Cys  Gly Lys Gly Tyr His  Leu Met Ser Phe Pro  Gln Ala Ala
    1025                1030                1035

Pro His  Gly Val Val Phe Leu  His Val Thr Tyr Val  Pro Ser Gln
    1040                1045                1050

Glu Arg Asn Phe Thr Thr Ala Pro Ala Ile Cys His Glu Gly Lys
    1055                1060                1065

Ala Tyr Phe Pro Arg Glu Gly Val Phe Val Phe Asn Gly Thr Ser
    1070                1075                1080

Trp Phe Ile Thr Gln Arg Asn Phe Phe Ser Pro Gln Ile Ile Thr
    1085                1090                1095

Thr Asp Asn Thr Phe Val Ser Gly Asn Cys Asp Val Val Ile Gly
    1100                1105                1110

Ile Ile Asn Asn Thr Val Tyr Asp Pro Leu Gln Pro Glu Leu Asp
    1115                1120                1125

Ser Phe Lys Glu Glu Leu Asp Lys Tyr Phe Lys Asn His Thr Ser
    1130                1135                1140

Pro Asp Val Asp Leu Gly Asp Ile Ser Gly Ile Asn Ala Ser Val
    1145                1150                1155

Val Asn Ile Gln Lys Glu Ile Asp Arg Leu Asn Glu Val Ala Lys
    1160                1165                1170

Asn Leu Asn Glu Ser Leu Ile Asp Leu Gln Glu Leu Gly Lys Tyr
    1175                1180                1185

Glu Gln Tyr Ile Lys Trp Pro Trp Tyr Val Trp Leu Gly Phe Ile
    1190                1195                1200

Ala Gly Leu Ile Ala Ile Val Met Val Thr Ile Leu Leu Cys Cys
    1205                1210                1215

Met Thr Ser Cys Cys Ser Cys Leu Lys Gly Ala Cys Ser Cys Gly
    1220                1225                1230

Ser Cys Cys Lys Phe Asp Glu Asp Asp Ser Glu Pro Val Leu Lys
    1235                1240                1245

Gly Val Lys Leu His Tyr Thr
    1250                1255

<210> 4

<211> 3943

<212> DNA

<213> CORONAVIRUS

<400> 4
ctcttctgga aaaaggtagg cttatcatta gagaaaacaa cagagttgtg gtttcaagtg 60

atattcttgt taacaactaa acgaacatgt ttattttctt attatttctt actctcacta 120
gtggtagtga ccttgaccgg tgcaccactt ttgatgatgt tcaagctcct aattacactc 180
aacatacttc atctatgagg ggggtttact atcctgatga aatttttaga tcagacactc 240
tttatttaac tcaggattta tttcttccat tttattctaa tgttacaggg tttcatacta 300
ttaatcatac gtttggcaac cctgtcatac cttttaagga tggtatttat tttgctgcca 360
cagagaaatc aaatgttgtc cgtggttggg tttttggttc taccatgaac aacaagtcac 420
agtcggtgat tattattaac aattctacta atgttgttat acgagcatgt aactttgaat 480
tgtgtgacaa ccctttcttt gctgtttcta aacccatggg tacacagaca catactatga 540
tattcgataa tgcatttaat tgcactttcg agtacatatc tgatgccttt tcgcttgatg 600
tttcagaaaa gtcaggtaat tttaaacact tacgagagtt tgtgtttaaa aataaagatg 660
ggtttctcta tgtttataag ggctatcaac ctatagatgt agttcgtgat ctaccttctg 720
gttttaacac tttgaaacct atttttaagt tgcctcttgg tattaacatt acaaatttta 780
gagccattct tacagccttt tcacctgctc aagacatttg gggcacgtca gctgcagcct 840
attttgttgg ctatttaaag ccaactacat ttatgctcaa gtatgatgaa aatggtacaa 900
tcacagatgc tgttgattgt tctcaaaatc cacttgctga actcaaatgc tctgttaaga 960
gctttgagat tgacaaagga atttaccaga cctctaattt cagggttgtt ccctcaggag 1020
atgttgtgag attccctaat attacaaact tgtgtccttt tggagaggtt tttaatgcta 1080
ctaaattccc ttctgtctat gcatgggaga gaaaaaaaat ttctaattgt gttgctgatt 1140
actctgtgct ctacaactca acattttttt caacctttaa gtgctatggc gtttctgcca 1200
ctaagttgaa tgatctttgc ttctccaatg tctatgcaga ttcttttgta gtcaagggag 1260
atgatgtaag acaaatagcg ccaggacaaa ctggtgttat tgctgattat aattataaat 1320
tgccagatga tttcatgggt tgtgtccttg cttggaatac taggaacatt gatgctactt 1380
caactggtaa ttataattat aaatataggt atcttagaca tggcaagctt aggccctttg 1440
agagagacat atctaatgtg cctttctccc ctgatggcaa accttgcacc ccacctgctc 1500
ttaattgtta ttggccatta aatgattatg gtttttacac cactactggc attggctacc 1560
aaccttacag agttgtagta ctttcttttg aacttttaaa tgcaccggcc acggtttgtg 1620
gaccaaaatt atccactgac cttattaaga accagtgtgt caattttaat tttaatggac 1680
tcactggtac tggtgtgtta actccttctt caaagagatt tcaaccattt caacaatttg 1740
gccgtgatgt ctctgatttc actgattccg ttcgagatcc taaaacatct gaaatattag 1800
acatttcacc ttgctctttt gggggtgtaa gtgtaattac acctggaaca aatgcttcat 1860
ctgaagttgc tgttctatat caagatgtta actgcactga tgtttctaca gcaatccatg 1920
cagatcaact cacaccagct tggcgcatat attctactgg aaacaatgta ttccagactc 1980
aagcaggctg tcttatagga gctgagcatg tcgacacttc ttatgagtgc gacattccta 2040
ttggagctgg catttgtgct agttaccata cagtttcttt attacgtagt actagccaaa 2100

aatctattgt ggcttatact atgtctttag gtgctgatag ttcaattgct tactctaata 2160
acaccattgc tatacctact aacttttcaa ttagcattac tacagaagta atgcctgttt 2220
ctatggctaa aacctccgta gattgtaata tgtacatctg cggagattct actgaatgtg 2280
ctaatttgct tctccaatat ggtagctttt gcacacaact aaatcgtgca ctctcaggta 2340
ttgctgctga acaggatcgc aacacacgtg aagtgttcgc tcaagtcaaa caaatgtaca 2400
aaaccccaac tttgaaatat tttggtggtt ttaatttttc acaaatatta cctgaccctc 2460
taaagccaac taagaggtct tttattgagg acttgctctt taataaggtg acactcgctg 2520
atgctggctt catgaagcaa tatggcgaat gcctaggtga tattaatgct agagatctca 2580
tttgtgcgca gaagttcaat gggcttacag tgttgccacc tctgctcact gatgatatga 2640
ttgctgccta cactgctgct ctagttagtg gtactgccac tgctggatgg acatttggtg 2700
ctggcgctgc tcttcaaata ccttttgcta tgcaaatggc atataggttc aatggcattg 2760
gagttaccca aaatgttctc tatgagaacc aaaaacaaat cgccaaccaa tttaacaagg 2820
cgattagtca aattcaagaa tcacttacaa caacatcaac tgcattgggc aagctgcaag 2880
acgttgttaa ccagaatgct caagcattaa acacacttgt taaacaactt agctctaatt 2940
ttggtgcaat ttcaagtgtg ctaaatgata tcctttcgcg acttgataaa gtcgaggcgg 3000
aggtacaaat tgacaggcta attacaggca gacttcaaag ccttcaaacc tatgtaacac 3060
aacaactaat cagggctgct gaaatcaggg cttctgctaa tcttgctgct actaaaatgt 3120
ctgagtgtgt tcttggacaa tcaaaaagag ttgacttttg tggaaagggc taccacctta 3180
tgtccttccc acaagcagcc ccgcatggtg ttgtcttcct acatgtcacg tatgtgccat 3240
cccaggagag gaacttcacc acagcgccag caatttgtca tgaaggcaaa gcatacttcc 3300
ctcgtgaagg tgtttttgtg tttaatggca cttcttggtt tattacacag aggaacttct 3360
tttctccaca aataattact acagacaata catttgtctc aggaaattgt gatgtcgtta 3420
ttggcatcat taacaacaca gtttatgatc ctctgcaacc tgagcttgac tcattcaaag 3480
aagagctgga caagtacttc aaaaatcata catcaccaga tgttgatctt ggcgacattt 3540
caggcattaa cgcttctgtc gtcaacattc aaaaagaaat tgaccgcctc aatgaggtcg 3600
ctaaaaattt aaatgaatca ctcattgacc ttcaagaatt gggaaaatat gagcaatata 3660
ttaaatggcc ttggtatgtt tggctcggct tcattgctgg actaattgcc atcgtcatgg 3720
ttacaatctt gctttgttgc atgactagtt gttgcagttg cctcaagggt gcatgctctt 3780
gtggttcttg ctgcaagttt gatgaggatg actctgagcc agttctcaag ggtgtcaaat 3840
tacattacac ataaacgaac ttatggattt gtttatgaga ttttttactc ttggatcaat 3900
tactgcacag ccagtaaaaa ttgacaatgc ttctcctgca agt 3943

<210> 5
<211> 2049

<212>  DNA
<213>  CORONAVIRUS

<400>  5
ctcttctgga aaaaggtagg cttatcatta gagaaaacaa cagagttgtg gtttcaagtg      60
atattcttgt taacaactaa acgaacatgt ttattttctt attatttctt actctcacta     120
gtggtagtga ccttgaccgg tgcaccactt ttgatgatgt tcaagctcct aattacactc     180
aacatacttc atctatgagg ggggtttact atcctgatga aatttttaga tcagacactc     240
tttatttaac tcaggattta tttcttccat tttattctaa tgttacaggg tttcatacta     300
ttaatcatac gtttggcaac cctgtcatac cttttaagga tggtatttat tttgctgcca     360
cagagaaatc aaatgttgtc cgtggttggg tttttggttc taccatgaac aacaagtcac     420
agtcggtgat tattattaac aattctacta atgttgttat acgagcatgt aactttgaat     480
tgtgtgacaa ccctttcttt gctgtttcta aacccatggg tacacagaca catactatga     540
tattcgataa tgcatttaat tgcactttcg agtacatatc tgatgccttt tcgcttgatg     600
tttcagaaaa gtcaggtaat tttaaacact tacgagagtt tgtgtttaaa aataaagatg     660
ggtttctcta tgtttataag ggctatcaac ctatagatgt agttcgtgat ctaccttctg     720
gttttaacac tttgaaacct atttttaagt tgcctcttgg tattaacatt acaaatttta     780
gagccattct tacagccttt tcacctgctc aagacatttg gggcacgtca gctgcagcct     840
attttgttgg ctatttaaag ccaactacat ttatgctcaa gtatgatgaa aatggtacaa     900
tcacagatgc tgttgattgt tctcaaaatc cacttgctga actcaaatgc tctgttaaga     960
gctttgagat tgacaaagga atttaccaga cctctaattt cagggttgtt ccctcaggag    1020
atgttgtgag attccctaat attacaaact tgtgtccttt tggagaggtt tttaatgcta    1080
ctaaattccc ttctgtctat gcatgggaga gaaaaaaaat ttctaattgt gttgctgatt    1140
actctgtgct ctacaactca acattttttt caacctttaa gtgctatggc gtttctgcca    1200
ctaagttgaa tgatctttgc ttctccaatg tctatgcaga ttcttttgta gtcaagggag    1260
atgatgtaag acaaatagcg ccaggacaaa ctggtgttat tgctgattat aattataaat    1320
tgccagatga tttcatgggt tgtgtccttg cttggaatac taggaacatt gatgctactt    1380
caactggtaa ttataattat aaatataggt atcttagaca tggcaagctt aggccctttg    1440
agagagacat atctaatgtg cctttctccc ctgatggcaa accttgcacc ccacctgctc    1500
ttaattgtta ttggccatta aatgattatg gtttttacac cactactggc attggctacc    1560
aaccttacag agttgtagta ctttcttttg aacttttaaa tgcaccggcc acggtttgtg    1620
gaccaaaatt atccactgac cttattaaga accagtgtgt caattttaat tttaatggac    1680
tcactggtac tggtgtgtta actccttctt caaagagatt tcaaccattt caacaatttg    1740
gccgtgatgt ctctgatttc actgattccg ttcgagatcc taaaacatct gaaatattag    1800
acatttcacc ttgctctttt gggggtgtaa gtgtaattac acctggaaca aatgcttcat    1860

ctgaagttgc tgttctatat caagatgtta actgcactga tgtttctaca gcaatccatg 1920
cagatcaact cacaccagct tggcgcatat attctactgg aaacaatgta ttccagactc 1980
aagcaggctg tcttatagga gctgagcatg tcgacacttc ttatgagtgc gacattccta 2040
ttggagctg 2049

<210> 6
<211> 2027
<212> DNA
<213> CORONAVIRUS

<400> 6
catgcagatc aactcacacc agcttggcgc atatattcta ctggaaacaa tgtattccag 60
actcaagcag gctgtcttat aggagctgag catgtcgaca cttcttatga gtgcgacatt 120
cctattggag ctggcatttg tgctagttac catacagttt ctttattacg tagtactagc 180
caaaaatcta ttgtggctta tactatgtct ttaggtgctg atagttcaat tgcttactct 240
aataacacca ttgctatacc tactaacttt tcaattagca ttactacaga agtaatgcct 300
gtttctatgg ctaaaacctc cgtagattgt aatatgtaca tctgcggaga ttctactgaa 360
tgtgctaatt tgcttctcca atatggtagc ttttgcacac aactaaatcg tgcactctca 420
ggtattgctg ctgaacagga tcgcaacaca cgtgaagtgt tcgctcaagt caaacaaatg 480
tacaaaaccc caactttgaa atattttggt ggttttaatt tttcacaaat attacctgac 540
cctctaaagc caactaagag gtcttttatt gaggacttgc tctttaataa ggtgacactc 600
gctgatgctg gcttcatgaa gcaatatggc gaatgcctag gtgatattaa tgctagagat 660
ctcatttgtg cgcagaagtt caatgggctt acagtgttgc cacctctgct cactgatgat 720
atgattgctg cctacactgc tgctctagtt agtggtactg ccactgctgg atggacattt 780
ggtgctggcg ctgctcttca aatacctttt gctatgcaaa tggcatatag gttcaatggc 840
attggagtta cccaaaatgt tctctatgag aaccaaaaac aaatcgccaa ccaatttaac 900
aaggcgatta gtcaaattca agaatcactt acaacaacat caactgcatt gggcaagctg 960
caagacgttg ttaaccagaa tgctcaagca ttaaacacac ttgttaaaca acttagctct 1020
aattttggtg caatttcaag tgtgctaaat gatatccttt cgcgacttga taaagtcgag 1080
gcggaggtac aaattgacag gttaattaca ggcagacttc aaagccttca aacctatgta 1140
acacaacaac taatcagggc tgctgaaatc agggcttctg ctaatcttgc tgctactaaa 1200
atgtctgagt gtgttcttgg acaatcaaaa agagttgact tttgtggaaa gggctaccac 1260
cttatgtcct tcccacaagc agccccgcat ggtgttgtct tcctacatgt cacgtatgtg 1320
ccatcccagg agaggaactt caccacagcg ccagcaattt gtcatgaagg caaagcatac 1380
ttccctcgtg aaggtgtttt tgtgtttaat ggcacttctt ggtttattac acagaggaac 1440

ttcttttctc cacaaataat tactacagac aatacatttg tctcaggaaa ttgtgatgtc 1500
gttattggcg tcattaacaa cacagtttat gatcctctgc aacctgagct tgactcattc 1560
aaagaagagc tggacaagta cttcaaaaat catacatcac cagatgttga tcttggcgac 1620
atttcaggca ttaacgcttc tgtcgtcaac attcaaaaag aaattgaccg cctcaatgag 1680
gtcgctaaaa atttaaatga atcactcatt gaccttcaag aattgggaaa atatgagcaa 1740
tatattaaat ggccttggta tgtttggctc ggcttcattg ctggactaat tgccatcgtc 1800
atggttacaa tcttgctttg ttgcatgact agttgttgca gttgcctcaa gggtgcatgc 1860
tcttgtggtt cttgctgcaa gtttgatgag gatgactctg agccagttct caagggtgtc 1920
aaattacatt acacataaac gaacttatgg atttgtttat gagatttttt actcttggat 1980
caattactgc acagccagta aaaattgaca atgcttctcc tgcaagt 2027

<210> 7
<211> 1096
<212> DNA
<213> CORONAVIRUS

<400> 7
tcttgctttg ttgcatgact agttgttgca gttgcctcaa gggtgcatgc tcttgtggtt 60
cttgctgcaa gtttgatgag gatgactctg agccagttct caagggtgtc aaattacatt 120
acacataaac gaacttatgg atttgtttat gagatttttt actcttggat caattactgc 180
acagccagta aaaattgaca atgcttctcc tgcaagtact gttcatgcta cagcaacgat 240
accgctacaa gcctcactcc ctttcggatg gcttgttatt ggcgttgcat ttcttgctgt 300
ttttcagagc gctaccaaaa taattgcgct caataaaaga tggcagctag ccctttataa 360
gggcttccag ttcatttgca atttactgct gctatttgtt accatctatt cacatctttt 420
gcttgtcgct gcaggtatgg aggcgcaatt tttgtacctc tatgccttga tatattttct 480
acaatgcatc aacgcatgta gaattattat gagatgttgg ctttgttgga agtgcaaatc 540
caagaaccca ttactttatg atgccaacta ctttgtttgc tggcacacac ataactatga 600
ctactgtata ccatataaca gtgtcacaga tacaattgtc gttactgaag gtgacggcat 660
ttcaacacca aaactcaaag aagactacca aattggtggt tattctgagg ataggcactc 720
aggtgttaaa gactatgtcg ttgtacatgg ctatttcacc gaagtttact accagcttga 780
gtctacacaa attactacag acactggtat tgaaaatgct acattcttca tctttaacaa 840
gcttgttaaa gacccaccga atgtgcaaat acacacaatc gacggctctt caggagttgc 900
taatccagca atggatccaa tttatgatga gccgacgacg actactagcg tgcctttgta 960
agcacaagaa agtgagtacg aacttatgta ctcattcgtt tcggaagaaa caggtacgtt 1020
aatagttaat agcgtacttc tttttcttgc tttcgtggta ttcttgctag tcacactagc 1080

catccttact gcgctt                                                    1096

<210>  8

<211>  1135

<212>  DNA

<213>  CORONAVIRUS


<400>  8
attgccatcg tcatggttac aatcttgctt tgttgcatga ctagttgttg cagttgcctc      60

aagggtgcat gctcttgtgg ttcttgctgc aagtttgatg aggatgactc tgagccagtt     120

ctcaagggtg tcaaattaca ttacacataa acgaacttat ggatttgttt atgagatttt     180

ttactcttgg atcaattact gcacagccag taaaaattga caatgcttct cctgcaagta     240

ctgttcatgc tacagcaacg ataccgctac aagcctcact ccctttcgga tggcttgtta     300

ttggcgttgc atttcttgct gtttttcaga gcgctaccaa aataattgcg ctcaataaaa     360

gatggcagct agccctttat aagggcttcc agttcatttg caatttactg ctgctatttg     420

ttaccatcta ttcacatctt ttgcttgtcg ctgcaggtat ggaggcgcaa tttttgtacc     480

tctatgcctt gatatatttt ctacaatgca tcaacgcatg tagaattatt atgagatgtt     540

ggctttgttg gaagtgcaaa tccaagaacc cattacttta tgatgccaac tactttgttt     600

gctggcacac acataactat gactactgta taccatataa cagtgtcaca gatacaattg     660

tcgttactga aggtgacggc atttcaacac caaaactcaa agaagactac caaattggtg     720

gttattctga ggataggcac tcaggtgtta aagactatgt cgttgtacat ggctatttca     780

ccgaagttta ctaccagctt gagtctacac aaattactac agacactggt attgaaaatg     840

ctacattctt catctttaac aagcttgtta aagacccacc gaatgtgcaa atacacacaa     900

tcgacggctc ttcaggagtt gctaatccag caatggatcc aatttatgat gagccgacga     960

cgactactag cgtgcctttg taagcacaag aaagtgagta cgaacttatg tactcattcg    1020

tttcggaaga aacaggtacg ttaatagtta atagcgtact tctttttctt gctttcgtgg    1080

tattcttgct agtcacacta gccatcctta ctgcgcttcg attgtgtgcg tactg         1135

<210>  9

<211>  1096

<212>  DNA

<213>  CORONAVIRUS


<220>

<221>  CDS

<222>  (137)..(958)

<223>

<400>  9
tcttgctttg ttgcatgact agttgttgca gttgcctcaa gggtgcatgc tcttgtggtt      60

cttgctgcaa gtttgatgag gatgactctg agccagttct caagggtgtc aaattacatt     120

acacataaac gaactt atg gat ttg ttt atg aga ttt ttt act ctt gga tca     172
                  Met Asp Leu Phe Met Arg Phe Phe Thr Leu Gly Ser
                  1               5                   10

att act gca cag cca gta aaa att gac aat gct tct cct gca agt act       220
Ile Thr Ala Gln Pro Val Lys Ile Asp Asn Ala Ser Pro Ala Ser Thr
        15                  20                  25

gtt cat gct aca gca acg ata ccg cta caa gcc tca ctc cct ttc gga       268
Val His Ala Thr Ala Thr Ile Pro Leu Gln Ala Ser Leu Pro Phe Gly
    30                  35                  40

tgg ctt gtt att ggc gtt gca ttt ctt gct gtt ttt cag agc gct acc       316
Trp Leu Val Ile Gly Val Ala Phe Leu Ala Val Phe Gln Ser Ala Thr
45                  50                  55                  60

aaa ata att gcg ctc aat aaa aga tgg cag cta gcc ctt tat aag ggc       364
Lys Ile Ile Ala Leu Asn Lys Arg Trp Gln Leu Ala Leu Tyr Lys Gly
                65                  70                  75

ttc cag ttc att tgc aat tta ctg ctg cta ttt gtt acc atc tat tca       412
Phe Gln Phe Ile Cys Asn Leu Leu Leu Leu Phe Val Thr Ile Tyr Ser
            80                  85                  90

cat ctt ttg ctt gtc gct gca ggt atg gag gcg caa ttt ttg tac ctc       460
His Leu Leu Leu Val Ala Ala Gly Met Glu Ala Gln Phe Leu Tyr Leu
        95                  100                 105

tat gcc ttg ata tat ttt cta caa tgc atc aac gca tgt aga att att       508
Tyr Ala Leu Ile Tyr Phe Leu Gln Cys Ile Asn Ala Cys Arg Ile Ile
    110                 115                 120

atg aga tgt tgg ctt tgt tgg aag tgc aaa tcc aag aac cca tta ctt       556
Met Arg Cys Trp Leu Cys Trp Lys Cys Lys Ser Lys Asn Pro Leu Leu
125                 130                 135                 140

tat gat gcc aac tac ttt gtt tgc tgg cac aca cat aac tat gac tac       604
Tyr Asp Ala Asn Tyr Phe Val Cys Trp His Thr His Asn Tyr Asp Tyr
                145                 150                 155

tgt ata cca tat aac agt gtc aca gat aca att gtc gtt act gaa ggt       652
Cys Ile Pro Tyr Asn Ser Val Thr Asp Thr Ile Val Val Thr Glu Gly
            160                 165                 170

gac ggc att tca aca cca aaa ctc aaa gaa gac tac caa att ggt ggt       700
Asp Gly Ile Ser Thr Pro Lys Leu Lys Glu Asp Tyr Gln Ile Gly Gly
        175                 180                 185

tat tct gag gat agg cac tca ggt gtt aaa gac tat gtc gtt gta cat       748
Tyr Ser Glu Asp Arg His Ser Gly Val Lys Asp Tyr Val Val Val His
    190                 195                 200

ggc tat ttc acc gaa gtt tac tac cag ctt gag tct aca caa att act       796
Gly Tyr Phe Thr Glu Val Tyr Tyr Gln Leu Glu Ser Thr Gln Ile Thr
205                 210                 215                 220

aca gac act ggt att gaa aat gct aca ttc ttc atc ttt aac aag ctt       844

Thr Asp Thr Gly Ile Glu Asn Ala Thr Phe Phe Ile Phe Asn Lys Leu
                225                 230                 235

gtt aaa gac cca ccg aat gtg caa ata cac aca atc gac ggc tct tca 892
Val Lys Asp Pro Pro Asn Val Gln Ile His Thr Ile Asp Gly Ser Ser
            240                 245                 250

gga gtt gct aat cca gca atg gat cca att tat gat gag ccg acg acg 940
Gly Val Ala Asn Pro Ala Met Asp Pro Ile Tyr Asp Glu Pro Thr Thr
        255                 260                 265

act act agc gtg cct ttg taagcacaag aaagtgagta cgaacttatg 988
Thr Thr Ser Val Pro Leu
    270

tactcattcg tttcggaaga aacaggtacg ttaatagtta atagcgtact tctttttctt 1048

gctttcgtgg tattcttgct agtcacacta gccatcctta ctgcgctt 1096

<210> 10

<211> 274

<212> PRT

<213> CORONAVIRUS

<400> 10

Met Asp Leu Phe Met Arg Phe Phe Thr Leu Gly Ser Ile Thr Ala Gln
1               5                   10                  15

Pro Val Lys Ile Asp Asn Ala Ser Pro Ala Ser Thr Val His Ala Thr
            20                  25                  30

Ala Thr Ile Pro Leu Gln Ala Ser Leu Pro Phe Gly Trp Leu Val Ile
        35                  40                  45

Gly Val Ala Phe Leu Ala Val Phe Gln Ser Ala Thr Lys Ile Ile Ala
    50                  55                  60

Leu Asn Lys Arg Trp Gln Leu Ala Leu Tyr Lys Gly Phe Gln Phe Ile
65                  70                  75                  80

Cys Asn Leu Leu Leu Leu Phe Val Thr Ile Tyr Ser His Leu Leu Leu
                85                  90                  95

Val Ala Ala Gly Met Glu Ala Gln Phe Leu Tyr Leu Tyr Ala Leu Ile
            100                 105                 110

Tyr Phe Leu Gln Cys Ile Asn Ala Cys Arg Ile Ile Met Arg Cys Trp
        115                 120                 125

Leu Cys Trp Lys Cys Lys Ser Lys Asn Pro Leu Leu Tyr Asp Ala Asn
    130                 135                 140

Tyr Phe Val Cys Trp His Thr His Asn Tyr Asp Tyr Cys Ile Pro Tyr
145                 150                 155                 160

Asn Ser Val Thr Asp Thr Ile Val Val Thr Glu Gly Asp Gly Ile Ser
                165                 170                 175

Thr Pro Lys Leu Lys Glu Asp Tyr Gln Ile Gly Gly Tyr Ser Glu Asp
            180                 185                 190

Arg His Ser Gly Val Lys Asp Tyr Val Val Val His Gly Tyr Phe Thr
        195                 200                 205

Glu Val Tyr Tyr Gln Leu Glu Ser Thr Gln Ile Thr Thr Asp Thr Gly
    210                 215                 220

Ile Glu Asn Ala Thr Phe Phe Ile Phe Asn Lys Leu Val Lys Asp Pro
225                 230                 235                 240

Pro Asn Val Gln Ile His Thr Ile Asp Gly Ser Ser Gly Val Ala Asn
                245                 250                 255

Pro Ala Met Asp Pro Ile Tyr Asp Glu Pro Thr Thr Thr Thr Ser Val
            260                 265                 270

Pro Leu

<210>  11

<211>  1096

<212>  DNA

<213>  CORONAVIRUS

<220>

<221>  CDS

<222>  (558)..(1019)

<223>

<400>  11
tcttgctttg ttgcatgact agttgttgca gttgcctcaa gggtgcatgc tcttgtggtt      60

cttgctgcaa gtttgatgag gatgactctg agccagttct caagggtgtc aaattacatt     120

acacataaac gaacttatgg atttgtttat gagatttttt actcttggat caattactgc     180

acagccagta aaaattgaca atgcttctcc tgcaagtact gttcatgcta cagcaacgat     240

accgctacaa gcctcactcc ctttcggatg gcttgttatt ggcgttgcat ttcttgctgt     300

ttttcagagc gctaccaaaa taattgcgct caataaaaga tggcagctag ccctttataa     360

gggcttccag ttcatttgca atttactgct gctatttgtt accatctatt cacatctttt 420

gcttgtcgct gcaggtatgg aggcgcaatt tttgtacctc tatgccttga tatattttct 480

acaatgcatc aacgcatgta gaattattat gagatgttgg ctttgttgga agtgcaaatc 540

caagaaccca ttacttt atg atg cca act act ttg ttt gct ggc aca cac 590
Met Met Pro Thr Thr Leu Phe Ala Gly Thr His
1               5                   10

ata act atg act act gta tac cat ata aca gtg tca cag ata caa ttg 638
Ile Thr Met Thr Thr Val Tyr His Ile Thr Val Ser Gln Ile Gln Leu
            15                  20                  25

tcg tta ctg aag gtg acg gca ttt caa cac caa aac tca aag aag act 686
Ser Leu Leu Lys Val Thr Ala Phe Gln His Gln Asn Ser Lys Lys Thr
        30                  35                  40

acc aaa ttg gtg gtt att ctg agg ata ggc act cag gtg tta aag act 734
Thr Lys Leu Val Val Ile Leu Arg Ile Gly Thr Gln Val Leu Lys Thr
    45                  50                  55

atg tcg ttg tac atg gct att tca ccg aag ttt act acc agc ttg agt 782
Met Ser Leu Tyr Met Ala Ile Ser Pro Lys Phe Thr Thr Ser Leu Ser
60                  65                  70                  75

cta cac aaa tta cta cag aca ctg gta ttg aaa atg cta cat tct tca 830
Leu His Lys Leu Leu Gln Thr Leu Val Leu Lys Met Leu His Ser Ser
                80                  85                  90

tct tta aca agc ttg tta aag acc cac cga atg tgc aaa tac aca caa 878
Ser Leu Thr Ser Leu Leu Lys Thr His Arg Met Cys Lys Tyr Thr Gln
            95                  100                 105

tcg acg gct ctt cag gag ttg cta atc cag caa tgg atc caa ttt atg 926
Ser Thr Ala Leu Gln Glu Leu Leu Ile Gln Gln Trp Ile Gln Phe Met
        110                 115                 120

atg agc cga cga cga cta cta gcg tgc ctt tgt aag cac aag aaa gtg 974
Met Ser Arg Arg Arg Leu Leu Ala Cys Leu Cys Lys His Lys Lys Val
    125                 130                 135

agt acg aac tta tgt act cat tcg ttt cgg aag aaa cag gta cgt 1019
Ser Thr Asn Leu Cys Thr His Ser Phe Arg Lys Lys Gln Val Arg
140                 145                 150

taatagttaa tagcgtactt ctttttcttg ctttcgtggt attcttgcta gtcacactag 1079

ccatccttac tgcgctt 1096

<210> 12

<211> 154

<212> PRT

<213> CORONAVIRUS

<400> 12

Met Met Pro Thr Thr Leu Phe Ala Gly Thr His Ile Thr Met Thr Thr
1               5                   10                  15

Val Tyr His Ile Thr Val Ser Gln Ile Gln Leu Ser Leu Leu Lys Val
            20                  25                  30

Thr Ala Phe Gln His Gln Asn Ser Lys Lys Thr Thr Lys Leu Val Val
        35                  40                  45

Ile Leu Arg Ile Gly Thr Gln Val Leu Lys Thr Met Ser Leu Tyr Met
    50                  55                  60

Ala Ile Ser Pro Lys Phe Thr Thr Ser Leu Ser Leu His Lys Leu Leu
65                  70                  75                  80

Gln Thr Leu Val Leu Lys Met Leu His Ser Ser Ser Leu Thr Ser Leu
                85                  90                  95

Leu Lys Thr His Arg Met Cys Lys Tyr Thr Gln Ser Thr Ala Leu Gln
            100                 105                 110

Glu Leu Leu Ile Gln Gln Trp Ile Gln Phe Met Met Ser Arg Arg Arg
        115                 120                 125

Leu Leu Ala Cys Leu Cys Lys His Lys Lys Val Ser Thr Asn Leu Cys
    130                 135                 140

Thr His Ser Phe Arg Lys Lys Gln Val Arg
145                 150

<210>  13

<211>  332

<212>  DNA

<213>  CORONAVIRUS


<220>

<221>  CDS

<222>  (36)..(263)

<223>


<400>  13
tgcctttgta agcacaagaa agtgagtacg aactt atg tac tca ttc gtt tcg        53
                                       Met Tyr Ser Phe Val Ser
                                       1               5

gaa gaa aca ggt acg tta ata gtt aat agc gta ctt ctt ttt ctt gct      101
Glu Glu Thr Gly Thr Leu Ile Val Asn Ser Val Leu Leu Phe Leu Ala
            10                  15                  20

ttc gtg gta ttc ttg cta gtc aca cta gcc atc ctt act gcg ctt cga      149
Phe Val Val Phe Leu Leu Val Thr Leu Ala Ile Leu Thr Ala Leu Arg
        25                  30                  35

ttg tgt gcg tac tgc tgc aat att gtt aac gtg agt tta gta aaa cca     197
Leu Cys Ala Tyr Cys Cys Asn Ile Val Asn Val Ser Leu Val Lys Pro
    40                  45                  50

acg gtt tac gtc tac tcg cgt gtt aaa aat ctg aac tct tct gaa gga     245
Thr Val Tyr Val Tyr Ser Arg Val Lys Asn Leu Asn Ser Ser Glu Gly
55                  60                  65                  70

gtt cct gat ctt ctg gtc taaacgaact aactattatt attattctgt            293
Val Pro Asp Leu Leu Val
                75

ttggaacttt aacattgctt atcatggcag acaacggta                          332

<210>  14

<211>  76

<212>  PRT

<213>  CORONAVIRUS

<400>  14

Met Tyr Ser Phe Val Ser Glu Glu Thr Gly Thr Leu Ile Val Asn Ser
1               5                   10                  15

Val Leu Leu Phe Leu Ala Phe Val Val Phe Leu Leu Val Thr Leu Ala
            20                  25                  30

Ile Leu Thr Ala Leu Arg Leu Cys Ala Tyr Cys Cys Asn Ile Val Asn
        35                  40                  45

Val Ser Leu Val Lys Pro Thr Val Tyr Val Tyr Ser Arg Val Lys Asn
    50                  55                  60

Leu Asn Ser Ser Glu Gly Val Pro Asp Leu Leu Val
65                  70                  75

<210>  15

<211>  332

<212>  DNA

<213>  CORONAVIRUS

<400>  15
tgcctttgta agcacaagaa agtgagtacg aacttatgta ctcattcgtt tcggaagaaa      60

caggtacgtt aatagttaat agcgtacttc tttttcttgc tttcgtggta ttcttgctag     120

tcacactagc catccttact gcgcttcgat tgtgtgcgta ctgctgcaat attgttaacg     180

tgagtttagt aaaaccaacg gtttacgtct actcgcgtgt taaaaatctg aactcttctg     240

aaggagttcc tgatcttctg gtctaaacga actaactatt attattattc tgtttggaac     300

tttaacattg cttatcatgg cagacaacgg ta                                    332

<210>  16

<211>  708

<212>  DNA

<213>  CORONAVIRUS


<220>

<221>  CDS

<222>  (41)..(703)

<223>


<400>  16
tattattatt attctgtttg gaactttaac attgcttatc atg gca gac aac ggt        55
                                            Met Ala Asp Asn Gly
                                            1               5

act att acc gtt gag gag ctt aaa caa ctc ctg gaa caa tgg aac cta       103
Thr Ile Thr Val Glu Glu Leu Lys Gln Leu Leu Glu Gln Trp Asn Leu
                10                  15                  20

gta ata ggt ttc cta ttc cta gcc tgg att atg tta cta caa ttt gcc       151
Val Ile Gly Phe Leu Phe Leu Ala Trp Ile Met Leu Leu Gln Phe Ala
            25                  30                  35

tat tct aat cgg aac agg ttt ttg tac ata ata aag ctt gtt ttc ctc       199
Tyr Ser Asn Arg Asn Arg Phe Leu Tyr Ile Ile Lys Leu Val Phe Leu
        40                  45                  50

tgg ctc ttg tgg cca gta aca ctt gct tgt ttt gtg ctt gct gct gtc       247
Trp Leu Leu Trp Pro Val Thr Leu Ala Cys Phe Val Leu Ala Ala Val
    55                  60                  65

tac aga att aat tgg gtg act ggc ggg att gcg att gca atg gct tgt       295
Tyr Arg Ile Asn Trp Val Thr Gly Gly Ile Ala Ile Ala Met Ala Cys
70                  75                  80                  85

att gta ggc ttg atg tgg ctt agc tac ttc gtt gct tcc ttc agg ctg       343
Ile Val Gly Leu Met Trp Leu Ser Tyr Phe Val Ala Ser Phe Arg Leu
                90                  95                  100

ttt gct cgt acc cgc tca atg tgg tca ttc aac cca gaa aca aac att       391
Phe Ala Arg Thr Arg Ser Met Trp Ser Phe Asn Pro Glu Thr Asn Ile
            105                 110                 115

ctt ctc aat gtg cct ctc cgg ggg aca att gtg acc aga ccg ctc atg       439
Leu Leu Asn Val Pro Leu Arg Gly Thr Ile Val Thr Arg Pro Leu Met
        120                 125                 130

gaa agt gaa ctt gtc att ggt gct gtg atc att cgt ggt cac ttg cga       487
Glu Ser Glu Leu Val Ile Gly Ala Val Ile Ile Arg Gly His Leu Arg
    135                 140                 145

atg gcc gga cac tcc cta ggg cgc tgt gac att aag gac ctg cca aaa       535
Met Ala Gly His Ser Leu Gly Arg Cys Asp Ile Lys Asp Leu Pro Lys
150                 155                 160                 165

gag atc act gtg gct aca tca cga acg ctt tct tat tac aaa tta gga 583
Glu Ile Thr Val Ala Thr Ser Arg Thr Leu Ser Tyr Tyr Lys Leu Gly
                170                 175                 180

gcg tcg cag cgt gta ggc act gat tca ggt ttt gct gca tac aac cgc 631
Ala Ser Gln Arg Val Gly Thr Asp Ser Gly Phe Ala Ala Tyr Asn Arg
            185                 190                 195

tac cgt att gga aac tat aaa tta aat aca gac cac gcc ggt agc aac 679
Tyr Arg Ile Gly Asn Tyr Lys Leu Asn Thr Asp His Ala Gly Ser Asn
        200                 205                 210

gac aat att gct ttg cta gta cag taagt 708
Asp Asn Ile Ala Leu Leu Val Gln
    215                 220

<210> 17

<211> 221

<212> PRT

<213> CORONAVIRUS

<400> 17

Met Ala Asp Asn Gly Thr Ile Thr Val Glu Glu Leu Lys Gln Leu Leu
1               5                   10                  15

Glu Gln Trp Asn Leu Val Ile Gly Phe Leu Phe Leu Ala Trp Ile Met
            20                  25                  30

Leu Leu Gln Phe Ala Tyr Ser Asn Arg Asn Arg Phe Leu Tyr Ile Ile
        35                  40                  45

Lys Leu Val Phe Leu Trp Leu Leu Trp Pro Val Thr Leu Ala Cys Phe
    50                  55                  60

Val Leu Ala Ala Val Tyr Arg Ile Asn Trp Val Thr Gly Gly Ile Ala
65                  70                  75                  80

Ile Ala Met Ala Cys Ile Val Gly Leu Met Trp Leu Ser Tyr Phe Val
                85                  90                  95

Ala Ser Phe Arg Leu Phe Ala Arg Thr Arg Ser Met Trp Ser Phe Asn
            100                 105                 110

Pro Glu Thr Asn Ile Leu Leu Asn Val Pro Leu Arg Gly Thr Ile Val
        115                 120                 125

Thr Arg Pro Leu Met Glu Ser Glu Leu Val Ile Gly Ala Val Ile Ile
    130                 135                 140

Arg Gly His Leu Arg Met Ala Gly His Ser Leu Gly Arg Cys Asp Ile
145                 150                 155                 160

Lys Asp Leu Pro Lys Glu Ile Thr Val Ala Thr Ser Arg Thr Leu Ser
                165                 170                 175

Tyr Tyr Lys Leu Gly Ala Ser Gln Arg Val Gly Thr Asp Ser Gly Phe
            180                 185                 190

Ala Ala Tyr Asn Arg Tyr Arg Ile Gly Asn Tyr Lys Leu Asn Thr Asp
        195                 200                 205

His Ala Gly Ser Asn Asp Asn Ile Ala Leu Leu Val Gln
    210                 215                 220

<210>  18

<211>  769

<212>  DNA

<213>  CORONAVIRUS

<400>  18
cctgatcttc tggtctaaac gaactaacta ttattattat tctgtttgga actttaacat      60

tgcttatcat ggcagacaac ggtactatta ccgttgagga gcttaaacaa ctcctggaac     120

aatggaacct agtaataggt ttcctattcc tagcctggat tatgttacta caatttgcct     180

attctaatcg gaacaggttt ttgtacataa taaagcttgt tttcctctgg ctcttgtggc     240

cagtaacact tgcttgtttt gtgcttgctg ctgtctacag aattaattgg gtgactggcg     300

ggattgcgat tgcaatggct tgtattgtag gcttgatgtg gcttagctac ttcgttgctt     360

ccttcaggct gtttgctcgt acccgctcaa tgtggtcatt caacccagaa acaaacattc     420

ttctcaatgt gcctctccgg gggacaattg tgaccagacc gctcatggaa agtgaacttg     480

tcattggtgc tgtgatcatt cgtggtcact tgcgaatggc cggacactcc ctagggcgct     540

gtgacattaa ggacctgcca aaagagatca ctgtggctac atcacgaacg ctttcttatt     600

acaaattagg agcgtcgcag cgtgtaggca ctgattcagg ttttgctgca tacaaccgct     660

accgtattgg aaactataaa ttaaatacag accacgccgg tagcaacgac aatattgctt     720

tgctagtaca gtaagtgaca acagatgttt catcttgttg acttccagg                 769

<210>  19

<211>  1231

<212>  DNA

<213>  CORONAVIRUS

<400>  19
taccgtattg gaaactataa attaaataca gaccacgccg gtagcaacga caatattgct      60

ttgctagtac agtaagtgac aacagatgtt tcatcttgtt gacttccagg ttacaatagc 120
agagatattg attatcatta tgaggacttt caggattgct atttggaatc ttgacgttat 180
aataagttca atagtgagac aattatttaa gcctctaact aagaagaatt attcggagtt 240
agatgatgaa gaacctatgg agttagatta tccataaaac gaacatgaaa attattctct 300
tcctgacatt gattgtattt acatcttgcg agctatatca ctatcaggag tgtgttagag 360
gtacgactgt actactaaaa gaaccttgcc catcaggaac atacgagggc aattcaccat 420
ttcaccctct tgctgacaat aaatttgcac taacttgcac tagcacacac tttgcttttg 480
cttgtgctga cggtactcga catacctatc agctgcgtgc aagatcagtt tcaccaaaac 540
ttttcatcag acaagaggag gttcaacaag agctctactc gccacttttt ctcattgttg 600
ctgctctagt atttttaata ctttgcttca ccattaagag aaagacagaa tgaatgagct 660
cactttaatt gacttctatt tgtgcttttt agcctttctg ctattccttg ttttaataat 720
gcttattata ttttggtttt cactcgaaat ccaggatcta gaagaacctt gtaccaaagt 780
ctaaacgaac atgaaacttc tcattgtttt gacttgtatt tctctatgca gttgcatatg 840
cactgtagta cagcgctgtg catctaataa acctcatgtg cttgaagatc cttgtaaggt 900
acaacactag gggtaatact tatagcactg cttggctttg tgctctagga aaggttttac 960
cttttcatag atggcacact atggttcaaa catgcacacc taatgttact atcaactgtc 1020
aagatccagc tggtggtgcg cttatagcta ggtgttggta ccttcatgaa ggtcaccaaa 1080
ctgctgcatt tagagacgta cttgttgttt taaataaacg aacaaattaa aatgtctgat 1140
aatggacccc aatcaaacca acgtagtgcc ccccgcatta catttggtgg acccacagat 1200
tcaactgaca ataaccagaa tggaggacgc a 1231

<210> 20
<211> 1242
<212> DNA
<213> CORONAVIRUS

<400> 20
gcatacaacc gctaccgtat tggaaactat aaattaaata cagaccacgc cggtagcaac 60
gacaatattg ctttgctagt acagtaagtg acaacagatg tttcatcttg ttgacttcca 120
ggttacaata gcagagatat tgattatcat tatgaggact ttcaggattg ctatttggaa 180
tcttgacgtt ataataagtt caatagtgag acagttattt aagcctctaa ctaagaagaa 240
ttattcggag ttagatgatg aagaacctat ggagttagat tatccataaa acgaacatga 300
aaattattct cttcctgaca ttgattgtat ttacatcttg cgagctatat cactatcagg 360
agtgtgttag aggtacgact gtactactaa aagaaccttg cccatcagga acatacgagg 420
gcaattcacc atttcaccct cttgctgaca ataaatttgc actaacttgc actagcacac 480

actttgcttt tgcttgtgct gacggtactc gacataccta tcagctgcgt gcaagatcag 540

tttcaccaaa acttttcatc agacaagagg aggttcaaca agagctctac tcgccacttt 600

ttctcattgt tgctgctcta gtatttttaa tactttgctt caccattaag agaaagacag 660

aatgaatgag ctcactttaa ttgacttcta tttgtgcttt ttagcctttc tgctattcct 720

tgttttaata atgcttatta tattttggtt ttcactcgaa atccaggatc tagaagaacc 780

ttgtaccaaa gtctaaacga acatgaaact tctcattgtt ttgacttgta tttctctatg 840

cagttgcata tgcactgtag tacagcgctg tgcatctaat aaacctcatg tgcttgaaga 900

tccttgtaag gtacaacact aggggtaata cttatagcac tgcttggctt tgtgctctag 960

gaaaggtttt accttttcat agatggcaca ctatggttca aacatgcaca cctaatgtta 1020

ctatcaactg tcaagatcca gctggtggtg cgcttatagc taggtgttgg taccttcatg 1080

aaggtcacca aactgctgca tttagagacg tacttgttgt tttaaataaa cgaacgaatt 1140

aaaatgtctg ataatggacc ccaatcaaac caacgtagtg ccccccgcat tacatttggt 1200

ggacccacag attcaactga caataaccag aatggaggac gc 1242

<210> 21

<211> 1231

<212> DNA

<213> CORONAVIRUS

<220>

<221> CDS

<222> (86)..(274)

<223>

<400> 21
taccgtattg gaaactataa attaaataca gaccacgccg gtagcaacga caatattgct 60

ttgctagtac agtaagtgac aacag atg ttt cat ctt gtt gac ttc cag gtt 112
Met Phe His Leu Val Asp Phe Gln Val
1               5

aca ata gca gag ata ttg att atc att atg agg act ttc agg att gct 160
Thr Ile Ala Glu Ile Leu Ile Ile Ile Met Arg Thr Phe Arg Ile Ala
10                  15                  20                  25

att tgg aat ctt gac gtt ata ata agt tca ata gtg aga caa tta ttt 208
Ile Trp Asn Leu Asp Val Ile Ile Ser Ser Ile Val Arg Gln Leu Phe
                30                  35                  40

aag cct cta act aag aag aat tat tcg gag tta gat gat gaa gaa cct 256
Lys Pro Leu Thr Lys Lys Asn Tyr Ser Glu Leu Asp Asp Glu Glu Pro
            45                  50                  55

atg gag tta gat tat cca taaaacgaac atgaaaatta ttctcttcct 304

Met Glu Leu Asp Tyr Pro
      60

gacattgatt gtatttacat cttgcgagct atatcactat caggagtgtg ttagaggtac      364

gactgtacta ctaaaagaac cttgcccatc aggaacatac gagggcaatt caccatttca      424

ccctcttgct gacaataaat ttgcactaac ttgcactagc acacactttg cttttgcttg      484

tgctgacggt actcgacata cctatcagct gcgtgcaaga tcagtttcac caaaactttt      544

catcagacaa gaggaggttc aacaagagct ctactcgcca ctttttctca ttgttgctgc      604

tctagtattt ttaatacttt gcttcaccat taagagaaag acagaatgaa tgagctcact      664

ttaattgact tctatttgtg ctttttagcc tttctgctat tccttgtttt aataatgctt      724

attatatttt ggttttcact cgaaatccag gatctagaag aaccttgtac caaagtctaa      784

acgaacatga aacttctcat tgttttgact tgtatttctc tatgcagttg catatgcact      844

gtagtacagc gctgtgcatc taataaacct catgtgcttg aagatccttg taaggtacaa      904

cactaggggt aatacttata gcactgcttg gctttgtgct ctaggaaagg ttttaccttt      964

tcatagatgg cacactatgg ttcaaacatg cacacctaat gttactatca actgtcaaga     1024

tccagctggt ggtgcgctta tagctaggtg ttggtacctt catgaaggtc accaaactgc     1084

tgcatttaga gacgtacttg ttgttttaaa taaacgaaca aattaaaatg tctgataatg     1144

gaccccaatc aaaccaacgt agtgcccccc gcattacatt tggtggaccc acagattcaa     1204

ctgacaataa ccagaatgga ggacgca                                         1231

<210>  22

<211>  63

<212>  PRT

<213>  CORONAVIRUS


<400>  22

Met Phe His Leu Val Asp Phe Gln Val Thr Ile Ala Glu Ile Leu Ile
1               5                   10                  15

Ile Ile Met Arg Thr Phe Arg Ile Ala Ile Trp Asn Leu Asp Val Ile
            20                  25                  30

Ile Ser Ser Ile Val Arg Gln Leu Phe Lys Pro Leu Thr Lys Lys Asn
        35                  40                  45

Tyr Ser Glu Leu Asp Asp Glu Glu Pro Met Glu Leu Asp Tyr Pro
    50                  55                  60

<210>  23

<211>  1231

<212>  DNA

<213>  CORONAVIRUS


<220>

<221>  CDS

<222>  (285)..(650)

<223>


<400>  23
taccgtattg gaaactataa attaaataca gaccacgccg gtagcaacga caatattgct      60

ttgctagtac agtaagtgac aacagatgtt tcatcttgtt gacttccagg ttacaatagc     120

agagatattg attatcatta tgaggacttt caggattgct atttggaatc ttgacgttat     180

aataagttca atagtgagac aattatttaa gcctctaact aagaagaatt attcggagtt     240

agatgatgaa gaacctatgg agttagatta tccataaaac gaac atg aaa att att      296
                                                 Met Lys Ile Ile
                                                 1

ctc ttc ctg aca ttg att gta ttt aca tct tgc gag cta tat cac tat      344
Leu Phe Leu Thr Leu Ile Val Phe Thr Ser Cys Glu Leu Tyr His Tyr
5                   10                  15                  20

cag gag tgt gtt aga ggt acg act gta cta cta aaa gaa cct tgc cca      392
Gln Glu Cys Val Arg Gly Thr Thr Val Leu Leu Lys Glu Pro Cys Pro
                25                  30                  35

tca gga aca tac gag ggc aat tca cca ttt cac cct ctt gct gac aat      440
Ser Gly Thr Tyr Glu Gly Asn Ser Pro Phe His Pro Leu Ala Asp Asn
            40                  45                  50

aaa ttt gca cta act tgc act agc aca cac ttt gct ttt gct tgt gct      488
Lys Phe Ala Leu Thr Cys Thr Ser Thr His Phe Ala Phe Ala Cys Ala
        55                  60                  65

gac ggt act cga cat acc tat cag ctg cgt gca aga tca gtt tca cca      536
Asp Gly Thr Arg His Thr Tyr Gln Leu Arg Ala Arg Ser Val Ser Pro
    70                  75                  80

aaa ctt ttc atc aga caa gag gag gtt caa caa gag ctc tac tcg cca      584
Lys Leu Phe Ile Arg Gln Glu Glu Val Gln Gln Glu Leu Tyr Ser Pro
85                  90                  95                  100

ctt ttt ctc att gtt gct gct cta gta ttt tta ata ctt tgc ttc acc      632
Leu Phe Leu Ile Val Ala Ala Leu Val Phe Leu Ile Leu Cys Phe Thr
                105                 110                 115

att aag aga aag aca gaa tgaatgagct cactttaatt gacttctatt             680
Ile Lys Arg Lys Thr Glu
            120

tgtgcttttt agcctttctg ctattccttg ttttaataat gcttattata ttttggtttt     740

cactcgaaat ccaggatcta gaagaacctt gtaccaaagt ctaaacgaac atgaaacttc     800

tcattgtttt gacttgtatt tctctatgca gttgcatatg cactgtagta cagcgctgtg     860

catctaataa acctcatgtg cttgaagatc cttgtaaggt acaacactag gggtaatact     920

tatagcactg cttggctttg tgctctagga aaggttttac cttttcatag atggcacact 980
atggttcaaa catgcacacc taatgttact atcaactgtc aagatccagc tggtggtgcg 1040
cttatagcta ggtgttggta ccttcatgaa ggtcaccaaa ctgctgcatt tagagacgta 1100
cttgttgttt taaataaacg aacaaattaa aatgtctgat aatggacccc aatcaaacca 1160
acgtagtgcc ccccgcatta catttggtgg acccacagat tcaactgaca ataaccagaa 1220
tggaggacgc a 1231

<210> 24
<211> 122
<212> PRT
<213> CORONAVIRUS

<400> 24
Met Lys Ile Ile Leu Phe Leu Thr Leu Ile Val Phe Thr Ser Cys Glu
1               5                   10                  15

Leu Tyr His Tyr Gln Glu Cys Val Arg Gly Thr Thr Val Leu Leu Lys
            20                  25                  30

Glu Pro Cys Pro Ser Gly Thr Tyr Glu Gly Asn Ser Pro Phe His Pro
        35                  40                  45

Leu Ala Asp Asn Lys Phe Ala Leu Thr Cys Thr Ser Thr His Phe Ala
    50                  55                  60

Phe Ala Cys Ala Asp Gly Thr Arg His Thr Tyr Gln Leu Arg Ala Arg
65                  70                  75                  80

Ser Val Ser Pro Lys Leu Phe Ile Arg Gln Glu Glu Val Gln Gln Glu
                85                  90                  95

Leu Tyr Ser Pro Leu Phe Leu Ile Val Ala Ala Leu Val Phe Leu Ile
            100                 105                 110

Leu Cys Phe Thr Ile Lys Arg Lys Thr Glu
        115                 120

<210> 25
<211> 1231
<212> DNA
<213> CORONAVIRUS

<220>

<221>  CDS

<222>  (650)..(781)

<223>

<400>  25
taccgtattg gaaactataa attaaataca gaccacgccg gtagcaacga caatattgct      60

ttgctagtac agtaagtgac aacagatgtt tcatcttgtt gacttccagg ttacaatagc     120

agagatattg attatcatta tgaggacttt caggattgct atttggaatc ttgacgttat     180

aataagttca atagtgagac aattatttaa gcctctaact aagaagaatt attcggagtt     240

agatgatgaa gaacctatgg agttagatta tccataaaac gaacatgaaa attattctct     300

tcctgacatt gattgtattt acatcttgcg agctatatca ctatcaggag tgtgttagag     360

gtacgactgt actactaaaa gaaccttgcc catcaggaac atacgagggc aattcaccat     420

ttcaccctct tgctgacaat aaatttgcac taacttgcac tagcacacac tttgcttttg     480

cttgtgctga cggtactcga catacctatc agctgcgtgc aagatcagtt tcaccaaaac     540

ttttcatcag acaagaggag gttcaacaag agctctactc gccacttttt ctcattgttg     600

ctgctctagt atttttaata ctttgcttca ccattaagag aaagacagaa atg aat gag     658
                                                       Met Asn Glu
                                                       1

ctc act tta att gac ttc tat ttg tgc ttt tta gcc ttt ctg cta ttc       706
Leu Thr Leu Ile Asp Phe Tyr Leu Cys Phe Leu Ala Phe Leu Leu Phe
    5                   10                  15

ctt gtt tta ata atg ctt att ata ttt tgg ttt tca ctc gaa atc cag       754
Leu Val Leu Ile Met Leu Ile Ile Phe Trp Phe Ser Leu Glu Ile Gln
20                  25                  30                  35

gat cta gaa gaa cct tgt acc aaa gtc taaacgaaca tgaaacttct             801
Asp Leu Glu Glu Pro Cys Thr Lys Val
                40

cattgttttg acttgtattt ctctatgcag ttgcatatgc actgtagtac agcgctgtgc     861

atctaataaa cctcatgtgc ttgaagatcc ttgtaaggta caacactagg ggtaatactt     921

atagcactgc ttggctttgt gctctaggaa aggttttacc ttttcataga tggcacacta     981

tggttcaaac atgcacacct aatgttacta tcaactgtca agatccagct ggtggtgcgc    1041

ttatagctag gtgttggtac cttcatgaag gtcaccaaac tgctgcattt agagacgtac    1101

ttgttgtttt aaataaacga acaaattaaa atgtctgata atggacccca atcaaaccaa    1161

cgtagtgccc cccgcattac atttggtgga cccacagatt caactgacaa taaccagaat    1221

ggaggacgca                                                           1231

<210>  26

<211>  44

<212>  PRT
<213>  CORONAVIRUS

<400>  26

Met Asn Glu Leu Thr Leu Ile Asp Phe Tyr Leu Cys Phe Leu Ala Phe
1               5                   10                  15

Leu Leu Phe Leu Val Leu Ile Met Leu Ile Ile Phe Trp Phe Ser Leu
            20                  25                  30

Glu Ile Gln Asp Leu Glu Glu Pro Cys Thr Lys Val
        35                  40

<210>  27
<211>  1231
<212>  DNA
<213>  CORONAVIRUS

<220>
<221>  CDS
<222>  (791)..(907)
<223>

<400>  27
taccgtattg gaaactataa attaaataca gaccacgccg gtagcaacga caatattgct      60
ttgctagtac agtaagtgac aacagatgtt tcatcttgtt gacttccagg ttacaatagc     120
agagatattg attatcatta tgaggacttt caggattgct atttggaatc ttgacgttat     180
aataagttca atagtgagac aattatttaa gcctctaact aagaagaatt attcggagtt     240
agatgatgaa gaacctatgg agttagatta tccataaaac gaacatgaaa attattctct     300
tcctgacatt gattgtattt acatcttgcg agctatatca ctatcaggag tgtgttagag     360
gtacgactgt actactaaaa gaaccttgcc catcaggaac atacgagggc aattcaccat     420
ttcaccctct tgctgacaat aaatttgcac taacttgcac tagcacacac tttgcttttg     480
cttgtgctga cggtactcga catacctatc agctgcgtgc aagatcagtt tcaccaaaac     540
ttttcatcag acaagaggag gttcaacaag agctctactc gccacttttt ctcattgttg     600
ctgctctagt atttttaata ctttgcttca ccattaagag aaagacagaa tgaatgagct     660
cactttaatt gacttctatt tgtgcttttt agcctttctg ctattccttg ttttaataat     720
gcttattata ttttggtttt cactcgaaat ccaggatcta gaagaacctt gtaccaaagt     780
ctaaacgaac atg aaa ctt ctc att gtt ttg act tgt att tct cta tgc        829

Met Lys Leu Leu Ile Val Leu Thr Cys Ile Ser Leu Cys
1               5                   10

agt tgc ata tgc act gta gta cag cgc tgt gca tct aat aaa cct cat     877
Ser Cys Ile Cys Thr Val Val Gln Arg Cys Ala Ser Asn Lys Pro His
    15                  20                  25

gtg ctt gaa gat cct tgt aag gta caa cac taggggtaat acttatagca     927
Val Leu Glu Asp Pro Cys Lys Val Gln His
30                  35

ctgcttggct ttgtgctcta ggaaaggttt taccttttca tagatggcac actatggttc     987

aaacatgcac acctaatgtt actatcaact gtcaagatcc agctggtggt gcgcttatag    1047

ctaggtgttg gtaccttcat gaaggtcacc aaactgctgc atttagagac gtacttgttg    1107

ttttaaataa acgaacaaat taaaatgtct gataatggac cccaatcaaa ccaacgtagt    1167

gccccccgca ttacatttgg tggacccaca gattcaactg acaataacca gaatggagga    1227

cgca                                                                 1231

<210>  28

<211>  39

<212>  PRT

<213>  CORONAVIRUS

<400>  28

Met Lys Leu Leu Ile Val Leu Thr Cys Ile Ser Leu Cys Ser Cys Ile
1               5                   10                  15

Cys Thr Val Val Gln Arg Cys Ala Ser Asn Lys Pro His Val Leu Glu
            20                  25                  30

Asp Pro Cys Lys Val Gln His
        35

<210>  29

<211>  1231

<212>  DNA

<213>  CORONAVIRUS

<220>

<221>  CDS

<222>  (876)..(1127)

<223>

<400> 29
taccgtattg gaaactataa attaaataca gaccacgccg gtagcaacga caatattgct 60

ttgctagtac agtaagtgac aacagatgtt tcatcttgtt gacttccagg ttacaatagc 120

agagatattg attatcatta tgaggacttt caggattgct atttggaatc ttgacgttat 180

aataagttca atagtgagac aattatttaa gcctctaact aagaagaatt attcggagtt 240

agatgatgaa gaacctatgg agttagatta tccataaaac gaacatgaaa attattctct 300

tcctgacatt gattgtattt acatcttgcg agctatatca ctatcaggag tgtgttagag 360

gtacgactgt actactaaaa gaaccttgcc catcaggaac atacgagggc aattcaccat 420

ttcaccctct tgctgacaat aaatttgcac taacttgcac tagcacacac tttgcttttg 480

cttgtgctga cggtactcga catacctatc agctgcgtgc aagatcagtt tcaccaaaac 540

ttttcatcag acaagaggag gttcaacaag agctctactc gccacttttt ctcattgttg 600

ctgctctagt atttttaata ctttgcttca ccattaagag aaagacagaa tgaatgagct 660

cactttaatt gacttctatt tgtgcttttt agcctttctg ctattccttg ttttaataat 720

gcttattata ttttggtttt cactcgaaat ccaggatcta gaagaacctt gtaccaaagt 780

ctaaacgaac atgaaacttc tcattgtttt gacttgtatt tctctatgca gttgcatatg 840

cactgtagta cagcgctgtg catctaataa acctc atg tgc ttg aag atc ctt 893
Met Cys Leu Lys Ile Leu
1               5

gta agg tac aac act agg ggt aat act tat agc act gct tgg ctt tgt 941
Val Arg Tyr Asn Thr Arg Gly Asn Thr Tyr Ser Thr Ala Trp Leu Cys
            10                  15                  20

gct cta gga aag gtt tta cct ttt cat aga tgg cac act atg gtt caa 989
Ala Leu Gly Lys Val Leu Pro Phe His Arg Trp His Thr Met Val Gln
        25                  30                  35

aca tgc aca cct aat gtt act atc aac tgt caa gat cca gct ggt ggt 1037
Thr Cys Thr Pro Asn Val Thr Ile Asn Cys Gln Asp Pro Ala Gly Gly
    40                  45                  50

gcg ctt ata gct agg tgt tgg tac ctt cat gaa ggt cac caa act gct 1085
Ala Leu Ile Ala Arg Cys Trp Tyr Leu His Glu Gly His Gln Thr Ala
55                  60                  65                  70

gca ttt aga gac gta ctt gtt gtt tta aat aaa cga aca aat 1127
Ala Phe Arg Asp Val Leu Val Val Leu Asn Lys Arg Thr Asn
                75                  80

taaaatgtct gataatggac cccaatcaaa ccaacgtagt gccccccgca ttacatttgg 1187

tggacccaca gattcaactg acaataacca gaatggagga cgca 1231

<210> 30

<211> 84

<212> PRT

<213> CORONAVIRUS

<400> 30

Met Cys Leu Lys Ile Leu Val Arg Tyr Asn Thr Arg Gly Asn Thr Tyr
1               5                   10                  15

Ser Thr Ala Trp Leu Cys Ala Leu Gly Lys Val Leu Pro Phe His Arg
            20                  25                  30

Trp His Thr Met Val Gln Thr Cys Thr Pro Asn Val Thr Ile Asn Cys
        35                  40                  45

Gln Asp Pro Ala Gly Gly Ala Leu Ile Ala Arg Cys Trp Tyr Leu His
    50                  55                  60

Glu Gly His Gln Thr Ala Ala Phe Arg Asp Val Leu Val Val Leu Asn
65                  70                  75                  80

Lys Arg Thr Asn

<210> 31
<211> 21221
<212> DNA
<213> CORONAVIRUS

<400> 31
atggagagcc ttgttcttgg tgtcaacgag aaaacacacg tccaactcag tttgcctgtc      60
cttcaggtta gagacgtgct agtgcgtggc ttcggggact ctgtggaaga ggccctatcg     120
gaggcacgtg aacacctcaa aaatggcact tgtggtctag tagagctgga aaaaggcgta     180
ctgccccagc ttgaacagcc ctatgtgttc attaaacgtt ctgatgcctt aagcaccaat     240
cacggccaca aggtcgttga gctggttgca gaaatggacg gcattcagta cggtcgtagc     300
ggtataacac tgggagtact cgtgccacat gtgggcgaaa ccccaattgc ataccgcaat     360
gttcttcttc gtaagaacgg taataaggga gccggtggtc atagctatgg catcgatcta     420
aagtcttatg acttaggtga cgagcttggc actgatccca ttgaagatta tgaacaaaac     480
tggaacacta agcatggcag tggtgcactc cgtgaactca ctcgtgagct caatggaggt     540
gcagtcactc gctatgtcga caacaatttc tgtggcccag atgggtaccc tcttgattgc     600
atcaaagatt ttctcgcacg cgcgggcaag tcaatgtgca ctctttccga acaacttgat     660
tacatcgagt cgaagagagg tgtctactgc tgccgtgacc atgagcatga aattgcctgg     720
ttcactgagc gctctgataa gagctacgag caccagacac ccttcgaaat taagagtgcc     780
aagaaatttg acactttcaa aggggaatgc ccaaagtttg tgtttcctct taactcaaaa     840
gtcaaagtca ttcaaccacg tgttgaaaag aaaaagactg agggtttcat ggggcgtata     900
cgctctgtgt accctgttgc atctccacag gagtgtaaca atatgcactt gtctaccttg     960

atgaaatgta atcattgcga tgaagtttca tggcagacgt gcgactttct gaaagccact 1020
tgtgaacatt gtggcactga aaatttagtt attgaaggac ctactacatg tgggtaccta 1080
cctactaatg ctgtagtgaa aatgccatgt cctgcctgtc aagacccaga gattggacct 1140
gagcatagtg ttgcagatta tcacaaccac tcaaacattg aaactcgact ccgcaaggga 1200
ggtaggacta gatgttttgg aggctgtgtg tttgcctatg ttggctgcta taataagcgt 1260
gcctactggg ttcctcgtgc tagtgctgat attggctcag gccatactgg cattactggt 1320
gacaatgtgg agaccttgaa tgaggatctc cttgagatac tgagtcgtga acgtgttaac 1380
attaacattg ttggcgattt tcatttgaat gaagaggttg ccatcatttt ggcatctttc 1440
tctgcttcta caagtgcctt tattgacact ataaagagtc ttgattacaa gtctttcaaa 1500
accattgttg agtcctgcgg taactataaa gttaccaagg gaaagcccgt aaaaggtgct 1560
tggaacattg gacaacagag atcagtttta acaccactgt gtggttttcc ctcacaggct 1620
gctggtgtta tcagatcaat ttttgcgcgc acacttgatg cagcaaacca ctcaattcct 1680
gatttgcaaa gagcagctgt caccatactt gatggtattt ctgaacagtc attacgtctt 1740
gtcgacgcca tggtttatac ttcagacctg ctcaccaaca gtgtcattat tatggcatat 1800
gtaactggtg gtcttgtaca acagacttct cagtggttgt ctaatctttt gggcactact 1860
gttgaaaaac tcaggcctat ctttgaatgg attgaggcga aacttagtgc aggagttgaa 1920
tttctcaagg atgcttggga gattctcaaa tttctcatta caggtgtttt tgacatcgtc 1980
aagggtcaaa tacaggttgc ttcagataac atcaaggatt gtgtaaaatg cttcattgat 2040
gttgttaaca aggcactcga aatgtgcatt gatcaagtca ctatcgctgg cgcaaagttg 2100
cgatcactca acttaggtga agtcttcatc gctcaaagca agggacttta ccgtcagtgt 2160
atacgtggca aggagcagct gcaactactc atgcctctta aggcaccaaa agaagtaacc 2220
tttcttgaag gtgattcaca tgacacagta cttacctctg aggaggttgt tctcaagaac 2280
ggtgaactcg aagcactcga gacgcccgtt gatagcttca caaatggagc tatcgttggc 2340
acaccagtct gtgtaaatgg cctcatgctc ttagagatta aggacaaaga acaatactgc 2400
gcattgtctc ctggtttact ggctacaaac aatgtctttc gcttaaaagg gggtgcacca 2460
attaaaggtg taacctttgg agaagatact gtttgggaag ttcaaggtta caagaatgtg 2520
agaatcacat ttgagcttga tgaacgtgtt gacaaagtgc ttaatgaaaa gtgctctgtc 2580
tacactgttg aatccggtac cgaagttact gagtttgcat gtgttgtagc agaggctgtt 2640
gtgaagactt tacaaccagt ttctgatctc cttaccaaca tgggtattga tcttgatgag 2700
tggagtgtag ctacattcta cttatttgat gatgctggtg aagaaaactt ttcatcacgt 2760
atgtattgtt ccttttaccc tccagatgag gaagaagagg acgatgcaga gtgtgaggaa 2820
gaagaaattg atgaaacctg tgaacatgag tacggtacag aggatgatta tcaaggtctc 2880
cctctggaat ttggtgcctc agctgaaaca gttcgagttg aggaagaaga agaggaagac 2940
tggctggatg atactactga gcaatcagag attgagccag aaccagaacc tacacctgaa 3000

gaaccagtta atcagtttac tggttattta aaacttactg acaatgttgc cattaaatgt 3060
gttgacatcg ttaaggaggc acaaagtgct aatcctatgg tgattgtaaa tgctgctaac 3120
atacacctga aacatggtgg tggtgtagca ggtgcactca acaaggcaac caatggtgcc 3180
atgcaaaagg agagtgatga ttacattaag ctaaatggcc ctcttacagt aggagggtct 3240
tgtttgcttt ctggacataa tcttgctaag aagtgtctgc atgttgttgg acctaaccta 3300
aatgcaggtg aggacatcca gcttcttaag gcagcatatg aaaatttcaa ttcacaggac 3360
atcttacttg caccattgtt gtcagcaggc atatttggtg ctaaaccact tcagtcttta 3420
caagtgtgcg tgcagacggt tcgtacacag gtttatattg cagtcaatga caaagctctt 3480
tatgagcagg ttgtcatgga ttatcttgat aacctgaagc ctagagtgga agcacctaaa 3540
caagaggagc caccaaacac agaagattcc aaaactgagg agaaatctgt cgtacagaag 3600
cctgtcgatg tgaagccaaa aattaaggcc tgcattgatg aggttaccac aacactggaa 3660
gaaactaagt ttcttaccaa taagttactc ttgtttgctg atatcaatgg taagctttac 3720
catgattctc agaacatgct tagaggtgaa gatatgtctt tccttgagaa ggatgcacct 3780
tacatggtag gtgatgttat cactagtggt gatatcactt gtgttgtaat accctccaaa 3840
aaggctggtg gcactactga gatgctctca agagctttga agaaagtgcc agttgatgag 3900
tatataacca cgtaccctgg acaaggatgt gctggttata cacttgagga agctaagact 3960
gctcttaaga aatgcaaatc tgcattttat gtactacctt cagaagcacc taatgctaag 4020
gaagagattc taggaactgt atcctggaat ttgagagaaa tgcttgctca tgctgaagag 4080
acaagaaaat taatgcctat atgcatggat gttagagcca taatggcaac catccaacgt 4140
aagtataaag gaattaaaat tcaagagggc atcgttgact atggtgtccg attcttcttt 4200
tatactagta aagagcctgt agcttctatt attacgaagc tgaactctct aaatgagccg 4260
cttgtcacaa tgccaattgg ttatgtgaca catggtttta atcttgaaga ggctgcgcgc 4320
tgtatgcgtt ctcttaaagc tcctgccgta gtgtcagtat catcaccaga tgctgttact 4380
acatataatg gatacctcac ttcgtcatca aagacatctg aggagcactt tgtagaaaca 4440
gtttctttgg ctggctctta cagagattgg tcctattcag gacagcgtac agagttaggt 4500
gttgaatttc ttaagcgtgg tgacaaaatt gtgtaccaca ctctggagag ccccgtcgag 4560
tttcatcttg acggtgaggt tctttcactt gacaaactaa agagtctctt atccctgcgg 4620
gaggttaaga ctataaaagt gttcacaact gtggacaaca ctaatctcca cacacagctt 4680
gtggatatgt ctatgacata tggacagcag tttggtccaa catacttgga tggtgctgat 4740
gttacaaaaa ttaaacctca tgtaaatcat gagggtaaga ctttctttgt actacctagt 4800
gatgacacac tacgtagtga agctttcgag tactaccata ctcttgatga gagttttctt 4860
ggtaggtaca tgtctgcttt aaaccacaca aagaaatgga aatttcctca agttggtggt 4920
ttaacttcaa ttaaatgggc tgataacaat tgttatttgt ctagtgtttt attagcactt 4980
caacagcttg aagtcaaatt caatgcacca gcacttcaag aggcttatta tagagcccgt 5040

gctggtgatg ctgctaactt ttgtgcactc atactcgctt acagtaataa aactgttggc 5100
gagcttggtg atgtcagaga aactatgacc catcttctac agcatgctaa tttggaatct 5160
gcaaagcgag ttcttaatgt ggtgtgtaaa cattgtggtc agaaaactac taccttaacg 5220
ggtgtagaag ctgtgatgta tatgggtact ctatcttatg ataatcttaa gacaggtgtt 5280
tccattccat gtgtgtgtgg tcgtgatgct acacaatatc tagtacaaca agagtcttct 5340
tttgttatga tgtctgcacc acctgctgag tataaattac agcaaggtac attcttatgt 5400
gcgaatgagt acactggtaa ctatcagtgt ggtcattaca ctcatataac tgctaaggag 5460
accctctatc gtattgacgg agctcacctt acaaagatgt cagagtacaa aggaccagtg 5520
actgatgttt tctacaagga aacatcttac actacaacca tcaagcctgt gtcgtataaa 5580
ctcgatggag ttacttacac agagattgaa ccaaaattgg atgggtatta taaaaaggat 5640
aatgcttact atacagagca gcctatagac cttgtaccaa ctcaaccatt accaaatgcg 5700
agttttgata atttcaaact cacatgttct aacacaaaat ttgctgatga tttaaatcaa 5760
atgacaggct tcacaaagcc agcttcacga gagctatctg tcacattctt cccagacttg 5820
aatggcgatg tagtggctat tgactataga cactattcag cgagtttcaa gaaaggtgct 5880
aaattactgc ataagccaat tgtttggcac attaaccagg ctacaaccaa gacaacgttc 5940
aaaccaaaca cttggtgttt acgttgtctt tggagtacaa agccagtaga tacttcaaat 6000
tcatttgaag ttctggcagt agaagacaca caaggaatgg acaatcttgc ttgtgaaagt 6060
caacaaccca cctctgaaga agtagtggaa aatcctacca tacagaagga agtcatagag 6120
tgtgacgtga aaactaccga agttgtaggc aatgtcatac ttaaaccatc agatgaaggt 6180
gttaaagtaa cacaagagtt aggtcatgag gatcttatgg ctgcttatgt ggaaaacaca 6240
agcattacca ttaagaaacc taatgagctt tcactagcct taggtttaaa aacaattgcc 6300
actcatggta ttgctgcaat taatagtgtt ccttggagta aaattttggc ttatgtcaaa 6360
ccattcttag gacaagcagc aattacaaca tcaaattgcg ctaagagatt agcacaacgt 6420
gtgtttaaca attatatgcc ttatgtgttt acattattgt tccaattgtg tacttttact 6480
aaaagtacca attctagaat tagagcttca ctacctacaa ctattgctaa aaatagtgtt 6540
aagagtgttg ctaaattatg tttggatgcc ggcattaatt atgtgaagtc acccaaattt 6600
tctaaattgt tcacaatcgc tatgtggcta ttgttgttaa gtatttgctt aggttctcta 6660
atctgtgtaa ctgctgcttt tggtgtactc ttatctaatt ttggtgctcc ttcttattgt 6720
aatggcgtta gagaattgta tcttaattcg tctaacgtta ctactatgga tttctgtgaa 6780
ggttcttttc cttgcagcat ttgtttaagt ggattagact cccttgattc ttatccagct 6840
cttgaaacca ttcaggtgac gatttcatcg tacaagctag acttgacaat tttaggtctg 6900
gccgctgagt gggttttggc atatatgttg ttcacaaaat tcttttattt attaggtctt 6960
tcagctataa tgcaggtgtt ctttggctat tttgctagtc atttcatcag caattcttgg 7020
ctcatgtggt ttatcattag tattgtacaa atggcacccg tttctgcaat ggttaggatg 7080

tacatcttct ttgcttcttt ctactacata tggaagagct atgttcatat catggatggt 7140
tgcacctctt cgacttgcat gatgtgctat aagcgcaatc gtgccacacg cgttgagtgt 7200
acaactattg ttaatggcat gaagagatct ttctatgtct atgcaaatgg aggccgtggc 7260
ttctgcaaga ctcacaattg gaattgtctc aattgtgaca cattttgcac tggtagtaca 7320
ttcattagtg atgaagttgc tcgtgatttg tcactccagt ttaaaagacc aatcaaccct 7380
actgaccagt catcgtatat tgttgatagt gttgctgtga aaaatggcgc gcttcacctc 7440
tactttgaca aggctggtca aaagacctat gagagacatc cgctctccca ttttgtcaat 7500
ttagacaatt tgagagctaa caacactaaa ggttcactgc ctattaatgt catagttttt 7560
gatggcaagt ccaaatgcga cgagtctgct tctaagtctg cttctgtgta ctacagtcag 7620
ctgatgtgcc aacctattct gttgcttgac caagctcttg tatcagacgt tggagatagt 7680
actgaagttt ccgttaagat gtttgatgct tatgtcgaca ccttttcagc aacttttagt 7740
gttcctatgg aaaaacttaa ggcacttgtt gctacagctc acagcgagtt agcaaagggt 7800
gtagctttag atggtgtcct ttctacattc gtgtcagctg cccgacaagg tgttgttgat 7860
accgatgttg acacaaagga tgttattgaa tgtctcaaac tttcacatca ctctgactta 7920
gaagtgacag gtgacagttg taacaatttc atgctcacct ataataaggt tgaaaacatg 7980
acgcccagag atcttggcgc atgtattgac tgtaatgcaa ggcatatcaa tgcccaagta 8040
gcaaaaagtc acaatgtttc actcatctgg aatgtaaaag actacatgtc tttatctgaa 8100
cagctgcgta aacaaattcg tagtgctgcc aagaagaaca acataccttt tagactaact 8160
tgtgctacaa ctagacaggt tgtcaatgtc ataactacta aaatctcact caagggtggt 8220
aagattgtta gtacttgttt taaacttatg cttaaggcca cattattgtg cgttcttgct 8280
gcattggttt gttatatcgt tatgccagta catacattgt caatccatga tggttacaca 8340
aatgaaatca ttggttacaa agccattcag gatggtgtca ctcgtgacat catttctact 8400
gatgattgtt ttgcaaataa acatgctggt tttgacgcat ggtttagcca gcgtggtggt 8460
tcatacaaaa atgacaaaag ctgccctgta gtagctgcta tcattacaag agagattggt 8520
ttcatagtgc ctggcttacc gggtactgtg ctgagagcaa tcaatggtga cttcttgcat 8580
tttctacctc gtgtttttag tgctgttggc aacatttgct acacaccttc caaactcatt 8640
gagtatagtg attttgctac ctctgcttgc gttcttgctg ctgagtgtac aatttttaag 8700
gatgctatgg gcaaacctgt gccatattgt tatgacacta atttgctaga gggttctatt 8760
tcttatagtg agcttcgtcc agacactcgt tatgtgctta tggatggttc catcatacag 8820
tttcctaaca cttacctgga gggttctgtt agagtagtaa caacttttga tgctgagtac 8880
tgtagacatg gtacatgcga aaggtcagaa gtaggtattt gcctatctac cagtggtaga 8940
tgggttctta ataatgagca ttacagagct ctatcaggag ttttctgtgg tgttgatgcg 9000
atgaatctca tagctaacat ctttactcct cttgtgcaac ctgtgggtgc tttagatgtg 9060
tctgcttcag tagtggctgg tggtattatt gccatattgg tgacttgtgc tgcctactac 9120

tttatgaaat tcagacgtgt ttttggtgag tacaaccatg ttgttgctgc taatgcactt 9180
ttgtttttga tgtctttcac tatactctgt ctggtaccag cttacagctt tctgccggga 9240
gtctactcag tcttttactt gtacttgaca ttctatttca ccaatgatgt ttcattcttg 9300
gctcaccttc aatggtttgc catgttttct cctattgtgc ctttttggat aacagcaatc 9360
tatgtattct gtatttctct gaagcactgc cattggttct ttaacaacta tcttaggaaa 9420
agagtcatgt ttaatggagt tacatttagt accttcgagg aggctgcttt gtgtaccttt 9480
ttgctcaaca aggaaatgta cctaaaattg cgtagcgaga cactgttgcc acttacacag 9540
tataacaggt atcttgctct atataacaag tacaagtatt tcagtggagc cttagatact 9600
accagctatc gtgaagcagc ttgctgccac ttagcaaagg ctctaaatga ctttagcaac 9660
tcaggtgctg atgttctcta ccaaccacca cagacatcaa tcacttctgc tgttctgcag 9720
agtggtttta ggaaaatggc attcccgtca ggcaaagttg aagggtgcat ggtacaagta 9780
acctgtggaa ctacaactct taatggattg tggttggatg acacagtata ctgtccaaga 9840
catgtcattt gcacagcaga agacatgctt aatcctaact atgaagatct gctcattcgc 9900
aaatccaacc atagctttct tgttcaggct ggcaatgttc aacttcgtgt tattggccat 9960
tctatgcaaa attgtctgct taggcttaaa gttgatactt ctaaccctaa gacacccaag 10020
tataaatttg tccgtatcca acctggtcaa acattttcag ttctagcatg ctacaatggt 10080
tcaccatctg gtgtttatca gtgtgccatg agacctaatc ataccattaa aggttctttc 10140
cttaatggat catgtggtag tgttggtttt aacattgatt atgattgcgt gtctttctgc 10200
tatatgcatc atatggagct tccaacagga gtacacgctg gtactgactt agaaggtaaa 10260
ttctatggtc catttgttga cagacaaact gcacaggctg caggtacaga cacaaccata 10320
acattaaatg ttttggcatg gctgtatgct gctgttatca atggtgatag gtggtttctt 10380
aatagattca ccactacttt gaatgacttt aaccttgtgg caatgaagta caactatgaa 10440
cctttgacac aagatcatgt tgacatattg ggacctcttt ctgctcaaac aggaattgcc 10500
gtcttagata tgtgtgctgc tttgaaagag ctgctgcaga atggtatgaa tggtcgtact 10560
atccttggta gcactatttt agaagatgag tttacaccat ttgatgttgt tagacaatgc 10620
tctggtgtta ccttccaagg taagttcaag aaaattgtta agggcactca tcattggatg 10680
cttttaactt tcttgacatc actattgatt cttgttcaaa gtacacagtg gtcactgttt 10740
ttctttgttt acgagaatgc tttcttgcca tttactcttg gtattatggc aattgctgca 10800
tgtgctatgc tgcttgttaa gcataagcac gcattcttgt gcttgtttct gttaccttct 10860
cttgcaacag ttgcttactt taatatggtc tacatgcctg ctagctgggt gatgcgtatc 10920
atgacatggc ttgaattggc tgacactagc ttgtctggtt ataggcttaa ggattgtgtt 10980
atgtatgctt cagctttagt tttgcttatt ctcatgacag ctcgcactgt ttatgatgat 11040
gctgctagac gtgtttggac actgatgaat gtcattacac ttgtttacaa agtctactat 11100
ggtaatgctt tagatcaagc tatttccatg tgggccttag ttatttctgt aacctctaac 11160

tattctggtg tcgttacgac tatcatgttt ttagctagag ctatagtgtt tgtgtgtgtt 11220
gagtattacc cattgttatt tattactggc aacaccttac agtgtatcat gcttgtttat 11280
tgtttcttag gctattgttg ctgctgctac tttggccttt tctgtttact caaccgttac 11340
ttcaggctta ctcttggtgt ttatgactac ttggtctcta cacaagaatt taggtatatg 11400
aactcccagg ggcttttgcc tcctaagagt agtattgatg ctttcaagct taacattaag 11460
ttgttgggta ttggaggtaa accatgtatc aaggttgcta ctgtacagtc taaaatgtct 11520
gacgtaaagt gcacatctgt ggtactgctc tcggttcttc aacaacttag agtagagtca 11580
tcttctaaat tgtgggcaca atgtgtacaa ctccacaatg atattcttct tgcaaaagac 11640
acaactgaag ctttcgagaa gatggtttct cttttgtctg ttttgctatc catgcagggt 11700
gctgtagaca ttaataggtt gtgcgaggaa atgctcgata accgtgctac tcttcaggct 11760
attgcttcag aatttagttc tttaccatca tatgccgctt atgccactgc ccaggaggcc 11820
tatgagcagg ctgtagctaa tggtgattct gaagtcgttc tcaaaaagtt aaagaaatct 11880
ttgaatgtgg ctaaatctga gtttgaccgt gatgctgcca tgcaacgcaa gttggaaaag 11940
atggcagatc aggctatgac ccaaatgtac aaacaggcaa gatctgagga caagagggca 12000
aaagtaacta gtgctatgca aacaatgctc ttcactatgc ttaggaagct tgataatgat 12060
gcacttaaca acattatcaa caatgcgcgt gatggttgtg ttccactcaa catcatacca 12120
ttgactacag cagccaaact catggttgtt gtccctgatt atggtaccta caagaacact 12180
tgtgatggta acacctttac atatgcatct gcactctggg aaatccagca agttgttgat 12240
gcggatagca agattgttca acttagtgaa attaacatgg acaattcacc aaatttggct 12300
tggcctctta ttgttacagc tctaagagcc aactcagctg ttaaactaca gaataatgaa 12360
ctgagtccag tagcactacg acagatgtcc tgtgcggctg gtaccacaca aacagcttgt 12420
actgatgaca atgcacttgc ctactataac aattcgaagg gaggtaggtt tgtgctggca 12480
ttactatcag accaccaaga tctcaaatgg gctagattcc ctaagagtga tggtacaggt 12540
acaatttaca cagaactgga accaccttgt aggtttgtta cagacacacc aaaagggcct 12600
aaagtgaaat acttgtactt catcaaaggc ttaaacaacc taaatagagg tatggtgctg 12660
ggcagtttag ctgctacagt acgtcttcag gctggaaatg ctacagaagt acctgccaat 12720
tcaactgtgc tttccttctg tgcttttgca gtagaccctg ctaaagcata taaggattac 12780
ctagcaagtg gaggacaacc aatcaccaac tgtgtgaaga tgttgtgtac acacactggt 12840
acaggacagg caattactgt aacaccagaa gctaacatgg accaagagtc ctttggtggt 12900
gcttcatgtt gtctgtattg tagatgccac attgaccatc caaatcctaa aggattctgt 12960
gacttgaaag gtaagtacgt ccaaatacct accacttgtg ctaatgaccc agtgggtttt 13020
acacttagaa acacagtctg taccgtctgc ggaatgtgga aaggttatgg ctgtagttgt 13080
gaccaactcc gcgaaccctt gatgcagtct gcggatgcat caacgttttt aaacgggttt 13140
gcggtgtaag tgcagcccgt cttacaccgt gcggcacagg cactagtact gatgtcgtct 13200

acagggcttt tgatatttac aacgaaaaag ttgctggttt tgcaaagttc ctaaaaacta 13260
attgctgtcg cttccaggag aaggatgagg aaggcaattt attagactct tactttgtag 13320
ttaagaggca tactatgtct aactaccaac atgaagagac tatttataac ttggttaaag 13380
attgtccagc ggttgctgtc catgactttt tcaagtttag agtagatggt gacatggtac 13440
cacatatatc acgtcagcgt ctaactaaat acacaatggc tgatttagtc tatgctctac 13500
gtcattttga tgagggtaat tgtgatacat taaaagaaat actcgtcaca tacaattgct 13560
gtgatgatga ttatttcaat aagaaggatt ggtatgactt cgtagagaat cctgacatct 13620
tacgcgtata tgctaactta ggtgagcgtg tacgccaatc attattaaag actgtacaat 13680
tctgcgatgc tatgcgtgat gcaggcattg taggcgtact gacattagat aatcaggatc 13740
ttaatgggaa ctggtacgat ttcggtgatt tcgtacaagt agcaccaggc tgcggagttc 13800
ctattgtgga ttcatattac tcattgctga tgcccatcct cactttgact agggcattgg 13860
ctgctgagtc ccatatggat gctgatctcg caaaaccact tattaagtgg gatttgctga 13920
aatatgattt tacggaagag agactttgtc tcttcgaccg ttattttaaa tattgggacc 13980
agacatacca tcccaattgt attaactgtt tggatgatag gtgtatcctt cattgtgcaa 14040
actttaatgt gttattttct actgtgtttc cacctacaag ttttggacca ctagtaagaa 14100
aaatatttgt agatggtgtt ccttttgttg tttcaactgg ataccatttt cgtgagttag 14160
gagtcgtaca taatcaggat gtaaacttac atagctcgcg tctcagtttc aaggaacttt 14220
tagtgtatgc tgctgatcca gctatgcatg cagcttctgg caatttattg ctagataaac 14280
gcactacatg cttttcagta gctgcactaa caaacaatgt tgcttttcaa actgtcaaac 14340
ccggtaattt taataaagac ttttatgact ttgctgtgtc taaaggtttc tttaaggaag 14400
gaagttctgt tgaactaaaa cacttcttct ttgctcagga tggcaacgct gctatcagtg 14460
attatgacta ttatcgttat aatctgccaa caatgtgtga tatcagacaa ctcctattcg 14520
tagttgaagt tgttgataaa tactttgatt gttacgatgg tggctgtatt aatgccaacc 14580
aagtaatcgt taacaatctg gataaatcag ctggtttccc atttaataaa tggggtaagg 14640
ctagacttta ttatgactca atgagttatg aggatcaaga tgcacttttc gcgtatacta 14700
agcgtaatgt catccctact ataactcaaa tgaatcttaa gtatgccatt agtgcaaaga 14760
atagagctcg caccgtagct ggtgtctcta tctgtagtac tatgacaaat agacagtttc 14820
atcagaaatt attgaagtca atagccgcca ctagaggagc tactgtggta attggaacaa 14880
gcaagtttta cggtggctgg cataatatgt taaaaactgt ttacagtgat gtagaaactc 14940
cacaccttat gggttgggat tatccaaaat gtgacagagc catgcctaac atgcttagga 15000
taatggcctc tcttgttctt gctcgcaaac ataacacttg ctgtaactta tcacaccgtt 15060
tctacaggtt agctaacgag tgtgcgcaag tattaagtga gatggtcatg tgtggcggct 15120
cactatatgt taaaccaggt ggaacatcat ccggtgatgc tacaactgct tatgctaata 15180
gtgtctttaa catttgtcaa gctgttacag ccaatgtaaa tgcacttctt tcaactgatg 15240

gtaataagat agctgacaag tatgtccgca atctacaaca caggctctat gagtgtctct 15300
atagaaatag ggatgttgat catgaattcg tggatgagtt ttacgcttac ctgcgtaaac 15360
atttctccat gatgattctt tctgatgatg ccgttgtgtg ctataacagt aactatgcgg 15420
ctcaaggttt agtagctagc attaagaact ttaaggcagt tctttattat caaaataatg 15480
tgttcatgtc tgaggcaaaa tgttggactg agactgacct tactaaagga cctcacgaat 15540
tttgctcaca gcatacaatg ctagttaaac aaggagatga ttacgtgtac ctgccttacc 15600
cagatccatc aagaatatta ggcgcaggct gttttgtcga tgatattgtc aaaacagatg 15660
gtacacttat gattgaaagg ttcgtgtcac tggctattga tgcttaccca cttacaaaac 15720
atcctaatca ggagtatgct gatgtctttc acttgtattt acaatacatt agaaagttac 15780
atgatgagct tactggccac atgttggaca tgtattccgt aatgctaact aatgataaca 15840
cctcacggta ctgggaacct gagttttatg aggctatgta cacaccacat acagtcttgc 15900
aggctgtagg tgcttgtgta ttgtgcaatt cacagacttc acttcgttgc ggtgcctgta 15960
ttaggagacc attcctatgt tgcaagtgct gctatgacca tgtcatttca acatcacaca 16020
aattagtgtt gtctgttaat ccctatgttt gcaatgcccc aggttgtgat gtcactgatg 16080
tgacacaact gtatctagga ggtatgagct attattgcaa gtcacataag cctcccatta 16140
gttttccatt atgtgctaat ggtcaggttt ttggtttata caaaaacaca tgtgtaggca 16200
gtgacaatgt cactgacttc aatgcgatag caacatgtga ttggactaat gctggcgatt 16260
acatacttgc caacacttgt actgagagac tcaagctttt cgcagcagaa acgctcaaag 16320
ccactgagga aacatttaag ctgtcatatg gtattgccac tgtacgcgaa gtactctctg 16380
acagagaatt gcatctttca tgggaggttg gaaaacctag accaccattg aacagaaact 16440
atgtctttac tggttaccgt gtaactaaaa atagtaaagt acagattgga gagtacacct 16500
ttgaaaaagg tgactatggt gatgctgttg tgtacagagg tactacgaca tacaagttga 16560
atgttggtga ttactttgtg ttgacatctc acactgtaat gccacttagt gcacctactc 16620
tagtgccaca agagcactat gtgagaatta ctggcttgta cccaacactc aacatctcag 16680
atgagttttc tagcaatgtt gcaaattatc aaaaggtcgg catgcaaaag tactctacac 16740
tccaaggacc acctggtact ggtaagagtc attttgccat cggacttgct ctctattacc 16800
catctgctcg catagtgtat acggcatgct ctcatgcagc tgttgatgcc ctatgtgaaa 16860
aggcattaaa atatttgccc atagataaat gtagtagaat catacctgcg cgtgcgcgcg 16920
tagagtgttt tgataaattc aaagtgaatt caacactaga acagtatgtt ttctgcactg 16980
taaatgcatt gccagaaaca actgctgaca ttgtagtctt tgatgaaatc tctatggcta 17040
ctaattatga cttgagtgtt gtcaatgcta gacttcgtgc aaaacactac gtctatattg 17100
gcgatcctgc tcaattacca gccccccgca cattgctgac taaaggcaca ctagaaccag 17160
aatattttaa ttcagtgtgc agacttatga aaacaatagg tccagacatg ttccttggaa 17220
cttgtcgccg ttgtcctgct gaaattgttg acactgtgag tgctttagtt tatgacaata 17280

agctaaaagc acacaaggat aagtcagctc aatgcttcaa aatgttctac aaaggtgtta 17340
ttacacatga tgtttcatct gcaatcaaca gacctcaaat aggcgttgta agagaatttc 17400
ttacacgcaa tcctgcttgg agaaaagctg tttttatctc accttataat tcacagaacg 17460
ctgtagcttc aaaaatctta ggattgccta cgcagactgt tgattcatca cagggttctg 17520
aatatgacta tgtcatattc acacaaacta ctgaaacagc acactcttgt aatgtcaacc 17580
gcttcaatgt ggctatcaca agggcaaaaa ttggcatttt gtgcataatg tctgatagag 17640
atctttatga caaactgcaa tttacaagtc tagaaatacc acgtcgcaat gtggctacat 17700
tacaagcaga aaatgtaact ggacttttta aggactgtag taagatcatt actggtcttc 17760
atcctacaca ggcacctaca cacctcagcg ttgatataaa gttcaagact gaaggattat 17820
gtgttgacat accaggcata ccaaaggaca tgacctaccg tagactcatc tctatgatgg 17880
gtttcaaaat gaattaccaa gtcaatggtt accctaatat gtttatcacc cgcgaagaag 17940
ctattcgtca cgttcgtgcg tggattggct ttgatgtaga gggctgtcat gcaactagag 18000
atgctgtggg tactaaccta cctctccagc taggattttc tacaggtgtt aacttagtag 18060
ctgtaccgac tggttatgtt gacactgaaa ataacacaga attcaccaga gttaatgcaa 18120
aacctccacc aggtgaccag tttaaacatc ttataccact catgtataaa ggcttgccct 18180
ggaatgtagt gcgtattaag atagtacaaa tgctcagtga tacactgaaa ggattgtcag 18240
acagagtcgt gttcgtcctt tgggcgcatg gctttgagct tacatcaatg aagtactttg 18300
tcaagattgg acctgaaaga acgtgttgtc tgtgtgacaa acgtgcaact tgcttttcta 18360
cttcatcaga tacttatgcc tgctggaatc attctgtggg ttttgactat gtctataacc 18420
catttatgat tgatgttcag cagtggggct ttacgggtaa ccttcagagt aaccatgacc 18480
aacattgcca ggtacatgga aatgcacatg tggctagttg tgatgctatc atgactagat 18540
gtttagcagt ccatgagtgc tttgttaagc gcgttgattg gtctgttgaa taccctatta 18600
taggagatga actgagggtt aattctgctt gcagaaaagt acaacacatg gttgtgaagt 18660
ctgcattgct tgctgataag tttccagttc ttcatgacat tggaaatcca aaggctatca 18720
agtgtgtgcc tcaggctgaa gtagaatgga agttctacga tgctcagcca tgtagtgaca 18780
aagcttacaa aatagaggaa ctcttctatt cttatgctac acatcacgat aaattcactg 18840
atggtgtttg tttgttttgg aattgtaacg ttgatcgtta cccagccaat gcaattgtgt 18900
gtaggtttga cacaagagtc ttgtcaaact tgaacttacc aggctgtgat ggtggtagtt 18960
tgtatgtgaa taagcatgca ttccacactc cagctttcga taaaagtgca tttactaatt 19020
taaagcaatt gcctttcttt tactattctg atagtccttg tgagtctcat ggcaaacaag 19080
tagtgtcgga tattgattat gttccactca aatctgctac gtgtattaca cgatgcaatt 19140
taggtggtgc tgtttgcaga caccatgcaa atgagtaccg acagtacttg gatgcatata 19200
atatgatgat ttctgctgga tttagcctat ggatttacaa acaatttgat acttataacc 19260
tgtggaatac atttaccagg ttacagagtt tagaaaatgt ggcttataat gttgttaata 19320

aaggacactt tgatggacac gccggcgaag cacctgtttc catcattaat aatgctgttt 19380
acacaaaggt agatggtatt gatgtggaga tctttgaaaa taagacaaca cttcctgtta 19440
atgttgcatt tgagctttgg gctaagcgta acattaaacc agtgccagag attaagatac 19500
tcaataattt gggtgttgat atcgctgcta atactgtaat ctgggactac aaaagagaag 19560
ccccagcaca tgtatctaca ataggtgtct gcacaatgac tgacattgcc aagaaaccta 19620
ctgagagtgc ttgttcttca cttactgtct tgtttgatgg tagagtggaa ggacaggtag 19680
acctttttag aaacgcccgt aatggtgttt taataacaga aggttcagtc aaaggtctaa 19740
caccttcaaa gggaccagca caagctagcg tcaatggagt cacattaatt ggagaatcag 19800
taaaaacaca gtttaactac tttaagaaag tagacggcat tattcaacag ttgcctgaaa 19860
cctactttac tcagagcaga gacttagagg attttaagcc cagatcacaa atggaaactg 19920
actttctcga gctcgctatg gatgaattca tacagcgata taagctcgag ggctatgcct 19980
tcgaacacat cgtttatgga gatttcagtc atggacaact tggcggtctt catttaatga 20040
taggcttagc caagcgctca caagattcac cacttaaatt agaggatttt atccctatgg 20100
acagcacagt gaaaaattac ttcataacag atgcgcaaac aggttcatca aaatgtgtgt 20160
gttctgtgat tgatctttta cttgatgact ttgtcgagat aataaagtca caagatttgt 20220
cagtgatttc aaaagtggtc aaggttacaa ttgactatgc tgaaatttca ttcatgcttt 20280
ggtgtaagga tggacatgtt gaaaccttct acccaaaact acaagcaagt caagcgtggc 20340
aaccaggtgt tgcgatgcct aacttgtaca agatgcaaag aatgcttctt gaaaagtgtg 20400
accttcagaa ttatggtgaa aatgctgtta taccaaaagg aataatgatg aatgtcgcaa 20460
agtatactca actgtgtcaa tacttaaata cacttacttt agctgtaccc tacaacatga 20520
gagttattca ctttggtgct ggctctgata aaggagttgc accaggtaca gctgtgctca 20580
gacaatggtt gccaactggc acactacttg tcgattcaga tcttaatgac ttcgtctccg 20640
acgcagattc tactttaatt ggagactgtg caacagtaca tacggctaat aaaatggacc 20700
ttattattag cgatatgtat gaccctagga ccaaacatgt gacaaaagag aatgactcta 20760
aagaagggtt tttcacttat ctgtgtggat ttataaagca aaaactagcc ctgggtggtt 20820
ctatagctgt aaagataaca gagcattctt ggaatgctga cctttacaag cttatgggcc 20880
atttctcatg gtggacagct tttgttacaa atgtaaatgc atcatcatcg gaagcatttt 20940
taattggggc taactatctt ggcaagccga aggaacaaat tgatggctat accatgcatg 21000
ctaactacat tttctggagg aacacaaatc ctatccagtt gtcttcctat tcactctttg 21060
acatgagcaa atttcctctt aaattaagag gaactgctgt aatgtctctt aaggagaatc 21120
aaatcaatga tatgatttat tctcttctgg aaaaaggtag gcttatcatt agagaaaaca 21180
acagagttgt ggtttcaagt gatattcttg ttaacaacta a 21221

<210> 32

<211>  297

<212>  DNA

<213>  CORONAVIRUS

<400>  32
atggacccca atcaaaccaa cgtagtgccc cccgcattac atttggtgga cccacagatt      60

caactgacaa taaccagaat ggaggacgca atggggcaag gccaaaacag cgccgacccc     120

aaggtttacc caataatact gcgtcttggt tcacagctct cactcagcat ggcaaggagg     180

aacttagatt ccctcgaggc cagggcgttc caatcaacac caatagtggt ccagatgacc     240

aaattggcta ctaccgaaga gctacccgac gagttcgtgg tggtgacggc aaaatga       297

<210>  33

<211>  98

<212>  PRT

<213>  CORONAVIRUS

<400>  33

Met Asp Pro Asn Gln Thr Asn Val Val Pro Pro Ala Leu His Leu Val
1               5                   10                  15

Asp Pro Gln Ile Gln Leu Thr Ile Thr Arg Met Glu Asp Ala Met Gly
            20                  25                  30

Gln Gly Gln Asn Ser Ala Asp Pro Lys Val Tyr Pro Ile Ile Leu Arg
        35                  40                  45

Leu Gly Ser Gln Leu Ser Leu Ser Met Ala Arg Arg Asn Leu Asp Ser
    50                  55                  60

Leu Glu Ala Arg Ala Phe Gln Ser Thr Pro Ile Val Val Gln Met Thr
65                  70                  75                  80

Lys Leu Ala Thr Thr Glu Glu Leu Pro Asp Glu Phe Val Val Val Thr
                85                  90                  95

Ala Lys

<210>  34

<211>  213

<212>  DNA

<213>  CORONAVIRUS

<400>  34
atgctgccac cgtgctacaa cttcctcaag gaacaacatt gccaaaaggc ttctacgcag      60

agggaagcag aggcggcagt caagcctctt ctcgctcctc atcacgtagt cgcggtaatt     120

caagaaattc aactcctggc agcagtaggg gaaattctcc tgctcgaatg gctagcggag     180

gtggtgaaac tgccctcgcg ctattgctgc tag                                  213

<210>  35

<211>  70

<212>  PRT

<213>  CORONAVIRUS

<400>  35

Met Leu Pro Pro Cys Tyr Asn Phe Leu Lys Glu Gln His Cys Gln Lys
1               5                   10                  15

Ala Ser Thr Gln Arg Glu Ala Glu Ala Ala Val Lys Pro Leu Leu Ala
            20                  25                  30

Pro His His Val Val Ala Val Ile Gln Glu Ile Gln Leu Leu Ala Ala
        35                  40                  45

Val Gly Glu Ile Leu Leu Leu Glu Trp Leu Ala Glu Val Val Lys Leu
    50                  55                  60

Pro Ser Arg Tyr Cys Cys
65                  70

<210>  36

<211>  1377

<212>  DNA

<213>  CORONAVIRUS

<220>

<221>  CDS

<222>  (67)..(1335)

<223>

<400>  36
atgaaggtca ccaaactgct gcatttagag acgtacttgt tgttttaaat aaacgaacaa      60

attaaa atg tct gat aat gga ccc caa tca aac caa cgt agt gcc ccc       108

Met Ser Asp Asn Gly Pro Gln Ser Asn Gln Arg Ser Ala Pro
1               5                   10

cgc att aca ttt ggt gga ccc aca gat tca act gac aat aac cag aat     156
Arg Ile Thr Phe Gly Gly Pro Thr Asp Ser Thr Asp Asn Asn Gln Asn
15                  20                  25                  30

gga gga cgc aat ggg gca agg cca aaa cag cgc cga ccc caa ggt tta     204
Gly Gly Arg Asn Gly Ala Arg Pro Lys Gln Arg Arg Pro Gln Gly Leu
                35                  40                  45

ccc aat aat act gcg tct tgg ttc aca gct ctc act cag cat ggc aag     252
Pro Asn Asn Thr Ala Ser Trp Phe Thr Ala Leu Thr Gln His Gly Lys
            50                  55                  60

gag gaa ctt aga ttc cct cga ggc cag ggc gtt cca atc aac acc aat     300
Glu Glu Leu Arg Phe Pro Arg Gly Gln Gly Val Pro Ile Asn Thr Asn
        65                  70                  75

agt ggt cca gat gac caa att ggc tac tac cga aga gct acc cga cga     348
Ser Gly Pro Asp Asp Gln Ile Gly Tyr Tyr Arg Arg Ala Thr Arg Arg
    80                  85                  90

gtt cgt ggt ggt gac ggc aaa atg aaa gag ctc agc ccc aga tgg tac     396
Val Arg Gly Gly Asp Gly Lys Met Lys Glu Leu Ser Pro Arg Trp Tyr
95                  100                 105                 110

ttc tat tac cta gga act ggc cca gaa gct tca ctt ccc tac ggc gct     444
Phe Tyr Tyr Leu Gly Thr Gly Pro Glu Ala Ser Leu Pro Tyr Gly Ala
                115                 120                 125

aac aaa gaa ggc atc gta tgg gtt gca act gag gga gcc ttg aat aca     492
Asn Lys Glu Gly Ile Val Trp Val Ala Thr Glu Gly Ala Leu Asn Thr
            130                 135                 140

ccc aaa gac cac att ggc acc cgc aat cct aat aac aat gct gcc acc     540
Pro Lys Asp His Ile Gly Thr Arg Asn Pro Asn Asn Asn Ala Ala Thr
        145                 150                 155

gtg cta caa ctt cct caa gga aca aca ttg cca aaa ggc ttc tac gca     588
Val Leu Gln Leu Pro Gln Gly Thr Thr Leu Pro Lys Gly Phe Tyr Ala
    160                 165                 170

gag gga agc aga ggc ggc agt caa gcc tct tct cgc tcc tca tca cgt     636
Glu Gly Ser Arg Gly Gly Ser Gln Ala Ser Ser Arg Ser Ser Ser Arg
175                 180                 185                 190

agt cgc ggt aat tca aga aat tca act cct ggc agc agt agg gga aat     684
Ser Arg Gly Asn Ser Arg Asn Ser Thr Pro Gly Ser Ser Arg Gly Asn
                195                 200                 205

tct cct gct cga atg gct agc gga ggt ggt gaa act gcc ctc gcg cta     732
Ser Pro Ala Arg Met Ala Ser Gly Gly Gly Glu Thr Ala Leu Ala Leu
            210                 215                 220

ttg ctg cta gac aga ttg aac cag ctt gag agc aaa gtt tct ggt aaa     780
Leu Leu Leu Asp Arg Leu Asn Gln Leu Glu Ser Lys Val Ser Gly Lys
        225                 230                 235

ggc caa caa caa caa ggc caa act gtc act aag aaa tct gct gct gag     828
Gly Gln Gln Gln Gln Gly Gln Thr Val Thr Lys Lys Ser Ala Ala Glu
    240                 245                 250

gca tct aaa aag cct cgc caa aaa cgt act gcc aca aaa cag tac aac     876
Ala Ser Lys Lys Pro Arg Gln Lys Arg Thr Ala Thr Lys Gln Tyr Asn
255                 260                 265                 270

gtc act caa gca ttt ggg aga cgt ggt cca gaa caa acc caa gga aat     924

Val Thr Gln Ala Phe Gly Arg Arg Gly Pro Glu Gln Thr Gln Gly Asn
                275                 280                 285

ttc ggg gac caa gac cta atc aga caa gga act gat tac aaa cat tgg      972
Phe Gly Asp Gln Asp Leu Ile Arg Gln Gly Thr Asp Tyr Lys His Trp
            290                 295                 300

ccg caa att gca caa ttt gct cca agt gcc tct gca ttc ttt gga atg      1020
Pro Gln Ile Ala Gln Phe Ala Pro Ser Ala Ser Ala Phe Phe Gly Met
        305                 310                 315

tca cgc att ggc atg gaa gtc aca cct tcg gga aca tgg ctg act tat      1068
Ser Arg Ile Gly Met Glu Val Thr Pro Ser Gly Thr Trp Leu Thr Tyr
    320                 325                 330

cat gga gcc att aaa ttg gat gac aaa gat cca caa ttc aaa gac aac      1116
His Gly Ala Ile Lys Leu Asp Asp Lys Asp Pro Gln Phe Lys Asp Asn
335                 340                 345                 350

gtc ata ctg ctg aac aag cac att gac gca tac aaa aca ttc cca cca      1164
Val Ile Leu Leu Asn Lys His Ile Asp Ala Tyr Lys Thr Phe Pro Pro
                355                 360                 365

aca gag cct aaa aag gac aaa aag aaa aag act gat gaa gct cag cct      1212
Thr Glu Pro Lys Lys Asp Lys Lys Lys Lys Thr Asp Glu Ala Gln Pro
            370                 375                 380

ttg ccg cag aga caa aag aag cag ccc act gtg act ctt ctt cct gcg      1260
Leu Pro Gln Arg Gln Lys Lys Gln Pro Thr Val Thr Leu Leu Pro Ala
        385                 390                 395

gct gac atg gat gat ttc tcc aga caa ctt caa aat tcc atg agt gga      1308
Ala Asp Met Asp Asp Phe Ser Arg Gln Leu Gln Asn Ser Met Ser Gly
    400                 405                 410

gct tct gct gat tca act cag gca taa acactcatga tgaccacaca            1355
Ala Ser Ala Asp Ser Thr Gln Ala
415                 420

aggcagatgg gctatgtaaa cg                                             1377

<210>  37

<211>  422

<212>  PRT

<213>  CORONAVIRUS

<400>  37

Met Ser Asp Asn Gly Pro Gln Ser Asn Gln Arg Ser Ala Pro Arg Ile
1               5                   10                  15

Thr Phe Gly Gly Pro Thr Asp Ser Thr Asp Asn Asn Gln Asn Gly Gly
            20                  25                  30

Arg Asn Gly Ala Arg Pro Lys Gln Arg Arg Pro Gln Gly Leu Pro Asn
        35                  40                  45

Asn Thr Ala Ser Trp Phe Thr Ala Leu Thr Gln His Gly Lys Glu Glu
    50                  55                  60

Leu Arg Phe Pro Arg Gly Gln Gly Val Pro Ile Asn Thr Asn Ser Gly
65                  70                  75                  80

Pro Asp Asp Gln Ile Gly Tyr Tyr Arg Arg Ala Thr Arg Arg Val Arg
                85                  90                  95

Gly Gly Asp Gly Lys Met Lys Glu Leu Ser Pro Arg Trp Tyr Phe Tyr
            100                 105                 110

Tyr Leu Gly Thr Gly Pro Glu Ala Ser Leu Pro Tyr Gly Ala Asn Lys
        115                 120                 125

Glu Gly Ile Val Trp Val Ala Thr Glu Gly Ala Leu Asn Thr Pro Lys
    130                 135                 140

Asp His Ile Gly Thr Arg Asn Pro Asn Asn Asn Ala Ala Thr Val Leu
145                 150                 155                 160

Gln Leu Pro Gln Gly Thr Thr Leu Pro Lys Gly Phe Tyr Ala Glu Gly
                165                 170                 175

Ser Arg Gly Gly Ser Gln Ala Ser Ser Arg Ser Ser Ser Arg Ser Arg
            180                 185                 190

Gly Asn Ser Arg Asn Ser Thr Pro Gly Ser Ser Arg Gly Asn Ser Pro
        195                 200                 205

Ala Arg Met Ala Ser Gly Gly Gly Glu Thr Ala Leu Ala Leu Leu Leu
    210                 215                 220

Leu Asp Arg Leu Asn Gln Leu Glu Ser Lys Val Ser Gly Lys Gly Gln
225                 230                 235                 240

Gln Gln Gln Gly Gln Thr Val Thr Lys Lys Ser Ala Ala Glu Ala Ser
                245                 250                 255

Lys Lys Pro Arg Gln Lys Arg Thr Ala Thr Lys Gln Tyr Asn Val Thr
            260                 265                 270

Gln Ala Phe Gly Arg Arg Gly Pro Glu Gln Thr Gln Gly Asn Phe Gly
        275                 280                 285

Asp Gln Asp Leu Ile Arg Gln Gly Thr Asp Tyr Lys His Trp Pro Gln
    290                 295                 300

Ile Ala Gln Phe Ala Pro Ser Ala Ser Ala Phe Phe Gly Met Ser Arg
305                 310                 315                 320

Ile Gly Met Glu Val Thr Pro Ser Gly Thr Trp Leu Thr Tyr His Gly
                325                 330                 335

Ala Ile Lys Leu Asp Asp Lys Asp Pro Gln Phe Lys Asp Asn Val Ile
            340                 345                 350

Leu Leu Asn Lys His Ile Asp Ala Tyr Lys Thr Phe Pro Pro Thr Glu
        355                 360                 365

Pro Lys Lys Asp Lys Lys Lys Lys Thr Asp Glu Ala Gln Pro Leu Pro
    370                 375                 380

Gln Arg Gln Lys Lys Gln Pro Thr Val Thr Leu Leu Pro Ala Ala Asp
385                 390                 395                 400

Met Asp Asp Phe Ser Arg Gln Leu Gln Asn Ser Met Ser Gly Ala Ser
                405                 410                 415

Ala Asp Ser Thr Gln Ala
            420

<210>  38

<211>  1377

<212>  DNA

<213>  CORONAVIRUS

<400>  38
atgaaggtca ccaaactgct gcatttagag acgtacttgt tgttttaaat aaacgaacaa      60

attaaaatgt ctgataatgg accccaatca aaccaacgta gtgccccccg cattacattt     120

ggtggaccca cagattcaac tgacaataac cagaatggag gacgcaatgg ggcaaggcca     180

aaacagcgcc gaccccaagg tttacccaat aatactgcgt cttggttcac agctctcact     240

cagcatggca aggaggaact tagattccct cgaggccagg gcgttccaat caacaccaat     300

agtggtccag atgaccaaat tggctactac cgaagagcta cccgacgagt tcgtggtggt     360

gacggcaaaa tgaaagagct cagccccaga tggtacttct attacctagg aactggccca     420

gaagcttcac ttccctacgg cgctaacaaa gaaggcatcg tatgggttgc aactgaggga     480

gccttgaata cacccaaaga ccacattggc acccgcaatc ctaataacaa tgctgccacc     540

gtgctacaac ttcctcaagg aacaacattg ccaaaaggct tctacgcaga gggaagcaga     600

ggcggcagtc aagcctcttc tcgctcctca tcacgtagtc gcggtaattc aagaaattca     660

actcctggca gcagtagggg aaattctcct gctcgaatgg ctagcggagg tggtgaaact     720

gccctcgcgc tattgctgct agacagattg aaccagcttg agagcaaagt ttctggtaaa     780

ggccaacaac aacaaggcca aactgtcact aagaaatctg ctgctgaggc atctaaaaag     840

cctcgccaaa aacgtactgc cacaaaacag tacaacgtca ctcaagcatt tgggagacgt     900

ggtccagaac aaacccaagg aaatttcggg gaccaagacc taatcagaca aggaactgat     960

tacaaacatt ggccgcaaat tgcacaattt gctccaagtg cctctgcatt ctttggaatg 1020
tcacgcattg gcatggaagt cacaccttcg ggaacatggc tgacttatca tggagccatt 1080
aaattggatg acaaagatcc acaattcaaa gacaacgtca tactgctgaa caagcacatt 1140
gacgcataca aaacattccc accaacagag cctaaaaagg acaaaaagaa aaagactgat 1200
gaagctcagc ctttgccgca gagacaaaag aagcagccca ctgtgactct tcttcctgcg 1260
gctgacatgg atgatttctc cagacaactt caaaattcca tgagtggagc ttctgctgat 1320
tcaactcagg cataaacact catgatgacc acacaaggca gatgggctat gtaaacg 1377

<210> 39
<211> 204
<212> DNA
<213> CORONAVIRUS

<400> 39
atattaggtt tttacctacc caggaaaagc caaccaacct cgatctcttg tagatctgtt 60
ctctaaacga actttaaaat ctgtgtagct gtcgctcggc tgcatgccta gtgcacctac 120
gcagtataaa caataataaa ttttactgtc gttgacaaga aacgagtaac tcgtccctct 180
tctgcagact gcttacggtt tcgt 204

<210> 40
<211> 809
<212> DNA
<213> CORONAVIRUS

<400> 40
actcaagcat ttgggagacg tggtccagaa caaacccaag gaaatttcgg ggaccaagac 60
ctaatcagac aaggaactga ttacaaacat tggccgcaaa ttgcacaatt tgctccaagt 120
gcctctgcat tctttggaat gtcacgcatt ggcatggaag tcacaccttc gggaacatgg 180
ctgacttatc atggagccat taaattggat gacaaagatc cacaattcaa agacaacgtc 240
atactgctga acaagcacat tgacgcatac aaaacattcc caccaacaga gcctaaaaag 300
gacaaaaaga aaaagactga tgaagctcag cctttgccgc agagacaaaa gaagcagccc 360
actgtgactc ttcttcctgc ggctgacatg gatgatttct ccagacaact tcaaaattcc 420
atgagtggag cttctgctga ttcaactcag gcataaacac tcatgatgac cacacaaggc 480
agatgggcta tgtaaacgtt ttcgcaattc cgtttacgat acatagtcta ctcttgtgca 540
gaatgaattc tcgtaactaa acagcacaag taggtttagt taactttaat ctcacatagc 600
aatctttaat caatgtgtaa cattagggag gacttgaaag agccaccaca ttttcatcga 660

ggccacgcgg agtacgatcg agggtacagt gaataatgct agggagagct gcctatatgg 720
aagagcccta atgtgtaaaa ttaattttag tagtgctatc cccatgtgat tttaatagct 780
tcttaggaga atgacaaaaa aaaaaaaaa 809

<210> 41
<211> 448
<212> DNA
<213> CORONAVIRUS

<400> 41
aatgaacaca tagggctgtt caagctgggg cagtacgcct ttttccagct ctactagacc 60
acaagtgcca tttttgaggt gttcacgtgc ctccgatagg gcctcttcca cagagtcccc 120
gaagccacgc actagcacgt ctctaacctg aaggacaggc aaactgagtt ggacgtgtgt 180
tttctcgttg acaccaagaa caaggctctc catcttacct ttcggtcaca cccggacgaa 240
acctaggtat gctgatgatc gactgcaaca cggacgaaac cgtaagcagt ctgcagaaga 300
gggacgagtt actcgtttct tgtcaacgac agtaaaattt attattgttt atactgcgta 360
ggtgcactag gcatgcagcc gagcgacagc tacacagatt ttaaagttcg tttagagaac 420
agatctacaa gagatcgagg ttggttgg 448

<210> 42
<211> 2033
<212> DNA
<213> CORONAVIRUS

<400> 42
atacctaggt ttcgtccggg tgtgaccgaa aggtaagatg gagagccttg ttcttggtgt 60
caacgagaaa acacacgtcc aactcagttt gcctgtcctt caggttagag acgtgctagt 120
gcgtggcttc ggggactctg tggaagaggc cctatcggag gcacgtgaac acctcaaaaa 180
tggcacttgt ggtctagtag agctggaaaa aggcgtactg ccccagcttg aacagcccta 240
tgtgttcatt aaacgttctg atgccttaag caccaatcac ggccacaagg tcgttgagct 300
ggttgcagaa atggacggca ttcagtacgg tcgtagcggt ataacactgg gagtactcgt 360
gccacatgtg ggcgaaaccc caattgcata ccgcaatgtt cttcttcgta agaacggtaa 420
taagggagcc ggtggtcata gctatggcat cgatctaaag tcttatgact taggtgacga 480
gcttggcact gatcccattg aagattatga acaaaactgg aacactaagc atggcagtgg 540
tgcactccgt gaactcactc gtgagctcaa tggaggtgca gtcactcgct atgtcgacaa 600
caatttctgt ggcccagatg ggtaccctct tgattgcatc aaagattttc tcgcacgcgc 660

gggcaagtca atgtgcactc tttccgaaca acttgattac atcgagtcga agagaggtgt 720
ctactgctgc cgtgaccatg agcatgaaat tgcctggttc actgagcgct ctgataagag 780
ctacgagcac cagacaccct tcgaaattaa gagtgccaag aaatttgaca ctttcaaagg 840
ggaatgccca aagtttgtgt ttcctcttaa ctcaaaagtc aaagtcattc aaccacgtgt 900
tgaaaagaaa aagactgagg gtttcatggg gcgtatacgc tctgtgtacc ctgttgcatc 960
tccacaggag tgtaacaata tgcacttgtc taccttgatg aaatgtaatc attgcgatga 1020
agtttcatgg cagacgtgcg actttctgaa agccacttgt gaacattgtg gcactgaaaa 1080
tttagttatt gaaggaccta ctacatgtgg gtacctacct actaatgctg tagtgaaaat 1140
gccatgtcct gcctgtcaag acccagagat tggacctgag catagtgttg cagattatca 1200
caaccactca aacattgaaa ctcgactccg caagggaggt aggactagat gttttggagg 1260
ctgtgtgttt gcctatgttg gctgctataa taagcgtgcc tactgggttc ctcgtgctag 1320
tgctgatatt ggctcaggcc atactggcat tactggtgac aatgtggaga ccttgaatga 1380
ggatctcctt gagatactga gtcgtgaacg tgttaacatt aacattgttg gcgattttca 1440
tttgaatgaa gaggttgcca tcattttggc atctttctct gcttctacaa gtgcctttat 1500
tgacactata aagagtcttg attacaagtc tttcaaaacc attgttgagt cctgcggtaa 1560
ctataaagtt accaagggaa agcccgtaaa aggtgcttgg aacattggac aacagagatc 1620
agttttaaca ccactgtgtg gttttccctc acaggctgct ggtgttatca gatcaatttt 1680
tgcgcgcaca cttgatgcag caaaccactc aattcctgat ttgcaaagag cagctgtcac 1740
catacttgat ggtatttctg aacagtcatt acgtcttgtc gacgccatgg tttatacttc 1800
agacctgctc accaacagtg tcattattat ggcatatgta actggtggtc ttgtacaaca 1860
gacttctcag tggttgtcta atcttttggg cactactgtt gaaaaactca ggcctatctt 1920
tgaatggatt gaggcgaaac ttagtgcagg agttgaattt ctcaaggatg cttgggagat 1980
tctcaaattt ctcattacag gtgtttttga catcgtcaag ggtcaaatac agg 2033

<210> 43
<211> 2018
<212> DNA
<213> CORONAVIRUS

<400> 43
ggattgaggc gaaacttagt gcaggagttg aatttctcaa ggatgcttgg gagattctca 60
aatttctcat tacaggtgtt tttgacatcg tcaagggtca aatacaggtt gcttcagata 120
acatcaagga ttgtgtaaaa tgcttcattg atgttgttaa caaggcactc gaaatgtgca 180
ttgatcaagt cactatcgct ggcgcaaagt tgcgatcact caacttaggt gaagtcttca 240
tcgctcaaag caagggactt taccgtcagt gtatacgtgg caaggagcag ctgcaactac 300

tcatgcctct taaggcacca aaagaagtaa cctttcttga aggtgattca catgacacag 360
tacttacctc tgaggaggtt gttctcaaga acggtgaact cgaagcactc gagacgcccg 420
ttgatagctt cacaaatgga gctatcgttg gcacaccagt ctgtgtaaat ggcctcatgc 480
tcttagagat taaggacaaa gaacaatact gcgcattgtc tcctggttta ctggctacaa 540
acaatgtctt tcgcttaaaa gggggtgcac caattaaagg tgtaaccttt ggagaagata 600
ctgtttggga agttcaaggt tacaagaatg tgagaatcac atttgagctt gatgaacgtg 660
ttgacaaagt gcttaatgaa aagtgctctg tctacactgt tgaatccggt accgaagtta 720
ctgagtttgc atgtgttgta gcagaggctg ttgtgaagac tttacaacca gtttctgatc 780
tccttaccaa catgggtatt gatcttgatg agtggagtgt agctacattc tacttatttg 840
atgatgctgg tgaagaaaac ttttcatcac gtatgtattg ttccttttac cctccagatg 900
aggaagaaga ggacgatgca gagtgtgagg aagaagaaat tgatgaaacc tgtgaacatg 960
agtacggtac agaggatgat tatcaaggtc tccctctgga atttggtgcc tcagctgaaa 1020
cagttcgagt tgaggaagaa gaagaggaag actggctgga tgatactact gagcaatcag 1080
agattgagcc agaaccagaa cctacacctg aagaaccagt taatcagttt actggttatt 1140
taaaacttac tgacaatgtt gccattaaat gtgttgacat cgttaaggag gcacaaagtg 1200
ctaatcctat ggtgattgta aatgctgcta acatacacct gaaacatggt ggtggtgtag 1260
caggtgcact caacaaggca accaatggtg ccatgcaaaa ggagagtgat gattacatta 1320
agctaaatgg ccctcttaca gtaggagggt cttgtttgct ttctggacat aatcttgcta 1380
agaagtgtct gcatgttgtt ggacctaacc taaatgcagg tgaggacatc cagcttctta 1440
aggcagcata tgaaaatttc aattcacagg acatcttact tgcaccattg ttgtcagcag 1500
gcatatttgg tgctaaacca cttcagtctt tacaagtgtg cgtgcagacg gttcgtacac 1560
aggtttatat tgcagtcaat gacaaagctc tttatgagca ggttgtcatg gattatcttg 1620
ataacctgaa gcctagagtg gaagcaccta aacaagagga gccaccaaac acagaagatt 1680
ccaaaactga ggagaaatct gtcgtacaga agcctgtcga tgtgaagcca aaaattaagg 1740
cctgcattga tgaggttacc acaacactgg aagaaactaa gtttcttacc aataagttac 1800
tcttgtttgc tgatatcaat ggtaagcttt accatgattc tcagaacatg cttagaggtg 1860
aagatatgtc tttccttgag aaggatgcac cttacatggt aggtgatgtt atcactagtg 1920
gtgatatcac ttgtgttgta ataccctcca aaaaggctgg tggcactact gagatgctct 1980
caagagcttt gaagaaagtg ccagttgatg agtatata 2018

<210> 44
<211> 1442
<212> DNA
<213> CORONAVIRUS

<400> 44
ttgatgaggt taccacaaca ctggaagaaa ctaagtttct taccaataag ttactcttgt 60

ttgctgatat caatggtaag ctttaccatg attctcagaa catgcttaga ggtgaagata 120

tgtctttcct tgagaaggat gcaccttaca tggtaggtga tgttatcact agtggtgata 180

tcacttgtgt tgtaataccc tccaaaaagg ctggtggcac tactgagatg ctctcaagag 240

ctttgaagaa agtgccagtt gatgagtata taaccacgta ccctggacaa ggatgtgctg 300

gttatacact tgaggaagct aagactgctc ttaagaaatg caaatctgca ttttatgtac 360

taccttcaga agcacctaat gctaaggaag agattctagg aactgtatcc tggaatttga 420

gagaaatgct tgctcatgct gaagagacaa gaaaattaat gcctatatgc atggatgtta 480

gagccataat ggcaaccatc caacgtaagt ataaaggaat taaaattcaa gagggcatcg 540

ttgactatgg tgtccgattc ttcttttata ctagtaaaga gcctgtagct tctattatta 600

cgaagctgaa ctctctaaat gagccgcttg tcacaatgcc aattggttat gtgacacatg 660

gttttaatct tgaagaggct gcgcgctgta tgcgttctct taaagctcct gccgtagtgt 720

cagtatcatc accagatgct gttactacat ataatggata cctcacttcg tcatcaaaga 780

catctgagga gcactttgta gaaacagttt ctttggctgg ctcttacaga gattggtcct 840

attcaggaca gcgtacagag ttaggtgttg aatttcttaa gcgtggtgac aaaattgtgt 900

accacactct ggagagcccc gtcgagtttc atcttgacgg tgaggttctt tcacttgaca 960

aactaaagag tctcttatcc ctgcgggagg ttaagactat aaaagtgttc acaactgtgg 1020

acaacactaa tctccacaca cagcttgtgg atatgtctat gacatatgga cagcagtttg 1080

gtccaacata cttggatggt gctgatgtta caaaaattaa acctcatgta aatcatgagg 1140

gtaagacttt ctttgtacta cctagtgatg acacactacg tagtgaagct ttcgagtact 1200

accatactct tgatgagagt tttcttggta ggtacatgtc tgctttaaac cacacaaaga 1260

aatggaaatt tcctcaagtt ggtggtttaa cttcaattaa atgggctgat aacaattgtt 1320

atttgtctag tgttttatta gcacttcaac agcttgaagt caaattcaat gcaccagcac 1380

ttcaagaggc ttattataga gcccgtgctg gtgatgctgc taacttttgt gcactcatac 1440

tc 1442

<210> 45

<211> 1050

<212> DNA

<213> CORONAVIRUS

<400> 45
atatgtctat gacatatgga cagcagtttg gtccaacata cttggatggt gctgatgtta 60

caaaaattaa acctcatgta aatcatgagg gtaagacttt ctttgtacta cctagtgatg 120

acacactacg tagtgaagct ttcgagtact accatactct tgatgagagt tttcttggta 180

ggtacatgtc tgctttaaac cacacaaaga aatggaaatt tcctcaagtt ggtggtttaa 240

cttcaattaa atgggctgat aacaattgtt atttgtctag tgttttatta gcacttcaac 300

agcttgaagt caaattcaat gcaccagcac ttcaagaggc ttattataga gcccgtgctg 360

gtgatgctgc taacttttgt gcactcatac tcgcttacag taataaaact gttggcgagc 420

ttggtgatgt cagagaaact atgacccatc ttctacagca tgctaatttg gaatctgcaa 480

agcgagttct taatgtggtg tgtaaacatt gtggtcagaa aactactacc ttaacgggtg 540

tagaagctgt gatgtatatg ggtactctat cttatgataa tcttaagaca ggtgtttcca 600

ttccatgtgt gtgtggtcgt gatgctacac aatatctagt acaacaagag tcttcttttg 660

ttatgatgtc tgcaccacct gctgagtata aattacagca aggtacattc ttatgtgcga 720

atgagtacac tggtaactat cagtgtggtc attacactca tataactgct aaggagaccc 780

tctatcgtat tgacggagct caccttacaa agatgtcaga gtacaaagga ccagtgactg 840

atgttttcta caaggaaaca tcttacacta caaccatcaa gcctgtgtcg tataaactcg 900

atggagttac ttacacagag attgaaccaa aattggatgg gtattataaa aaggataatg 960

cttactatac agagcagcct atagaccttg taccaactca accattacca aatgcgagtt 1020

ttgataattt caaactcaca tgttctaaca 1050

<210> 46

<211> 1995

<212> DNA

<213> CORONAVIRUS

<400> 46
tttgtgcact catactcgct tacagtaata aaactgttgg cgagcttggt gatgtcagag 60

aaactatgac ccatcttcta cagcatgcta atttggaatc tgcaaagcga gttcttaatg 120

tggtgtgtaa acattgtggt cagaaaacta ctaccttaac gggtgtagaa gctgtgatgt 180

atatgggtac tctatcttat gataatctta agacaggtgt ttccattcca tgtgtgtgtg 240

gtcgtgatgc tacacaatat ctagtacaac aagagtcttc ttttgttatg atgtctgcac 300

cacctgctga gtataaatta cagcaaggta cattcttatg tgcgaatgag tacactggta 360

actatcagtg tggtcattac actcatataa ctgctaagga gaccctctat cgtattgacg 420

gagctcacct tacaaagatg tcagagtaca aaggaccagt gactgatgtt ttctacaagg 480

aaacatctta cactacaacc atcaagcctg tgtcgtataa actcgatgga gttacttaca 540

cagagattga accaaaattg gatgggtatt ataaaaagga taatgcttac tatacagagc 600

agcctataga ccttgtacca actcaaccat taccaaatgc gagttttgat aatttcaaac 660

tcacatgttc taacacaaaa tttgctgatg atttaaatca aatgacaggc ttcacaaagc 720

cagcttcacg agagctatct gtcacattct tcccagactt gaatggcgat gtagtggcta 780
ttgactatag acactattca gcgagtttca agaaaggtgc taaattactg cataagccaa 840
ttgtttggca cattaaccag gctacaacca agacaacgtt caaaccaaac acttggtgtt 900
tacgttgtct ttggagtaca aagccagtag atacttcaaa ttcatttgaa gttctggcag 960
tagaagacac acaaggaatg gacaatcttg cttgtgaaag tcaacaaccc acctctgaag 1020
aagtagtgga aaatcctacc atacagaagg aagtcataga gtgtgacgtg aaaactaccg 1080
aagttgtagg caatgtcata cttaaaccat cagatgaagg tgttaaagta acacaagagt 1140
taggtcatga ggatcttatg gctgcttatg tggaaaacac aagcattacc attaagaaac 1200
ctaatgagct ttcactagcc ttaggtttaa aaacaattgc cactcatggt attgctgcaa 1260
ttaatagtgt tccttggagt aaaattttgg cttatgtcaa accattctta ggacaagcag 1320
caattacaac atcaaattgc gctaagagat tagcacaacg tgtgtttaac aattatatgc 1380
cttatgtgtt tacattattg ttccaattgt gtacttttac taaaagtacc aattctagaa 1440
ttagagcttc actacctaca actattgcta aaaatagtgt taagagtgtt gctaaattat 1500
gtttggatgc cggcattaat tatgtgaagt cacccaaatt ttctaaattg ttcacaatcg 1560
ctatgtggct attgttgtta agtatttgct taggttctct aatctgtgta actgctgctt 1620
ttggtgtact cttatctaat tttggtgctc cttcttattg taatggcgtt agagaattgt 1680
atcttaattc gtctaacgtt actactatgg atttctgtga aggttctttt ccttgcagca 1740
tttgtttaag tggattagac tcccttgatt cttatccagc tcttgaaacc attcaggtga 1800
cgatttcatc gtacaagcta gacttgacaa ttttaggtct ggccgctgag tgggttttgg 1860
catatatgtt gttcacaaaa ttcttttatt tattaggtct ttcagctata atgcaggtgt 1920
tctttggcta ttttgctagt catttcatca gcaattcttg gctcatgtgg tttatcatta 1980
gtattgtaca aatgg 1995

<210> 47
<211> 1884
<212> DNA
<213> CORONAVIRUS

<400> 47
aattcttggc tcatgtggtt tatcattagt attgtacaaa tggcacccgt ttctgcaatg 60
gttaggatgt acatcttctt tgcttctttc tactacatat ggaagagcta tgttcatatc 120
atggatggtt gcacctcttc gacttgcatg atgtgctata agcgcaatcg tgccacacgc 180
gttgagtgta caactattgt taatggcatg aagagatctt tctatgtcta tgcaaatgga 240
ggccgtggct tctgcaagac tcacaattgg aattgtctca attgtgacac attttgcact 300
ggtagtacat tcattagtga tgaagttgct cgtgatttgt cactccagtt taaaagacca 360

atcaacccta ctgaccagtc atcgtatatt gttgatagtg ttgctgtgaa aaatggcgcg 420
cttcacctct actttgacaa ggctggtcaa aagacctatg agagacatcc gctctcccat 480
tttgtcaatt tagacaattt gagagctaac aacactaaag gttcactgcc tattaatgtc 540
atagtttttg atggcaagtc caaatgcgac gagtctgctt ctaagtctgc ttctgtgtac 600
tacagtcagc tgatgtgcca acctattctg ttgcttgacc aagctcttgt atcagacgtt 660
ggagatagta ctgaagtttc cgttaagatg tttgatgctt atgtcgacac cttttcagca 720
acttttagtg ttcctatgga aaaacttaag gcacttgttg ctacagctca cagcgagtta 780
gcaaagggtg tagctttaga tggtgtcctt tctacattcg tgtcagctgc ccgacaaggt 840
gttgttgata ccgatgttga cacaaaggat gttattgaat gtctcaaact ttcacatcac 900
tctgacttag aagtgacagg tgacagttgt aacaatttca tgctcaccta taataaggtt 960
gaaaacatga cgcccagaga tcttggcgca tgtattgact gtaatgcaag gcatatcaat 1020
gcccaagtag caaaaagtca caatgtttca ctcatctgga atgtaaaaga ctacatgtct 1080
ttatctgaac agctgcgtaa acaaattcgt agtgctgcca agaagaacaa catacctttt 1140
agactaactt gtgctacaac tagacaggtt gtcaatgtca taactactaa aatctcactc 1200
aagggtggta agattgttag tacttgtttt aaacttatgc ttaaggccac attattgtgc 1260
gttcttgctg cattggtttg ttatatcgtt atgccagtac atacattgtc aatccatgat 1320
ggttacacaa atgaaatcat tggttacaaa gccattcagg atggtgtcac tcgtgacatc 1380
atttctactg atgattgttt tgcaaataaa catgctggtt ttgacgcatg gtttagccag 1440
cgtggtggtt catacaaaaa tgacaaaagc tgccctgtag tagctgctat cattacaaga 1500
gagattggtt tcatagtgcc tggcttaccg ggtactgtgc tgagagcaat caatggtgac 1560
ttcttgcatt ttctacctcg tgtttttagt gctgttggca acatttgcta cacaccttcc 1620
aaactcattg agtatagtga ttttgctacc tctgcttgcg ttcttgctgc tgagtgtaca 1680
atttttaagg atgctatggg caaacctgtg ccatattgtt atgacactaa tttgctagag 1740
ggttctattt cttatagtga gcttcgtcca gacactcgtt atgtgcttat ggatggttcc 1800
atcatacagt ttcctaacac ttacctggag ggttctgtta gagtagtaac aacttttgat 1860
gctgagtact gtagacatgg taca 1884

<210> 48
<211> 2020
<212> DNA
<213> CORONAVIRUS

<400> 48
cactcgttat gtgcttatgg atggttccat catacagttt cctaacactt acctggaggg 60
ttctgttaga gtagtaacaa cttttgatgc tgagtactgt agacatggta catgcgaaag 120

gtcagaagta ggtatttgcc tatctaccag tggtagatgg gttcttaata atgagcatta 180
cagagctcta tcaggagttt tctgtggtgt tgatgcgatg aatctcatag ctaacatctt 240
tactcctctt gtgcaacctg tgggtgcttt agatgtgtct gcttcagtag tggctggtgg 300
tattattgcc atattggtga cttgtgctgc ctactacttt atgaaattca gacgtgtttt 360
tggtgagtac aaccatgttg ttgctgctaa tgcacttttg tttttgatgt ctttcactat 420
actctgtctg gtaccagctt acagctttct gccgggagtc tactcagtct tttacttgta 480
cttgacattc tatttcacca atgatgtttc attcttggct caccttcaat ggtttgccat 540
gttttctcct attgtgcctt tttggataac agcaatctat gtattctgta tttctctgaa 600
gcactgccat tggttcttta acaactatct taggaaaaga gtcatgttta atggagttac 660
atttagtacc ttcgaggagg ctgctttgtg tacctttttg ctcaacaagg aaatgtacct 720
aaaattgcgt agcgagacac tgttgccact tacacagtat aacaggtatc ttgctctata 780
taacaagtac aagtatttca gtggagcctt agatactacc agctatcgtg aagcagcttg 840
ctgccactta gcaaaggctc taaatgactt tagcaactca ggtgctgatg ttctctacca 900
accaccacag acatcaatca cttctgctgt tctgcagagt ggttttagga aaatggcatt 960
cccgtcaggc aaagttgaag ggtgcatggt acaagtaacc tgtggaacta caactcttaa 1020
tggattgtgg ttggatgaca cagtatactg tccaagacat gtcatttgca cagcagaaga 1080
catgcttaat cctaactatg aagatctgct cattcgcaaa tccaaccata gctttcttgt 1140
tcaggctggc aatgttcaac ttcgtgttat tggccattct atgcaaaatt gtctgcttag 1200
gcttaaagtt gatacttcta accctaagac acccaagtat aaatttgtcc gtatccaacc 1260
tggtcaaaca ttttcagttc tagcatgcta caatggttca ccatctggtg tttatcagtg 1320
tgccatgaga cctaatcata ccattaaagg ttctttcctt aatggatcat gtggtagtgt 1380
tggttttaac attgattatg attgcgtgtc tttctgctat atgcatcata tggagcttcc 1440
aacaggagta cacgctggta ctgacttaga aggtaaattc tatggtccat ttgttgacag 1500
acaaactgca caggctgcag gtacagacac aaccataaca ttaaatgttt tggcatggct 1560
gtatgctgct gttatcaatg gtgataggtg gtttcttaat agattcacca ctactttgaa 1620
tgactttaac cttgtggcaa tgaagtacaa ctatgaacct ttgacacaag atcatgttga 1680
catattggga cctctttctg ctcaaacagg aattgccgtc ttagatatgt gtgctgcttt 1740
gaaagagctg ctgcagaatg gtatgaatgg tcgtactatc cttggtagca ctattttaga 1800
agatgagttt acaccatttg atgttgttag acaatgctct ggtgttacct tccaaggtaa 1860
gttcaagaaa attgttaagg gcactcatca ttggatgctt ttaactttct tgacatcact 1920
attgattctt gttcaaagta cacagtggtc actgtttttc tttgtttacg agaatgcttt 1980
cttgccattt actcttggta ttatggcaat tgctgcatgt 2020

<210> 49

<211>  2040

<212>  DNA

<213>  CORONAVIRUS

<400>  49
agcatttcca gcctgaagac gtactgtagc agctaaactg cccagcacca tacctctatt      60

taggttgttt aagcctttga tgaagtacaa gtatttcact ttaggccctt ttggtgtgtc     120

tgtaacaaac ctacaaggtg gttccagttc tgtgtaaatt gtacctgtac catcactctt     180

agggaatcta gcccatttga gatcttggtg gtctgatagt aatgccagca caaacctacc     240

tcccttcgaa ttgttatagt aggcaagtgc attgtcatca gtacaagctg tttgtgtggt     300

accagccgca caggacatct gtcgtagtgc tactggactc agttcattat tctgtagttt     360

aacagctgag ttggctctta gagctgtaac aataagaggc caagccaaat ttggtgaatt     420

gtccatgtta atttcactaa gttgaacaat cttgctatcc gcatcaacaa cttgctggat     480

ttcccagagt gcagatgcat atgtaaaggt gttaccatca caagtgttct tgtaggtacc     540

ataatcaggg acaacaacca tgagtttggc tgctgtagtc aatggtatga tgttgagtgg     600

aacacaacca tcacgcgcat tgttgataat gttgttaagt gcatcattat caagcttcct     660

aagcatagtg aagagcattg tttgcatagc actagttact tttgccctct tgtcctcaga     720

tcttgcctgt ttgtacattt gggtcatagc ctgatctgcc atcttttcca acttgcgttg     780

catggcagca tcacggtcaa actcagattt agccacattc aaagatttct ttaacttttt     840

gagaacgact tcagaatcac cattagctac agcctgctca taggcctcct gggcagtggc     900

ataagcggca tatgatggta aagaactaaa ttctgaagca atagcctgaa gagtagcacg     960

gttatcgagc atttcctcgc acaacctatt aatgtctaca gcaccctgca tggatagcaa    1020

aacagacaaa agagaaacca tcttctcgaa agcttcagtt gtgtcttttg caagaagaat    1080

atcattgtgg agttgtacac attgtgccca caatttagaa gatgactcta ctctaagttg    1140

ttgaagaacc gagagcagta ccacagatgt gcactttacg tcagacattt tagactgtac    1200

agtagcaacc ttgatacatg gtttacctcc aatacccaac aacttaatgt taagcttgaa    1260

agcatcaata ctactcttag gaggcaaaag cccctgggag ttcatatacc taaattcttg    1320

tgtagagacc aagtagtcat aaacaccaag agtaagcctg aagtaacggt tgagtaaaca    1380

gaaaaggcca aagtagcagc agcaacaata gcctaagaaa caataaacaa gcatgataca    1440

ctgtaaggtg ttgccagtaa taaataacaa tgggtaatac tcaacacaca caaacactat    1500

agctctagct aaaaacatga tagtcgtaac gacaccagaa tagttagagg ttacagaaat    1560

aactaaggcc cacatggaaa tagcttgatc taaagcatta ccatagtaga ctttgtaaac    1620

aagtgtaatg acattcatca gtgtccaaac acgtctagca gcatcatcat aaacagtgcg    1680

agctgtcatg agaataagca aaactaaagc tgaagcatac ataacacaat ccttaagcct    1740

ataaccagac aagctagtgt cagccaattc aagccatgtc atgatacgca tcacccagct    1800

agcaggcatg tagaccatat taaagtaagc aactgttgca agagaaggta acagaaacaa 1860
gcacaagaat gcgtgcttat gcttaacaag cagcatagca catgcagcaa ttgccataat 1920
accaagagta aatggcaaga aagcattctc gtaaacaaag aaaaacagtg accactgtgt 1980
actttgaaca agaatcaata gtgatgtcaa gaaagttaaa agcatccaat gatgagtgca 2040

<210> 50
<211> 2012
<212> DNA
<213> CORONAVIRUS

<400> 50
cttgtaggtt tgttacagac acaccaaaag ggcctaaagt gaaatacttg tacttcatca 60
aaggcttaaa caacctaaat agaggtatgg tgctgggcag tttagctgct acagtacgtc 120
ttcaggctgg aaatgctaca gaagtacctg ccaattcaac tgtgctttcc ttctgtgctt 180
ttgcagtaga ccctgctaaa gcatataagg attacctagc aagtggagga caaccaatca 240
ccaactgtgt gaagatgttg tgtacacaca ctggtacagg acaggcaatt actgtaacac 300
cagaagctaa catggaccaa gagtcctttg gtggtgcttc atgttgtctg tattgtagat 360
gccacattga ccatccaaat cctaaaggat tctgtgactt gaaaggtaag tacgtccaaa 420
tacctaccac ttgtgctaat gacccagtgg gttttacact tagaaacaca gtctgtaccg 480
tctgcggaat gtggaaaggt tatggctgta gttgtgacca actccgcgaa cccttgatgc 540
agtctgcgga tgcatcaacg tttttaaacg ggtttgcggt gtaagtgcag cccgtcttac 600
accgtgcggc acaggcacta gtactgatgt cgtctacagg gcttttgata tttacaacga 660
aaaagttgct ggttttgcaa agttcctaaa aactaattgc tgtcgcttcc aggagaagga 720
tgaggaaggc aatttattag actcttactt tgtagttaag aggcatacta tgtctaacta 780
ccaacatgaa gagactattt ataacttggt taaagattgt ccagcggttg ctgtccatga 840
ctttttcaag tttagagtag atggtgacat ggtaccacat atatcacgtc agcgtctaac 900
taaatacaca atggctgatt tagtctatgc tctacgtcat tttgatgagg gtaattgtga 960
tacattaaaa gaaatactcg tcacatacaa ttgctgtgat gatgattatt tcaataagaa 1020
ggattggtat gacttcgtag agaatcctga catcttacgc gtatatgcta acttaggtga 1080
gcgtgtacgc caatcattat taaagactgt acaattctgc gatgctatgc gtgatgcagg 1140
cattgtaggc gtactgacat tagataatca ggatcttaat gggaactggt acgatttcgg 1200
tgatttcgta caagtagcac caggctgcgg agttcctatt gtggattcat attactcatt 1260
gctgatgccc atcctcactt tgactagggc attggctgct gagtcccata tggatgctga 1320
tctcgcaaaa ccacttatta agtgggattt gctgaaatat gattttacgg aagagagact 1380
ttgtctcttc gaccgttatt ttaaatattg ggaccagaca taccatccca attgtattaa 1440

ctgtttggat gataggtgta tccttcattg tgcaaacttt aatgtgttat tttctactgt 1500
gtttccacct acaagttttg gaccactagt aagaaaaata tttgtagatg gtgttccttt 1560
tgttgtttca actggatacc attttcgtga gttaggagtc gtacataatc aggatgtaaa 1620
cttacatagc tcgcgtctca gtttcaagga acttttagtg tatgctgctg atccagctat 1680
gcatgcagct tctggcaatt tattgctaga taaacgcact acatgctttt cagtagctgc 1740
actaacaaac aatgttgctt ttcaaactgt caaacccggt aattttaata aagactttta 1800
tgactttgct gtgtctaaag gtttctttaa ggaaggaagt tctgttgaac taaaacactt 1860
cttctttgct caggatggca acgctgctat cagtgattat gactattatc gttataatct 1920
gccaacaatg tgtgatatca gacaactcct attcgtagtt gaagttgttg ataaatactt 1980
tgattgttac gatggtggct gtattaatgc ca 2012

<210> 51
<211> 1877
<212> DNA
<213> CORONAVIRUS

<400> 51
gtacttcgcg tacagtggca ataccatatg acagcttaaa tgtttcctca gtggctttga 60
gcgtttctgc tgcgaaaagc ttgagtctct cagtacaagt gttggcaagt atgtaatcgc 120
cagcattagt ccaatcacat gttgctatcg cattgaagtc agtgacattg tcactgccta 180
cacatgtgtt tttgtataaa ccaaaaacct gaccattagc acataatgga aaactaatgg 240
gaggcttatg tgacttgcaa taatagctca tacctcctag atacagttgt gtcacatcag 300
tgacatcaca acctggggca ttgcaaacat agggattaac agacaacact aatttgtgtg 360
atgttgaaat gacatggtca tagcagcact tgcaacatag gaatggtctc ctaatacagg 420
caccgcaacg aagtgaagtc tgtgaattgc acaatacaca agcacctaca gcctgcaaga 480
ctgtatgtgg tgtgtacata gcctcataaa actcaggttc ccagtaccgt gaggtgttat 540
cattagttag cattacggaa tacatgtcca acatgtggcc agtaagctca tcatgtaact 600
ttctaatgta ttgtaaatac aagtgaaaga catcagcata ctcctgatta ggatgttttg 660
taagtgggta agcatcaata gccagtgaca cgaacctttc aatcataagt gtaccatctg 720
ttttgacaat atcatcgaca aaacagcctg cgcctaatat tcttgatgga tctgggtaag 780
gcaggtacac gtaatcatct ccttgtttaa ctagcattgt atgctgtgag caaaattcgt 840
gaggtccttt agtaaggtca gtctcagtcc aacattttgc ctcagacatg aacacattat 900
tttgataata aagaactgcc ttaaagttct taatgctagc tactaaacct tgagccgcat 960
agttactgtt atagcacaca acggcatcat cagaaagaat catcatggag aaatgtttac 1020
gcaggtaagc gtaaaactca tccacgaatt catgatcaac atccctattt ctatagagac 1080

actcatagag cctgtgttgt agattgcgga catacttgtc agctatctta ttaccatcag 1140
ttgaaagaag tgcatttaca ttggctgtaa cagcttgaca aatgttaaag acactattag 1200
cataagcagt tgtagcatca ccggatgatg ttccacctgg tttaacatat agtgagccgc 1260
cacacatgac catctcactt aatacttgcg cacactcgtt agctaacctg tagaaacggt 1320
gtgataagtt acagcaagtg ttatgtttgc gagcaagaac aagagaggcc attatcctaa 1380
gcatgttagg catggctctg tcacattttg gataatccca acccataagg tgtggagttt 1440
ctacatcact gtaaacagtt tttaacatat tatgccagcc accgtaaaac ttgcttgttc 1500
caattaccac agtagctcct ctagtggcgg ctattgactt caataatttc tgatgaaact 1560
gtctatttgt catagtacta cagatagaga caccagctac ggtgcgagct ctattctttg 1620
cactaatggc atacttaaga ttcatttgag ttatagtagg gatgacatta cgcttagtat 1680
acgcgaaaag tgcatcttga tcctcataac tcattgagtc ataataaagt ctagccttac 1740
cccatttatt aaatgggaaa ccagctgatt tatccagatt gttaacgatt acttggttgg 1800
cattaataca gccaccatcg taacaatcaa agtatttatc aacaacttca actacgaata 1860
ggagttgtct gatatca 1877

<210> 52
<211> 2051
<212> DNA
<213> CORONAVIRUS

<400> 52
tcaggtccaa tcttgacaaa gtacttcatt gatgtaagct caaagccatg cgcccaaagg 60
acgaacacga ctctgtctga caatcctttc agtgtatcac tgagcatttg tactatctta 120
atacgcacta cattccaggg caagccttta tacatgagtg gtataagatg tttaaactgg 180
tcacctggtg gaggttttgc attaactctg gtgaattctg tgttattttc agtgtcaaca 240
taaccagtcg gtacagctac taagttaaca cctgtagaaa atcctagctg gagaggtagg 300
ttagtaccca cagcatctct agttgcatga cagccctcta catcaaagcc aatccacgca 360
cgaacgtgac gaatagcttc ttcgcgggtg ataaacatat tagggtaacc attgacttgg 420
taattcattt tgaaacccat catagagatg agtctacggt aggtcatgtc ctttggtatg 480
cctggtatgt caacacataa tccttcagtc ttgaacttta tatcaacgct gaggtgtgta 540
ggtgcctgtg taggatgaag accagtaatg atcttactac agtccttaaa aagtccagtt 600
acattttctg cttgtaatgt agccacattg cgacgtggta tttctagact tgtaaattgc 660
agtttgtcat aaagatctct atcagacatt atgcacaaaa tgccaatttt tgcccttgtg 720
atagccacat tgaagcggtt gacattacaa gagtgtgctg tttcagtagt ttgtgtgaat 780
atgacatagt catattcaga accctgtgat gaatcaacag tctgcgtagg caatcctaag 840

atttttgaag ctacagcgtt ctgtgaatta taaggtgaga taaaaacagc ttttctccaa 900
gcaggattgc gtgtaagaaa ttctcttaca acgcctattt gaggtctgtt gattgcagat 960
gaaacatcat gtgtaataac acctttgtag aacattttga agcattgagc tgacttatcc 1020
ttgtgtgctt ttagcttatt gtcataaact aaagcactca cagtgtcaac aatttcagca 1080
ggacaacggc gacaagttcc aaggaacatg tctggaccta ttgttttcat aagtctgcac 1140
actgaattaa aatattctgg ttctagtgtg cctttagtca gcaatgtgcg gggggctggt 1200
aattgagcag gatcgccaat atagacgtag tgttttgcac gaagtctagc attgacaaca 1260
ctcaagtcat aattagtagc catagagatt tcatcaaaga ctacaatgtc agcagttgtt 1320
tctggcaatg catttacagt gcagaaaaca tactgttcta gtgttgaatt cactttgaat 1380
ttatcaaaac actctacgcg cgcacgcgca ggtatgattc tactacattt atctatgggc 1440
aaatatttta atgccttttc acatagggca tcaacagctg catgagagca tgccgtatac 1500
actatgcgag cagatgggta atagagagca agtccgatgg caaaatgact cttaccagta 1560
ccaggtggtc cttggagtgt agagtacttt tgcatgccga ccttttgata atttgcaaca 1620
ttgctagaaa actcatctga gatgttgagt gttgggtaca agccagtaat tctcacatag 1680
tgctcttgtg gcactagagt aggtgcacta agtggcatta cagtgtgaga tgtcaacaca 1740
aagtaatcac caacattcaa cttgtatgtc gtagtacctc tgtacacaac agcatcacca 1800
tagtcacctt tttcaaaggt gtactctcca atctgtactt tactattttt agttacacgg 1860
taaccagtaa agacatagtt tctgttcaat ggtggtctag gttttccaac ctcccatgaa 1920
agatgcaatt ctctgtcaga gagtacttcg cgtacagtgg caataccata tgacagctta 1980
aatgtttcct cagtggcttt gagcgtttct gctgcgaaaa gcttgagtct ctcagtacaa 2040
gtgttggcaa g 2051

<210> 53
<211> 2075
<212> DNA
<213> CORONAVIRUS

<400> 53
tgcttgtagt tttgggtaga aggtttcaac atgtccatcc ttacaccaaa gcatgaatga 60
aatttcagca tagtcaattg taaccttgac cacttttgaa atcactgaca aatcttgtga 120
ctttattatc tcgacaaagt catcaagtaa aagatcaatc acagaacaca cacattttga 180
tgaacctgtt tgcgcatctg ttatgaagta atttttcact gtgctgtcca tagggataaa 240
atcctctaat ttaagtggtg aatcttgtga gcgcttggct aagcctatca ttaaatgaag 300
accgccaagt tgtccatgac tgaaatctcc ataaacgatg tgttcgaagg catagccctc 360
gagcttatat cgctgtatga attcatccat agcgagctcg agaaagtcag tttccatttg 420

tgatctgggc ttaaaatcct ctaagtctct gctctgagta aagtaggttt caggcaactg 480
ttgaataatg ccgtctactt tcttaaagta gttaaactgt gtttttactg attctccaat 540
taatgtgact ccattgacgc tagcttgtgc tggtcccttt gaaggtgtta gacctttgac 600
tgaaccttct gttattaaaa caccattacg ggcgtttcta aaaaggtcta cctgtccttc 660
cactctacca tcaaacaaga cagtaagtga agaacaagca ctctcagtag gtttcttggc 720
aatgtcagtc attgtgcaga cacctattgt agatacatgt gctggggctt ctcttttgta 780
gtcccagatt acagtattag cagcgatatc aacacccaaa ttattgagta tcttaatctc 840
tggcactggt ttaatgttac gcttagccca aagctcaaat gcaacattaa caggaagtgt 900
tgtcttattt tcaaagatct ccacatcaat accatctacc tttgtgtaaa cagcattatt 960
aatgatggaa acaggtgctt cgccggcgtg tccatcaaag tgtcctttat taacaacatt 1020
ataagccaca ttttctaaac tctgtaacct ggtaaatgta ttccacaggt tataagtatc 1080
aaattgtttg taaatccata ggctaaatcc agcagaaatc atcatattat atgcatccaa 1140
gtactgtcgg tactcatttg catggtgtct gcaaacagca ccacctaaat tgcatcgtgt 1200
aatacacgta gcagatttga gtggaacata atcaatatcc gacactactt gtttgccatg 1260
agactcacaa ggactatcag aatagtaaaa gaaaggcaat tgctttaaat tagtaaatgc 1320
acttttatcg aaagctggag tgtggaatgc atgcttattc acatacaaac taccaccatc 1380
acagcctggt aagttcaagt ttgacaagac tcttgtgtca aacctacaca caattgcatt 1440
ggctgggtaa cgatcaacgt tacaattcca aaacaaacaa acaccatcag tgaatttatc 1500
gtgatgtgta gcataagaat agaagagttc ctctattttg taagctttgt cactacatgg 1560
ctgagcatcg tagaacttcc attctacttc agcctgaggc acacacttga tagcctttgg 1620
atttccaatg tcatgaagaa ctggaaactt atcagcaagc aatgcagact tcacaaccat 1680
gtgttgtact tttctgcaag cagaattaac cctcagttca tctcctataa tagggtattc 1740
aacagaccaa tcaacgcgct taacaaagca ctcatggact gctaaacatc tagtcatgat 1800
agcatcacaa ctagccacat gtgcatttcc atgtacctgg caatgttggt catggttact 1860
ctgaaggtta cccgtaaagc cccactgctg aacatcaatc ataaatgggt tatagacata 1920
gtcaaaaccc acagaatgat tccagcaggc ataagtatct gatgaagtag aaaagcaagt 1980
tgcacgtttg tcacacagac aacacgttct ttcaggtcca atcttgacaa agtacttcat 2040
tgatgtaagc tcaaagccat gcgcccaaag gacga 2075

<210> 54
<211> 1891
<212> DNA
<213> CORONAVIRUS

<400> 54
aagattcacc acttaaatta gaggatttta tccctatgga cagcacagtg aaaaattact 60
tcataacaga tgcgcaaaca ggttcatcaa aatgtgtgtg ttctgtgatt gatcttttac 120
ttgatgactt tgtcgagata ataaagtcac aagatttgtc agtgatttca aaagtggtca 180
aggttacaat tgactatgct gaaatttcat tcatgctttg gtgtaaggat ggacatgttg 240
aaaccttcta cccaaaacta caagcaagtc aagcgtggca accaggtgtt gcgatgccta 300
acttgtacaa gatgcaaaga atgcttcttg aaaagtgtga ccttcagaat tatggtgaaa 360
atgctgttat accaaaagga ataatgatga atgtcgcaaa gtatactcaa ctgtgtcaat 420
acttaaatac acttacttta gctgtaccct acaacatgag agttattcac tttggtgctg 480
gctctgataa aggagttgca ccaggtacag ctgtgctcag acaatggttg ccaactggca 540
cactacttgt cgattcagat cttaatgact tcgtctccga cgcagattct actttaattg 600
gagactgtgc aacagtacat acggctaata aatgggacct tattattagc gatatgtatg 660
accctaggac caaacatgtg acaaaagaga atgactctaa agaagggttt ttcacttatc 720
tgtgtggatt tataaagcaa aaactagccc tgggtggttc tatagctgta aagataacag 780
agcattcttg gaatgctgac ctttacaagc ttatgggcca tttctcatgg tggacagctt 840
ttgttacaaa tgtaaatgca tcatcatcgg aagcattttt aattggggct aactatcttg 900
gcaagccgaa ggaacaaatt gatggctata ccatgcatgc taactacatt ttctggagga 960
acacaaatcc tatccagttg tcttcctatt cactctttga catgagcaaa tttcctctta 1020
aattaagagg aactgctgta atgtctctta aggagaatca aatcaatgat atgatttatt 1080
ctcttctgga aaaaggtagg cttatcatta gagaaaacaa cagagttgtg gtttcaagtg 1140
atattcttgt taacaactaa acgaacatgt ttattttctt attatttctt actctcacta 1200
gtggtagtga ccttgaccgg tgcaccactt ttgatgatgt tcaagctcct aattacactc 1260
aacatacttc atctatgagg ggggtttact atcctgatga aatttttaga tcagacactc 1320
tttatttaac tcaggattta tttcttccat tttattctaa tgttacaggg tttcatacta 1380
ttaatcatac gtttggcaac cctgtcatac cttttaagga tggtatttat tttgctgcca 1440
cagagaaatc aaatgttgtc cgtggttggg tttttggttc taccatgaac aacaagtcac 1500
agtcggtgat tattattaac aattctacta atgttgttat acgagcatgt aactttgaat 1560
tgtgtgacaa ccctttcttt gctgtttcta aacccatggg tacacagaca catactatga 1620
tattcgataa tgcatttaat tgcactttcg agtacatatc tgatgccttt tcgcttgatg 1680
tttcagaaaa gtcaggtaat tttaaacact tacgagagtt tgtgtttaaa aataaagatg 1740
ggtttctcta tgtttataag ggctatcaac ctatagatgt agttcgtgat ctaccttctg 1800
gttttaacac tttgaaacct atttttaagt tgcctcttgg tattaacatt acaaatttta 1860
gagccattct tacagccttt tcacctgctc a 1891

<210> 55

<211>  32

<212>  DNA

<213>  ARTIFICIAL SEQUENCE



<220>

<223>  amorce N sens

<400>  55
cccatatgtc tgataatgga ccccaatcaa ac                                    32

<210>  56

<211>  32

<212>  DNA

<213>  artificial sequence



<220>

<223>  amorce N antisens

<400>  56
cccccgggtg cctgagttga atcagcagaa gc                                    32

<210>  57

<211>  31

<212>  DNA

<213>  artificial sequence



<220>

<223>  amorce Sc sens

<400>  57
cccatatgag tgaccttgac cggtgcacca c                                     31

<210>  58

<211>  30

<212>  DNA

<213>  artificial sequence



<220>

<223>  amorce SL sens

<400>  58
cccatatgaa accttgcacc ccacctgctc                                       30

<210> 59

<211> 33

<212> DNA

<213> amorce Sc et SL antisens

<400> 59
cccccgggtt taatatattg ctcatatttt ccc 33

<210> 60

<211> 16

<212> DNA

<213> amorce sens série 1

<400> 60
ggcatcgtat gggttg 16

<210> 61

<211> 16

<212> DNA

<213> amorce antisens série 2 (28774-28759)

<400> 61
cagtttcacc acctcc 16

<210> 62

<211> 16

<212> DNA

<213> amorce sens série 2 (28375-28390)

<400> 62
ggctactacc gaagag 16

<210> 63

<211> 16

<212> DNA

<213> amorce antisens série 2 (28702-28687)

<400> 63
aattaccgcg actacg 16

<210> 64

<211> 26

<212> DNA

<213> sonde 1/série 1 (28561-28586)

<400> 64
ggcacccgca atcctaataa caatgc 26

<210> 65

<211> 21

<212> DNA

<213> sonde 2/série 1 (28588-28608)

<400> 65
gccaccgtgc tacaacttcc t 21

<210> 66

<211> 23

<212> DNA

<213> sonde 1/série 2 /sonde N/FL (28541-28563)

<400> 66
atacacccaa agaccacatt ggc 23

<210> 67

<211> 25

<212> DNA

<213> sonde 2/série 2/sonde SARS/N/LC705 (28565-28589)

<400> 67
cccgcaatcc taataacaat gctgc 25

<210> 68

<211> 30

<212> DNA

<213> artificial sequence

<220>

<223>  amorce ancre 14T

<400>  68
agatgaattc ggtacctttt tttttttttt                                        30

<210>  69

<211>  13

<212>  PRT

<213>  artificial sequence

<220>

<223>  peptide M2-14

<400>  69

Ala Asp Asn Gly Thr Ile Thr Val Glu Glu Leu Lys Gln
1               5                   10

<210>  70

<211>  12

<212>  PRT

<213>  artificial sequence

<220>

<223>  peptide E1-12

<400>  70

Met Tyr Ser Phe Val Ser Glu Glu Thr Gly Thr Leu
1               5                   10

<210>  71

<211>  24

<212>  PRT

<213>  artificial sequence

<220>

<223>  peptide E53-72

<400>  71

Lys Pro Thr Val Tyr Val Tyr Ser Arg Val Lys Asn Leu Asn Ser Ser
1               5                   10                  15

Glu Gly Val Pro Asp Leu Leu Val
            20

<210>  72

<211>  153

<212>  DNA

<213>  CORONAVIRUS

<400>  72
gatattaggt ttttacctac ccaggaaaag ccaaccaacc tcgatctctt gtagatctgt      60

tctctaaacg aactttaaaa tctgtgtagc tgtcgctcgg ctgcatgcct agtgcaccta     120

cgcagtataa acaataataa attttactgt cgt                                  153

<210>  73

<211>  410

<212>  DNA

<213>  CORONAVIRUS

<400>  73
ttctccagac aacttcaaaa ttccatgagt ggagcttctg ctgattcaac tcaggcataa      60

acactcatga tgaccacaca aggcagatgg gctatgtaaa cgttttcgca attccgttta     120

cgatacatag tctactcttg tgcagaatga attctcgtaa ctaaacagca caagtaggtt     180

tagttaactt taatctcaca tagcaatctt taatcaatgt gtaacattag ggaggacttg     240

aaagagccac cacattttca tcgaggccac gcggagtacg atcgagggta cagtgaataa     300

tgctagggag agctgcctat atggaagagc cctaatgtgt aaaattaatt ttagtagtgc     360

tatccccatg tgattttaat agcttcttag gagaatgaca aaaaaaaaaa                410

<210>  74

<211>  4382

<212>  PRT

<213>  CORONAVIRUS

<400>  74
   Met Glu Ser Leu Val Leu Gly Val Asn Glu Lys Thr His Val Gln Leu
   1               5                   10                  15
   Ser Leu Pro Val Leu Gln Val Arg Asp Val Leu Val Arg Gly Phe Gly
               20                  25                  30
   Asp Ser Val Glu Glu Ala Leu Ser Glu Ala Arg Glu His Leu Lys Asn
           35                  40                  45
   Gly Thr Cys Gly Leu Val Glu Leu Glu Lys Gly Val Leu Pro Gln Leu
       50                  55                  60
   Glu Gln Pro Tyr Val Phe Ile Lys Arg Ser Asp Ala Leu Ser Thr Asn
   65                  70                  75                  80
   His Gly His Lys Val Val Glu Leu Val Ala Glu Met Asp Gly Ile Gln
                   85                  90                  95
   Tyr Gly Arg Ser Gly Ile Thr Leu Gly Val Leu Val Pro His Val Gly
               100                 105                 110
   Glu Thr Pro Ile Ala Tyr Arg Asn Val Leu Leu Arg Lys Asn Gly Asn
           115                 120                 125
   Lys Gly Ala Gly Gly His Ser Tyr Gly Ile Asp Leu Lys Ser Tyr Asp
       130                 135                 140
   Leu Gly Asp Glu Leu Gly Thr Asp Pro Ile Glu Asp Tyr Glu Gln Asn
   145                 150                 155                 160
   Trp Asn Thr Lys His Gly Ser Gly Ala Leu Arg Glu Leu Thr Arg Glu
                   165                 170                 175
   Leu Asn Gly Gly Ala Val Thr Arg Tyr Val Asp Asn Asn Phe Cys Gly
               180                 185                 190
   Pro Asp Gly Tyr Pro Leu Asp Cys Ile Lys Asp Phe Leu Ala Arg Ala
           195                 200                 205
   Gly Lys Ser Met Cys Thr Leu Ser Glu Gln Leu Asp Tyr Ile Glu Ser
       210                 215                 220
   Lys Arg Gly Val Tyr Cys Cys Arg Asp His Glu His Glu Ile Ala Trp
   225                 230                 235                 240
   Phe Thr Glu Arg Ser Asp Lys Ser Tyr Glu His Gln Thr Pro Phe Glu
                   245                 250                 255
   Ile Lys Ser Ala Lys Lys Phe Asp Thr Phe Lys Gly Glu Cys Pro Lys
               260                 265                 270
   Phe Val Phe Pro Leu Asn Ser Lys Val Lys Val Ile Gln Pro Arg Val
           275                 280                 285
   Glu Lys Lys Lys Thr Glu Gly Phe Met Gly Arg Ile Arg Ser Val Tyr
       290                 295                 300
   Pro Val Ala Ser Pro Gln Glu Cys Asn Asn Met His Leu Ser Thr Leu
   305                 310                 315                 320
   Met Lys Cys Asn His Cys Asp Glu Val Ser Trp Gln Thr Cys Asp Phe
                   325                 330                 335
   Leu Lys Ala Thr Cys Glu His Cys Gly Thr Glu Asn Leu Val Ile Glu
               340                 345                 350
   Gly Pro Thr Thr Cys Gly Tyr Leu Pro Thr Asn Ala Val Val Lys Met
           355                 360                 365
   Pro Cys Pro Ala Cys Gln Asp Pro Glu Ile Gly Pro Glu His Ser Val
       370                 375                 380
   Ala Asp Tyr His Asn His Ser Asn Ile Glu Thr Arg Leu Arg Lys Gly
   385                 390                 395                 400
   Gly Arg Thr Arg Cys Phe Gly Gly Cys Val Phe Ala Tyr Val Gly Cys
                   405                 410                 415
   Tyr Asn Lys Arg Ala Tyr Trp Val Pro Arg Ala Ser Ala Asp Ile Gly
               420                 425                 430
   Ser Gly His Thr Gly Ile Thr Gly Asp Asn Val Glu Thr Leu Asn Glu
           435                 440                 445
   Asp Leu Leu Glu Ile Leu Ser Arg Glu Arg Val Asn Ile Asn Ile Val
       450                 455                 460
   Gly Asp Phe His Leu Asn Glu Glu Val Ala Ile Ile Leu Ala Ser Phe
   465                 470                 475                 480
   Ser Ala Ser Thr Ser Ala Phe Ile Asp Thr Ile Lys Ser Leu Asp Tyr
                   485                 490                 495
   Lys Ser Phe Lys Thr Ile Val Glu Ser Cys Gly Asn Tyr Lys Val Thr
               500                 505                 510
   Lys Gly Lys Pro Val Lys Gly Ala Trp Asn Ile Gly Gln Gln Arg Ser
           515                 520                 525
   Val Leu Thr Pro Leu Cys Gly Phe Pro Ser Gln Ala Ala Gly Val Ile
       530                 535                 540
   Arg Ser Ile Phe Ala Arg Thr Leu Asp Ala Ala Asn His Ser Ile Pro
   545                 550                 555                 560
   Asp Leu Gln Arg Ala Ala Val Thr Ile Leu Asp Gly Ile Ser Glu Gln
                   565                 570                 575
   Ser Leu Arg Leu Val Asp Ala Met Val Tyr Thr Ser Asp Leu Leu Thr
   580 585 590
   Asn Ser Val Ile Ile Met Ala Tyr Val Thr Gly Gly Leu Val Gln Gln
   595 600 605
   Thr Ser Gln Trp Leu Ser Asn Leu Leu Gly Thr Thr Val Glu Lys Leu
   610 615 620
   Arg Pro Ile Phe Glu Trp Ile Glu Ala Lys Leu Ser Ala Gly Val Glu
   625 630 635 640
   Phe Leu Lys Asp Ala Trp Glu Ile Leu Lys Phe Leu Ile Thr Gly Val
   645 650 655
   Phe Asp Ile Val Lys Gly Gln Ile Gln Val Ala Ser Asp Asn Ile Lys
   660 665 670
   Asp Cys Val Lys Cys Phe Ile Asp Val Val Asn Lys Ala Leu Glu Met
   675 680 685
   Cys Ile Asp Gln Val Thr Ile Ala Gly Ala Lys Leu Arg Ser Leu Asn
   690 695 700
   Leu Gly Glu Val Phe Ile Ala Gln Ser Lys Gly Leu Tyr Arg Gln Cys
   705 710 715 720
   Ile Arg Gly Lys Glu Gln Leu Gln Leu Leu Met Pro Leu Lys Ala Pro
   725 730 735
   Lys Glu Val Thr Phe Leu Glu Gly Asp Ser His Asp Thr Val Leu Thr
   740 745 750
   Ser Glu Glu Val Val Leu Lys Asn Gly Glu Leu Glu Ala Leu Glu Thr
   755 760 765
   Pro Val Asp Ser Phe Thr Asn Gly Ala Ile Val Gly Thr Pro Val Cys
   770 775 780
   Val Asn Gly Leu Met Leu Leu Glu Ile Lys Asp Lys Glu Gln Tyr Cys
   785 790 795 800
   Ala Leu Ser Pro Gly Leu Leu Ala Thr Asn Asn Val Phe Arg Leu Lys
   805 810 815
   Gly Gly Ala Pro Ile Lys Gly Val Thr Phe Gly Glu Asp Thr Val Trp
   820 825 830
   Glu Val Gln Gly Tyr Lys Asn Val Arg Ile Thr Phe Glu Leu Asp Glu
   835 840 845
   Arg Val Asp Lys Val Leu Asn Glu Lys Cys Ser Val Tyr Thr Val Glu
       850                 855                 860
   Ser Gly Thr Glu Val Thr Glu Phe Ala Cys Val Val Ala Glu Ala Val
   865                 870                 875                 880
   Val Lys Thr Leu Gln Pro Val Ser Asp Leu Leu Thr Asn Met Gly Ile
                   885                 890                 895
   Asp Leu Asp Glu Trp Ser Val Ala Thr Phe Tyr Leu Phe Asp Asp Ala
               900                 905                 910
   Gly Glu Glu Asn Phe Ser Ser Arg Met Tyr Cys Ser Phe Tyr Pro Pro
           915                 920                 925
   Asp Glu Glu Glu Glu Asp Asp Ala Glu Cys Glu Glu Glu Glu Ile Asp
       930                 935                 940
   Glu Thr Cys Glu His Glu Tyr Gly Thr Glu Asp Asp Tyr Gln Gly Leu
   945                 950                 955                 960
   Pro Leu Glu Phe Gly Ala Ser Ala Glu Thr Val Arg Val Glu Glu Glu
                   965                 970                 975
   Glu Glu Glu Asp Trp Leu Asp Asp Thr Thr Glu Gln Ser Glu Ile Glu
               980                 985                 990
   Pro Glu Pro Glu Pro Thr Pro Glu  Glu Pro Val Asn Gln  Phe Thr Gly
           995                 1000                 1005
   Tyr Leu  Lys Leu Thr Asp Asn  Val Ala Ile Lys Cys  Val Asp Ile
       1010                 1015                 1020
   Val Lys  Glu Ala Gln Ser Ala  Asn Pro Met Val Ile  Val Asn Ala
       1025                 1030                 1035
   Ala Asn  Ile His Leu Lys His  Gly Gly Gly Val Ala  Gly Ala Leu
       1040                 1045                 1050
   Asn Lys  Ala Thr Asn Gly Ala  Met Gln Lys Glu Ser  Asp Asp Tyr
       1055                 1060                 1065
   Ile Lys  Leu Asn Gly Pro Leu  Thr Val Gly Gly Ser  Cys Leu Leu
       1070                 1075                 1080
   Ser Gly  His Asn Leu Ala Lys  Lys Cys Leu His Val  Val Gly Pro
       1085                 1090                 1095
   Asn Leu  Asn Ala Gly Glu Asp  Ile Gln Leu Leu Lys  Ala Ala Tyr
       1100                 1105                 1110
   Glu Asn Phe Asn Ser Gln Asp Ile Leu Leu Ala Pro Leu Leu Ser
   1115 1120 1125
   Ala Gly Ile Phe Gly Ala Lys Pro Leu Gln Ser Leu Gln Val Cys
   1130 1135 1140
   Val Gln Thr Val Arg Thr Gln Val Tyr Ile Ala Val Asn Asp Lys
   1145 1150 1155
   Ala Leu Tyr Glu Gln Val Val Met Asp Tyr Leu Asp Asn Leu Lys
   1160 1165 1170
   Pro Arg Val Glu Ala Pro Lys Gln Glu Glu Pro Pro Asn Thr Glu
   1175 1180 1185
   Asp Ser Lys Thr Glu Glu Lys Ser Val Val Gln Lys Pro Val Asp
   1190 1195 1200
   Val Lys Pro Lys Ile Lys Ala Cys Ile Asp Glu Val Thr Thr Thr
   1205 1210 1215
   Leu Glu Glu Thr Lys Phe Leu Thr Asn Lys Leu Leu Leu Phe Ala
   1220 1225 1230
   Asp Ile Asn Gly Lys Leu Tyr His Asp Ser Gln Asn Met Leu Arg
   1235 1240 1245
   Gly Glu Asp Met Ser Phe Leu Glu Lys Asp Ala Pro Tyr Met Val
   1250 1255 1260
   Gly Asp Val Ile Thr Ser Gly Asp Ile Thr Cys Val Val Ile Pro
   1265 1270 1275
   Ser Lys Lys Ala Gly Gly Thr Thr Glu Met Leu Ser Arg Ala Leu
   1280 1285 1290
   Lys Lys Val Pro Val Asp Glu Tyr Ile Thr Thr Tyr Pro Gly Gln
   1295 1300 1305
   Gly Cys Ala Gly Tyr Thr Leu Glu Glu Ala Lys Thr Ala Leu Lys
   1310 1315 1320
   Lys Cys Lys Ser Ala Phe Tyr Val Leu Pro Ser Glu Ala Pro Asn
   1325 1330 1335
   Ala Lys Glu Glu Ile Leu Gly Thr Val Ser Trp Asn Leu Arg Glu
   1340 1345 1350
   Met Leu Ala His Ala Glu Glu Thr Arg Lys Leu Met Pro Ile Cys
   1355 1360 1365
   Met Asp Val Arg Ala Ile Met Ala Thr Ile Gln Arg Lys Tyr Lys
       1370                1375                1380
   Gly Ile Lys Ile Gln Glu Gly Ile Val Asp Tyr Gly Val Arg Phe
       1385                1390                1395
   Phe Phe Tyr Thr Ser Lys Glu Pro Val Ala Ser Ile Ile Thr Lys
       1400                1405                1410
   Leu Asn Ser Leu Asn Glu Pro Leu Val Thr Met Pro Ile Gly Tyr
       1415                1420                1425
   Val Thr His Gly Phe Asn Leu Glu Glu Ala Ala Arg Cys Met Arg
       1430                1435                1440
   Ser Leu Lys Ala Pro Ala Val Val Ser Val Ser Ser Pro Asp Ala
       1445                1450                1455
   Val Thr Thr Tyr Asn Gly Tyr Leu Thr Ser Ser Ser Lys Thr Ser
       1460                1465                1470
   Glu Glu His Phe Val Glu Thr Val Ser Leu Ala Gly Ser Tyr Arg
       1475                1480                1485
   Asp Trp Ser Tyr Ser Gly Gln Arg Thr Glu Leu Gly Val Glu Phe
       1490                1495                1500
   Leu Lys Arg Gly Asp Lys Ile Val Tyr His Thr Leu Glu Ser Pro
       1505                1510                1515
   Val Glu Phe His Leu Asp Gly Glu Val Leu Ser Leu Asp Lys Leu
       1520                1525                1530
   Lys Ser Leu Leu Ser Leu Arg Glu Val Lys Thr Ile Lys Val Phe
       1535                1540                1545
   Thr Thr Val Asp Asn Thr Asn Leu His Thr Gln Leu Val Asp Met
       1550                1555                1560
   Ser Met Thr Tyr Gly Gln Gln Phe Gly Pro Thr Tyr Leu Asp Gly
       1565                1570                1575
   Ala Asp Val Thr Lys Ile Lys Pro His Val Asn His Glu Gly Lys
       1580                1585                1590
   Thr Phe Phe Val Leu Pro Ser Asp Asp Thr Leu Arg Ser Glu Ala
       1595                1600                1605
   Phe Glu Tyr Tyr His Thr Leu Asp Glu Ser Phe Leu Gly Arg Tyr
       1610                1615                1620
   Met Ser Ala Leu Asn His Thr Lys Lys Trp Lys Phe Pro Gln Val
   1625 1630 1635
   Gly Gly Leu Thr Ser Ile Lys Trp Ala Asp Asn Asn Cys Tyr Leu
   1640 1645 1650
   Ser Ser Val Leu Leu Ala Leu Gln Gln Leu Glu Val Lys Phe Asn
   1655 1660 1665
   Ala Pro Ala Leu Gln Glu Ala Tyr Tyr Arg Ala Arg Ala Gly Asp
   1670 1675 1680
   Ala Ala Asn Phe Cys Ala Leu Ile Leu Ala Tyr Ser Asn Lys Thr
   1685 1690 1695
   Val Gly Glu Leu Gly Asp Val Arg Glu Thr Met Thr His Leu Leu
   1700 1705 1710
   Gln His Ala Asn Leu Glu Ser Ala Lys Arg Val Leu Asn Val Val
   1715 1720 1725
   Cys Lys His Cys Gly Gln Lys Thr Thr Thr Leu Thr Gly Val Glu
   1730 1735 1740
   Ala Val Met Tyr Met Gly Thr Leu Ser Tyr Asp Asn Leu Lys Thr
   1745 1750 1755
   Gly Val Ser Ile Pro Cys Val Cys Gly Arg Asp Ala Thr Gln Tyr
   1760 1765 1770
   Leu Val Gln Gln Glu Ser Ser Phe Val Met Met Ser Ala Pro Pro
   1775 1780 1785
   Ala Glu Tyr Lys Leu Gln Gln Gly Thr Phe Leu Cys Ala Asn Glu
   1790 1795 1800
   Tyr Thr Gly Asn Tyr Gln Cys Gly His Tyr Thr His Ile Thr Ala
   1805 1810 1815
   Lys Glu Thr Leu Tyr Arg Ile Asp Gly Ala His Leu Thr Lys Met
   1820 1825 1830
   Ser Glu Tyr Lys Gly Pro Val Thr Asp Val Phe Tyr Lys Glu Thr
   1835 1840 1845
   Ser Tyr Thr Thr Thr Ile Lys Pro Val Ser Tyr Lys Leu Asp Gly
   1850 1855 1860
   Val Thr Tyr Thr Glu Ile Glu Pro Lys Leu Asp Gly Tyr Tyr Lys
   1865 1870 1875
   Lys Asp Asn Ala Tyr Tyr Thr Glu Gln Pro Ile Asp Leu Val Pro
       1880                1885                1890
   Thr Gln Pro Leu Pro Asn Ala Ser Phe Asp Asn Phe Lys Leu Thr
       1895                1900                1905
   Cys Ser Asn Thr Lys Phe Ala Asp Asp Leu Asn Gln Met Thr Gly
       1910                1915                1920
   Phe Thr Lys Pro Ala Ser Arg Glu Leu Ser Val Thr Phe Phe Pro
       1925                1930                1935
   Asp Leu Asn Gly Asp Val Val Ala Ile Asp Tyr Arg His Tyr Ser
       1940                1945                1950
   Ala Ser Phe Lys Lys Gly Ala Lys Leu Leu His Lys Pro Ile Val
       1955                1960                1965
   Trp His Ile Asn Gln Ala Thr Thr Lys Thr Thr Phe Lys Pro Asn
       1970                1975                1980
   Thr Trp Cys Leu Arg Cys Leu Trp Ser Thr Lys Pro Val Asp Thr
       1985                1990                1995
   Ser Asn Ser Phe Glu Val Leu Ala Val Glu Asp Thr Gln Gly Met
       2000                2005                2010
   Asp Asn Leu Ala Cys Glu Ser Gln Gln Pro Thr Ser Glu Glu Val
       2015                2020                2025
   Val Glu Asn Pro Thr Ile Gln Lys Glu Val Ile Glu Cys Asp Val
       2030                2035                2040
   Lys Thr Thr Glu Val Val Gly Asn Val Ile Leu Lys Pro Ser Asp
       2045                2050                2055
   Glu Gly Val Lys Val Thr Gln Glu Leu Gly His Glu Asp Leu Met
       2060                2065                2070
   Ala Ala Tyr Val Glu Asn Thr Ser Ile Thr Ile Lys Lys Pro Asn
       2075                2080                2085
   Glu Leu Ser Leu Ala Leu Gly Leu Lys Thr Ile Ala Thr His Gly
       2090                2095                2100
   Ile Ala Ala Ile Asn Ser Val Pro Trp Ser Lys Ile Leu Ala Tyr
       2105                2110                2115
   Val Lys Pro Phe Leu Gly Gln Ala Ala Ile Thr Thr Ser Asn Cys
       2120                2125                2130
   Ala Lys Arg Leu Ala Gln Arg Val Phe Asn Asn Tyr Met Pro Tyr
       2135                2140                2145
   Val Phe Thr Leu Leu Phe Gln Leu Cys Thr Phe Thr Lys Ser Thr
       2150                2155                2160
   Asn Ser Arg Ile Arg Ala Ser Leu Pro Thr Thr Ile Ala Lys Asn
       2165                2170                2175
   Ser Val Lys Ser Val Ala Lys Leu Cys Leu Asp Ala Gly Ile Asn
       2180                2185                2190
   Tyr Val Lys Ser Pro Lys Phe Ser Lys Leu Phe Thr Ile Ala Met
       2195                2200                2205
   Trp Leu Leu Leu Leu Ser Ile Cys Leu Gly Ser Leu Ile Cys Val
       2210                2215                2220
   Thr Ala Ala Phe Gly Val Leu Leu Ser Asn Phe Gly Ala Pro Ser
       2225                2230                2235
   Tyr Cys Asn Gly Val Arg Glu Leu Tyr Leu Asn Ser Ser Asn Val
       2240                2245                2250
   Thr Thr Met Asp Phe Cys Glu Gly Ser Phe Pro Cys Ser Ile Cys
       2255                2260                2265
   Leu Ser Gly Leu Asp Ser Leu Asp Ser Tyr Pro Ala Leu Glu Thr
       2270                2275                2280
   Ile Gln Val Thr Ile Ser Ser Tyr Lys Leu Asp Leu Thr Ile Leu
       2285                2290                2295
   Gly Leu Ala Ala Glu Trp Val Leu Ala Tyr Met Leu Phe Thr Lys
       2300                2305                2310
   Phe Phe Tyr Leu Leu Gly Leu Ser Ala Ile Met Gln Val Phe Phe
       2315                2320                2325
   Gly Tyr Phe Ala Ser His Phe Ile Ser Asn Ser Trp Leu Met Trp
       2330                2335                2340
   Phe Ile Ile Ser Ile Val Gln Met Ala Pro Val Ser Ala Met Val
       2345                2350                2355
   Arg Met Tyr Ile Phe Phe Ala Ser Phe Tyr Tyr Ile Trp Lys Ser
       2360                2365                2370
   Tyr Val His Ile Met Asp Gly Cys Thr Ser Ser Thr Cys Met Met
       2375                2380                2385
   Cys Tyr Lys Arg Asn Arg Ala Thr Arg Val Glu Cys Thr Thr Ile
   2390 2395 2400
   Val Asn Gly Met Lys Arg Ser Phe Tyr Val Tyr Ala Asn Gly Gly
   2405 2410 2415
   Arg Gly Phe Cys Lys Thr His Asn Trp Asn Cys Leu Asn Cys Asp
   2420 2425 2430
   Thr Phe Cys Thr Gly Ser Thr Phe Ile Ser Asp Glu Val Ala Arg
   2435 2440 2445
   Asp Leu Ser Leu Gln Phe Lys Arg Pro Ile Asn Pro Thr Asp Gln
   2450 2455 2460
   Ser Ser Tyr Ile Val Asp Ser Val Ala Val Lys Asn Gly Ala Leu
   2465 2470 2475
   His Leu Tyr Phe Asp Lys Ala Gly Gln Lys Thr Tyr Glu Arg His
   2480 2485 2490
   Pro Leu Ser His Phe Val Asn Leu Asp Asn Leu Arg Ala Asn Asn
   2495 2500 2505
   Thr Lys Gly Ser Leu Pro Ile Asn Val Ile Val Phe Asp Gly Lys
   2510 2515 2520
   Ser Lys Cys Asp Glu Ser Ala Ser Lys Ser Ala Ser Val Tyr Tyr
   2525 2530 2535
   Ser Gln Leu Met Cys Gln Pro Ile Leu Leu Leu Asp Gln Ala Leu
   2540 2545 2550
   Val Ser Asp Val Gly Asp Ser Thr Glu Val Ser Val Lys Met Phe
   2555 2560 2565
   Asp Ala Tyr Val Asp Thr Phe Ser Ala Thr Phe Ser Val Pro Met
   2570 2575 2580
   Glu Lys Leu Lys Ala Leu Val Ala Thr Ala His Ser Glu Leu Ala
   2585 2590 2595
   Lys Gly Val Ala Leu Asp Gly Val Leu Ser Thr Phe Val Ser Ala
   2600 2605 2610
   Ala Arg Gln Gly Val Val Asp Thr Asp Val Asp Thr Lys Asp Val
   2615 2620 2625
   Ile Glu Cys Leu Lys Leu Ser His His Ser Asp Leu Glu Val Thr
   2630 2635 2640
   Gly Asp Ser Cys Asn Asn Phe Met Leu Thr Tyr Asn Lys Val Glu
       2645                2650                2655
   Asn Met Thr Pro Arg Asp Leu Gly Ala Cys Ile Asp Cys Asn Ala
       2660                2665                2670
   Arg His Ile Asn Ala Gln Val Ala Lys Ser His Asn Val Ser Leu
       2675                2680                2685
   Ile Trp Asn Val Lys Asp Tyr Met Ser Leu Ser Glu Gln Leu Arg
       2690                2695                2700
   Lys Gln Ile Arg Ser Ala Ala Lys Lys Asn Asn Ile Pro Phe Arg
       2705                2710                2715
   Leu Thr Cys Ala Thr Thr Arg Gln Val Val Asn Val Ile Thr Thr
       2720                2725                2730
   Lys Ile Ser Leu Lys Gly Gly Lys Ile Val Ser Thr Cys Phe Lys
       2735                2740                2745
   Leu Met Leu Lys Ala Thr Leu Leu Cys Val Leu Ala Ala Leu Val
       2750                2755                2760
   Cys Tyr Ile Val Met Pro Val His Thr Leu Ser Ile His Asp Gly
       2765                2770                2775
   Tyr Thr Asn Glu Ile Ile Gly Tyr Lys Ala Ile Gln Asp Gly Val
       2780                2785                2790
   Thr Arg Asp Ile Ile Ser Thr Asp Asp Cys Phe Ala Asn Lys His
       2795                2800                2805
   Ala Gly Phe Asp Ala Trp Phe Ser Gln Arg Gly Gly Ser Tyr Lys
       2810                2815                2820
   Asn Asp Lys Ser Cys Pro Val Val Ala Ala Ile Ile Thr Arg Glu
       2825                2830                2835
   Ile Gly Phe Ile Val Pro Gly Leu Pro Gly Thr Val Leu Arg Ala
       2840                2845                2850
   Ile Asn Gly Asp Phe Leu His Phe Leu Pro Arg Val Phe Ser Ala
       2855                2860                2865
   Val Gly Asn Ile Cys Tyr Thr Pro Ser Lys Leu Ile Glu Tyr Ser
       2870                2875                2880
   Asp Phe Ala Thr Ser Ala Cys Val Leu Ala Ala Glu Cys Thr Ile
       2885                2890                2895
   Phe Lys Asp Ala Met Gly Lys Pro Val Pro Tyr Cys Tyr Asp Thr
       2900                2905                2910
   Asn Leu Leu Glu Gly Ser Ile Ser Tyr Ser Glu Leu Arg Pro Asp
       2915                2920                2925
   Thr Arg Tyr Val Leu Met Asp Gly Ser Ile Ile Gln Phe Pro Asn
       2930                2935                2940
   Thr Tyr Leu Glu Gly Ser Val Arg Val Val Thr Thr Phe Asp Ala
       2945                2950                2955
   Glu Tyr Cys Arg His Gly Thr Cys Glu Arg Ser Glu Val Gly Ile
       2960                2965                2970
   Cys Leu Ser Thr Ser Gly Arg Trp Val Leu Asn Asn Glu His Tyr
       2975                2980                2985
   Arg Ala Leu Ser Gly Val Phe Cys Gly Val Asp Ala Met Asn Leu
       2990                2995                3000
   Ile Ala Asn Ile Phe Thr Pro Leu Val Gln Pro Val Gly Ala Leu
       3005                3010                3015
   Asp Val Ser Ala Ser Val Val Ala Gly Gly Ile Ile Ala Ile Leu
       3020                3025                3030
   Val Thr Cys Ala Ala Tyr Tyr Phe Met Lys Phe Arg Arg Val Phe
       3035                3040                3045
   Gly Glu Tyr Asn His Val Val Ala Ala Asn Ala Leu Leu Phe Leu
       3050                3055                3060
   Met Ser Phe Thr Ile Leu Cys Leu Val Pro Ala Tyr Ser Phe Leu
       3065                3070                3075
   Pro Gly Val Tyr Ser Val Phe Tyr Leu Tyr Leu Thr Phe Tyr Phe
       3080                3085                3090
   Thr Asn Asp Val Ser Phe Leu Ala His Leu Gln Trp Phe Ala Met
       3095                3100                3105
   Phe Ser Pro Ile Val Pro Phe Trp Ile Thr Ala Ile Tyr Val Phe
       3110                3115                3120
   Cys Ile Ser Leu Lys His Cys His Trp Phe Phe Asn Asn Tyr Leu
       3125                3130                3135
   Arg Lys Arg Val Met Phe Asn Gly Val Thr Phe Ser Thr Phe Glu
       3140                3145                3150
   Glu Ala Ala Leu Cys Thr Phe Leu Leu Asn Lys Glu Met Tyr Leu
   3155 3160 3165
   Lys Leu Arg Ser Glu Thr Leu Leu Pro Leu Thr Gln Tyr Asn Arg
   3170 3175 3180
   Tyr Leu Ala Leu Tyr Asn Lys Tyr Lys Tyr Phe Ser Gly Ala Leu
   3185 3190 3195
   Asp Thr Thr Ser Tyr Arg Glu Ala Ala Cys Cys His Leu Ala Lys
   3200 3205 3210
   Ala Leu Asn Asp Phe Ser Asn Ser Gly Ala Asp Val Leu Tyr Gln
   3215 3220 3225
   Pro Pro Gln Thr Ser Ile Thr Ser Ala Val Leu Gln Ser Gly Phe
   3230 3235 3240
   Arg Lys Met Ala Phe Pro Ser Gly Lys Val Glu Gly Cys Met Val
   3245 3250 3255
   Gln Val Thr Cys Gly Thr Thr Thr Leu Asn Gly Leu Trp Leu Asp
   3260 3265 3270
   Asp Thr Val Tyr Cys Pro Arg His Val Ile Cys Thr Ala Glu Asp
   3275 3280 3285
   Met Leu Asn Pro Asn Tyr Glu Asp Leu Leu Ile Arg Lys Ser Asn
   3290 3295 3300
   His Ser Phe Leu Val Gln Ala Gly Asn Val Gln Leu Arg Val Ile
   3305 3310 3315
   Gly His Ser Met Gln Asn Cys Leu Leu Arg Leu Lys Val Asp Thr
   3320 3325 3330
   Ser Asn Pro Lys Thr Pro Lys Tyr Lys Phe Val Arg Ile Gln Pro
   3335 3340 3345
   Gly Gln Thr Phe Ser Val Leu Ala Cys Tyr Asn Gly Ser Pro Ser
   3350 3355 3360
   Gly Val Tyr Gln Cys Ala Met Arg Pro Asn His Thr Ile Lys Gly
   3365 3370 3375
   Ser Phe Leu Asn Gly Ser Cys Gly Ser Val Gly Phe Asn Ile Asp
   3380 3385 3390
   Tyr Asp Cys Val Ser Phe Cys Tyr Met His His Met Glu Leu Pro
   3395 3400 3405
   Thr Gly Val His Ala Gly Thr Asp Leu Glu Gly Lys Phe Tyr Gly
       3410                3415                3420
   Pro Phe Val Asp Arg Gln Thr Ala Gln Ala Ala Gly Thr Asp Thr
       3425                3430                3435
   Thr Ile Thr Leu Asn Val Leu Ala Trp Leu Tyr Ala Ala Val Ile
       3440                3445                3450
   Asn Gly Asp Arg Trp Phe Leu Asn Arg Phe Thr Thr Thr Leu Asn
       3455                3460                3465
   Asp Phe Asn Leu Val Ala Met Lys Tyr Asn Tyr Glu Pro Leu Thr
       3470                3475                3480
   Gln Asp His Val Asp Ile Leu Gly Pro Leu Ser Ala Gln Thr Gly
       3485                3490                3495
   Ile Ala Val Leu Asp Met Cys Ala Ala Leu Lys Glu Leu Leu Gln
       3500                3505                3510
   Asn Gly Met Asn Gly Arg Thr Ile Leu Gly Ser Thr Ile Leu Glu
       3515                3520                3525
   Asp Glu Phe Thr Pro Phe Asp Val Val Arg Gln Cys Ser Gly Val
       3530                3535                3540
   Thr Phe Gln Gly Lys Phe Lys Lys Ile Val Lys Gly Thr His His
       3545                3550                3555
   Trp Met Leu Leu Thr Phe Leu Thr Ser Leu Leu Ile Leu Val Gln
       3560                3565                3570
   Ser Thr Gln Trp Ser Leu Phe Phe Phe Val Tyr Glu Asn Ala Phe
       3575                3580                3585
   Leu Pro Phe Thr Leu Gly Ile Met Ala Ile Ala Ala Cys Ala Met
       3590                3595                3600
   Leu Leu Val Lys His Lys His Ala Phe Leu Cys Leu Phe Leu Leu
       3605                3610                3615
   Pro Ser Leu Ala Thr Val Ala Tyr Phe Asn Met Val Tyr Met Pro
       3620                3625                3630
   Ala Ser Trp Val Met Arg Ile Met Thr Trp Leu Glu Leu Ala Asp
       3635                3640                3645
   Thr Ser Leu Ser Gly Tyr Arg Leu Lys Asp Cys Val Met Tyr Ala
       3650                3655                3660
   Ser Ala Leu Val Leu Leu Ile Leu Met Thr Ala Arg Thr Val Tyr
   3665 3670 3675
   Asp Asp Ala Ala Arg Arg Val Trp Thr Leu Met Asn Val Ile Thr
   3680 3685 3690
   Leu Val Tyr Lys Val Tyr Tyr Gly Asn Ala Leu Asp Gln Ala Ile
   3695 3700 3705
   Ser Met Trp Ala Leu Val Ile Ser Val Thr Ser Asn Tyr Ser Gly
   3710 3715 3720
   Val Val Thr Thr Ile Met Phe Leu Ala Arg Ala Ile Val Phe Val
   3725 3730 3735
   Cys Val Glu Tyr Tyr Pro Leu Leu Phe Ile Thr Gly Asn Thr Leu
   3740 3745 3750
   Gln Cys Ile Met Leu Val Tyr Cys Phe Leu Gly Tyr Cys Cys Cys
   3755 3760 3765
   Cys Tyr Phe Gly Leu Phe Cys Leu Leu Asn Arg Tyr Phe Arg Leu
   3770 3775 3780
   Thr Leu Gly Val Tyr Asp Tyr Leu Val Ser Thr Gln Glu Phe Arg
   3785 3790 3795
   Tyr Met Asn Ser Gln Gly Leu Leu Pro Pro Lys Ser Ser Ile Asp
   3800 3805 3810
   Ala Phe Lys Leu Asn Ile Lys Leu Leu Gly Ile Gly Gly Lys Pro
   3815 3820 3825
   Cys Ile Lys Val Ala Thr Val Gln Ser Lys Met Ser Asp Val Lys
   3830 3835 3840
   Cys Thr Ser Val Val Leu Leu Ser Val Leu Gln Gln Leu Arg Val
   3845 3850 3855
   Glu Ser Ser Ser Lys Leu Trp Ala Gln Cys Val Gln Leu His Asn
   3860 3865 3870
   Asp Ile Leu Leu Ala Lys Asp Thr Thr Glu Ala Phe Glu Lys Met
   3875 3880 3885
   Val Ser Leu Leu Ser Val Leu Leu Ser Met Gln Gly Ala Val Asp
   3890 3895 3900
   Ile Asn Arg Leu Cys Glu Glu Met Leu Asp Asn Arg Ala Thr Leu
   3905 3910 3915
   Gln Ala Ile Ala Ser Glu Phe Ser Ser Leu Pro Ser Tyr Ala Ala
   3920 3925 3930
   Tyr Ala Thr Ala Gln Glu Ala Tyr Glu Gln Ala Val Ala Asn Gly
   3935 3940 3945
   Asp Ser Glu Val Val Leu Lys Lys Leu Lys Lys Ser Leu Asn Val
   3950 3955 3960
   Ala Lys Ser Glu Phe Asp Arg Asp Ala Ala Met Gln Arg Lys Leu
   3965 3970 3975
   Glu Lys Met Ala Asp Gln Ala Met Thr Gln Met Tyr Lys Gln Ala
   3980 3985 3990
   Arg Ser Glu Asp Lys Arg Ala Lys Val Thr Ser Ala Met Gln Thr
   3995 4000 4005
   Met Leu Phe Thr Met Leu Arg Lys Leu Asp Asn Asp Ala Leu Asn
   4010 4015 4020
   Asn Ile Ile Asn Asn Ala Arg Asp Gly Cys Val Pro Leu Asn Ile
   4025 4030 4035
   Ile Pro Leu Thr Thr Ala Ala Lys Leu Met Val Val Val Pro Asp
   4040 4045 4050
   Tyr Gly Thr Tyr Lys Asn Thr Cys Asp Gly Asn Thr Phe Thr Tyr
   4055 4060 4065
   Ala Ser Ala Leu Trp Glu Ile Gln Gln Val Val Asp Ala Asp Ser
   4070 4075 4080
   Lys Ile Val Gln Leu Ser Glu Ile Asn Met Asp Asn Ser Pro Asn
   4085 4090 4095
   Leu Ala Trp Pro Leu Ile Val Thr Ala Leu Arg Ala Asn Ser Ala
   4100 4105 4110
   Val Lys Leu Gln Asn Asn Glu Leu Ser Pro Val Ala Leu Arg Gln
   4115 4120 4125
   Met Ser Cys Ala Ala Gly Thr Thr Gln Thr Ala Cys Thr Asp Asp
   4130 4135 4140
   Asn Ala Leu Ala Tyr Tyr Asn Asn Ser Lys Gly Gly Arg Phe Val
   4145 4150 4155
   Leu Ala Leu Leu Ser Asp His Gln Asp Leu Lys Trp Ala Arg Phe
   4160 4165 4170
   Pro Lys Ser Asp Gly Thr Gly Thr Ile Tyr Thr Glu Leu Glu Pro
       4175                    4180                    4185
   Pro Cys Arg Phe Val Thr Asp Thr Pro Lys Gly Pro Lys Val Lys
       4190                    4195                    4200
   Tyr Leu Tyr Phe Ile Lys Gly Leu Asn Asn Leu Asn Arg Gly Met
       4205                    4210                    4215
   Val Leu Gly Ser Leu Ala Ala Thr Val Arg Leu Gln Ala Gly Asn
       4220                    4225                    4230
   Ala Thr Glu Val Pro Ala Asn Ser Thr Val Leu Ser Phe Cys Ala
       4235                    4240                    4245
   Phe Ala Val Asp Pro Ala Lys Ala Tyr Lys Asp Tyr Leu Ala Ser
       4250                    4255                    4260
   Gly Gly Gln Pro Ile Thr Asn Cys Val Lys Met Leu Cys Thr His
       4265                    4270                    4275
   Thr Gly Thr Gly Gln Ala Ile Thr Val Thr Pro Glu Ala Asn Met
       4280                    4285                    4290
   Asp Gln Glu Ser Phe Gly Gly Ala Ser Cys Cys Leu Tyr Cys Arg
       4295                    4300                    4305
   Cys His Ile Asp His Pro Asn Pro Lys Gly Phe Cys Asp Leu Lys
       4310                    4315                    4320
   Gly Lys Tyr Val Gln Ile Pro Thr Thr Cys Ala Asn Asp Pro Val
       4325                    4330                    4335
   Gly Phe Thr Leu Arg Asn Thr Val Cys Thr Val Cys Gly Met Trp
       4340                    4345                    4350
   Lys Gly Tyr Gly Cys Ser Cys Asp Gln Leu Arg Glu Pro Leu Met
       4355                    4360                    4365
   Gln Ser Ala Asp Ala Ser Thr Phe Leu Asn Gly Phe Ala Val
       4370                    4375                    4380

<210> 75

<211> 2695

<212> PRT

<213> CORONAVIRUS

<400> 75

Arg Val Cys Gly Val Ser Ala Ala Arg Leu Thr Pro Cys Gly Thr Gly
1               5                   10                  15

Thr Ser Thr Asp Val Val Tyr Arg Ala Phe Asp Ile Tyr Asn Glu Lys
            20                  25                  30

Val Ala Gly Phe Ala Lys Phe Leu Lys Thr Asn Cys Cys Arg Phe Gln
        35                  40                  45

Glu Lys Asp Glu Glu Gly Asn Leu Leu Asp Ser Tyr Phe Val Val Lys
    50                  55                  60

Arg His Thr Met Ser Asn Tyr Gln His Glu Glu Thr Ile Tyr Asn Leu
65                  70                  75                  80

Val Lys Asp Cys Pro Ala Val Ala Val His Asp Phe Phe Lys Phe Arg
                85                  90                  95

Val Asp Gly Asp Met Val Pro His Ile Ser Arg Gln Arg Leu Thr Lys
            100                 105                 110

Tyr Thr Met Ala Asp Leu Val Tyr Ala Leu Arg His Phe Asp Glu Gly
        115                 120                 125

Asn Cys Asp Thr Leu Lys Glu Ile Leu Val Thr Tyr Asn Cys Cys Asp
    130                 135                 140

Asp Asp Tyr Phe Asn Lys Lys Asp Trp Tyr Asp Phe Val Glu Asn Pro
145                 150                 155                 160

Asp Ile Leu Arg Val Tyr Ala Asn Leu Gly Glu Arg Val Arg Gln Ser
                165                 170                 175

Leu Leu Lys Thr Val Gln Phe Cys Asp Ala Met Arg Asp Ala Gly Ile
            180                 185                 190

Val Gly Val Leu Thr Leu Asp Asn Gln Asp Leu Asn Gly Asn Trp Tyr
        195                 200                 205

Asp Phe Gly Asp Phe Val Gln Val Ala Pro Gly Cys Gly Val Pro Ile
    210                 215                 220

Val Asp Ser Tyr Tyr Ser Leu Leu Met Pro Ile Leu Thr Leu Thr Arg
225                 230                 235                 240

Ala Leu Ala Ala Glu Ser His Met Asp Ala Asp Leu Ala Lys Pro Leu
                245                 250                 255

Ile Lys Trp Asp Leu Leu Lys Tyr Asp Phe Thr Glu Glu Arg Leu Cys
            260                 265                 270

Leu Phe Asp Arg Tyr Phe Lys Tyr Trp Asp Gln Thr Tyr His Pro Asn
        275                 280                 285

Cys Ile Asn Cys Leu Asp Asp Arg Cys Ile Leu His Cys Ala Asn Phe
    290                 295                 300

Asn Val Leu Phe Ser Thr Val Phe Pro Pro Thr Ser Phe Gly Pro Leu
305                 310                 315                 320

Val Arg Lys Ile Phe Val Asp Gly Val Pro Phe Val Val Ser Thr Gly
                325                 330                 335

Tyr His Phe Arg Glu Leu Gly Val Val His Asn Gln Asp Val Asn Leu
            340                 345                 350

His Ser Ser Arg Leu Ser Phe Lys Glu Leu Leu Val Tyr Ala Ala Asp
        355                 360                 365

Pro Ala Met His Ala Ala Ser Gly Asn Leu Leu Leu Asp Lys Arg Thr
    370                 375                 380

Thr Cys Phe Ser Val Ala Ala Leu Thr Asn Asn Val Ala Phe Gln Thr
385                 390                 395                 400

Val Lys Pro Gly Asn Phe Asn Lys Asp Phe Tyr Asp Phe Ala Val Ser
                405                 410                 415

Lys Gly Phe Phe Lys Glu Gly Ser Ser Val Glu Leu Lys His Phe Phe
            420                 425                 430

Phe Ala Gln Asp Gly Asn Ala Ala Ile Ser Asp Tyr Asp Tyr Tyr Arg
        435                 440                 445

Tyr Asn Leu Pro Thr Met Cys Asp Ile Arg Gln Leu Leu Phe Val Val
    450                 455                 460

Glu Val Val Asp Lys Tyr Phe Asp Cys Tyr Asp Gly Gly Cys Ile Asn
465                 470                 475                 480

Ala Asn Gln Val Ile Val Asn Asn Leu Asp Lys Ser Ala Gly Phe Pro
                485                 490                 495

Phe Asn Lys Trp Gly Lys Ala Arg Leu Tyr Tyr Asp Ser Met Ser Tyr
            500                 505                 510

Glu Asp Gln Asp Ala Leu Phe Ala Tyr Thr Lys Arg Asn Val Ile Pro
        515                 520                 525

Thr Ile Thr Gln Met Asn Leu Lys Tyr Ala Ile Ser Ala Lys Asn Arg
    530                 535                 540

Ala Arg Thr Val Ala Gly Val Ser Ile Cys Ser Thr Met Thr Asn Arg
545                 550                 555                 560

Gln Phe His Gln Lys Leu Leu Lys Ser Ile Ala Ala Thr Arg Gly Ala
                565                 570                 575

Thr Val Val Ile Gly Thr Ser Lys Phe Tyr Gly Gly Trp His Asn Met
            580                 585                 590

Leu Lys Thr Val Tyr Ser Asp Val Glu Thr Pro His Leu Met Gly Trp
        595                 600                 605

Asp Tyr Pro Lys Cys Asp Arg Ala Met Pro Asn Met Leu Arg Ile Met
    610                 615                 620

Ala Ser Leu Val Leu Ala Arg Lys His Asn Thr Cys Cys Asn Leu Ser
625                 630                 635                 640

His Arg Phe Tyr Arg Leu Ala Asn Glu Cys Ala Gln Val Leu Ser Glu
                645                 650                 655

Met Val Met Cys Gly Gly Ser Leu Tyr Val Lys Pro Gly Gly Thr Ser
            660                 665                 670

Ser Gly Asp Ala Thr Thr Ala Tyr Ala Asn Ser Val Phe Asn Ile Cys
        675                 680                 685

Gln Ala Val Thr Ala Asn Val Asn Ala Leu Leu Ser Thr Asp Gly Asn
    690                 695                 700

Lys Ile Ala Asp Lys Tyr Val Arg Asn Leu Gln His Arg Leu Tyr Glu
705                 710                 715                 720

Cys Leu Tyr Arg Asn Arg Asp Val Asp His Glu Phe Val Asp Glu Phe
                725                 730                 735

Tyr Ala Tyr Leu Arg Lys His Phe Ser Met Met Ile Leu Ser Asp Asp
            740                 745                 750

Ala Val Val Cys Tyr Asn Ser Asn Tyr Ala Ala Gln Gly Leu Val Ala
        755                 760                 765

Ser Ile Lys Asn Phe Lys Ala Val Leu Tyr Tyr Gln Asn Asn Val Phe
    770                 775                 780

Met Ser Glu Ala Lys Cys Trp Thr Glu Thr Asp Leu Thr Lys Gly Pro
785                 790                 795                 800

His Glu Phe Cys Ser Gln His Thr Met Leu Val Lys Gln Gly Asp Asp
                805                 810                 815

Tyr Val Tyr Leu Pro Tyr Pro Asp Pro Ser Arg Ile Leu Gly Ala Gly
            820                     825                     830

Cys Phe Val Asp Asp Ile Val Lys Thr Asp Gly Thr Leu Met Ile Glu
        835                     840                     845

Arg Phe Val Ser Leu Ala Ile Asp Ala Tyr Pro Leu Thr Lys His Pro
    850                     855                     860

Asn Gln Glu Tyr Ala Asp Val Phe His Leu Tyr Leu Gln Tyr Ile Arg
865                     870                     875                     880

Lys Leu His Asp Glu Leu Thr Gly His Met Leu Asp Met Tyr Ser Val
                885                     890                     895

Met Leu Thr Asn Asp Asn Thr Ser Arg Tyr Trp Glu Pro Glu Phe Tyr
            900                     905                     910

Glu Ala Met Tyr Thr Pro His Thr Val Leu Gln Ala Val Gly Ala Cys
        915                     920                     925

Val Leu Cys Asn Ser Gln Thr Ser Leu Arg Cys Gly Ala Cys Ile Arg
    930                     935                     940

Arg Pro Phe Leu Cys Cys Lys Cys Cys Tyr Asp His Val Ile Ser Thr
945                     950                     955                     960

Ser His Lys Leu Val Leu Ser Val Asn Pro Tyr Val Cys Asn Ala Pro
                965                     970                     975

Gly Cys Asp Val Thr Asp Val Thr Gln Leu Tyr Leu Gly Gly Met Ser
            980                     985                     990

Tyr Tyr Cys Lys Ser His Lys Pro  Pro Ile Ser Phe Pro  Leu Cys Ala
        995                     1000                    1005

Asn Gly  Gln Val Phe Gly Leu  Tyr Lys Asn Thr Cys  Val Gly Ser
    1010                    1015                    1020

Asp Asn  Val Thr Asp Phe Asn  Ala Ile Ala Thr Cys  Asp Trp Thr
    1025                    1030                    1035

Asn Ala  Gly Asp Tyr Ile Leu  Ala Asn Thr Cys Thr  Glu Arg Leu
    1040                    1045                    1050

Lys Leu  Phe Ala Ala Glu Thr  Leu Lys Ala Thr Glu  Glu Thr Phe
    1055                    1060                    1065

Lys Leu  Ser Tyr Gly Ile Ala  Thr Val Arg Glu Val  Leu Ser Asp
    1070                    1075                    1080

Arg Glu Leu His Leu Ser Trp Glu Val Gly Lys Pro Arg Pro Pro
    1085                1090                1095

Leu Asn Arg Asn Tyr Val Phe Thr Gly Tyr Arg Val Thr Lys Asn
    1100                1105                1110

Ser Lys Val Gln Ile Gly Glu Tyr Thr Phe Glu Lys Gly Asp Tyr
    1115                1120                1125

Gly Asp Ala Val Val Tyr Arg Gly Thr Thr Thr Tyr Lys Leu Asn
    1130                1135                1140

Val Gly Asp Tyr Phe Val Leu Thr Ser His Thr Val Met Pro Leu
    1145                1150                1155

Ser Ala Pro Thr Leu Val Pro Gln Glu His Tyr Val Arg Ile Thr
    1160                1165                1170

Gly Leu Tyr Pro Thr Leu Asn Ile Ser Asp Glu Phe Ser Ser Asn
    1175                1180                1185

Val Ala Asn Tyr Gln Lys Val Gly Met Gln Lys Tyr Ser Thr Leu
    1190                1195                1200

Gln Gly Pro Pro Gly Thr Gly Lys Ser His Phe Ala Ile Gly Leu
    1205                1210                1215

Ala Leu Tyr Tyr Pro Ser Ala Arg Ile Val Tyr Thr Ala Cys Ser
    1220                1225                1230

His Ala Ala Val Asp Ala Leu Cys Glu Lys Ala Leu Lys Tyr Leu
    1235                1240                1245

Pro Ile Asp Lys Cys Ser Arg Ile Ile Pro Ala Arg Ala Arg Val
    1250                1255                1260

Glu Cys Phe Asp Lys Phe Lys Val Asn Ser Thr Leu Glu Gln Tyr
    1265                1270                1275

Val Phe Cys Thr Val Asn Ala Leu Pro Glu Thr Thr Ala Asp Ile
    1280                1285                1290

Val Val Phe Asp Glu Ile Ser Met Ala Thr Asn Tyr Asp Leu Ser
    1295                1300                1305

Val Val Asn Ala Arg Leu Arg Ala Lys His Tyr Val Tyr Ile Gly
    1310                1315                1320

Asp Pro Ala Gln Leu Pro Ala Pro Arg Thr Leu Leu Thr Lys Gly
    1325                1330                1335

Thr Leu Glu Pro Glu Tyr Phe Asn Ser Val Cys Arg Leu Met Lys
    1340                1345                1350

Thr Ile Gly Pro Asp Met Phe Leu Gly Thr Cys Arg Arg Cys Pro
    1355                1360                1365

Ala Glu Ile Val Asp Thr Val Ser Ala Leu Val Tyr Asp Asn Lys
    1370                1375                1380

Leu Lys Ala His Lys Asp Lys Ser Ala Gln Cys Phe Lys Met Phe
    1385                1390                1395

Tyr Lys Gly Val Ile Thr His Asp Val Ser Ser Ala Ile Asn Arg
    1400                1405                1410

Pro Gln Ile Gly Val Val Arg Glu Phe Leu Thr Arg Asn Pro Ala
    1415                1420                1425

Trp Arg Lys Ala Val Phe Ile Ser Pro Tyr Asn Ser Gln Asn Ala
    1430                1435                1440

Val Ala Ser Lys Ile Leu Gly Leu Pro Thr Gln Thr Val Asp Ser
    1445                1450                1455

Ser Gln Gly Ser Glu Tyr Asp Tyr Val Ile Phe Thr Gln Thr Thr
    1460                1465                1470

Glu Thr Ala His Ser Cys Asn Val Asn Arg Phe Asn Val Ala Ile
    1475                1480                1485

Thr Arg Ala Lys Ile Gly Ile Leu Cys Ile Met Ser Asp Arg Asp
    1490                1495                1500

Leu Tyr Asp Lys Leu Gln Phe Thr Ser Leu Glu Ile Pro Arg Arg
    1505                1510                1515

Asn Val Ala Thr Leu Gln Ala Glu Asn Val Thr Gly Leu Phe Lys
    1520                1525                1530

Asp Cys Ser Lys Ile Ile Thr Gly Leu His Pro Thr Gln Ala Pro
    1535                1540                1545

Thr His Leu Ser Val Asp Ile Lys Phe Lys Thr Glu Gly Leu Cys
    1550                1555                1560

Val Asp Ile Pro Gly Ile Pro Lys Asp Met Thr Tyr Arg Arg Leu
    1565                1570                1575

Ile Ser Met Met Gly Phe Lys Met Asn Tyr Gln Val Asn Gly Tyr
    1580                1585                1590

Pro Asn Met Phe Ile Thr Arg Glu Glu Ala Ile Arg His Val Arg
    1595                1600                1605

Ala Trp Ile Gly Phe Asp Val Glu Gly Cys His Ala Thr Arg Asp
    1610                1615                1620

Ala Val Gly Thr Asn Leu Pro Leu Gln Leu Gly Phe Ser Thr Gly
    1625                1630                1635

Val Asn Leu Val Ala Val Pro Thr Gly Tyr Val Asp Thr Glu Asn
    1640                1645                1650

Asn Thr Glu Phe Thr Arg Val Asn Ala Lys Pro Pro Pro Gly Asp
    1655                1660                1665

Gln Phe Lys His Leu Ile Pro Leu Met Tyr Lys Gly Leu Pro Trp
    1670                1675                1680

Asn Val Val Arg Ile Lys Ile Val Gln Met Leu Ser Asp Thr Leu
    1685                1690                1695

Lys Gly Leu Ser Asp Arg Val Val Phe Val Leu Trp Ala His Gly
    1700                1705                1710

Phe Glu Leu Thr Ser Met Lys Tyr Phe Val Lys Ile Gly Pro Glu
    1715                1720                1725

Arg Thr Cys Cys Leu Cys Asp Lys Arg Ala Thr Cys Phe Ser Thr
    1730                1735                1740

Ser Ser Asp Thr Tyr Ala Cys Trp Asn His Ser Val Gly Phe Asp
    1745                1750                1755

Tyr Val Tyr Asn Pro Phe Met Ile Asp Val Gln Gln Trp Gly Phe
    1760                1765                1770

Thr Gly Asn Leu Gln Ser Asn His Asp Gln His Cys Gln Val His
    1775                1780                1785

Gly Asn Ala His Val Ala Ser Cys Asp Ala Ile Met Thr Arg Cys
    1790                1795                1800

Leu Ala Val His Glu Cys Phe Val Lys Arg Val Asp Trp Ser Val
    1805                1810                1815

Glu Tyr Pro Ile Ile Gly Asp Glu Leu Arg Val Asn Ser Ala Cys
    1820                1825                1830

Arg Lys Val Gln His Met Val Val Lys Ser Ala Leu Leu Ala Asp
    1835                1840                1845

Lys Phe Pro Val Leu His Asp Ile Gly Asn Pro Lys Ala Ile Lys
    1850                1855                1860

Cys Val Pro Gln Ala Glu Val Glu Trp Lys Phe Tyr Asp Ala Gln
    1865                1870                1875

Pro Cys Ser Asp Lys Ala Tyr Lys Ile Glu Glu Leu Phe Tyr Ser
    1880                1885                1890

Tyr Ala Thr His His Asp Lys Phe Thr Asp Gly Val Cys Leu Phe
    1895                1900                1905

Trp Asn Cys Asn Val Asp Arg Tyr Pro Ala Asn Ala Ile Val Cys
    1910                1915                1920

Arg Phe Asp Thr Arg Val Leu Ser Asn Leu Asn Leu Pro Gly Cys
    1925                1930                1935

Asp Gly Gly Ser Leu Tyr Val Asn Lys His Ala Phe His Thr Pro
    1940                1945                1950

Ala Phe Asp Lys Ser Ala Phe Thr Asn Leu Lys Gln Leu Pro Phe
    1955                1960                1965

Phe Tyr Tyr Ser Asp Ser Pro Cys Glu Ser His Gly Lys Gln Val
    1970                1975                1980

Val Ser Asp Ile Asp Tyr Val Pro Leu Lys Ser Ala Thr Cys Ile
    1985                1990                1995

Thr Arg Cys Asn Leu Gly Gly Ala Val Cys Arg His His Ala Asn
    2000                2005                2010

Glu Tyr Arg Gln Tyr Leu Asp Ala Tyr Asn Met Met Ile Ser Ala
    2015                2020                2025

Gly Phe Ser Leu Trp Ile Tyr Lys Gln Phe Asp Thr Tyr Asn Leu
    2030                2035                2040

Trp Asn Thr Phe Thr Arg Leu Gln Ser Leu Glu Asn Val Ala Tyr
    2045                2050                2055

Asn Val Val Asn Lys Gly His Phe Asp Gly His Ala Gly Glu Ala
    2060                2065                2070

Pro Val Ser Ile Ile Asn Asn Ala Val Tyr Thr Lys Val Asp Gly
    2075                2080                2085

Ile Asp Val Glu Ile Phe Glu Asn Lys Thr Thr Leu Pro Val Asn
    2090                2095                2100

Val Ala Phe Glu Leu Trp Ala Lys Arg Asn Ile Lys Pro Val Pro
    2105                2110                2115

Glu Ile Lys Ile Leu Asn Asn Leu Gly Val Asp Ile Ala Ala Asn
    2120                2125                2130

Thr Val Ile Trp Asp Tyr Lys Arg Glu Ala Pro Ala His Val Ser
    2135                2140                2145

Thr Ile Gly Val Cys Thr Met Thr Asp Ile Ala Lys Lys Pro Thr
    2150                2155                2160

Glu Ser Ala Cys Ser Ser Leu Thr Val Leu Phe Asp Gly Arg Val
    2165                2170                2175

Glu Gly Gln Val Asp Leu Phe Arg Asn Ala Arg Asn Gly Val Leu
    2180                2185                2190

Ile Thr Glu Gly Ser Val Lys Gly Leu Thr Pro Ser Lys Gly Pro
    2195                2200                2205

Ala Gln Ala Ser Val Asn Gly Val Thr Leu Ile Gly Glu Ser Val
    2210                2215                2220

Lys Thr Gln Phe Asn Tyr Phe Lys Lys Val Asp Gly Ile Ile Gln
    2225                2230                2235

Gln Leu Pro Glu Thr Tyr Phe Thr Gln Ser Arg Asp Leu Glu Asp
    2240                2245                2250

Phe Lys Pro Arg Ser Gln Met Glu Thr Asp Phe Leu Glu Leu Ala
    2255                2260                2265

Met Asp Glu Phe Ile Gln Arg Tyr Lys Leu Glu Gly Tyr Ala Phe
    2270                2275                2280

Glu His Ile Val Tyr Gly Asp Phe Ser His Gly Gln Leu Gly Gly
    2285                2290                2295

Leu His Leu Met Ile Gly Leu Ala Lys Arg Ser Gln Asp Ser Pro
    2300                2305                2310

Leu Lys Leu Glu Asp Phe Ile Pro Met Asp Ser Thr Val Lys Asn
    2315                2320                2325

Tyr Phe Ile Thr Asp Ala Gln Thr Gly Ser Ser Lys Cys Val Cys
    2330                2335                2340

Ser Val Ile Asp Leu Leu Leu Asp Asp Phe Val Glu Ile Ile Lys
    2345                2350                2355

Ser Gln Asp Leu Ser Val Ile Ser Lys Val Val Lys Val Thr Ile
    2360                2365                2370

Asp Tyr Ala Glu Ile Ser Phe Met Leu Trp Cys Lys Asp Gly His
    2375                2380                2385

Val Glu Thr Phe Tyr Pro Lys Leu Gln Ala Ser Gln Ala Trp Gln
    2390                2395                2400

Pro Gly Val Ala Met Pro Asn Leu Tyr Lys Met Gln Arg Met Leu
    2405                2410                2415

Leu Glu Lys Cys Asp Leu Gln Asn Tyr Gly Glu Asn Ala Val Ile
    2420                2425                2430

Pro Lys Gly Ile Met Met Asn Val Ala Lys Tyr Thr Gln Leu Cys
    2435                2440                2445

Gln Tyr Leu Asn Thr Leu Thr Leu Ala Val Pro Tyr Asn Met Arg
    2450                2455                2460

Val Ile His Phe Gly Ala Gly Ser Asp Lys Gly Val Ala Pro Gly
    2465                2470                2475

Thr Ala Val Leu Arg Gln Trp Leu Pro Thr Gly Thr Leu Leu Val
    2480                2485                2490

Asp Ser Asp Leu Asn Asp Phe Val Ser Asp Ala Asp Ser Thr Leu
    2495                2500                2505

Ile Gly Asp Cys Ala Thr Val His Thr Ala Asn Lys Trp Asp Leu
    2510                2515                2520

Ile Ile Ser Asp Met Tyr Asp Pro Arg Thr Lys His Val Thr Lys
    2525                2530                2535

Glu Asn Asp Ser Lys Glu Gly Phe Phe Thr Tyr Leu Cys Gly Phe
    2540                2545                2550

Ile Lys Gln Lys Leu Ala Leu Gly Gly Ser Ile Ala Val Lys Ile
    2555                2560                2565

Thr Glu His Ser Trp Asn Ala Asp Leu Tyr Lys Leu Met Gly His
    2570                2575                2580

Phe Ser Trp Trp Thr Ala Phe Val Thr Asn Val Asn Ala Ser Ser
    2585                2590                2595

Ser Glu Ala Phe Leu Ile Gly Ala Asn Tyr Leu Gly Lys Pro Lys
    2600                2605                2610

Glu Gln Ile Asp Gly Tyr Thr Met His Ala Asn Tyr Ile Phe Trp
    2615                2620                2625

Arg Asn Thr Asn Pro Ile Gln Leu Ser Ser Tyr Ser Leu Phe Asp
    2630                2635                2640

Met Ser Lys Phe Pro Leu Lys Leu Arg Gly Thr Ala Val Met Ser
    2645                2650                2655

Leu Lys Glu Asn Gln Ile Asn Asp Met Ile Tyr Ser Leu Leu Glu
    2660                2665                2670

Lys Gly Arg Leu Ile Ile Arg Glu Asn Asn Arg Val Val Val Ser
    2675                2680                2685

Ser Asp Ile Leu Val Asn Asn
    2690                2695

<210> 76

<211> 20

<212> DNA

<213> Artificial sequence

<220>

<223> amorce S/L3/+/4932

<400> 76
ccacacacag cttgtggata 20

<210> 77

<211> 20

<212> DNA

<213> Artificial sequence

<220>

<223> amorce S/L4/+/6401

<400> 77
ccgaagttgt aggcaatgtc 20

<210> 78

<211> 20

<212> DNA

<213>  Artificial sequence

<220>

<223>  amorce S/L4/+/6964

<400>  78
tttggtgctc cttcttattg                                                    20

<210>  79

<211>  20

<212>  DNA

<213>  Artificial sequence

<220>

<223>  amorce S/L4/-/6817

<400>  79
ccggcatcca aacataattt                                                    20

<210>  80

<211>  20

<212>  DNA

<213>  Artificial sequence

<220>

<223>  amorce S/L5/-/7633

<400>  80
tggtcagtag ggttgattgg                                                    20

<210>  81

<211>  20

<212>  DNA

<213>  Artificial sequence

<220>

<223>  S/L5/-/8127

<400>  81
catcctttgt gtcaacatcg                                                    20

<210>  82

<211>  20

<212>  DNA

<213>  Artificial sequence

<220>

<223>  amorce S/L5/-/8633

<400>  82
gtcacgagtg acaccatcct                                                    20

<210>  83

<211>  20

<212>  DNA

<213>  Artificial sequence

<220>

<223>  amorce S/L5/+/7839

<400>  83
atgcgacgag tctgcttcta                                                    20

<210>  84

<211>  20

<212>  DNA

<213>  Artificial sequence

<220>

<223>  amorce S/L5/+/8785

<400>  84
ttcatagtgc ctggcttacc                                                    20

<210>  85

<211>  20

<212>  DNA

<213>  Artificial sequence

<220>

<223>  amorce S/L5/+/8255

<400>  85
atcttggcgc atgtattgac                                                    20

<210> 86

<211> 20

<212> DNA

<213> Artificial sequence

<220>

<223> amorce S/L6/-/9422

<400> 86
tgcattagca gcaacaacat 20

<210> 87

<211> 20

<212> DNA

<213> Artificial sequence

<220>

<223> amorce S/L6/-/9966

<400> 87
tctgcagaac agcagaagtg 20

<210> 88

<211> 20

<212> DNA

<213> Artificial sequence

<220>

<223> amorce S/L6/-/10542

<400> 88
cctgtgcagt ttgtctgtca 20

<210> 89

<211> 20

<212> DNA

<213> Artificial sequence

<220>

<223>  amorce S/L6/+/10677

<400>  89
ccttgtggca atgaagtaca                                                    20

<210>  90

<211>  20

<212>  DNA

<213>  Artificial sequence

<220>

<223>  amorce S/L6/+/10106

<400>  90
atgtcatttg cacagcagaa                                                    20

<210>  91

<211>  20

<212>  DNA

<213>  Artificial sequence

<220>

<223>  amorce S/L6/+/9571

<400>  91
cttcaatggt ttgccatgtt                                                    20

<210>  92

<211>  20

<212>  DNA

<213>  Artificial sequence

<220>

<223>  amorce S/L7/-/11271

<400>  92
tgcgagctgt catgagaata                                                    20

<210>  93

<211>  20

<212>  DNA

<213>  Artificial sequence

<220>

<223>  amorce S/L7/-/11801

<400>  93
aaccgagagc agtaccacag                                                    20

<210>  94

<211>  20

<212>  DNA

<213>  Artificial sequence

<220>

<223>  amorce S/L7/-/12383

<400>  94
tttggctgct gtagtcaatg                                                    20

<210>  95

<211>  20

<212>  DNA

<213>  Artificial sequence

<220>

<223>  amorce S/L7/+/12640

<400>  95
ctacgacaga tgtcctgtgc                                                    20

<210>  96

<211>  20

<212>  DNA

<213>  Artificial sequence

<220>

<223>  amorce S/L7/+/12088

<400>  96
gagcaggctg tagctaatgg                                                    20

<210>  97

<211>  20

<212>  DNA

<213>  Artificial sequence

<220>

<223>  amorce S/L7/+/11551

<400>  97
ttaggctatt gttgctgctg                                                    20

<210>  98

<211>  20

<212>  DNA

<213>  Artificial sequence

<220>

<223>  amorce S/L8/-/13160

<400>  98
cagacaacat gaagcaccac                                                    20

<210>  99

<211>  20

<212>  DNA

<213>  Artificial sequence

<220>

<223>  amorce S/L8/-/13704

<400>  99
cgctgacgtg atatatgtgg                                                    20

<210>  100

<211>  20

<212>  DNA

<213>  Artificial sequence

<220>

<223>  amorce S/L8/-/14284

<400>  100
tgcacaatga aggatacacc                                                    20

<210>  101

<211>  20

<212>  DNA

<213>  Artificial sequence



<220>

<223>  amorce S/L8/+/14453

<400>  101
acatagctcg cgtctcagtt                                                    20

<210>  102

<211>  20

<212>  DNA

<213>  Artificial sequence



<220>

<223>  amorce S/L8/+/13968

<400>  102
ggcattgtag gcgtactgac                                                    20

<210>  103

<211>  19

<212>  DNA

<213>  Artificial sequence



<220>

<223>  amorce S/L8/+/13401

<400>  103
gtttgcggtg taagtgcag                                                     19

<210>  104

<211>  20

<212>  DNA

<213>  Artificial sequence



<220>

<223>  amorce S/L9/-/15098

<400>  104
tagtggcggc tattgacttc                                                    20

<210>  105

<211>  20

<212>  DNA

<213>  Artificial sequence


<220>

<223>  amorce S/L9/-/15677

<400>  105
ctaaaccttg agccgcatag                                                    20

<210>  106

<211>  20

<212>  DNA

<213>  Artificial sequence


<220>

<223>  amorce S/L9/-/16247

<400>  106
catggtcata gcagcacttg                                                    20

<210>  107

<211>  21

<212>  DNA

<213>  Artificial sequence


<220>

<223>  amorce S/L9/+/16323

<400>  107
ccaggttgtg atgtcactga t                                                  21

<210>  108

<211>  20

<212>  DNA

<213>  Artificial sequence

<220>

<223>  amorce S/L9/+/15858

<400>  108
ccttacccag atccatcaag                                                    20

<210>  109

<211>  20

<212>  DNA

<213>  Artificial sequence

<220>

<223>  amorce S/L9/+/15288

<400>  109
cgcaaacata acacttgctg                                                    20

<210>  110

<211>  20

<212>  DNA

<213>  Artificial sequence

<220>

<223>  amorce S/L10/-/16914

<400>  110
agtgttgggt acaagccagt                                                    20

<210>  111

<211>  20

<212>  DNA

<213>  Artificial sequence

<220>

<223>  amorce S/L10/-/17466

<400>  111
gttccaagga acatgtctgg                                                    20

<210>  112

<211>  20

<212>  DNA

<213>  Artificial sequence

<220>
<223>  amorce S/L10/-/18022

<400>  112
aggtgcctgt gtaggatgaa                                                    20

<210>  113
<211>  20
<212>  DNA
<213>  Artificial sequence

<220>
<223>  amorce S/L10/+/18245

<400>  113
gggctgtcat gcaactagag                                                    20

<210>  114
<211>  20
<212>  DNA
<213>  Artificial sequence

<220>
<223>  amorce S/L10/+/17663

<400>  114
tcttacacgc aatcctgctt                                                    20

<210>  115
<211>  20
<212>  DNA
<213>  Artificial sequence

<220>
<223>  amorce S/L10/+/17061

<400>  115
tacccatctg ctcgcatagt                                                    20

<210>  116

<211>  20

<212>  DNA

<213>  Artificial sequence

<220>

<223>  amorce S/L11/-/18877

<400>  116
gcaagcagaa ttaaccctca                                                    20

<210>  117

<211>  20

<212>  DNA

<213>  Artificial sequence

<220>

<223>  amorce S/L11/-/19396

<400>  117
agcaccacct aaattgcatc                                                    20

<210>  118

<211>  20

<212>  DNA

<213>  Artificial sequence

<220>

<223>  amorce S/L11/-/20002

<400>  118
tggtcccttt gaaggtgtta                                                    20

<210>  119

<211>  20

<212>  DNA

<213>  Artificial sequence

<220>

<223>  amorce S/L11/+/20245

<400>  119
tcgaacacat cgtttatgga                                                    20

<210>  120

<211>  20

<212>  DNA

<213>  Artificial sequence



<220>

<223>  amorce S/L11/+/19611

<400>  120
gaagcacctg tttccatcat                                                    20

<210>  121

<211>  20

<212>  DNA

<213>  Artificial sequence



<220>

<223>  amorce S/L11/+/19021

<400>  121
acgatgctca gccatgtagt                                                    20

<210>  122

<211>  20

<212>  DNA

<213>  Artificial sequence



<220>

<223>  amorce SARS/L1/F3/+/800

<400>  122
gaggtgcagt cactcgctat                                                    20

<210>  123

<211>  20

<212>  DNA

<213>  Artificial sequence



<220>

<223>  amorce SARS/L1/F4/+/1391

<400>  123
cagagattgg acctgagcat                                                    20

<210>  124

<211>  20

<212>  DNA

<213>  Artificial sequence

<220>

<223>  amorce SARS/L1/F5/+/1925

<400>  124
cagcaaacca ctcaattcct                                                    20

<210>  125

<211>  20

<212>  DNA

<213>  Artificial sequence

<220>

<223>  amorce SARS/L1/R3/-/1674

<400>  125
aaatgatggc aacctcttca                                                    20

<210>  126

<211>  20

<212>  DNA

<213>  Artificial sequence

<220>

<223>  amorce SARS/L1/R4/-/1107

<400>  126
cacgtggttg aatgactttg                                                    20

<210>  127

<211>  20

<212>  DNA

<213>  Artificial sequence

<220>

<223>  amorce SARS/L1/R5/-/520

<400>  127
atttctgcaa ccagctcaac                                                    20

<210>  128

<211>  20

<212>  DNA

<213>  Artificial sequence

<220>

<223>  amorce SARS/L2/F3/+/2664

<400>  128
cgcattgtct cctggtttac                                                    20

<210>  129

<211>  20

<212>  DNA

<213>  Artificial sequence

<220>

<223>  amorce SARS/L2/F4/+/3232

<400>  129
gagattgagc cagaaccaga                                                    20

<210>  130

<211>  20

<212>  DNA

<213>  Artificial sequence

<220>

<223>  amorce SARS/L2/F5/+/3746

<400>  130
atgagcaggt tgtcatggat                                                    20

<210>  131

<211>  20

<212>  DNA

<213>  Artificial sequence

<220>

<223>  amorce SARS/L2/R3/-/3579

<400>  131
ctgccttaag aagctggatg                                                    20

<210>  132

<211>  20

<212>  DNA

<213>  Artificial sequence

<220>

<223>  amorce SARS/L2/R4/-/2991

<400>  132
tttcttcacc agcatcatca                                                    20

<210>  133

<211>  20

<212>  DNA

<213>  Artificial sequence

<220>

<223>  amorce SARS/L2/R5/-/2529

<400>  133
caccgttctt gagaacaacc                                                    20

<210>  134

<211>  20

<212>  DNA

<213>  Artificial sequence

<220>

<223>  amorce SARS/L3/F3/+/4708

<400>  134
tctttggctg gctcttacag                                                    20

<210>  135

<211>  20

<212>  DNA

<213>  Artificial sequence

<220>

<223>  amorce SRAS/L3/F4/+/5305

<400>  135
gctggtgatg ctgctaactt                                                    20

<210>  136

<211>  20

<212>  DNA

<213>  Artificial sequence

<220>

<223>  amorce SARS/L3/F5/+/5822

<400>  136
ccatcaagcc tgtgtcgtat                                                    20

<210>  137

<211>  20

<212>  DNA

<213>  Artificial sequence

<220>

<223>  amorce SARS/L3/R3/-/5610

<400>  137
caggtggtgc agacatcata                                                    20

<210>  138

<211>  20

<212>  DNA

<213>  Artificial sequence

<220>

<223>  amorce SARS/L3/R4/-/4988

<400>  138
aacatcagca ccatccaagt                                                    20

<210>  139

<211>  20

<212>  DNA

<213>  Artificial sequence

<220>

<223>  amorce SARS/L3/R5/-/4437

<400>  139
atcggacacc atagtcaacg                                                    20

<210>  140

<211>  7788

<212>  DNA

<213>  Artificial sequence

<220>

<223>  gène S synthétique

<400>  140
tcaatattgg ccattagcca tattattcat tggttatata gcataaatca atattggcta      60

ttggccattg catacgttgt atctatatca taatatgtac atttatattg gctcatgtcc     120

aatatgaccg ccatgttggc attgattatt gactagttat taatagtaat caattacggg     180

gtcattagtt catagcccat atatggagtt ccgcgttaca taacttacgg taaatggccc     240

gcctggctga ccgcccaacg acccccgccc attgacgtca ataatgacgt atgttcccat     300

agtaacgcca atagggactt tccattgacg tcaatgggtg gagtatttac ggtaaactgc     360

ccacttggca gtacatcaag tgtatcatat gccaagtccg ccccctattg acgtcaatga     420

cggtaaatgg cccgcctggc attatgccca gtacatgacc ttacgggact ttcctacttg     480

gcagtacatc tacgtattag tcatcgctat taccatggtg atgcggtttt ggcagtacac     540

caatgggcgt ggatagcggt ttgactcacg gggatttcca agtctccacc ccattgacgt     600

caatgggagt ttgttttggc accaaaatca acgggacttt ccaaaatgtc gtaataaccc     660

cgccccgttg acgcaaatgg gcggtaggcg tgtacggtgg gaggtctata taagcagagc     720

tcgtttagtg aaccgtcaga tcactagaag ctttattgcg gtagtttatc acagttaaat     780

tgctaacgca gtcagtgctt ctgacacaac agtctcgaac ttaagctgca gaagttggtc     840

gtgaggcact gggcaggtaa gtatcaaggt tacaagacag gtttaaggag accaatagaa     900

actgggcttg tcgagacaga gaagactctt gcgtttctga taggcaccta ttggtcttac     960

tgacatccac tttgcctttc tctccacagg tgtccactcc cagttcaatt acagctctta 1020
aggctagagt acttaatacg actcactata ggctagcgga tccaccatgt tcatcttcct 1080
gctgttcctg accctgacca gcggcagcga cctggaccgg tgcaccacct tcgacgacgt 1140
gcaggccccc aactacaccc agcacaccag cagcatgcgg ggcgtgtact accccgacga 1200
gatctttcgg agcgacaccc tgtacctgac ccaggacctg ttcctgccct tctacagcaa 1260
cgtgaccggc ttccacacca tcaaccacac cttcggcaac cccgtgatcc ccttcaagga 1320
cggcatctac ttcgccgcca ccgagaagag caacgtggtg cggggctggg tgttcggcag 1380
caccatgaac aacaagagcc agagcgtgat catcatcaac aacagcacca acgtggtgat 1440
ccgggcctgc aacttcgagc tgtgcgacaa ccccttcttc gccgtgtcca aacccatggg 1500
cacccagacc cacaccatga tcttcgacaa cgccttcaac tgcaccttcg agtacatcag 1560
cgacgccttc agcctggacg tgagcgagaa gagcggcaac ttcaagcacc tgcgggagtt 1620
cgtgttcaag aacaaggacg gcttcctgta cgtgtacaag ggctaccagc ccatcgacgt 1680
ggtgagagac ctgcccagcg gcttcaacac cctgaagccc atcttcaagc tgcccctggg 1740
catcaacatc accaacttcc gggccatcct gaccgccttt agccctgccc aggacatctg 1800
gggcaccagc gccgccgcct acttcgtggg ctacctgaag cctaccacct tcatgctgaa 1860
gtacgacgag aacggcacca tcaccgacgc cgtggactgc agccagaacc ccctggccga 1920
gctgaagtgc agcgtgaaga gcttcgagat cgacaagggc atctaccaga ccagcaactt 1980
cagagtggtg cctagcggcg atgtggtgcg gttccccaat atcaccaacc tgtgcccctt 2040
cggcgaagtg ttcaacgcca ccaagttccc cagcgtgtac gcctgggagc ggaagaagat 2100
cagcaactgc gtggccgact acagcgtgct gtacaactcc accttcttca gcaccttcaa 2160
gtgctacggc gtgagcgcca ccaagctgaa cgacctgtgc ttcagcaacg tgtacgccga 2220
cagcttcgtg gtgaagggcg acgacgtgag acagatcgcc cctggccaga ccggcgtgat 2280
cgccgactac aactacaagc tgcccgacga cttcatgggc tgcgtgctgg cctggaacac 2340
ccggaacatc gacgccacaa gcaccggcaa ctacaattac aagtaccgct acctgcggca 2400
cggcaagctg cggcccttcg agcgggacat ctccaacgtg cccttcagcc ccgacggcaa 2460
gccctgcacc ccccctgccc tgaactgcta ctggcccctg aacgactacg gcttctacac 2520
caccaccggc atcggctatc agccctacag agtggtggtg ctgagcttcg agctgctgaa 2580
cgcccctgcc accgtgtgcg gccccaagct gagcaccgac ctgatcaaga accagtgcgt 2640
gaacttcaac ttcaacggcc tgaccggcac cggcgtgctg acccccagca gcaagcgctt 2700
ccagcccttc cagcagttcg gccgggatgt gagcgacttc accgacagcg tgcgggaccc 2760
caagaccagc gagatcctgg acatcagccc ctgcagcttc ggcggcgtgt ccgtgatcac 2820
ccccggcacc aacgccagca gcgaagtggc cgtgctgtac caggacgtga actgcaccga 2880
cgtgagcacc gccatccacg ccgaccagct gacccccgcc tggcggatct acagcaccgg 2940
gaacaacgtg ttccagaccc aggccggctg cctgatcggc gccgagcacg tggacaccag 3000

ctacgagtgc gacatcccca ttggcgccgg aatctgcgcc agctaccaca ccgtgagcct 3060
gctgcggagc accagccaga agtccatcgt ggcctacacc atgagcctgg gcgccgacag 3120
cagcatcgcc tacagcaaca acaccatcgc catccccacc aacttcagca tctccatcac 3180
caccgaagtg atgcccgtga gcatggccaa gacaagcgtg gattgcaaca tgtacatctg 3240
cggcgacagc accgagtgcg ccaacctgct gctgcagtac ggcagcttct gcacccagct 3300
gaaccgggcc ctgagcggca tcgccgccga gcaggaccgg aacaccagag aagtgttcgc 3360
ccaagtgaag cagatgtata agacccccac cctgaagtac ttcgggggct tcaacttctc 3420
tcagatcctg cccgaccctc tgaagcccac caagcgctcc ttcatcgagg acctgctgtt 3480
caacaaagtg accctggccg acgccggctt tatgaagcag tacggcgagt gcctgggcga 3540
catcaacgcc cgggacctga tctgcgccca gaagtttaac gggctgaccg tgctgccccc 3600
cctgctgacc gacgacatga tcgccgccta tacagccgcc ctggtgagcg gcaccgccac 3660
cgccggctgg accttcggag ccggagccgc cctgcagatc cccttcgcca tgcagatggc 3720
ctaccggttc aacggcatcg gcgtgaccca gaacgtgctg tacgagaacc agaagcagat 3780
cgccaaccag ttcaacaagg ccatcagcca gatccaggag agcctgacca caaccagcac 3840
cgccctgggc aagctgcagg acgtggtgaa ccagaacgcc caggccctga acaccctggt 3900
gaagcagctg agcagcaact tcggcgccat cagctctgtg ctgaacgaca tcctgagcag 3960
gctggacaaa gtggaggccg aagtgcagat cgaccggctg atcaccggac gcctgcagtc 4020
cctgcagacc tacgtgaccc agcagctgat cagagccgcc gagatccggg ccagcgccaa 4080
tctggccgcc accaagatga gcgagtgcgt gctgggccag agcaagagag tggacttctg 4140
cggcaagggc tatcacctga tgagcttccc ccaggccgcc ccccacggcg tggtgttcct 4200
gcacgtgacc tacgtgccta gccaggagcg gaacttcacc accgccccag ccatctgcca 4260
cgagggcaag gcctacttcc cccgggaggg cgtgttcgtg tttaacggca ccagctggtt 4320
catcacccag cgcaacttct tcagccccca gatcatcacc acagacaaca ccttcgtgtc 4380
cggcaactgt gatgtggtga tcggcatcat caataacacc gtgtacgacc ccctgcagcc 4440
cgagctggac agcttcaagg aggagctgga caaatacttc aagaaccaca cctcccccga 4500
cgtggacctg ggcgatatca gcggcatcaa cgcctccgtg gtgaacatcc agaaggagat 4560
cgacagactg aacgaagtgg ccaagaacct gaacgagagc ctgatcgacc tgcaggagct 4620
gggcaagtac gagcagtaca tcaagtggcc ctggtacgtg tggctgggct tcatcgccgg 4680
cctgatcgcc atcgtgatgg tgaccatcct gctgtgctgc atgaccagct gctgtagctg 4740
cctgaaaggc gcctgcagct gtggcagctg ctgcaagttc gacgaggacg acagcgagcc 4800
cgtgctgaag ggcgtgaagc tgcactacac ctgataactc gagaattcac gcgtggtacc 4860
tctagagtcg acccgggcgg ccgcttcgag cagacatgat aagatacatt gatgagtttg 4920
gacaaaccac aactagaatg cagtgaaaaa aatgctttat ttgtgaaatt tgtgatgcta 4980
ttgctttatt tgtaaccatt ataagctgca ataaacaagt taacaacaac aattgcattc 5040

attttatgtt tcaggttcag ggggagatgt gggaggtttt ttaaagcaag taaaacctct 5100
acaaatgtgg taaaatcgat aaggatccgg gctggcgtaa tagcgaagag gcccgcaccg 5160
atcgcccttc ccaacagttg cgcagcctga atggcgaatg gacgcgccct gtagcggcgc 5220
attaagcgcg gcgggtgtgg tggttacgcg cagcgtgacc gctacacttg ccagcgccct 5280
agcgcccgct cctttcgctt tcttcccttc ctttctcgcc acgttcgccg gctttccccg 5340
tcaagctcta aatcgggggc tccctttagg gttccgattt agagctttac ggcacctcga 5400
ccgcaaaaaa cttgatttgg gtgatggttc acgtagtggg ccatcgccct gatagacggt 5460
ttttcgccct ttgacgttgg agtccacgtt ctttaatagt ggactcttgt tccaaactgg 5520
aacaacactc aaccctatct cggtctattc ttttgattta taagggattt tgccgatttc 5580
ggcctattgg ttaaaaaatg agctgattta acaaatattt aacgcgaatt ttaacaaaat 5640
attaacgttt acaatttcgc ctgatgcggt attttctcct tacgcatctg tgcggtattt 5700
cacaccgcat atggtgcact ctcagtacaa tctgctctga tgccgcatag ttaagccagc 5760
cccgacaccc gccaacaccc gctgacgcgc cctgacgggc ttgtctgctc ccggcatccg 5820
cttacagaca agctgtgacc gtctccggga gctgcatgtg tcagaggttt tcaccgtcat 5880
caccgaaacg cgcgagacga aagggcctcg tgatacgcct atttttatag gttaatgtca 5940
tgataataat ggtttcttag acgtcaggtg gcacttttcg gggaaatgtg cgcggaaccc 6000
ctatttgttt atttttctaa atacattcaa atatgtatcc gctcatgaga caataaccct 6060
gataaatgct tcaataatat tgaaaaagga agagtatgag tattcaacat ttccgtgtcg 6120
cccttattcc cttttttgcg gcattttgcc ttcctgtttt tgctcaccca gaaacgctgg 6180
tgaaagtaaa agatgctgaa gatcagttgg gtgcacgagt gggttacatc gaactggatc 6240
tcaacagcgg taagatcctt gagagttttc gccccgaaga acgttttcca atgatgagca 6300
cttttaaagt tctgctatgt ggcgcggtat tatcccgtat tgacgccggg caagagcaac 6360
tcggtcgccg catacactat tctcagaatg acttggttga gtactcacca gtcacagaaa 6420
agcatcttac ggatggcatg acagtaagag aattatgcag tgctgccata accatgagtg 6480
ataacactgc ggccaactta cttctgacaa cgatcggagg accgaaggag ctaaccgctt 6540
ttttgcacaa catgggggat catgtaactc gccttgatcg ttgggaaccg gagctgaatg 6600
aagccatacc aaacgacgag cgtgacacca cgatgcctgt agcaatggca acaacgttgc 6660
gcaaactatt aactggcgaa ctacttactc tagcttcccg gcaacaatta atagactgga 6720
tggaggcgga taaagttgca ggaccacttc tgcgctcggc ccttccggct ggctggttta 6780
ttgctgataa atctggagcc ggtgagcgtg ggtctcgcgg tatcattgca gcactggggc 6840
cagatggtaa gccctcccgt atcgtagtta tctacacgac ggggagtcag gcaactatgg 6900
atgaacgaaa tagacagatc gctgagatag gtgcctcact gattaagcat tggtaactgt 6960
cagaccaagt ttactcatat atactttaga ttgatttaaa acttcatttt taatttaaaa 7020
ggatctaggt gaagatcctt tttgataatc tcatgaccaa aatcccttaa cgtgagtttt 7080

cgttccactg agcgtcagac cccgtagaaa agatcaaagg atcttcttga gatccttttt 7140

ttctgcgcgt aatctgctgc ttgcaaacaa aaaaaccacc gctaccagcg gtggtttgtt 7200

tgccggatca agagctacca actctttttc cgaaggtaac tggcttcagc agagcgcaga 7260

taccaaatac tgtccttcta gtgtagccgt agttaggcca ccacttcaag aactctgtag 7320

caccgcctac atacctcgct ctgctaatcc tgttaccagt ggctgctgcc agtggcgata 7380

agtcgtgtct taccgggttg gactcaagac gatagttacc ggataaggcg cagcggtcgg 7440

gctgaacggg gggttcgtgc acacagccca gcttggagcg aacgacctac accgaactga 7500

gatacctaca gcgtgagcta tgagaaagcg ccacgcttcc cgaagggaga aaggcggaca 7560

ggtatccggt aagcggcagg gtcggaacag gagagcgcac gagggagctt ccagggggaa 7620

acgcctggta tctttatagt cctgtcgggt ttcgccacct ctgacttgag cgtcgatttt 7680

tgtgatgctc gtcagggggg cggagcctat ggaaaaacgc cagcaacgcg gcctttttac 7740

ggttcctggc cttttgctgg ccttttgctc acatggctcg acagatct 7788

<210> 141

<211> 23

<212> DNA

<213> Artificial sequence

<220>

<223> amorce SNE-S1

<400> 141
ggttgggatt atccaaaatg tga 23

<210> 142

<211> 24

<212> DNA

<213> Artificial sequence

<220>

<223> amorce SNE-AS1

<400> 142
gcatcatcag aaagaatcat catg 24

<210> 143

<211> 21

<212> DNA

<213>  Artificial sequence

<220>
<223>  amorce SAR1-S

<400>  143
cctctcttgt tcttgctcgc a                                                21

<210>  144
<211>  21
<212>  DNA
<213>  Artificial sequence

<220>
<223>  amorce SAR1-AS

<400>  144
tatagtgagc cgccacacat g                                                21

<210>  145
<211>  45
<212>  DNA
<213>  Artificial sequence

<220>
<223>  amorce PCR

<400>  145
ataggatcca ccatgtttat tttcttatta tttcttactc tcact                      45

<210>  146
<211>  37
<212>  DNA
<213>  Artificial sequence

<220>
<223>  amorce PCR

<400>  146
   atactcgagt tatgtgtaat gtaatttgac acccttg                                37

<210>  147

<211>  45

<212>  DNA

<213>  Artificial sequence



<220>

<223>  amorce PCR

<400>  147
ataggatcca ccatgtttat tttcttatta tttcttactc tcact                        45

<210>  148

<211>  36

<212>  DNA

<213>  Artificial sequence



<220>

<223>  amorce PCR

<400>  148
acctccggat ttaatatatt gctcatattt tcccaa                                  36

<210>  149

<211>  13

<212>  PRT

<213>  Artificial sequence



<220>

<223>  extrémité N-terminale de la protéine S du SRAS-CoV (acides amines
        1 à 13)

<400>  149

Met Phe Ile Phe Leu Leu Phe Leu Thr Leu Thr Ser Gly
1               5                   10

<210>  150

<211>  10

<212>  PRT

<213>  Artificial sequence



<220>

<223>  oligopeptide

<400>  150

Ser Gly Asp Tyr Lys Asp Asp Asp Asp Lys
1               5                   10

<210>  151

<211>  34

<212>  DNA

<213>  Artificial sequence

<220>

<223>  amorce PCR

<400>  151
actagctagc ggatccacca tgttcatctt cctg                                34

<210>  152

<211>  33

<212>  DNA

<213>  Artificial sequence

<220>

<223>  amorce PCR

<400>  152
agtatccgga cttgatgtac tgctcgtact tgc                                 33

<210>  153

<211>  59

<212>  DNA

<213>  Artificial sequence

<220>

<223>  oligonucléotide

<400>  153
tatgagcttt tttttttttt tttttttggc atataaatag actcggcgcg ccatctgca      59

<210>  154

<211>  53

<212>  DNA

<213>  Artificial sequence

<220>

<223>  oligonucléotide

<400>  154
gatggcgcgc cgagtctatt tatatgccaa aaaaaaaaaa aaaaaaaagc tca                53

<210>  155

<211>  45

<212>  DNA

<213>  Artificial sequence


<220>

<223>  amorce PCR

<400>  155
atacgtacga ccatgtttat tttcttatta tttcttactc tcact                        45

<210>  156

<211>  40

<212>  DNA

<213>  Artificial sequence


<220>

<223>  amorce PCR

<400>  156
atagcgcgct cattatgtgt aatgtaattt gacacccttg                              40

<210>  157

<211>  20

<212>  DNA

<213>  Artificial sequence


<220>

<223>  amorce PCR

<400>  157
ccatttcaac aatttggccg                                                    20

<210>  158

<211>  45

<212>  DNA

<213>  Artificial sequence

<220>

<223>  amorce PCR

<400>  158
ataggatccg cgcgctcatt atttatcgtc gtcatcttta taatc                    45

## Description

La présente invention est relative à une nouvelle souche de coronavirus associé au syndrome respiratoire aigu sévère (SRAS), issue d'un prélèvement répertorié sous le n° 031589 et prélevé à Hanoi (Vietnam), à des molécules d'acide nucléique issues de son génome, aux protéines et peptides codés par lesdites molécules d'acide nucléique ainsi qu'à leurs applications, notamment en tant que réactifs de diagnostic et/ou comme vaccin.

Le coronavirus est un virus à ARN monocaténaire, de polarité positive, d'approximativement 30 kilobases qui se réplique dans le cytoplasme des cellules hôtes ; l'extrémité 5' du génome a une structure en coiffe et l'extrémité 3' comporte une queue polyA. Ce virus est enveloppé et comprend, à sa surface, des structures péplomériques dénommées spicules.

Le génome comprend les cadres ouverts de lecture ou ORF suivants, de son extrémité 5' vers son extrémité 3' : ORF1a et ORF1b correspondant aux protéines du complexe de transcription-réplication, et ORF-S, ORF-E, ORF-M et ORF-N correspondant aux protéines structurales S, E, M et N. Il comprend également des ORFs correspondant à des protéines de fonction inconnue codées par : la région située entre l'ORF-S et l'ORF-E et chevauchant cette dernière, la région située entre l'ORF-M et l'ORF-N, et la région incluse dans l'ORF-N.

La protéine S est une glycoprotéine membranaire (200-220 kDa) qui se présente sous la forme de spicules ou "Spike" émergeant de la surface de l'enveloppe virale. Elle est responsable de l'attachement du virus aux récepteurs de la cellule hôte et de l'induction de la fusion de l'enveloppe virale avec la membrane cellulaire.

La petite protéine d'enveloppe (E) également dénommée sM (*small membrane*) qui est une protéine trans-membranaire non glycosylée d'environ 10 kDa, est la protéine présente en plus faible quantité dans le virion. Elle joue un rôle moteur dans le processus de bourgeonnement des coronavirus qui se produit au niveau du compartiment intermédiaire dans le réticulum endoplasmique et l'appareil de Golgi

La protéine M ou protéine de matrice (25-30 kDa) est une glycoprotéine membranaire plus abondante qui est intégrée dans la particule virale par une interaction M/E, tandis que l'incorporation de S dans les particules est dirigée par une interaction S/M. Elle semble être importante pour la maturation virale des coronavirus et pour la détermination du site au niveau duquel les particules virales sont assemblées.

La protéine N ou protéine de nucléocapside (45-50 kDa) qui est la plus conservée parmi les protéines structurales des coronavirus, est nécessaire pour encapsider l'ARN génomique puis pour diriger son incorporation dans le virion. Cette protéine est vraisemblablement également impliquée dans la réplication de l'ARN.

Lorsqu'une cellule hôte est infectée, le cadre de lecture (ORF) situé en 5' du génome viral est traduit en une polyprotéine qui est clivée par les protéases virales et libère alors plusieurs protéines non-structurales telles que l'ARN-polymérase ARN dépendante (Rep) et l'ATPase hélicase (Hel). Ces deux protéines sont impliquées dans la réplication du génome viral ainsi que dans la génération de transcrits qui sont utilisés dans la synthèse des protéines virales. Les mécanismes par lesquels ces ARNms sub-génomiques sont produits, ne sont pas complètement compris ; cependant des faits récents indiquent que les séquences de régulation de la transcription à l'extrémité 5' de chaque gène représentent des signaux qui régulent la transcription discontinue des ARNms sub-génomiques.

Les protéines de la membrane virale (protéines S, E et M) sont insérées dans le compartiment intermédiaire, alors que l'ARN répliqué (brin +) s'assemble avec la protéine N (nucléocapside). Ce complexe protéine-ARN s'associe ensuite avec la protéine M incluse dans les membranes du réticulum endoplasmique et les particules virales se forment lorsque le complexe de la nucléocapside bourgeonne dans le réticulum endoplasmique. Le virus migre ensuite à travers le complexe du Golgi et éventuellement sort de la cellule, par exemple par exocytose. Le site de l'attachement du virus à la cellule hôte se trouve au niveau de la protéine S.

Les coronavirus sont responsables de 15 à 30 % des rhumes chez l'Homme et d'infections respiratoires ou digestives chez les animaux, notamment le chat (FIPV : *Feline infectious peritonitis virus*), la volaille (IBV : *Avian Infectious bronchitis virus*), la souris (MHV : *Mouse Hepatitis virus*), le porc (TGEV : Transmissible gastroenterititis virus, PEDV : *Porcine Epidemic Diarrhea virus,* PRCoV: *Porcine Respiratory Coronavirus,* HEV : *Hemagglutinating encephalomyelitis Virus*) et les bovins (BcoV : *Bovine coronavirus*).

En général, chaque coronavirus n'affecte qu'une seule espèce ; chez les individus immunocompétents, l'infection induit des anticorps éventuellement neutralisants et une immunité cellulaire, capables de détruire les cellules infectées.

Une épidémie de pneumonie atypique, dénommée syndrome respiratoire aigu sévère (SARS ou *Severe acute respiratory syndrome,* SRAS en français) s'est propagée dans différents pays (Vietnam, Hong-Kong, Singapour, Thaïlande et Canada) au cours du premier trimestre 2003, à partir d'un foyer initial apparu en Chine dans le dernier trimestre de 2002. La sévérité de cette maladie est telle que son taux de mortalité est d'environ 3 à 6 %. La détermination de l'agent causatif de cette maladie a été entreprise par de nombreux laboratoires, à travers le monde.

En mars 2003, un nouveau coronavirus (SARS-CoV, SARS virus ou virus SRAS, en français) a été isolé, en association avec des cas de syndrome respiratoire aigu sévère (T.G.KSIAZEK et al., The New England Journal of Medicine, 2003, 348, 1319-1330 ; C. DROSTEN et al., The New England Journal of Medicine, 2003, 348, 1967-1976 ; Peiris et al., Lancet, 2003, 361, 1319-).

Des séquences génomiques de ce nouveau coronavirus ont ainsi été obtenues, notamment celles de l'isolat Urbani (Genbank n° d'accès AY274119.3 et A. MARRA et al., Science, May 1, 2003, 300, 1399-1404) et de l'isolat de Toronto (Tor2, Genbank n° d'accès AY 278741 et A. ROTA et al., Science, 2003, 300, 1394-1399).

L'organisation du génome est comparable à celle des autres coronavirus connus permettant ainsi de confirmer l'appartenance du SARS-CoV à la famille des *Coronaviridae*; les cadres ouverts de lecture ORF1a et 1b et les cadres ouverts de lecture correspondant aux protéines S, E, M, et N, ainsi qu'à des protéines codées par : la région située entre l'ORF-S et l'ORF-E (ORF3), la région située entre l'ORF-S et l'ORF-E et chevauchant l'ORF-E (ORF4), la région située entre l'ORF-M et l'ORF-N (ORF7 à ORF11) et la région correspondant à l'ORF-N (ORF 13 et ORF 14), ont notamment été identifiées.

Sept différences ont été mises en évidence entre les séquences des isolats Tor2 et Urbani ; 3 correspondent à des mutations silencieuses (c/t en position 16622 et a/g en position 19064 de l'ORF1b, t/c en position 24872 de l'ORF-S) et 4 modifient la séquence en acides aminés de respectivement : les protéines codées par l'ORF1a (c/t en position 7919 correspondant à la mutation A/V), la protéine S (g/t en position 23220 correspondant à la mutation A/S), la protéine codée par l'ORF3 (a/g en position 25298 correspondant à la mutation R/G) et de la protéine M (t/c en position 26857 correspondant à la mutation S/P).

En outre, l'analyse phylogénétique montre que le SARS-CoV est éloigné des autres coronavirus et qu'il est apparu, ni par mutation de coronavirus respiratoires humains, ni par recombinaison entre des coronavirus connus (pour une revue, voir Holmes, J.C.I., 2003, 111, 1605-1609).

La mise en évidence et la prise en compte de nouveaux variants sont importantes pour la mise au point de réactifs de détection et de diagnostic du SRAS suffisamment sensibles et spécifiques ainsi qu'à des compositions immunogènes aptes à protéger des populations contre des épidémies de SRAS.

Les Inventeurs ont maintenant mis en évidence une autre souche de coronavirus associé au SRAS, qui se distingue des isolats Tor2 et Urbani.

La présente invention a donc pour objet, une souche isolée ou purifiée de coronavirus humain associé au syndrome respiratoire aigu sévère, **caractérisée en ce que** son génome présente sous la forme d'ADN complémentaire un codon sérine en position 23220-23222 du gène de la protéine S ou un codon glycine en position 25298-25300 du gène de l'ORF3, et un codon alanine en position 7918-7920 de l'ORF1a ou un codon sérine en position 26857-26859 du gène de la protéine M, lesdites positions étant indiquées en référence à la séquence Genbank AY274119.3.

Selon un mode de réalisation avantageux de ladite souche, l'équivalent ADN de son génome présente une séquence correspondant à la séquence SEQ ID NO : 1 ; cette souche de coronavirus est issue du prélèvement de lavage bronchoalvéolaire d'un patient atteint de SRAS, répertorié sous le n° 031589 et effectué à l'hôpital français de Hanoi (Vietnam).

Conformément à l'invention, ladite séquence SEQ ID NO :1 est celle de l'acide désoxyribonucléique correspondant à la molécule d'acide ribonucléique du génome de la souche isolée de coronavirus telle que définie ci-dessus.

La séquence SEQ ID NO : 1 se distingue de la séquence Genbank AY274119.3 (isolat Tor2) en ce qu'elle possède les mutations suivantes :
- g/t en position 23220 ; le codon alanine (gct) en position 577 de la séquence en acides aminés de la protéine S de Tor2 est remplacé par un codon sérine (tct),
- a/g en position 25298 ; le codon arginine (aga) en position 11 de la séquence en acide aminés de la protéine codée par l'ORF3 de Tor 2 est remplacé par un codon glycine (gga).

En outre, la séquence SEQ ID NO : 1 se distingue de la séquence Genbank AY278741 (isolat Urbani) en ce qu'elle possède les mutations suivantes :
- t/c en position 7919 ; le codon valine (gtt) en position 2552 de la séquence en acides aminés de la protéine codée par l'ORF1a est remplacé par un codon alanine (gct),
- t/c en position 16622 : cette mutation ne modifie pas la séquence en acides aminés des protéines codées par l'ORF1b (mutation silencieuse),
- g/a en position 19064 : cette mutation ne modifie pas la séquence en acides aminés des protéines codées par l'ORF1b (mutation silencieuse),
- c/t en position 24872 : cette mutation ne modifie pas la séquence en acides aminés de la protéine S, et
- c/t en position 26857 : le codon proline (ccc) en position 154 de la séquence en acides aminés de la protéine M est remplacé par un codon sérine (tcc).

En l'absence de mention particulière, les positions des séquences nucléotidiques et peptidiques sont indiquées en référence à la séquence Genbank AY274119.3.

La présente invention a également pour objet un polynucléotide isolé ou purifié, **caractérisé en ce que** sa séquence est celle du génome de la souche isolée de coronavirus telle que définie ci-dessus.

Selon un mode de réalisation avantageux dudit polynucléotide il présente la séquence SEQ ID NO : 1.

La présente invention a également pour objet un polynucléotide isolé ou purifié, **caractérisé en ce que** sa séquence hybride dans des conditions de forte stringence avec la séquence du polynucléotide tel que défini ci-dessus.

Les termes « isolé ou purifié » signifient modifié « par la main de l'homme » à partir de l'état naturel ; autrement dit si un objet existe dans la nature, il est dit isolé ou purifié s'il a été modifié ou extrait de son environnement naturel ou les deux. Par exemple, un polynucléotide ou une protéine/un peptide naturellement présent dans un organisme vivant n'est ni isolé, ni purifié ; en revanche le même polynucléotide ou protéine /peptide séparé des molécules coexistantes dans son environnement naturel, obtenu par clonage, amplification et/ou synthèse chimique est isolé au sens de la présente invention. De plus, un polynucléotide ou une protéine/peptide qui est introduit dans un organisme par transformation, manipulation génétique ou par toute autre méthode, est « isolé » même s'il est présent dans ledit organisme. Le terme purifié tel qu'utilisé dans la présente invention, signifie que les protéines /peptides selon l'invention sont essentiellement libres d'association avec les autres protéines ou polypeptides, comme l'est par exemple le produit purifié de la culture de cellules hôtes recombinantes ou le produit purifié à partir d'une source non-recombinante.

Au sens de la présente invention, on entend par conditions d'hybridation de forte stringence, des conditions de température et de force ionique choisies de telle manière qu'elles permettent le maintien de l'hybridation spécifique et sélective entre polynucléotides complémentaires.

A titre d'illustration, des conditions de forte stringence aux fins de définir les polynucléotides ci-dessus, sont avantageusement les suivantes : l'hybridation ADN-ADN ou ADN-ARN est réalisée en deux étapes : (1) préhybridation à 42°C pendant 3 heures en tampon phosphate (20 mM pH 7,5) contenant 5 x SSC (1 x SSC correspond à une solution 0,15 M NaCl + 0, 015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation pendant 20 heures à 42°C suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C.

La présente invention a également pour objet un fragment représentatif du polynucléotide tel que défini ci-dessus, **caractérisé en ce qu**'il est susceptible d'être obtenu, soit par l'utilisation d'enzymes de restriction dont les sites de reconnaissance et de coupure sont présents dans ledit polynucléotide tel que défini ci-dessus, soit par amplification à l'aide d'amorces oligonucléotidiques spécifiques dudit polynucléotide tel que défini ci-dessus, soit par transcription *in vitro,* soit par synthèse chimique.

Selon un mode de réalisation avantageux dudit fragment, il est sélectionné dans le groupe constitué par : l'ADNc correspondant à au moins un cadre ouvert de lecture (ORF) choisi parmi : ORF1a, ORF1b, ORF-S, ORF-E, ORF-M, ORF-N, ORF3, ORF4, ORF7 à ORF11, ORF 13 et ORF 14, et l'ADNc correspondant aux extrémités 5' ou 3' non-codantes dudit polynucléotide.

Selon une disposition avantageuse de ce mode de réalisation, ledit fragment présente une séquence sélectionnée dans le groupe constitué par :
- les séquences SEQ ID NO : 2 et 4 représentant l'ADNc correspondant à l'ORF-S qui code pour la protéine S,
- les séquences SEQ ID NO : 13 et 15 représentant l'ADNc correspondant à l'ORF-E qui code pour la protéine E,
- les séquences séquence SEQ ID NO: 16 et 18 représentant l'ADNc correspondant à l'ORF-M qui code pour la protéine M,
- les séquences SEQ ID NO : 36 et 38 représentant l'ADNc correspondant à l'ORF-N qui code pour la protéine N,
- les séquences représentant les ADNc correspondant respectivement : aux ORF1a et ORF1b (ORF1ab, SEQ ID NO : 31), aux ORF3 et ORF4 (SEQ ID NO : 7, 8), aux ORF 7 à 11 (SEQ ID NO : 19, 20), à l'ORF13 (SEQ ID NO : 32) et à l'ORF14 (SEQ ID NO : 34), et
- les séquences représentant les ADNc correspondant respectivement aux extrémités 5'(SEQ ID NO : 39 et 72) et 3' non-codantes (SEQ ID NO : 40, 73) dudit polynucléotide.

La présente invention a également pour objet un fragment de l'ADNc codant pour la protéine S, tel que défini ci-dessus, **caractérisé en ce qu**'il présente une séquence sélectionnée dans le groupe constitué par les séquences SEQ ID NO : 5 et 6 (fragments Sa et Sb).

La présente invention a également pour objet un fragment de l'ADNc correspondant aux ORF1a et ORF1b tel que défini ci-dessus, **caractérisé en ce qu**'il présente une séquence sélectionnée dans le groupe constitué par les séquences SEQ ID NO : 41 à 54 (fragments L0 à L12).

La présente invention a également pour objet un fragment du polynucléotide tel que défini ci dessus, caractérisé en ce qu'il présente au moins 15 bases ou paires de bases consécutives de la séquence du génome de ladite souche incluant au moins une de celles situées en position 7979, 16622, 19064, 23220, 24872, 25298 et 26857. De préférence, il s'agit d'un fragment de 20 à 2500 bases ou paires de bases, de manière préférée de 20 à 400.

Selon un mode de réalisation avantageux dudit fragment, il inclut au moins un couple de bases ou de paires de bases correspondant aux positions suivantes : 7919 et 23220, 7919 et 25298, 16622 et 23220, 19064 et 23220, 16622 et 25298, 19064 et 25298, 23220 et 24872, 23220 et 26857, 24872 et 25298, 25298 et 26857.

La présente invention a également pour objet des amorces d'au moins 18 bases aptes à amplifier un fragment du génome d'un coronavirus associé au SRAS ou de l'équivalent ADN de celui-ci.

Selon un mode de réalisation desdites amorces, elles sont sélectionnées dans le groupe constitué par :
- la paire d'amorces n° 1 correspondant respectivement aux positions 28507 à 28522 (amorce sens, SEQ ID NO : 60) et 28774 à 28759 (amorce anti-sens, SEQ ID NO : 61) de la séquence du polynucléotide tel que défini ci-dessus,
- la paire d'amorces n° 2 correspondant respectivement aux positions 28375 à 28390 (amorce sens, SEQ ID NO : 62) et 28702 à 28687 (amorce anti-sens, SEQ ID NO : 63) de la séquence du polynucléotide tel que défini ci-dessus., et
- la paire d'amorces constituée des amorces SEQ ID Nos 55 et 56.

La présente invention a également pour objet une sonde apte à détecter la présence du génome d'un coronavirus associé au SRAS ou d'un fragment de celui-ci, **caractérisée en ce qu**'elle est sélectionnée dans le groupe constitué par : les fragments tels que définis ci-dessus et les fragments correspondant aux positions suivantes de la séquence du polynucléotide tel que défini ci-dessus : 28561 à 28586, 28588 à 28608, 28541 à 28563 et 28565 à 28589 (SEQ ID NO : 64 à 67).

Les sondes et amorces selon l'Invention peuvent être marquées directement ou indirectement par un composé radioactif ou non radioactif par des méthodes bien connues de l'Homme du Métier, afin d'obtenir un signal détectable et/ou quantifiable. Parmi les isotopes radioactifs utilisés, on peut citer le ³²P, le ³³P, le ³⁵S, le ³H ou l'¹²⁵I. Les entités non radioactives sont sélectionnées parmi les ligands tels que la biotine, l'avidine, la streptavidine, la digoxygénine, les haptènes, les colorants, les agents luminescents tels que les agents radioluminescents, chémoluminescents, bioluminescents, fluorescents, phosphorescents.

L'invention englobe les sondes et les amorces marquées dérivées des séquences précédentes.

De telles sondes et amorces sont utiles pour le diagnostic de l'infection par un coronavirus associé au SRAS.

La présente invention a également pour objet une méthode de détection d'un coronavirus associé au SRAS, à partir d'un échantillon biologique, laquelle méthode est **caractérisée en ce qu**'elle comprend au moins :
(a) l'extraction d'acides nucléiques présents dans ledit échantillon biologique,
(b) l'amplification d'un fragment de l'ORF-N par RT-PCR à l'aide d'une paire d'amorces telle que définie ci-dessus, et
(c) la détection par tout moyen approprié des produits d'amplifications obtenus en (b).

Les produits d'amplifications (amplicons) en (b) sont de 268 pb pour la paire d'amorces n° 1 et de 328 pb pour la paire d'amorces n°2.

Selon un mode de mise en oeuvre avantageux dudit procédé, l'étape (b) de détection est réalisée à l'aide d'au moins une sonde correspondant aux positions 28561 à 28586, 28588 à 28608, 28541 à 28563 et 28565 à 28589 de la séquence du polynucléotide tel que défini ci-dessus.

De préférence, le génome du coronavirus associé au SRAS est détecté et éventuellement quantifié par PCR en temps réel, à l'aide de la paire d'amorces n°2 et des sondes correspondant aux positions 28541 à 28563 et 28565 à 28589 marquées avec des composés différents, notamment des agents fluorescents différents.

La RT-PCR en temps réel qui met en oeuvre cette paire d'amorces et cette sonde est très sensible puisqu'elle permet de détecter 10² copies d'ARN et jusqu'à 10 copies d'ARN, elle est en outre fiable et reproductible.

L'invention englobe les polydésoxyribonucléotides et les polyribonucléotides simple-brin, double-brin et tripe-brin correspondant à la séquence du génome de la souche isolée de coronavirus et de ses fragments tels que définis ci-dessus, ainsi qu'à leurs séquences complémentaires, sens ou anti-sens, notamment les ARN et les ADNc correspondant à la séquence du génome et de ses fragments tels que définis ci-dessus.

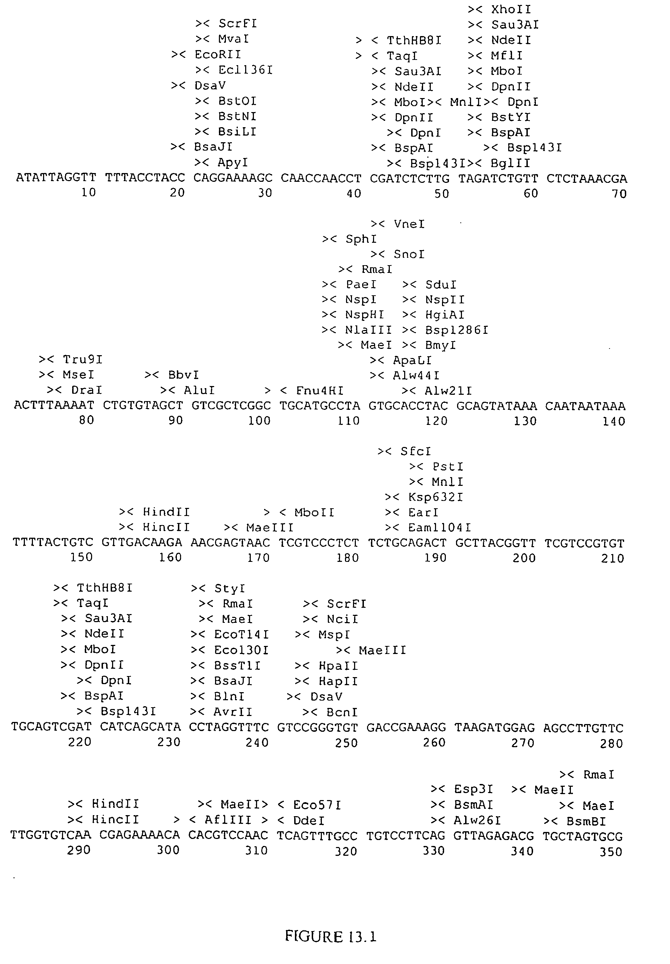

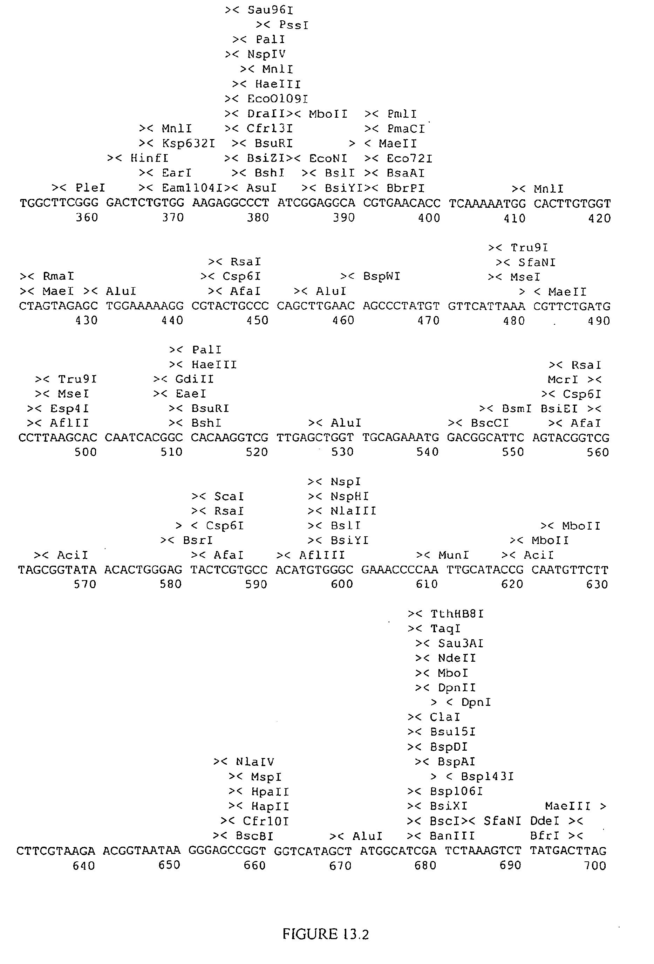

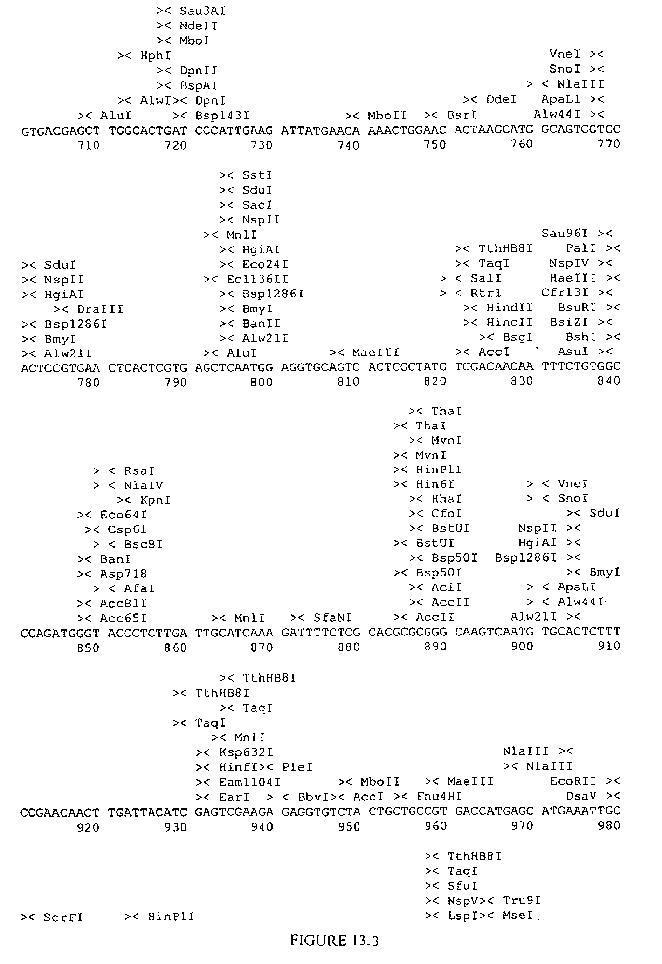

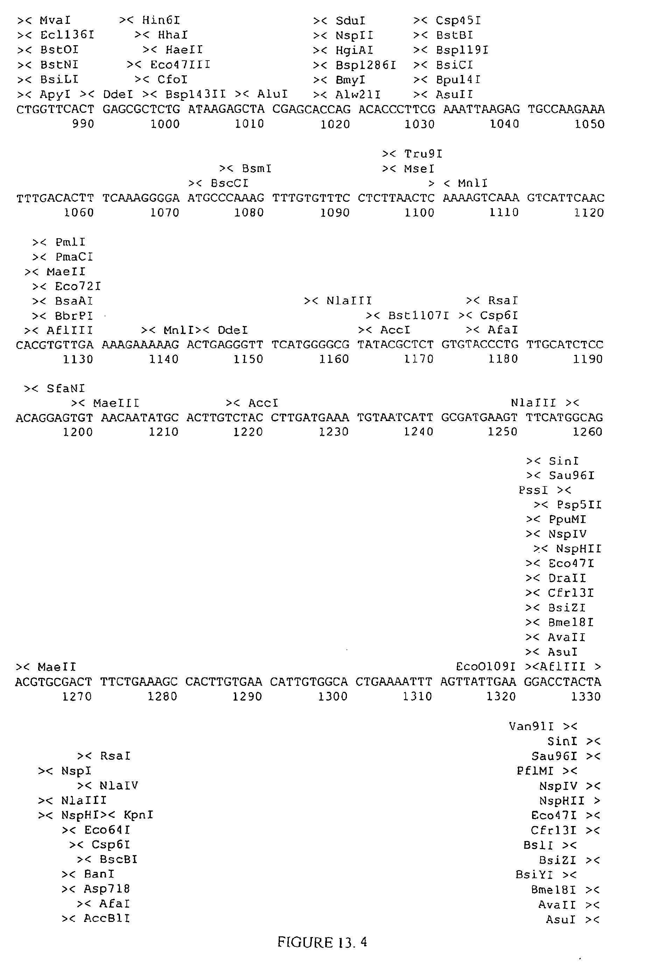

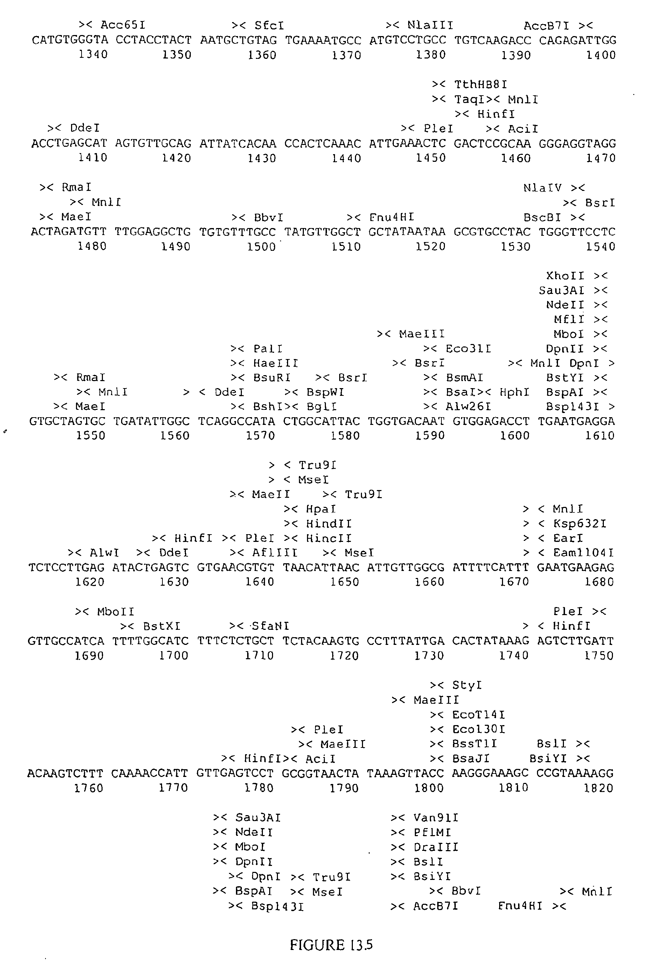

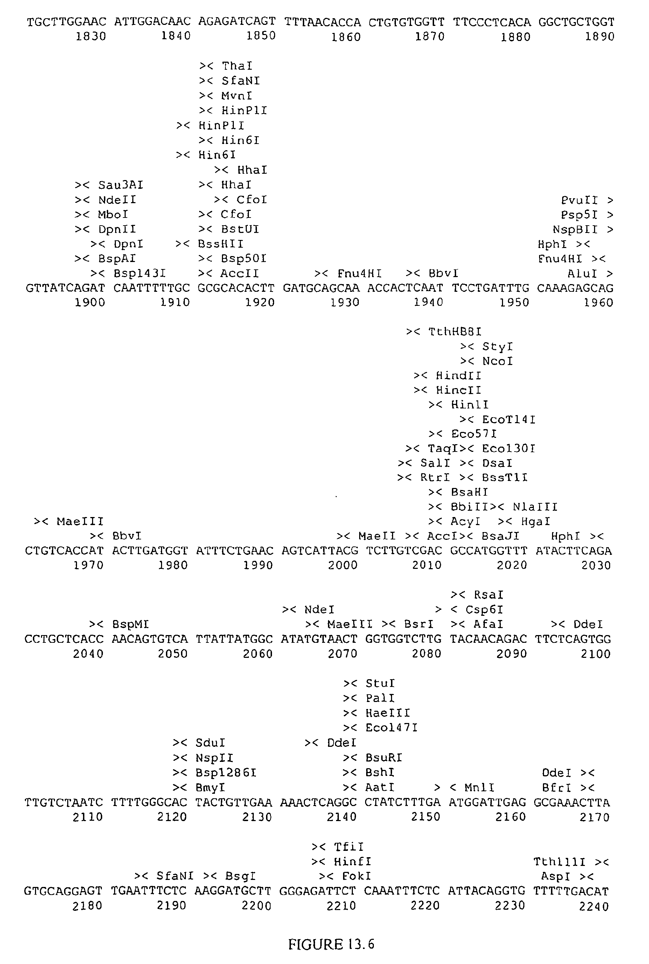

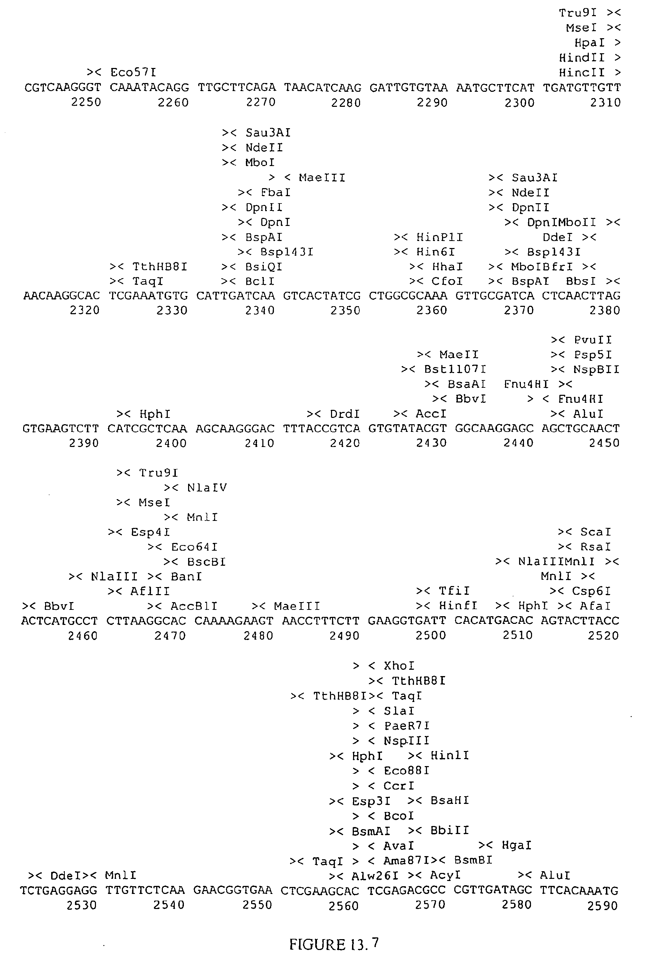

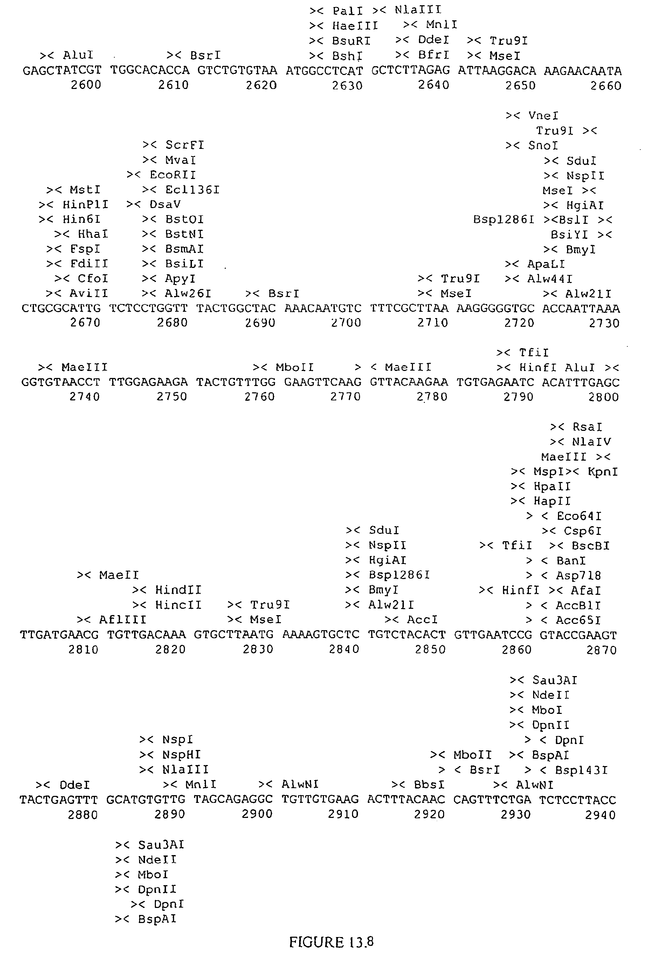

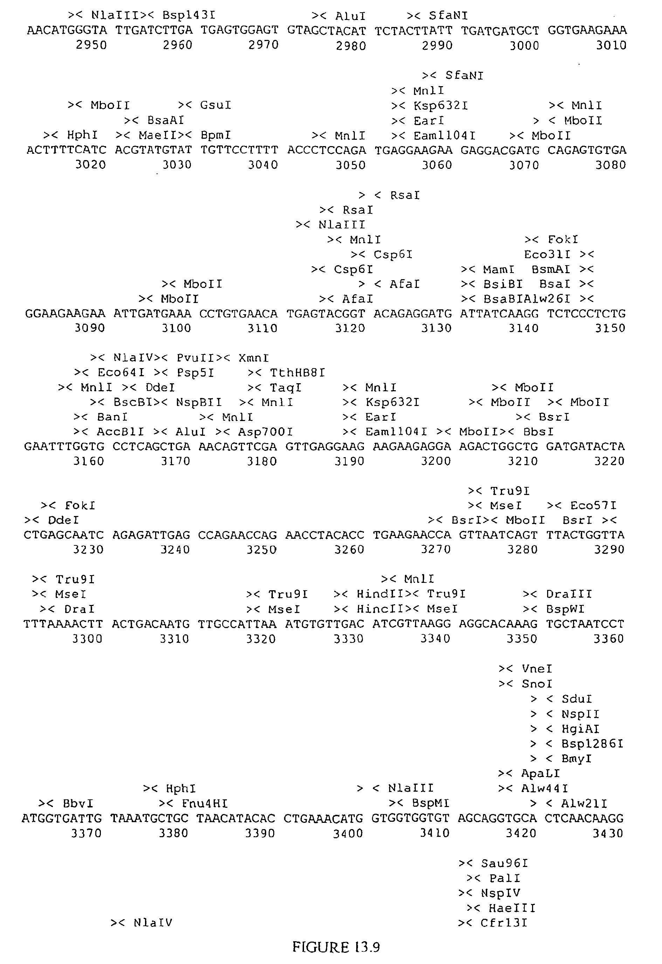

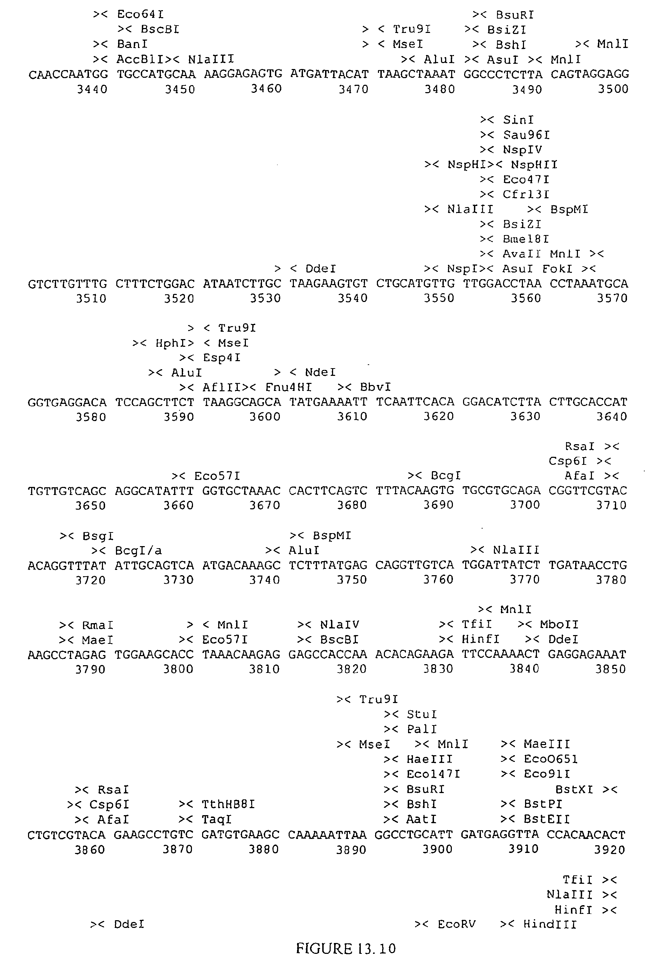

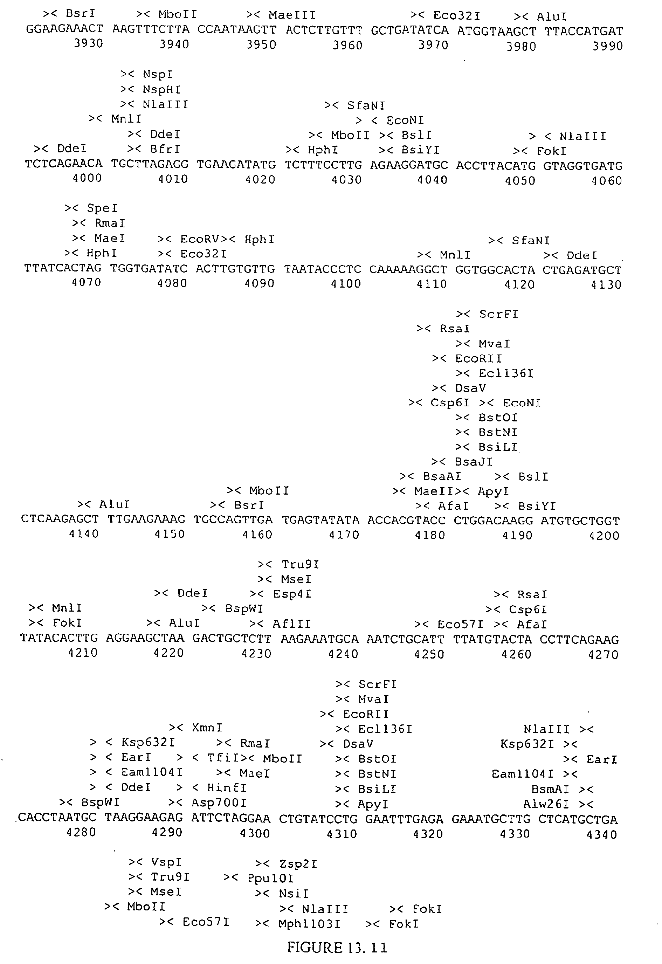

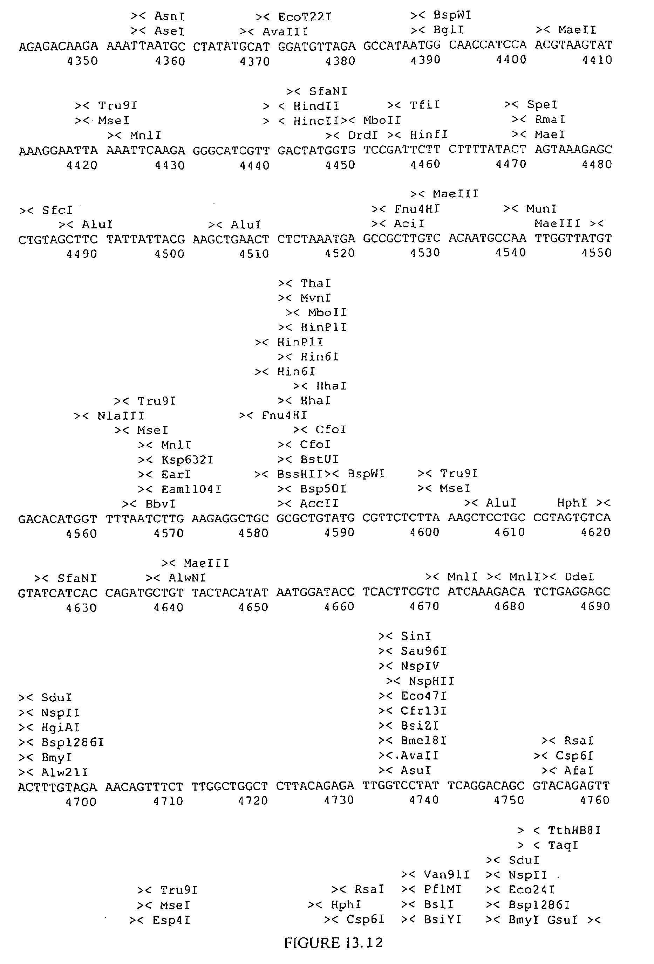

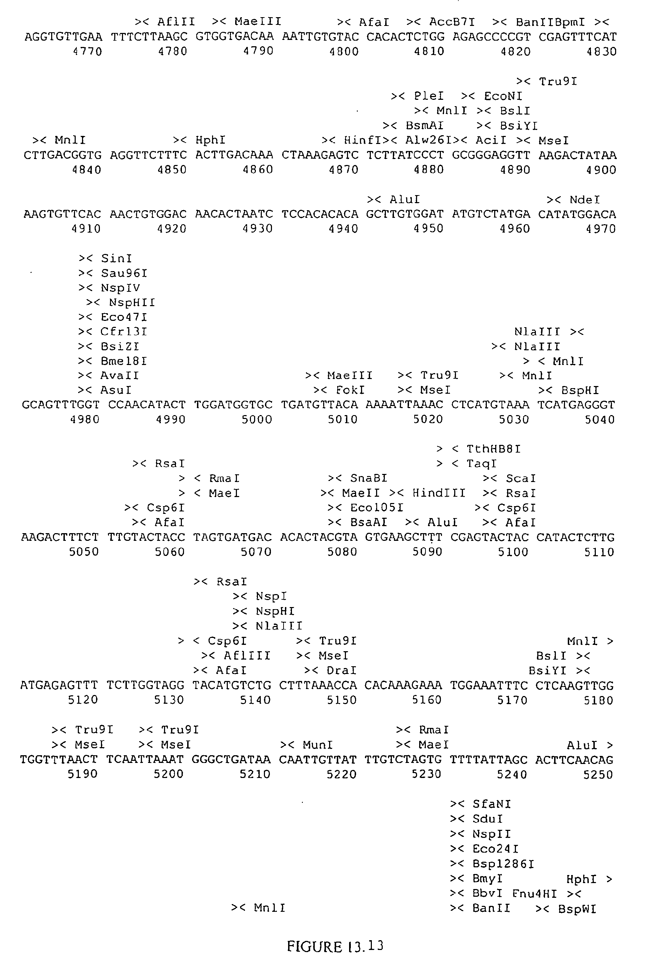

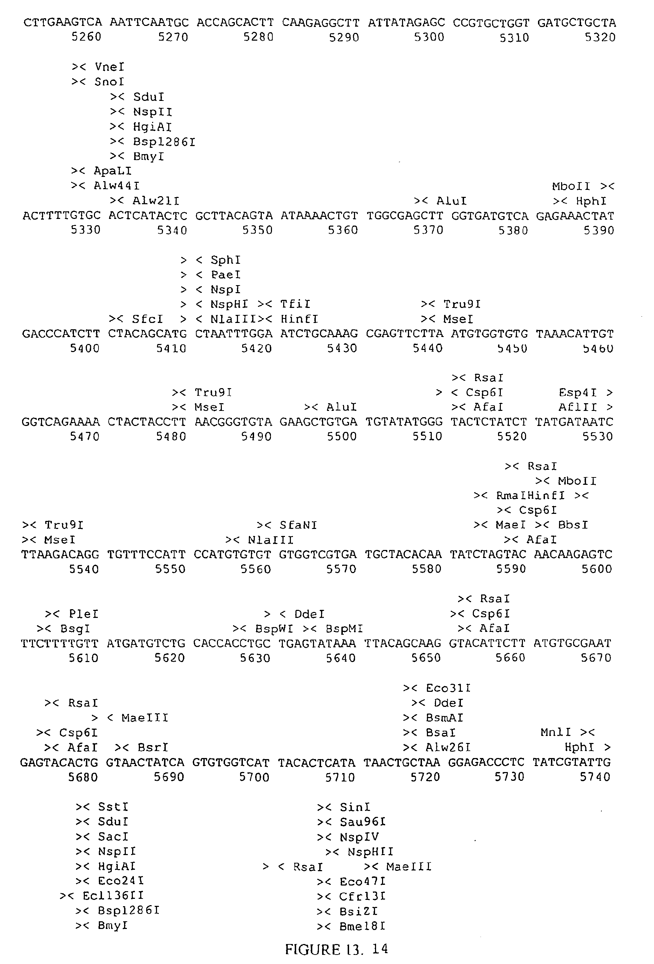

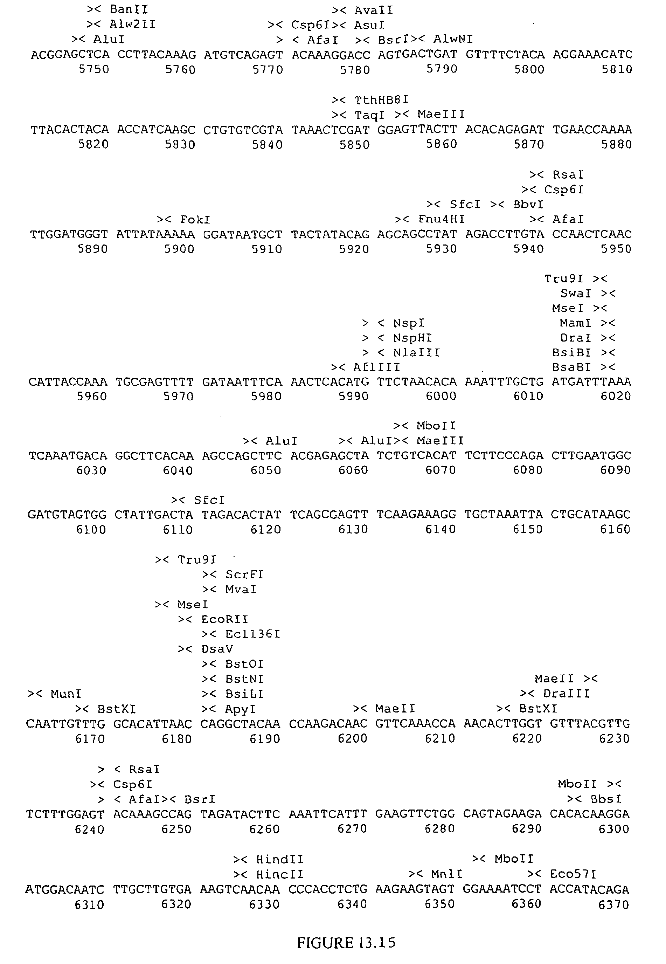

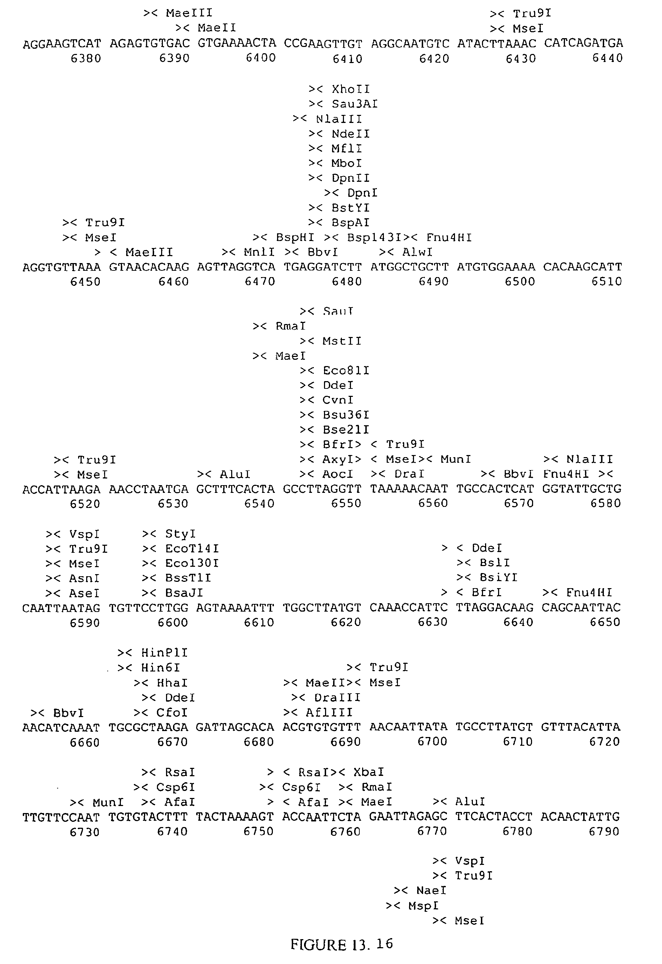

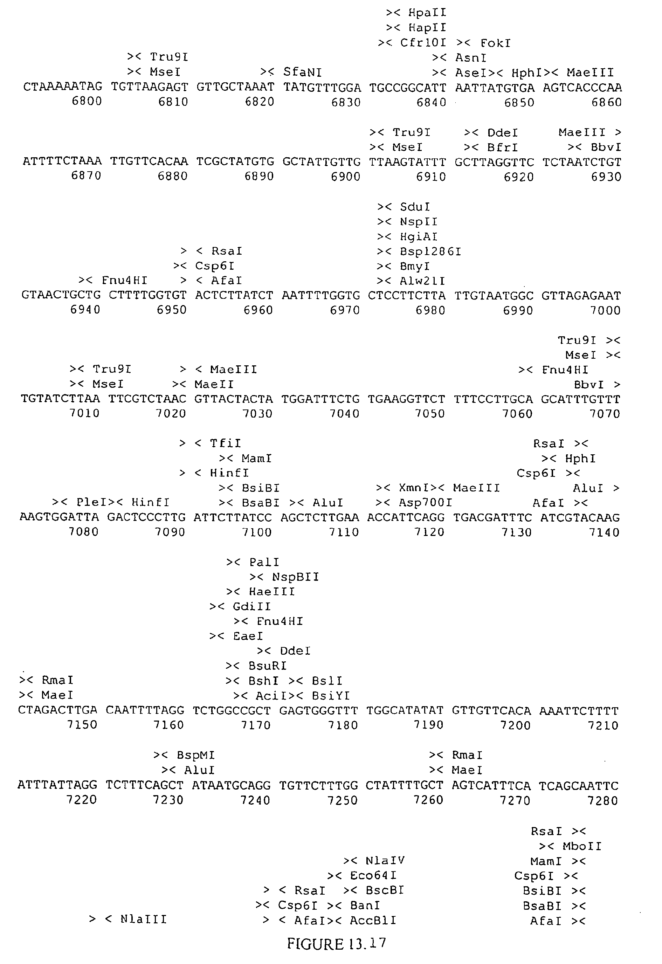

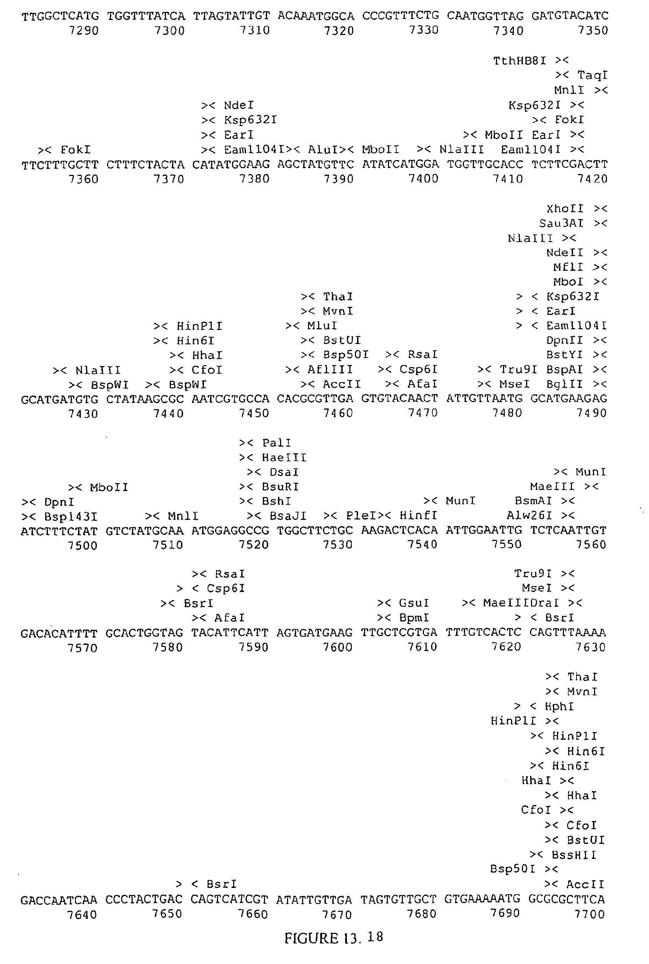

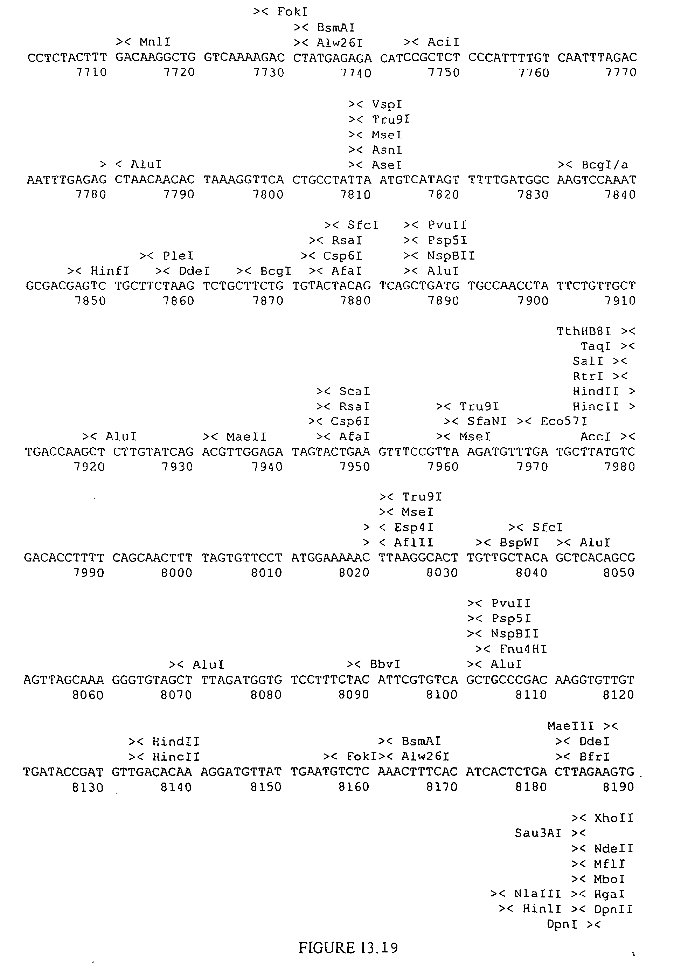

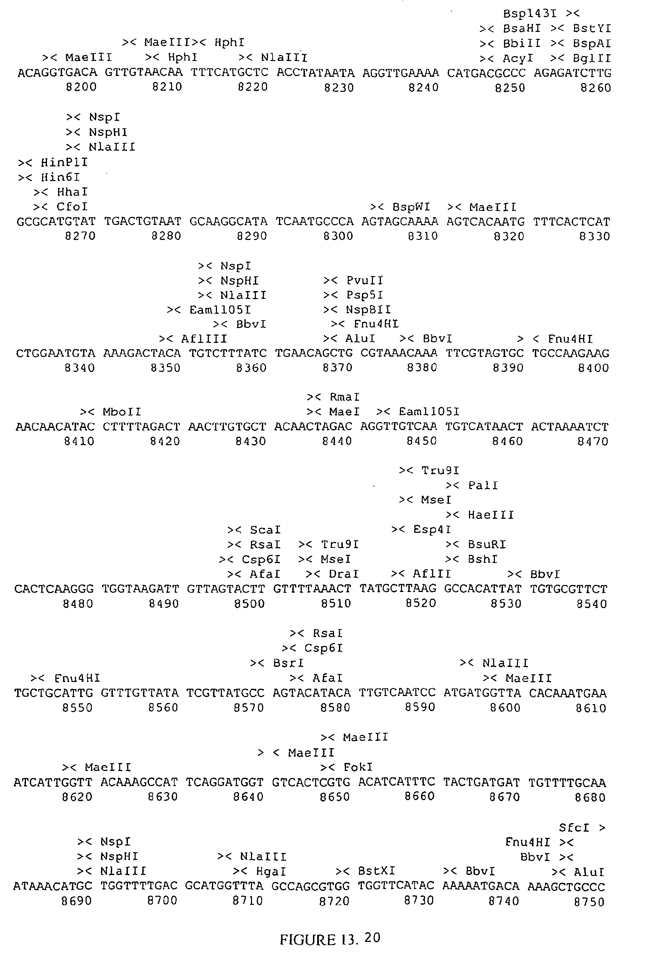

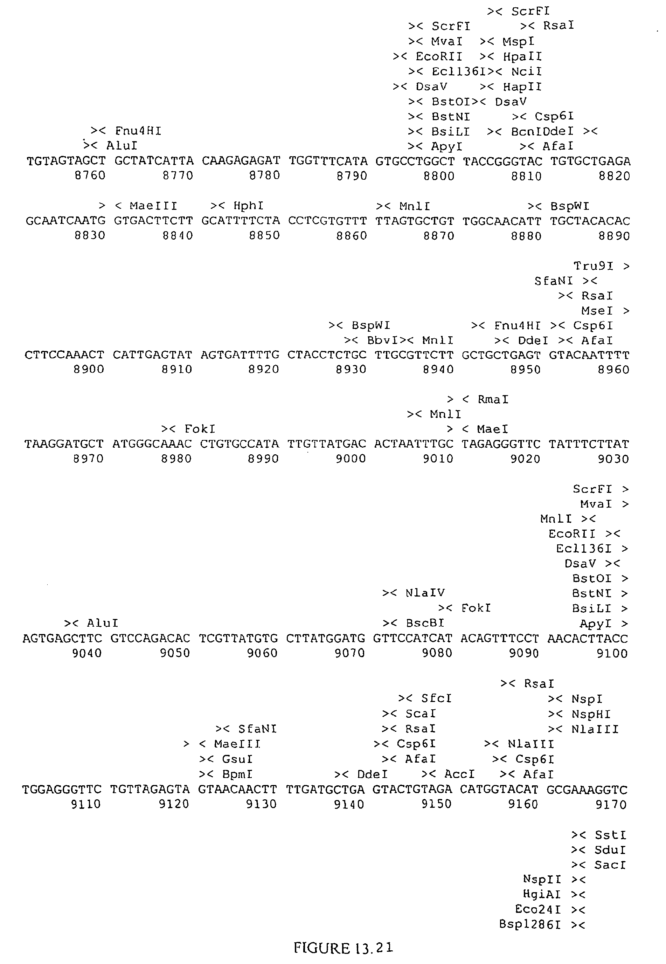

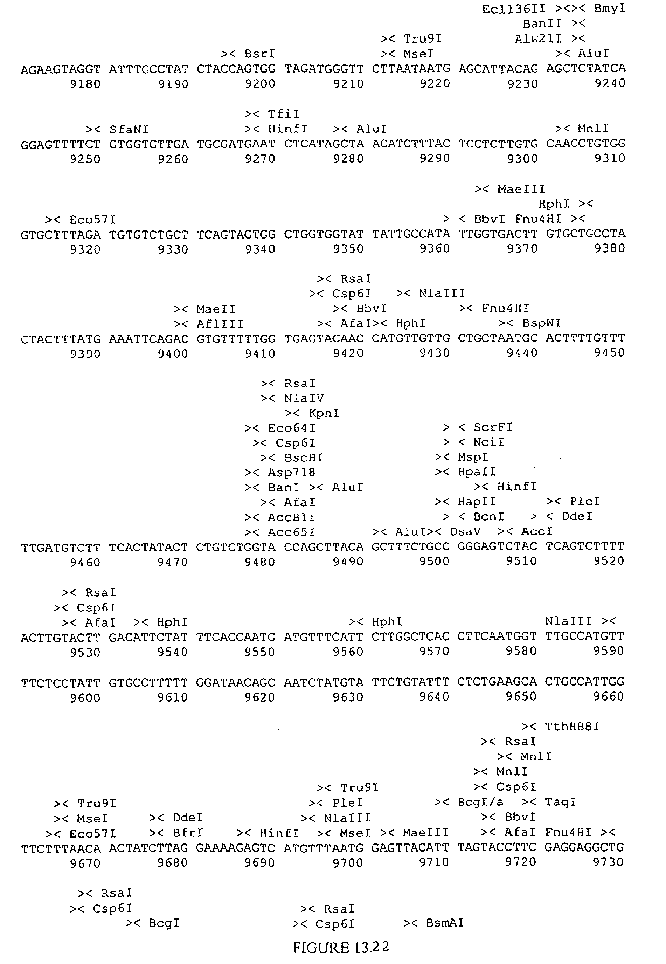

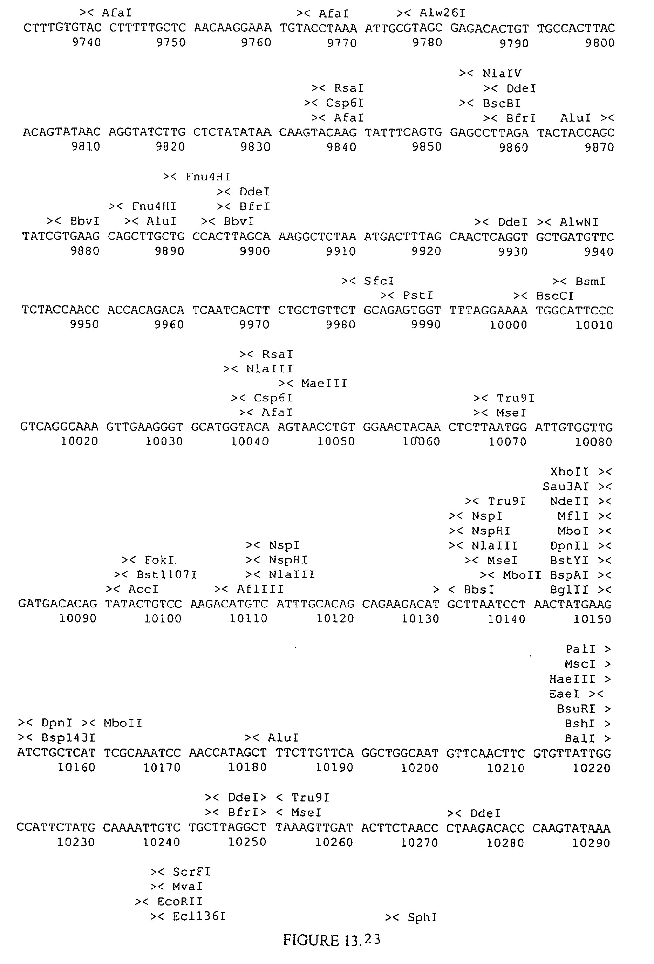

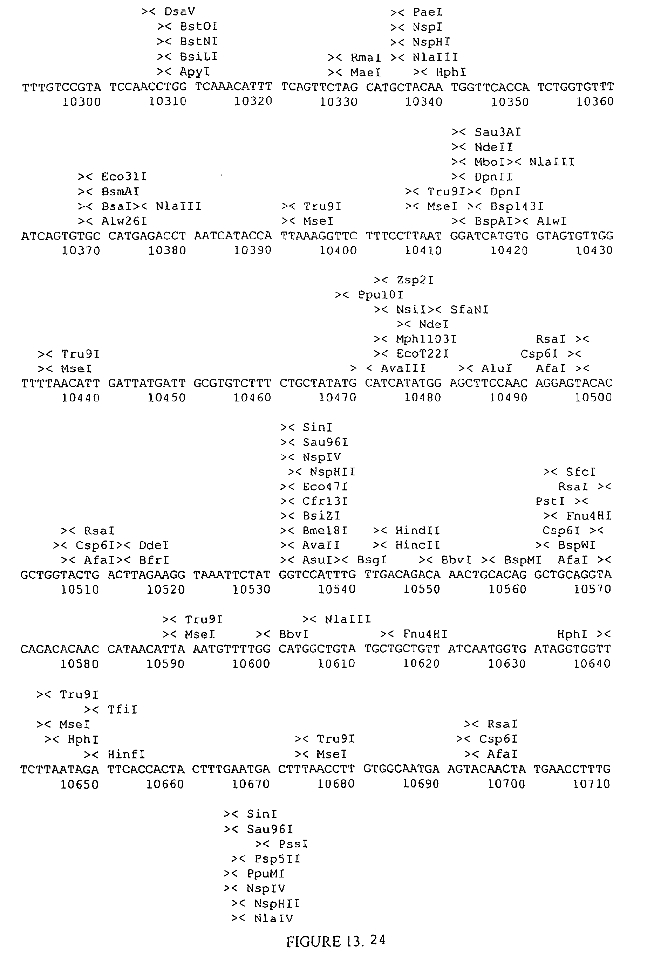

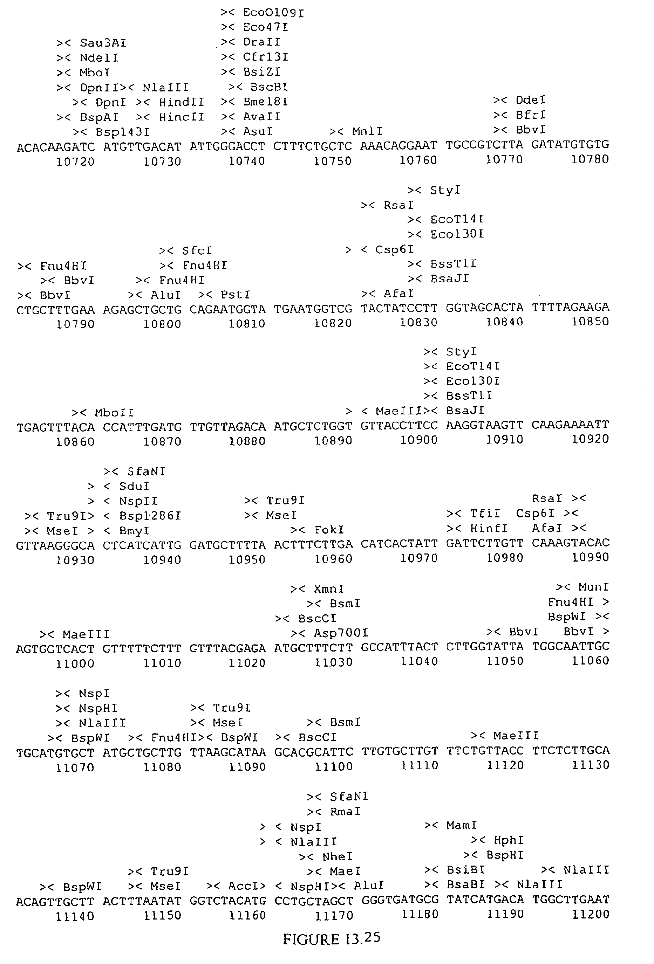

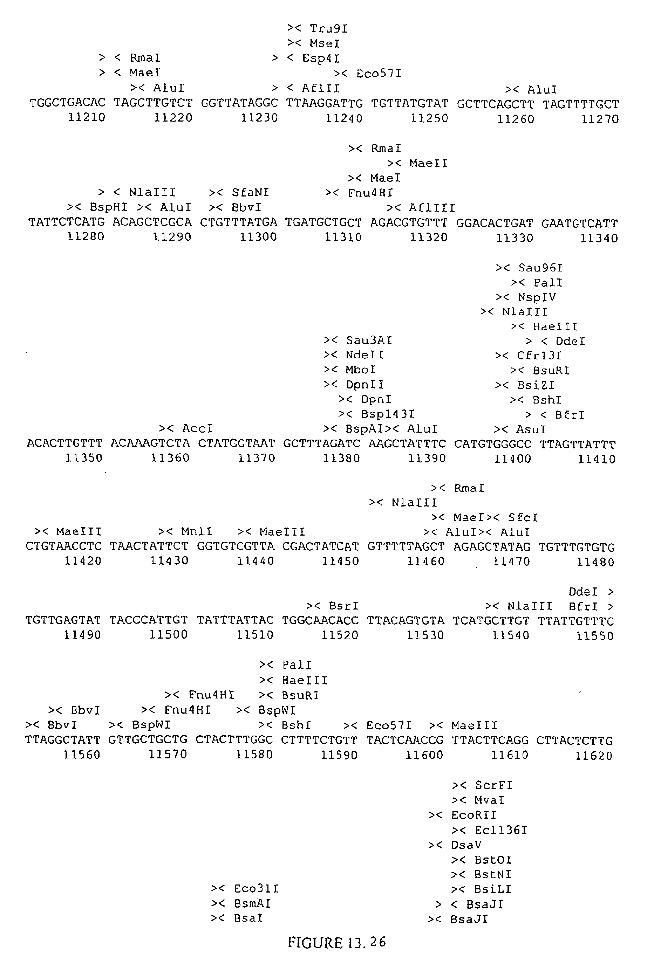

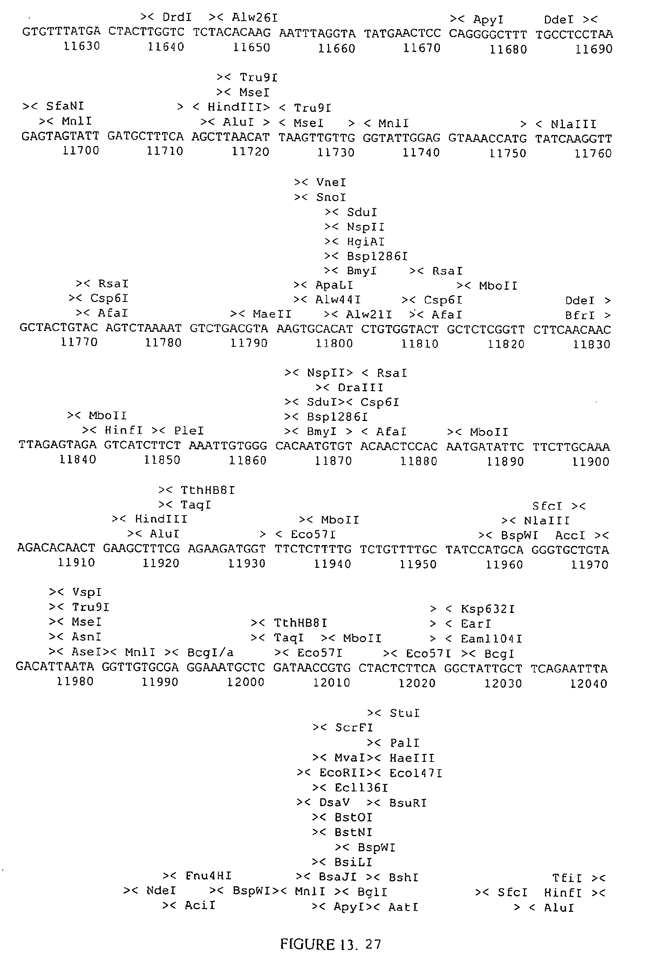

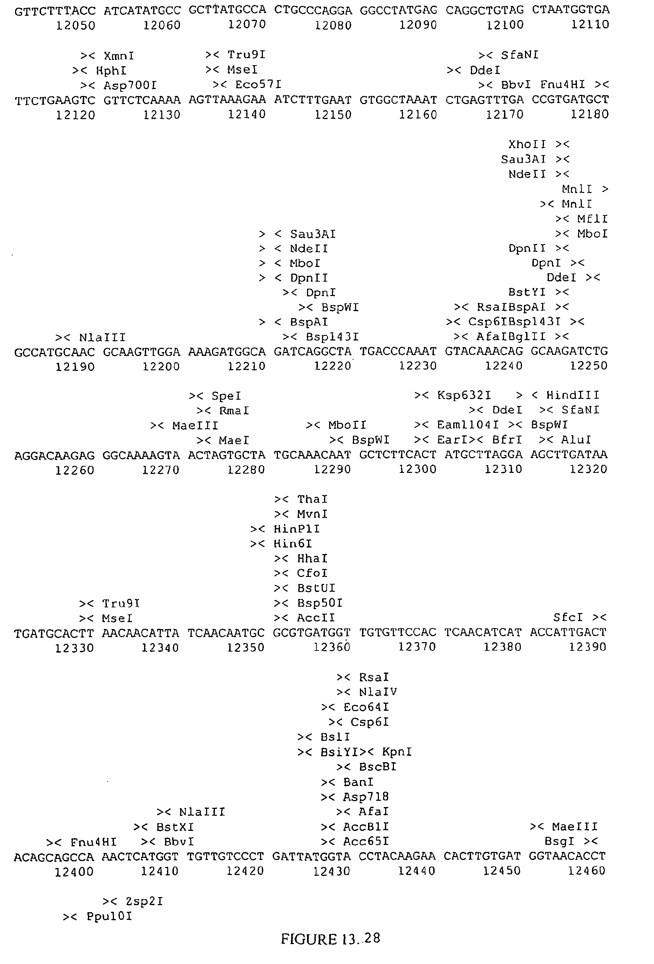

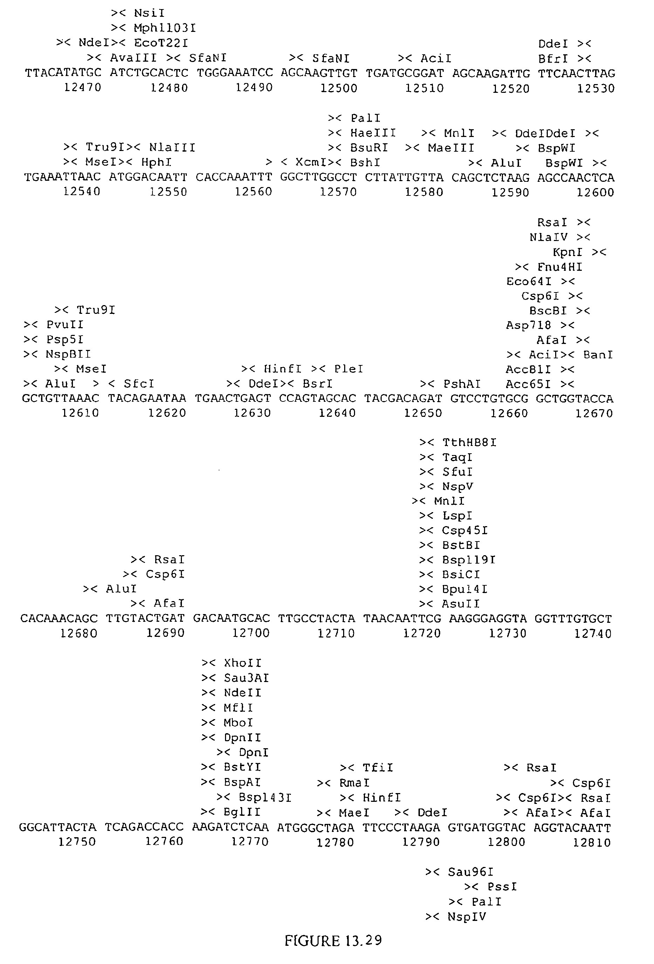

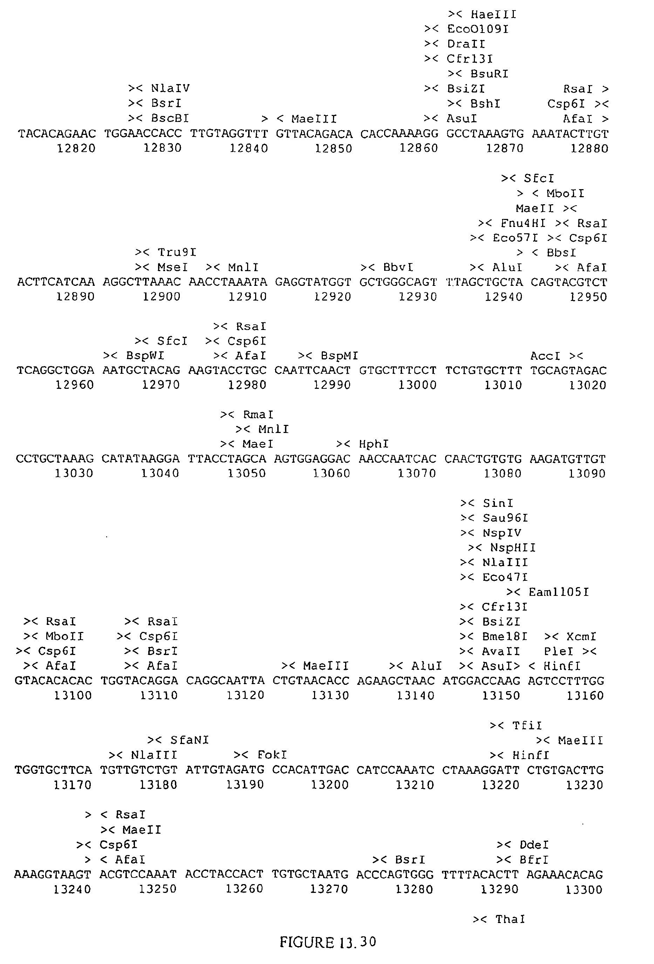

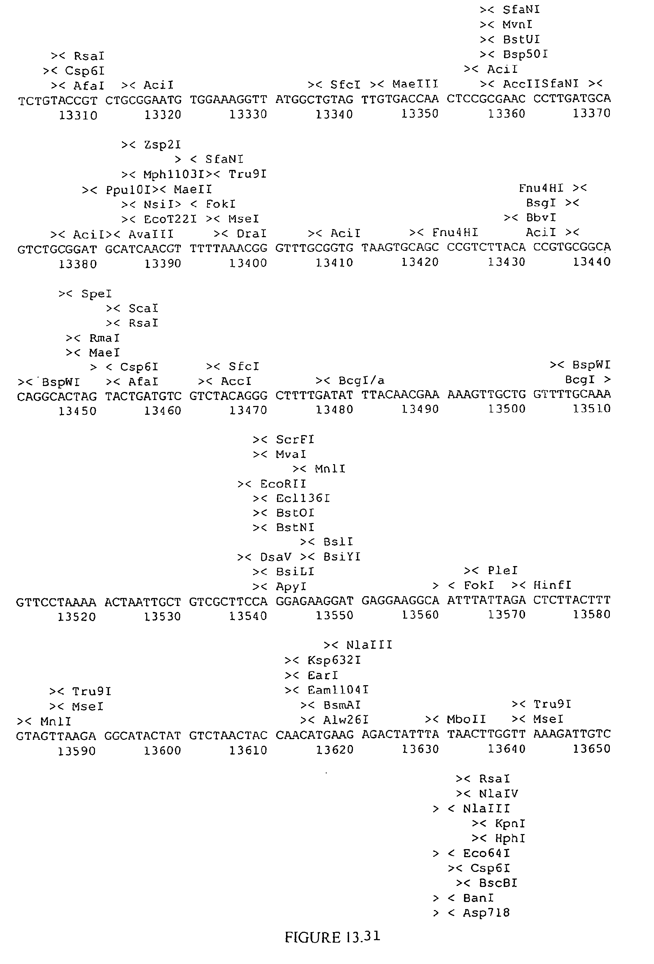

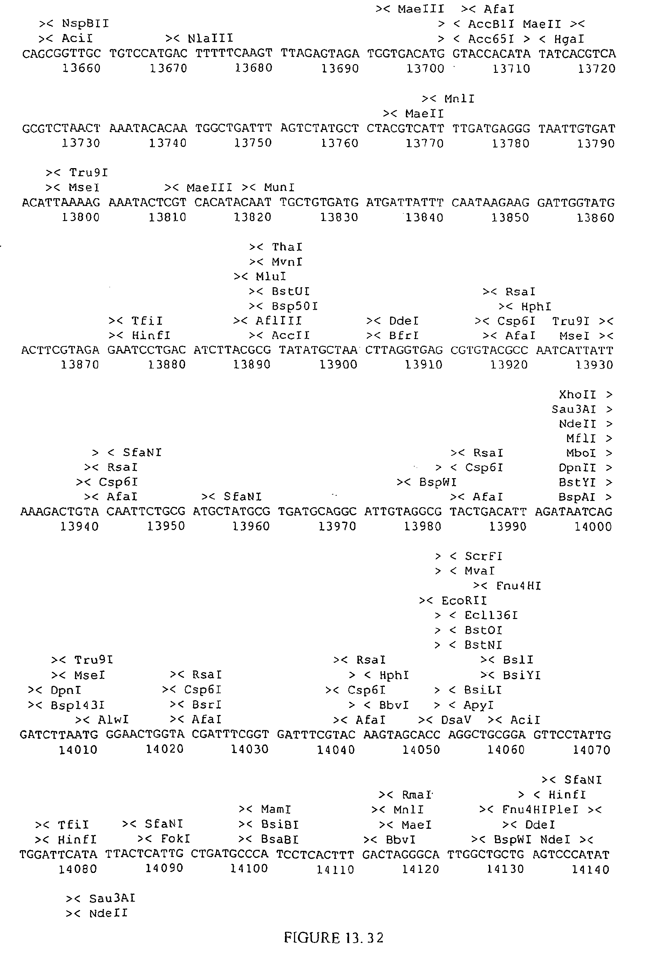

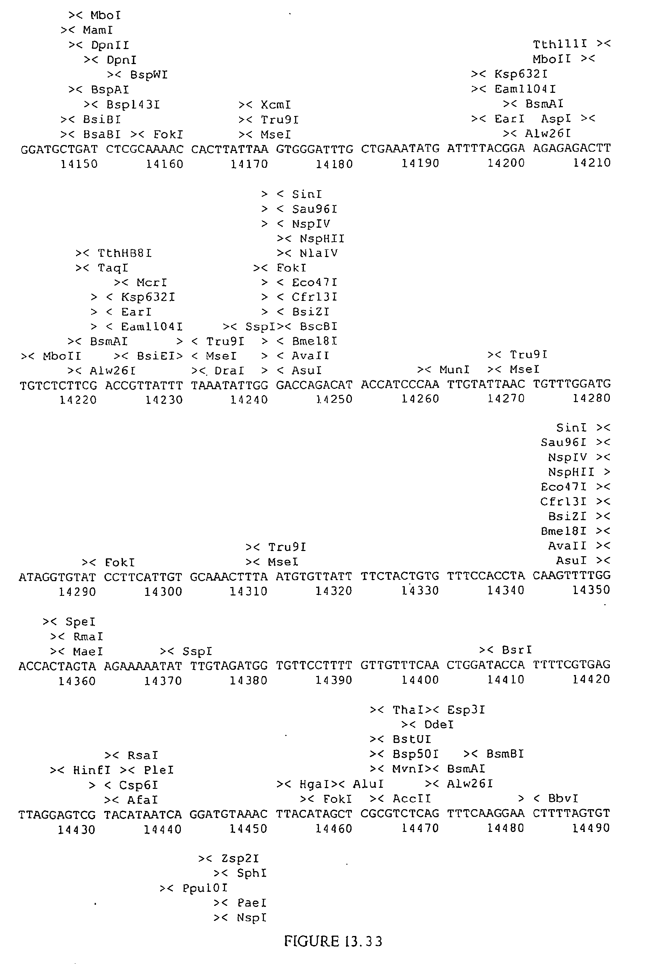

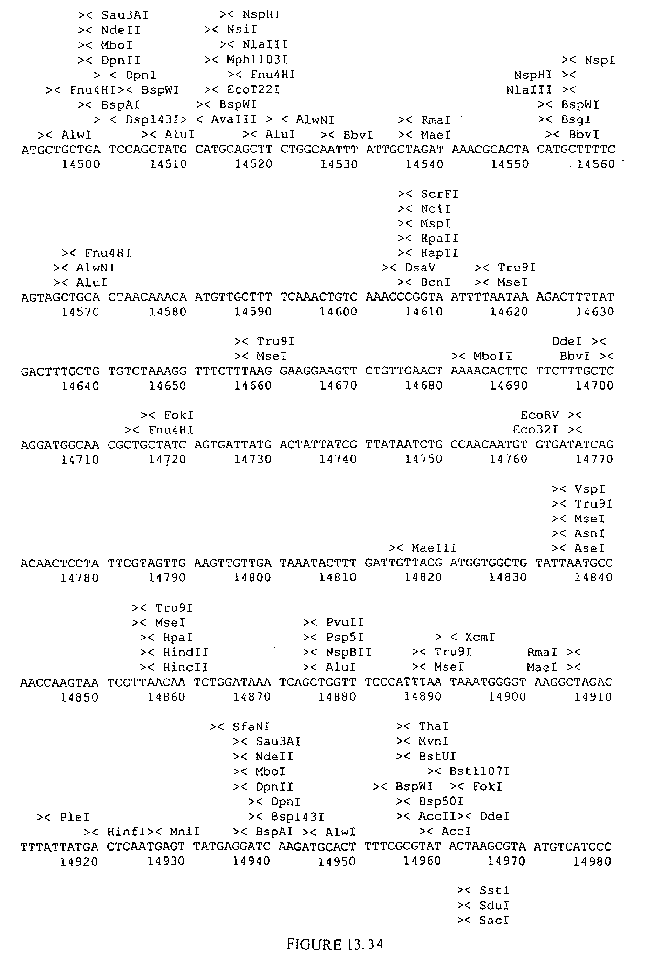

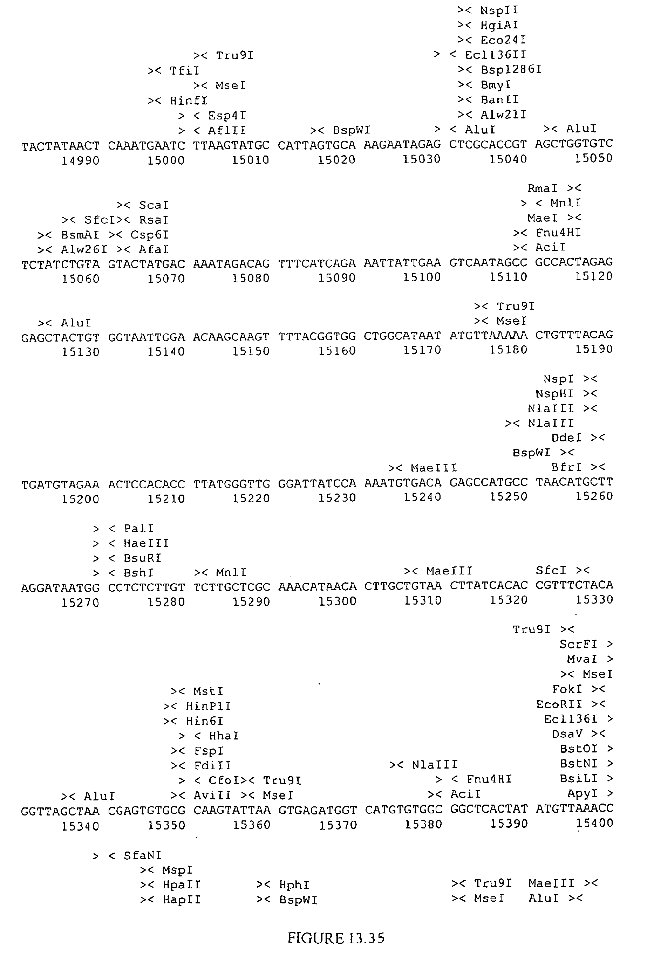

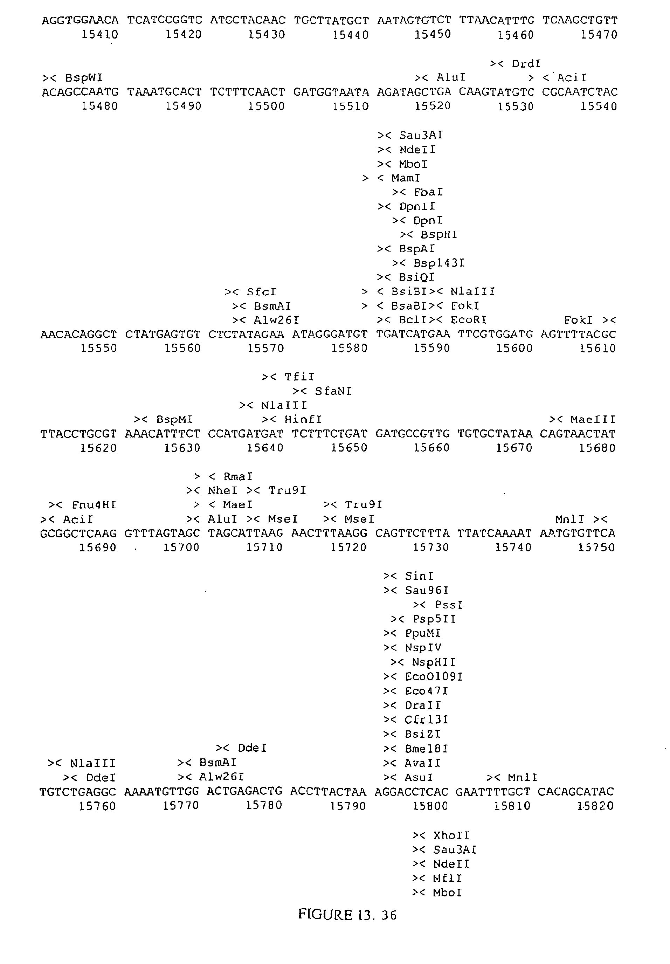

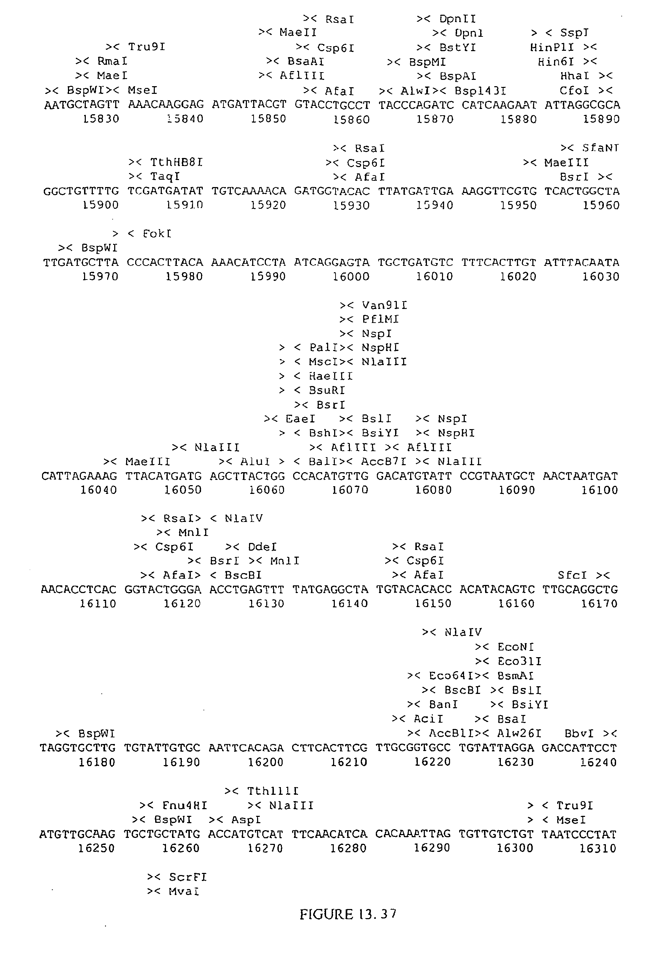

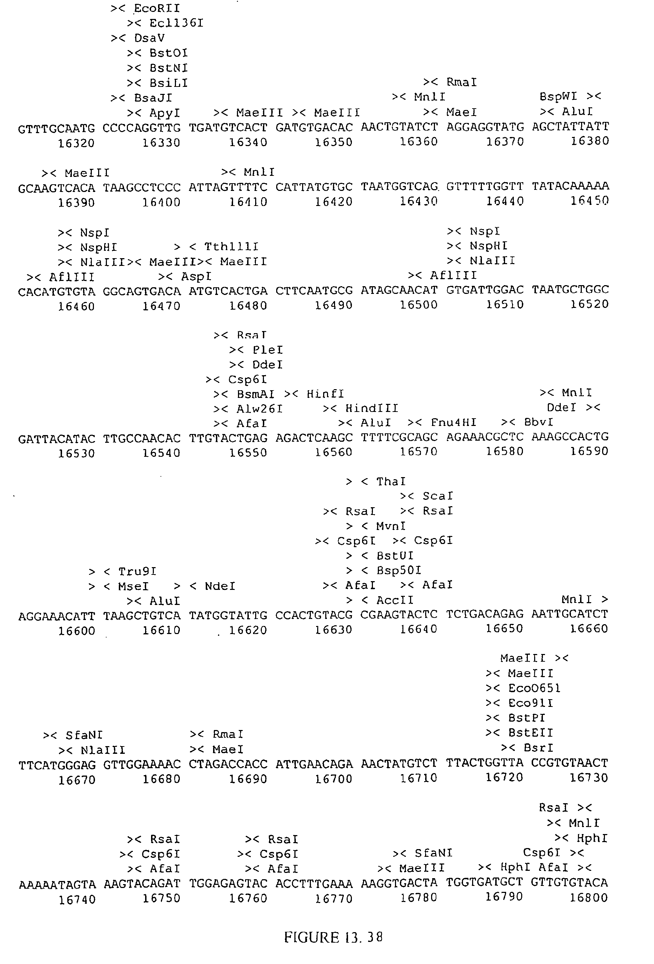

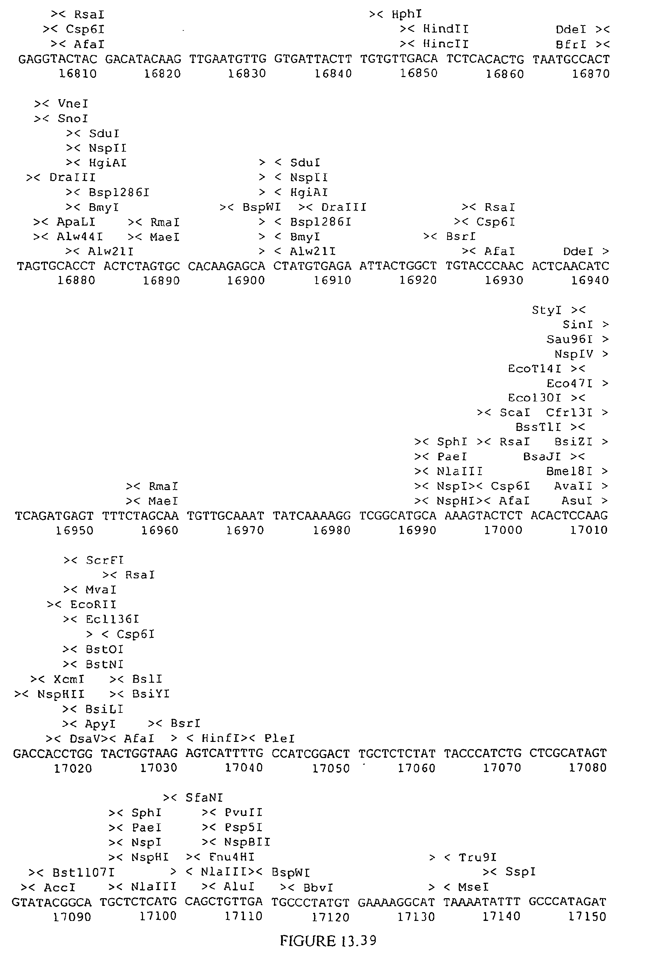

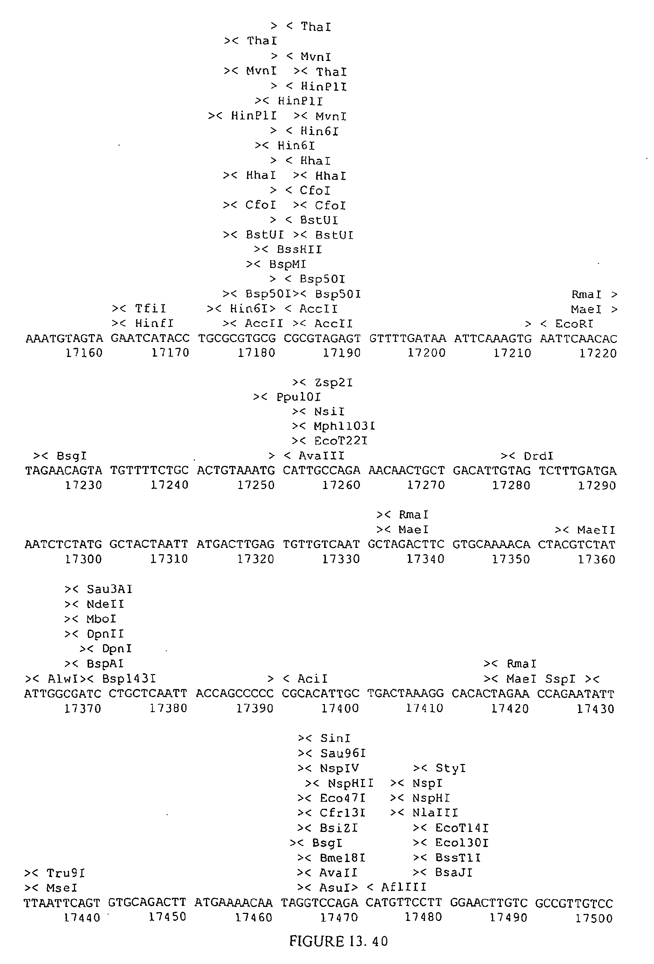

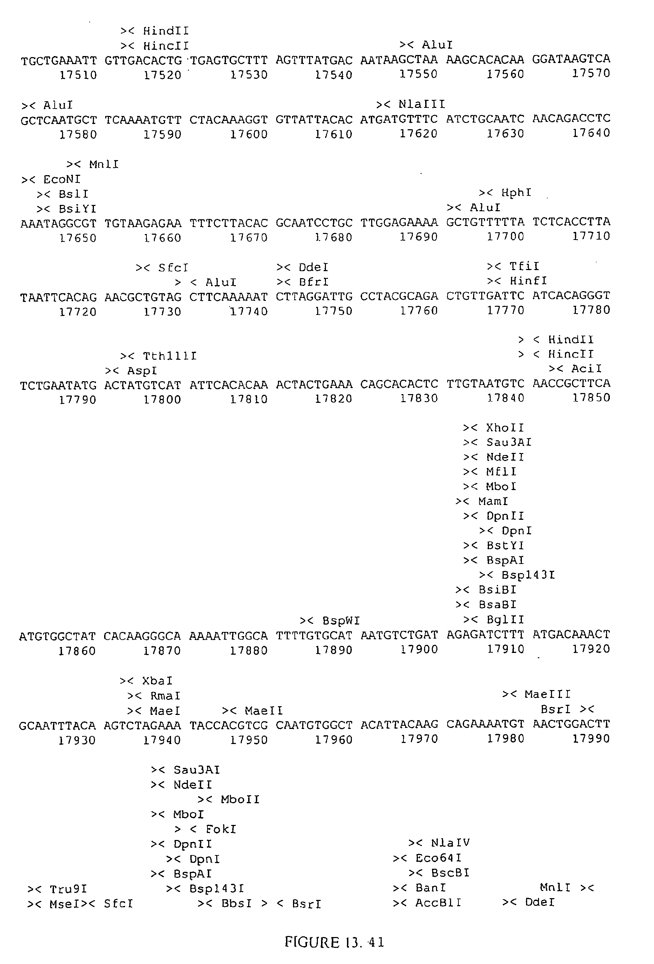

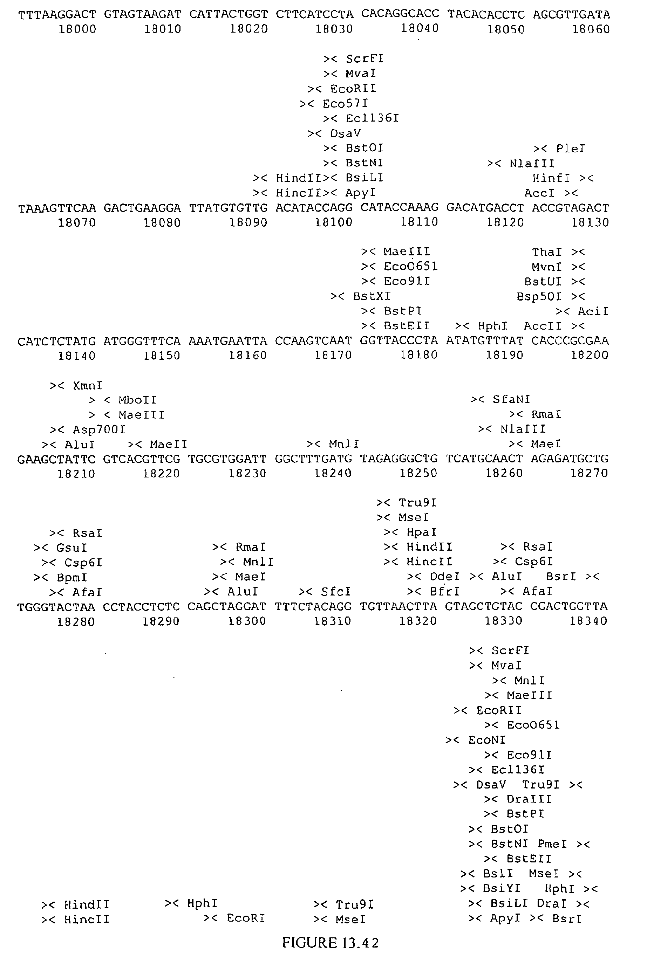

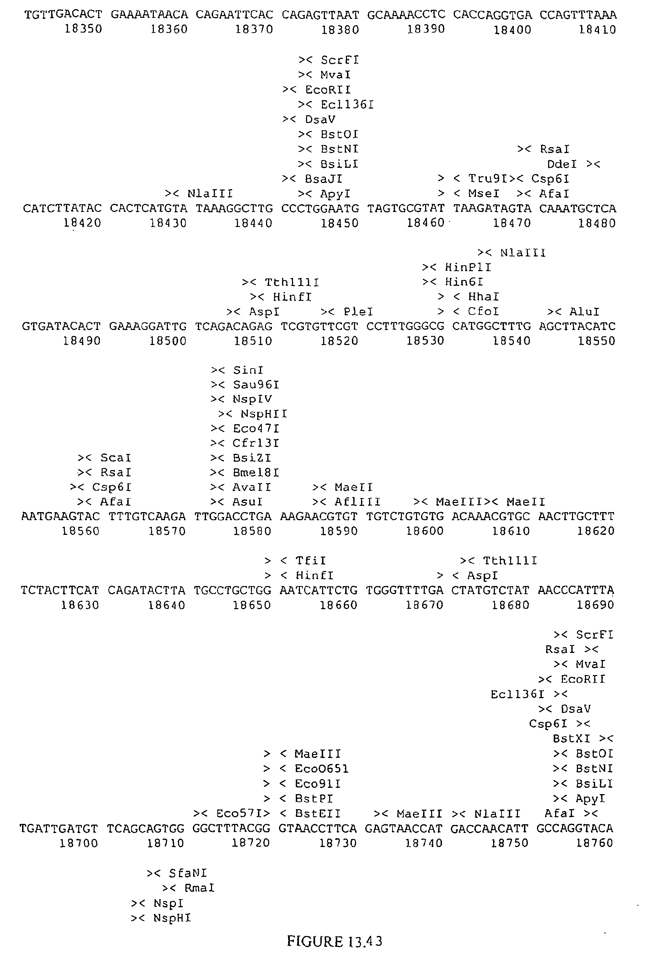

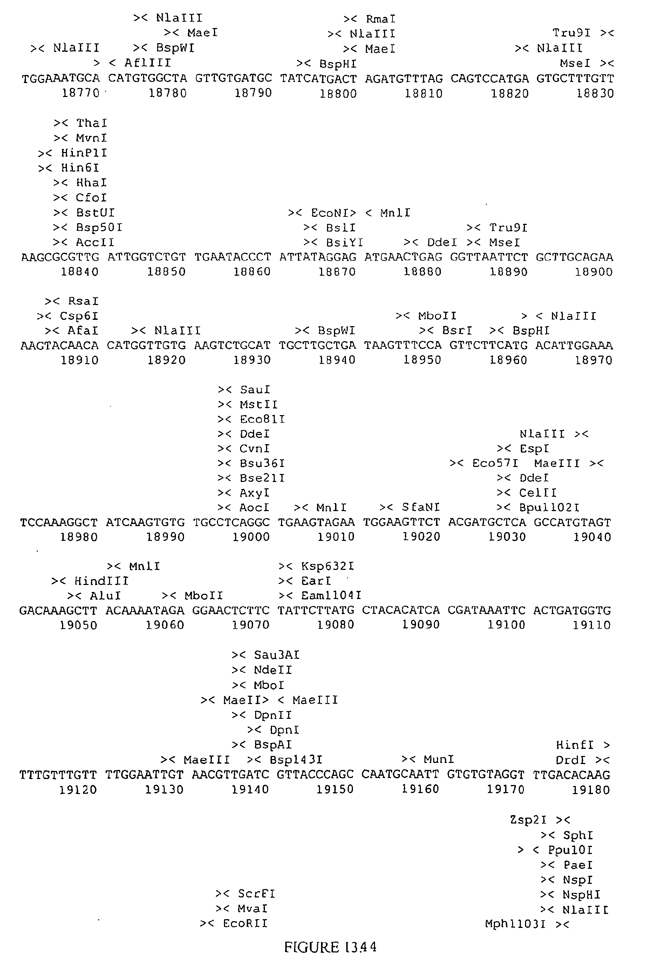

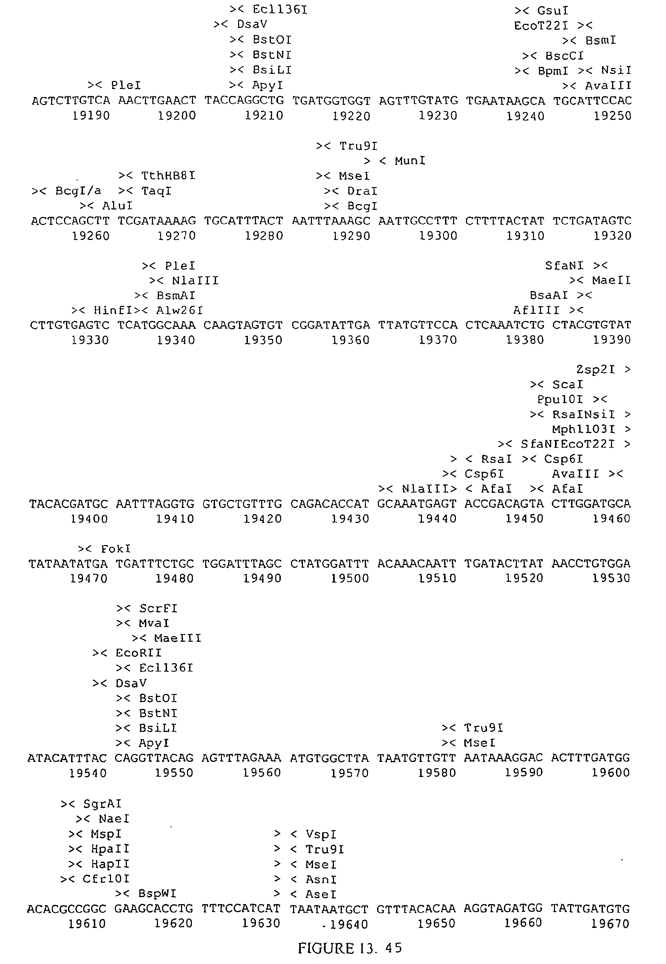

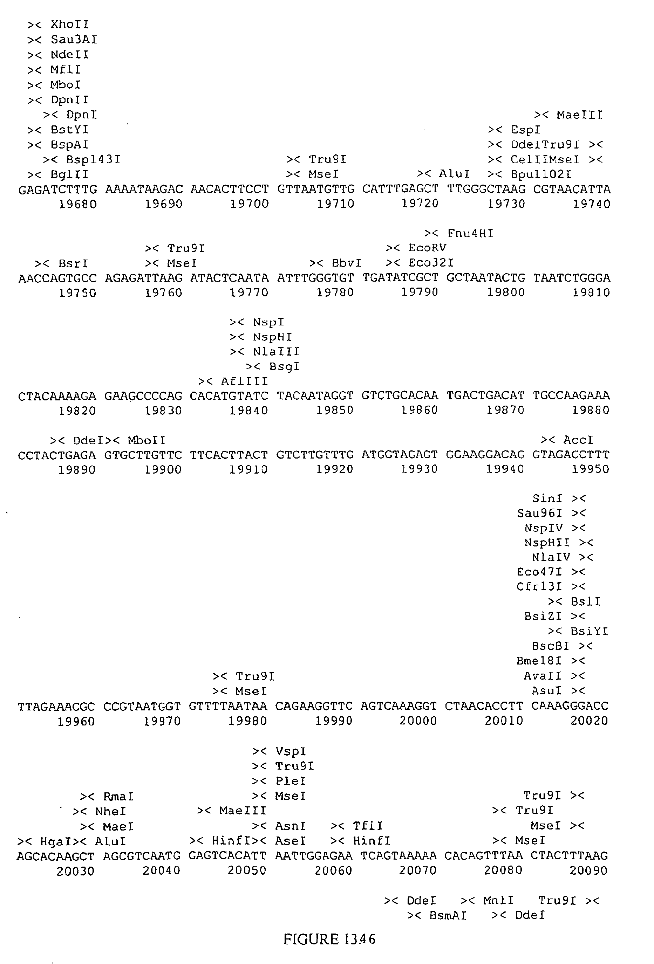

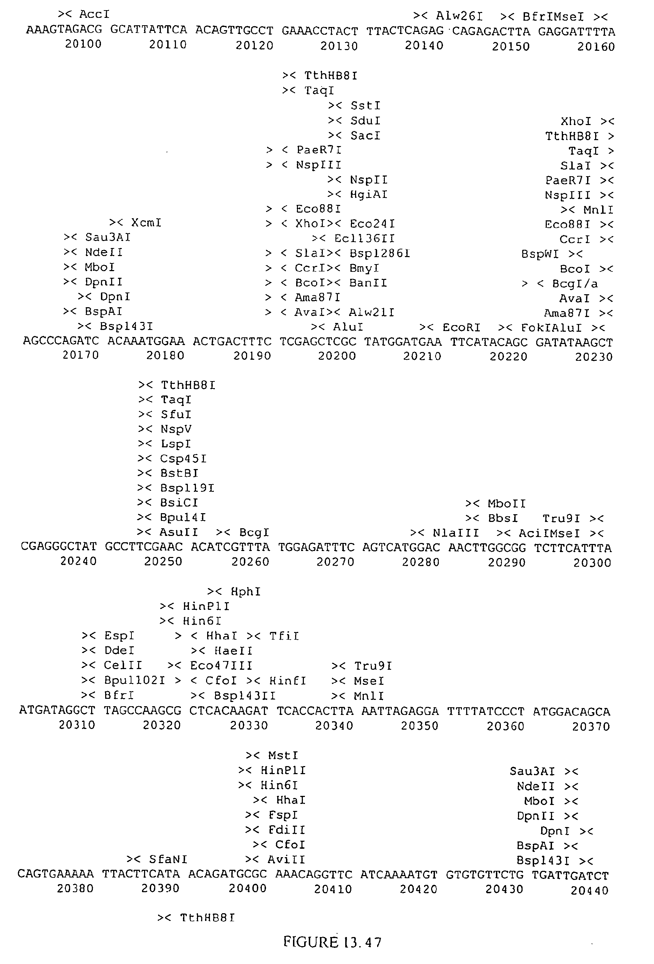

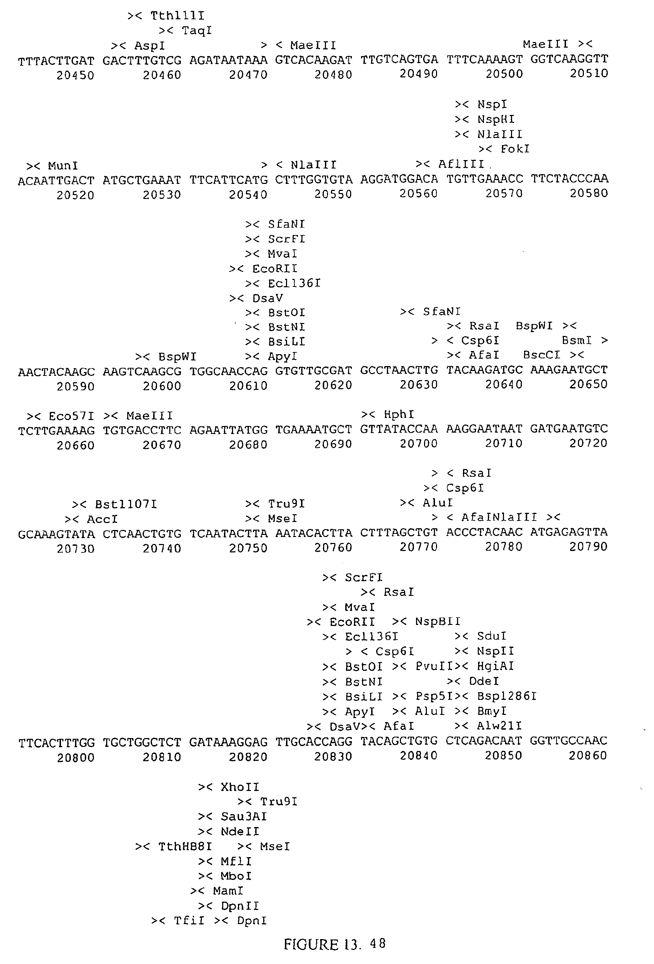

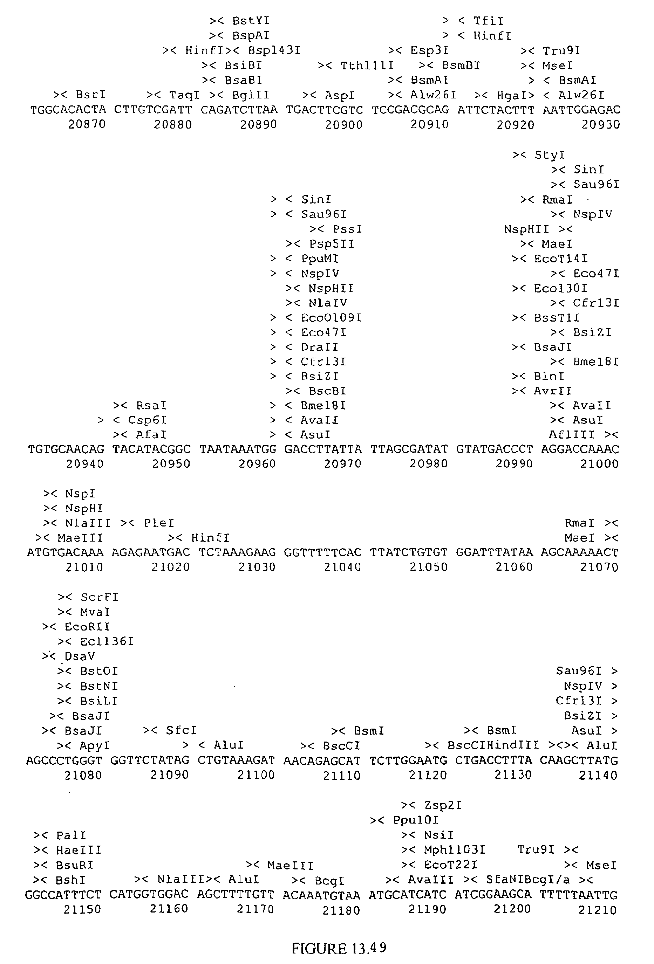

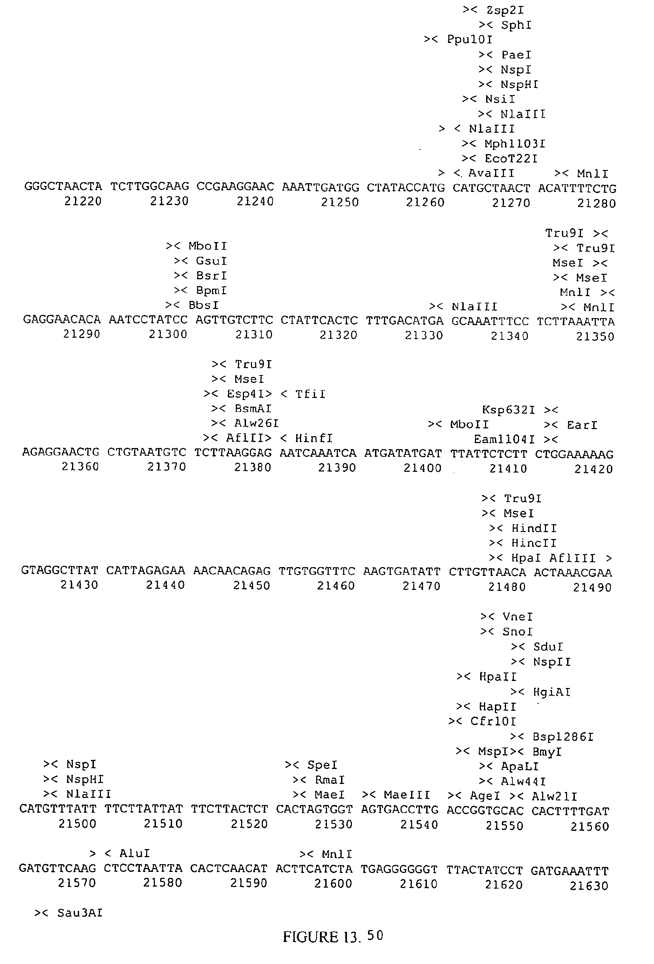

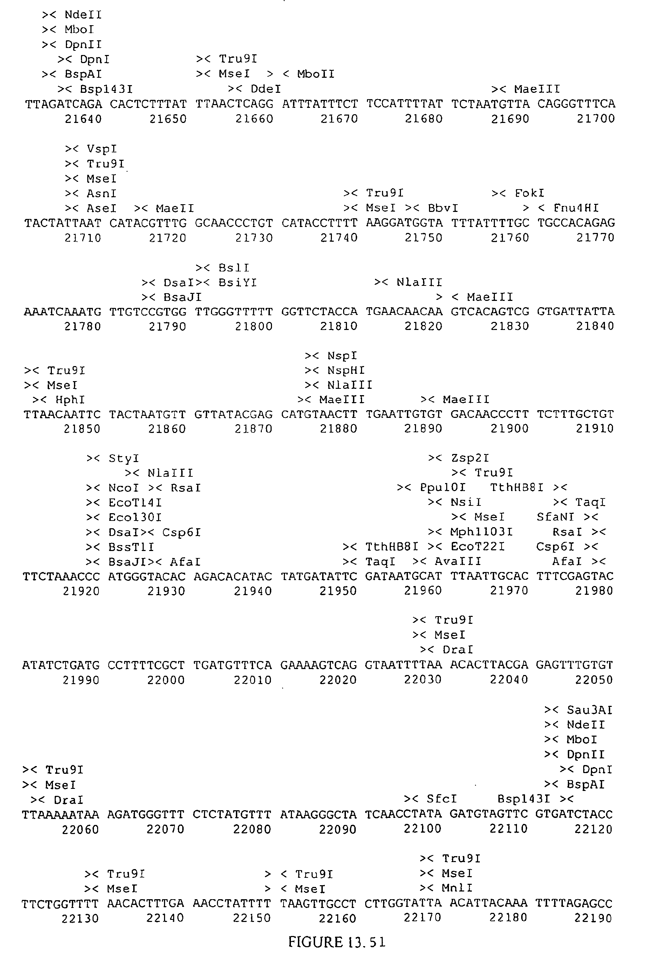

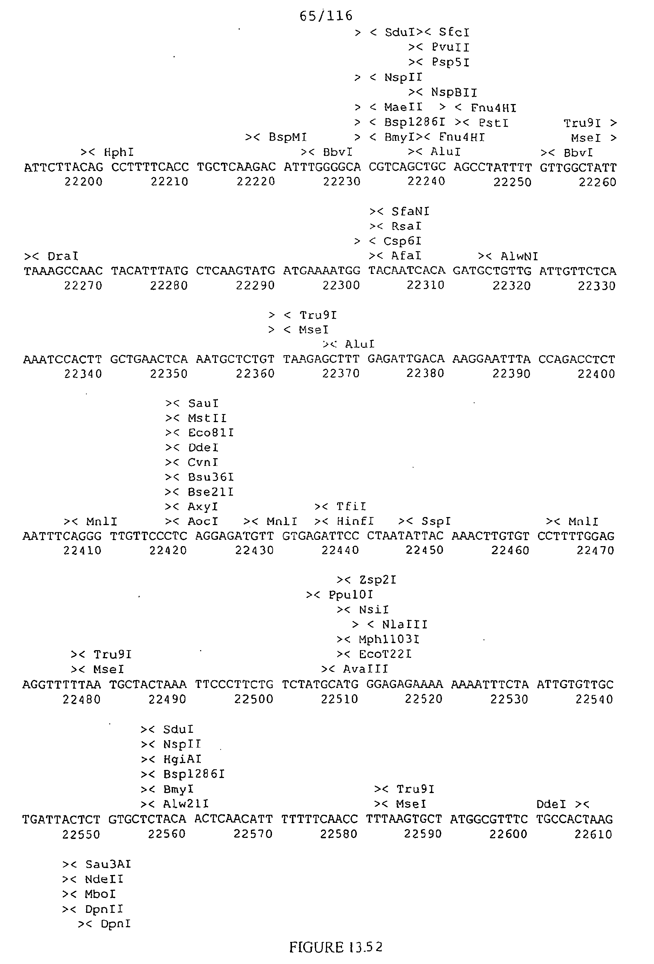

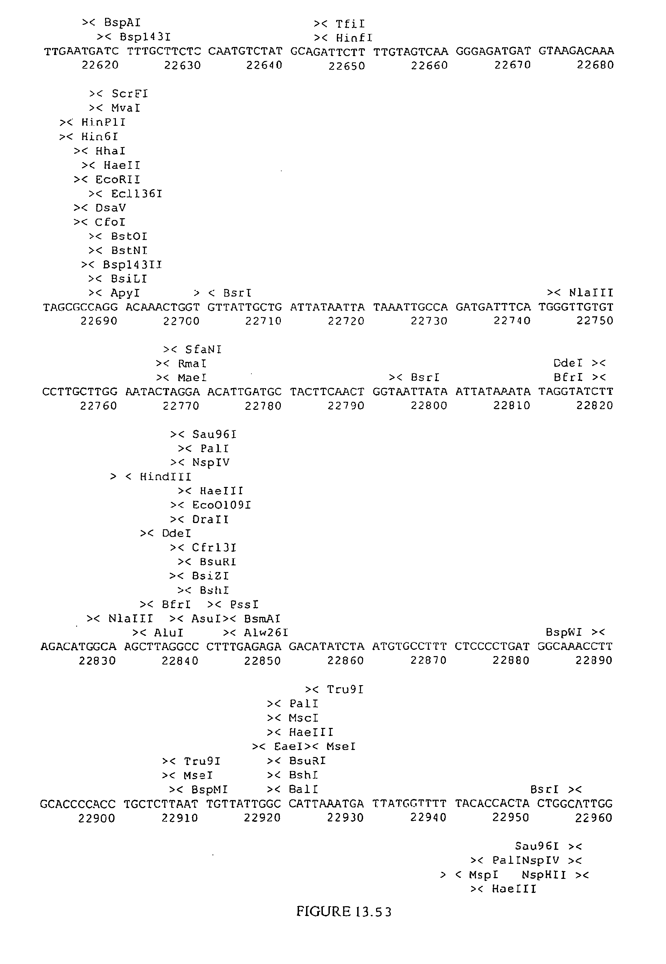

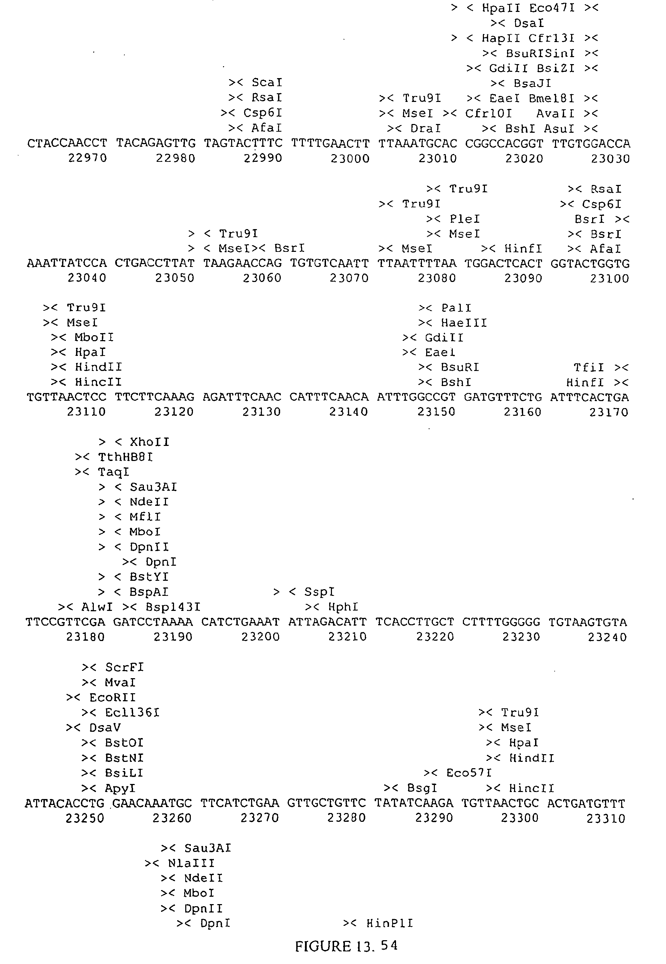

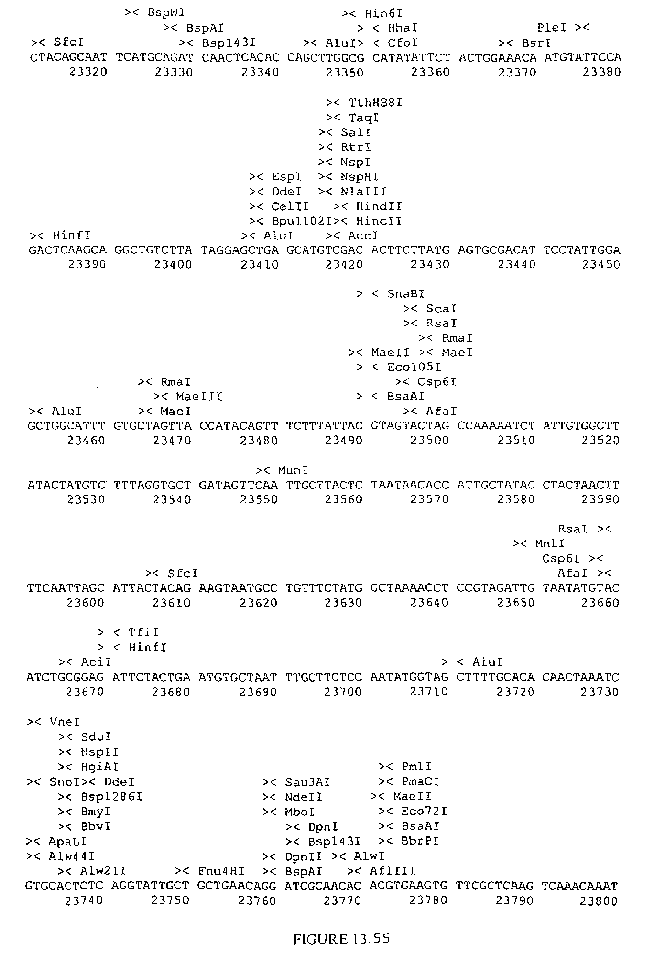

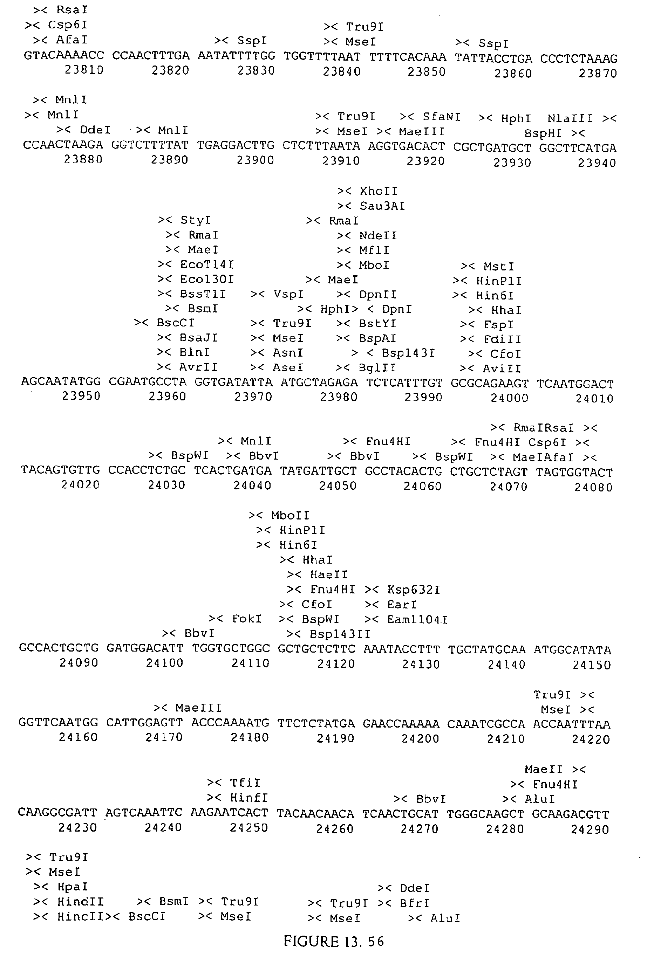

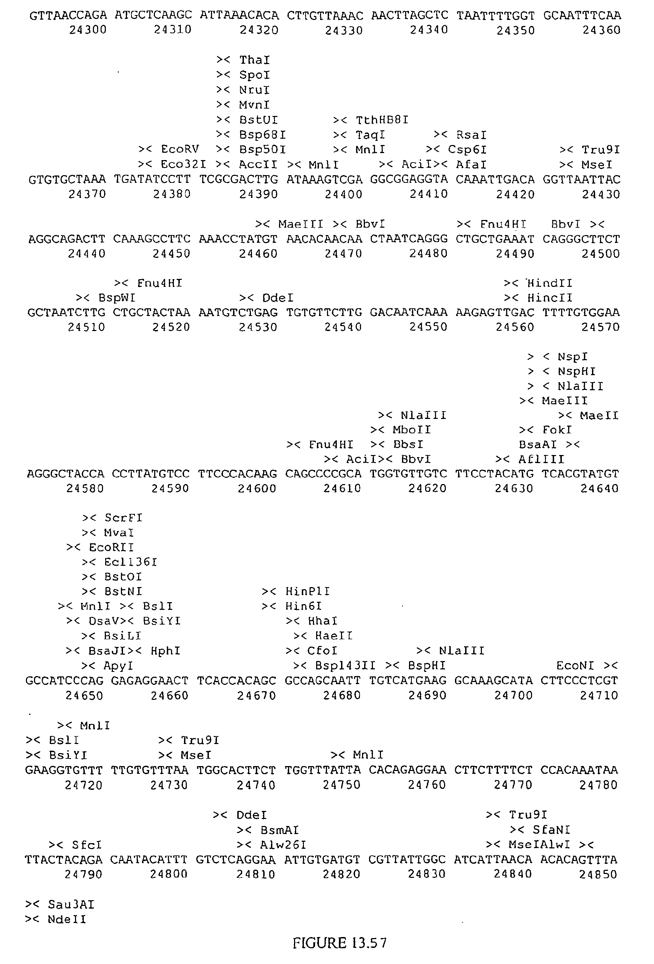

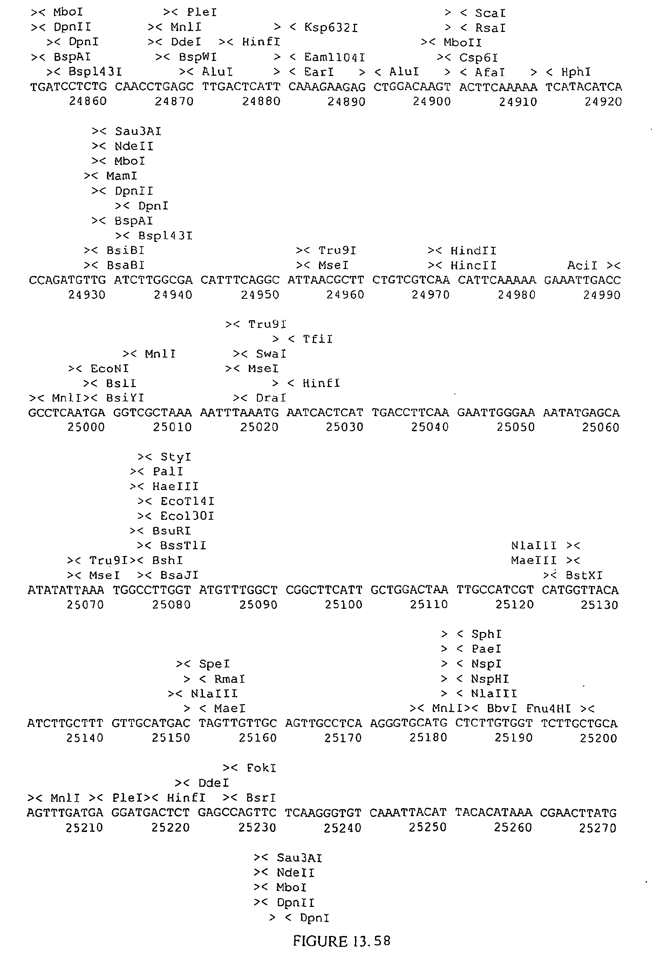

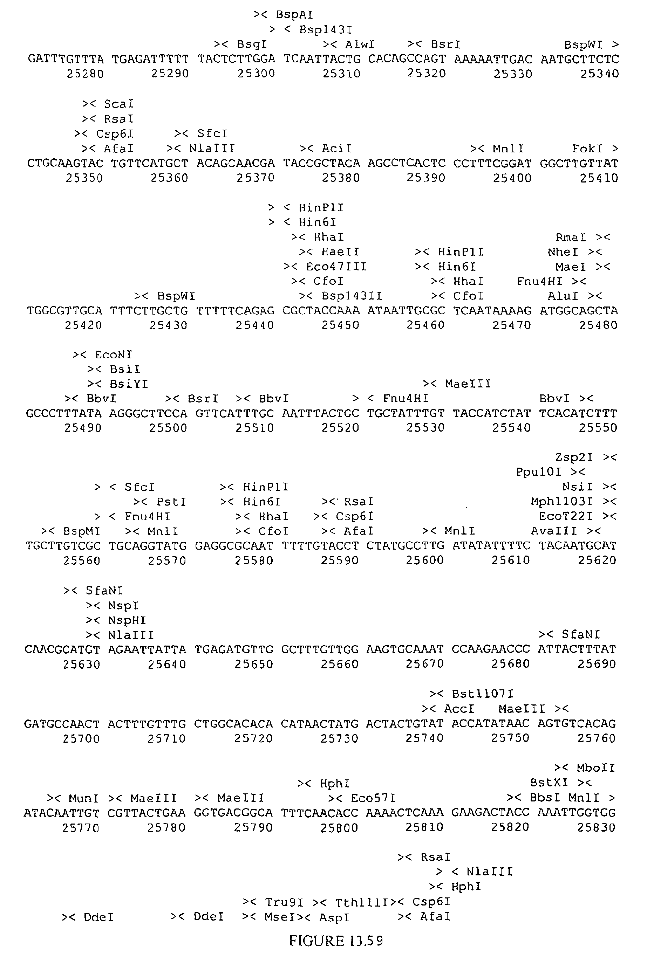

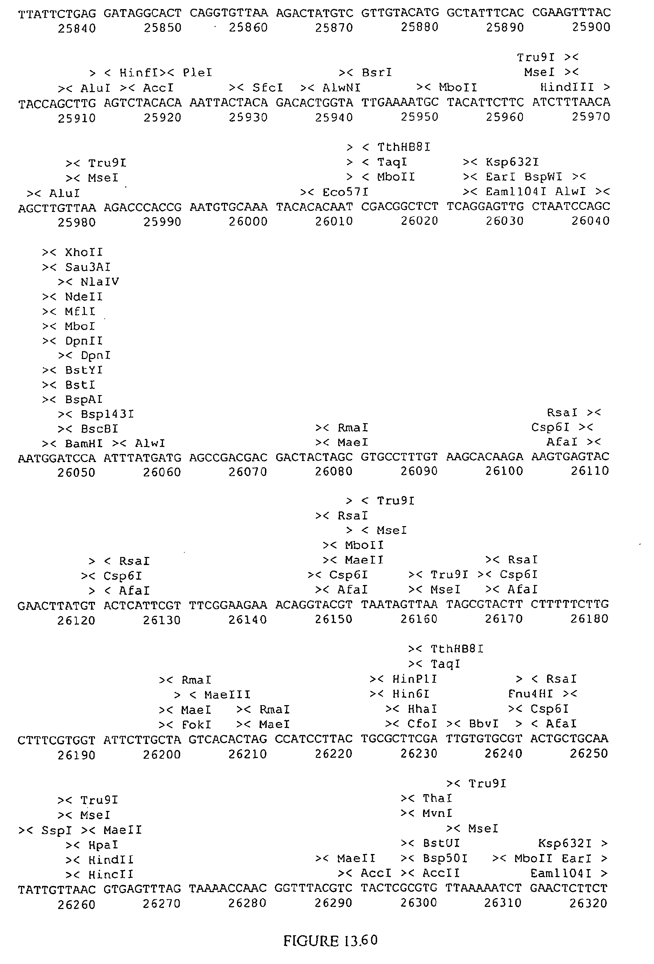

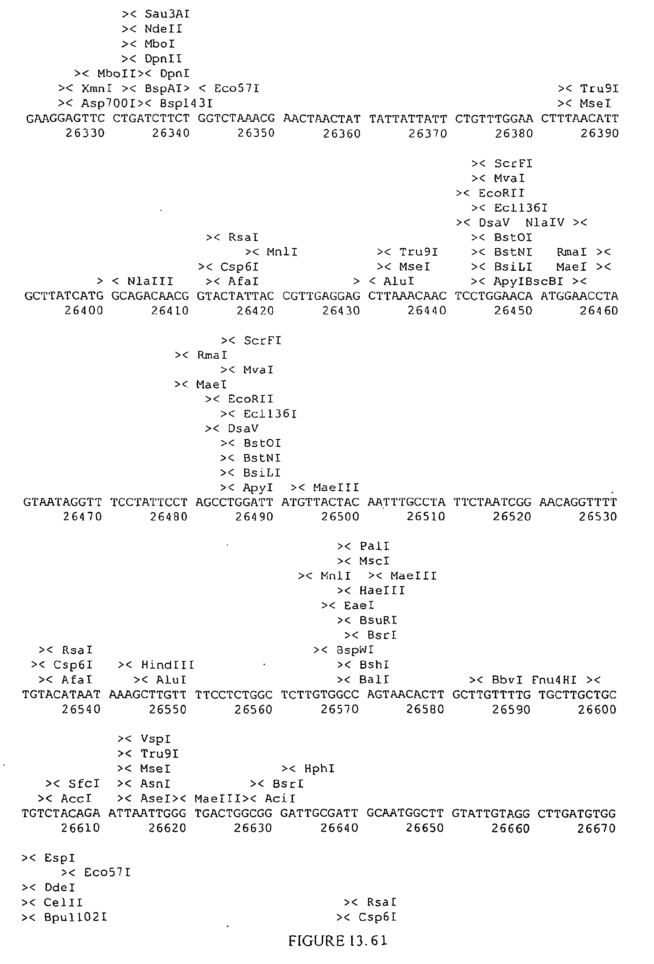

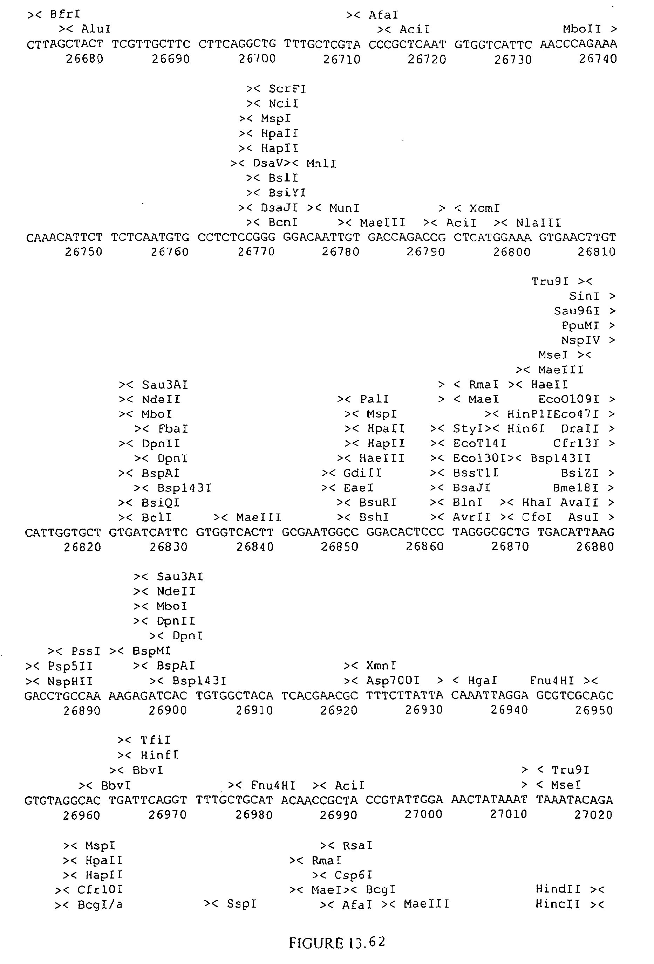

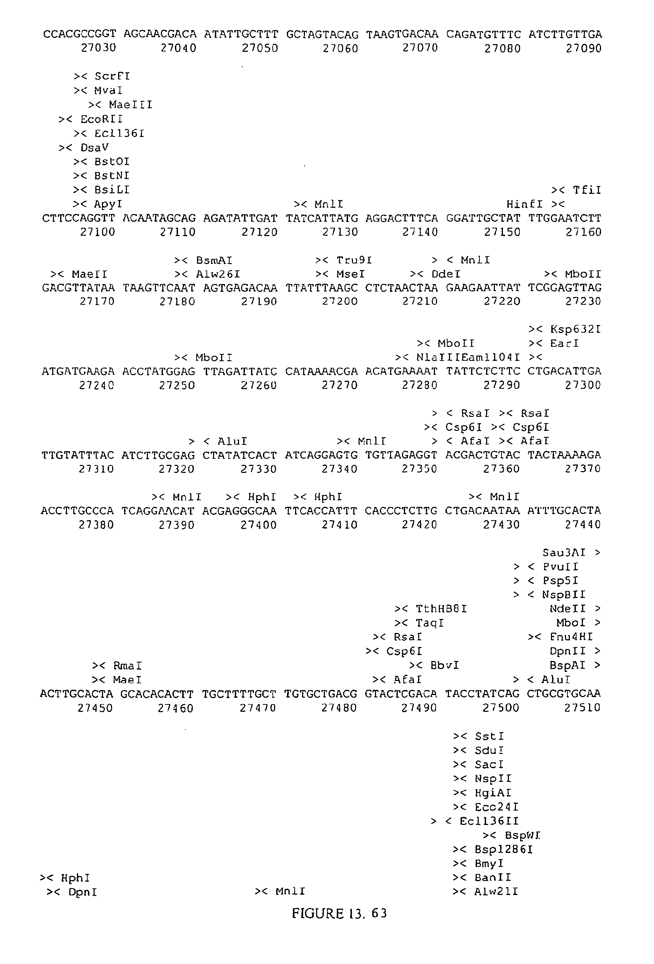

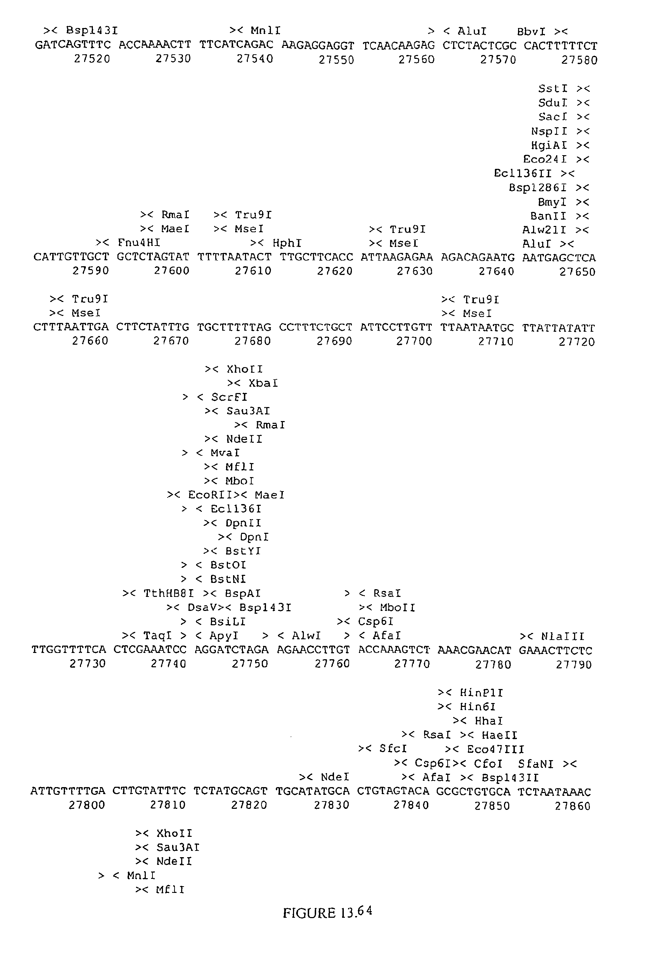

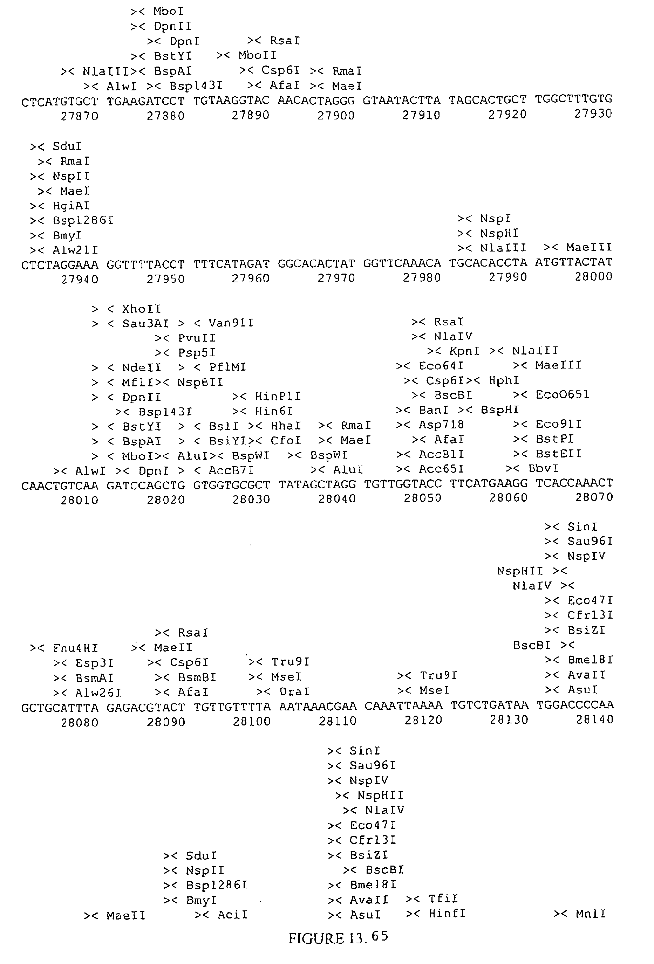

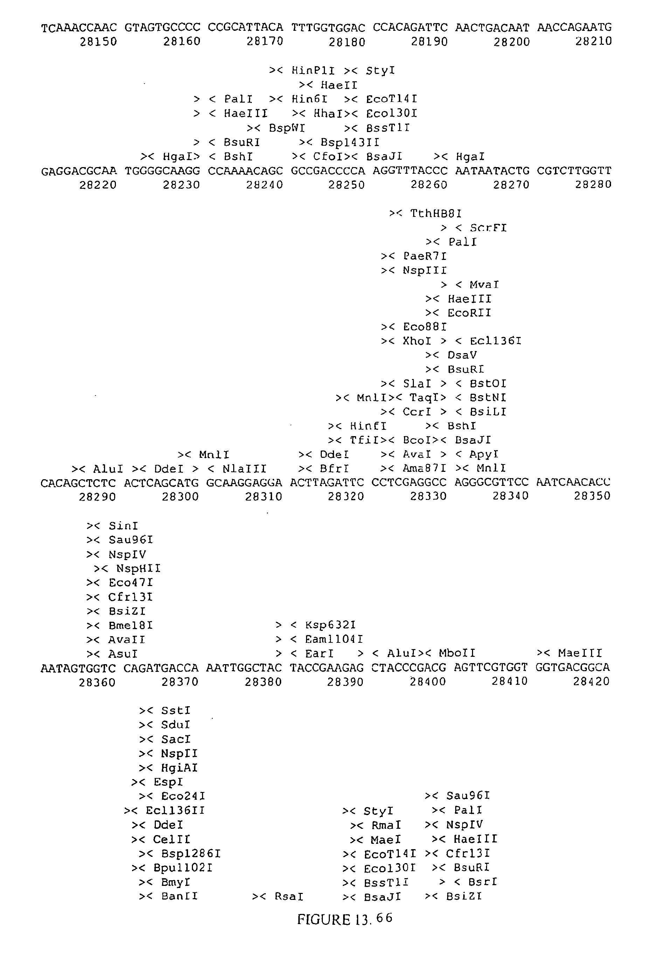

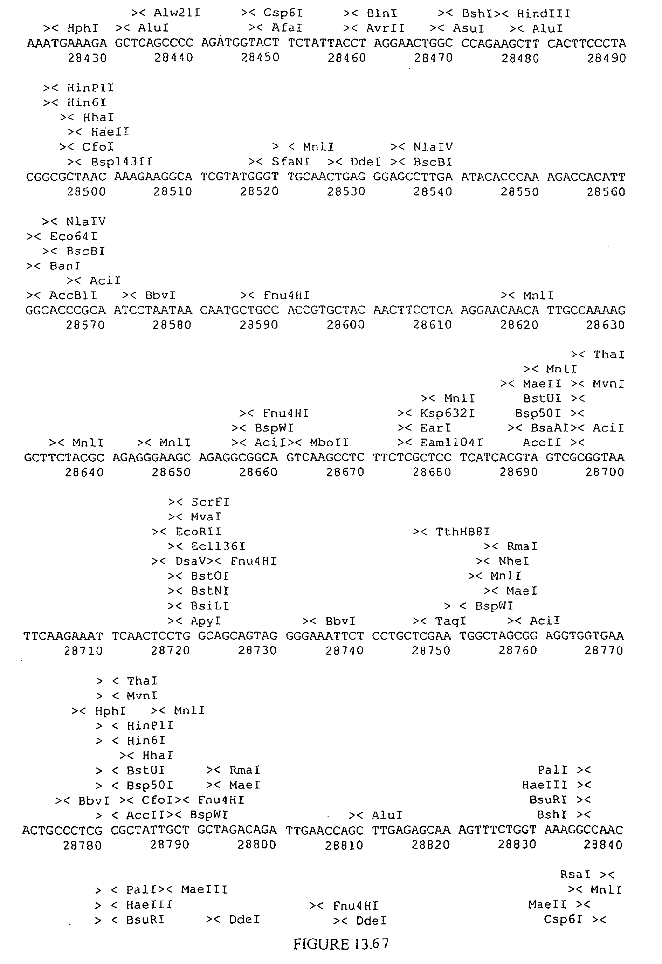

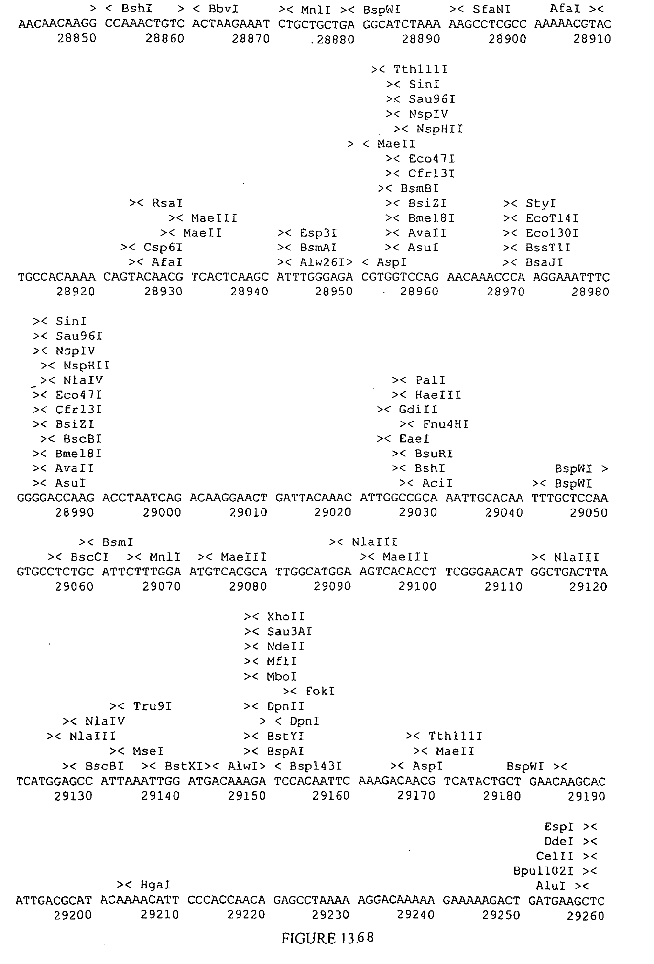

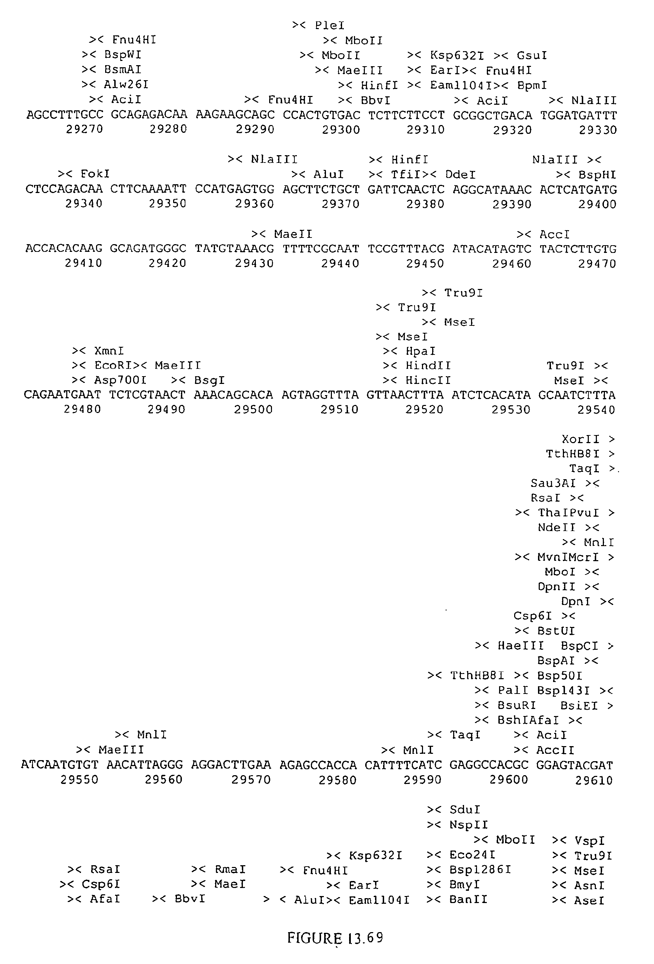

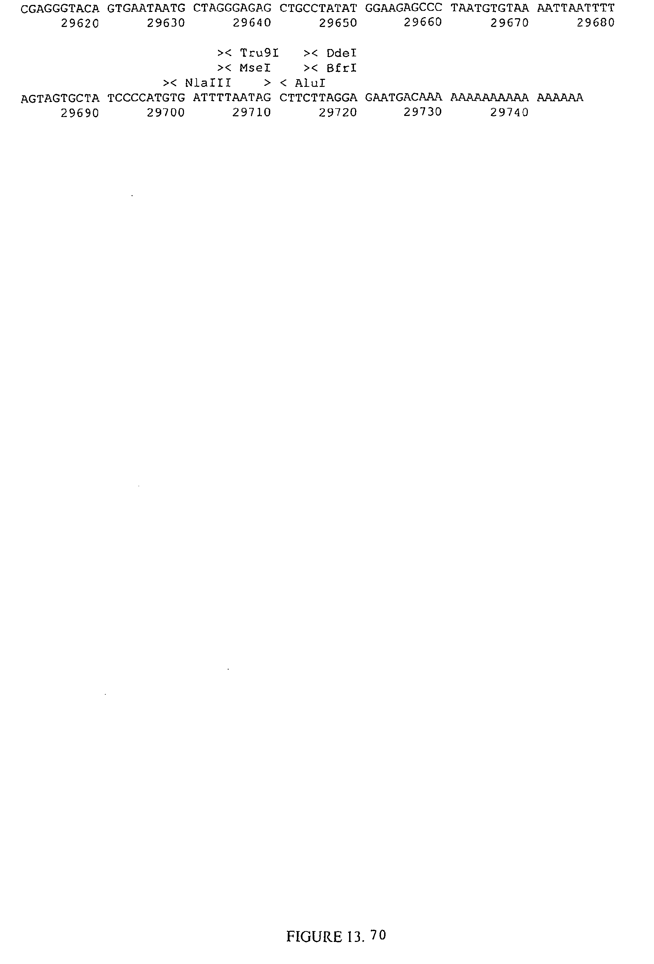

La présente invention englobe également les fragments d'amplification obtenus à l'aide d'amorces spécifiques du génome de la souche purifiée ou isolée tel que défini ci-dessus, notamment à l'aide d'amorces et de paires d'amorces telles que définies ci-dessus, les fragments de restriction constitués par ou comprenant la séquence des fragments tels que définis ci-dessus, les fragments obtenus par transcription *in vitro* à partir d'un vecteur contenant la séquence SEQ ID NO : 1 ou un fragment tel que défini ci-dessus, ainsi que des fragments obtenus par synthèse chimique. Des exemples de fragments de restriction sont déduits de la carte de restriction de la séquence SEQ ID NO : 1 illustrée par la figure 13. Conformément à l'invention lesdits fragments sont, soit sous forme de fragments isolés, soit sous forme de mélanges de fragments. L'invention englobe également les fragments modifiés, par rapport aux précédents, par enlèvement, ou addition de nucléotides dans une proportion d'environ 15 %, par rapport à la longueur des fragments ci-dessus et/ou modifiés au niveau de la nature des nucléotides, dès lors que les fragments nucléotidiques modifiés conservent une capacité d'hybridation avec les séquences d'ARN génomiques ou antigénomiques de l'isolat tel que défini ci-dessus.

Les molécules d'acide nucléique selon l'invention sont obtenues par les méthodes classiques, connues en elles-mêmes, en suivant les protocoles standards tels que ceux décrits dans *Current Protocols in Molecular Biology (Frederick M. A USUBEL,2000, Wiley and son Inc, Library of Congress, USA).* Par exemple, elles peuvent être obtenues par amplification d'une séquence nucléique par PCR ou RT-PCR ou bien par synthèse chimique totale ou partielle.

La présente invention a également pour objet une puce ou filtre à ADN ou à ARN, **caractérisé en ce qu**'il comprend au moins un polynucléotide ou l'un de ses fragments tels que définis ci-dessus.

Les puces ou filtres à ADN ou à ARN selon l'invention sont préparés par les méthodes classiques, connues en elles-mêmes, comme par exemple greffage chimique ou électrochimique d'oligonucléotides sur support de verre ou de nylon.

La présente invention a également pour objet un vecteur de clonage et/ou d'expression recombinant, notamment un plasmide, un virus, un vecteur viral ou un phage comprenant un fragment d'acide nucléique tel que défini ci-dessus. De préférence, ledit vecteur recombinant est un vecteur d'expression dans lequel ledit fragment d'acide nucléique est placé sous le contrôle d'éléments régulateurs de la transcription et de la traduction appropriés. En outre, ledit vecteur peut comprendre des séquences (étiquettes ou tag) fusionnées en phase avec l'extrémité 5' et/ou 3' dudit insert, utiles pour l'immobilisation, et/ou la détection et/ou la purification de la protéine exprimée à partir dudit vecteur.

Ces vecteurs sont construits et introduits dans des cellules hôtes par les méthodes classiques d'ADN recombinant et de génie génétique, qui sont connues en elles-mêmes. De nombreux vecteurs dans lesquels on peut insérer une molécule d'acide nucléique d'intérêt afin de l'introduire et de la maintenir dans une cellule hôte, sont connus en eux-mêmes ; le choix d'un vecteur approprié dépend de l'utilisation envisagée pour ce vecteur (par exemple réplication de la séquence d'intérêt, expression de cette séquence, maintien de la séquence sous forme extrachromosomique ou bien intégration dans le matériel chromosomique de l'hôte), ainsi que de la nature de la cellule hôte.

Conformément à l'invention, ledit plasmide est notamment sélectionné parmi les plasmides suivants :
- le plasmide, dénommé SARS-S, compris dans la souche bactérienne déposée sous le n° I-3059, le 20 juin 2003, auprès de la Collection Nationale de Cultures de Microorganismes, 25 rue du Docteur Roux, 75724 Paris Cedex 15 ; il contient la séquence d'ADNc codant pour la protéine S de la souche de SARS-CoV issue du prélèvement répertorié sous le n° 031589, laquelle séquence correspondant aux nucléotides des positions 21406 à 25348 (SEQ ID NO : 4), en référence à la séquence Genbank AY274119.3,
- le plasmide, dénommé SARS-S1, compris dans la souche bactérienne déposée sous le n° 1-3020, le 12 mai 2003, auprès de la Collection Nationale de Cultures de Microorganismes, 25 rue du Docteur Roux, 75724 Paris Cedex 15 ; il contient un fragment 5' de la séquence d'ADNc codant pour la protéine S de la souche de SARS-CoV issue du prélèvement répertorié sous le n° 031589, telle que définie ci-dessus, lequel fragment correspondant aux nucléotides des positions 21406 à 23454 (SEQ ID NO :5), en référence à la séquence Genbank AY274119.3 Tor2,
- le plasmide, dénommé SARS-S2, compris dans la souche bactérienne déposée sous le n° I-3019, le 12 mai 2003, auprès de la Collection Nationale de Cultures de Microorganismes, 25 rue du Docteur Roux, 75724 Paris Cedex 15 ; il contient un fragment 3'de la séquence d'ADNc codant pour la protéine S de la souche de SARS-CoV issue du prélèvement répertorié sous le n° 031589, telle que définie ci-dessus, lequel fragment correspondant aux nucléotides des positions 23322 à 25348 (SEQ ID NO :6), en référence à la séquence Genbank n° d'accès AY274119.3,
- le plasmide, dénommé SARS-SE, compris dans la souche bactérienne déposée sous le n° I-3126, le, 13 novembre 2003, auprès de la Collection Nationale de Cultures de Microorganismes, 25 rue du Docteur Roux, 75724 Paris Cedex 15; il contient l'ADNc correspondant à la région située entre l'ORF-S et l'ORF-E et chevauchant l'ORF-E de la souche de SARS-CoV issue du prélèvement répertorié sous le n° 031589, telle que définie ci-dessus, laquelle région correspondant aux nucléotides des positions 25110 à 26244 (SEQ ID NO :8), en référence à la séquence Genbank n° d'accès AY274119.3,
- le plasmide, dénommé SARS-E, compris dans la souche bactérienne déposée sous le n° I-3046, le 28 mai 2003, auprès de la Collection Nationale de Cultures de Microorganismes, 25 rue du Docteur Roux, 75724 Paris Cedex 15 ; il contient la séquence d'ADNc codant pour la protéine E de la souche de SARS-CoV issue du prélèvement répertorié sous le n° 031589, telle que définie ci-dessus, laquelle séquence correspondant aux nucléotides des positions 26082 à 26413 (SEQ ID NO :15), en référence à la séquence Genbank n° d'accès AY274119.3,
- le plasmide, dénommé SARS-M ; compris dans la souche bactérienne déposée sous le n° I-3047, le 28 mai 2003, auprès de la Collection Nationale de Cultures de Microorganismes, 25 rue du Docteur Roux, 75724 Paris Cedex 15 ; il contient la séquence d'ADNc codant pour la protéine M de la souche de SARS-CoV issue du prélèvement répertorié sous le n° 031589, telle que définie ci-dessus ; laquelle séquence correspondant aux nucléotides des positions 26330 à 27098 (SEQ ID NO :18), en référence à la séquence Genbank n° d'accès AY274119.3,
- le plasmide dénommé SARS-MN, compris dans la souche bactérienne déposée sous le n° I-3125, le 13 novembre 2003, auprès de la Collection Nationale de Cultures de Microorganismes, 25 rue du Docteur Roux, 75724 Paris Cedex 15 ; il contient la séquence d'ADNc correspondant à la région située entre l'ORF-M et l'ORF-N de la souche de SARS-CoV issue du prélèvement répertorié sous le n° 031589 et prélevée à Hanoi, telle que définie ci-dessus, laquelle séquence correspondant aux nucléotides des positions 26977 à 28218 (SEQ ID NO :20), en référence à la séquence Genbank n° d'accès AY274119.3,
- le plasmide dénommé SARS-N, compris dans la souche bactérienne déposée sous le n° 1-3048, le 5 juin 2003, auprès de la Collection Nationale de Cultures de Microorganismes, 25 rue du Docteur Roux, 75724 Paris Cedex 15 ; il contient l'ADNc codant pour la protéine N de la souche de SARS-CoV issue du prélèvement répertorié sous le n° 031589, telle que définie ci-dessus, laquelle séquence correspondant aux nucléotides des positions 28054 à 29430 (SEQ ID NO :38), en référence à la séquence Genbank n° d'accès AY274119.3 ; ainsi ce plasmide comprend un insert de séquence SEQ ID NO :38 et est compris dans une souche bactérienne qui et qu'il a été déposée sous le n° I-3048, le 5 juin 2003, auprès de la Collection Nationale de Cultures de Microorganismes, 25 rue du Docteur Roux, 75724 Paris Cedex 15,
- le plasmide dénommé SARS-5'NC, compris dans la souche bactérienne déposée sous le n° I- 3124, le 7 novembre 2003, auprès de la Collection Nationale de Cultures de Microorganismes, 25 rue du Docteur Roux, 75724 Paris Cedex 15 ; il contient l'ADNc correspondant à l'extrémité 5'non codante du génome de la souche de SARS-CoV issue du prélèvement répertorié sous le n° 031589, telle que définie ci-dessus, laquelle séquence correspondant aux nucléotides des positions 1 à 204 (SEQ ID NO :39), en référence à la séquence Genbank n° d'accès AY274119.3,
- le plasmide dénommé SARS-3'NC, compris dans la souche bactérienne déposée sous le n° I-3123 le 7 novembre 2003, auprès de la Collection Nationale de Cultures de Microorganismes, 25 rue du Docteur Roux, 75724 Paris Cedex 15. ; il contient la séquence d'ADNc correspondant à l'extrémité 3'non codante du génome de la souche de SARS-CoV issue du prélèvement répertorié sous le n° 031589, telle que définie ci-dessus, laquelle séquence correspondant à celle située entre le nucléotide en position 28933 à 29727 (SEQ ID NO :40), en référence à la séquence Genbank n° d'accès AY274119.3, se termine par une série de nucléotides a.,
- le plasmide d'expression dénommé pIV2.3N, contenant un fragment d'ADNc codant pour une fusion C-terminale de la protéine N (SEQ ID NO : 37) avec une étiquette polyhistidine,
- le plasmide d'expression dénommé pIV2.3S_{C}, contenant un fragment d'ADNc codant pour une fusion C-terminale du fragment correspondant aux positions 475 à 1193 de la séquence en acides aminés de la protéine S (SEQ ID NO : 3) avec une étiquette polyhistidine,
- le plasmide d'expression pIV2.3S_{L}, contenant un fragment d'ADNc codant pour une fusion C-terminale du fragment correspondant aux positions 14 à 1193 de la séquence en acides aminés de la protéine S (SEQ ID NO : 3) avec une étiquette polyhistidine,
- le plasmide d'expression dénommé pIV2.4N, contenant un fragment d'ADNc codant pour une fusion N-terminale de la protéine N (SEQ ID NO : 3) avec une étiquette polyhistidine,
- le plasmide d'expression dénommé pIV2.4S_{C} ou pIV2.4S_{I}, contenant un insert codant pour une fusion N-terminale du fragment correspondant aux positions 475 à 1193 de la séquence en acides aminés de la protéine S (SEQ ID NO : 3) avec une étiquette polyhistidine, et
- le plasmide d'expression dénommé pIV2.4S_{L} contenant un fragment d'ADNc codant pour une fusion N-terminale du fragment correspondant aux positions 14 à 1193 de la séquence en acides aminés de la protéine S (SEQ ID NO : 3) avec une étiquette polyhistidine.

Selon une disposition avantageuse du plasmide d'expression tel que défini ci-dessus, il est compris dans une souche bactérienne qui a été déposée sous le n° I- 3117, le 23 octobre 2003, auprès de la Collection Nationale de Cultures de Microorganismes, 25 rue du Docteur Roux, 75724 Paris Cedex 15.

Selon une autre disposition avantageuse du plasmide d'expression tel que défini ci-dessus, il est compris dans une souche bactérienne qui a été déposée sous le n° I- 3118, le 23 octobre 2003, auprès de la Collection Nationale de Cultures de Microorganismes, 25 rue du Docteur Roux, 75724 Paris Cedex 15.

Selon une autre disposition du plasmide d'expression tel que défini ci-dessus, il est compris dans une souche bactérienne qui a été déposée à la CNCM, 25 rue du Docteur Roux, 75724 Paris Cedex 15 sous les numéros suivants :
a) souche n° I- 3118, déposée le 23 octobre 2003,
b) souche n° I- 3019, déposée le 12 mai 2003,
c) souche n° I- 3020, déposée le 12 mai 2003,
d) souche n'I-3059, déposée le 20 juin 2003,
e) souche n° I-3323, déposée le 22 novembre 2004,
f) souche n° I-3324, déposée le 22 novembre 2004,
g) souche n° I-3326, déposée le 1^{er} décembre 2004,
h) souche n° I-3327, déposée le 1^{er} décembre 2004,
i) souche n° I-3332, déposée le 1^{er} décembre 2004,
j) souche n° I-3333, déposée le 1^{er} décembre 2004,
k) souche n°I-3334, déposée le 1^{er} décembre 2004,
l) souche n° I-3335, déposée le 1^{er} décembre 2004,
m) souche n° I-3336, déposée le 1^{er} décembre 2004,
n) souche n° I-3337, déposée le 1^{er} décembre 2004,
o) souche n° I-3338, déposée le 2 décembre 2004,
p) souche n° I-3339, déposée le 2 décembre 2004,
q) souche n° I-3340, déposée le 2 décembre 2004,
r) souche n° I-3341, déposée le 2 décembre 2004.

La présente invention a également pour objet un insérat d'acide nucléique d'origine virale, **caractérisé en ce qu**'il est contenu dans l'une quelconque des souches telles que définies ci-dessus en a)-r).

La présente invention a également pour objet un acide nucléique comportant un gène synthétique permettant une expression optimisée de la protéine S dans des cellules eucaryotes, **caractérisé en ce qu**'il possède la séquence SEQ ID NO: 140.

La présente invention a également pour objet un vecteur d'expression comportant un acide nucléique comportant un gène synthétique permettant une expression optimisée de la protéine S, lequel vecteur est contenu dans la souche bactérienne déposée à la CNCM, le 1^{er} décembre 2004, sous le n°I-3333.

Selon un mode de réalisation dudit vecteur d'expression, il s'agit d'un vecteur viral, sous forme de particule virale ou sous forme de génome recombinant.

Selon une disposition avantageuse de ce mode de réalisation, il s'agit d'une particule virale recombinante ou d'un génome viral recombinant susceptible d'être obtenu par transfection d'un plasmide selon les alinéas g), h) et k) à r) tels que définis ci-dessus, dans un système cellulaire approprié, c'est-à-dire, par exemple, des cellules transfectées avec un ou plusieurs autres plasmide(s), destinés à trancomplémenter certaines fonctions du virus délétées dans le vecteur et nécessaires à la formation des particules virales.

On entend ici par « famille de la protéine S » la protéine S complète, son ectodomaine et des fragments de cet ectodomaine qui sont de préférence produits dans un système eucaryote.

La présente invention a également pour objet un vecteur lentiviral codant pour un polypeptide de la famille de la protéine S, telle que définie ci-dessus.

La présente invention a également pour objet un virus rougeole recombinant codant pour un polypeptide de la famille de la protéine S, telle que définie ci-dessus.

La présente invention a également pour objet un virus vaccine recombinant codant pour un polypeptide de la famille de la protéine S, telle que définie ci-dessus.

La présente invention a également pour objet l'utilisation d'un vecteur selon les alinéas e) à r) tels que définis ci-dessus, ou d'un vecteur comportant un gène synthétique de la protéine S, tel que défini ci-dessus, pour la production, en système eucaryote, de la protéine S du coronavirus associé au SRAS, ou d'un fragment de cette protéine.

La présente invention a également pour objet une méthode de production de la protéine S en système eucaryote, comportant une étape de transfection de cellules eucaryotes en culture par un vecteur choisi parmi les vecteurs contenus dans les souches bactériennes mentionnées aux alinéas e) à r) ci-dessus ou un vecteur comportant un gène synthétique permettant une expression optimisée de la protéine S.

La présente invention a également pour objet une banque d'ADNc **caractérisée en ce qu**'elle comprend des fragments tels que définis ci-dessus, en particulier des fragments d'amplification ou des fragments de restriction, clonés dans un vecteur recombinant, notamment un vecteur d'expression (banque d'expression).

La présente invention a également pour objet des cellules, notamment des cellules procaryotes, modifiées par un vecteur recombinant tel que défini ci-dessus.

La présente invention a également pour objet une cellule eucaryote génétiquement modifiée exprimant une protéine ou un polypeptide tels que définis ci-après. Bien évidemment, les termes "cellule eucaryote génétiquement modifiée" ne désignent pas une cellule modifiée par un virus sauvage.

Selon un mode de réalisation avantageux de ladite cellule, elle est susceptible d'être obtenue par transfection par l'un quelconque des vecteurs mentionnés aux alinéas k) à n) ci-dessus.

Selon une disposition avantageuse de ce mode de réalisation, il s'agit de la cellule FRhK4-Ssol-30, déposée à la CNCM le 22 novembre 2004, sous le n°I-3325.

Les vecteurs recombinants tels que définis ci-dessus et les cellules transformées par lesdits vecteurs d'expression sont avantageusement utilisés pour la production des protéines et des peptides correspondants. Les banques d'expression dérivées desdits vecteurs, ainsi que les cellules transformées par lesdites banques d'expression sont avantageusement utilisées pour identifier les épitopes immunogènes (épitopes B et T) des protéines du coronavirus associé au SRAS.

La présente invention a également pour objet les protéines et les peptides purifiées ou isolées, **caractérisés en ce qu**'ils sont codés par le polynucléotide ou l'un de ses fragments tels que définis ci-dessus.

Selon un mode de réalisation avantageux de l'invention, ladite protéine est sélectionnée dans le groupe constitué par :
- la protéine S de séquence SEQ ID NO :3 ou son ectodomaine
- la protéine E de séquence SEQ ID NO :14
- la protéine M de séquence SEQ ID NO :17
- la protéine N de séquence SEQ ID NO : 37
- les protéines codées par les ORFs : ORF1a, ORF1b, ORF3, ORF4 et ORF7 à ORF11, ORF 13 et ORF 14 de séquence respectivement, SEQ ID NO :74, 75, 10, 12, 22, 24, 26, 28, 30, 33 et 35.

On utilisera ci-après indifféremment les termes « ectodomaine de la protéine S » et « forme soluble de la protéine S ».

Selon un mode de réalisation avantageux de l'invention, ledit polypeptide est constitué des acides aminés correspondant aux positions 1 à 1193 de la séquence en acides aminés de la protéine S

Selon un autre mode de réalisation avantageux de l'invention, ledit peptide est sélectionné dans le groupe constitué par :
a) les peptides correspondant aux positions 14 à 1193 et 475 à 1193 de la séquence en acides aminés de la protéine S,
b) les peptides correspondant aux positions 2 à 14 (SEQ ID NO : 69) et 100 à 221 de la séquence en acides aminés de la protéine M ; ces peptides correspondent respectivement à l'ectodomaine et à l'endodomaine de la protéine M, et
c) les peptides correspondant aux positions 1 à 12 (SEQ ID NO : 70) et 53 à 76 (SEQ ID NO : 71) de la séquence en acides aminés de la protéine E; ces peptides correspondent respectivement à l'ectodomaine et à l'extrémité C-terminale de la protéine E, et
d) les peptides de 5 à 50 acides aminés consécutifs, de préférence de 10 à 30 acides aminés, inclus ou chevauchant partiellement ou totalement la séquence des peptides tels que définis en a), b) ou c).

La présente invention a également pour objet un peptide **caractérisé en ce qu**'il présente une séquence de 7 à 50 acides aminés incluant un résidu d'acide aminé sélectionné dans le groupe constitué par :
- l'alanine située en position 2552 de la séquence en acides aminés de la protéine codée par l'ORF1a.
- la sérine située en position 577 de la séquence en acides aminés de la protéine S de la souche de SARS-CoV telle que définie ci-dessus,
- la glycine en position 11 de la séquence en acides aminés de la protéine codée par l'ORF3 de la souche de SARS-CoV telle que définie ci-dessus,
- la sérine en position 154 de la séquence en acides aminés de la protéine M de la souche de SARS-CoV telle que définie ci-dessus.

La présente invention a également pour objet un anticorps ou un fragment d'anticorps polyclonal ou monoclonal, susceptible d'être obtenu par immunisation d'un animal avec un vecteur recombinant tel que défini ci-dessus, une banque d'ADNc telle que définie ci-dessus ou bien une protéine ou un peptide tels que définis ci-dessus, **caractérisé en ce qu**'il se lie avec l'une au moins des protéines codées par le SARS-CoV telles que définies ci-dessus.

L'invention englobe les anticorps polyclonaux, les anticorps monoclonaux, les anticorps chimériques tels que les anticorps humanisés, ainsi que leurs fragments (Fab, Fv, scFv).

La présente invention a également pour objet un hybridome produisant un anticorps monoclonal contre la protéine N, **caractérisé en ce qu**'il est choisi parmi les hybridomes suivants :
- l'hybridome produisant l'anticorps monoclonal 87, déposé à la CNCM le 1^{er} décembre 2004 sous le numéro 1-3328,
- l'hybridome produisant l'anticorps monoclonal 86, déposé à la CNCM le 1^{er} décembre 2004 sous le numéro I-3329,
- l'hybridome produisant l'anticorps monoclonal 57, déposé à la CNCM le 1^{er} décembre 2004 sous le numéro I-3330, et
- l'hybridome produisant l'anticorps monoclonal 156, déposé à la CNCM le 1^{er} décembre 2004 sous le numéro I-3331.

La présente invention a également pour objet un anticorps ou fragment d'anticorps polyclonal ou monoclonal dirigé contre la protéine N, **caractérisé en ce qu**'il est produit par un hybridome tel que défini ci-dessus.

Au sens de la présente invention, on entend par anticorps chimérique, relativement à un anticorps d'une espèce animale particulière ou d'une classe particulière d'anticorps, un anticorps comprenant tout ou partie d'une chaîne lourde et/ou d'une chaîne légère d'un anticorps d'une autre espèce animale ou d'une autre classe d'anticorps.

Au sens de la présente invention, on entend par anticorps humanisé une immmunoglobuline humaine dans laquelle les résidus des CDRs (*Complementary-Determining Regions)* qui forment le site de liaison à l'antigène sont remplacés par ceux d'un anticorps monoclonal non-humain possédant la spécificité, l'affinité ou l'activité recherchées. Par comparaison avec les anticorps non-humains, les anticorps humanisés sont moins immunogènes et possèdent une demi-vie prolongée chez l'Homme car ils ne possèdent qu'une faible proportion de séquences non-humaines étant donné que la quasi-totalité des résidus des régions FR (Framework) et de la région constante (Fc) de ces anticorps sont ceux d'une séquence consensus d'immunoglobulines humaines.

La présente invention a également pour objet une puce ou filtre à protéine, **caractérisé en ce qu**'il comprend une protéine, un peptide ou bien un anticorps tels que définis ci-dessus.

Les puces à protéine selon l'invention sont préparées par les méthodes classiques, connues en elles-mêmes. Parmi les supports appropriés sur lesquels peuvent être immobilisés des protéines, on peut citer ceux en matière plastique ou en verre, notamment sous la forme de microplaques.

La présente invention a également pour objet des réactifs dérivés de la souche isolée de coronavirus associé au SRAS, issue du prélèvement répertorié sous le n° 031589, utiles pour l'étude et le diagnostic de l'infection provoquée par un coronavirus associé au SRAS, lesquels réactifs sont sélectionnés dans le groupe constitué par :
(a) une paire d'amorces, une sonde ou une puce à ADN telles que définies ci-dessus,
(b) un vecteur recombinant ou une cellule modifiée tels que définis ci-dessus,
(c) une souche isolée de coronavirus ou un polynucléotide tels que définis ci-dessus,
(d) une protéine ou un peptide tel que défini ci-dessus,
(e) un anticorps ou fragment d'anticorps tels que définis ci-dessus, et
(f) une puce à protéine telle que définie ci-dessus.

Ces différents réactifs sont préparés et utilisés selon les techniques classiques de biologie moléculaire et d'immunologie, en suivant les protocoles standards tels que ceux décrits dans *Current Protocols in Molecular Biology* (Frederick M. AUSUBEL, 2000, Wiley and Son Inc., Library of Congress, USA), dans Current Protocols in Immunology (John E. Cologan, 2000, Wiley and Son Inc. Library of Congress, USA) et dans Antibodies : A Laboratory Manual (E. Howell and D Lane, Cold Spring Harbor Laboratory, 1988).

Les fragments d'acide nucléique selon l'invention sont préparés et utilisés selon les techniques classiques telles que définies ci-dessus. Les peptides et les protéines selon l'invention sont préparés par les techniques d'ADN recombinant, connues de l'Homme du métier, notamment à l'aide des vecteurs recombinants tels que définis ci-dessus. Alternativement, les peptides selon l'invention peuvent être préparés par les techniques classiques de synthèse en phase solide ou liquide, connues de l'Homme du métier.

Les anticorps polyclonaux sont préparés par immunisation d'un animal approprié avec une protéine ou un peptide tels que définis ci-dessus, éventuellement couplé à la KLH ou à l'albumine et/ou associé à un adjuvant approprié tel que l'adjuvant de Freund (complet ou incomplet) ou l'hydroxyde d'alumine ; après obtention d'un titre en anticorps satisfaisant, les anticorps sont récoltés par prélèvement du sérum des animaux immunisés et enrichis en IgG par précipitation, selon les techniques classiques, puis les IgG spécifiques des protéines du SARS-CoV sont éventuellement purifiées par chromatographie d'affinité sur une colonne appropriée sur laquelle sont fixés ledit peptide ou ladite protéine, tels que définis ci-dessus, de façon à obtenir une préparation d'IgG monospécifiques.

Les anticorps monoclonaux sont produits à partir d'hybridomes obtenus par fusion de lymphocytes B d'un animal immunisé par une protéine ou un peptide tels que définis ci-dessus avec des myélomes, selon la technique de Köhler et Milstein (Nature, 1975, 256, 495-497) ; les hybridomes sont cultivés *in vitro,* notamment dans des fermenteurs ou produits *in vivo,* sous forme d'ascite ; alternativement lesdits anticorps monoclonaux sont produits par génie génétique comme décrit dans le brevet américain US 4,816,567.

Les anticorps humanisés sont produits par des méthodes générales comme celles décrites dans la Demande Internationale WO 98/45332.

Les fragments d'anticorps sont produits à partir des régions V_{H} et V_{L} clonées, à partir des ARNm d'hybridomes ou de lymphocytes spléniques d'une souris immunisée ; par exemple, les fragments Fv, scFv ou Fab sont exprimés à la surface de phages filamenteux selon la technique de Winter et Milstein (Nature, 1991, 349, 293-299) ; après plusieurs étapes de sélection, les fragments d'anticorps spécifiques de l'antigène sont isolés et exprimés dans un système d'expression approprié, par les techniques classiques de clonage et d'expression d'ADN recombinant.

Les anticorps ou leur fragments tels que définis ci-dessus, sont purifiés par les techniques classiques connues de l'Homme du métier, telles que la chromatographie d'affinité.

La présente invention a en outre pour objet l'utilisation d'un produit sélectionné dans le groupe constitué par : une paire d'amorces, une sonde, une puce à ADN, un vecteur recombinant, une cellule modifiée, une souche isolée de coronavirus, un polynucléotide, une protéine ou un peptide, un anticorps ou un fragment d'anticorps et une puce à protéine tels que définis ci-dessus, pour la préparation d'un réactif de détection et éventuellement de génotypage/sérotypage, d'un coronavirus associé au SRAS.

Les protéines et les peptides selon l'invention, qui sont aptes à être reconnus et/ou à induire la production d'anticorps spécifiques du coronavirus associé au SRAS, sont utiles pour le diagnostic de l'infection par un tel coronavirus ; l'infection est détectée, par une technique appropriée- notamment EIA, ELISA, RIA, immunofluorescence-, à partir d'un échantillon biologique prélevé chez un individu susceptible d'être infecté.

Selon une disposition avantageuse de ladite utilisation, lesdites protéines sont sélectionnées dans le groupe constitué par les protéines S, E, M et/ou N et les peptides tels que définis ci-dessus.

Les protéines S, E, M et/ou N et les peptides dérivés de ces protéines tels que définis ci-dessus, par exemple la protéine N, sont utilisées pour le diagnostic indirect d'une infection à coronavirus associé au SRAS (diagnostic sérologique ; détection d'anticorps spécifiques du SARS-CoV), notamment par une méthode immunoenzymatique (ELISA).

Les anticorps et les fragments d'anticorps selon l'invention, notamment ceux dirigés contre les protéines S, E, M et/ou N et les peptides dérivés tels que définis ci-dessus, sont utiles pour le diagnostic direct d'une infection à coronavirus associé au SRAS ; la détection de protéine(s) du SARS-CoV est réalisée par une technique appropriée, notamment EIA, ELISA, RIA, immunofluorescence à partir d'un échantillon biologique prélevé chez un individu susceptible d'être infecté.

La présente invention a également pour objet une méthode de détection d'un coronavirus associé au SRAS, à partir d'un échantillon biologique, laquelle méthode est **caractérisée en ce qu**'elle comprend au moins :
(a) la mise en contact dudit échantillon biologique avec au moins un anticorps ou un fragment d'anticorps, une protéine, un peptide ou bien une puce ou un filtre à protéine ou à peptide tels que définis ci-dessus, et
(b) la révélation par tout moyen approprié des complexes antigène-anticorps formés en (a), par exemple par EIA, ELISA, RIA, ou par immunofluorescence.

Selon un mode de mise en oeuvre avantageux dudit procédé l'étape (a) comprend :
(a₁) la mise en contact dudit échantillon biologique avec au moins un premier anticorps ou un fragment d'anticorps qui est fixé sur un support approprié, notamment une microplaque,
(a₂) le lavage de la phase solide, et
(a₃) l'addition d'au moins un second anticorps ou un fragment d'anticorps, différent du premier, ledit anticorps ou fragment d'anticorps étant éventuellement marqué de façon appropriée.

Ce procédé qui permet de capturer les particules virales présentes dans l'échantillon biologique est également dénommé procédé d'immunocapture.

Par exemple :
- l'étape (a₁) est réalisée avec au moins un premier anticorps monoclonal ou polyclonal ou un fragment de ceux-ci, dirigé contre la protéine S, M, et/ou E, et/ou un peptide correspondant à l'ectodomaine de l'une de ces protéines (peptides M2-14 ou E1-12)
- l'étape (a₃) est réalisée avec au moins un anticorps ou un fragment d'anticorps dirigé contre un autre épitope de la même protéine ou de préférence contre une autre protéine, de manière préférée contre une protéine interne telle que la nucléoprotéine N ou l'endodomaine de la protéine E ou M, de manière encore plus préférée il s'agit d'anticorps ou de fragments d'anticorps dirigés contre la protéine N qui est très abondante dans la particule virale ; lorsqu'un anticorps ou un fragment d'anticorps dirigé contre une protéine interne (N) ou contre l'endodomaine des protéines E ou M est utilisé, le dit anticorps est incubé en présence de détergent, comme le Tween 20 par exemple, à des concentrations de l'ordre de 0,1 %.
- l'étape (b) de révélation des complexes antigène-anticorps formés est réalisée, soit directement à l'aide d'un second anticorps marqué par exemple avec de la biotine ou une enzyme appropriée telle que la peroxydase ou la phosphatase alcaline, soit indirectement à l'aide d'un sérum anti-immunoglobulines marqué comme ci-dessus. Les complexes ainsi formés sont révélés à l'aide d'un substrat approprié.

Selon une mise en oeuvre préférée de cet aspect de l'invention, l'échantillon biologique est mélangé à l'anticorps monoclonal de révélation préàlablement à sa mise en contact avec les anticorps monoclonaux de capture. Le cas échéant, le mélange sérum-anticorps de révélation est incubé pendant au moins 10 minutes à température ambiante avant d'être appliqué sur la plaque.

La présente invention a également pour objet un test d'immunocapture destiné à détecter une infection par le coronavirus associé au SRAS par détection de la nucléoprotéine native (protéine N), en particulier **caractérisé en ce qu**e l'anticorps utilisé pour la capture de la nucléoprotéine virale native est un anticorps monoclonal spécifique de la région centrale et/ou d'un épitope conformationnel.

Selon un mode de réalisation dudit test, l'anticorps utilisé pour la capture de la protéine N est l'anticorps monoclonal Acm87, produit par l'hybridome déposé à la CNCM le 1^{er} décembre 2004 sous le numéro I-3328.

Selon un autre mode de réalisation dudit test d'immunocapture, l'anticorps utilisé pour la capture de la protéine N est l'anticorps monoclonal Acm86, produit par l'hybridome déposé à la CNCM le 1^{er} décembre 2004 sous le numéro I-3329.

Selon un autre mode de réalisation dudit test d'immunocapture, les anticorps monoclonaux Acm86 et Acm87 sont utilisés pour la capture de la protéine N.

Dans les tests d'immunocapture selon l'invention, on peut utiliser pour la révélation de la protéine N, l'anticorps monoclonal Acm57, produit par l'hybridome déposé à la CNCM le 1^{er} décembre 2004 sous le numéro I-3330, ledit anticorps étant conjugué à une molécule ou une particule révélatrice.

Conformément audit test d'immunocapture, une combinaison des anticorps Acm57 et Acm87, conjugués à une molécule ou une particule révélatrice, est utilisée pour la révélation de la protéine N.

Une molécule révélatrice peut être un atome radioactif, un colorant, une molécule fluorescente, un fluorophore, une enzyme ; une particule révélatrice peut être, par exemple : de l'or colloïdal, une particule magnétique ou une bille de latex.

La présente invention a également pur objet un réactif de détection d'un coronavirus associé au SRAS, **caractérisé en ce qu**'il est sélectionné dans le groupe constitué par :
(a) une paire d'amorces ou une sonde telles que définies ci-dessus,
(b) un vecteur recombinant tel que défini ci-dessus ou une cellule modifiée telle que définie ci-dessus,
(c) une souche isolée de coronavirus telle que définie ci-dessus ou un polynucléotide tel que défini ci-dessus,
(d) un anticorps ou un fragment d'anticorps tel que défini ci-dessus,
(e) une combinaison d'anticorps comprenant les anticorps monoclonaux Acm86 et/ou Acm87, et l'anticorps monoclonal Acm57, telle que définie ci-dessus,
(f) une puce ou un filtre tels que définis ci-dessus.

La présente invention a également pour objet une méthode de détection d'une infection par un coronavirus associé au SRAS, à partir d'un échantillon biologique, par ELISA IgG indirect utilisant la protéine N, laquelle méthode est **caractérisée en ce que** les plaques sont sensibilisées par une solution de protéine N à une concentration comprise entre 0,5 et 4µg/mL, de préférence 2µg/mL, dans un tampon PBS 10mM pH 7,2, rouge de phénol à 0,25mL /L.

La présente invention a, en outre, pour objet une méthode de détection d'une infection par un coronavirus associé au SRAS, à partir d'un échantillon biologique, par ELISA double épitope, **caractérisée en ce que** le sérum à tester est mélangé à l'antigène de révélation, ledit mélange étant ensuite mis au contact de l'antigène fixé sur un support solide.

Selon une variante des tests de détection des coronavirus associé au SRAS, ces tests combinent un ELSA utilisant la protéine N, et un autre ELSA utilisant la protéine S, tel que décrit plus bas.

La présente invention a aussi pour objet un complexe immun formé d'un anticorps ou d'un fragment d'anticorps polyclonal ou monoclonal tel que défini ci-dessus, et d'une protéine ou d'un peptide du coronavirus associé au SRAS.

La présente invention a en outre pour objet un kit de détection d'un coronavirus associé au SRAS, **caractérisé en ce qu**'il comprend au moins un réactif sélectionné dans le groupe constitué par : une paire d'amorces, une sonde, une puce à ADN ou à ARN, un vecteur recombinant, une cellule modifiée, une souche isolée de coronavirus, un polynucléotide, une protéine ou un peptide, un anticorps, et une puce à protéine tels que définis ci-dessus.

La présente invention a en outre pour objet, une composition immunogène, **caractérisée en ce qu**'elle comprend au moins un produit sélectionné dans le groupe constitué par :
a) une protéine ou un peptide tels que définis ci-dessus,
b) un polynucléotide de type ADN ou ARN ou l'un de ses fragments représentatifs tels que définis ci-dessus, de séquence choisie parmi :
   (i) la séquence SEQ ID NO : 1 ou son équivalent ARN
   (ii) la séquence hybridant dans des conditions de forte stringence avec la séquence SEQ ID NO : 1,
   (iii) la séquence complémentaire de la séquence SEQ ID NO : 1 ou de la séquence hybridant dans des conditions de forte stringence avec la séquence SEQ ID NO : 1,
   (iv) la séquence nucléotidique d'un fragment représentatif du polynucléotide tel que défini en (i), (ii) ou (iii),
   (v) la séquence telle que définie en (i), (ii), (iii) ou (iv), modifiée, et
c) un vecteur d'expression recombinant comprenant un polynucléotide tel que défini en b), et
d) une banque d'ADNc telle que définie ci-dessus, ladite composition immunogène étant capable d'induire une immunité humorale ou cellulaire protectrice spécifique du coronavirus associé au SRAS, notamment la production d'un anticorps dirigé contre un épitope spécifique du coronavirus associé au SRAS.

Les protéines et les peptides tels que définis ci-dessus, notamment les protéines S, M, E et/ou N et les peptides dérivés, ainsi que les molécules d'acide nucléique (ADN ou ARN) codant lesdites protéines ou lesdits peptides, sont de bons candidats vaccin et peuvent être utilisées dans des compositions immunogènes pour la production d'un vaccin contre le coronavirus associé au SRAS.

Selon un mode de réalisation avantageux des compositions selon l'invention, elles contiennent en outre, au moins un véhicule pharmaceutiquement acceptable et éventuellement des substances porteuses et/ou des adjuvants.

Les véhicules pharmaceutiquement acceptables, les substances porteuses et les adjuvants sont ceux classiquement utilisés.

Les adjuvants sont avantageusement choisis dans le groupe constitué par des émulsions huileuses, de la saponine, des substances minérales, des extraits bactériens, de l'hydroxyde d'alumine et le squalène.

Les substances porteuses sont avantageusement sélectionnées dans le groupe constitué par des liposomes unilamellaires, des liposomes multilamellaires, des micelles de saponine ou des microsphères solides de nature saccharidique ou aurifère.

Les compositions selon l'invention, sont administrées par voie générale, notamment intramusculaire ou sous-cutanée ou bien par voie locale notamment nasale (aérosol).

La présente invention a également pour objet l'utilisation d'une protéine ou d'un peptide isolé ou purifié présentant une séquence sélectionnée dans le groupe constitué par les séquences SEQ ID NO : 3, 10, 12, 14, 17, 22, 24, 26, 28, 30, 33, 35, 37, 69, 70, 71, 74 et 75 pour former un complexe immun avec un anticorps dirigé spécifiquement contre un épitope du coronavirus associé au SRAS.

La présente invention a également pour objet un complexe immun formé d'une protéine ou d'un peptide isolé ou purifié présentant une séquence sélectionnée dans le groupe constitué par les séquences SEQ ID NO : 3, 10, 12, 14, 17, 22, 24, 26, 28, 30, 33, 35, 37, 69, 70, 71, 74 et 75, et d'un anticorps dirigé spécifiquement contre un épitope du coronavirus associé au SRAS.

La présente invention a également pour objet l'utilisation d'une protéine ou d'un peptide isolé ou purifié présentant une séquence sélectionnée dans le groupe constitué par les séquences SEQ ID NO : 3, 10, 12, 14, 17, 22, 24, 26, 28, 30, 33, 35, 37, 69, 70, 71, 74 et 75 pour induire la production d'un anticorps capable de reconnaître spécifiquement un épitope du coronavirus associé au SRAS.

La présente invention a également pour objet l'utilisation d'un polynucléotide isolé ou purifié présentant une séquence sélectionnée dans le groupe constitué par les séquences SEQ ID NO : 1, 2, 4, 7, 8, 13, 15, 16, 18, 19, 20, 31, 36 et 38 pour induire la production d'un anticorps dirigé contre la protéine codée par ledit polynucléotide et capable de reconnaître spécifiquement un épitope du coronavirus associé au SRAS

La présente invention a également pour objet des anticorps monoclonaux reconnaissant la protéine S native d'un coronavirus associé au SRAS.

La présente invention a également pour objet l'utilisation d'une protéine ou d'un polypeptide de la famille de la protéine S, telle que définie ci-dessus, ou d'un anticorps reconnaissant la protéine S native, tel que défini ci-dessus, pour détecter une infection par un coronavirus associé au SRAS, à partir d'un échantillon biologique.

La présente invention a également pour objet une méthode de détection d'une infection par un coronavirus associé au SRAS, à partir d'un échantillon biologique, **caractérisée en ce que** la détection est effectuée par ELISA utilisant la protéine S recombinante, exprimée dans un système eucaryote.

Selon un mode de mise en oeuvre avantageux de ladite méthode, il s'agit d'une méthode par ELISA double épitope, et le sérum à tester est mélangé à l'antigène de révélation, ledit mélange étant ensuite mis au contact de l'antigène fixé sur un support solide.

La présente invention a aussi pour objet un complexe immun formé d'un anticorps ou d'un fragment d'anticorps monoclonal reconnaissant la protéine S native, et d'une protéine ou d'un peptide du coronavirus associé au SRAS.

La présente invention a également pour objet un complexe immun formé d'une protéine ou d'un polypeptide de la famille de la protéine S, telle que définie ci-dessus, et d'un anticorps dirigé spécifiquement contre un épitope du coronavirus associé au SRAS.

La présente invention a en outre pour objet un kit ou coffret de détection d'un coronavirus associé au SRAS, **caractérisé en ce qu**'il comprend au moins un réactif sélectionné dans le groupe constitué par : une protéine ou polypeptide de la famille de la protéine S, telle que définie ci-dessus, un acide nucléique codant pour une protéine ou polypeptide de la famille de la protéine S, telle que définie ci-dessus, une cellule exprimant une protéine ou polypeptide de la famille de la protéine S, telle que définie ci-dessus, ou un anticorps reconnaissant la protéine S native d'un coronavirus associé au SRAS.

La présente invention a pour objet une composition immunogène et/ou vaccinale, **caractérisée en ce qu**'elle comprend un polypeptide ou une protéine recombinante de la famille de la protéine S, telle que définie ci-dessus, obtenu dans un système d'expression eucaryote.

La présente invention a également pour objet une composition immunogène et/ou vaccinale, **caractérisée en ce qu**'elle comprend un vecteur ou virus recombinant, exprimant une protéine ou un polypeptide de la famille de la protéine S, telle que définie ci-dessus.

Outre les dispositions qui précèdent, l'invention comprend encore d'autres dispositions, qui ressortiront de la description qui va suivre, qui se réfère à des exemples de mise en oeuvre du polynucléotide représentant le génome de la souche de SARS-CoV issue du prélèvement répertorié sous le numéro 031589, et des fragments d'ADNc dérivés objets de la présente invention, ainsi qu'au Tableau 1 présentant la liste des séquences :

**Tableau I : Liste des séquences**

| **numéro d'identification** | **Séquence** | **Position de l'ADNc en référence à Genbank AY274119.3** | **Numéro de dépôt à la CNCM du plasmide correspondant** |
|---|---|---|---|
| SEQ ID NO : 1 | génome de la souche issue du prélèvement 031589 | - | - |
| SEQ ID NO : 2 | ORF-S* | 21406-25348 | - |
| SEQ ID NO : 3 | Protéine S | - | - |
| SEQ ID NO : 4 | ORF-S** | 21406-25348 | I-3059 |
| SEQ ID NO : 5 | fragment Sa | 21406-23454 | I-3020 |
| SEQ ID NO : 6 | fragment Sb | 23322-25348 | I-3019 |
| SEQ ID NO : 7 | ORF-3+ORF-4* | 25110-26244 | - |
| SEQ ID NO : 8 | ORF-3+ORF-4** | 25110-26244 | I-3126 |
| SEQ ID NO : 9 | ORF3 | - | - |
| SEQ ID NO : 10 | Protéine ORF-3 | - | - |
| SEQ ID NO : 11 | ORF4 | - | - |
| SEQ ID NO : 12 | Protéine ORF-4 | - | - |
| SEQ ID NO : 13 | ORF-E* | 26082-26413 | - |
| SEQ ID NO : 14 | Protéine E | - | - |
| SEQ ID NO : 15 | ORF-E** | 26082-26413 | I-3046 |
| SEQ ID NO : 16 | ORF-M* | 26330-27098 | - |
| SEQ ID NO : 17 | Protéine M | - | - |
| SEQ ID NO : 18 | ORF-M** | 26330-27098 | I-3047 |
| SEQ ID NO : 19 | ORF7 à 11* | 26977-28218 | - |
| SEQ ID NO : 20 | ORF7 à 11** | 26977-28218 | I-3125 |
| SEQ ID NO : 21 | ORF7 | - | - |
| SEQ ID NO : 22 | Protéine ORF7 | - | - |
| SEQ ID NO : 23 | ORF8 | - | - |
| SEQ ID NO : 24 | Protéine ORF8 | - | - |
| SEQ ID NO : 25 | ORF9 | - | - |
| SEQ ID NO : 26 | Protéine ORF9 | - | - |
| SEQ ID NO : 27 | ORF10 | - | - |
| SEQ ID NO : 28 | Protéine ORF10 | - | - |
| SEQ ID NO : 29 | ORF11 | - | - |
| SEQ ID NO : 30 | Protéine ORF11 | - | - |
| SEQ ID NO: 31 | OrF1ab | 265-21485 | - |
| SEQ ID NO : 32 | ORF13 | 28130-28426 | - |
| SEQ ID NO : 33 | Protéine ORF13 | - | - |
| SEQ ID NO : 34 | ORF14 | - | - |
| SEQ ID NO : 35 | Protéine ORF14 | 28583-28795 | - |
| SEQ ID NO : 36 | ORF-N* | 28054-29430 | - |
| SEQ ID NO : 37 | Protéine N | - | - |
| SEQ ID NO : 38 | ORF-N** | 28054-29430 | I-3048 |
| SEQ ID NO : 39 | 5'non-codante** | 1-204 | I-3124 |
| SEQ ID NO : 40 | 3'non-codante** | 28933-29727 | I-3123 |
| SEQ ID NO : 41 | **ORF1ab** Fragment L0 | 30-500 | - |
| SEQ ID NO : 42 | Fragment L1 | 211-2260 | - |
| SEQ ID NO : 43 | Fragment L2 | 2136-4187 | - |
| SEQ ID NO : 44 | Fragment L3 | 3892-5344 | - |
| SEQ ID NO : 45 | Fragment L4b | 4932-6043 | - |
| SEQ ID NO : 46 | Fragment L4 | 5305-7318 | - |
| SEQ ID NO : 47 | Fragment L5 | 7275-9176 | - |
| SEQ ID NO : 48 | Fragment L6 | 9032-11086 | - |
| SEQ ID NO : 49 | Fragment L7 | 10298-12982 | - |
| SEQ ID NO : 50 | Fragment L8 | 12815-14854 | - |
| SEQ ID NO : 51 | Fragment L9 | 14745-16646 | - |
| SEQ ID NO : 52 | Fragment L10 | 16514-18590 | - |
| SEQ ID NO : 53 | Fragment L11 | 18500-20602 | - |
| SEQ ID NO : 54 | Fragment L12 | 20319-22224 | - |
| SEQ ID NO : 55 | Amorce N sens | - | - |
| SEQ ID NO : 56 | Amorce N antisens | - | - |
| SEQ ID NO : 57 | Amorce S_{C} sens | - | - |
| SEQ ID NO : 58 | Amorce S_{L} sens | - | - |
| SEQ ID NO : 59 | Amorce S_{C} e t S_{L} antisens | - | - |
| SEQ ID NO : 60 | Amorce sens série 1 | 28507-28522 | - |
| SEQ ID NO : 61 | Amorce antisens série 1 | 28774-28759 | - |
| SEQ ID NO : 62 | Amorce sens série 2 | 28375-28390 | - |
| SEQ ID NO : 63 | Amorce antisens série 2 | 28702-28687 | - |
| SEQ ID NO : 64 | Sonde 1/série 1 | 28561-28586 | - |
| SEQ ID NO : 65 | Sonde 2/série 1 | 28588-28608 | - |
| SEQ ID NO : 66 | Sonde 1/série 2 | 28541-28563 | - |
| SEQ ID NO : 67 | Sonde 2/série 2 | 28565-28589 | - |
| SEQ ID NO : 68 | Amorce ancre 14T | | |
| SEQ ID NO : 69 | Peptide M2-14 | - | - |
| SEQ ID NO : 70 | Peptide E1-12 | - | - |
| SEQ ID NO : 71 | Peptide E53-76 | - | - |
| SEQ ID NO : 72 | 5'non-codante* | 1-204 | - |
| SEQ ID NO : 73 | 3'non-codante* | 28933-29727 | - |
| SEQ ID NO : 74 | Protéine ORF1a | - | - |
| SEQ ID NO : 75 | Protéine ORF1b | - | - |
| SEQ ID NO:76-139 | Amorces | | |
| SEQ ID NO:140 | Pseudogène de S | | |
| SEQ ID NO:141-148 | amorces | | |
| SEQ ID NO:149 | Aa1-13 de S | | |
| SEQ ID NO:150 | polypeptide | | |
| SEQ ID NO:151-158 | amorces | | |

| | | | |
|---|---|---|---|
| * produit d'amplification PCR (amplicon) ** insert cloné dans le plasmide déposé à la CNCM | | | |

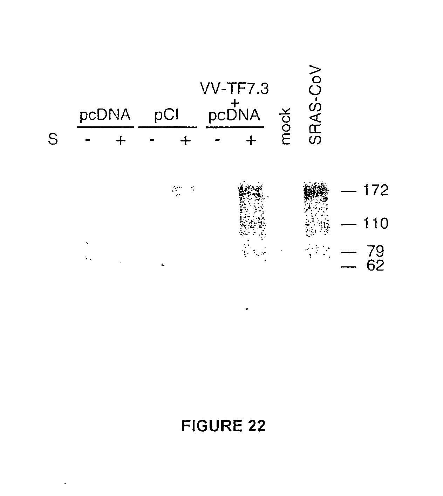

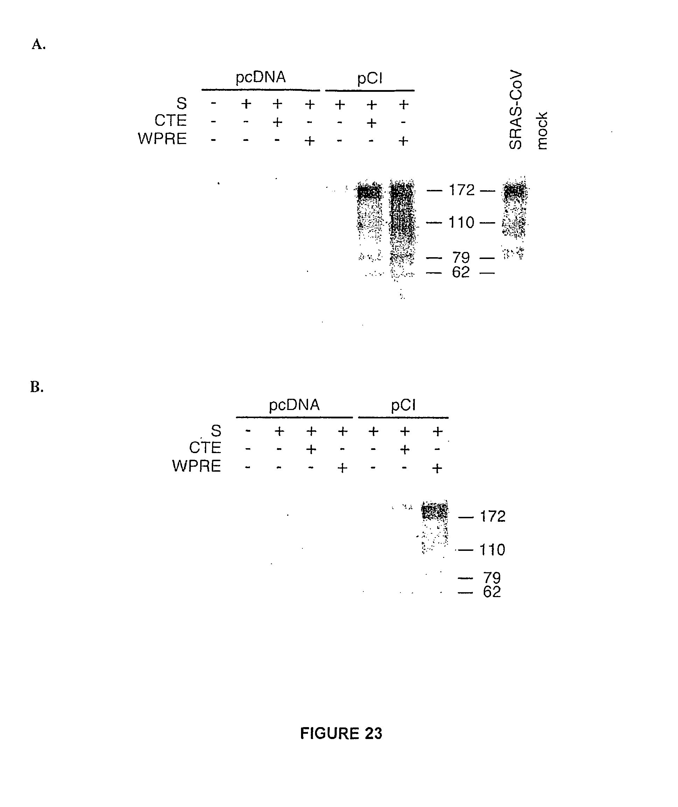

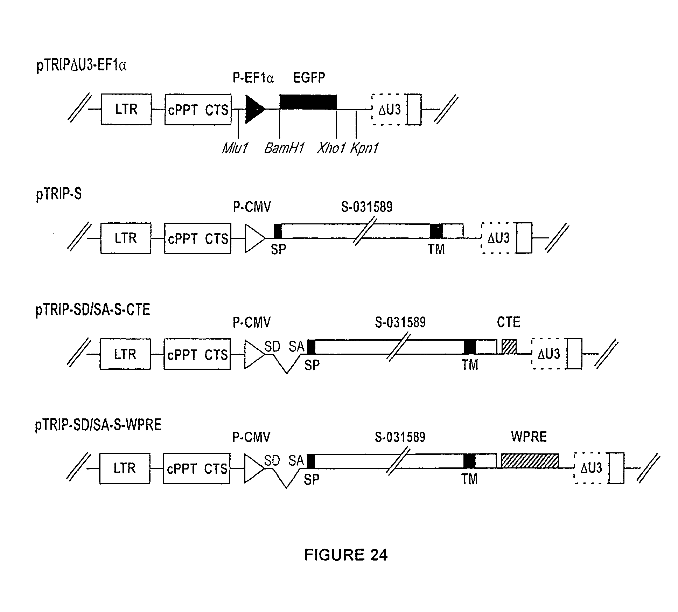

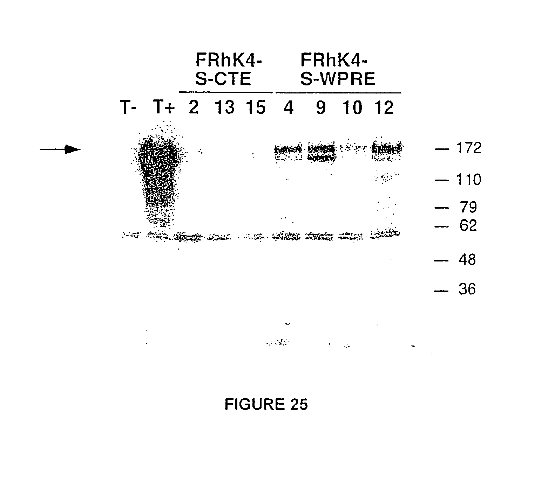

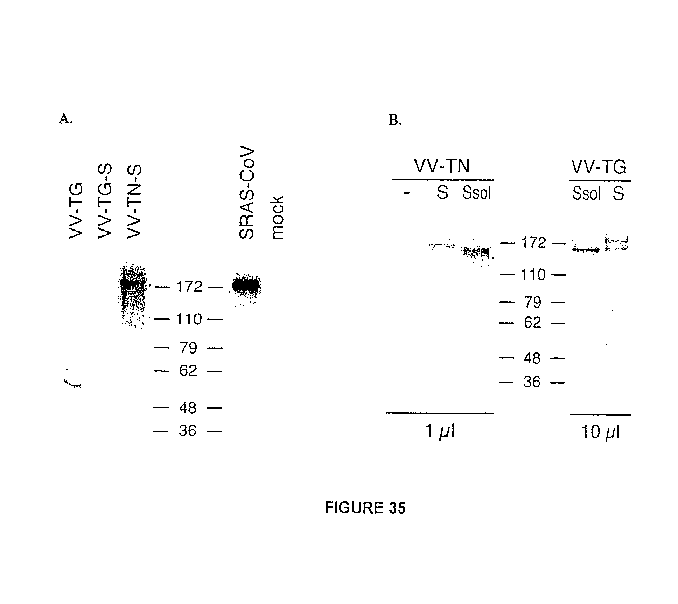

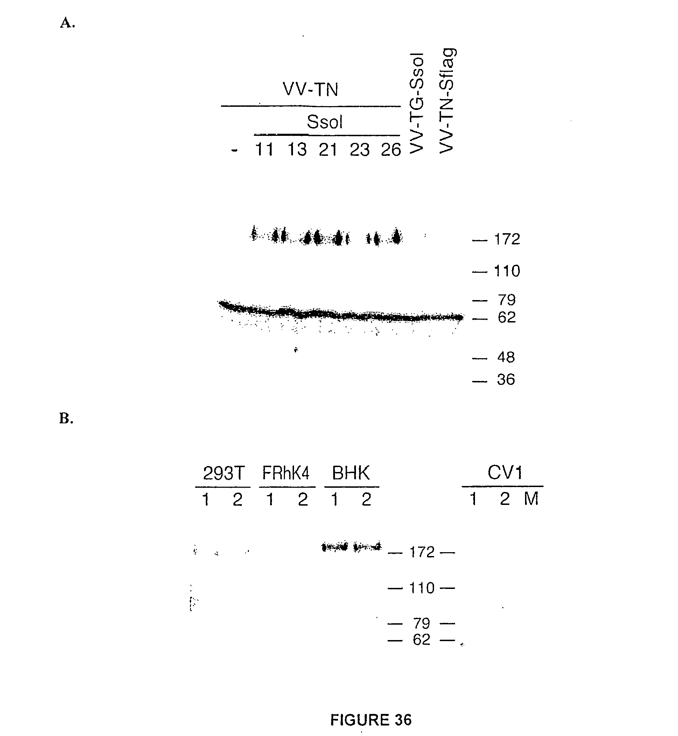

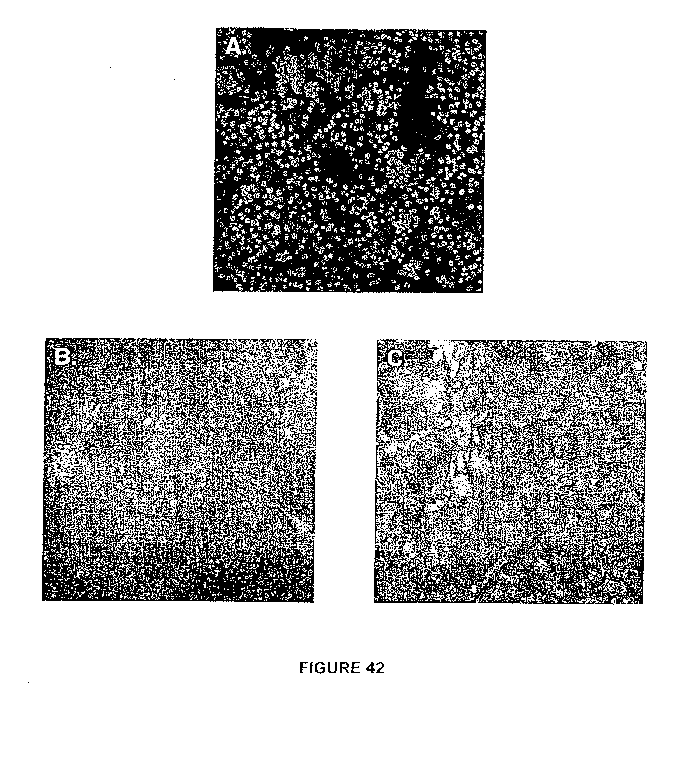

ainsi qu'aux dessins annexés dans lesquels :

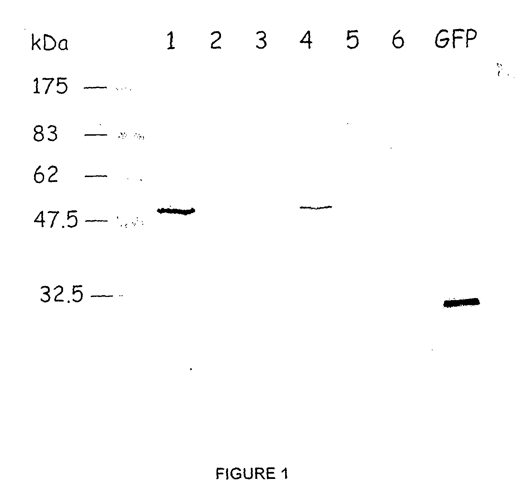

- la figure 1 illustre l'analyse par Western-blot de l'expression *in vitro* des protéines recombinantes N, S_{C} et S_{L} à partir des vecteurs d'expression pIVEX. Piste 1 : pIV2.3N. Piste 2 : pIV2.3S_{C}. Piste 3 : pIV2.3S_{L}. Piste 4 : pIV2.4N. Piste 5 : pIV2.4S_{I} ou pIV2.4S_{C}. Piste 6 : pIV2.4S_{L}. L'expression de la protéine GFP exprimée à partir du même vecteur est utilisée comme contrôle.

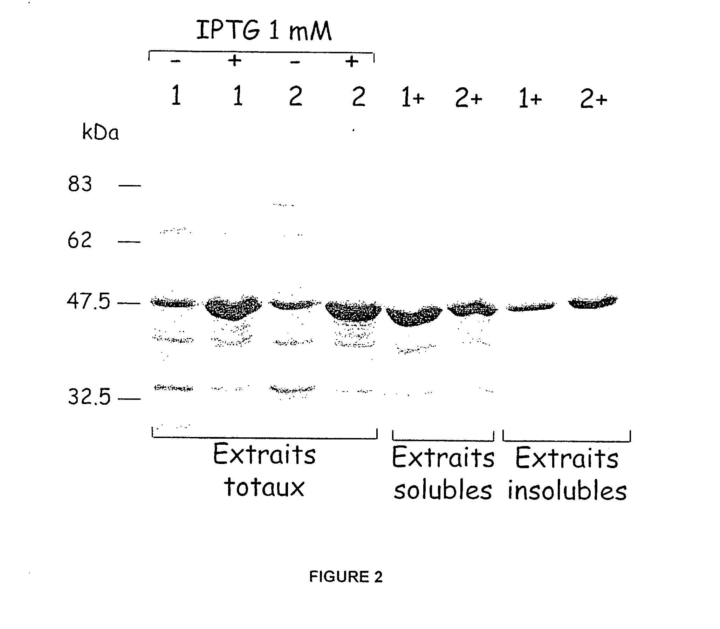

- la figure 2 illustre l'analyse par électrophorèse en gel de polyacrylamide en conditions dénaturantes (SDS-PAGE) et coloration au bleu de Coomassie, de l'expression *in vivo* de la protéine N à partir des vecteurs d'expression pIVEX. La souche d'*E.coli* BL21(DE3)pDIA17 transformée par les vecteurs pIVEX recombinants est cultivée à 30°C dans du milieu LB, en présence ou en l'absence d'inducteur (IPTG 1mM). Piste 1 : pIV2.3N Piste 2 : pIV2.4N.

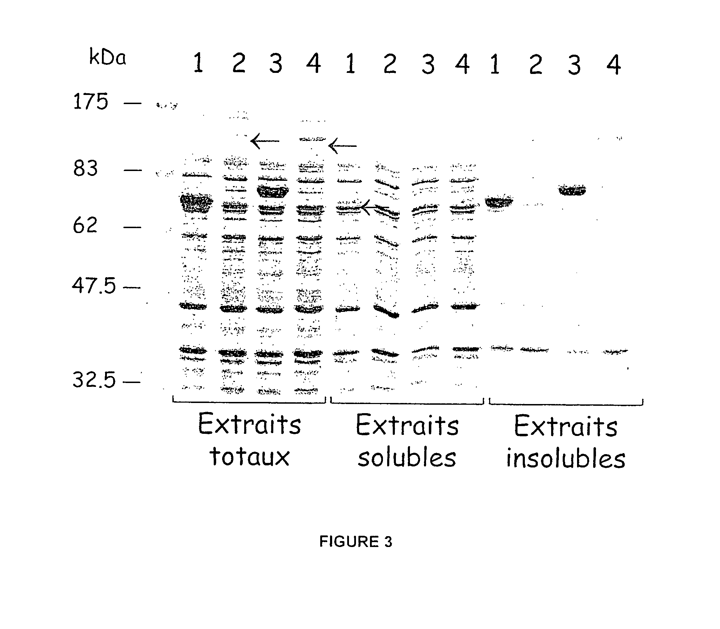

- la figure 3 illustre l'analyse par électrophorèse en gel de polyacrylamide en conditions dénaturantes (SDS-PAGE) et coloration au bleu de Coomassie, de l'expression *in vivo* des polypeptides S_{L} et S_{C} à partir des vecteurs d'expression pIVEX. La souche d'*E.coli* BL21(DE3)pDIA17 transformée par les vecteurs pIVEX recombinants est cultivée à 30°C dans du milieu LB, en présence ou en l'absence d'inducteur (IPTG 1mM). Piste 1 : pIV2.3S_{C} Piste 2 : pIV2.3S_{L}. Piste 3 : pIV2.4S_{I} Piste 4 : pIV2.4S_{L}.

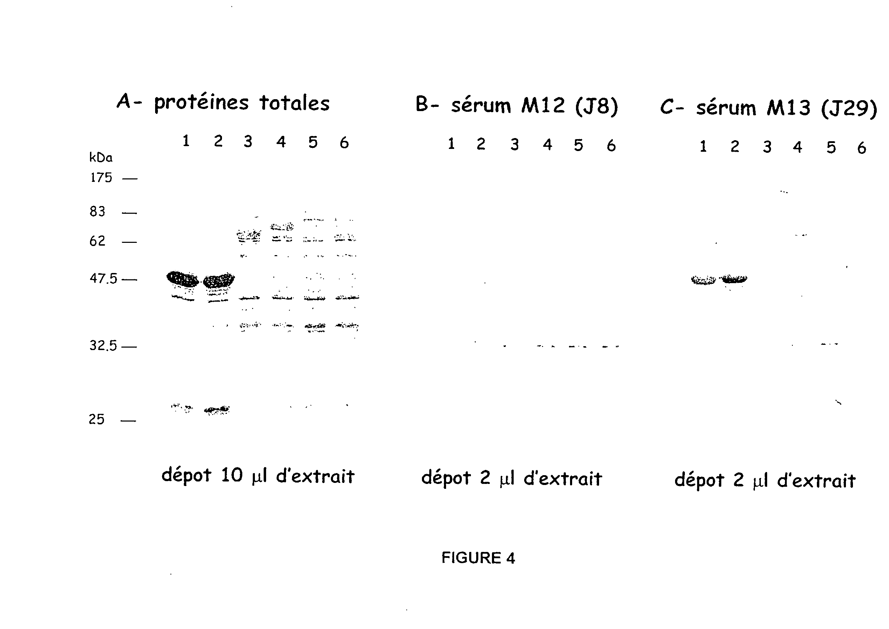

- la figure 4 illustre l'activité antigénique des protéines N, S_{L} et S_{C} recombinantes produites dans la souche *E. coli* BL21(DE3)pDIA17 transformée par les vecteurs pIVEX recombinants. A : électrophorèse (SDS-PAGE) des lysats bactériens. B et C : Western-blot avec les sérums, provenant d'un même patient infecté par le SARS-CoV, prélevés respectivement 8 jours (B : sérum M12) et 29 jours-(C : sérum M13) après le début des symptômes du SRAS. Piste 1 : pIV2.3N. Piste 2 : pIV2.4N. Piste 3 : pIV2.3S_{C}. Piste 4 : pIV2.4 S_{I}. Piste 5 : pIV2.3S_{L}. Piste 6 : pIV2.4S_{L}

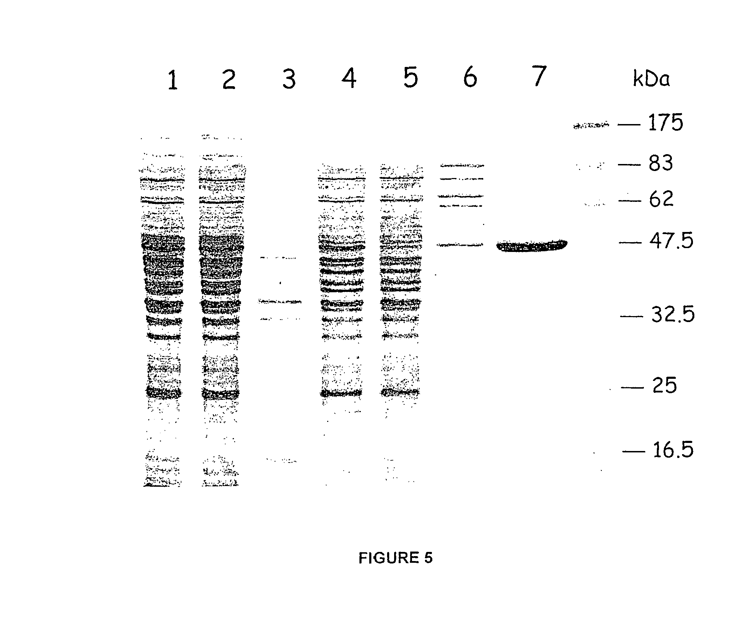

- la figure 5 illustre la purification sur colonne Ni-NTA agarose de la protéine N recombinante produite dans la souche *E. coli* BL21(DE3)pDIA17 à partir du vecteur pIV2.3N. Piste 1 : Extrait bactérien total. Piste 2 : Extrait soluble. Piste 3 : Extrait insoluble. Piste 4 : Extrait déposé sur la colonne Ni-NTA. Piste 5 : protéines non-retenues. Piste 6 : Fractions du pic 1. Piste 7 : Fractions du pic 2.

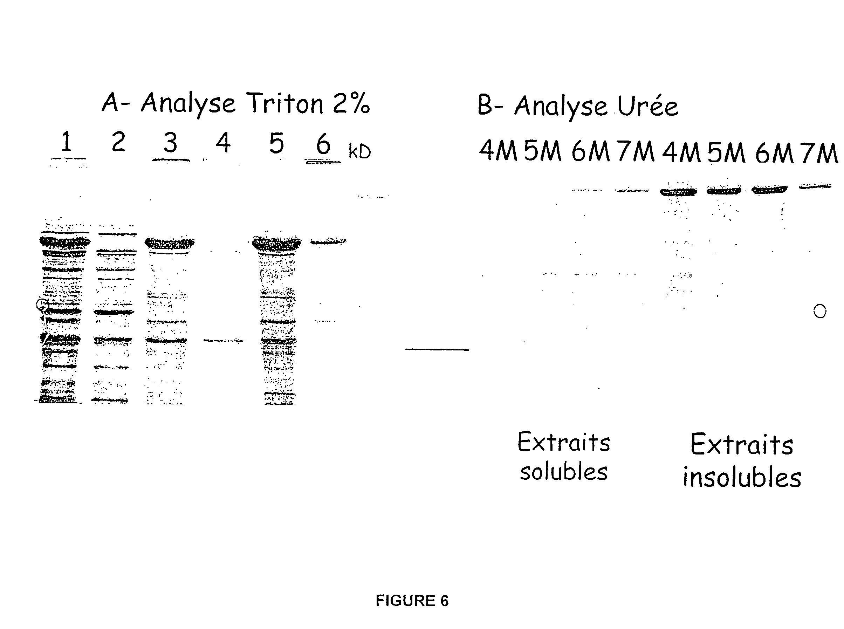

- la figure 6 illustre la purification de la protéine S_{C} recombinante à partir des corps d'inclusions produits dans la souche *E. coli* BL21(DE3)pDIA17 transformée par le pIV2.4S_{I}.A. Traitement au Triton X-100 (2%) : Piste 1 : Extrait bactérien total. Piste 2 : Extrait soluble. Piste 3 : Extrait insoluble. Piste 4 : Surnageant après traitement au Triton X-100 (2 %). Pistes 5 et 6 : Culot après traitement au Triton X-100 (2 %).B : Traitement à l'urée 4M, 5M, 6M et 7M des extraits solubles et insolubles.

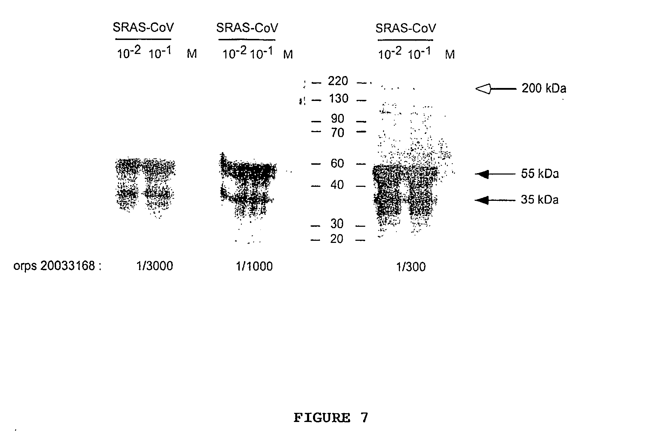

- la figure 7 représente l'immunoempreinte réalisée à l'aide d'un lysat de cellules infectées par le SARS-CoV et d'un sérum de patient atteint de pneumopathie atypique.

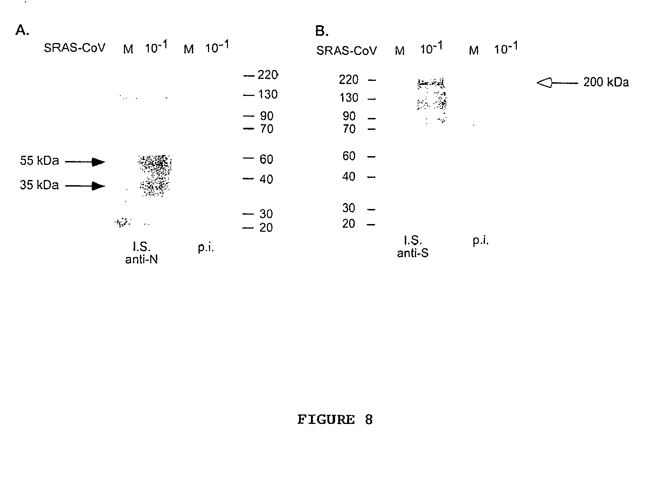

- la figure 8 représente des immunoempreintes réalisées à l'aide d'un lysat de cellules infectées par le SARS-CoV et d'immunsérums de lapins spécifiques de la nucléoprotéine N (A) et de la protéine de spicule S (B). I.S. : sérum immun. p.i. : sérum pré-immun. L'immunsérum anti-N a été utilisé au 1/50000 et l'immunsérum anti-S au 1/10000.

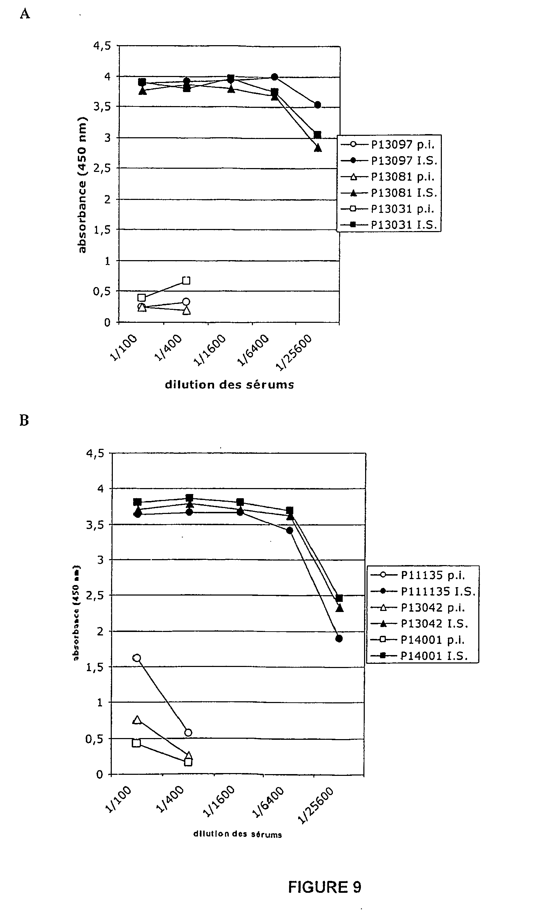

- la figure 9 illustre la réactivité en ELISA des sérums polyclonaux monospécifiques de lapin dirigés contre la protéine N ou le fragment court de la protéine S (S_{C}), vis-à-vis des protéines recombinantes correspondantes utilisées pour l'immunisation. A : lapins P13097, P13081, et P13031 immunisés avec la protéine N recombinante purifié. B : lapins P11135, P13042, et P14001 immunisés avec une préparation de corps d'inclusions correspondants au fragment court de la protéine S (S_{C}). I.S. : sérum immun. p.i. : sérum pré-immun.

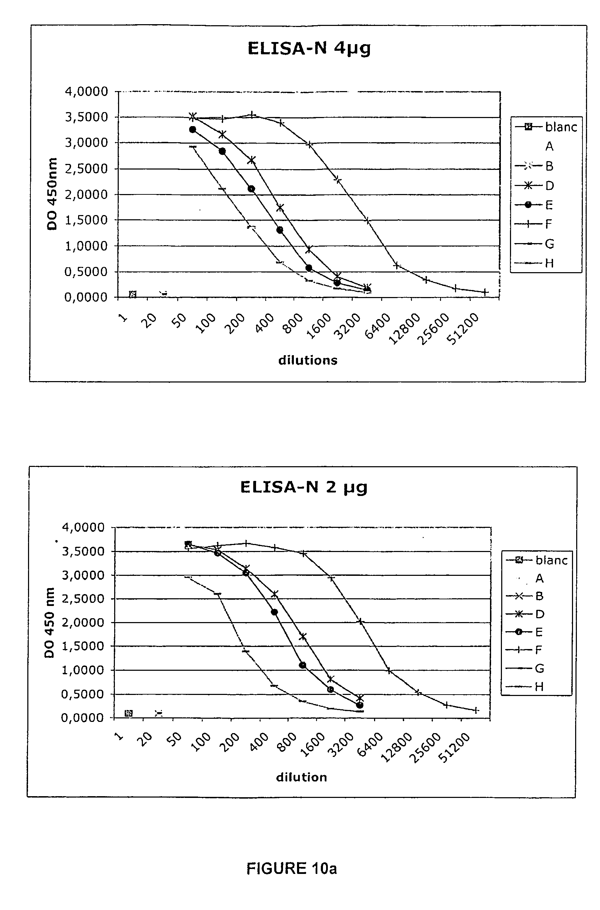

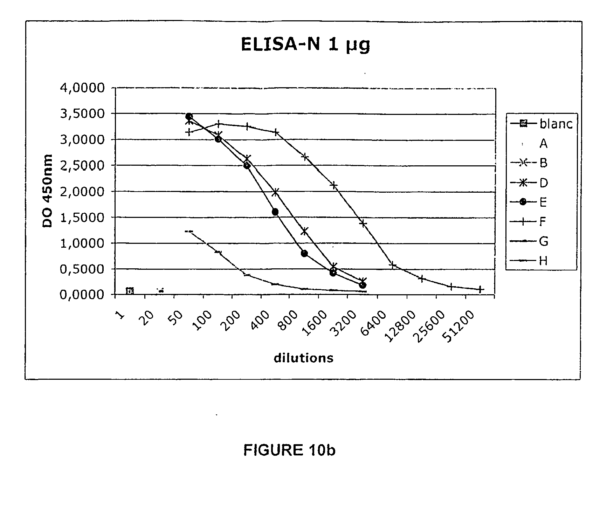

- la figure 10 illustre la réactivité en ELISA de la protéine N recombinante purifiée, vis-à-vis de sérum de patients atteints de pneumonie atypique causée par le SARS-CoV. Figure 10a : plaques ELISA préparés avec la protéine N à la concentration de 4 µg/ml et 2 µg/ml. Figure 10b : plaque ELISA préparée avec la protéine N à la concentration de 1 µg/ml. Les sérums désignés A, B, D, E, F, G, H correspondent à ceux du Tableau IV.

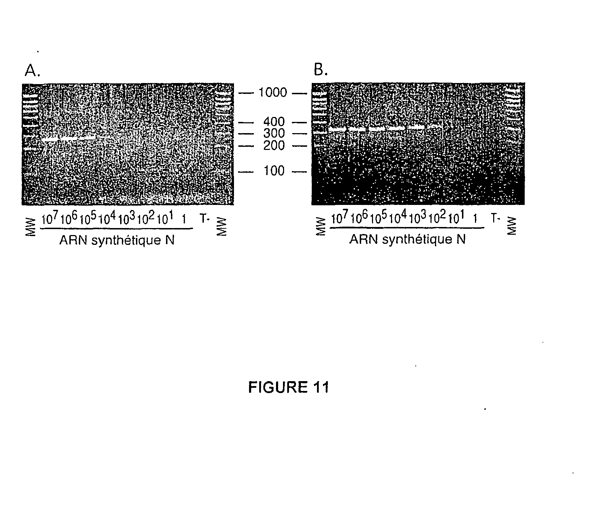

- la figure 11 illustre l'amplification par RT-PCR de quantités décroissantes d'ARN synthétique du gène N du SARS-CoV (10⁷ à 1 copie), à l'aide des couples d'amorces n° 1 (N/+/28507,N/-/28774) (A) et n° 2 (N/+/28375,N/-/28702) (B). T : amplification réalisée en l'absence d'ARN. MW : marqueur d'ADN.

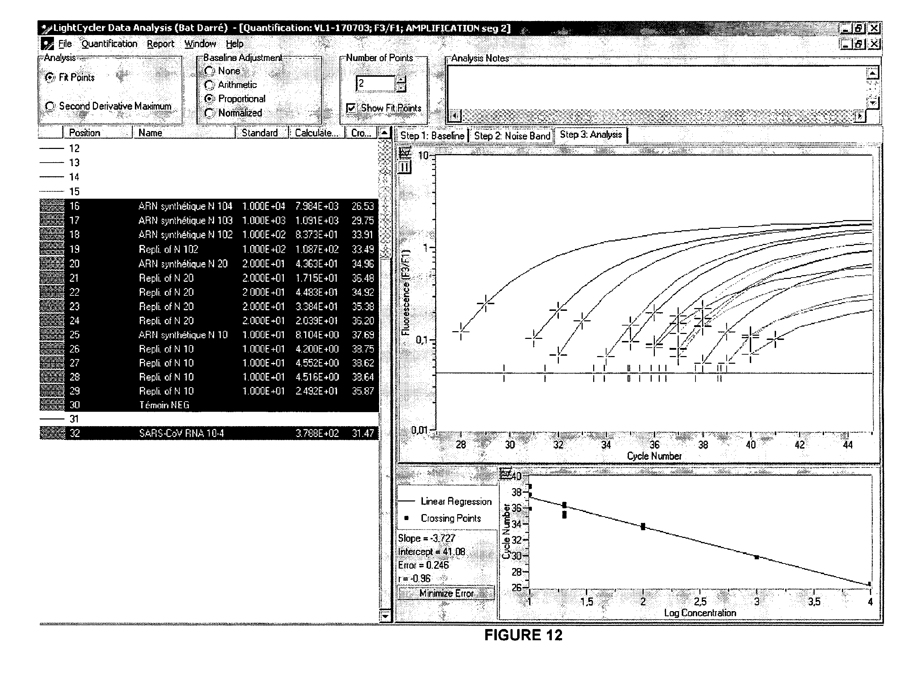

- la figure 12 illustre l'amplification par RT-PCR en temps réel d'ARN synthétique du gène N du SARS-CoV : des quantités décroissantes d'ARN synthétique en répliquat (repli. ; pistes 16 à 29) ainsi que de l'ARN viral dilué au 1/20x10⁻⁴ (piste 32) ont été amplifiés par RT-PCR en temps réel à l'aide du kit "Light Cycler RNA Amplification Kit Hybridization Probes" et des couples d'amorces et de sondes de la série n° 2, dans les conditions décrites à l'exemple 8.
- la figure 13 (figure 13) représente la carte de restriction de la séquence SEQ ID NO : 1 correspondant à l'équivalent ADN du génome de la souche de SARS-CoV issue du prélèvement répertorié sous le numéro 031589.

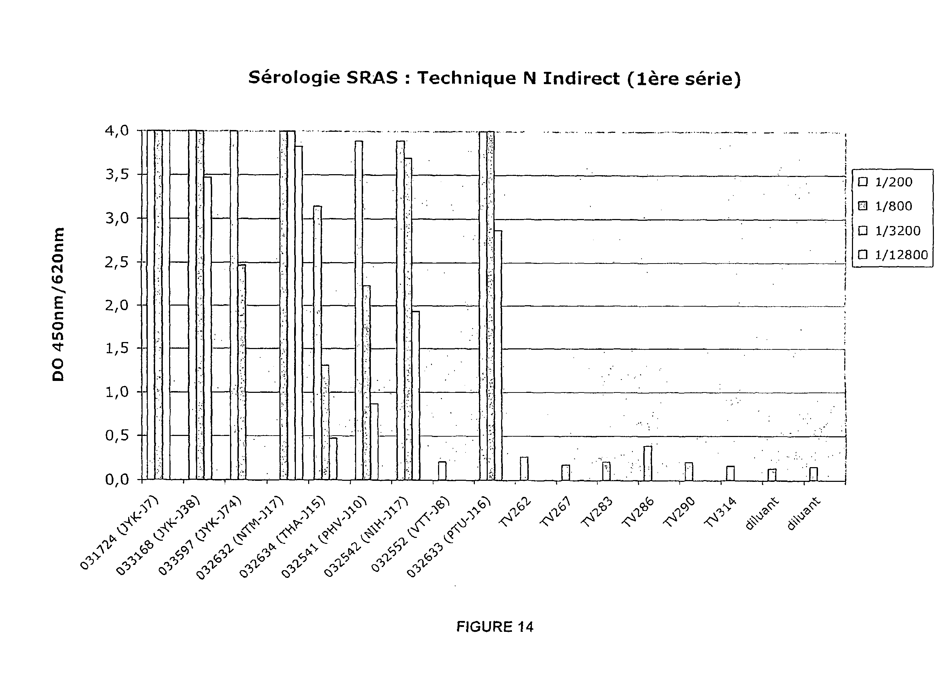

- la figure 14 montre le résultat du test de sérologie SRAS par ELISA N indirect (1ère série de sérums testés)

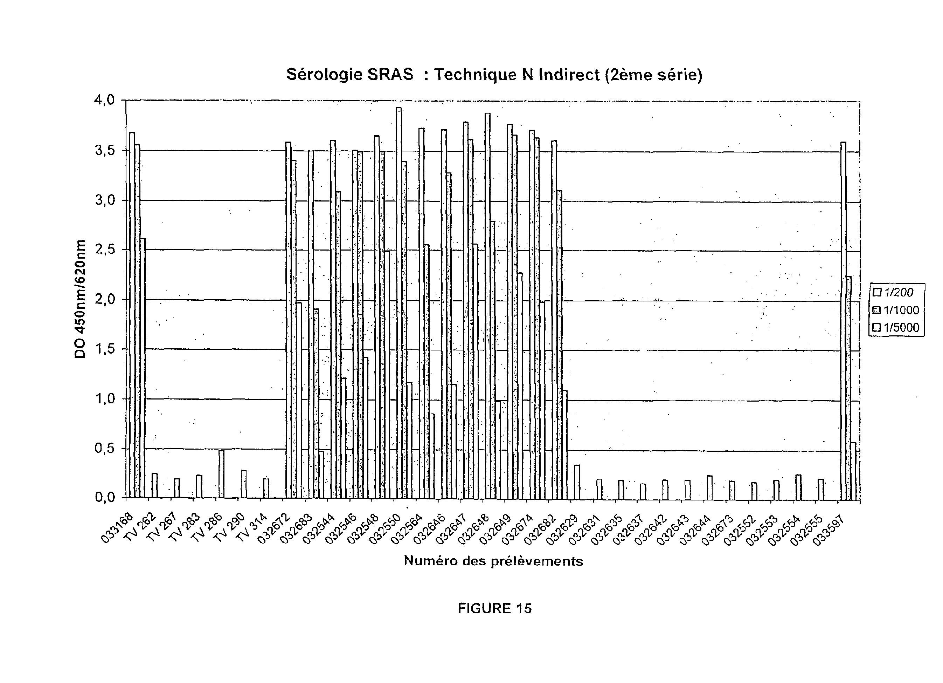

- la figure 15 montre le résultat du test de sérologie SRAS par ELISA N indirect (2ème série de sérums testés)

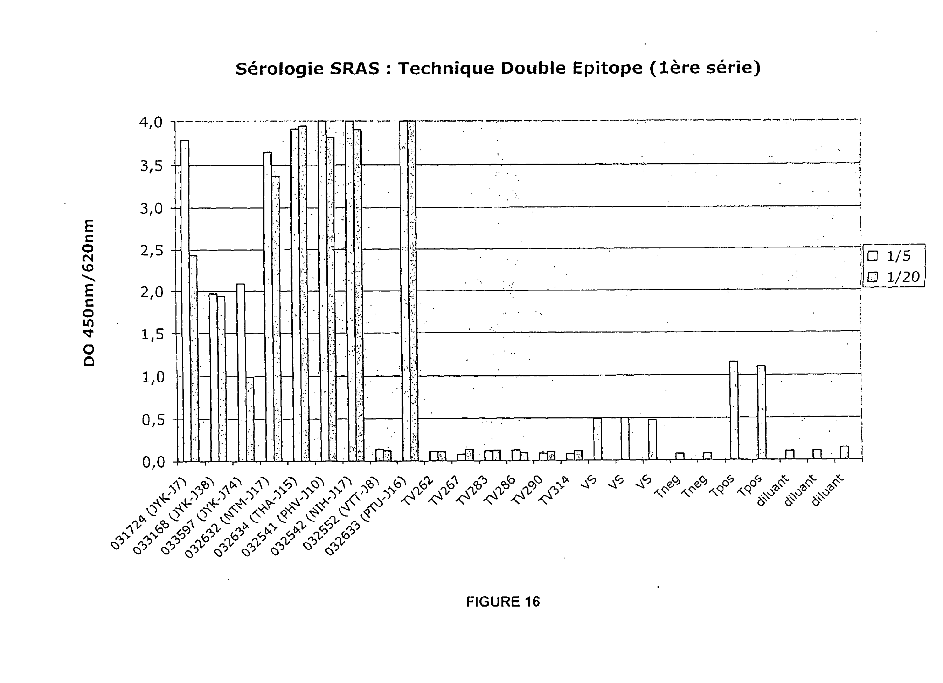

- la figure 16 présente le résultat du test de sérologie SRAS par ELISA N double épitope (1ère série de sérums testés)

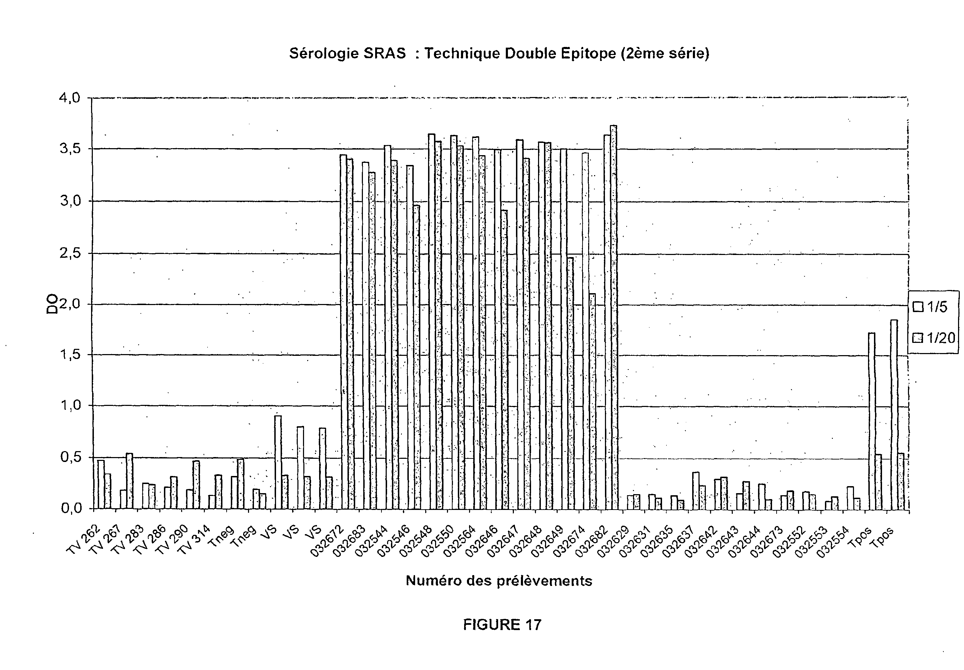

- la figure 17 montre le résultat du test de sérologie SRAS par ELISA N double épitope (2ème série de sérums testés)

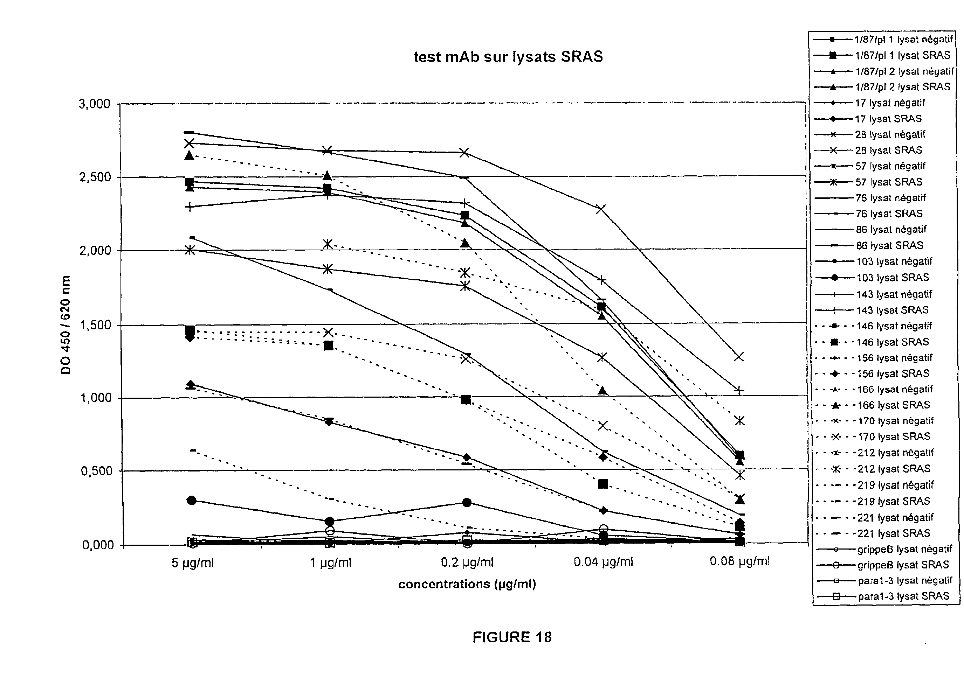

- la figure 18 illustre le test de réactivité des anticorps monoclonaux anti-N par ELISA sur la nucléoprotéine N native du SRAS-CoV. Les anticorps ont été testés sous la forme de surnageants de culture d'hybridomes par un ELISA indirect utilisant un lysat irradié de cellules VeroE6 infectées par le SRAS-CoV comme antigène (courbes lysat SRAS). Un contrôle négatif de réactivité est réalisé pour chaque anticorps sur un lysat de cellules VeroE6 non infectées (courbes lysat négatif). Plusieurs anticorps monoclonaux de spécificité connue ont été utilisés comme anticorps témoins négatifs : para1-3 dirigé contre les antigènes des virus parainfluenza de type 1-3 (Bio-Rad) et grippe B dirigé contre les antigènes du virus de la grippe de type B (Bio-Rad).

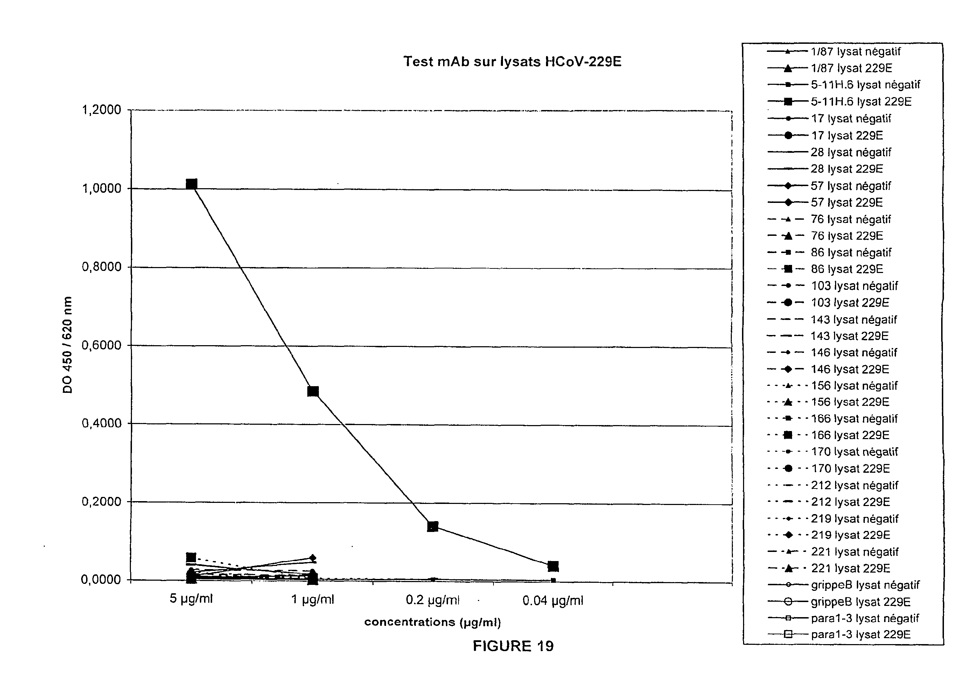

- la figure 19 illustre le test de réactivité des anticorps monoclonaux anti-N du SRAS-CoV par ELISA sur les antigènes natifs du coronavirus humain 229E (HCoV-229E). Les anticorps ont été testés sous la forme de surnageants de culture d'hybridomes par un test ELISA indirect utilisant un lysat de cellules MRC-5 infectées par le coronavirus humain 229E comme antigène (courbes lysat 229E). Un contrôle négatif d'immunoréactivité est réalisé pour chaque anticorps sur un lysat de cellules MRC-5 non infectées (courbes lysat négatif). L'anticorps monoclonal 5-11H.6 dirigé contre la protéine S du coronavirus humain 229E (Sizun et al. 1998, J. Virol. Met. 72 : 145-152) est utilisé comme anticorps témoin positif. Les anticorps para1-3 dirigé contre les antigènes des virus parainfluenza de type 1-3 (Bio-Rad) et grippe B dirigé contre les antigènes du virus de la grippe de type B (Bio-Rad) ont été ajoutés au panel des anticorps monoclonaux testés.

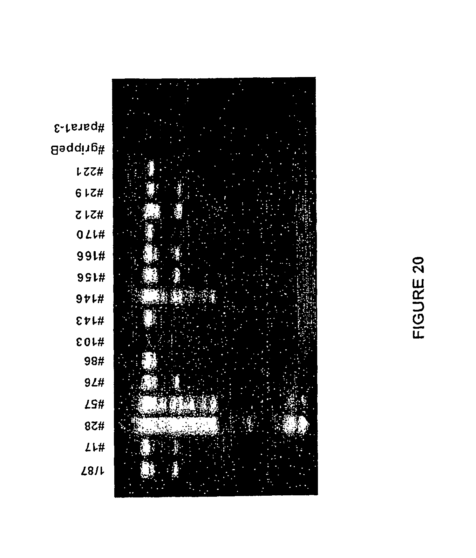

- la figure 20 montre un test de réactivité des anticorps monoclonaux anti-N du SRAS-CoV par western blot sur la nucléoprotéine N native du SRAS-CoV dénaturée. Un lysat de cellules VeroE6 infectées par le SRAS-CoV a été préparé dans le tampon de dépôt selon Laemmli et mis à migrer dans un gel SDS à 12% de polyacrylamide puis les protéines ont été transférées sur membrane de PVDF. Les anticorps monoclonaux anti-N testés ont été utilisés pour l'immunoessai à la concentration de 0.05 µg/ml. La révélation est faite avec des anticorps anti-IgG(H+L) de souris couplés à la peroxydase (NA93IV, Amersham) et le système ECL+. Deux anticorps monoclonaux ont été utilisés comme témoins négatifs de réactivité : grippe B dirigé contre les antigènes du virus de la grippe de type B (Bio-Rad) et para1-3 dirigé contre les antigènes des virus parainfluenza de type 1-3 (Bio-Rad).

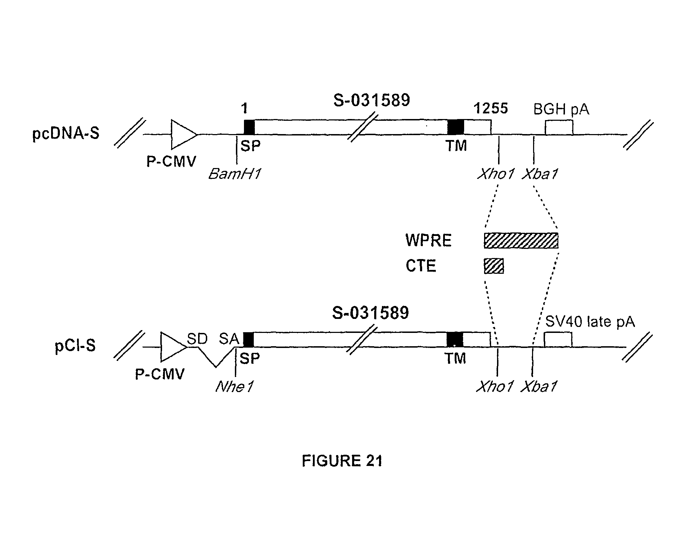

- la figure 21 présente les plasmides d'expression en cellules de mammifères de la protéine S du SRAS-CoV. Le cDNA de la S du SRAS-CoV a été inséré entre les sites BamH1 et Xho1 du plasmide d'expression pcDNA3.1(+) (Clontech) pour obtenir le plasmide pcDNA-S et entre les sites Nhe1 et Xho1 du plasmide d'expression pCI (Promega) pour obtenir le plasmide pCI-S. Les séquences WPRE et CTE ont été insérées dans chacun des deux plasmides pcDNA-S et pCI-S entre les sites Xho1 et Xba1 pour obtenir respectivement les plasmides pcDNA-S-CTE, pcDNA-S-WPRE, pCI-S-CTE et pCI-S-WPRE.
   SP : peptide signal prédit (aa 1-13) avec le logiciel signalP v2.0 (Nielsen et al., 1997, Protein Engineering, 10 : 1-6)
   TM : région transmembranaire prédite (aa 1196-1218) avec le logiciel TMHMM v2.0 (Sonnhammer et al., 1998, Proc. of Sixth Int. Conf. on Intelligent Systems for Molecular Biology, p 175-182, AAAI Press). Il faut noter que les acides aminés W1194 et P1195 font possiblement partie de la région trans-membranaire avec des probabilités respectives de 0,13 et 0,42
   P-CMV : promoteur immédiat/précoce du cytomégalovirus. BGH pA : signal de polyadénylation du gène de l'hormone de croissance bovine
   SV40 late pA : signal de polyadénylation tardif du virus SV40
   SD/SA : sites donneur et accepteur d'épissage
   WPRE : séquences du "Woodchuck Hepatitis Virus posttranscriptional regulatory element" du virus de l'hépatite de la marmotte
   CTE : séquences du "constitutive transport element" du rétrovirus simien de Mason-Pfizer
- la figure 22 illustre l'expression de la protéine S après transfection de cellules VeroE6. Des extraits cellulaires ont été préparés 48 heures après transfection de cellulesVeroE6 par les plasmides pcDNA, pcDNA-S, pCI et pCI-S. Des extraits cellulaires ont également été préparés 18 heures après infection par le virus recombinant de la vaccine VV-TF7.3 et transfection par les plasmides pcDNA ou pcDNA-S. A titre de contrôle, des extraits de cellules VeroE6 ont été préparés 8 heures après infection par le SRAS-CoV à une multiplicité d'infection de 3. Ils ont été séparés sur un gel SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham). Une échelle de masse moléculaire (kDa) est portée sur la figure.
   SRAS-CoV : extrait de cellules VeroE6 infectées par le SRAS-CoV
   Mock : extrait contrôle de cellules non infectées
- la figure 23 illustre l'effet des séquences CTE et WPRE sur l'expression de la protéine S après transfection de cellules VeroE6 et 293T. Des extraits cellulaires ont été préparés 48 heures après transfection de cellulesVeroE6 (A) ou 293T (B) par les plasmides pcDNA, pcDNA-S, pcDNA-S-CTE, pcDNA-S-WPRE, pCI-S, pCI-S-CTE et pCI-S-WPRE, séparés sur un gel SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham). Une échelle de masse moléculaire (kDa) est portée sur la figure.
   SRAS-CoV : extrait de cellules VeroE6 préparés 8 heures après infection par le SRAS-CoV à une multiplicité d'infection de 3.
   Mock : extrait contrôle de cellules VeroE6 non infectées
- la figure 24 présente des vecteurs lentiviraux défectifs à DNA flap central pour l'expression de la S du SRAS-CoV. Le cADN de la protéine S du SRAS-CoV a été cloné sous la forme d'un fragment BamH1-Xho1 dans le plasmide pTRIPΔU3-CMV contenant un vecteur lentiviral défectif TRIP à DNA flap central (Sirven et al, 2001, Mol. Ther., 3 :438-448) pour obtenir le plasmide pTRIP-S. Les cassettes d'expression optimales constituées du promoteur immédiat/précoce du virus CMV, d'un signal d'épissage, du cDNA de la S et de l'un ou l'autre des signaux post-transcriptionnels CTE ou WPRE ont été substituées à la cassette EF1α-EGFP du vecteur d'expression lentiviral défectif à DNA FLAP central TRIPΔU3-EF1α (Sirven et al, 2001, Mol. Ther., 3 :438-448) pour obtenir les plasmides pTRIP-SD/SA-S-CTE et pTRIP-SD/SA-S-WPRE.
   SP : peptide signal
   TM : région transmembranaire
   P-CMV : promoteur immédiat/précoce du cytomégalovirus
   P-EF1α : promoteur du gène EF1α
   SD/SA : sites donneur et accepteur d'épissage
   WPRE : séquences du "Woodchuck Hepatitis Virus posttranscriptional regulatory element" du virus de l'hépatite de la marmotte
   CTE : séquences du "constitutive transport element" du rétrovirus simien de Mason-Pfizer
   LTR : « Long terminal repeat »
   ΔU3 : LTR délété des séquences « promoter/enhancer »
   cPPT : « polypurine tract cis-active sequence »
   CTS : « central termination sequence »
- la figure 25 montre l'analyse par western blot de l'expression de la S du SRAS-CoV par des lignées cellulaires transduites par les vecteurs lentiviraux TRIP-SD/SA-S-WPRE et TRIP-SD/SA-S-CTE. Des extraits cellulaires ont été préparés à partir de lignées FrhK4-S-CTE et FrhK4-S-WPRE établies après transduction par les vecteurs lentiviraux TRIP-SD/SA-S-CTE et TRIP-SD /SA-S-WPRE respectivement. Ils ont été séparés sur un gel SDS à 8% d'acrylamide et analysés par western blot à l'aide d'un anticorps polyclonal de lapin anti-S et d'un conjugué anti-IgG(H+L) de lapin couplé à la peroxidase. Une échelle de masse moléculaire (kDa) est portée sur la figure.
   T- : extrait contrôle de cellules FrhK-4
   T+ : extrait de cellules FrhK-4 préparées 24h après infection par le SRAS-CoV à une multiplicité d'infection de 3.

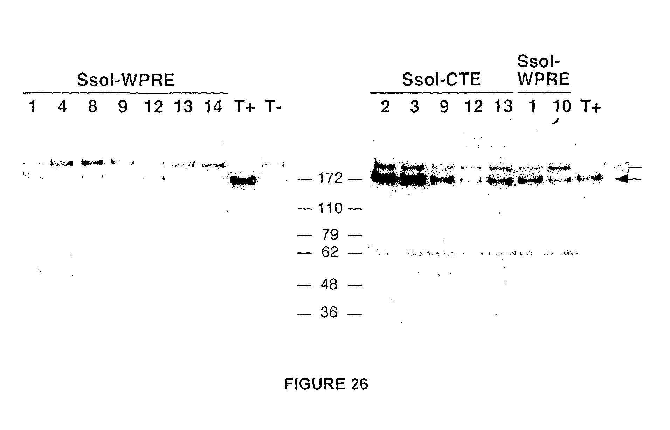

- la figure 26 concerne l'analyse de l'expression de polypeptide Ssol par des lignées cellulaires transduites par les vecteurs lentiviraux TRIP-SD/SA-Ssol-WPRE et TRIP-SD/SA-Ssol-CTE. La sécrétion du polypeptide Ssol a été recherchée dans le surnageant d'une série de clones cellulaires isolés après transduction de cellules FRhK-4 par les vecteurs lentiviraux TRIP-SD/SA-Ssol-WPRE et TRIP-SD/SA-Ssol-CTE. 5 µl de surnageant dilués au 1/2 dans du tampon de dépôt selon Laemmli ont été analysés par western blot révélé par un anticorps monoclonal anti-FLAG (M2, Sigma) et un conjugué anti-IgG(H+L) de souris couplé à la peroxidase. T- : surnageant de la lignée FRhK-4 parentale. T+ : surnageant de cellules BHK infectées par un virus recombinant de la vaccine exprimant le polypeptide Ssol. La flèche pleine indique le polypeptide Ssol, tandis que la flèche creuse indique une réaction croisée avec une protéine d'origine cellulaire.

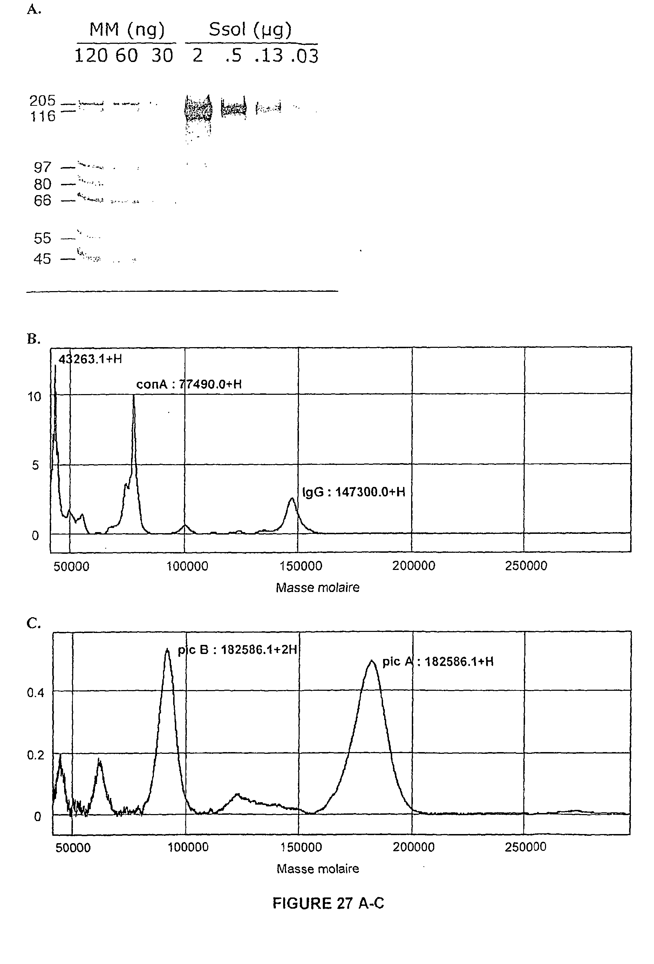

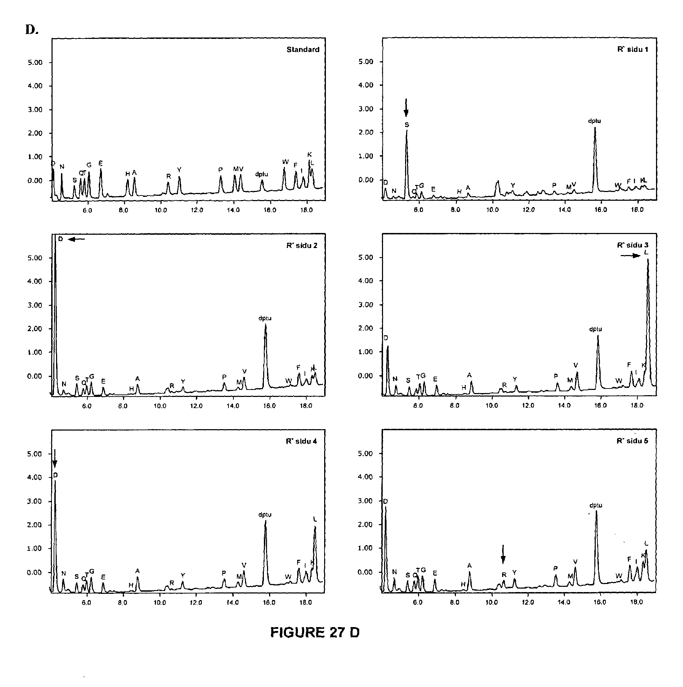

- la figure 27 montre les résultats relatifs à l'analyse du polypeptide Ssol purifié
   A. 8, 2, 0.5 et 0.125 µg de polypeptide recombinant Ssol purifié par chromatographie d'affinité anti-FLAG et gel filtration (G75) ont été séparés sur gel SDS à 8% de polyacrylamide. Le polypeptide Ssol ainsi que des quantités variables de marqueurs de masse moléculaire (MM) ont été révélés par coloration au nitrate d'argent (Gelcode SilverSNAP stain kit II, Pierce).
   B. Marqueurs étalons pour l'analyse par spectrométrie de masse SELDI-TOF
      IgG : IgG bovine de MM 147300
      ConA : conalbumine de MM 77490
      HRP : péroxidase de raifort analysée à titre de contrôle et de MM 43240
   C. Analyse par spectrométrie de masse (SELDI-TOF) du polypeptide recombinant Ssol.
      Les pics A et B correspondent au polypeptide Ssol simplement et doublement chargé.
   D. Séquençage de l'extrémité N-terminale du polypeptide recombinant Ssol. 5 cycles de dégradation d'Edman en phase liquide ont été réalisés sur un séquenceur ABI494 (Applied Biosystems).

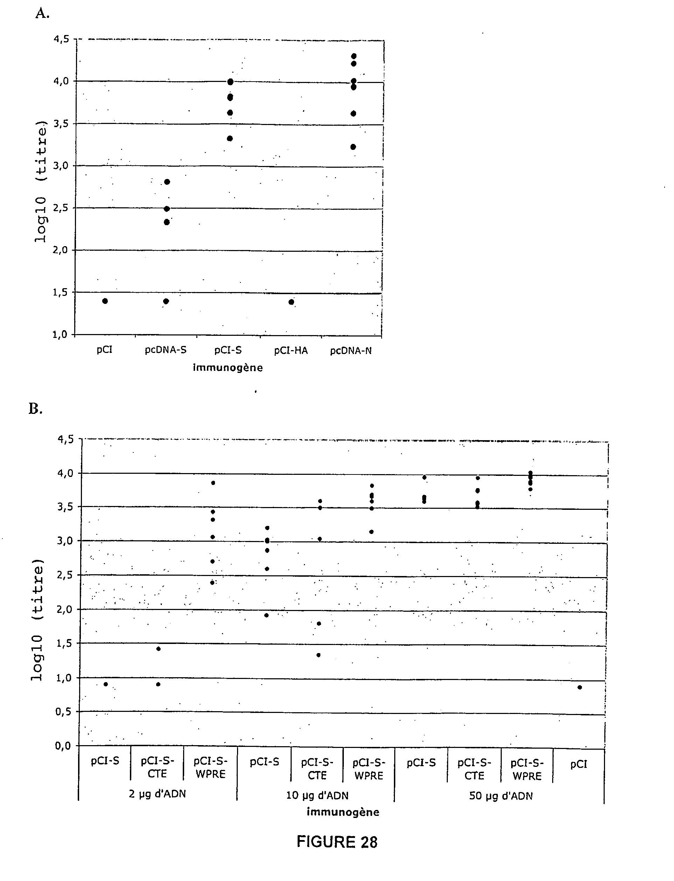

- la figure 28 illustre l'influence d'un signal d'épissage et des séquences CTE et WPRE sur l'efficacité de l'immunisation génique à l'aide d'ADN plasmidique codant la S du SRAS-CoV
   A.Des groupes de 7 souris BALB/c ont été immunisés à deux reprises à 4 semaines d'intervalle à l'aide de 50 µg d'ADN plasmidique de pCI, pcDNA-S, pCI-S, pcDNA-N et pCI-HA.
   B. Des groupes de 6 souris BALB/c ont été immunisés à deux reprises à 4 semaines d'intervalle à l'aide de 2 µg, 10µg ou 50 µg d'ADN plasmidique de pCI, pCI-S, pCI-S-CTE et pCI-S-WPRE.
   Les sérums immuns prélevés 3 semaines après la deuxième immunisation ont été analysés par ELISA indirect en utilisant un lysat de cellules VeroE6 infectées par le SRAS-CoV comme antigène. Les titres en anticorps anti-SRAS-CoV sont calculés comme l'inverse de la dilution produisant une DO spécifique de 0,5 après révélation par un anticorps polyclonal anti-IgG de souris couplé à la peroxidase (NA931V, Amersham) et du TMB (KPL).

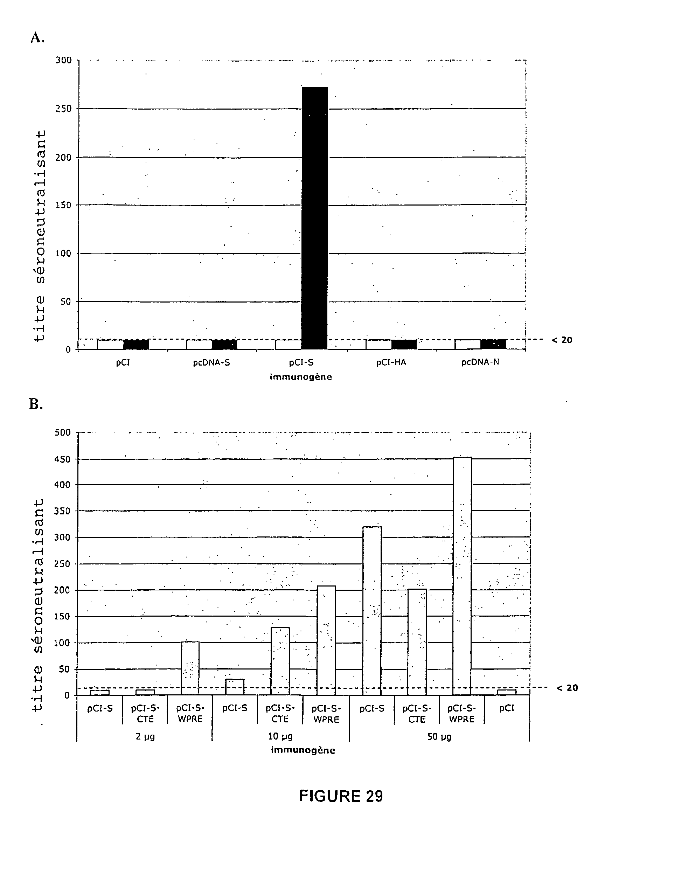

- la figure 29 montre la séroneutralisation de l'infectivité du SRAS-CoV par les anticorps induits chez la souris après immunisation génique à l'aide d'ADN plasmidique codant la S du SRAS-CoV. Des pools de sérums immuns prélevés 3 semaines après la deuxième immunisation ont été réalisés pour chacun des groupes des expériences décrites dans la figure 28 et évalués pour leur capacité à séroneutraliser l'infectivité de 100 TCID50 du SRAS-CoV sur cellules FRhK-4. 4 points sont réalisés pour chacune des dilutions de raison 2 testées à partir du 1/20. Le titre séroneutralisant est calculé selon la méthode de Reed et Munsch comme l'inverse de la dilution neutralisant l'infectivité de 2 cupules sur 4.
   A. Groupes de souris BALB/c immunisés à deux reprises à 4 semaines d'intervalle à l'aide de 50 µg d'ADN plasmidique de pCI, pcDNA-S, pCI-S, pcDNA-N et pCI-HA. □ : sérum préimmun. ■ : sérum immun.
   B. Groupes de souris BALB/c immunisés à deux reprises à 4 semaines d'intervalle à l'aide de 2 µg, 10µg ou 50 µg d'ADN plasmidique de pCI, pCI-S, pCI-S-CTE et pCI-S-WPRE.

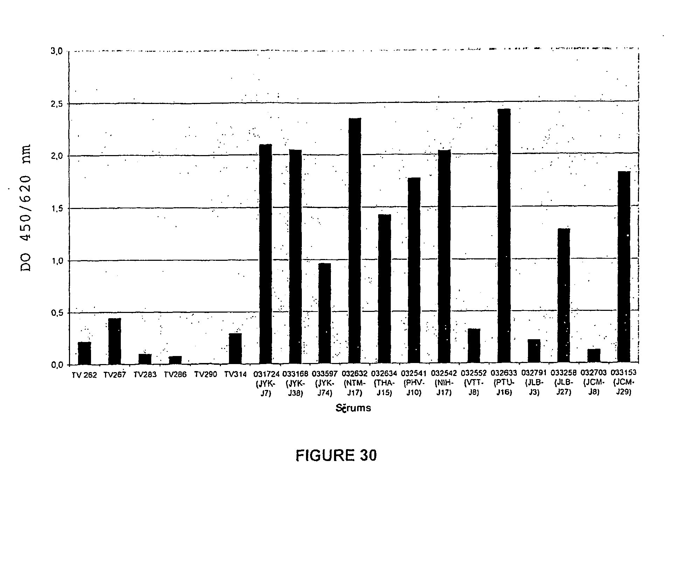

- la figure 30 illustre l'immunoréactivité du polypeptide recombinant Ssol vis à vis de sérums de patients atteints de SRAS. La réactivité de sérums de patients a été analysée par test ELISA indirect contre des phases solides préparées à l'aide du polypeptide recombinant Ssol purifié. Les anticorps de patients réagissant avec la phase solide à une dilution de 1/400 sont révélés par un anticorps polyclonal anti-IgG(H+L) humain couplé à la peroxidase (Amersham NA933V) et du TMB plus H2O2 (KPL). Les sérums de cas probables de SRAS sont identifiés par un numéro d'ordre du Centre National de Référence des Virus Influenzae ainsi que par les initiales du patient et le nombre de jours écoulés depuis le début des symptômes, le cas échéant. Les sérums TV sont des sérums témoins de sujets qui ont été prélevés en France avant l'épidémie de SRAS survenue en 2003.

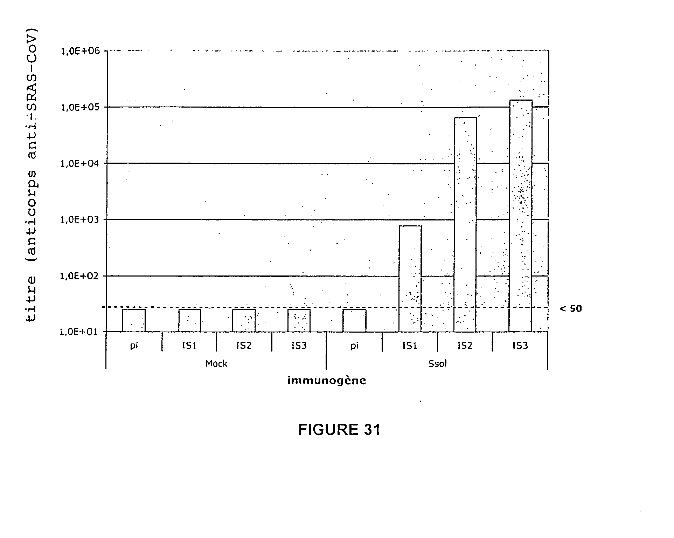

- La figure 31 montre l'induction d'anticorps dirigés contre le SRAS-CoV après immunisation avec le polypeptide recombinant Ssol. Deux groupe de 6 souris ont été immunisés à 3 semaines d'intervalle avec 10 µg de polypeptide recombinant Ssol (groupe Ssol) adjuvé avec de l'hydroxyde d'aluminium ou, à titre de contrôle, de l'adjuvant seul (groupe mock).Trois immunisations successives ont été réalisées et les sérums immuns ont été prélevés 3 semaines après chacune des trois immunisations (IS1, IS2, IS3). Les sérums immuns ont été analysés par pool pour chacun des 2 groupes par ELISA indirect en utilisant un lysat de cellules VeroE6 infectées par le SRAS-CoV comme antigène. Les titres en anticorps anti-SRAS-CoV sont calculés comme l'inverse de la dilution produisant une DO spécifique de 0,5 après révélation par un anticorps polyclonal anti-IgG de souris couplé à la peroxidase (Amersham) et du TMB (KPL).

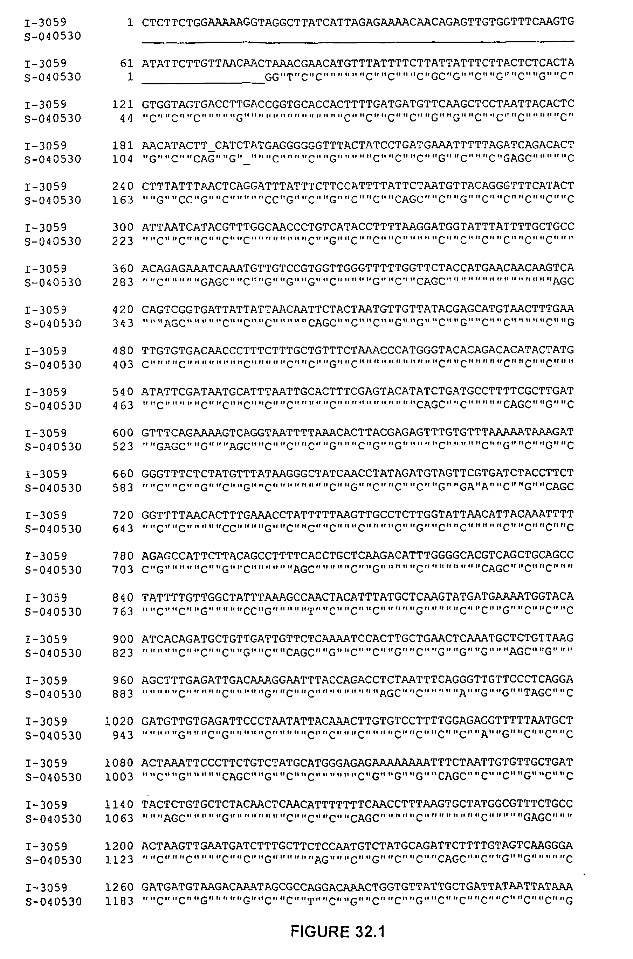

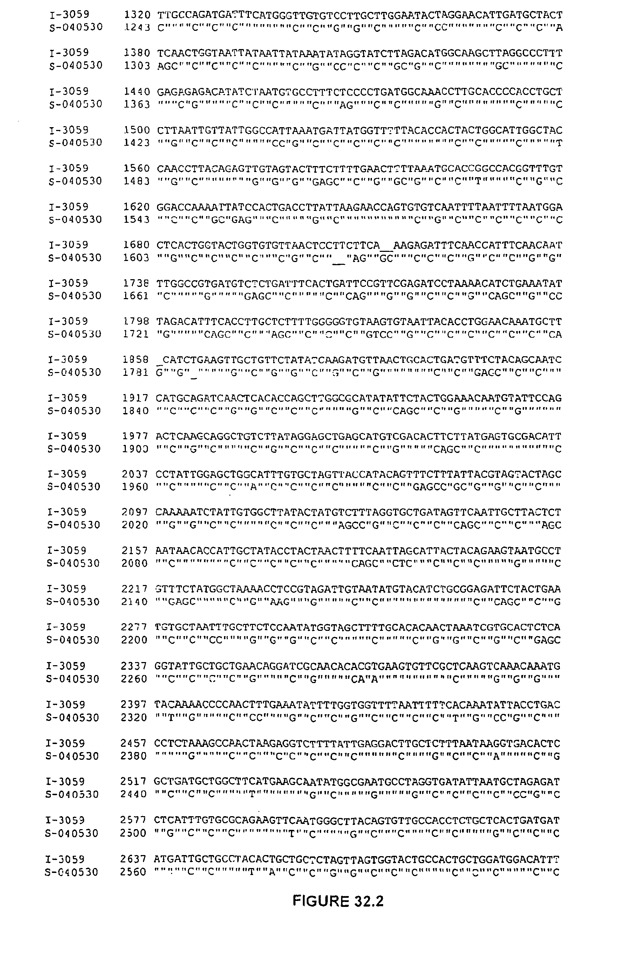

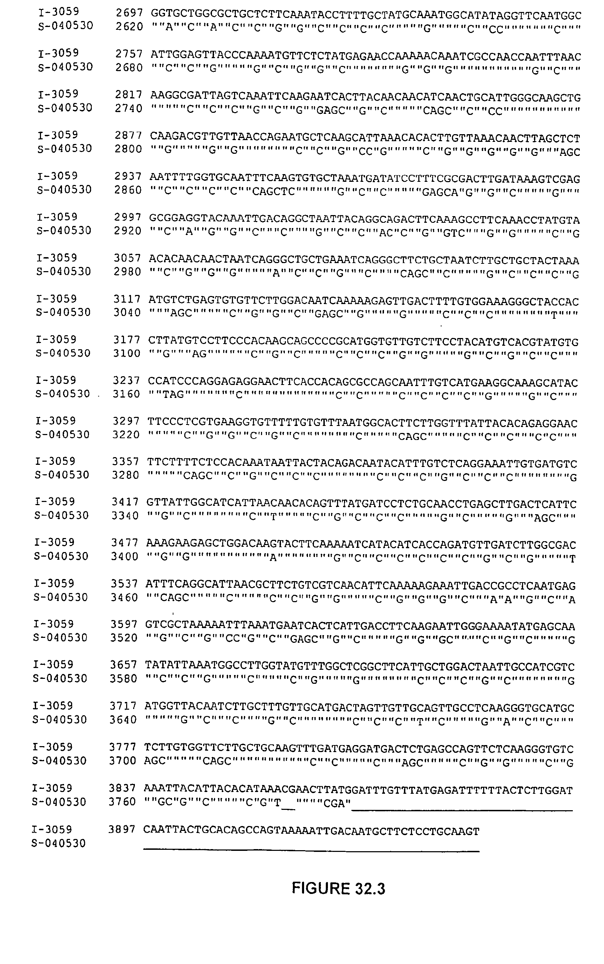

- La figure 32 présente l'alignement nucléotidique des séquences du gène synthétique 040530 avec la séquence du gène sauvage de l'isolat 031589 du SRAS-CoV. I-3059 correspond aux nucléotides 21406-25348 de l'isolat 031589 du SRAS-CoV déposé à la C.N.C.M. sous le numéro I-3059 (SEQ ID NO : 4, plasmide pSRAS-S). S-040530 est la séquence du gène synthétique 040530.

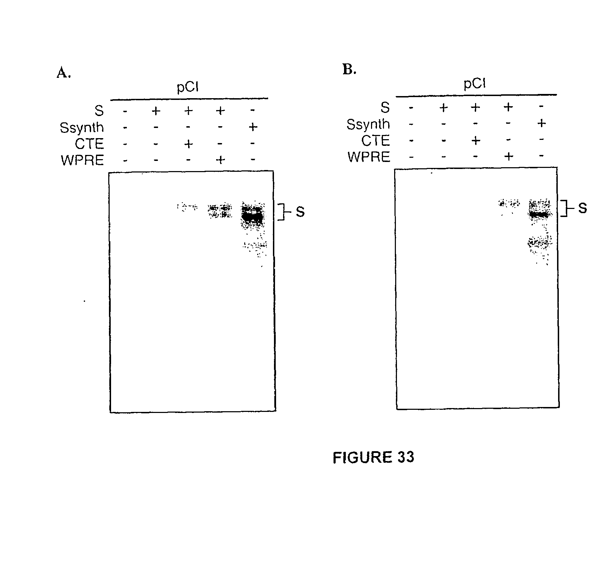

- la figure 33 illustre l'utilisation d'un gène synthétique pour l'expression de la S du SRAS-CoV. Des extraits cellulaires préparés 48 heures après transfection de cellulesVeroE6 (A) ou 293T (B) par les plasmides pCI, pCI-S, pCI-S-CTE, pCI-S-WPRE et pCI-Ssynth ont été séparés sur un gel SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham). Le western blot est révélé par luminescence (ECL+, Amersham) et acquisition sur un dispositif d'imagerie numérique (FluorS, BioRad). Les niveaux d'expression de la protéine S ont été mesurés en quantifiant les 2 bandes majoritaires repérées sur l'image.

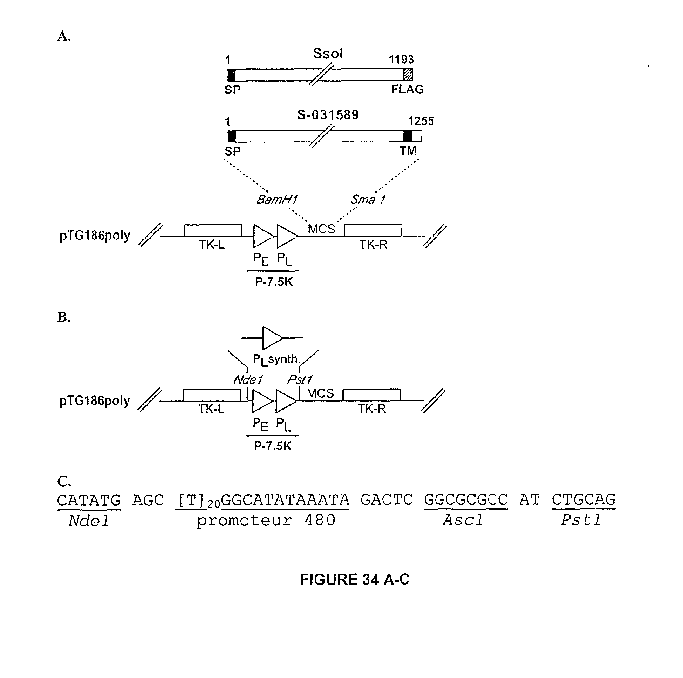

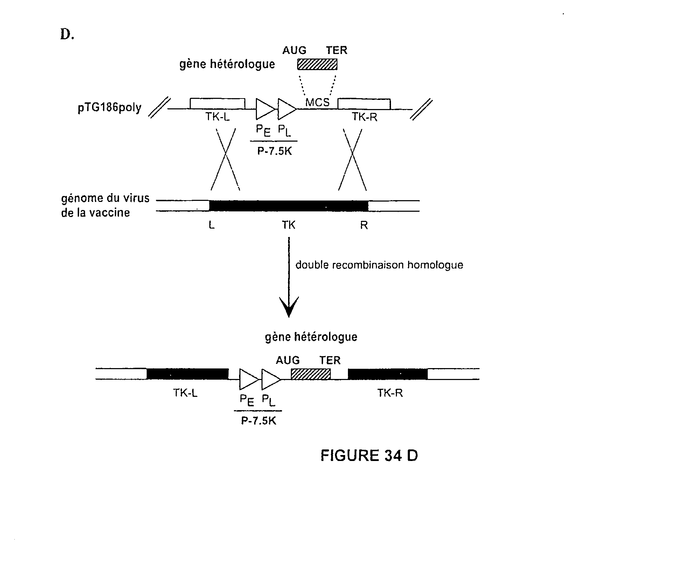

- la figure 34 présente un schéma de la construction des virus vaccine recombinants VV-TG-S, VV-TG-Ssol, VV-TN-S et VV-TN-Ssol
   A. Les cDNA de la protéine S et du polypeptide Ssol du SRAS-CoV ont été inséré entre les sites BamH1 et Sma1 du plasmide de transfert pTG186 pour obtenir les plasmides pTG-S et pTG-Ssol.
   B. Les séquences du promoteur synthétique 480 ont ensuite été substituées à celles du promoteur 7.5 par échange du fragment Nde1-Pst1 des plasmides pTG186poly, pTG-S et pTG-Ssol pour obtenir les plasmides de transfert pTN480, pTN-S et pTN -Ssol.
   C. Séquence du promoteur synthétique 480 tel que contenu entre les sites Nde1 et Pst1 des plasmides de transfert de la série pTN. Un site Asc1 a été inséré pour faciliter les manipulations ultérieures. Les sites de restriction ainsi que la séquence du promoteur sont soulignés
   D. Les virus recombinants de la vaccine sont obtenus par double recombinaison homologue *in vivo* entre la cassette TK des plasmides de transfert des séries pTG et pTN et le gène TK de la souche Copenhague du virus de la vaccine.
      SP : peptide signal prédit (aa 1-13) avec le logiciel signalP v2.0 (Nielsen et al., 1997, Protein Engineering, 10 : 1-6)
      TM : région transmembranaire prédite (aa 1196-1218) avec le logiciel TMHMM v2.0 (Sonnhammer et al., 1998, Proc. of Sixth Int. Conf. on Intelligent Systems for Molecular Biology, p 175-182, AAAI Press). Il faut noter que les acides aminés W1194 et P1195 font possiblement partie de la région trans-membranaire avec des probabilités respectives de 0,13 et 0,42.
      TK-L, TK-R : parties gauche et droite du gène de la thymidine kinase du virus de la vaccine
      MCS : site multiple de clonage
      PE : promoteur précoce
      PL : promoteur tardif
      PL synth : promoteur tardif synthétique 480
- la figure 35 illustre l'expression de la protéine S par les virus vaccine recombinants, analysée par western blot. Des extraits cellulaires ont été préparés 18 heures après infection de cellules CV1 par les virus vaccine recombinants VV-TG, VV-TG-S et VV-TN-S à une M.O.I. de 2 (A). A titre de contrôle, des extraits de cellules VeroE6 ont été préparés 8 heures après infection par le SRAS-CoV à une multiplicité d'infection de 2. Des extraits cellulaires ont également été préparés 18 heures après infection de cellules CV1 par les virus vaccine recombinants VV-TG-S, VV-TG-Ssol, VV-TN, VV-TN-S et VV-TN-Ssol (B). Ils ont été séparés sur des gels SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham). « 1µl » et « 10µl » indique les quantités d'extraits cellulaires déposées sur le gel. Une échelle de masse moléculaire (kDa) est portée sur la figure.
   SRAS-CoV : extrait de cellules VeroE6 infectées par le SRAS-CoV
   Mock : extrait contrôle de cellules non infectées
- la figure 36 montre le résultat d'une analyse par western blot de la sécrétion du polypeptide Ssol par les virus vaccine recombinants.
   A. Des surnageants de cellules CV1 infectées par le virus vaccine recombinant VV-TN, différents clones du virus VV-TN-Ssol et par les virus VV-TG-Ssol ou VV-TN-Sflag ont été récoltés 18 heures après infection de cellules CV1 à une M.O.I. de 2.
   B. Des surnageants de cellules 293T, FRhK-4, BHK-21 et CV1 infectées en dupliqués (1,2) par le virus vaccine recombinant VV-TN-Ssol à une M .O.I. de 2 ont été récoltés 18 heures après infection. Le surnageant de cellules CV1 infectées par le virus VV-TN a également été récolté à titre de contrôle (M).
   Tous les surnageants ont été séparés sur un gel SDS à 8% d'acrylamide selon Laemmli et analysés par western blot à l'aide d'un anticorps monoclonal de souris anti-FLAG et d'un anticorps polyclonal anti-IgG(H+L) de souris couplé à la peroxidase (NA931 V, Amersham) (A) ou à l'aide d'un anticorps polyclonal de lapin anti-S et d'un anticorps polyclonal anti-IgG(H+L) de lapin couplé à la peroxidase (NA934V, Amersham) (B).
   Une échelle de masse moléculaire (kDa) est portée sur la figure.

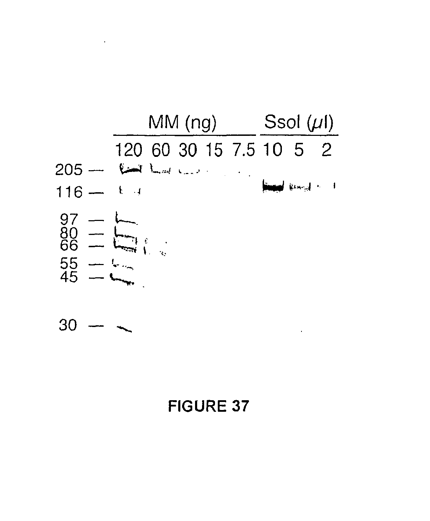

- la figure 37 montre l'analyse du polypeptide Ssol, purifié par gel SDS de polyacrylamide
   10, 5 et 2 µl de polypeptide recombinant Ssol purifié par chromatographie d'affinité anti-FLAG ont été séparés sur gel SDS en gradient de 4 à 15 % de polyacrylamide. Le polypeptide Ssol ainsi que des quantités variables de marqueurs de masse moléculaire (MM) ont été révélés par coloration au nitrate d'argent (Gelcode SilverSNAP stain kit II, Pierce).

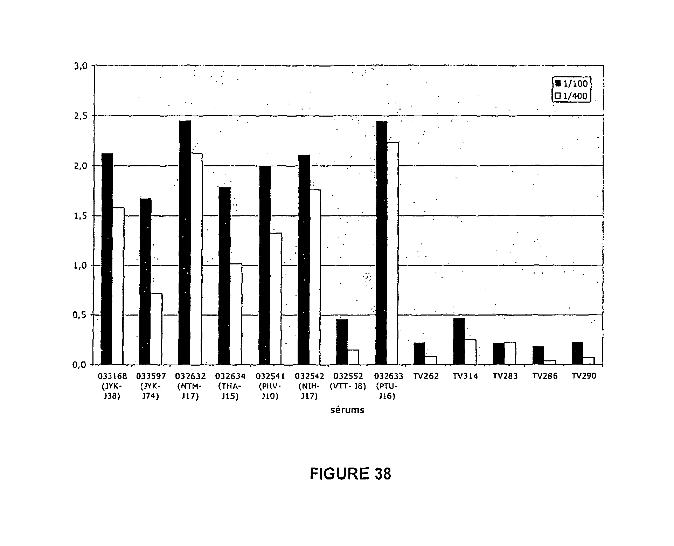

- la figure 38 illustre l'immunoréactivité du polypeptide recombinant Ssol produit par le virus vaccine recombinant VV-TN-Ssol vis-à-vis de sérums de patients atteints de SRAS. La réactivité de sérums de patients a été analysée par test ELISA indirect contre des phases solides préparées à l'aide du polypeptide recombinant Ssol purifié. Les anticorps de patients réagissant avec la phase solide à une dilution de 1/100 et 1/400 sont révélés par un anticorps polyclonal anti-IgG(H+L) humain couplé à la peroxidase (Amersham NA933V) et du TMB plus H2O2 (KPL). Les sérums de cas probables de SRAS sont identifiés par un numéro d'ordre du Centre National de Référence des Virus Influenzae ainsi que par les initiales du patient et le nombre de jours écoulés depuis le début des symptômes, le cas échéant. Les sérums TV sont des sérums témoins de sujets qui ont été prélevés en France avant l'épidémie de SRAS survenue en 2003.

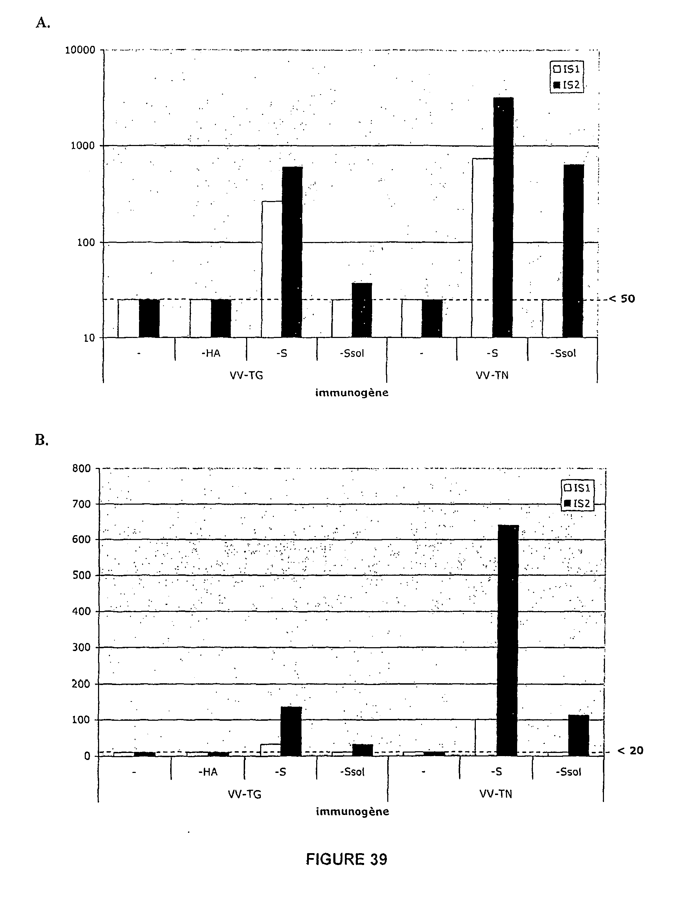

- la figure 39 montre la réponse en anticorps anti-SRAS-CoV chez la souris après immunisation par les virus vaccine recombinants. Des groupes de 7 souris BALB/c ont été immunisés par voie i.v. à deux reprises à 4 semaines d'intervalle par 106 u.f.p. de virus vaccine recombinants VV-TG, VV-TG-HA, VV-TG-S, VV-TG-Ssol, VV-TN, VV-TN-S, VV-TN-Ssol.
   A. Des pools de sérums immuns prélevés 3 semaines après chacune des deux immunisations ont été réalisés pour chacun des groupes et ont été analysés par ELISA indirect en utilisant un lysat de cellules VeroE6 infectées par le SRAS-CoV comme antigène. Les titres en anticorps anti-SRAS-CoV sont calculés comme l'inverse de la dilution produisant une DO spécifique de 0,5 après révélation par un anticorps polyclonal anti-IgG de souris couplé à la peroxidase (NA931V, Amersham) et du TMB (KPL).
   B. Les pools de sérums immuns ont été évalués pour leur capacité à séroneutraliser l'infectivité de 100 TCID50 du SRAS-CoV sur cellules FRhK-4. 4 points sont réalisés pour chacune des dilutions de raison 2 testées à partir du 1/20. Le titre séroneutralisant est calculé selon la méthode de Reed et Munsch comme l'inverse de la dilution neutralisant l'infectivité de 2 cupules sur 4.

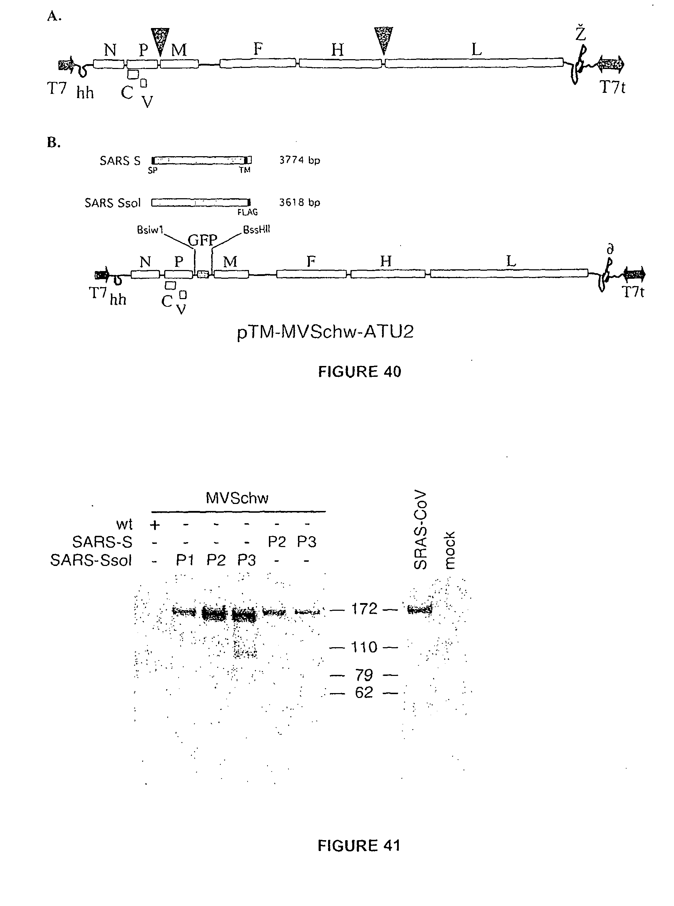

- la figure 40 décrit la construction des virus recombinants MVSchw2-SARS-S et MVSchw2-SARS-Ssol.
   A. Le vecteur rougeole est un génome complet de la souche vaccinale Schwarz du virus de la rougeole (MV) dans lequel une unité de transcription supplémentaire a été introduite (Combredet, 2003, Journal of Virology, 77 : 11546-11554). L'expression des phases ouvertes de lecture (ORF) supplémentaires est contrôlée par les éléments agissant en cis nécessaires à la transcription, à la formation de la coiffe et à la polyadénylation du transgène, qui ont été copiés des éléments présents à la jonction N/P. 2 vecteurs différents permettent l'insertion entre les gènes P (phosphoprotéine) et M (matrice) d'une part et H (hémagglutinine) et L (polymérase) d'autre part.
   B. Les génomes recombinants MVSchw2-SARS-S et MVSchw2-SARS-Ssol du virus de la rougeole ont été construits par l'insertion des ORFs de la protéine S et du polypeptide Ssol au sein d'une unité de transcription supplémentaire localisée entre les gènes P et M du vecteur.
   Les différents gènes du virus de la rougeole (MV) sont indiqués : N (nucléoprotéine), PVC (phosphoprotéine et protéine V/C), M (matrice), F (fusion), H (hémagglutinine), L (polymérase). T7 = promoteur de l'ARN polymérase T7, hh = ribozyme hammerhead, T7t = séquence terminatrice de l'ARN polymérase du phage T7, ∂ = ribozyme du virus de l'hépatite ∂, (2), (3) = unités de transcription supplémentaires (ATU).
   Taille du génome du MV : 15894 nt.
   SP : peptide signal
   TM : région transmembranaire
   FLAG : étiquette FLAG
- la figure 41 illustre l'expression de la protéine S par les virus rougeole recombinants, analysée par western blot.
   Des extraits cytoplasmiques ont été préparés après infection de cellules Vero par différents passages des virus MVSchw2-SARS-S et MVSchw2-SARS-Ssol et le virus sauvage MWSchw à titre de contrôle. Des extraits cellulaires en tampon de dépôt selon Laemmli ont également été préparés 8 heures après infection de cellules VeroE6 par le SRAS-CoV à une multiplicité d'infection de 3. Ils ont été séparés sur un gel SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham).
   Une échelle de masse moléculaire (kDa) est portée sur la figure.
   Pn : nième passage du virus après coculture de cellules 293-3-46 et Vero.
   SRAS-CoV : extrait de cellules VeroE6 infectées par le SRAS-CoV
   Mock : extrait contrôle de cellules VeroE6 non infectées
- la figure 42 montre l'expression de la protéine S par les virus rougeole recombinants, analysée par immunofluorescence
   Des cellules Vero en monocouches sur lamelles de verre ont été infectées par le virus sauvage MWSchw (A) ou les virus MVSchw2-SARS-S (B) et MVSchw2-SARS-Ssol (C). Quand les syncytia ont atteint 30 à 40% de confluence (A., B.) ou 90-100% (C), les cellules ont été fixées, perméabilisées et marquées par des anticorps polyclonaux de lapins anti-SRAS-CoV et un conjugué anti-IgG(H+L) de lapin couplé au FITC (Jackson).

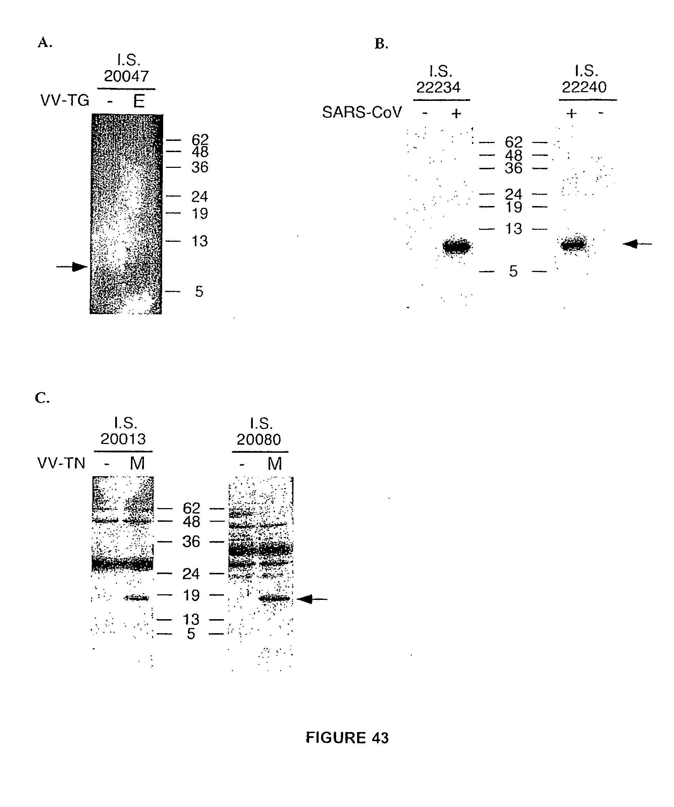

- la figure 43 illustre l'analyse par western blot de l'immunoréactivité de sérums de lapins dirigés contre les peptides E1-12, E53-76 et M2-14. Le lapin 20047 a été immunisé avec le peptide E1-12 couplé à la KLH. Les lapins 22234 et 22240 ont été immunisés avec le peptide E53-76 couplé à la KLH. Les lapins 20013 et 20080 ont été immunisés avec le peptide M2-14 couplé à la KLH. Les immunsérums ont été analysés par western blot à l'aide d'extraits de cellules infectées par le SRAS-CoV (B) ou à l'aide d'extraits de cellules infectées par un virus recombinant de la vaccine exprimant la protéine E (A) ou M (C) de l'isolat 031589 du SRAS-CoV. Les immunoempreintes ont été révélées à l'aide d'un conjugué anti-IgG(H+L) de lapin couplé à la peroxidase (NA934V, Amersham).
   La position des protéines E etM est indiquée par une flèche.
   Une échelle de masse moléculaire (kDa) est portée sur la figure.
   Il doit être bien entendu, toutefois, que ces exemples sont donnés uniquement à titre d'illustration de l'objet de l'invention, dont ils ne constituent en aucune manière une limitation.

### Exemple 1: Clonage et séquençage du génome de la souche de SARS-CoV issue du prélèvement répertorié sous le numéro 031589

L'ARN de la souche de SARS-CoV a été extrait à partir du prélèvement de lavage bronchoalvéolaire répertorié sous le numéro 031589, effectué sur un patient de l'hôpital français de Hanoi (Vietnam) atteint de SRAS.

L'ARN isolé a été utilisé comme matrice pour amplifier les ADNc correspondant aux différents cadres ouverts de lecture du génome (ORF 1a, ORF1b, ORF-S, ORF-E, ORF-M, ORF-N (incluant les ORF-13 et ORF-14), ORF3, ORF4, ORF7 à ORF11), et aux extrémités 5' et 3' non-codantes. Les séquences des amorces et des sondes utilisées pour l'amplification/détection ont été définies d'après la séquence nucléotidique disponible du SARS-CoV.

Dans ce qui suit les amorces et les sondes sont identifiées par : la lettre S, suivie d'une lettre qui indique la région correspondante du génome (L pour l'extrémité 5'incluant ORF1a et ORF1b; S, M et N pour les ORF-S, ORF-M, ORF-N, SE et MN pour les régions intergéniques correspondantes), puis éventuellement de Fn, Rn, avec n inclus entre 1 et 6 correspondant aux amorces utilisées pour la PCR nichée ou imbriquée (paire F1 + R1 pour la première amplification, paire F2 + R2 pour la deuxième amplification, etc...), puis de /+/ ou /-/ correspondant à une amorce sens ou antisens et enfin des positions des amorces en référence à la séquence Genbank AY27411.3; pour les amorces S et N sens et antisens et les autres amorces sens uniquement, lorsqu'une seule position est indiquée elle correspond à celle de l'extrémité 5' d'une sonde ou d'une amorce d'environ 20 bases ; pour les amorces antisens autres que les amorces S et N, lorsqu'une seule position est indiquée elle correspond à celle de l'extrémité 3' d'une sonde ou d'une amorce d'environ 20 bases. Les produits d'amplifications ainsi générés ont été séquencés à l'aide d'amorces spécifiques afin de déterminer la séquence complète du génome de la souche de SARS-CoV issue du prélèvement répertorié sous le numéro 031589. Ces produits d'amplification, à l'exception de ceux correspondant aux ORF1a et ORF1b, ont ensuite été clonés dans des vecteurs d'expression afin de produire les protéines virales correspondantes et les anticorps dirigés contre ces protéines, notamment par immunisation à base d'ADN.

### 1. Extraction des ARN

Les ARN ont été extraits à l'aide du kit *QIamp viral RNA extraction mini* (QIAGEN) en suivant les recommandations du fabricant. De manière plus précise : 140 µl du prélèvement et 560 µl de tampon AVL ont été mélangés vigoureusement pendant 15 secondes, incubés 10 min à température ambiante puis centrifugés brièvement à vitesse maximale. 560 µl d'éthanol à 100% ont été ajoutés au surnageant et le mélange ainsi obtenu a été agité très vigoureusement pendant 15 sec. 630 µl du mélange ont ensuite été déposés sur la colonne.

La colonne a été placée sur un tube de 2 ml, centrifugée 1 min à 8000 rpm, puis le reste du mélange précédent a été déposé sur la même colonne, centrifugé à nouveau, 1 min à 8000 rpm et la colonne a été transférée sur un tube de 2 ml propre. Ensuite, 500 µl de tampon AW1 ont été ajoutés sur la colonne, puis la colonne a été centrifugée 1 min à 8000 rpm et l'éluat a été éliminé. 500 µl de tampon AW2 ont été ajoutés sur la colonne qui a ensuite été centrifugée 3 min à 14000 rpm et transférée sur un tube de 1,5 ml. Enfin, 60 µl de tampon AVE ont été ajoutés sur la colonne qui a été incubée 1 à 2 min à température ambiante puis centrifugée 1 min à 8000 rpm. L'éluat correspondant à l'ARN purifié a été récupéré et congelé à -20°C.

### 2. Amplification, séquençage et clonage des ADNc

### 2.1) ADNc codant pour la protéine S

Les ARN extraits à partir du prélèvement ont été soumis à une transcription inverse à l'aide d'oligonucléotides hexamériques de séquence aléatoire (pdN6), afin de produire des fragments d'ADNc.

La séquence codant pour la glycoprotéine S du SARS-CoV a été amplifiée sous la forme de deux fragments d'ADN chevauchants : fragment 5' (SRAS-Sa, SEQ ID NO:5) et fragment 3'(SRAS-Sb, SEQ ID NO:6), en réalisant deux amplifications successives à l'aide d'amorces imbriquées. Les amplicons ainsi obtenus ont été séquencés, clonés dans le vecteur plasmidique PCR 2.1-TOPO™ (IN VITROGEN), puis la séquence des ADNc clonés a été déterminée.

### a)clonage et séquençage des fragments Sa et Sb

### a₁) synthèse de l'ADNc

Le mélange réactionnel contenant : ARN (5 µl) , H₂O ppi (3,5 µl), tampon de transcriptase inverse5X (4 µl,), dNTP 5 mM (2 µl), pdN6 100 ug/ml (4 µl), RNasin 40 UI/ul (0,5 µl) et transcriptase inverse AMV-RT, 10 UI/ul, PROMEGA (1µl) a été incubé dans un thermocycleur dans les conditions suivantes : 45 min à 42°C, 15 min à 55°C, 5 min à 95°C, puis l'ADNc obtenu a été maintenu à +4°C.

### a₂) première amplification PCR

Les extrémités 5' et 3' du gène S ont été amplifiées respectivement avec les paires d'amorces S/F1/+/ 21350-21372 et S/R1/-/ 23518-23498, S/F3/+/ 23258-23277 et S/R3/-/25382-25363. Le mélange réactionnel de 50 µl contenant : ADNc (2 µl), amorces 50 µM (0,5 µl), tampon 10 X (5 µl), dNTP 5 mM (2 µl), Taq Expand High Fidelity, Roche (0,75 µl) et H₂O (39, 75 µl) a été amplifié dans un thermocycleur, dans les conditions suivantes : une étape initiale de dénaturation à 94°C pendant 2 min a été suivie de 40 cycles comprenant : une étape de dénaturation à 94°C pendant 30 sec, une étape d'hybridation à 55°C pendant 30 sec puis une étape d'élongation à 72°C pendant 2 min 30 sec, avec 10 sec d'élongation supplémentaire à chaque cycle, puis d'une étape finale d'élongation à 72°C pendant 5 min.

### a₃) deuxième amplification PCR

Les produits de la première amplification PCR (amplicons 5' et 3') ont subi une seconde étape d'amplification PCR (PCR nichée) dans des conditions identiques à celles de la première amplification, avec les paires d'amorces S/F2/+/21406-21426 et S/R2/-/23454-23435, et S/F4/+/23322-23341 et S/R4/-/25348-25329, respectivement pour l'amplicon 5' et l'amplicon 3'.

### a₄)clonage et séquençage des fragments Sa et Sb

Les amplicons Sa (extrémité 5') et Sb (extrémité 3') ainsi obtenus ont été purifiés à l'aide du kit *QIAquick PCR purification* (QIAGEN), en suivant les recommandations du fabricant, puis ils ont été clones dans le vecteur PCR2.1-TOPO (kit Invitrogen), pour donner les plasmides dénommés SRAS-S 1 et SRAS-S2.

L'ADN des clones Sa et Sb a été isolé puis l'insert correspondant a été séquencé à l'aide du Kit Big Dye, Applied Biosystem® et des amorces universelles M 13 forward et M 13 reverse, ainsi que des amorces: S/S/+/21867, S/S/+/22353, S/S/+/22811, S/S/+/23754, S/S/+/24207, S/S/+/24699, S/S/+/24348, S/S/-/24209, S/S/-/23630, S/S/-/23038, S/S/-/22454, S/S/-/21815, S/S/-/24784, S/S/+/21556, S/S/+/23130 et S/S/+/24465, en suivant les instructions du fabricant ; les séquences des fragments Sa et Sb ainsi obtenues correspondent aux séquences SEQ ID NO :5 et SEQ ID NO :6 dans la liste de séquences jointe en annexe.

Le plasmide, dénommé SARS-S1 a été déposé sous le n° I-3020, le 12 mai 2003, auprès de la Collection Nationale de Cultures de Microorganismes, 25 rue du Docteur Roux, 75724 Paris Cedex 15 ; il contient un fragment 5' de la séquence du gène S de la souche de SARS-CoV issue du prélèvement répertorié sous le n° 031589, telle que définie ci-dessus, lequel fragment dénommé Sa correspondant aux nucléotides des positions 21406 à 23454 (SEQ ID NO :5), en référence à la séquence Genbank AY274119.3 Tor2.

Le plasmide, dénommé TOP10F'-SARS-S2 a été déposé sous le n° I-3019, le 12 mai 2003, auprès de la Collection Nationale de Cultures de Microorganismes, 25 rue du Docteur Roux, 75724 Paris Cedex 15 ; il contient un fragment 3'de la séquence du gène S de la souche de SARS-CoV issue du prélèvement répertorié sous le n° 031589, telle que définie ci-dessus, lequel fragment dénommé Sb correspondant aux nucléotides des positions 23322 à 25348 (SEQ ID NO : 6), en référence à la séquence Genbank n° d'accès AY274119.3.

### b) clonase et séquençage de l'ADNc complet (clone SRAS-S de 4 kb)

L'ADNc S complet a été obtenu à partir des clones SARS-S1 et SARS-S2 précités, de la façon suivante :
1) une réaction d'amplification PCR a été réalisée sur un clone SARS-S2 en présence de l'amorce S/R4/-/25348-25329 précitée et de l'amorce S/S/+/24696-24715: un amplicon de 633 pb a été obtenu,
2) une autre réaction d'amplification PCR a été réalisée sur un autre clone SARS-S2, en présence des amorces S/F4/+/23322-23341 précitée et S/S/- /24803-24784: un amplicon de 1481 pb a été obtenu,
   La réaction d'amplification a été réalisée dans les conditions telles que définies ci-dessus pour l'amplification des fragments Sa et Sb, à l'exception que 30 cycles d'amplifications comprenant une étape de dénaturation à 94° C pendant 20 sec et une étape d'élongation à 72° C pendant 2 min 30 sec ont été effectués.
3) les 2 amplicons (633 pb et 1481 pb) ont été purifiés dans les conditions telles que définies ci-dessus pour les fragments Sa et Sb.
4) une autre réaction d'amplification PCR à l'aide des amorces S/F4/+/23322-23341 et S/R4/-/25348-25329 précitées, a été réalisée sur les amplicons purifiés obtenus en 3). La réaction d'amplification a été réalisée dans les conditions telles que définies ci-dessus pour l'amplification des fragments Sa et Sb, à l'exception que 30 cycles d'amplifications ont été effectués.
   L'amplicon de 2026 pb ainsi obtenu a été purifié, cloné dans le vecteur PCR2.1-TOPO puis séquencé comme ci-dessus, à l'aide des amorces telles que définies ci-dessus pour les fragments Sa et Sb. Le clone ainsi obtenu a été dénommé clone 3'.
5) Le clone SARS-S1 précédemment obtenu et le clone 3'ont été digérés par *EcoR I,* les bandes d'environ 2kb ainsi obtenues ont été purifiées sur gel puis amplifiées par PCR avec les amorces S/F2/+/21406-21426 et S/R4/-/25348-25329 précitées. La réaction d'amplification a été réalisée dans les conditions telles que définies ci-dessus pour l'amplification des fragments Sa et Sb, à l'exception que 30 cycles d'amplifications ont été effectués. L'amplicon d'environ 4 kb a été purifié et séquencé. Il a ensuite été cloné dans le vecteur PCR2.1-TOPO pour donner le plasmide, dénommé SARS-S, et l'insert contenu dans ce plasmide a été séquencé comme ci-dessus, à l'aide des amorces telles que définies ci-dessus pour les fragments Sa et Sb. Les séquences d'ADNc de l'insert et de l'amplicon codant pour la protéine S, correspondent respectivement aux séquences SEQ ID NO : 4 et SEQ ID NO : 2 dans la liste de séquences jointe en annexe, elles codent pour la protéine S (SEQ ID NO : 3).

La séquence de l'amplicon correspondant à l'ADNc codant pour la protéine S de la souche de SARS-CoV issue du prélèvement n°031589 présente les deux mutations suivantes par rapport aux séquences correspondantes de respectivement les isolats Tor2 et Urbani, les positions des mutations étant indiquées en référence à la séquence complète du génome de l'isolat Tor2 (Genbank AY274119.3) :
- g/t en position 23220 ; le codon alanine (gct) en position 577 de la séquence en acides aminés de la protéine S de Tor2 est remplacé par un codon sérine (tct),
- c/t en position 24872 : cette mutation ne modifie pas la séquence en acides aminés de la protéine S, et

Le plasmide, dénommé SARS-S, a été déposé sous le n° I-3059, le 20 juin 2003, auprès de la Collection Nationale de Cultures de Microorganismes, 25 rue du Docteur Roux, 75724 Paris Cedex 15 ; il contient la séquence d'ADNc codant pour la protéine S de la souche de SARS-CoV issue du prélèvement répertorié sous le n° 031589, laquelle séquence correspondant aux nucléotides des positions 21406 à 25348 (SEQ ID NO :4), en référence à la séquence Genbank AY274119.3.

### 2.2) ADNc codant pour les protéines M et E

Les ARN issus du prélèvement 031589, extraits comme ci-dessus, ont été soumis à une transcription inverse, associée, lors de la même étape (kit *Titan One Step RT-PCR*®, Roche), à une réaction d'amplification par PCR, à l'aide des couples d'amorces :
- S/E/F1/+/26051-26070 et S/E/R1/-/26455-26436 pour amplifier l'ORF-E, et
- S/M/F1/+/26225-26244 et S/M/R1/-/27148-27129 pour amplifier l'ORF-M.

Un premier mélange réactionnel contenant : 8,6 µl d'H₂Oppi, 1 µl de dNTP (5mM), 0,2 µl de chacune des amorces (50µM), 1,25 µl de DTT (100mM) et 0,25 µl de RNAsin (40UI/µl) a été combiné avec un deuxième mélange réactionnel contenant : 1 µl d'ARN, 7 µl d'H₂Oppi, 5 µl de tampon de RT-PCR 5X et 0,5 µl de mélange d'enzyme et les mélanges combinés ont été incubés dans un thermocycleur dans les conditions suivantes : 30 min à 42°C, 10 min à 55°C, 2 min à 94°C suivi de 40 cycles comprenant une étape de dénaturation à 94°C pendant 10 sec, une étape d'hybridation à 55°C pendant 30 sec et une étape d'élongation à 68°C pendant 45 sec, avec 3 sec d'incrément par cycle et enfin une étape d'élongation terminale à 68°C pendant 7 min.

Les produits d'amplification ainsi obtenus (amplicons M et E) ont subi une deuxième amplification PCR (PCR nichée) en utilisant le kit Expand High-Fi®, Roche), à l'aide des couples d'amorces :
- S/E/F2/+/26082-26101 et S/E/R2/-/26413-26394 pour l'amplicon E, et
- S/M/F2/+/26330-26350 et S/M/R2/-/27098-27078 pour l'amplicon M.

Le mélange réactionnel contenant : 2 µl du produit de la première PCR, 39,25 µl d'H₂Oppi, 5 µl de tampon 10X contenant du MgCl₂, 2 µl de dNTP (5mM), 0,5µl de chacune des amorces (50 µM) et 0,75µl de mélange d'enzyme a été incubé dans un thermocycleur dans les conditions suivantes : une étape de dénaturation à 94°C pendant 2 min a été suivie de 30 cycles comprenant une étape de dénaturation à 94°C pendant 15 sec, une étape d'hybridation à 60°C pendant 30 sec et une étape d'élongation à 72°C pendant 45 sec, avec 3 sec d'incrément par cycle, et enfin une étape d'élongation terminale à 72°C pendant 7 min. Les produits d'amplification obtenus correspondant aux ADNc codant pour les protéines E et M ont été séquencés comme ci-dessus, à l'aide des amorces : S/E/F2/+/26082 et S/E/R2/-/26394, S/M/F2/+/26330, S/M/R2/-/27078 précitées et des amorces S/M/+/26636-26655 et S/M/-/26567-26548. Ils ont ensuite été clonés, comme ci-dessus, pour donner les plasmides dénommés SARS-E et SARS-M. L'ADN de ces clones a ensuite été isolé et séquencé à l'aide des amorces universelles M13 forward et M 13 reverse ainsi que des amorces S/M/+/26636 et S/M/-/26548 précitées.

La séquence de l'amplicon représentant l'ADNc codant pour la protéine E (SEQ ID NO : 13) de la souche de SARS-CoV issue du prélèvement n°031589 ne comporte pas de différences par rapport aux séquences correspondantes des isolats AY274119.3-Tor2 et AY278741-Urbani. La séquence de la protéine E de la souche de SARS-CoV 031589 correspond à la séquence SEQ ID NO : 14 dans la liste de séquences jointe en annexe.

Le plasmide, dénommé SARS-E a été déposé sous le n° I-3046, le 28 mai 2003, auprès de la Collection Nationale de Cultures de Microorganismes, 25 rue du Docteur Roux, 75724 Paris Cedex 15 ; il contient la séquence d'ADNc codant pour la protéine E de la souche de SARS-CoV issue du prélèvement répertorié sous le n° 031589, telle que définie ci-dessus, laquelle séquence correspondant aux nucléotides des positions 26082 à 26413 (SEQ ID NO :15), en référence à la séquence Genbank n° d'accès AY274119.3.

La séquence de l'amplicon représentant l'ADNc codant pour la M (SEQ ID NO :16) de la souche de SARS-CoV issue du prélèvement n°031589 ne comporte pas de différences par rapport à la séquence correspondante de l'isolat AY274119.3-Tor2. En revanche, en position 26857, l'isolat AY278741-Urbani comporte un c et la séquence de la souche de SARS-CoV issue du prélèvement répertorié sous le n°031589 un t. Cette mutation aboutit à une modification de la séquence en acides aminés de la protéine correspondante: en position 154, une proline (AY278741-Urbani) est changée en sérine dans la souche de SARS-CoV issue du prélèvement répertorié sous le n°031589. La séquence de la protéine M de la souche de SARS-CoV issue du prélèvement répertorié sous le n°031589 correspond à la séquence SEQ ID NO :17 dans la liste de séquences jointe en annexe.

Le plasmide, dénommé SARS-M a été déposé sous le n° I-3047, le 28 mai 2003, auprès de la Collection Nationale de Cultures de Microorganismes, 25 rue du Docteur Roux, 75724 Paris Cedex 15 ; il contient la séquence d'ADNc codant pour la protéine M de la souche de SARS-CoV issue du prélèvement répertorié sous le n° 031589, telle que définie ci-dessus ; laquelle séquence correspondant aux nucléotides des positions 26330 à 27098 (SEQ ID NO :18), en référence à la séquence Genbank n° d'accès AY274119.3.

### 2.3) ADNc correspondant aux ORF3, ORF4, ORF7 à ORF11

La même stratégie d'amplification, de clonage et de séquençage a été utilisée pour obtenir les fragments d'ADNc correspondant respectivement aux ORF suivantes: ORF 3, ORF4, ORF7, ORF8, ORF9, ORF10 et ORF11. Les couples d'amorces utilisées pour la première amplification sont :
- ORF3 et ORF4 : S/SE/F1/+/25069-25088 et S/SE/R1/-/26300-26281
- ORF7 à ORF11 : S/MN/F1/+/26898-26917 et S/MN/R1/-/28287-28266

Les couples d'amorces utilisées pour la deuxième amplification sont :
- ORF3 et ORF4 : S/SE/F2/+/25110-25129 et S/SE/R2/-/26244-26225
- ORF7 à ORF11 : S/MN/F2/+/26977-26996 et S/MN/R2/-/28218-28199

Les conditions de la première amplification (RT-PCR) sont les suivantes : 45 min à 42°C, 10 min à 55°C, 2 min à 94°C suivi de 40 cycles comprenant une étape de dénaturation à 94°C pendant 15 sec, une étape d'hybridation à 58°C pendant 30 sec et une étape d'élongation à 68°C pendant 1 min, avec 5 sec d'incrément par cycle et enfin une étape d'élongation terminale à 68°C pendant 7 min.
Les conditions de la PCR nichée sont les suivantes : une étape de dénaturation à 94°C pendant 2 min a été suivie de 40 cycles comprenant une étape de dénaturation à 94°C pendant 20 sec, une étape d'hybridation à 58°C pendant 30 sec et une étape d'élongation à 72°C pendant 50 sec, avec 4 sec d'incrément par cycle et enfin une étape d'élongation terminale à 72°C pendant 7 min.

Les produits d'amplification obtenus correspondant aux ADNc contenant respectivement les ORF3 et 4 et les ORF7 à 11 ont été séquencés à l'aide des amorces : S/SE/+/25363, S/SE/+/25835, S/SE/-/25494, S/SE/-/25875, S/MN/+/27839, S/MN/+/27409, S/MN/-/27836 S/MN/-/27799 et clonés comme ci-dessus pour les autres ORF, pour donner les plasmides dénommés SARS-SE et SARS-MN. L'ADN de ces clones a été isolé et séquencé à l'aide de ces mêmes amorces et des amorces universelles M 13 sens et M13 anti-sens.

La séquence de l'amplicon représentant l'ADNc de la région contenant les ORF 3 et 4 (SEQ ID NO :7) de la souche de SARS-CoV issue du prélèvement n°031589 comporte une différence nucléotidique par rapport à la séquence correspondante de l'isolat AY274119-Tor2. Cette mutation en position 25298 aboutit à une modification de la séquence en acides aminés de la protéine correspondante (ORF 3): en position 11, une arginine (AY274119-Tor2) est changée en glycine dans la souche de SARS-CoV issue du prélèvement n°031589. En revanche, aucune mutation n'a été identifiée par rapport à la séquence correspondante de l'isolat AY278741-Urbani. Les séquences des ORF 3 et 4 la souche de SARS-CoV issue du prélèvement n°031589 correspondent respectivement aux séquences SEQ ID NO :10 et 12 dans la liste de séquences jointe en annexe.

Le plasmide, dénommé SARS-SE a été déposé sous le n° I-3126, le 13 novembre 2003, auprès de la Collection Nationale de Cultures de Microorganismes, 25 rue du Docteur Roux, 75724 Paris Cedex 15 ; il contient l'ADNc correspondant à la région située entre l'ORF-S et l'ORF-E et chevauchant l'ORF-E de la souche de SARS-CoV issue du prélèvement répertorié sous le n° 031589, telle que définie ci-dessus, laquelle région correspondant aux nucléotides des positions 25110 à 26244 (SEQ ID NO :8), en référence à la séquence Genbank n° d'accès AY274119.3,

La séquence de l'amplicon représentant l'ADNc correspondant à la région contenant les ORF7 à ORF 11 (SEQ ID NO :19) de la souche de SARS-CoV issue du prélèvement n°031589 ne comporte pas de différences par rapport aux séquences correspondantes des isolats AY274119-Tor2 et AY278741-Urbani. Les séquences des ORF7 à 11 de la souche de SARS-CoV issue du prélèvement n°031589 correspondent respectivement aux séquences SEQ ID NO : 22, 24, 26, 28 et 30 dans la liste de séquences jointe en annexe.

Le plasmide dénommé SARS-MN a été déposé sous le n° I-3125, le 13 novembre 2003, auprès de la Collection Nationale de Cultures de Microorganismes, 25 rue du Docteur Roux, 75724 Paris Cedex 15 ; il contient la séquence d'ADNc correspondant à la région située entre l'ORF-M et l'ORF-N de la souche de SARS-CoV issue du prélèvement répertorié sous le n° 031589 et prélevée à Hanoi, telle que définie ci-dessus, laquelle séquence correspondant aux nucléotides des positions 26977 à 28218 (SEQ ID NO :20 ), en référence à la séquence Genbank n° d'accès AY274119.3.

La séquence de l'amplicon représentant l'ADNc correspondant à la région contenant les ORF7 à ORF11 (SEQ ID NO :19) de la souche de SARS-CoV issue du prélèvement n°031589 ne comporte pas de différences par rapport aux séquences correspondantes des isolats AY274119-Tor2 et AY278741-Urbani. Les séquences des ORF7 à 11 de la souche de SARS-CoV issue du prélèvement n°031589 correspondent respectivement aux séquences SEQ ID NO : 22, 24, 26, 28 et 30 dans la liste de séquences jointe en annexe.

### 2.4) ADNc codant pour la protéine N et incluant les ORF13 et ORF14

L'ADNc a été synthétisé et amplifié comme décrit ci-dessus pour les fragments Sa et Sb. De manière plus précise, le mélange réactionnel contenant : 5 µl d'ARN, 5 µl d'H₂O ppi 4 µl de tampon de reverse transcriptase 5X, 2 µl de dNTP (5 mM), 2 µl d'oligo 20T (5 µM), 0,5 µl de RNasin (40 Ul/ul) et 1, 5 µl de AMV-RT (10 Ul/ul Promega) a été incubé dans un thermocycleur dans les conditions suivantes : 45 min à 42°C, 15 min à 55°C, 5 min à 95°C, puis il a été maintenu à +4°C.

Une première amplification PCR a été réalisée avec la paire d'amorces S/N/F3/+/28023 et S/N/R3/-/29480.

Le mélange réactionnel comme ci-dessus pour l'amplification des fragments S1 et S2 a été incubé dans un thermocycleur, dans les conditions suivantes : une étape initiale de dénaturation à 94°C pendant 2 min a été suivie de 40 cycles comprenant une étape de dénaturation à 94°C pendant 20 sec, une étape d'hybridation à 55°C pendant 30 sec puis une étape d'élongation à 72°C pendant 1 min 30 sec avec 10 sec d'élongation supplémentaire à chaque cycle, puis d'une étape finale d'élongation à 72°C pendant 5 min.

L'amplicon obtenu à la première amplification PCR a subi une seconde étape d'amplification PCR (PCR nichée) avec la paires d'amorce S/N/F4/+/28054 et S/N/R4/-/29430 dans des conditions identiques à celles de la première amplification.

Le produit d'amplification obtenu correspondant à l'ADNc codant pour la protéine N de la souche de SARS-CoV issue du prélèvement n°031589a été séquencé à l'aide des amorces : S/N/F4/+/28054, S/N/R4/-/29430, S/N/+/28468, S/N/+/28918 et S/N/-/28607 et cloné comme ci-dessus pour les autres ORF, pour donner le plasmide dénommé SARS-N. L'ADN de ces clones a été isolé et séquencé à l'aide des amorces universelles M13 sens et M13 anti-sens, ainsi que des amorces S/N/+/28468, S/N/+/28918 et S/N/-/28607.

La séquence de l'amplicon représentant l'ADNc correspondant à l'ORF-N et incluant les ORF13 et ORF14 (SEQ ID NO :36) de la souche de SARS-CoV issue du prélèvement n°031589 ne comporte pas de différences par rapport aux séquences correspondantes des isolats AY274119.3-Tor2 et AY278741-Urbani. La séquence de la protéine N de la souche de SARS-CoV issue du prélèvement n°031589 correspond à la séquence SEQ ID NO : 37 dans la liste de séquences jointe en annexe.

Les séquences des ORF13 et 14 de la souche de SARS-CoV issue du prélèvement n°031589 correspondent respectivement aux séquences SEQ ID NO : 32 et 34 dans la liste de séquences jointe en annexe.

Le plasmide dénommé SARS-N a été déposé sous le n° I-3048, le 5 juin 2003, auprès de la Collection Nationale de Cultures de Microorganismes, 25 rue du Docteur Roux, 75724 Paris Cedex 15 ; il contient l'ADNc codant pour la protéine N de la souche de SARS-CoV issue du prélèvement répertorié sous le n° 031589, telle que définie ci-dessus, laquelle séquence correspondant aux nucléotides des positions 28054 à 29430 (SEQ ID NO :38), en référence à la séquence Genbank n° d'accès AY274119.3.

### 2.5) extrémités 5' et 3' non-codantes

### a) extrémité 5'non-codante (5'NC)

### a₁) synthèse de l'ADNc

Les ARN issus du prélèvement 031589, extraits comme ci-dessus, ont été soumis à une transcription inverse dans les conditions suivantes :
L'ARN (15 µl) et l'amorce S/L/-/443 (3 µl à la concentration de 5µm, ont été incubés 10 min à 75°C.

Ensuite, du Tampon de transcriptase inverse 5X (6 µl, INVITROGEN), des dNTP 10 mM (1 µl), du DTT 0,1M (3 µl) ont été ajoutés et le mélange a été incubé à 50°C pendant 3 min.

Enfin la transcriptase inverse (3 µl de Superscript®, INVITROGEN) a été ajoutée au mélange précédent qui a été incubé à 50°C pendant 1h30 puis à 90 °C pendant 2 min.

L'ADNc ainsi obtenu a été purifié à l'aide du kit *QIAquick PCR purification* (QIAGEN), selon les recommandations du fabricant.

### b₁) Réaction à la Terminal Transferase (TdT)

L'ADNc (10 µl) est incubé 2 min à 100°C, conservé dans la glace, puis sont ajoutés : H₂O (2,5 µl), tampon TdT 5X (4 µl, AMERSHAM), dATP 5mM (2 µl) et TdT (1,5 µl, AMERSHAM). Le mélange ainsi obtenu est incubé 45 min à 37°C puis 2 min à 65°C.

Le produit obtenu est amplifié par une première réaction PCR à l'aide des amorces : S/L/-/225-206 et ancre 14T : 5'-AGATGAATTCGGTACCTTTTTTTTTTTTTT-3' (SEQ ID NO :68). Les conditions de l'amplification sont les suivantes : une étape initiale de dénaturation à 94°C pendant 2 min est suivie de 10 cycles comprenant une étape de dénaturation à 94°C pendant 10 sec, une étape d'hybridation à 45°C pendant 30 sec puis une étape d'élongation à 72°C pendant 30 sec puis de 30 cycles comprenant une étape de dénaturation à 94°C pendant 10 sec, une étape d'hybridation à 50°C pendant 30 sec puis une étape d'élongation à 72°C pendant 30 sec, puis d'une étape finale d'élongation à 72°C pendant 5 min.

Le produit de la première amplification PCR a subi une seconde étape d'amplification à l'aide des amorces : S/L/-/204-185 et ancre 14T précitée dans des conditions identiques à celles de la première amplification. L'amplicon ainsi obtenu a été purifié, séquencé à l'aide de l'amorce S/L/-/182-163 puis il a été cloné comme ci-dessus pour les différentes ORF, pour donner le plasmide dénommé SARS-5'NC. L'ADN de ce clone a été isolé et séquencé à l'aide des amorces universelles M13 sens et M13 anti-sens et de l'amorce S/L/-/182-163 précitée.

L'amplicon représentant l'ADNc correspondant à l'extrémité 5'NC de la souche de SARS-CoV issue du prélèvement répertorié sous le n° 031589 correspond à la séquence SEQ ID NO : 72 dans la liste de séquences jointe en annexe ; cette séquence ne comporte pas de différences par rapport aux séquences correspondantes des isolats AY274119.3-Tor2 et AY278741-Urbani.

Le plasmide dénommé SARS-5'NC a été déposé sous le n° 1- 3124, le 7 novembre 2003, auprès de la Collection Nationale de Cultures de Microorganismes, 25 rue du Docteur Roux, 75724 Paris Cedex 15 ; il contient l'ADNc correspondant à l'extrémité 5'non codante du génome de la souche de SARS-CoV issue du prélèvement répertorié sous le n° 031589, telle que définie ci-dessus, laquelle séquence correspondant aux nucléotides des positions 1 à 204 (SEQ ID NO :39), en référence à la séquence Genbank n° d'accès AY274119.3.

### b) extrémité 3'non-codante (3'NC)

### a₁) synthèse de l'ADNc

Les ARN issus du prélèvement 031589, extraits comme ci-dessus, ont été soumis à une transcription inverse, selon le protocole suivant : le mélange réactionnel contenant : ARN (5 µl), H₂O (5 µl), tampon de transcriptase inverse 5X (4 µl), dNTP 5 mM (2 µl), Oligo 20T 5µM (2 µl), RNasin 40 U/ µl (0,5 µl) et RT-AMV 10 UI/ µl (1,5 µl, PROMEGA) a été incubé dans un thermocycleur, dans les conditions suivantes : 45 min à 42°C, 15 min à 55°C, 5 min à 95°C, puis il a été maintenu à +4°C.

L'ADNc obtenu a été amplifié par une première réaction PCR à l'aide des amorces S/N/+/28468-28487 et ancre 14T précitée. Les conditions de l'amplification sont les suivantes : une étape initiale de dénaturation à 94°C pendant 2 min est suivie de 10 cycles comprenant une étape de dénaturation à 94°C pendant 20 sec, une étape d'hybridation à 45°C pendant 30 sec puis une étape d'élongation à 72°C pendant 50 sec puis de 30 cycles comprenant une étape de dénaturation à 94°C pendant 20 sec, une étape d'hybridation à 50°C pendant 30 sec puis une étape d'élongation à 72°C pendant 50 sec, puis d'une étape finale d'élongation à 72°C pendant 5 min.

Le produit de la première amplification PCR a subi une seconde étape d'amplification à l'aide des amorces S/N/+/28933-28952 et ancre 14T précitée, dans des conditions identiques à celles de la première amplification. L'amplicon ainsi obtenu a été purifié, séquencé à l'aide de l'amorce S/N/+/29257-29278 et cloné comme ci-dessus pour les différentes ORF, pour donner le plasmide dénommé SARS-3'NC. L'ADN de ce clone a été isolé et séquencé à l'aide des amorces universelles M13 sens et M13 anti-sens et de l'amorce S/N/+/29257-29278 précitée.

L'amplicon représentant l'ADNc correspondant à l'extrémité 3'NC de la souche de SARS-CoV issue du prélèvement répertorié sous le n° 031589 correspond à la séquence SEQ ID NO :73 dans la liste de séquences jointe en annexe ; cette séquence ne comporte pas de différences par rapport aux séquences correspondantes des isolats AY274119.3-Tor2 et AY278741-Urbani.

Le plasmide dénommé SARS-3'NC a été déposé sous le n° I-3123 le 7 novembre 2003, auprès de la Collection Nationale de Cultures de Microorganismes, 25 rue du Docteur Roux, 75724 Paris Cedex 15. ; il contient la séquence d'ADNc correspondant à l'extrémité 3'non codante du génome de la souche de SARS-CoV issue du prélèvement répertorié sous le n° 031589, telle que définie ci-dessus, laquelle séquence correspondant à celle située entre le nucléotide en position 28933 à 29727 (SEQ ID NO :40), en référence à la séquence Genbank n° d'accès AY274119.3, se termine par une série de nucléotides a.

### 2.6) ORF1a et ORF1b

L'amplification de la région 5' contenant les ORF1a et ORF1b du génome du SARS-CoV issu du prélèvement 031589 a été réalisée en pratiquant des réactions de RT-PCR suivies de PCR nichées selon les mêmes principes que ceux précédemment décrits pour les autres ORF. Les fragments amplifiés sont chevauchants sur plusieurs dizaines de bases, permettant ainsi la reconstruction informatique de la séquence complète de cette partie du génome. En moyenne, les fragments amplifiés sont de deux kilobases.

14 fragments chevauchants dénommés L0 à L12 ont ainsi été amplifiés à l'aide des amorces suivantes :

**Tableau II: Amorces utilisées pour l'amplification de la région 5'(ORF1a et ORF1b)**

| **REGION AMPLIFIEE ET SEQUENCEE (ne tient pas compte des amorces)** | **Amorce sens RT-PCR** | **Amorce antisens RT-PCR** | **Amorce sens PCR nichée** | **Amorce antisens PCR nichée** |
|---|---|---|---|---|
| **L0 50-480** | S/L0/F1/+30 | S/L0/R1/-481 | | |
| **L1 231-2240** | S/L1/F1/+147 | S/L1/R1/-2336 | S/L1/F2/+211 | S/L1/R2/-2241 |
| **L2 2156-4167** | S/L2/F1/+2033 | S/L2/R1/-4192 | S/L2/F2/+2136 | S/L2/R2/-4168 |
| **L3 3913-5324** | S/L3bis/F1/+3850 | S/L3bis/R1/-5365 | S/L3bis/F2/+3892 | S/L3bis/R2/-5325 |
| **L4b 4952-6023** | S/L4b/F1/+4878 | S/L4b/R1/-6061 | S/L4b/F2/+4932 | S/L4b/R2/-6024 |
| **L4 5325-7318** | S/L4/F1/+5272 | S/L4/R1/-7392 | S/L4/F2/+5305 | S/L4/R2/-7323 |
| **L5 7296-9156** | S/L5/F1/+7111 | S/L5/R1/-9253 | S/L5/F2/+7275 | S/L5/R2/-9157 |
| **L6 9053-11066** | S/L6/F1/+8975 | S/L6/R1/-11151 | S/L6/F2/+9032 | S/L6/R2/-11067 |
| **L7 10928-12962** | S/L7/F1/+10883 | S/L7/R1/-13050 | S/L7/F2/+10928 | S/L7/R2/-12963 |
| **L8 12835-14834** | S/L8/F1/+12690 | S/L8/R1/-14857 | S/L8/F2/+12815 | S/L8/R2/-14835 |
| **L9 14765-16624** | S/L9/F1/+14688 | S/L9/R1/-16678 | S/L9/F2/+14745 | S/L9/R2/-16625 |
| **L10 16534-18570** | S/L10/F1/+16451 | S/L10/R1/-18594 | S/L10/F2/+16514 | S/L10/R2/-18571 |
| **L11 18521-20582** | S/L11/F1/+18441 | S/L11/R1/-20612 | S/L11/F2/+18500 | S/L11/R2/-20583 |
| **L12 20338-22205.** | S/L12/F1/+20279 | S/L12/R1/-22229 | S/L12/F2/+20319 | S/L12/R2/-22206 |

Tous les fragments ont été amplifiés dans les conditions suivantes, excepté le fragment L0 qui a été amplifié comme décrit ci-dessus pour l'ORF-M :
- RT-PCR: 30 min à 42°C, 15 min à 55°C, 2 min à 94°C, puis l'ADNc obtenu est amplifié dans les conditions suivantes : 40 cycles comprenant : une étape de dénaturation à 94°C pendant 15 sec, une étape d'hybridation à 58°C pendant 30 sec puis une étape d'élongation à 68°C pendant 1 min 30 sec, avec 5 sec d'élongation supplémentaire à chaque cycle, puis une étape finale d'élongation à 68°C pendant 7 min.
- PCR nichée : une étape initiale de dénaturation à 94°C pendant 2 min est suivie de 35 cycles comprenant : une étape de dénaturation à 94°C pendant 15 sec, une étape d'hybridation à 60°C pendant 30 sec puis une étape d'élongation à 72°C pendant 1 min 30 sec, avec 5 sec d'élongation supplémentaire à chaque cycle, puis une étape finale d'élongation à 72°C pendant 7 min.

Les produits d'amplifications ont été séquencés à l'aide des amorces définies dans le Tableau III ci-après :

**Tableau III : Amorces utilisées pour le séquençage de la région 5' (ORF1a et ORF1b)**

| Noms | Séquences (SEQ ID NO : 76 à 139) |
|---|---|
| S/L3/+/4932 | 5'-CCACACACAGCTTGTGGATA-3' |
| S/L4/+/6401 | 5'-CCGAAGTTGTAGGCAATGTC-3' |
| S/L4/+/6964 | 5'-TTTGGTGCTCCTTCTTATTG-3' |
| S/L4/-/6817 | 5'-CCGGCATCCAAACATAATTT-3' |
| S/L5/-/7633 | 5'-TGGTCAGTAGGGTTGATTGG-3' |
| S/L5/-/8127 | 5'-CATCCTTTGTGTCAACATCG-3' |
| S/L5/-/8633 | 5'-GTCACGAGTGACACCATCCT-3' |
| S/L5/+/7839 | 5'-ATGCGACGAGTCTGCTTCTA-3' |
| S/L5/+/8785 | 5'-TTCATAGTGCCTGGCTTACC-3' |
| S/L5/+/8255 | 5'-ATCTTGGCGCATGTATTGAC-3' |
| S/L6/-/9422 | 5'-TGCATTAGCAGCAACAACAT-3' |
| S/L6/-/9966 | 5'-TCTGCAGAACAGCAGAAGTG-3' |
| S/L6/-/10542 | 5'-CCTGTGCAGTTTGTCTGTCA-3' |
| S/L6/+/10677 | 5'-CCTTGTGGCAATGAAGTACA-3' |
| S/L6/+/10106 | 5'-ATGTCATTTGCACAGCAGAA-3' |
| S/L6/+/9571 | 5'-CTTCAATGGTTTGCCATGTT-3' |
| S/L7/-/11271 | 5'-TGCGAGCTGTCATGAGAATA-3' |
| S/L7/-/11801 | 5'-AACCGAGAGCAGTACCACAG-3' |
| S/L7/-/12383 | 5'-TTTGGCTGCTGTAGTCAATG-3' |
| S/L7/+/12640 | 5'-CTACGACAGATGTCCTGTGC-3' |
| S/L7/+/12088 | 5'-GAGCAGGCTGTAGCTAATGG-3' |
| S/L7/+/11551 | 5'-TTAGGCTATTGTTGCTGCTG-3' |
| S/L8/-13160 | 5'-CAGACAACATGAAGCACCAC-3' |
| S/L8/-/13704 | 5'-CGCTGACGTGATATATGTGG-3' |
| S/L8/-14284 | 5'-TGCACAATGAAGGATACACC-3' |
| S/L8/+/14453 | 5'-ACATAGCTCGCGTCTCAGTT-3' |
| S/L8/+/13968 | 5'-GGCATTGTAGGCGTACTGAC-3' |
| S/L8/+/13401 | 5'-GTTTGCGGTGTAAGTGCAG-3' |
| S/L9/-15098 | 5'-TAGTGGCGGCTATTGACTTC-3' |
| S/L9/-15677 | 5'-CTAAACCTTGAGCCGCATAG-3' |
| S/L9/-16247 | 5'-CATGGTCATAGCAGCACTTG-3' |
| S/L9/+16323 | 5'-CCAGGTTGTGATGTCACTGAT-3' |
| S/L9/+15858 | 5'-CCTTACCCAGATCCATCAAG-3' |
| S/L9/+15288 | 5'-CGCAAACATAACACTTGCTG-3' |
| S/L10/-16914 | 5'-AGTGTTGGGTACAAGCCAGT-3' |
| S/L 10/-17466 | 5'-GTTCCAAGGAACATGTCTGG-3' |
| S/L 10/-18022 | 5'-AGGTGCCTGTGTAGGATGAA-3' |
| S/L10/+18245 | 5'-GGGCTGTCATGCAACTAGAG-3' |
| S/L10/+17663 | 5'-TCTTACACGCAATCCTGCTT-3' |
| S/L10/+17061 | 5'-TACCCATCTGCTCGCATAGT-3' |
| S/L11/-/18877 | 5'-GCAAGCAGAATTAACCCTCA-3' |
| S/L11/-19396 | 5'-AGCACCACCTAAATTGCATC-3' |
| S/L11/-20002 | 5'-TGGTCCCTTTGAAGGTGTTA-3' |
| S/L11/+20245 | 5'-TCGAACACATCGTTTATGGA-3' |
| S/L11/+/19611 | 5'-GAAGCACCTGTTTCCATCAT-3' |
| S/L11/+/19021 | 5'-ACGATGCTCAGCCATGTAGT-3' |
| SARS/L1/F3/+800 | 5'-GAGGTGCAGTCACTCGCTAT-3' |
| SARS/L1/F4/+1391 | 5'-CAGAGATTGGACCTGAGCAT-3' |
| SARS/L1/F5/+1925 | 5'-CAGCAAACCACTCAATTCCT-3' |
| SARS/L1/R3/-1674 | 5'-AAATGATGGCAACCTCTTCA-3' |
| SARS/L1/R4/-1107 | 5'-CACGTGGTTGAATGACTTTG-3' |
| SARS/L1/R5/-520 | 5'-ATTTCTGCAACCAGCTCAAC-3' |
| SARS/L2/F3/+2664 | 5'-CGCATTGTCTCCTGGTTTAC-3' |
| SARS/L2/F4/+3232 | 5'-GAGATTGAGCCAGAACCAGA-3' |
| SARS/L2/F5/+3746 | 5'-ATGAGCAGGTTGTCATGGAT-3' |
| SARS/L2/R3/-3579 | 5'-CTGCCTTAAGAAGCTGGATG-3' |
| SARS/L2/R4/-2991 | 5'-TTTCTTCACCAGCATCATCA-3' |
| SARS/L2/R5/-2529 | 5'-CACCGTTCTTGAGAACAACC-3' |
| SARS/L3/F3/+4708 | 5'-TCTTTGGCTGGCTCTTACAG-3' |
| SARS/L3/F4/+5305 | 5'-GCTGGTGATGCTGCTAACTT-3' |
| SARS/L3/F5/+5822 | 5'-CCATCAAGCCTGTGTCGTAT-3' |
| SARS/L3/R3/-5610 | 5'-CAGGTGGTGCAGACATCATA-3' |
| SARS/L3/R4/-4988 | 5'-AACATCAGCACCATCCAAGT-3' |
| SARS/L3/R5/-4437 | 5'-ATCGGACACCATAGTCAACG-3' |

Les séquences des fragments L0 à L12 de la souche de SARS-CoV issue du prélèvement répertorié sous le n° 031589, correspondent respectivement aux séquences SEQ ID NO :41 à SEQ ID NO :54 dans la liste de séquences jointe en annexe. Parmi ces séquences, seule celle correspondant aux fragments L5 comporte une différence nucléotidique par rapport à la séquence correspondante de l'isolat AY278741-Urbani. Cette mutation t/c en position 7919 aboutit à une modification de la séquence en acides aminés de la protéine correspondante, codée par l'ORF 1a: en position 2552, une valine (codon gtt ; AY278741) est changée en alanine (codon gct) dans la souche de SARS-CoV 031589. En revanche, aucune mutation n'a été identifiée par rapport à la séquence correspondante de l'isolat AY274119.3-Tor2. Les autres fragments ne présentent pas de différences par rapport aux séquences correspondantes des isolats Tor2 et Urbani.

### Exemple 2 : Production et purification de protéines N et S recombinantes de la souche de SARS-CoV issue du prélèvement répertorié sous le numéro 031589

La protéine entière N et deux fragments polypeptidiques de la protéine S de la souche de SARS-CoV issue du prélèvement répertorié sous le numéro 031589 ont été produites chez *E. coli,* sous forme de protéines de fusion comprenant une étiquette polyhistidine N-ou C-terminale. Dans les deux polypeptides S, les séquences hydrophobes N et C-terminales de la protéine S (peptide signal : positions 1 à 13 et hélice transmembranaire : positions 1196 à 1218) ont été délétées alors que l'hélice β (positions 565 à 687) et les deux motifs de type coiled-coils (positions 895 à 980 et 1155 à 1186) de la protéine S ont été préservés. Ces deux polypeptides sont constitués par : un fragment long (S_{L}) correspondant aux positions 14 à 1193 de la séquence en acides aminés de la protéine S et un fragment court (S_{C}) correspondant aux positions 475 à 1193 de la séquence en acides aminés de la protéine S.

### 1) Clonage des ADNc N, S_{L} et S_{C} dans les vecteurs d'expression pIVEX2.3 et pIVEX2.4

Les ADNc correspondant à la protéine N et aux fragments S_{L} et S_{C} ont été amplifiés par PCR dans des conditions standard, à l'aide de l'ADN polymérase Platinium Pfx® (INVITROGEN). Les plasmides SRAS-N et SRAS-S ont été utilisés comme matrice et les oligonucléotides suivants comme amorces :
5'-CCCATATGTCTGATAATGGACCCCAATCAAAC-3' (N sens, SEQ ID NO :55)
5'-CCCCCGGGTGCCTGAGTTGAATCAGCAGAAGC-3' (N antisens, SEQ ID NO :56)
5'-CCCATATGAGTGACCTTGACCGGTGCACCAC-3' (S_{C} sens, SEQ ID NO :57)
5'-CCCATATGAAACCTTGCACCCCACCTGCTC-3' (S_{L} sens, SEQ ID NO :58)
5'-CCCCCGGGTTTAATATATTGCTCATATTTTCCC-3' (S_{C} et S_{L} antisens, SEQ ID NO :59).

Les amorces sens introduisent un site *NdeI* (souligné) alors que les amorces antisens introduisent un site *XmaI* ou *SmaI* (souligné). Les 3 produits d'amplification on été purifiés sur colonne (kit *QIAquick PCR Purification,* QIAGEN) et clonés dans un vecteur approprié. L'ADN plasmidique purifié des 3 constructions (kit *QIAFilter Midi Plasmid,* QIAGEN) a été vérifié par séquençage et digéré par les enzymes *NdeI* et *XmaI.* Les 3 fragments correspondants aux ADNc N, S_{L} et S_{C} ont été purifiés sur gel d'agarose puis insérés dans les plasmides pIVEX2.3MCS (étiquette polyhistidine C-terminale) et pIVEX2.4d (étiquette polyhistidine N-terminale) préalablement digérés par les mêmes enzymes. Après vérification des constructions, les 6 vecteurs d'expressions ainsi obtenus (pIV2.3N, pIV2.3S_{C}, pIV2.3S_{L}, pIV2.4N, pIV2.4S_{C} également dénommé pIV2.4S_{I}, pIV2.4S_{L}) ont été ensuite utilisés, d'une part pour tester l'expression des protéines *in-vitro,* et d'autre part pour transformer la souche bactérienne BL21 (DE3)pDIA 17 (NOVAGEN). Ces constructions codent pour des protéines dont la masse moléculaire attendue est la suivante : pIV2.3N (47174 Da), pIV2.3S_{C} (82897 Da), pIV2.3S_{L} (132056 Da), pIV2.4N (48996 Da), pIV2.4S₁ (81076 Da) et pIV2.4S_{L}(133877 Da). Des bactéries transformées par pIV2.3N ont été déposées à la CNCM le 23 octobre 2003, sous le numéro I-3117, et des bactéries transformées par pIV2.4S_{I} ont été déposées à la CNCM le 23 octobre 2003, sous le numéro I-3118.

### 2) Analyse de l'expression des protéines recombinantes in-vitro et in vivo

L'expression de protéines recombinantes à partir des 6 vecteurs recombinants a été testée, dans un premier temps, dans un système *in-vitro* (RTS 100, Roche). Les protéines produites *in vitro,* après une incubation des vecteurs recombinants pIVEX, 4h à 30°C, dans le système RTS100, ont été analysées par western-blot à l'aide d'un anticorps anti-(his)₆ couplé à la péroxydase. Le résultat d'expression *in-vitro* (Figure 1) montre que seule la protéine N est exprimée en quantités importantes, cela quelle que soit la position, N- ou C-terminale, de l'étiquette polyhistidine. Dans une seconde étape, l'expression des protéines N et S a été testée *in-vivo* à 30°C dans du milieu LB, en présence ou en l'absence d'inducteur (IPTG 1mM). La protéine N est très bien produite dans ce système bactérien (Figure 2) et se retrouve principalement dans une fraction soluble après lyse des bactéries. En revanche, la version longue de S (S_{L}) est très peu produite et complètement insoluble (Figure 3). La version courte (S_{C}) présente également une très faible solubilité, mais un taux d'expression beaucoup plus élevé que celui de la version longue. Par ailleurs, la construction S_{C} fusionnée à une étiquette polyhistidine en position C-terminale présente une taille plus faible que celle attendue. Une expérience d'immunodétection avec un anticorps anti-polyhistidine a montré que cette construction était incomplète. En conclusion, les deux constructions, pIV2.3N et pIV2.4S_{I}, exprimant respectivement la protéine N entière fusionnée à l'étiquette polyhistidine en C-terminal et la protéine S courte fusionnée à l'étiquette polyhistidine en N-terminal, ont été retenues pour produire les deux protéines en grande quantité afin de les purifier. Les plasmides pIV2.3N et pIV2.4S_{I} ont été déposés respectivement sous le n° I-3117 et I-3118 auprès de la CNCM, 25 rue du Docteur Roux, 75724 PARIS 15, le 23 octobre 2003.

### 3) Analyse de l'activité antigénique des protéines recombinantes

L'activité antigénique des protéines N, S_{L} et S_{C} a été testée par western-blot, à l'aide de deux échantillons de sérum, provenant d'un même patient infecté par le SARS-CoV, prélevés 8 jours (M12) et 29 jours-(M13) après le début des symptômes du SRAS. Le protocole expérimental est comme décrit à l'exemple 3. Les résultats illustrés par la figure 4 montrent (i) la séroconversion du patient, et (ii) que la protéine N possède une plus forte réactivité antigénique que la protéine S courte.

### 4) Purification de la protéine N à partir de pIV2.3N

Plusieurs expériences de purification de la protéine N, produite à partir du vecteur pIV2.3N, ont été réalisées selon le protocole suivant. Les bactéries BL21(DE3)pDIA17, transformées par le vecteur d'expression pIV2.3N, ont été cultivées à 30°C dans 1 litre de milieu de culture contenant 0,1 mg/ml d'ampicilline, et induites par 1 mM IPTG quand la densité cellulaire, équivalente à A₆₀₀ = 0,8, est atteinte (environ 3 heures). Après 2 heures de culture en présence d'inducteur, les cellules ont été récupérées par centrifugation (10 min à 5000 rpm), remises en suspension dans le tampon de lyse (50 mM NaH₂PO₄, NaCl 0,3 M, 20 mM imidazole, pH 8 contenant le mélange d'inhibiteurs de protéases *Complete*® , Roche), et lysées par la presse de French (12000 psi). Après centrifugation du lysat bactérien (15 min à 12000 rpm), le surnageant (50 ml) a été déposé à un débit de 1ml/min sur une colonne (15 ml) de chélation métallique (Ni-NTA superflow, Qiagen), équilibrée par le tampon de lyse. Après lavage de la colonne par 200 ml de tampon de lyse, la protéine N a été éluée par un gradient d'imidazole (20 →250 mM) en 10 volumes de colonne. Les fractions contenant la protéine N ont été rassemblées et analysées par électrophorèse en gel de polyacrylamide en conditions dénaturantes puis coloration au bleu de Coomassie. Les résultats illustrés par la figure 5 montrent que le protocole employé permet de purifier la protéine N avec une homogénéité très satisfaisante (95%) et un rendement moyen de 15 mg de protéine par litre de culture.

### 5) Purification de la protéine S_{C} à partir de pIV2.4S_{C} (pIV2.4S₁)

Le protocole suivi pour purifier la protéine S courte est très différent de celui décrit ci-dessus car la protéine est fortement agrégée dans le système bactérien (corps d'inclusion). Les bactéries BL21(DE3)pDIA17, transformées par le vecteur d'expression pIV2.4S₁ ont été cultivées à 30°C dans 1 litre de milieu de culture contenant 0,1 mg/ml d'ampicilline, et induites par 1 mM IPTG quand la densité cellulaire, équivalente à A₆₀₀ = 0,8, est atteinte (environ 3 heures). Après 2 heures de culture en présence d'inducteur, les cellules ont été récupérées par centrifugation (10 min à 5000 rpm), remises en suspension dans le tampon de lyse (0,1 M Tris-HCl, EDTA 1 mM, pH 7,5), et lysées par la presse de French (1200 psi). Après centrifugation du lysat bactérien (15 min à 12000 rpm), le culot a été remis en suspension dans 25 ml de tampon de lyse contenant 2% Triton X100 et 10 mM β-mercaptoéthanol, puis centrifugé pendant 20 min à 12000 rpm. Le culot a été remis en suspension dans un tampon Tris-HCl 10 mM contenant 7 M urée, et mis en agitation douce pendant 30 min à température ambiante. Ce dernier lavage des corps d'inclusion avec 7 M urée est nécessaire pour éliminer la plupart des protéines membranaires d'*E*. *coli* qui co-sédimentent avec la protéine S_{C} agrégée. Après une dernière centrifugation pendant 20 min à 12000 rpm, le culot final est remis en suspension dans le tampon Tris-HCl 10 mM. L'analyse électrophorétique de cette préparation (Figure 6) montre que la protéine S courte peut être purifiée avec une homogénéité satisfaisante (environ 90%) à partir des corps d'inclusion (extrait insoluble).

### Exemple 3 : Immunodominance de la protéine N

La réactivité des anticorps présents dans le sérum des patients atteints de pneumopathie atypique causée par le coronavirus associé au SRAS (SARS-CoV), vis-à-vis des différentes protéines de ce virus, a été analysée par western-blot dans les conditions décrites ci-après.

### 1) Matériel

### a) lysat de cellules infectées par le SARS-CoV

Des cellules Vero E6 (2x10⁶) ont été infectées par le SARS-CoV (isolat répertorié sous le numéro FFM/MA104) à une multiplicité d'infection (M.O.I.) de 10⁻¹ ou 10⁻² puis incubées dans du milieu DMEM contenant 2% de SVF, à 35°C dans une atmosphère contenant 5% de CO₂. 48 heures plus tard, le tapis cellulaire a été lavé avec du PBS puis lysé avec 500 µl de tampon de dépôt préparé selon Laemmli et contenant du β-mercaptoéthanol. Les échantillons ont ensuite été bouillis 10 minutes puis soniqués 3 fois 20 secondes.

### b) anticorps

### b₁) sérum de patient atteint de pneumopathie atypique

Le sérum référencé au Centre National de Référence des virus influenzae (Région-Nord) sous le N° 20033168 est celui d'un patient français atteint d'une pneumopathie atypique causée par le SARS-CoV prélevé au jour 38 après le début des symptômes ; le diagnostic d'infection par le SARS-CoV a été réalisé par RT-PCR nichée et PCR quantitative.

### b₂) sérums polyclonaux de lapin monospécifiques dirigés contre la protéine N ou la protéine S

Les sérums sont ceux produits à partir des protéines recombinantes N et S_{C} (exemple 2), selon le protocole d'immunisation décrit à l'exemple 4 ; il s'agit du sérum du lapin P13097 (sérum anti-N) et du sérum du lapin P11135 (sérum anti-S).

### 2) Méthode

20 µl de lysat de cellules infectées par le SARS-CoV à des M.O.I. de 10⁻¹ et 10⁻² et, à titre de contrôle, 20 µl d'un lysat de cellules non infectées (mock) ont été séparés sur un gel SDS à 10% de polyacrylamide puis transférés sur une membrane de nitrocellulose. Après blocage dans une solution de PBS/lait 5%/Tween 0,1% et lavage en PBS/Tween 0,1%, cette membrane a été hybridée pendant une nuit à 4°C avec : (i) l'immun-sérum N° 20033168 dilué au 1/300, 1/1000 et 1/3000 dans le tampon PBS/BSA 1 %/Tween 0,1%, (ii) le sérum du lapin P13097 (sérum anti-N) dilué au 1/50000 dans le même tampon et (iii) le sérum du lapin P11135 (sérum anti-S) dilué au 1/10000 dans le même tampon. Après lavage en PBS/Tween, une hybridation secondaire a été réalisée à l'aide, soit d'anticorps polyclonaux de mouton dirigés contre les chaînes lourdes et légères des immunoglobulines G humaines et couplés à la peroxidase (NA933V, Amersham), soit d'anticorps polyclonaux d'âne dirigés contre les chaînes lourdes et légères des immunoglobulines G de lapin et couplés à la peroxidase (NA934V, Amersham). Les anticorps fixés ont été révélés à l'aide du kit ECL+ (Amersham) et de films d'autoradiographie Hyperfilm MP (Amersham). Une échelle de masse moléculaire (kDa) est portée sur la figure.

### 3) Résultats

La figure 7 montre que trois polypeptides de masse moléculaire apparente 35, 55 et 200 kDa sont détectés spécifiquement dans les extraits de cellules infectées par le SARS-CoV.

Afin d'identifier ces polypeptides, deux autres immunoempreintes (figure 8) ont été réalisées sur les mêmes échantillons et dans les mêmes conditions avec des anticorps polyclonaux de lapins spécifique de la nucléoprotéine N (lapin P13097, figure 8A) et de la protéine de spicule S (lapin P11135, figure 8B) Cette expérience montre que le polypeptide de 200 kDa correspond à la glycoprotéine de spicule S du SARS-CoV, que le polypeptide de 55 kDa correspond à la nucléoprotéine N tandis que le polypeptide de 35 kDa représente vraisemblablement une forme tronquée ou dégradée de la N.

Les données présentées dans la figure 7 montrent donc que le sérum 20033168 réagit fortement avec la N et beaucoup plus faiblement avec la S du SARS-CoV, puisque les polypeptides de 35 et 55 kDa sont révélés sous la forme de bandes intenses pour des dilutions de 1/300, 1/1000 et 1/3000 de l'immunsérum alors que le polypeptide de 200 kDa n'est que faiblement révélé pour une dilution de 1/300. On peut noter également qu'aucun autre polypeptide du SARS-CoV n'est détecté pour des dilutions supérieures au 1/300 du sérum 20033168.

Cette expérience indique que la réponse en anticorps spécifique de la N du SARS-CoV domine les réponses en anticorps spécifiques des autres polypeptides du SARS-CoV et en particulier la réponse en anticorps dirigée contre la glycoprotéine S. Elle indique une immunodominance de la nucléoprotéine N lors des infections humaines par le SARS-CoV.

### Exemple 4 : Préparation d'anticorps polyclonaux monospécifiques dirigés contre les protéines N et S du coronavirus associé au SRAS (SARS-CoV)

### 1) Matériel et méthode

Trois lapins (P13097, P13081, P13031) ont été immunisés avec le polypeptide recombinant purifié correspondant à l'intégralité de la nucléoprotéine (N), préparé selon le protocole décrit à l'exemple 2. Après une première injection de 0,35 mg par lapin de protéine émulsionnée en adjuvant complet de Freund (voie intradermique), les animaux ont reçus 3 injections de rappel à 3 puis 4 semaines d'intervalle, de 0,35 mg de protéine recombinante émulsionnée en adjuvant incomplet de Freund.

Trois lapins (P11135, P13042, P14001) ont été immunisés avec le polypeptide recombinant correspondant au fragment court de la protéine S (S_{C}), produit comme décrit à l'exemple 2. Comme ce polypeptide est retrouvé principalement sous la forme de corps d'inclusion dans le cytoplasme bactérien, les animaux ont reçus 4 injections intra-dermiques à 3-4 semaines d'intervalle d'une préparation de corps d'inclusion correspondant à 0,5 mg de protéine recombinante émulsionnée en adjuvant incomplet de Freund. Les 3 premières injections ont été réalisées avec une préparation de corps d'inclusion préparés selon le protocole décrit à l'exemple 2, tandis que la quatrième injection a été réalisée avec une préparation de corps d'inclusion qui ont été préparés selon le protocole décrit à l'exemple 2 puis purifiés sur gradient de saccharose et lavés en 2 % Triton X100.

Pour chaque lapin, un sérum pré-immun (p.i.) a été préparé avant la première immunisation et un immun-sérum (I.S.) 5 semaines après la quatrième immunisation.

Dans un premier temps, la réactivité des sérums a été analysée par test ELISA vis à vis de préparations de protéines recombinantes semblables à celles utilisées pour les immunisations ; les tests ELISA ont été réalisés selon le protocole et avec les réactifs tels que décrits à l'exemple 6.

Dans un deuxième temps, la réactivité des sérums a été analysée en réalisant une immunoempreinte (western blot) d'un lysat de cellules infectées par le SARS-CoV, en suivant le protocole tel que décrit à l'exemple 3.

### 2) Résultats

Les tests ELISA (figure 9) démontrent que les préparations de protéine N recombinante et de corps d'inclusion du fragment court de la protéine S (S_{C}) sont immunogènes chez l'animal et que le titre des sérums immuns est élevé (plus de 1/25000).

L'immunoempreinte (figure 8) montre que le sérum immun du lapin P13097 reconnaît deux polypeptides présents dans les lysats de cellules infectées par le SARS-CoV : un polypeptide dont la masse moléculaire apparente (50-55 kDa selon les expériences) est compatible avec celle de la nucléoprotéine N (422 résidus, masse moléculaire prédite de 46 kDa) et un polypeptide de 35 kDa, qui représente vraisemblablement une forme tronquée ou dégradée de la N.

Cette expérience montre également que le sérum du lapin P11135 reconnaît principalement un polypeptide dont la masse moléculaire apparente (180-220 kDa selon les expériences) est compatible avec une forme glycosylée de la S (1255 résidus, chaîne polypeptidique non glycosylée de 139 kDa), ainsi que des polypeptides plus légers, qui représentent vraisemblablement des formes tronquées et/ou non glycosylées de la S.

En conclusion, l'ensemble de ces expériences démontrent que des polypeptides recombinants exprimés chez *E. coli* et correspondant aux protéines N et S du SARS-CoV permettent d'induire chez l'animal des anticorps polyclonaux capables de reconnaître les formes natives de ces protéines.

### Exemple 5 : Préparation d'anticorps polyclonaux monospécifiques dirigés contre les protéines M et E du coronavirus associé au SRAS (SARS-CoV)

### 1) Analyse de la structure des protéines M et E

### a) Protéine E

La structure de la protéine E du SARS-CoV (76 acides aminés) a été *analysée in silico* , à l'aide de différents logiciels comme signalP v1.1, NetNGlyc 1.0, THMM 1.0 et 2.0 (Krogh et al., 2001, J. Mol. Biol., 305(3):567-580) ou encore TOPPRED (von Heijne, 1992, J. Mol. Biol. 225, 487-494). L'analyse montre que ce polypeptide non glycosylé est une protéine membranaire de type 1, contenant une seule hélice transmembranaire (aa 12-34 d'après THMM), et dont la plus grande partie du domaine hydrophile (42 résidus) est localisée à l'extrémité C-terminale et vraisemblablement à l'intérieur de la particule virale (endodomaine). On peut noter une inversion dans la topologie prédite par les versions 1.0 (N-ter est externe) et 2.0 (N-ter est interne) du logiciel THMM, mais que d'autres algorithmes, notamment TOPPRED et THUMBUP (Zhou et Zhou, 2003, Protein Science 12 :1547-1555) confirment une localisation externe de l'extrémité N-terminale de E.

### b) Protéine M

Une analyse similaire réalisée sur la protéine M du SARS-CoV (221 acides aminés) montre que ce polypeptide ne possède pas de peptide signal (d'après le logiciel signalP v1.1) mais trois domaines transmembranaires (résidus 15-37, 50-72, 77-99 d'après THMM2.0) et un grand domaine hydrophile (aa 100-221) localisé à l'intérieur de la particule virale (endodomaine). Elle est vraisemblablement glycosylée sur l'asparagine en position 4 (d'après NetNGlyc 1.0).

Ainsi, en accord avec les données expérimentales connues pour les autres coronavirus, il est remarquable que les deux protéines M et E présentent des endodomaines correspondant à la majeure partie des polypeptides et des ectodomaines de très petite taille.
- l'ectodomaine de E correspond vraisemblablement aux résidus 1 à 11 ou 1 à 12 de la protéine : MYSFVSEETGT(L), SEQ ID NO : 70. En effet, la probabilité associée à la localisation transmembranaire du résidu 12 est intermédiaire (0,56 d'après THMM 2.0).
- l'ectodomaine de M correspond vraisemblablement aux résidus 2 à 14 de la protéine : ADNGTITVEELKQ, SEQ ID NO : 69. En effet, la méthionine N-terminale de M est très probablement clivée du polypeptide mature car le résidu en position 2 est une Alanine (Varshavsky, 1996, 93:12142-12149).

Par ailleurs, l'analyse de l'hydrophobicité (Kyte & Doolittle, Hopp & Woods) de la protéine E met en évidence que l'extrémité C-terminale de l'endodomaine de E est hydrophile et donc vraisemblablement exposée à la surface de ce domaine. Ainsi, un peptide synthétique correspondant à cette extrémité est un bon candidat immunogène pour induire chez l'animal des anticorps dirigés contre l'endodomaine de E. En conséquence, un peptide correspondant aux 24 résidus C-terminaux de E a été synthétisé.

### 2) Préparation d'anticorps dirigés contre l'ectodomaine des protéines M et E et l'endodomaine de la protéine E

Les peptides M2-14 (ADNGTITVEELKQ, SEQ ID NO : 69), E1-12 (MYSFVSEETGTL, SEQ ID NO: 70) et E53-76 (KPTVYVYSRV KNLNSSEGVP DLLV, SEQ ID NO : 71) ont été synthétisés par Neosystem. Ils ont été couplés à la KLH (*Keyhole Limpet Hemocyanin)* à l'aide du MBS (m-maleimido-benzoyl-N-hydroxysuccinimide ester) via une cystéine ajoutée au cours de la synthèse soit en N-terminal du peptide (cas de E53-76) soit en C-terminal (cas de M2-14 et E1-12).

Deux lapins ont été immunisés avec chacun des conjugués, en suivant le protocole d'immunisation suivant : après une première injection de 0,5 mg de peptide couplé à la KLH et émulsionné en adjuvant complet de Freund (voie intradermique), les animaux reçoivent 2 à 4 injections de rappel à 3 ou 4 semaines d'intervalle de 0,25 mg de peptide couplé à la KLH et émulsionné en adjuvant incomplet de Freund.

Pour chaque lapin, un sérum pré-immun (p.i.) a été préparé avant la première immunisation et un immun-sérum (I.S.) est préparé 3 à 5 semaines après les injections de rappel.

La réactivité des sérums a été analysée par western blot à l'aide d'extraits de cellules infectées par le SRAS-CoV (figure 43B) ou à l'aide d'extraits de cellules infectées par un virus recombinant de la vaccine exprimant la protéine E (VV-TG-E, figure 43A) ou M (VV-TN-M, figure 43C) de l'isolat 031589 du SRAS-CoV.

Les immunsérums des lapins 22234 et 22240, immunisés par le conjugué KLH-E53-76, reconnaissent un polypeptide d'environ 9 à 10kD, qui est présent dans les extraits de cellules infectées par le SRAS-CoV mais absent dans les extraits de cellules non infectées (figure 43B). La masse apparente de ce polypeptide est compatible avec la masse prédite de la protéine E, qui est de 8,4 kD. De façon similaire, l'immunsérum du lapin 20047, immunisé par le conjugué KLH-E1-12, reconnaît un polypeptide présent dans les extraits de cellules infectées par le virus VV-TG-E, dont la masse molaire apparente est compatible avec celle de la protéine E (figure 43A).

L'immunsérum des lapin 20013 et 20080, immunisés par le conjugué KLH-M2-14, reconnaît un polypeptide présent dans les extraits de cellules infectées par le virus VV-TN-M (figure 43C), dont la masse molaire apparente (18 kD environ) est compatible avec celle de la glycoprotéine M, qui est de 25,1 kD et présente un point isoélectrique élevé (9.1 pour le polypeptide nu).

Ces résultats démontrent que les peptides E1-12 et E53-76 d'une part et le peptide M2-14 d'autre part permettent d'induire chez l'animal des anticorps polyclonaux capables de reconnaître les formes natives des protéines E et M respectivement du SRAS-CoV.

### Exemple 6 : Analyse de la réactivité en ELISA de la protéine N recombinante, vis-à-vis de sérums de patients atteints de SRAS

### 1) Matériel

L'antigène utilisé pour préparer les phases solides est la nucléoprotéine N recombinante purifiée préparée selon le protocole décrit à l'exemple 2.

Les sérums à tester (Tableau IV) ont été choisis sur la base des résultats d'analyse de leur réactivité par immunofluorescence (titre IF-SRAS), vis-à-vis de cellules infectées par le SARS-CoV.

**Tableau IV: Sérums testés en ELISA**

| **Référence** | **N° sérum** | **Type de sérum** | **Date du Sérum***** | **Titre IF-SRAS** |
|---|---|---|---|---|
| 3050 | A | Témoin | na* | nt** |
| 3048 | B | Témoin | na | nt |
| 033168 | D | Patient 1- SRAS | 27/04/03 (J38) | 320 |
| 033397 | E | Patient-1 SRAS | 11/05/03 (J52) | 320 |
| 032632 | F | Patient-2 SRAS | 21/03/03 (J17) | 2500 |
| 032791 | G | Patient-3 SRAS | 04/04/03 (J3) | <40 |
| 033258 | H | Patient-3 SRAS | 28/04/03 (J27) | 160 |

| | | | | |
|---|---|---|---|---|
| *na : non-applicable. ** nt : non-testé. *** les dates indiquées correspondent au nombre de jours après le début des symptômes de SRAS. | | | | |

### 2) Méthode

La protéine N (100 µl) diluée à différentes concentrations dans du tampon carbonate 0,1 M, pH 9,6 (1, 2 ou 4 µg/ml) est distribuée dans les puits de plaques ELISA, puis les plaques sont incubées une nuit à température du laboratoire. Les plaques sont lavées avec du tampon PBS-Tween, saturées avec du tampon PBS-lait écrémé-saccharose (5 %). Les sérums à tester (100 µl) préalablement dilués (1/50, 1/100, 1/200, 1/400, 1/800, 1/1600 et 1/3200) sont ajoutés, puis les plaques sont incubées 1 h à 37° C. Après 3 lavages, le conjugué anti-IgG humaines marqué à la peroxydase (référence 209-035-098, JACKSON) dilué au 1/18000 est ajouté puis les plaques sont incubées 1h à 37 °C. Après 4 lavages, le chromogène (TMB) et le substrat (H₂O₂) sont ajoutés et les plaques sont incubées 30min à température ambiante, à l'abri de la lumière. La réaction est ensuite arrêtée puis l'absorbance à 450 nm est mesurée à l'aide d'un lecteur automatique.

### 3) Résultats

Les tests ELISA (figure 10) démontrent que la préparation de protéine N recombinante est reconnue spécifiquement par les anticorps de sérums de patients atteints de SRAS prélevés en phase tardive de l'infection (≥ 17 jours après le début des symptômes) alors qu'elle n'est pas reconnue de façon significative par les anticorps d'un sérum de patient prélevé en phase précoce de l'infection (3 jours après le début des symptômes) ni par des sérums témoins de sujets non atteints de SRAS.

### Exemple 7 : Tests ELISA élaborés pour une détection très spécifique et sensible d'une infection par le coronavirus associé au SRAS, à partir de sérums de patients

### 1) Test ELISA IgG indirect

### a) Réactifs

### Préparation des plaques

Les plaques sont sensibilisées par une solution de protéine N à 2µg/mL dans un tampon PBS 10mM pH 7,2, rouge de phénol à 0,25mL /L. 100 µL de solution sont déposés dans les puits et laissés incubés à température ambiante pendant une nuit. La saturation se fait par un prélavage en tampon PBS 10mM / tween 0,1%, suivi d'un lavage avec une solution de saturation PBS, 25% lait /saccharose.

### Diluant serums

Tampon TRIS 0,48g/L, PBS 10mM, EDTA 3,7g/L,lait 15% v/v , pH 6,7

### Diluant conjugué

Tampon citrate (15g/L), tween 0,5% , serum bovin 25%, NaCl 12%, lait écrémé 6% v/v PH 6,5

### Conjugué

Conjugué anti-IgG humaines 50X, commercialisés par Bio-Rad : kit Platelia H. pylori ref 72778

### Autres Solutions :

Solution de lavage R2, Solutions de révélation au TMB R8 diluant, R9 chromogène, R10 solution d'arrêt : réactifs commercialisés commercialisées par Bio-Rad (ex : kit *Platelia pylori,* ref 72778)

### b) Mode opératoire

Diluer les serums au 1/200 dans le diluant échantillons
Distribuer 100µL/puits
Incubation 1h à 37°C
3 lavages en solution de LAVAGE R2 10x préalablement diluée 10 fois en eau déminéralisée (*i.e.*, solution de lavage 1X)
Distribuer 100µL de conjugué (conjugué 50x à diluer extemporanément dans le diluant conjugué fourni)
Incubation 1 h à 37°C
4 lavages en solution de lavage 1X
distribuer 200µL/puits de solution de révélation (à diluer extemporanément ex:1 mL de R9 dans 10m de R8)
Incubation 30 min à température ambiante à l'obscurité
arrêter la réaction avec 100µL/puits de R10
LECTURE à 450/620nm

Les résultats peuvent être interprétés en prenant un serum SEUIL donnant une réponse au delà de laquelle les serums testés seront considérés comme positifs. Ce sérum est choisi et dilué de façon à donner un signal significativement supérieur au bruit de fond.

### 2) Test ELISA DOUBLE EPITOPE

### a) Réactifs

### Préparation des plaques

Les plaques sont sensibilisées par une solution de protéine N à 1µg/mL dans un tampon PBS 10mM pH 7,2, rouge de phénol à 0,25mL /L. 100 µL de solution sont déposés dans les puits et laissés incubés à température ambiante pendant une nuit. La saturation se fait par un prélavage en tampon PBS 10mM / 0,1% tween suivi d'un lavage avec une solution de saturation PBS 10mM, lait 25% (V/V)

### Diluant serums et conjugué

Tampon TRIS saline 50mM pH8, lait 2%

### Conjugué

Il s'agit de la protéine N recombinante purifiée, couplée à la peroxidase selon le protocole de Nakane (Nakane P.K. and Kawaoi A; (1974) : Peroxydase-labeled antibody, a new method of conjugation. The Journal of Histochemistry and Cytochemistry Vol22, N)23, pp 1084-1091.), dans des ratios molaires respectifs 1/2. Ce conjugué ProtN POD est utilisé à une concentration de 2µg/mL dans du diluant serum/conjugué.

### Autres solutions :

Solution de lavage R2, Solutions de révélation au TMB R8, diluant, R9 chromogène, R10 solution d'arrêt : réactifs commercialisées par Bio-Rad (ex kit platelia pylori ref 72778).

### b) Mode opératoire

### 1ere étape en plaque de "prédilution"

■ Diluer chaque sérum au 1/5 dans la plaque de prédilution (48 µL de diluant +12 µL de sérum).
■ Après avoir dilués l'ensemble des serums, distribuer 60µL de conjugué
■ Le cas échéant, le mélange sérum + conjugué est laissé à incuber.

### 2eme étape en plaque de "réaction"

■ Transférer 100µLde mélange/puits dans la plaque de réaction
■ Incubation 1h 37°C
■ 5 lavages en solution de LAVAGE R2 10x préalablement diluée 10 fois en eau déminéralisée (--> solution de lavage 1x)
■ distribuer 200µL/puits de solution de révélation (à diluer extemporanément ex:1 mL de R9 dans 10m de R8)
■ incubation 30min à température ambiante à l'abri de la lumière
■ arrêter la réaction avec 1 00µL/puits de R10
■ LECTURE à 450/620nm

De même que pour le test ELISA indirect, les résultats peuvent être interprétés en utilisant un serum "valeur seuil". Tout serum ayant une réponse supérieure au sérum valeur seuil sera considéré comme positif.

### 2) Résultats

Les sérums de patients classés comme cas probables de SRAS de l'hôpital français de Hanoi, Vietnam ou en relation avec l'hôpital français de Hanoi (JYK) ont été analysés en utilisant le test IgG-N indirect et le test N double épitope.

Les résultats du test IgG-N indirect (figures 14 et 15) et N double épitope (figures 16 et 17) montrent une excellente corrélation entre eux ainsi qu'avec un test ELISA indirect comparant la réactivité des sérums vis-à-vis d'un lysat de cellules VeroE6 non infectées ou infectées par le SRAS-CoV (ELISA-lysat SRAS-CoV ; voir le Tableau V ci-après). Tous les sérums prélevés 12 jours ou plus après le début des symptômes ont été trouvés positifs, y compris chez des patients pour lesquels l'infection par le virus du SRAS-CoV n'avait pas pu être documentée par analyse de prélèvements respiratoires par RT-PCR, vraisemblablement en raison d'un prélèvement trop tardif au cours de l'infection (≥ J12). Dans le cas du patient TTH pour lequel un prélèvement nasal réalisé à J7 a été trouvé négatif par RT-PCR, la qualité du prélèvement pourrait être en cause.

Certains sérums ont été trouvés négatifs alors que la présence de SRAS-CoV a été détectée par RT-PCR. Il s'agit dans tous les cas de sérums précoces prélevés moins de 10 jours après le début des symptômes (ex : sérum # 032637). Dans le cas d'un patient PTTH (sérum # 032673), seule une suspicion de SRAS était évoquée au moment où les prélèvements ont été réalisés.

En conclusion, les tests sérologiques IgG-N indirect et N-double épitope permettent de documenter l'infection par le SRAS-CoV chez tous les patients pour des sérums prélevés 12 jours ou plus après l'infection.

**Tableau V : Résultats des tests ELISA**

| **Num Pvt** | **Patient** | **Jour** | **PCR-SARS (1)** | **ELISA lysat SRAS- CoV (2)** | **IgG-N (2^{ème} série)** | **2Xepitope (2^{ème} série)** |
|---|---|---|---|---|---|---|
| 033168 | JYK | 38 | POS | +++ | >5000 | NT |
| 033597 | JYK | 74 | POS | NT | ≈ 5000 | NT |
| 032552 | VTT | 8 | NEG- J3&J8&J12 | NEG | <200 | <5 |
| 032544 | CTP | 16 | NEG J16&J20 | ++ | >5000 | >>20 |
| 032546 | CJF | 15 | NEG J15&J19 | ++ | >5000 | >>20 |
| 032548 | PTL | 17 | NEG J17&J21 | ++ | >5000 | >>20 |
| 032550 | NTH | 17 | NEG-J17&J21 | ++ | >5000 | >>20 |
| 032553 | VTT | 8 | NEG- J3&J8&J12 | NEG | <200 | <5 |
| 032554 | NTBV | 4 | POS | NEG | <200 | <5 |
| 032555 | NTBV | 4 | POS | NEG | <200 | |
| 032564 | NTP | 15 | POS | ++ | >5000 | >>20 |
| 032629 | NVH | 4 | POS | NEG | <200 | <5 |
| 032631 | BTTX | 9 | POS | NEG | <200 | <5 |
| 032635 | NHH | 4 | POS | NEG | <200 | <5 |
| 032637 | NHB | 10 | POS | NEG | <200 | <5 |
| 032642 | BTTX | 9 | POS | NEG | <200 | <5 |
| 032643 | LTDH | 1 | POS | NEG | <200 | <5 |
| 032644 | NTBV | 4 | POS | NEG | <200 | <5 |
| 032646 | TTH | 12 | NEG J7&J12&J16 | ++ | >5000 | >>20 |
| 032647 | DTH | 17 | NEG J17&J21 | ++ | >5000 | >>20 |
| 032648 | NNT | 15 | NEG J15&J19 | ++ | >5000 | >>20 |
| 032649 | PTH | 17 | NEG J17&J21 | ++ | >5000 | >>20 |
| 032672 | LW | 16 | NEG J16&J20 | + | >5000 | >>20 |
| 032673 | PTTH | NA | NEG | NEG | <200 | <5 |
| 032674 | PNB | 17 | NEG J17&J21 | ++ | >5000 | >>20 |
| 032682 | VTH | 12 | NEG J12&J16 | ++ | >5000 | >>20 |
| 032683 | DTV | 17 | NEG J17&J21 | + | >1000 | >>20 |

| | | | | | | |
|---|---|---|---|---|---|---|
| Notes: (1) : Les analyses par RT-PCR ont été réalisées par RT-PCR nichée BNI, LC Artus et LC-N sur des écouvillonnages nasaux ou pharyngés; POS signifie qu'au moins un prélèvement a été trouvé positif chez ce patient. (2) : La réactivité des sérums dans le test ELISA utilisant un lysat de cellules infectées par le SRAS-CoV a été classée en très fortement réactif (+++), fortement réactif (++), réactif (+) et négatif en fonction de la valeur DO obtenue aux dilutions testées | | | | | | |

### Exemple 8 : Détection du coronavirus associé au SRAS (SARS-CoV) par RT-PCR

### 1) Mise au point de conditions de RT-PCR en temps réel à l'aide d'amorces spécifiques du gène de la protéine de nucléocapside - test "Light Cycler N"

### a) conception des amorces et des sondes

La conception des amorces et sondes a été réalisée à partir de la séquence du génome de la souche de SARS-CoV issue du prélèvement répertorié sous le numéro 031589, à l'aide du programme "Light Cycler Probe Design (Roche)". Ainsi les deux séries d'amorces et de sondes suivantes ont été sélectionnées :
- **série 1 (SEQ ID NO : 60, 61, 64, 65):**
   - amorce sens : N/+/28507 : 5'-GGC ATC GTA TGG GTT G-3' [28507-28522]
   - amorce antisens : N/-/28774 : 5'-CAG TTT CAC CAC CTC C-3' [28774-28759]
   - sonde 1 : 5'-GGC ACC CGC AAT CCT AAT AAC AAT GC-fluorescéine 3' [28561-28586]
   - sonde 2 : 5' Red705 -GCC ACC GTG CTA CAA CTT CCT-phosphate [28588-28608]
- **série 2 (SEQ ID NO : 62, 63, 66, 67)**
   - amorce sens : N/+/28375 : 5'-GGC TAC TAC CGA AGA G-3' [28375-28390]
   - amorce antisens : N/-/28702 : 5'-AAT TAC CGC GAC TAC G-3' [28702-28687]
   - sonde 1 : SRAS/N/FL : 5'-ATA CAC CCA AAG ACC ACA TTG GC - fluorescéine 3' [28541-28563]
   - sonde 2 : SRAS/N/LC705 : 5' Red705 -CCC GCA ATC CTA ATA ACA ATG CTG C-phosphate 3' [28565-28589]

### b) analyse de l'efficacité des deux couples amorces

Afin de tester l'efficacité respective des deux couples d'amorces, une amplification par RT-PCR a été réalisée sur un ARN synthétique correspondant aux nucléotides 28054-29430 du génome de la souche de SARS-CoV issue du prélèvement répertorié sous le numéro 031589et contenant la séquence du gène N.

De manière plus précise :
Cet ARN synthétique a été préparé par *transcription in vitro* à l'aide de l'ARN polymérase du phage T7, d'une matrice d'ADN obtenu par linéarisation du plasmide SRAS-N avec l'enzyme *Bam H1.* Après élimination de la matrice d'ADN par digestion à l'aide de DNAse 1, les ARN synthétiques sont purifiés par une extraction au phénol-chloroforme suivie de deux précipitations successives en acétate d'ammonium et isopropanol. Ils sont alors quantifiés par mesure de l'absorbance à 260 nm et leur qualité est contrôlée par le rapport des absorbances à 260 et 280 nm ainsi que par une électrophorèse en gel d'agarose. Ainsi, la concentration de la préparation d'ARN synthétique utilisée pour ces études est de 1,6 mg/ml, ce qui correspond à 2,1.10¹⁵ copies/ml d'ARN.

Des quantités décroissantes d'ARN synthétique ont été amplifiés par RT-PCR à l'aide du kit "Superscript™ One-Step RT-PCR with Platinum® Taq" et les couples d'amorces n° 1 (N/+/28507, N/-/28774) (figure 1A) et n° 2 (N/+/28375, N//28702) (figure 1B), en suivant les indications du fournisseur. Les conditions d'amplification utilisées sont les suivantes : l'ADNc a été synthétisé par incubation 30 min à 45 °C, 15 min à 55°C puis 2 min à 94 °C puis il a été amplifié par 5 cycles comprenant : une étape de dénaturation à 94°C pendant 15 sec, une étape d'hybridation à 45°C pendant 30 sec puis une étape d'élongation à 72°C pendant 30 sec, suivis de 35 cycles comprenant : une étape de dénaturation à 94°C pendant 15 sec, une étape d'hybridation à 55°C pendant 30 sec puis une étape d'élongation à 72°C pendant 30 sec, avec 2 sec d'élongation supplémentaire à chaque cycle, et d'une étape finale d'élongation à 72°C pendant 5 min. Les produits d'amplification obtenus ont ensuite été maintenus à 10°C.

Les résultats présentés à la figure 11 montrent que le couple d'amorces n° 2 (N/+/28375, N/-/28702) permet de détecter jusqu'à 10 copies d'ARN (bande de faible intensité) ou 10² copies (bande de bonne intensité) contre 10⁴ copies pour le couple d'amorces n° 1 (N/+/28507, N/-/28774). Les amplicons sont respectivement de 268 pb (couple 1) et de 328 pb (couple 2).

### c) mise au point de la RT-PCR en temps réel

Une RT-PCR en temps réel a été mise au point à l'aide du couple d'amorces n°2 et du couple de sonde constitué par SRAS/N/FL et SRAS/N/LC705 (figure 2).

L'amplification a été réalisée sur un LightCycler™ (Roche) à l'aide du kit "Light Cycler RNA Amplification Kit Hybridization Probes " (référence 2 015 145, Roche) dans les conditions optimisées suivantes. Un Mélange réactionnel contenant : H₂O (6,8 µl), MgCl₂ 25 mM (0,8 µl, 4 µM final de Mg2+), mélange réactionnel 5X (4 µl), sonde SRAS/N/FL 3µM (0,5 µl, 0,075 µM final), sonde SRAS/N/LC705 3 µM (0,5 µl, 0,075 µM final), amorce N/+/28375 10 µM (1 µl, 0,5 µM final), amorce N/-/28702 10 µM (1 al, 0,5 µM final), mélange d'enzyme (0,4 µl) et échantillon (ARN viral, 5 µl) a été amplifié en suivant le programme suivant :

| | | | |
|---|---|---|---|
| - Transcription inverse : | **50°C** | 10:00min | analysis mode: none |
| - Dénaturation : | 95°C | 30sec x1 | analysis mode: none |
| - Amplification : | 95°C | 2sec | □ |
| | **50°C** | 15sec | analysis mode: quantification*□ x45 |
| | 72°C | 13sec | rampe thermique 2,0°C/sec □ |
| - refroidissement : | 40°C | 30sec x1 | analysis mode: none |

| | | | |
|---|---|---|---|
| *La mesure de fluorescence se fait à la fin de l'hybridation et à chaque cycle (en mode SINGLE). | | | |

Les résultats présentés à la figure 12 montrent que cette RT-PCR en temps réel est très sensible puisqu'elle permet de détecter 10² copies d'ARN synthétique dans 100% des 5 échantillons analysés (29/29 échantillons dans 8 expériences) et jusqu'à 10 copies d'ARN dans 100% des 5 échantillons analysés (40/45 échantillons dans 8 expériences). Elle montre également que cette RT-PCR permet de détecter la présence du génome du SARS-CoV dans un échantillon et de quantifier le nombre de génomes présents. A titre d'exemple, l'ARN viral d'un stock de SARS-CoV cultivé sur cellules Vero E6 a été extrait à l'aide du kit "Qiamp viral RNA extraction" (Qiagen), dilué à 0,05.10⁻⁴ et analysé par RT-PCR en temps réel selon le protocole décrit ci-dessus; l'analyse présentée à la figure 12 montre que ce stock de virus contient 6,5.10⁹ génomes -équivalents/ml (geq/ml), ce qui est tout à fait similaire à la valeur de 1,0.10¹⁰ geq/ml mesurée à l'aide du kit "RealArt™ HPA-Coronavirus LC RT PCR Reagents" commercialisé par Artus.

### 2) Mise au point de conditions de RT-PCR nichée ciblant le gène de l'ARN polymérase - test "RT-PCR nichée CDC (Centers for Disease Control and Prevention) /IP"

### a) Extraction de l'ARN viral

Echantillon clinique : QIAamp viral RNA Mini Kit (QIAGEN) selon les indications du fabricant, ou une technique équivalente. L'ARN est élué dans un volume de 60 µl.

### b) RT-PCR nichée "SNE/SAR"

### Première étape : RT-PCR couplée « SNE »

Le kit Invitrogen *"Superscript*™ *One-Step RT-PCR with Platinum*® *Tag*"a été utilisé, mais le kit *"Titan"* de Roche Boehringer peut lui être substitué avec des résultats similaires.
Oligonucléotides :
- SNE-S1 5' GGT TGG GAT TAT CCA AAA TGT GA 3'
- SNE-AS1 5'GCA TCA TCA GAA AGA ATC ATC ATG 3'
-> Taille attendue :**440 pb**
1. Préparer un mix :

| | |
|---|---|
| H2O | 6,5 µl |
| Reaction mix 2X | 12,5 µl |
| Oligo SNE-S1 50 µM | 0,2 µl |
| Oligo SNE-AS1 50 µM | 0,2 µl |
| RNAsin 40 U/µl | 0,12 µl |
| RT/Platinum Taq mix | 0, 5 µl |

2. A 20 µl du mix, ajouter 5 µl d'ARN et procéder à l'amplification sur thermocycleur (conditions ABI 9600) :

| | | | |
|---|---|---|---|
| 2.1 | 45°C | 30 min. | |
| | 55°C | 15 min. | |
| | 94°C | 2 min. | |
| 2.2. | 94°C | 15 sec. | } |
| | 45°C | 30 sec. | } x 5 cycles |
| | 72°C | 30 sec. | } |
| 2.3. | 94°C | 15 sec. | } |
| | 55°C | 30 sec. | } x 35 cycles |
| | 72°C | 30 sec. + 2sec./cycle | } |
| 2.4. | 72°C | 5 min. | |
| 2.5 | 10°C | ∞ | |

Conservation à +4°C.

La RNAsin (N2511/N2515) de Promega a été utilisée comme inhibiteurs de RNase.

Des ARN synthétiques ont servi de témoin positif. A titre de contrôle, 10³, 10² et 10 copies d'ARN synthétique R_{SNE} ont été amplifiées dans chaque expérience.

### Seconde étape : PCR nichée "SAR"

Oligonucléotides :
- SAR1-S 5' CCT CTC TTG TTC TTG CTC GCA 3'
- SAR1-AS 5' TAT AGT GAG CCG CCA CAC ATG 3'
-> Taille attendue : **121 pb**
1. Préparer un mix :

| | |
|---|---|
| H2O | 35,8 µl |
| Tampon Taq 10X | 5 µl |
| MgCl₂ 25 mM | 4 µl |
| Mix dNTPs 5 mM | 2 µl |
| Oligo SAR1-S 50 µM | 0,5 µl |
| Oligo SAR1-AS 50 µM | 0,5 µl |
| Taq ADN pol 5 U/µl | 0,25 µl |

*L'AmpliTaq DNA Pol* d'Applied Biosystems a été utilisée (tampon 10X sans MgCl₂, réf 2721660 1).
2. A 48 µl du mix, ajouter 2 µl du produit de la première PCR et procéder à l'amplification (conditions ABI 9600) :

| | | | |
|---|---|---|---|
| 2.1. | 94°C | 2 min. | |
| 2.2. | 94°C | 30 sec. | } |
| | 45°C | 45 sec. | } x 5 cycles |
| | 72°C | 30 sec. | } |
| 2.3. | 94°C | 30 sec. | } |
| | 55°C | 30 sec. | } x 35 cycles |
| | 72°C | 30 sec. + 1sec./cycle | } |
| 2.4. | 72°C | 5 min. | |
| 2.5 | 10°C | ∞ | |

3. Analyser 10 µl du produit réactionnel sur gel *"low-melting"* (type Seakem GTG) à 3% d'agarose.
La sensibilité du test niché est en routine, dans les conditions décrites, de 10 copies d'ARN.
4. Les fragments peuvent ensuite être purifiés sur QIAquick PCR kit (QIAGEN) et séquencés avec les oligos SAR1-S et SAR1-AS.

### 3) Détection de l'ARN du SARS-CoV par PCR à partir de prélèvements respiratoires

### a) Première étude comparative

Une étude comparative a été réalisée sur une série de prélèvements respiratoires reçus par le Centre National de Référence du Virus Influenzae (région nord) et susceptibles de contenir du SARS-CoV. Pour ce faire, l'ARN a été extrait des prélèvements à l'aide du kit "Qiamp viral RNA extraction" (Qiagen) et analysé par RT-PCR en temps réel, d'une part à l'aide des couples d'amorces et de sondes de la série n° 2 dans les conditions décrites ci-dessus d'une part, et d'autre part à l'aide du kit "LightCycler SARS-CoV quantification kit" commercialisé par Roche (référence 03 604 438). Les résultats sont résumés dans le Tableau VI ci-dessous. Ils montrent que 18 des 26 prélèvements sont négatifs et 5 des 26 prélèvements sont positifs pour les deux kits, tandis qu'un prélèvement est positif pour le seul kit Roche et deux pour les seuls réactifs N "série2". En outre, pour 3 prélèvements (20032701, 20032712, 20032714) les quantités d'ARN détectés sont nettement supérieures avec les réactifs (sondes et amorces) de la série n°2. Ces résultats indiquent que les amorces et sondes N "série2" sont plus sensibles pour la détection du génome du SARS-CoV dans des prélèvements biologiques que celles du kit actuellement disponible.

**Tableau VI: Analyse par RT-PCR en temps réel des ARN extraits d'une série de prélèvements de 5 patients à l'aide des couples d'amorces et de sondes de la série n° 2 (N "série 2") ou du kit "LightCycler SARS-CoV quantification kit" (Roche). Le type de prélèvement est indiqué ainsi que le nombre de copies de génome viral mesurées dans chacun des deux tests. NEG : RT-PCR négative.**

| **Prélèvements n°** | **Patient** | **Type de prélèvement** | **KIT ROCHE** | **N "série2"** |
|---|---|---|---|---|
| 20033082 | K | nasal | NEG | NEG |
| 20033083 | K | pharyngé | NEG | NEG |
| 20033086 | K | nasal | NEG | NEG |
| 20033087 | K | pharyngé | NEG | NEG |
| 20032802 | M | nasal | NEG | NEG |
| 20032803 | M | expectoration | NEG | NEG |
| 20032806 | M | nasal ou pharyngé | NEG | NEG |
| 20031746ARN2 | C | pharyngé | NEG | NEG |
| 20032711 | C | nasal ou pharyngé | **39** | NEG |
| 20032910 | B | nasal | NEG | NEG |
| 20032911 | B | pharyngé | NEG | NEG |
| 20033356 | V | expectoration | NEG | NEG |
| 20033357 | V | expectoration | NEG | NEG |
| 20031725 | K | asp. endotrachéale | NEG | 150 |
| 20032657 | K | asp. endotrachéale | NEG | NEG |
| 20032698 | K | asp. endotrachéale | NEG | NEG |
| 20032720 | K | asp. endotrachéale | **3** | **5** |
| 20033074 | K | selles | **115** | **257** |
| 20032701 | M | pharyngé | **443** | **1676** |
| 20032702 | M | expectoration | NEG | **249** |
| 20031747ARN2 | C | pharyngé | NEG | NEG |
| 20032712 | C | inconnu | **634** | **6914** |
| 20032714 | C | pharyngé | **17** | **223** |
| 20032800 | B | nasal | NEG | NEG |
| 20033353 | V | nasal | NEG | NEG |
| 20033384 | V | nasal | NEG | NEG |

### b) Deuxième étude comparative

Les performances de différentes méthodes de RT-PCR nichée et de RT-PCR en temps réel ont ensuite été comparées pour 121 prélèvements respiratoires de cas possibles de SRAS de l'hôpital français de Hanoi, Vietnam, réalisés entre le 4^{ème} et le 17^{ème} jour après le début des symptômes. Parmi ces prélèvements, 14 avaient été trouvés positifs lors d'un premier test utilisant la méthode de RT-PCR nichée ciblant l'ORF1b (codant pour la réplicase) telle que décrite initialement par le Bernhard Nocht Institute (RT-PCR nichée BNI). Des informations concernant ce test sont disponibles sur internet, à l'adresse http://www15.bnihamburg.de/bni/bni2/neu2/getfile.acgi?area_engl=diagnostics&pid=4112.

Les différents tests comparés dans cette étude sont :
- la méthode de RT-PCR quantitative selon l'invention, avec les amorces et sonde N "série 2" décrite ci-dessus (colonne Light Cycler N),
- le test de RT-PCR nichée ciblant le gène de l'ARN polymérase décrit ci-dessus, développé par le CDC, le BNI et l'Institut Pasteur (RT-PCR nichée CDC/IP),
- le kit ARTUS de référence "HPA Corona LC RT-PCR Kit # 5601-02", qui est un test de RT-PCR en temps réel ciblant le gène ORF1b,
- le test de RT-PCR nichée du BNI, ciblant également le gène de l'ARN polymérase, mentionné ci-dessus.

Les inventeurs ont constaté :
1) une variabilité inter-test pour une même technique, liée à la dégradation de la préparation d'ARN lors de décongélations répétées, notamment pour les échantillons contenant les quantités d'ARN les plus faibles,
2) une sensibilité réduite de la RT-PCR nichée CDC/IP par rapport à la RT-PCR nichée BNI, et
3) une sensibilité comparable du test de RT-PCR quantitative selon l'invention (Light Cycler N) par rapport au test Light Cycler (LC) Artus.

Ces résultats, présentés dans le Tableau VII ci-dessous, montrent que le test par RT-PCR quantitative selon l'invention constitue un excellent complément - ou une alternative - aux tests actuellement disponibles. En effet, le coronavirus lié au SRAS est un virus émergent, susceptible d'évoluer rapidement. En particulier, le gène de la RNA polymérase du coronavirus lié au SRAS, qui est ciblé dans la plupart des tests actuellement disponibles, peut recombiner avec celui d'autres coronavirus non liés au SRAS. L'utilisation d'un test ciblant exclusivement ce gène pourrait alors conduire à l'obtention de faux négatifs.

Le test par RT-PCR quantitative selon l'invention ne cible pas la même région génomique que le kit ARTUS, puisqu'il cible le gène codant pour la protéine N. En réalisant un test de diagnostique ciblant deux gènes différents du coronavirus lié au SRAS, on peut donc espérer s'affranchir de résultats de type faux négatifs, qui pourraient être dus à l'évolution génétique du virus.

De plus, il apparaît particulièrement avantageux de cibler le gène de la protéine de nucléocapside, car il est très stable, du fait de la forte pression de sélection liée aux contraintes structurales élevées concernant cette protéine.

**Tableau VII: Comparaison de différentes méthodes d'analyse par amplification génique, à partir de 121 Prélèvements de cas probables de SRAS de l'hôpital français de Hanoi, Vietnam (épidémie 2003)**

| **N° CNR** | **Type prélèvement (1)** | **Jour prélèvement** | **Patient** | **RT- PCR nichée CDC/IP** | **RT- PCR nichée BNI** | **kit Light Cycler Artus** | **Light Cycler N (IP)** |
|---|---|---|---|---|---|---|---|
| 107 prélèvements | NetP | | | Négatif | Négatif | Négatif | Négatif |
| 032529 | P | 10 | NHB | Négatif | Positif | Négatif | Négatif |
| 032530 | N | 10 | NHB | Positif | Positif | 3,10E+01 | 4,20E+01 |
| 032531 | P | 7 | LP | Positif | Positif | 7,70E+00 | 3,10E+00 |
| 032534 | N | 15 | BND | Positif | Positif | 1,60E+00 | Négatif |
| 032600 | P | 4 | NHH | Négatif | Positif | Négatif | 1,30E+02 |
| 032612 | P | 17 | NTS | Négatif | Positif | Négatif | Négatif |
| 032688 | P | 9 | BTX | Positif | Positif | Négatif | Négatif |
| 032689 | N | 4 | NVH | Positif | Positif | 1,20E+01 | 2,30E+02 |
| 032690 | P | 4 | NVH | Négatif | Positif | 1,60E+00 | Négatif |
| 032727 | P | 8 | NVH | Positif | Positif | 2,30E+02 | 4,00E+02 |
| 032728 | N | 8 | NVH | Positif | Positif | 1,10E+03 | 1,60E+04 |
| 032729 | P | 14 | NHB | Positif | Positif | 5,90E+00 | 3,40E+01 |
| 032730 | N | 14 | NHB | Positif | Positif | 1,30E+02 | 4,80E+02 |
| 032741 | P | 8 | NHH | Positif | Positif | 2,10E+02 | 1,30E+02 |
| | | | | | | | |
| positifs | | | | 10 | 14 | 10 | 9 |
| fraction détectée des 14 positifs | | | | 71,4% | 100,0% | 71.4% | 64,3% |

| | | | | | | | |
|---|---|---|---|---|---|---|---|
| (1) P= écouvillonage pharyngé N= écouvillonage nasal | | | | | | | |

### Exemple 9 : Obtention et caractérisation d'anticorps monoclonaux dirigés contre la protéine N

Des souris Balb C ont été immunisées à l'aide de la protéine N recombinante purifiée et leurs cellules spléniques fusionnées avec un myélome murin approprié selon les techniques de Köhler et Milstein.

Dix-neuf hybridomes sécréteurs d'anticorps anti N ont été présélectionnés et leurs immuno réactivités précisées. Ces anticorps reconnaissent bien la protéine N recombinante (en ELISA) avec des intensités variables, ainsi que la protéine virale, naturelle N en ELISA et/ou en Western Blot. Les figures 18 à 20 montrent les résultats de ces tests pour 15 de ces 19 anticorps monoclonaux.

Les clones 12, 17, 28, 57, 72, 76, 86, 87, 98, 103, 146, 156, 166, 170, 199, 212, 218, 219 et 222, fortement réactifs, ont été sous clonés. Les études de spécificité ont été poursuivies avec les outils appropriés afin de préciser les épitopes reconnus et vérifier l'absence de réactivité vis-à-vis des autres Coronavirus humains et de certains virus respiratoires.

Les études de cartographie (*mapping*) épitopique (réalisées sur membrane spot, à l'aide de peptides chevauchants de 15 aa) et les études supplémentaires réalisées sur la protéine N naturelle en Western Blot ont révélé l'existence de 4 groupes d'anticorps monoclonaux :
1° Anticorps monoclonaux spécifiques d'un épitope linéaire majeur en position N-ter (75-81, séquence : INTNSVP)
   Le représentant de ce groupe est l'anticorps 156. L'hybridome produisant cet anticorps a été déposé à la Collection Nationale de Cultures de Microorganismes (CNCM) de l'Institut Patseur (Paris, France) le 1^{er} décembre 2004, sous le numéro I-3331. Ce même épitope est également reconnu par un sérum de lapin (polyclonal anti N) obtenu par immunisation classique à l'aide de cette même protéine N.
2° Anticorps monoclonaux spécifiques d'un épitope linéaire majeur situé en position centrale (position 217-224, séquence : ETALALL); les représentants de ce groupe sont les anticorps monoclonaux 87 et 166. L'hybridome produisant l'anticorps 87 a été déposé à la CNCM le 1^{er} décembre 2004, sous le numéro I-3328.
3° Anticorps monoclonaux spécifiques d'un épitope linéaire majeur situé en position C-terminale (position 403-408, séquence : DFFRQL), les représentants de ce groupe sont les anticorps 28, 57 et 143. L'hybridome produisant l'anticorps 57 a été déposé à la CNCM le 1^{er} décembre 2004, sous le numéro I-3330.
4° Anticorps monoclonaux spécifiques d'un épitope discontinu, conformationel. Ce groupe d'anticorps ne reconnaît aucun des peptides recouvrant la séquence de la protéine N, mais réagissent fortement sur la protéine naturelle non dénaturée. Le représentant de ce dernier groupe est l'anticorps 86. L'hybridome produisant cet anticorps a été déposé à la CNCM le 1^{er} décembre 2004, sous le numéro I-3329.

Le Tableau VIII ci-après résume les résultats de cartographie épitopique obtenus :

**Tableau VIII : Mapping épitopique des anticorps monoclonaux**

| **Anticorps** | **Epitope** | **Position** | **Région** |
|---|---|---|---|
| **28** | **DFSRQL** *Q* | **403 ... 408** | **C - Ter.** |
| **143** | **DFSRQL** *Q* | | |
| **76** | **DFSRQL** *Q* | | |
| **57** | **DFSRQL** *Q* | | |
| | ***FFGMS** RI* | **315 ... 319** | |
| **146** | **LPQRQ** | **383 ...387** | |
| **166** | **ETALALL***LL* | **217 ... 224** | **centrale** |
| **87** | **ETALALL** | **217... 224** | |
| **156** | ***INTNSGP*** | ***75... 81*** | ***N - Ter.*** |
| ***86*** | ***Conformationnel*** | | |
| **212** | ***Conformationnel*** | | |
| **170** | **Conformationnel** | | |

En outre, comme illustré notamment aux figures 18 et 19, ces anticorps ne présentent pas de réactivité en ELISA et/ou en WB, vis à vis de la protéine N du coronavirus humain 229 E.

### Exemple 10 : Combinaisons des anticorps monoclonaux pour le développement d'un test d'immunocapture sensible et spécifique de l'antigène viral N dans le sérum ou les fluides biologiques des patients contaminés par le virus SARS Co V

Les anticorps listés ci-dessous ont été retenus en raison de leurs propriétés bien particulières pour une étude supplémentaire de capture et détection de la protéine virale N, dans le sérum des sujets ou patients.

Ces anticorps ont été produits en ascite sur souris, purifiés par chromatographie d'affinité et utilisés seuls ou en combinaison comme anticorps de capture, et comme anticorps signal.

Liste des anticorps sélectionnés pour le propos :
- Acm anti région C-ter (n° 28, 57, 143)
- Acm anti région centrale (n° 87, 166)
- Acm anti région N-ter (n° 156)
- Acm anti épitope discontinu, conformationnel (86)

### 1) Préparation des réactifs :

### a) Plaques ELISA immunocapture

Les plaques sont sensibilisées avec les solutions d'anticorps à 5 µg/ml en tampon carbonate 0,1 M, pH.9,6. Les solutions (monovalentes ou plurivalentes) sont déposées sous volume de 100 µl dans les puits et incubées pendant une nuit à température ambiante. Ces plaques sont ensuite lavées en tampon PBS (10 mM pH 7,4 additionné de 0,1% de Tween 20) puis saturées avec une solution de PBS additionnée de 0,3% de BSA et de 5% de Saccharose). Les plaques sont ensuite séchées puis conditionnées dans un sachet en présence d'un dessicant. Elles sont prêtes à l'emploi.

### b) Conjugués

Les anticorps purifiés ont été couplés à la péroxydase selon le protocole de Nakane (Nakane et al. - 1974, J.of Histo and cytochemistry, vol.22, p1084-1091) dans un rapport de une molécule d'IgG pour 3 molécules de péroxydase. Ces conjugués ont été purifiés par chromatographie d'exclusion et conservés concentrés (concentration comprise entre 1 à 2 mg/ml) en présence de 50% de glycerol et à -20°C. Ils sont dilués pour leur mise en oeuvre dans les essais à la concentration finale de 1 ou 2 µg/ml en tampon PBS (pH 7,4) additionné de 1% de BSA.

### c) Autres réactifs

- Sérums humains négatifs pour tous les marqueurs sériques des virus HIV, HBV, HCV et THLV
- Pool de sérums humains négatifs additionné de 0,5% de Triton X 100
- Ag viral inactivé : surnageant de culture virale inactivé par irradiation et inactivation vérifiée après mise en culture sur cellules sensibles - titre de la suspension avant inactivation environ 10⁷ particules infectieuses par ml ou encore environ 5x10⁹ particules virales physiques par ml d'antigène
- Les échantillons d'Ag dilués en sérum humain négatif : ces échantillons ont été préparés par dilution au 1:100^{e} puis par dilution en série de raison 5.
   Ces échantillons non infectieux miment des échantillons humains supposés contenir des concentrations faibles à très faibles de nucléoprotéine virale N. De tels échantillons ne sont pas accessibles pour les travaux de routine
- Solution de lavage R2, solution de révélation TMB R8, chromogène R9 et solution d'arrêt R10, sont les réactifs génériques commercialisés par Bio-Rad dans ses trousses ELISA (ex : trousse *Platelia Pylori* ref.72778).

### 2) Mode opératoire

Les échantillons de sérums humains surchargés en Ag viral inactivé sont distribués à raison de 100 µl par cupule, directement dans les plaques sensibilisées, prêtes à l'emploi puis incubés pendant 1 heure à 37°C (incubation Bio-Rad IPS).

Le matériel non retenu par la phase solide est éliminé par 3 lavages (lavage avec solution R2 diluée, laveur automatique LP 35).

Les conjugués appropriés, dilués à la concentration finale de 1 ou 2 µg/ml sont distribués à raison de 100 µl par cupule et les plaques incubées à nouveau pendant une heure à 37° C (incubation IPS).

L'excès de conjugué est éliminé par 4 lavages successifs (solution R2 diluée - laveur LP 35).

La présence de conjugué fixé sur les plaques est révélée après addition de 100 µl de solution de révélation préparée avant emploi (1 ml de R9 et 10 ml de R8) et après incubation pendant 30 minutes, à température ambiante et à l'abri de la lumière.

La réaction enzymatique est finalement bloquée par addition de 100 µl de réactif R10 (H2SO4 1N) dans toutes les cupules.

La lecture est effectuée à l'aide d'un lecteur de microplaque approprié à double longueur d'onde (450/620 nm).

Les résultats peuvent être interprétés en utilisant comme valeur seuil provisoire la moyenne d'au moins deux contrôles négatifs multiplié par un facteur 2 ou encore la moyenne de 100 sérums négatifs additionnée d'un incrément correspondant à 6 SD (*Standart Deviation* calculée sur les 100 mesures individuelles).

### 3) Résultats

Différentes combinaisons anticorps de capture et anticorps signal ont été testées en se fondant sur les propriétés des anticorps sélectionnés, et en évitant les combinaisons d'anticorps spécifiques des mêmes épitopes en phase solide et en conjugués.

Les meilleurs résultats ont été obtenus avec les 4 combinaisons listées ci-dessous. Ces résultats sont reproduits dans le tableau IX ci-après.
1. Combinaison F/28
   Phase solide (Acm 166 + 87 région centrale) : conjugué anticorps 28 (C-ter)
2. Combinaison G/28
   Phase solide (Acm 86 - épitope conformationel) : conjugué anticorps 28 (C-ter)
3. Combinaison H/28
   Phase solide (Acms 86, 166 et 87 région centrale et épitope conformationel) : conjugué anticorps 28 (C-ter)
4. Combinaison H/28 + 87
   Phase solide (Acms 86, 166 et 87 région centrale et épitope conformationel) : conjugué mixte anticorps 28 (C-ter) et 87 (central)
5. Combinaison G/87
   Phase solide (Acm 86 - épitope conformationel) : conjugué anticorps 87 (région centrale)

Les 4 premières combinaisons présentent des performances équivalentes et reproduites, supérieures aux autres combinaisons mises en oeuvre (comme par exemple la combinaison G/87). Bien entendu, dans ces combinaisons, un anticorps monoclonal peut être remplacé par un autre anticorps reconnaissant le même épitope. Ainsi, on peut citer les variantes suivantes :
6. Variante de la combinaison F/28
   Phase solide (Acm 87 uniquement) : conjugué anticorps 57 (C-ter)
7. Variante de la combinaison G/28
   Phase solide (Acm 86 - épitope conformationel) : conjugué anticorps 57 (C-ter)
8. Variante de la combinaison H/28
   Phase solide (Acms 86 et 87 région centrale et épitope conformationel) : conjugué anticorps 57 (C-ter)
9. Variante de la combinaison H/28 + 87
   Phase solide (Acms 86 et 87 région centrale et épitope conformationel) : conjugué mixte anticorps 57 (C-ter) et 87 (central)

**Tableau IX : Contrôle d'immunoréactivité des Acm anti nucléoprotéines SARS CoV : densités optiques mesurées avec chaque combinaison d'anticorps, en fonction des dilutions de l'antigène viral inactivé.**

| **N°** | **Dilution** | **F/28** | **G/28** | **G/87** | **H/28** | **H/28+87** |
|---|---|---|---|---|---|---|
| 0 | 1/100 | 5 | 5 | 3,495 | 3,900 | 5 |
| 1 | 1/500 | 3,795 | 3,814 | 1,379 | 3,702 | 3,804 |
| 2 | ½ 500 | 2,815 | 2,950 | 0,275 | 3,268 | 2,680 |
| 3 | 1/12500 | 0,987 | 1,038 | 0,135 | 1,374 | 0,865 |
| 4 | 1/62 500 | 0,404 | 0,348 | 0,125 | 0,480 | 0,328 |
| 5 | 1/312 500 | 0,285 | 0,211 | 0,123 | 0,240 | 0,215 |
| 6 | Témoin | 0,210 | 0,200 | 0,098 | 0,186 | 0,156 |
| 7 | Témoin | 0,269 | 0,153 | 0,104 | 0,193 | 0,202 |

La limite de détection de ces 4 essais expérimentaux correspond à la dilution d'antigène en sérum négatif 1:62500. Une extrapolation rapide laisse supposer la détection de moins de 10³ particules infectieuses par ml de sérums.

De cette étude, il ressort que les anticorps les mieux appropriés pour la capture de la nucleoprotéine virale native sont les anticorps spécifiques de la région centrale et/ou d'un épitope conformationnel, l'un et l'autre étant des anticorps sélectionnés également pour leur forte affinité pour l'antigène natif.

Ayant déterminé les meilleurs anticorps pour la composition de la phase solide, les anticorps à retenir en priorité pour la détection des antigènes fixés sur la phase solide, sont les anticorps complémentaires spécifiques d'un épitope dominant en région C-ter. L'emploi de tout autre anticorps complémentaire, mais spécifique des épitopes localisés en région N-ter de la protéine conduit à des résultats moyens ou médiocres.

### Exemple 11 : Systèmes d'expression eucaryotes de la proteine de spicule (S) du coronavirus associé au SRAS (SRAS-CoV)

### 1) Optimisation des conditions d'expression de la S du SRAS-CoV en cellules de mammifères.

Les conditions d'expression transitoire de la protéine de spicule (S) du SRAS-CoV ont été optimisées en cellules de mammifères (293T, VeroE6).

Pour cela, un fragment d'ADN contenant le cDNA de la S du SRAS-CoV a été amplifié par PCR à l'aide des oligonucléotides 5'-ATAGGATCCA CCATGTTTAT TTTCTTATTA TTTCTTACTC TCACT-3' et 5'-ATACTCGAGTT ATGTGTAATG TAATTTGACA CCCTTG-3' à partir du plasmide pSARS-S (C.N.C.M . n° I-3059) puis inséré entre les sites BamH1 et Xho1 du plasmide pTRIPΔU3-CMV contenant un vecteur lentiviral TRIP (Sirven, 2001, Mol. Ther., 3, 438-448) pour obtenir le plasmide pTRIP-S. Le fragment BamH1 et Xho1 contenant le cDNA de la S a ensuite été sous-cloné entre les BamH1 et Xho1 du plasmide d'expression eucaryote pcDNA3.1(+) (Clontech) pour obtenir le plasmide pcDNA-S. Le fragment Nhe1 et Xho1 contenant le cADN de la S a ensuite été sous-cloné entre les sites correspondants du plasmide d'expression pCI (Promega) pour obtenir le plasmide pCI-S. Les séquences WPRE du virus de l'hépatite de la marmotte (*"Woodchuck Hepatitis Virus posttranscriptional regulatory element"*) et les séquences CTE (*"constitutive transport element"*) du rétrovirus simien de Mason-Pfizer ont été insérées dans chacun des deux plasmides pcDNA-S et pCI-S entre les sites Xho1 et Xba1 pour obtenir respectivement les plasmides pcDNA-S-CTE, pcDNA-S-WPRE, pCI-S-CTE et pCI-S-WPRE (figure 21). Le plasmide pCI-S-WPRE a été déposé à la CNCM, le 22 novembre 2004, sous le numéro I-3323. Tous les inserts ont été séquencés à l'aide d'un kit BigDye Terminator v1.1 (Applied Biosystems) et d'un séquenceur automatique ABI377.

La capacité des plasmides construits à diriger l'expression de la S du SRAS-CoV dans des cellules de mammifères a été recherchée après transfection de cellules VeroE6 (figure 22). Dans cette expérience, des monocouches de 5x10⁵ cellules VeroE6 en boites de Pétri de 35mm ont été transfectées avec 2 µg des plasmides pcDNA (à titre de contrôle), pcDNA-S, pCI et pCI-S et 6 µl de réactif Fugene6 selon les indications du fabricant (Roche). Après 48 heures d'incubation à 37°C et sous 5% de CO₂, des extraits cellulaires ont été préparé en tampon de dépôt selon Laemmli, séparés sur un gel SDS à 8% de polyacrylamide, puis transférés sur une membrane de PVDF (BioRad). La détection de cette immunoempreinte (« western blot ») a été réalisée à l'aide d'un sérum polyclonal de lapin anti-S (immun-sérum du lapin P11135 : cf exemple 4 ci-dessus) et d'anticorps polyclonaux d'âne dirigés contre les IgG de lapin et couplés à la peroxidase (NA934V, Amersham). Les anticorps fixés ont été révélés par luminescence à l'aide du kit ECL+ (Amersham) et de films d'autoradiographie Hyperfilm MP (Amersham).

Cette expérience (figure 22) montre que le plasmide pcDNA-S ne permet pas de diriger l'expression de la S du SRAS-CoV à des niveaux détectables tandis que le plasmide pCI-S permet une expression faible, proche de la limite de détection, qui peut être mise en évidence lorsque le film est surexposé. Des résultats similaires ont été obtenus lorsque l'expression de la S a été recherchée par immunofluorescence (données non montrées). Cette impossibilité de détecter une expression efficace de la S ne peut pas être imputée aux techniques de détection utilisées puisque la protéine S peut être mise en évidence à la taille attendue (180 kDa) dans un extrait de cellules infectées par le SRAS-CoV ou dans un extrait de cellules VeroE6 infectées par le virus recombinant de la vaccine VV-TF7.3 et transfectées par le plasmide pcDNA-S. Dans cette dernière expérience, le virus VV-TF7.3 exprime l'ARN polymérase du phage T7 et permet la transcription cytoplasmique d'un ARN non coiffé susceptible d'être traduit efficacement. Cette expérience suggère que les défauts d'expression décrits ci-dessus sont dus à une incapacité intrinsèque du cADN de la S à être exprimé efficacement lorsque l'étape de transcription en ARN messager est réalisée au niveau nucléaire.

Dans une seconde expérience, l'effet des signaux CTE et WPRE sur l'expression de la S a été recherchée après transfection de cellules VeroE6 (figure 23A) et 293T (figure 23B) et selon un protocole similaire à celui décrit ci-dessus. Alors que l'expression de la S ne peut pas être mis en évidence après transfection des plasmides pcDNA-S-CTE et pcDNA-S-WPRE dérivés de pcDNA-S, l'insertion des signaux WPRE et CTE améliore fortement l'expression de la S dans le contexte du plasmide d'expression pCI-S.

Pour préciser ce résultat, une deuxième série d'expériences a été réalisée, où l'immunoempreinte est révélée de façon quantitative par luminescence et acquisition sur un dispositif d'imagerie numérique (FluorS, BioRad). L'analyse des résultats obtenus avec le logiciel QuantityOne v4.2.3 (BioRad) montre que les séquences WPRE et CTE augmentent respectivement l'expression de la S d'un facteur 20 à 42 et 10 à 26 en cellules Vero E6 (Tableau X). En cellules 293T (Tableau X), l'effet de la séquence CTE est plus modéré (4 à 5 fois) tandis que celui de la séquence WPRE reste important (13 à 28 fois).

**Tableau X analyse quantitative de l'effet des signaux CTE et WPRE sur l'expression de la S du SRAS-CoV : Des extraits cellulaires ont été préparés 48 heures après transfection de cellulesVeroE6 ou 293T par les plasmides pCI, pCI-S, pCI-S-CTE et pCI-S-WPRE et analysés par western blot comme décrit dans la légende de la figure 22.Le western blot est révélé par luminescence (ECL+, Amersham) et acquisition sur un dispositif d'imagerie numérique (FluorS, BioRad). Les niveaux d'expression sont indiqués en fonction d'une échelle arbitraire où la valeur de 1 représente le niveau mesuré après transfection du plasmide pCI-S. Deux expériences indépendantes ont été réalisées pour chacun des deux types cellulaires. Dans l'expérience 1 sur cellules VeroE6, les transfections ont été réalisées en dupliqués et les résultats sont indiqués sous la forme de moyennes et écart-types des niveaux d'expression mesurés.**

| **Plasmide** | **Cellule** | **exp. 1** | **exp. 2** |
|---|---|---|---|
| **PCI** | VeroE6 | 0,0 | 0,0 |
| **pCI-S** | VeroE6 | 1,0±0,1 | 1,0 |
| **pCI-S-CTE** | VeroE6 | 9,8 ±0,9 | 26,4 |
| **pCI-S-WPRE** | VeroE6 | 20,1±2,0 | 42,3 |
| **PCI** | 293T | 0,0 | 0,0 |
| **PCI-S** | 293T | 1,0 | 1,0 |
| **PCI-S-CTE** | 293T | 4,6 | 4,0 |
| **PCI-S-WPRE** | 293T | 27,6 | 12,8 |

En résumé, l'ensemble de ces résultats montre que l'expression dans des cellules de mammifères du cADN de la S du SRAS-CoV sous la dépendance de séquences promotrices de l'ARN polymérase II, requiert, pour être efficace, la présence d'un signal d'épissage ainsi que de l'une ou l'autre des séquences WPRE et CTE.

### 2) Obtention de lignées stables permettant l'expression de la S du SRAS-CoV

Le cADN de la protéine S du SRAS-CoV a été cloné sous la forme d'un fragment BamH1-Xho1 dans le plasmide pTRIPΔU3-CMV contenant un vecteur lentiviral défectif TRIP à DNA flap central (Sirven et al, 2001, Mol. Ther., 3 :438-448) pour obtenir le plasmide pTRIP-S (figure 24). La co-transfection transitoire selon Zennou et al (2000, Cell, 101 : 173-185) de ce plasmide, d'un plasmide d'encapsidation (p8.2) et d'un plasmide d'expression de la glycoprotéine d'enveloppe G du VSV (pHCMV-G) dans des cellules 293T a permis la préparation de pseudoparticules rétrovirales contenant le vecteur TRIP -S et pseudotypées par la protéine d'enveloppe G. Ces vecteurs TRIP-S pseudotypés ont été utilisés pour transduire des cellules 293T et FRhK-4 : aucune expression de la protéine S n'a pu être mis en évidence par western blot et immunofluorescence dans les cellules transduites (données non présentées).

Les cassettes d'expression optimales constituées du promoteur immédiat/précoce du virus CMV, d'un signal d'épissage, du cDNA de la S et de l'un ou l'autre des signaux post-transcriptionnels WPRE ou CTE décrites ci-dessus ont alors été substituées à la cassette EF1α-EGFP du vecteur d'expression lentiviral défectif à DNA FLAP central TRIPΔU3-EF1α (Sirven et al, 2001, Mol. Ther., 3 :438-448) (figure 25). Ces substitutions ont été réalisées par une série de sous-clonages successifs des cassettes d'expression de S qui ont été excisées des plasmides pCT-S-CTE (BglII-Apa1) ou respectivement pCI-S-WPRE (BglII-Sal1) puis insérées entre les sites Mlu1 et Kpn1 ou respectivement Mlu1 et Xho1 du plasmide TRIPΔU3-EF1α pour obtenir les plasmides pTRIP-SD/SA-S-CTE et pTRIP-SD/SA-S-WPRE, déposés à la CNCM, le 1^{er} décembre 2004, sous les numéros I-3336 et I-3334, respectivement. Des vecteurs pseudotypés ont été produits selon Zennou et al (2000, Cell, 101 : 173-185) et utilisés pour transduire des cellules 293T (10000 cellules) et FRhK-4 (15000 cellules) selon une série de 5 cycles successifs de transduction avec une quantité de vecteur correspondant à 25 ng (TRIP-SD/SA-S-CTE) ou 22 ng TRIP-SD/SA-S-WPRE) de p24 par cycle.

Les cellules transduites ont été clonées par dilution limite et une série de clones ont été analysés pour l'expression de la S du SRAS-CoV qualitativement par immunofluorescence (données non montrées), puis quantitativement par western blot (figure 25) à l'aide d'un sérum polyclonal de lapin anti-S. Les résultats présentés dans la figure 25 montrent que les clones 2 et 15 de cellules FrhK4-s-CTE transduites par TRIP-SD/SA-S-CTE et les clones 4, 9 et 12 de cellules FRhK4-S-WPRE transduites par TRIP-SD/SA-S-WPRE permettent l'expression de la S du SRAS-CoV à des niveaux respectivement faibles et modérés si on les compare à ceux que l'on peut observer au cours de l'infection par le SRAS-CoV.

En résumé, les vecteurs TRIP-SD/SA-S-CTE et TRIP-SD/SA-S-WPRE permettent l'obtention de clones stables de cellules FRhK-4 et de façon similaire 293T exprimant la S du SRAS-CoV, alors que les essais réalisés avec le vecteur "de base" TRIP-S sont restés infructueux, ce qui démontre la nécessité d'un signal d'épissage ainsi que de l'une, ou l'autre, des séquences CTE et WPRE pour l'obtention de clones cellulaires stables exprimant la protéine S.

En outre, ces modifications du vecteur TRIP (insertion d'un signal d'épissage et d'un signal post-transcriptionnel comme CTE et WPRE) pourraient s'avérer intéressantes pour améliorer l'expression d'autres cDNA que celui de la S.

### 3) Obtention de lignées stables permettant l'expression d'une forme soluble de la S du SRAS-CoV. Purification de cet antigène recombinant

Un cDNA codant pour une forme soluble de la protéine S (Ssol) a été obtenu en fusionnant les séquences codant pour l'ectodomaine de la protéine (acides aminés 1 à 1193) à celles d'une étiquette (FLAG : DYKDDDDK) *via* un linker BspE1 codant pour le dipeptide SG. Pratiquement, pour obtenir le plasmide pcDNA-Ssol, un fragment d'ADN codant pour l'ectodomaine de la S du SRAS-CoV a été amplifié par PCR à l'aide des oligonucléotides 5'-ATAGGATCCA CCATGTTTAT TTTCTTATTA TTTCTTACTC TCACT-3' et 5'-ACCTCCGGAT TTAATATATT GCTCATATTT TCCCAA-3' à partir du plasmide pcDNA-S, puis inséré entre les sites uniques BamH1 et BspE1 uniques d'un plasmide d'expression eucaryote pcDNA3.1(+) (Clontech) modifié contenant entre ses sites BamH1 et Xho1 la séquence de l'étiquette FLAG :

// GGATCC ...nnn... TCC GGA GAT TAT AAA GAT GAC GAC GAT AAA TAA
BamH1 S G D Y K D D D D K ter
CTCGAG //
Xho1

Les fragments Nhe1-Xho1 et BamH1-Xho1, contenant le cADN de la S, ont ensuite été excisés du plasmide pcDNA-Sso1, et sous-clonés entre les sites correspondants du plasmide pTRIP-SD/SA-S-CTE et du plasmide pTRIP-SD/SA-S-WPRE, respectivement, pour obtenir les plasmides pTRIP-SD/SA-Ssol-CTE et pTRIP-SD/SA-Sso1-WPRE, déposés à la CNCM, le 1^{er} décembre 2004, sous les numéros I-3337 et I-3335, respectivement.

Des vecteurs pseudotypés ont été produits selon Zennou et coll. (2000, Cell, 101 : 173-185) et utilisés pour transduire des cellules FRhK-4 (15000 cellules) selon une série de 5 cycles successifs de transduction (15000 cellules) avec une quantité de vecteur correspondant à 24 ng (TRIP-SD/SA-Ssol-CTE) ou 40 ng TRIP-SD/SA-Ssol-WPRE) de p24 par cycle. Les cellules transduites ont été clonées par dilution limite et une série de 16 clones transduits par TRIP-SD/SA-Ssol-CTE et de 15 clones par TRIP-SD/SA-Sso1-WPRE ont été analysés pour l'expression du polypeptide Ssol par western blot révélé par un anticorps monoclonal anti-FLAG (figure 26 et données non montrées), ainsi que par un ELISA-capture spécifique du polypeptide Ssol qui a été mis au point dans ce but (Tableau XI et données non montrées). Une partie du processus de sélection des meilleurs clones sécréteurs est montré dans la figure 26. L'ELISA-capture repose sur l'utilisation de phases solides recouvertes d'anticorps polyclonaux de lapins immunisés par du SRAS-CoV purifié et inactivé. Ces phases solides permettent la capture du polypeptide Ssol sécrété dans les surnageants cellulaires, dont la présence est ensuite révélée par une série d'étapes impliquant successsivement la fixation d'un anticorps monoclonal anti-FLAG (M2, SIGMA), d'anticorps biotinylés polyclonaux de lapin anti-IgG(H+L) de souris (Jackson) et d'un conjugué streptavidine-peroxidase (Amersham) puis l'addition de chromogène et de substrat (TMB + H₂0₂, KPL).

**Tableau XI : analyse de l'expression de polypeptide Ssol par des lignées cellulaires transduites par les vecteurs lentiviraux TRIP-SD/SA-Ssol-WPRF, et TRIP-SD/SA-Ssol-CTE. La sécrétion du polypeptide Ssol a été recherchée dans le surnageant d'une série de clones cellulaires isolés après transduction de cellules FRhK-4 par les vecteurs lentiviraux TRIP-SD/SA-Ssol-WPRE et TRIP-SD/SA-Ssol-CTE. Les surnageants dilués au 1/50 ont été analysés par un test ELISA-capture spécifique de la S du SRAS-CoV.**

| Vecteur | Clone | DO (450 |
|---|---|---|
| | | nm) |
| Témoin | - | 0,031 |
| | CTE2 | 0,547 |
| | CTE3 | 0,668 |
| TRIP- | CTE9 | 0,171 |
| SD/SA-Ssol-CTE | CTE12 | 0,208 |
| | CTE 13 | 0,133 |
| TRIP- | WPRE1 | 0,061 |
| SD/SA-Ssol-WPRE | WPRE10 | 0,134 |

La lignée cellulaire sécrétant les quantités les plus élevées de polypeptide Ssol dans le surnageant de culture est la lignée FRhK4-Ssol-CTE3. Elle a été soumise à une deuxième série de 5 cycles de transduction par le vecteur TRIP-SD/SA-Ssol-CTE dans des conditions similaires à celles décrites ci-dessus puis clonée. Le sous-clone sécrétant les quantités les plus élevés de Ssol a été sélectionné par une combinaison d'analyse par western blot et ELISA-capture : il s'agit du sous-clone FRhK4-Ssol-30, qui a été déposé à la CNCM, le 22 novembre 2004, sous le numéro I-3325.

La lignée FRhK4-Ssol-30 permet la production et la purification en quantité du polypeptide recombinant Ssol. Dans une expérience type où les conditions expérimentales de croissance, de production et de purification ont été optimisées, les cellules de la lignée FRhK4-Ssol-30 sont ensemencées en milieu de culture standard (DMEM sans pyruvate contenant 4.5g/l de glucose et supplémenté par 5% de SVF, 100 U/ml de pénicilline et 100 µg/ml de streptomycine) sous la forme d'une monocouche sous-confluente (1 million de cellules pour chaque 100 cm² dans 20 ml de milieu). A confluence, le milieu standard est remplacé par le milieu de sécrétion où la quantité de SVF est abaissée à 0.5% et la quantité de milieu réduite à 16 ml pour chaque 100 cm². Le surnageant de culture est prélevé après 4 à 5 jours d'incubation à 35°C et sous 5% de CO₂. Le polypeptide recombinant Ssol est purifié à partir du surnageant par l'enchaînement d'étapes de filtration sur membrane de polyethersulfone (PES) de 0.1 µm, de concentration par ultrafiltration sur une membrane de PES de point de coupure 50kD, de chromatographie d'affinité sur matrice anti-FLAG avec élution par une solution de peptide FLAG (DYKDDDDK) à 100 µg/ml en TBS (Tris 50mM pH 7.4, 150 mMNaCl) puis de chromatographie de gel filtration en TBS sur billes de sephadex G-75 (Pharmacia). La concentration du polypeptide recombinant Ssol purifié a été déterminée par test micro-BCA (Pierce) puis ses caractéristiques biochimiques analysées.

L'analyse par gel SDS à 8 % d'acrylamide coloré au nitrate d'argent met en évidence un polypeptide majoritaire dont la masse moléculaire est d'environ 180kD et dont le degré de pureté peut être évalué à 98% (figure 27A). Par spectrométrie de masse SELDI-TOF (Cyphergen), deux pics principaux sont mis en évidence : ils correspondent à des formes simplement, et doublement chargées d'un polypeptide majoritaire dont la masse moléculaire est ainsi déterminée à 182,6 ± 3,7 kD (figures 27B et C). Après transfert sur membrane Prosorb et rincage en TFA 0, 1 %, l'extrémité N-terminale du polypeptide Ssol a été séquencée en phase liquide par dégradation d'Edman sur 5 résidus (ABI494, Applied Biosystems) et déterminée comme étant SDLDR (figure 27D). Ceci démontre que le peptide signal localisé à l'extrémité N-terminale de la protéine S du SRAS-CoV, composé des aa 1 à 13 (MFIFLLFLTLTSG) d'après une analyse réalisée avec le logiciel signalP v2.0 (Nielsen et al., 1997, Protein Engineering, 10 : 1-6), est clivé du polypeptide Ssol mature. Le polypeptide recombinant Ssol est donc constitué des acides aminés 14 à 1193 de la protéine S du SRAS-CoV fusionnés en C-terminal à une séquence SGDYKDDDDK contenant la séquence de l'étiquette FLAG (soulignée). L'écart entre la masse molaire théorique du polypeptide Ssol nu (132,0 kD) et la masse molaire réelle du polypeptide mature (182,6 kD) suggère que le polypeptide Ssol est glycosylé.

Une préparation de polypeptide Ssol purifié et dont la concentration protéique a été déterminée par test micro-BCA, permet de réaliser une gamme étalon pour mesurer à l'aide du test ELISA-capture décrit plus haut les concentrations de Ssol présent dans les surnageants de culture et de revisiter les caractéristiques des lignées sécrétrices. Selon ce test, la lignée FRhK4-Ssol-CT3 sécrète 4 à 6 µg/ml de polypeptide Ssol tandis que la lignée FRhK4-Ssol-30 sécrète 9 à 13 µg/ml de Ssol après 4 à 5 jours de culture à confluence. En outre, le schéma de purification présenté plus haut permet en routine de purifier de 1 à 2 mg de polypeptide Ssol par litre de surnageant de culture.

### Exemple 12 : Immunisation génique visant la proteine de spicule (S) du coronavirus associé au SRAS (SRAS-CoV)

L'effet d'un signal d'épissage et des signaux post-transcriptionnels WPRE et CTE a été analysé après immunisation génique de souris BALB/c (figure 28).

Pour cela, des souris BALB/c ont été immunisées à des intervalles de 4 semaines par injection dans le *tibialis anterior* d'une solution saline de 50 µg d'ADN plasmidique de pcDNA-S et pCI-S ainsi que, à titre de contrôle, par 50 µg d'ADN plasmidique de pcDNA-N (dirigeant l'expression de la N du SRAS-CoV) ou de pCI-HA (dirigeant l'expression de la HA du virus grippal A/PR/8/34) et les sérums immuns collectés 3 semaines après la 2ème injection. La présence d'anticorps dirigés contre la S du SRAS-CoV a été recherchée par ELISA indirect en utilisant comme antigène un lysat de cellules VeroE6 infectées par le SRAS-CoV et à titre de contrôle, un lysat de cellules VeroE6 non infectées. Les titres (TI) en anticorps anti-SRAS-CoV sont calculés comme l'inverse de la dilution produisant une DO spécifique de 0,5 (différence entre DO mesurée sur lysat de cellules infectées et DO mesurée sur lysat de cellules non infectées) après révélation par un anticorps polyclonal anti-IgG de souris couplé à la peroxidase (NA931 V, Amersham) et du TMB additionné de H₂O₂ (KPL) (figure 28A).

Dans ces conditions, le plasmide d'expression pcDNA-S ne permet l'induction que de faibles titres d'anticorps dirigées contre la S du SRAS-CoV chez 3 souris sur 6 (LOG₁₀(TI)=1,9±0,6) alors que le plasmide pcDNA-N permet l'induction d'anticorps anti-N à des titres élevés (LOG₁₀(TI)= 3,9±0,3) chez tous les animaux, et les plasmides contrôles (pCI, pCI-HA) n'entraînent aucun anticorps détectable (LOG₁₀(TI)<1,7). Le plasmide pCI-S muni d'un signal d'épissage permet l'induction d'anticorps à des titres élevés (LOG₁₀(TI)= 3,7±0,2), qui sont environ 60 fois supérieurs à ceux observés après injection du plasmide pcDNA-S (p<10⁻⁵).

L'efficacité des signaux post-transcriptionnels a été étudiée en réalisant une étude dose-réponse des titres en anticorps anti-S induits chez la souris BALB/c en fonction de la quantité d'ADN plasmidique utilisé comme immunogène (2µg, 10 µg et 50 µg). Cette étude (figure 28B) démontre que le signal post-transcriptionnel WPRE améliore fortement l'efficacité de l'immunisation génique lorsque de faibles doses d'ADN sont utilisées (p<10⁻⁵ pour une dose de 2 µg d'ADN et p<10⁻² pour une dose de 10 µg), alors que l'effet du signal CTE reste marginal (p=0,34 pour une dose de 2 µg d'ADN).

Enfin, les anticorps induits chez la souris après immunisation génique neutralisent l'infectivité du SRAS-CoV *in vitro* (figures 29A et 29B) à des titres qui sont en rapport avec les titres mesurés par ELISA.

En résumé, l'utilisation d'un signal d'épissage et du signal post-transcriptionnel WPRE du virus de l'hépatite de la marmotte améliore de façon considérable l'induction d'anticorps neutralisants dirigés contre le SRAS-CoV après immunisation génique à l'aide d'ADN plasmidique dirigeant l'expression du cADN de la S du SRAS-CoV.

### Exemple 13 : Applications diagnostiques de la protéine S

La réactivité en ELISA du polypeptide recombinant Ssol a été analysée vis-à-vis de sérums de patients atteints de SRAS.

Les sérums de cas probables de SRAS testés ont été choisis sur la base des résultats (positifs ou négatifs) d'analyse de leur réactivité spécifique vis-à-vis des antigènes natifs du SRAS-CoV par test d'immunofluorescence sur cellules VeroE6 infectées par le SRAS-CoV et/ou par test ELISA indirect en utilisant comme antigène un lysat de cellules VeroE6 infectées par le SRAS-CoV. Les sérums de ces patients sont identifiés par un numéro d'ordre du Centre National de Référence des Virus Influenzae ainsi que par les initiales du patient et le nombre de jours écoulés depuis le début des symptomes. Tous les sérums de cas probables (cf Tableau XII) reconnaissent les antigènes natifs du SRAS-CoV, à l'exception du sérum 032552 du patient VTT, pour lequel l'infection par le SRAS-CoV n'a pas pu être confirmée par RT-PCR réalisée sur prélèvements respiratoires des jours 3, 8 et 12. Un panel de sérums témoin a été utilisé à titre de contrôle (sérums TV): il s'agit de sérums prélevés en France avant l'épidémie de SRAS survenue en 2003.

**Tableau XII : sérums de cas probables de SRAS**

| **sérum** | **patient** | **jour de prélèvement** |
|---|---|---|
| 031724 | JYK | 7 |
| 033168 | JYK | 38 |
| 033597 | JYK | 74 |
| 032632 | NTM | 17 |
| 032634 | THA | 15 |
| 032541 | PHV | 10 |
| 032542 | NIH | 17 |
| 032552 | VTT | 8 |
| 032633 | PTU | 16 |
| 032791 | JLB | 3 |
| 033258 | JLB | 27 |
| 032703 | JCM | 8 |
| 033153 | JCM | 29 |

Des phases solides sensibilisées par le polypeptide recombinant Ssol ont été préparées par adsorption d'une solution de polypeptide Ssol purifié à 2 µg/ml en PBS dans les puits d'une plaque ELISA, puis les plaques sont incubées une nuit à 4°C et lavées avec du tampon PBS-Tween (PBS, 0,1% Tween20). Après saturation des plaques ELISA par une solution de PBS-lait écrémé à 10% (poids/volume) et lavage en PBS-tween, les sérums à tester (100 µl) sont dilués au 1/400 dans du tampon PBS-lait écrémé-Tween (PBS, 3% lait écrémé, 0,1% Tween) puis ajoutés dans les puits de la plaque ELISA sensibilisée. Les plaques sont incubées 1h à 37°C. Après 3 lavages avec du tampon PBS-Tween, le conjugué anti-IgG humaines marqué à la peroxidase (réf NA933V, Amersham) dilué au 1/4000 dans du tampon PBS-lait écrémé-Tween est ajouté, puis les plaques sont incubées une heure à 37°C. Après 6 lavages avec du tampon PBS-Tween, le chromogène (TMB) et le substrat (H₂O₂) sont ajoutés et les plaques sont incubées 10 minutes à l'abri de la lumière. La réaction est arrêtée par ajout d'une solution 1N de H₃PO₄, puis l'absorbance est mesurée à 450 nm avec un référence à 620nm.

Les tests ELISA (figure 30) démontrent que le polypeptide recombinant Ssol est reconnu spécifiquement par les anticorps sériques de patients atteints de SRAS prélevés en phase moyenne ou tardive de l'infection (≥ 10 jours après le début des symptômes alors qu'il n'est pas reconnu de façon significative par les anticorps sériques de deux patients (JLB et JCM) prélevés en phase précoce de l'infection (3 à 8 jours après le début des symptômes) ni par des sérums témoins de sujets non atteints de SRAS. Les anticorps sériques des patients JLB et JCM montrent une séroconversion entre les jours 3 et 27 pour le premier et 8 et 29 pour le second après le début des symptômes, ce qui confirme la spécificité de la réactivité de ces sérums vis-à-vis du polypeptide Ssol.

En conclusion, ces résultats démontrent que le polypeptide recombinant Ssol peut être utilisé comme antigène pour la mise au point d'un test ELISA de diagnostic sérologique de l'infection par le SRAS-CoV.

### Exemple 14 : Applications vaccinales de la protéine S soluble recombinante

L'immunogénicité du polypeptide recombinant Ssol a été étudiée chez la souris.

Pour cela, un groupe de 6 souris a été immunisé à 3 semaines d'intervalle avec 10 µg de polypeptide recombinant Ssol adjuvé par 1 mg d'hydroxyde d'aluminium (Alu-gel-S, Serva) dilué en PBS. Trois immunisations successives ont été réalisées et les sérums immuns ont été prélevés 3 semaines après chacune des immunisations (IS1, IS2, IS3). A titre de contrôle, un groupe de souris (groupe mock) a reçu de l'hydroxide d'aluminium seul selon le même protocole.

Les sérums immuns ont été analysés par pool pour chacun des 2 groupes par ELISA indirect en utilisant un lysat de cellules VeroE6 infectées par le SRAS-CoV comme antigène et à titre de contrôle, un lysat de cellules VeroE6 non infectées. Les titres en anticorps anti-SRAS-CoV sont calculés comme l'inverse de la dilution produisant une DO spécifique de 0,5 après révélation par un anticorps polyclonal anti-IgG(H+L) de souris couplé à la peroxidase (NA931V, Amersham) et du TMB additionné de H₂O₂ (KPL). Cette analyse (figure 31) montre que l'immunisation par le polypeptide Ssol induit chez la souris dès la première immunisation des anticorps dirigés contre la forme native de la protéine de spicule du SRAS-CoV présente dans le lysat de cellules VeroE6 infectées. Après 2 puis 3 immunisations, les titres en anticorps anti-S deviennent très élevés.

Les sérums immuns ont été analysés par pool pour chacun des 2 groupes pour leur capacité à séroneutraliser l'infectivité du SRAS-CoV. 4 points de séroneutralisation sur cellules FRhK-4 (100 TCID50 de SRAS-CoV) sont réalisés pour chacune des dilutions de raison 2 testées à partir du 1/20. Le titre séroneutralisant est calculé selon la méthode de Reed et Munsch comme l'inverse de la dilution neutralisant l'infectivité de 2 cupules sur 4. Cette analyse montre que les anticorps induits chez la souris par le polypeptide Ssol sont neutralisants : les titres observés sont très élevés après 2 puis 3 immunisations (supérieurs à 2560 et 5120 respectivement, Tableau XIII).

**Tableau XIII : induction d'anticorps dirigés contre le SRAS-CoV après immunisation avec le polypeptide recombinant Ssol. Les sérums immuns ont été analysés par pool pour chacun des 2 groupes pour leur capacité à séroneutraliser l'infectivité de 100 TCID50 du SRAS-CoV sur cellules FRhK-4. 4 points sont réalisés pour chacune des dilutions de raison 2 testées à partir du 1/20. Le titre séroneutralisant est calculé selon la méthode de Reed et Munsch comme l'inverse de la dilution neutralisant l'infectivité de 2 cupules sur 4.**

| **groupe** | **sérums** | **Ac. Neutralisants** |
|---|---|---|
| Mock | pi | < 20 |
| | IS1 | < 20 |
| | IS2 | < 20 |
| | IS3 | < 20 |
| Ssol | pi | < 20 |
| | IS1 | 57 |
| | IS2 | > 2560 |
| | IS3 | > 5120 |

Les titres neutralisants observés chez les souris immunisées avec le polypeptide Ssol atteignent des niveaux très supérieurs aux titres observés par Yang et coll. chez la souris (2004, Nature, 428: 561-564) et à ceux observés par Buchholz chez le hamster (2004, PNAS 101 : 9804-9809), qui protègent respectivement la souris et le hamster de l'infection par le SRAS-CoV. Il est donc vraisemblable que les anticorps neutralisants induits chez la souris après immunisation par le polypeptide Ssol protègent ces animaux contre l'infection par le SRAS-CoV.

### Exemple 15 : Gène synthétique optimisé pour l'expression en cellules de mammifères de la protéine de spicule (S) du coronavirus associé au SRAS (SRAS-CoV).

### 1) Conception du gène synthétique

Un gène synthétique codant pour la protéine de spicule du SRAS-CoV a été conçu à partir du gène de l'isolat 031589 (plasmide pSARS-S, C.N.C.M . n° I-3059) de façon à permettre des niveaux d'expression élevés dans des cellules de mammifères et en particulier dans les cellules d'origine humaine.

Pour cela :
- l'usage des codons du gène sauvage de l'isolat 031589 a été modifié de façon à se rapprocher du biais observé chez l'homme et à améliorer l'efficacité de traduction du mRNA correspondant
- le contenu global en GC du gène a été augmenté de façon à prolonger la demi-vie du mRNA correspondant
- les motifs, éventuellement cryptiques, susceptibles d'interférer avec une expression efficace du gène ont été supprimés (sites donneurs et accepteurs d'épissage, signaux de polyadénylation, séquences très riches (>80%) ou très pauvres (<30%) en GC, séquences répétées, séquences impliquées dans la formation de structures secondaires de l'ARN, boites TATA)
- un deuxième codon STOP a été ajouté pour permettre une terminaison efficace de la traduction.

En outre, des motifs CpG ont été introduits dans le gène de façon à augmenter son immunogénicité comme vaccin à ADN. Afin de faciliter la manipulation du gène synthétique, deux sites de restriction BamH1 et Xho1 ont été placés de part et d'autre de la phase ouverte de lecture de la protéine S, et les sites de restriction BamH1, Xho1, Nhe1, Kpn1, BspE1 et Sal1 ont été évités dans le gène synthétique.

La séquence du gène synthétique conçu (gène 040530) est donnée en SEQ ID No: 140.

Un alignement du gène synthétique 040530 avec la séquence du gène sauvage de l'isolat 031589 du SRAS-CoV déposé à la C.N.CM. sous le numéro I-3059 (SEQ ID NO : 4, plasmide pSRAS-S) est présenté dans la figure 32.

### 2) Constructions plasmidiques

Le gène synthétique SEQ ID No: 140 a été assemblé à partir d'oligonucléotides synthétiques et cloné entre les sites Kpn1 et Sac1 du plasmide pUC-Kana pour donner le plasmide 040530pUC-kana. La séquence nucléotidique de l'insert du plasmide 040530pUC-kana a été vérifiée par séquençage automatique (Applied).

Un fragment Kpn1-Xho1 contenant le gène synthétique 040530 a été excisé du plasmide 040530pUC-kana et sous-cloné entre les sites Nhe1 et Xho1 du plasmide d'expression pCI (Promega) pour obtenir le plasmide pCI-SSYNTH, déposé à la CNCM le 1^{er} décembre 2004, sous le numéro I-3333.

Un gène synthétique codant pour la forme soluble de la protéine S a ensuite été obtenu en fusionnant les séquences synthétiques codant pour l'ectodomaine de la protéine S (acides aminés 1 à 1193) à celles de l'étiquette (FLAG : DYKDDDDK) via un linker BspE1 codant pour le dipeptide SG. Pratiquement, un fragment d'ADN codant pour l'ectodomaine de la S du SRAS-CoV a été amplifié par PCR à l'aide des oligonucléotides 5'-ACTAGCTAGC GGATCCACCA TGTTCATCTT CCTG -3' et 5'- AGTATCCGGAC TTGATGTACT GCTCGTACTT GC-3' à partir du plasmide 040530pUC-kana, digéré par Nhe1 et BspE1 puis inséré entre les sites uniques Nhe1 et BspE1 du plasmide pCI-Ssol, pour donner le plasmide pCI-SCUBE, déposé à la CNCM le 1^{er} décembre 2004, sous le numéro I-3332. (Les plasmides pCI-Ssol, pCI-Ssol-CTE et pCI-Sso1-WPRE (déposé à la CNCM, le 22 novembre 2004, sous le numéro 1-3324) avaient été précédemment obtenus par sous-clonage du fragment Kpn1-Xho1 excisé du plasmide pcDNA-Sso1 (voir note technique de la DI 2004-106) entre les sites Nhe1 et Xho1 des plasmides pCI, pCI-S-CTE et pCI-S-WPRE respectivement.)

Les plasmides pCI-Scube et pCI-Ssol codent pour le même polypeptide recombinant Ssol.

### 3) Résultats

La capacité du gène synthétique codant pour la protéine S à diriger efficacement l'expression de la S du SRAS-CoV dans des cellules de mammifères a été comparée à celle du gène sauvage après transfection transitoire de cellules de primates (VeroE6) et de cellules humaines (293T).

Dans l'expérience présentée par la figure 33 et au Tableau XIV, des monocouches de 5x10⁵ cellules VeroE6 ou 7x10⁵ cellules 293T en boites de Pétri de 35mm ont été transfectées avec 2 µg des plasmides pCI (à titre de contrôle), pCI-S, pCI-S-CTE, pCI-S-WPRE et pCI-Ssynth et 6 µl de réactif Fugene6 selon les indications du fabricant (Roche). Après 48 heures d'incubation à 37°C et sous 5% de CO₂, des extraits cellulaires ont été préparés en tampon de dépôt selon Laemmli, séparés sur un gel SDS à 8% de polyacrylamide puis transférés sur une membrane de PVDF (BioRad). La détection de cette immunoempreinte (« western blot ») a été réalisée à l'aide d'un sérum polyclonal de lapin anti-S (immun-sérum du lapin P11135 : cf exemple 4 ci-dessus) et d'anticorps polyclonaux d'âne dirigés contre les IgG de lapin et couplés à la peroxidase (NA934V, Amersham). L'immunoempreinte est révélée de façon quantitative par luminescence à l'aide du kit ECL+ (Amersham) et acquisition sur un dispositif d'imagerie numérique (FluorS, BioRad).

L'analyse des résultats obtenus avec le logiciel QuantityOne v4.2.3 (BioRad) montre que dans cette expérience, le plasmide pCI-Synth permet l'expression transitoire de la protéine S à des niveaux élevés dans les cellules VeroE6 et 293T, alors que le plasmide pCI-S ne permet pas d'induire une expression à des niveaux suffisants pour être détectée. Les niveaux d'expression observés sont de l'ordre de 2 fois supérieurs à ceux observés avec le plasmide pCI-S-WPRE.

**Tableau XIV : utilisation d'un gène synthétique pour l'expression de la S du SRAS-CoV. Des extraits cellulaires préparés 48 heures après transfection de cellulesVeroE6 ou 293T par les plasmides pCI, pCI-S, pCI-S-CTE, pCI-S-WPRE et pCI-Ssynth ont été séparés sur un gel SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham). Le western blot est révélé par luminescence (ECL+, Amersham) et acquisition sur un dispositif d'imagerie numérique (FluorS, BioRad). Les niveaux d'expression de la protéine S ont été mesurés en quantifiant les 2 bandes majoritaires repérées sur l'image (voir figure 33) et sont indiqués en fonction d'une échelle arbitraire où la valeur de 1 représente le niveau mesuré après transfection du plasmide pCI-S-WPRE.**

| **plasmide** | **VeroE6** | **293T** |
|---|---|---|
| **pCI** | 0,0 | 0,0 |
| **pCI-S** | ≤ 0,1 | ≤ 0,1 |
| **pCI-S-CTE** | 0,5 | ≤ 0,1 |
| **pCI-S-WPRE** | 1,0 | 1,0 |
| **PCI-Ssynth** | 1,8 | 1,9 |

Dans un second temps, la capacité du gène synthétique Scube à diriger efficacement la synthèse et la sécrétion du polypeptide Ssol par des cellules de mammifères a été comparée à celle du gène sauvage après transfection transitoire de cellules de hamster (BHK-21) et de cellules humaines (293T).

Dans l'expérience présentée par le Tableau XV, des monocouches de 6x10⁵ cellules BHK-21 et de 7x10⁵ cellules 293T en boites de Pétri de 35mm ont été transfectées avec 2 µg des plasmides pCI (à titre de contrôle), pCI-Ssol, pCI-Ssol-CTE, pCI-Ssol-WPRE et pCI-Scube et 6 µl de réactif Fugene6 selon les indications du fabricant (Roche). Après 48 heures d'incubation à 37°C et sous 5% de CO2, les surnageants cellulaires ont été prélevés et analysés de façon quantitative pour la sécrétion du polypeptide Ssol par un test ELISA-capture spécifique du polypeptide Ssol.

L'analyse des résultats montre que, dans cette expérience, le plasmide pCI-Scube permet l'expression du polypeptide Ssol à des niveaux 8 fois (cellules BHK-21) à 20 fois (cellules 293T) plus élevés que le plasmide pCI-Ssol. Les niveaux d'expression observés sont de l'ordre de 2 fois (cellules 293T) à 5 fois (cellules BHK-21) supérieurs à ceux observés avec le plasmide pCI-Ssol-WPRE.

**Tableau XV : utilisation d'un gène synthétique pour l'expression du polypeptide Ssol. Les surnageants ont été récoltés 48 heures après transfection de cellules BHK ou 293T par les plasmides pCI, pCI-Ssol, pCI-Ssol-CTE, pCI-Ssol-WPRE et pCI-Scube et analysés de façon quantitative pour la sécrétion du polypeptide Ssol par un test ELISA-capture spécifique du polypeptide Ssol. Les transfections ont été réalisées en dupliqués et les résultats sont indiqués sous la forme de moyennes et écart-types des concentrations de polypeptide Ssol (ng/ml) mesurées dans les surnageants.**

| **Plasmide** | **BHK** | **293T** |
|---|---|---|
| **Pci** | < 20 | < 20 |
| **pCI-Ssol** | < 20 | 56 ± 10 |
| **pCI-Ssol-CTE** | < 20 | 63 ± 8 |
| **pCI-Ssol-WPRE** | 28 ± 1 | 531 ± 15 |
| **PCI-Scube** | 152 ± 6 | 1140 ± 20 |

En résumé, ces résultats montrent que l'expression dans des cellules de mammifères du gène synthétique 040530 codant pour la S du SRAS-CoV sous la dépendance de séquences promotrices de l'ARN polymérase II est bien plus efficace que celle du gène sauvage de l'isolat 031589. Cette expression est même plus efficace que celle dirigée par le gène sauvage en présence des séquences WPRE du virus de l'hépatite de la marmotte.

### 4) Applications

L'utilisation du gène synthétique 040530 codant pour la S du SRAS-CoV ou de sa variante Scube codant pour le polypeptide Ssol est susceptible de remplacer avantageusement le gène sauvage dans de nombreuses applications où l'expression de la S est nécessaire à des niveaux élevés. En particulier pour :
- améliorer l'efficacité de l'immunisation génique par des plasmides du type pCI-Ssynth voire pCI-Ssynth-CTE ou pCI-Ssynth-WPRE
- établir de nouvelles lignées cellulaires exprimant des quantités plus élevées de la protéine S ou du polypeptide Ssol à l'aide de vecteurs lentiviraux recombinants porteurs du gène Ssynth ou du gène Scube respectivement
- améliorer l'immunogénicité des vecteurs lentiviraux recombinants permettant l'expression de la protéine S ou du polypeptide Ssol
- améliorer l'immunogénicité de vecteurs vivants permettant l'expression de la protéine S ou du polypeptide Ssol comme des virus recombinants de la vaccine ou des virus rougeole recombinants (voir exemples 16 et 17 ci-après)

### Exemple 16 : Expression de la protéine de spicule (S) du coronavirus associé au SRAS (SRAS-CoV) à l'aide de virus recombinants de la Vaccine.

### Application vaccinale.

### Application à la production d'une forme soluble de la protéine de spicule (S) et conception d'un test de sérologie du SRAS.

### 1) Introduction

Le but de cet exemple est d'évaluer la capacité de virus recombinants de la vaccine (VV) exprimant différents antigènes du coronavirus associé au SRAS (SRAS-CoV) à constituer de nouveaux candidats vaccins contre le SRAS et un moyen de produire des antigènes recombinants en cellules de mammifères.

Pour cela, les inventeurs se sont intéressés à la protéine de spicule (S) du SRAS-CoV, qui permet d'induire après immunisation génique chez l'animal des anticorps neutralisants l'infectivité du SRAS-CoV, ainsi qu'à une forme soluble et sécrétée de cette protéine, le polypeptide Ssol, qui est composé de l'ectodomaine (aa 1-1193) de la S fusionné en son extrémité C-ter à une étiquette FLAG (DYKDDDDK) via un linker BspE1 codant pour le dipeptide SG. Ce polypeptide Ssol présente une antigénicité similaire à celle de la protéine S et permet, après injection à la souris sous la forme d'une protéine purifiée adjuvée en hydroxide d'aluminium, l'induction de titres élevés d'anticorps neutralisants contre le SRAS-CoV.

Les différentes formes du gène S ont été placées sous la dépendance du promoteur du gène 7.5K puis introduites au sein du locus de la thymidine kinase (TK) de la souche Copenhague du virus de la vaccine par double recombinaison *homologue in vivo.* Afin d'améliorer l'immunogénicité des virus vaccine recombinants, un promoteur tardif synthétique a été choisi à la place du promoteur 7.5K, pour augmenter la production de S et Ssol au cours des phases tardives du cycle viral.

Après avoir isolé les virus vaccine recombinants et vérifié leur capacité à exprimer l'antigène S du SRAS-CoV, leur capacité à induire chez la souris une réponse immunitaire contre le SRAS a été testée. Après avoir purifié l'antigène Ssol du surnageant de cellules infectées, un test ELISA de sérodiagnostic du SRAS a été conçu, et son efficacité a été évaluée à l'aide de sérums de cas probables de SRAS.

### 2) Construction des virus recombinants

Des virus recombinants de la vaccine dirigeant l'expression de la glycoprotéine S de l'isolat 031589 du SRAS-CoV et d'une forme soluble et sécrétée de cette protéine, le polypeptide Ssol, sous la dépendance du promoteur 7.5K ont été obtenus. Dans le but d'augmenter les niveaux d'expression de S et Ssol, des virus recombinants dans lesquels les cDNA de S et de Ssol sont placés sous la dépendance d'un promoteur synthétique tardif ont également été obtenus.

Le plasmide pTG186poly est un plasmide de transfert pour la construction de virus recombinants de la vaccine (Kieny, 1986, Biotechnology, 4 :790-795). A ce titre, il contient le gène de la thymidine kinase du VV dans lequel a été inséré le promoteur du gène 7.5K suivi d'un site multiple de clonage permettant l'insertion de gènes hétérologues (figure 34A). Le promoteur du gène 7.5K contient en fait un tandem de deux séquences promotrices actives respectivement durant les phases précoces (P_{E}) et tardives (P_{L}) du cycle de réplication du virus de la vaccine. Les fragments BamH1-Xho1 ont été excisés des plasmides pTRIP-S et pcDNA-Ssol respectivement et insérés entre les sites BamH1 et Sma1 du plasmide pTG186poly pour donner les plasmides pTG-S et pTG-Ssol (figure 34A). Les plasmides pTG-S et pTG-Ssol ont été déposés à la CNCM, le 2 décembre 2004, sous les numéros I-3338 et I-3339, respectivement.

Les plasmides pTN480, pTN-S et pTN-Ssol ont été obtenus à partir des plasmides pTG186poly, pTG-S et pTG-Ssol respectivement, en substituant le fragment Nde1-Pst1 contenant le promoteur 7.5K par un fragment d'ADN contenant le promoteur tardif synthétique 480, qui a été obtenu par hybridation des oligonucléotides 5'- TATGAGCTTT TTTTTTTTTT TTTTTTTGGC ATATAAATAG ACTCGGCGCG CCATCTGCA-3' et 5'- GATGGCGCGC CGAGTCTATT TATATGCCAA AAAAAAAAAA AAAAAAAAGC TCA-3' (figure 34B). L'insert a été séquencé à l'aide d'un kit BigDye Terminator v1.1 (Applied Biosystems) et d'un séquenceur automatique ABI377. La séquence du promoteur synthétique tardif 480 tel que cloné dans les plasmides de transfert de la série pTN est indiquée figure 34C. Les plasmides pTN-S et pTN-Ssol ont été déposés à la CNCM, le 2 décembre 2004, sous les numéros I-3340 et I-3341, respectivement.

Les virus recombinants de la vaccine ont été obtenus par double recombinaison homologue *in vivo* entre la cassette TK des plasmides de transfert des séries pTG et pTN et le gène TK de la souche Copenhague du virus de la vaccine selon une procédure décrite par Kieny et coll. (1984, Nature, 312 : 163-166). Brièvement, des cellules CV-1 sont transfectées à l'aide de DOTAP (Roche) par de l'ADN génomique de la souche copenhague du virus de la vaccine et chacun des plasmides de transfert des séries pTG et pTN décrits ci-dessus, puis surinfectées par le virus vaccine auxiliaire VV-ts7 pendant 24 heures à 33°C. Le virus auxiliaire est contre-sélectionné par incubation à 40°C pendant 2 jours puis les virus recombinants (phénotype TK-) sélectionnés par deux cycles de clonage sous milieu gélosé sur cellules 143Btk- en présence de BuDr (25 µg/ml). Les 6 virus VV-TG, VV-TG-S, VV-TG-Ssol, VV-TN, VV-TN-S et VV-TN-Ssol ont été respectivement obtenus à l'aide des plasmides de transfert pTG186poly, pTG-S, pTG-Ssol, pTN480, pTN-S, pTN-Ssol. Les virus VV-TG et VV-TN n'expriment aucun gène hétérologue et ont été utilisés comme contrôle TK- dans les expériences. Les préparations de virus recombinants ont été réalisés sur monocouches de cellules CV-1 ou BHK-21 et le titre en unités formant plage (u.f.p.) déterminé sur cellules CV-1 selon Earl et Moss (1998, Current Protocols in Molecular Biology, 16.16.1-16.16.13).

### 3) Caractérisation des virus recombinants

L'expression des transgènes codant la protéine S et le polypeptide Ssol a été recherchée par western blot.

Des monocouches de cellules CV-1 ont été infectées à une multiplicité de 2 par les différents virus vaccine recombinants VV-TG, VV-TG-S, VV-TG-Ssol, VV-TN, VV-TN-S et VV-TN-Ssol. Après 18 heures d'incubation à 37°C et sous 5% de CO2, des extraits cellulaires ont été préparés en tampon de dépôt selon Laemmli, séparés sur un gel SDS à 8% de polyacrylamide puis transférés sur une membrane de PVDF (BioRad). La détection de cette immunoempreinte (« western blot ») a été réalisée à l'aide d'un sérum polyclonal de lapin anti-S (immun-sérum du lapin P11135 : cf exemple 4) et d'anticorps polyclonaux d'âne dirigés contre les IgG de lapin et couplés à la peroxidase (NA934V, Amersham). Les anticorps fixés ont été révélés par luminescence à l'aide du kit ECL+ (Amersham) et de films d'autoradiographie Hyperfilm MP (Amersham).

Comme le montre la figure 35A, le virus recombinant VV-TN-S dirige l'expression de la protéine S à des niveaux qui sont comparables à ceux que l'on peut observer 8h après infection par le SRAS-CoV mais qui sont bien plus élevés que ceux que l'on peut observer après infection par VV-TG-S. Dans une deuxième expérience (figure 35B), l'analyse de quantités variables d'extraits cellulaires montre que les niveaux d'expression observés après infection par les virus de la série TN (VV-TN-S et VV-TN-Ssol) sont environ 10 fois plus élevés que ceux observés avec les virus de la série TG (VV-TG-S et VV-TG-Ssol respectivement). En outre, le polypeptide Ssol est sécrété dans le surnageant de cellules CV-1 infectées par le virus VV-TN-Ssol plus efficacement que dans le surnageant de cellules infectées par VV-TG-Ssol (figure 36A). Dans cette expérience, le virus VV-TN-Sflag a été utilisé à titre de contrôle, car il exprime la forme membranaire de la protéine S fusionnée en son extrémité C-ter à l'étiquette FLAG. La protéine Sflag n'est pas détectée dans le surnageant des cellules infectées par VV-TN-Sflag, démontrant que le polypeptide Ssol est bien sécrété de façon active après infection par VV-TN-Ssol.

Ces résultats démontrent que les virus vaccine recombinants sont bien porteurs des transgènes et permettent l'expression de la glycoprotéine du SRAS sous sa forme membranaire (S) ou sous une forme soluble et sécrétée (Ssol). Les virus vaccine porteurs du promoteur synthétique 480 permettent l'expression de S et la sécrétion de Ssol à des niveaux bien plus élevés que les virus porteurs du promoteur du gène 7.5K.

### 4) Application à la production d'une forme soluble de la S du SRAS-CoV. Purification de cet antigène recombinant et applications diagnostiques.

La lignée BHK-21 est la lignée cellulaire qui sécrète les quantités les plus élevées de polypeptide Ssol après infection par le virus VV-TN-Ssol parmi les lignées testées (BHK-21, CV1, 293T et FrhK-4, figure 36B); elle permet la production et la purification en quantité du polypeptide recombinant Ssol. Dans une expérience type où les conditions expérimentales d'infection, de production et de purification ont été optimisées, les cellules BHK-21 sont ensemencées en milieu de culture standard (DMEM sans pyruvate contenant 4.5g/l de glucose et supplémenté par 5% de TPB, 5% de SVF, 100 U/ml de pénicilline et 100 µg/ml de streptomycine) sous la forme d'une monocouche sous-confluente (10 millions de cellules pour chaque 100 cm² dans 25 ml de milieu). Après 24h d'incubation à 37°C sous 5% de CO₂, les cellules sont infectées à une M.O.I. de 0.03 et le milieu standard remplacé par le milieu de sécrétion où la quantité de SVF est abaissée à 0.5% et le TPB supprimé. Le surnageant de culture est prélevé après 2,5 jours d'incubation à 35°C et sous 5% de CO₂ et le virus de la vaccine inactivé par addition de triton X-100 (0,1%). Après filtration sur membrane de polyethersulfone (PES) de 0.1 µm, le polypeptide recombinant Ssol est purifié par une chromatographie d'affinité sur matrice anti-FLAG avec élution par une solution de peptide FLAG (DYKDDDDK) à 100 µg/ml en TBS (Tris 50mM pH 7.4, 150 mMNaCl).

L'analyse par gel SDS à 8 % d'acrylamide coloré au nitrate d'argent a mis en évidence un polypeptide majoritaire dont la masse moléculaire est d'environ 180kD et dont le degré de pureté est supérieur à 90% (figure 37).La concentration du polypeptide recombinant Ssol purifié a été déterminée par comparaison avec les marqueurs de masse moléculaire et estimée à 24 ng/µl.

Cette préparation de polypeptide Ssol purifié permet de réaliser une gamme étalon pour mesurer à l'aide d'un test ELISA-capture les concentrations de Ssol présentes dans les surnageants de culture. Selon ce test, la lignée BHK-21 sécrète environ 1 µg/ml de polypeptide Ssol dans les conditions de production décrites plus haut. En outre, le schéma de purification présenté permet de purifier de l'ordre de 160 µg de polypeptide Ssol par litre de surnageant de culture.

La réactivité en ELISA du polypeptide recombinant Ssol a été analysée vis-à-vis de sérums de patients atteints de SRAS.

Les sérums de cas probables de SRAS testés ont été choisis sur la base des résultats (positifs ou négatifs) d'analyse de leur réactivité spécifique vis à vis des antigènes natifs du SRAS-CoV par test d'immunofluorescence sur cellules VeroE6 infectées par le SRAS-CoV et/ou par test ELISA indirect en utilisant comme antigène un lysat de cellules VeroE6 infectées par le SRAS-CoV. Les sérums de ces patients sont identifiés par un numéro d'ordre du Centre National de Référence des Virus Influenzae ainsi que par les initiales du patient et le nombre de jours écoulés depuis le début des symptômes. Tous les sérums de cas probables (cf Tableau XVI) reconnaissent les antigènes natifs du SRAS-CoV à l'exception du sérum 032552 du patient VTT, pour lequel l'infection par le SRAS-CoV n'a pas pu être confirmée par RT-PCR réalisée sur prélèvements respiratoires des jours 3, 8 et 12. Un panel de sérums témoin a été utilisé à titre de contrôle (sérums TV): il s'agit de sérums prélevés en France avant l'épidémie de SRAS survenue en 2003.

**Tableau XVI : sérums de cas probables de SRAS**

| **sérum** | **patient** | **jour de prélèvement** |
|---|---|---|
| 033168 | JYK | 38 |
| 033597 | JYK | 74 |
| 032632 | NTM | 17 |
| 032634 | THA | 15 |
| 032541 | PHV | 10 |
| 032542 | NIH | 17 |
| 032552 | VTT | 8 |
| 032633 | PTU | 16 |

Des phases solides sensibilisées par le polypeptide recombinant Ssol ont été préparées par adsorption d'une solution de polypeptide Ssol purifié à 4 µg/ml en PBS dans les puits d'une plaque ELISA. Les plaques sont incubées une nuit à 4°C puis lavées avec du tampon PBS-Tween (PBS, 0,1% Tween20). Après lavage en PBS-tween, les sérums à tester (100 µl) sont dilués au 1/100 et 1/400 dans du tampon PBS-lait écrémé-Tween (PBS, 3% lait écrémé, 0,1% Tween) puis ajoutés dans les puits de la plaque ELISA sensibilisée. Les plaques sont incubées 1h à 37°C. Après 3 lavages avec du tampon PBS-Tween, le conjugué anti-IgG humaines marqué à la peroxidase (réf NA933V, Amersham) dilué au 1/4000 dans du tampon PBS-lait écrémé-Tween est ajouté puis les plaques sont incubées une heure à 37°C. Après 6 lavages avec du tampon PBS-Tween, le chromogène (TMB) et le substrat (H₂O₂) sont ajoutés et les plaques sont incubées 10 minutes à l'abri de la lumière. La réaction est arrêtée par ajout d'une solution 1M de H₃PO₄ puis l'absorbance est mesurée à 450 nm avec une référence à 620nm.

Les tests ELISA (figure 38) démontrent que le polypeptide recombinant Ssol est reconnu spécifiquement par les anticorps sériques de patients atteints de SRAS prélevés en phase moyenne ou tardive de l'infection (≥ 10 jours après le début des symptômes), alors qu'il n'est pas reconnu de façon significative par les anticorps sériques des sérums témoins de sujets non atteints de SRAS.

En conclusion, ces résultats démontrent que le polypeptide recombinant Ssol peut être purifié à partir du surnageant de cellules de mammifères infectées par le virus vaccine recombinant VV-TN-Ssol et être utilisé comme antigène pour la mise au point d'un test ELISA de diagnostic sérologique de l'infection par le SRAS-CoV.

### 5) Applications vaccinales

L'immunogénicité des virus vaccine recombinants a été étudiée chez la souris.

Pour cela, des groupes de 7 souris BALB/c ont été immunisés par voie i.v. à deux reprises à 4 semaines d'intervalle par 10⁶ u.f.p. de virus vaccine recombinants VV-TG, VV-TG-S, VV-TG-Ssol, VV-TN, VV-TN-S et VV-TN-Ssol ainsi que, à titre de contrôle, VV-TG-HA qui dirige l'expression de l'hémagglutinine de la souche A/PR/8/34 du virus de la grippe. Les sérums immuns ont été prélevés 3 semaines après chacune des immunisations (IS1, IS2).

Les sérums immuns ont été analysés par pool pour chacun des groupes par ELISA indirect en utilisant un lysat de cellules VeroE6 infectées par le SRAS-CoV comme antigène et à titre de contrôle, un lysat de cellules VeroE6 non infectées. Les titres (TI) en anticorps anti-SRAS-CoV sont calculés comme l'inverse de la dilution produisant une DO spécifique de 0,5 après révélation par un anticorps polyclonal anti-IgG(H+L) de souris couplé à la peroxidase (NA931V, Amersham) et du TMB additionné de H₂O₂ (KPL). Cette analyse (figure 39A) montre que l'immunisation par le virus VV-TG-S et VV-TN-S induit chez la souris dès la première immunisation des anticorps dirigés contre la forme native de la protéine de spicule du SRAS-CoV présente dans le lysat de cellules VeroE6 infectées. Les réponses induites par le virus VV-TN-S sont plus élevées que celles induites par le virus VV-TG-S après la première (TI=740 et TI=270 respectivement) et la deuxième (TI=3230 et TI=600 respectivement) immunisation. Le virus VV-TN-Ssol induit de forts titres d'anticorps anti-SRAS-CoV après deux immunisations (TI=640), alors que le virus VV-TG-Ssol induit une réponse à la limite de la détection (TI=40).

Les sérums immuns ont été analysés par pool pour chacun des groupes pour leur capacité à séroneutraliser l'infectivité du SRAS-CoV. 4 points de séroneutralisation sur cellules FRhK-4 (100 TCID50 de SRAS-CoV) sont réalisés pour chacune des dilutions de raison 2 testées à partir du 1/20. Le titre séroneutralisant est calculé selon la méthode de Reed et Munsch comme l'inverse de la dilution neutralisant l'infectivité de 2 cupules sur 4. Cette analyse montre que les anticorps induits chez la souris par les virus vaccine exprimant la protéine S ou le polypeptide Ssol sont neutralisants et que les virus à promoteurs synthétiques sont des immunogènes plus efficaces que les virus porteurs du promoteur 7.5K : les titres les plus élevés (640) sont observés après 2 immunisations par le virus VV-TN-S (figure 39B).

Le pouvoir protecteur des anticorps neutralisants induits chez la souris après immunisation par les virus vaccine recombinants est évalué à l'aide d'une infection d'épreuve par le SRAS-CoV.

### 6) Autres applications

Des virus vaccine recombinants de troisième génération sont construits en substituant les séquences sauvages des gènes S et Ssol par des gènes synthétiques optimisés pour l'expression en cellules de mammifères, décrits ci-dessus. Ces virus vaccine recombinants sont susceptibles d'exprimer des quantités plus importantes des antigènes S et Ssol et donc de présenter une immunogénicité accrue.

Le virus vaccine recombinant VV-TN-Ssol peut être utilisé pour la production en quantité et la purification de l'antigène Ssol en vue d'applications diagnostiques (sérologie par ELISA) et vaccinales (vaccin sous-unitaire).

### Exemple 17 : Virus recombinant de la rougeole exprimant la protéine de spicule (S) du coronavirus associé au SRAS (SRAS-CoV). Applications vaccinales.

### 1) Introduction

Le vaccin rougeole (MV) induit chez l'homme une immunité protectrice de longue durée après une seule injection (Hilleman, 2002, Vaccine, 20 : 651-665). La protection conférée est très robuste et repose sur l'induction d'une réponse en anticorps et d'une réponse cellulaire CD4 et CD8. Le génome du MV est très stable et aucune réversion vers la virulence des souches vaccinales n'a jamais été observée. Le virus de la rougeole appartient au genre des *Morbillivirus* de la famille des *Paramyxoviridae* ; c'est un virus enveloppé dont le génome est un ARN monocaténaire de polarité négative de 16kb (figure 40A) et dont le cycle de réplication exclusivement cytoplasmique exclut toute possibilité d'intégration dans le génome de l'hôte. Le vaccin rougeole est ainsi l'un des vaccins vivants les plus efficaces et les plus sûrs utilisés dans la population humaine. L'équipe de Frédéric Tangy a développé récemment un vecteur d'expression sur la base de la souche Schwarz du virus de la rougeole, qui est la souche atténuée la plus sûre et la plus utilisée chez l'homme comme vaccin contre la rougeole. Cette souche vaccinale peut être isolée à partir d'un clone moléculaire infectieux en conservant son immunogénicité chez les primates ainsi que chez la souris susceptible à l'infection. Elle constitue, après insertion d'unités de transcription supplémentaires, un vecteur pour l'expression de séquences hétérologues (Combredet, 2003, J. Virol. 77 : 11546-11554). En outre, un MV Schwarz recombinant exprimant la glycoprotéine d'enveloppe du virus West Nile (WNV) induit une réponse en anticorps efficace et de longue durée qui protège la souris d'une infection d'épreuve létale par le WNV (Despres et al, 2004, J. Infect. Dis., sous presse). Toutes ces caractéristiques font de la souche atténuée Schwarz du virus de la rougeole un candidat vecteur extrêmement prometteur pour la construction de nouveaux vaccins vivants recombinants.

Le but de cet exemple est d'évaluer la capacité de virus recombinants de la rougeole (MV) exprimant différents antigènes du coronavirus associé au SRAS (SRAS-CoV) à constituer de nouveaux candidats vaccins contre le SRAS.

Les inventeurs se sont intéressés à la protéine de spicule (S) du SRAS-CoV, qui permet d'induire après immunisation génique chez l'animal des anticorps neutralisants l'infectivité du SRAS-CoV, ainsi qu'à une forme soluble et sécrétée de cette protéine, le polypeptide Ssol, qui est composé de l'ectodomaine (aa 1-1193) de la S fusionné en son extrémité C-ter à une étiquette FLAG (DYKDDDDK) via un linker BspE1 codant pour le dipeptide SG. Ce polypeptide Ssol présente une antigénicité similaire à celle de la protéine S et permet, après injection à la souris sous la forme d'une protéine purifiée adjuvée en hydroxide d'aluminium, l'induction de titres élevés d'anticorps neutralisants contre le SRAS-CoV.

Les différentes formes du gène S ont été introduites sous la forme d'une unité de transcription supplémentaire entre les gènes P (phosphoprotéine) et M (matrice) dans le cADN de la souche Schwarz du MV précédemment décrite (Combredet, 2003, J. Virol. 77 : 11546-11554 ; Demande EP N° 02291551.6 du 20 juin 2002, et Demande EP n° 02291550.8 du 20 juin 2002). Après avoir isolé les virus recombinants MVSchw2-SARS-S et MVSchw2-SARS-Ssol et vérifié leur capacité à exprimer l'antigène S du SRAS-CoV, leur capacité à induire chez la souris puis chez le singe une réponse immunitaire protectrice contre le SRAS est testée.

### 2) Construction des virus recombinants

Le plasmide pTM-MVSchw-ATU2 (figure 40B) contient un cADN infectieux correspondant à l'antigénome de la souche vaccinale Schwarz du virus de la rougeole (MV) dans lequel une unité de transcription supplémentaire (ATU) a été introduite entre les gènes P (phosphoprotéine) et M (matrice) (Combredet, 2003, Journal of Virology, 77 : 11546-11554). Des génomes recombinants MVSchw2-SARS-S et MVSchw2-SARS-Ssol du virus de la rougeole ont été construits par l'insertion des ORFs de la protéine S et du polypeptide Ssol au sein de l'unité de transcription supplémentaire du vecteur MVSchw-ATU2.

Pour cela, un fragment d'ADN contenant le cADN de la S du SRAS-CoV a été amplifié par PCR à l'aide des oligonucléotides 5'-ATACGTACGA CCATGTTTAT TTTCTTATTA TTTCTTACTC TCACT-3' et 5'-ATAGCGCGCT CATTATGTGT AATGTAATTT GACACCCTTG-3' en utilisant le plasmide pcDNA-S comme matrice puis inséré dans le plasmide pCR®2.1-TOPO (Invitrogen) pour obtenir le plasmide pTOPO-S-MV. Les deux oligonucléotides utilisés contiennent des sites de restriction BsiW1 et BssHII, de façon à permettre l'insertion ultérieure dans le vecteur rougeole, et ont été conçus de façon à générer une séquence de 3774 nt incluant les codons d'initiation et de terminaison, afin de respecter la règle des 6 qui stipule que la longueur du génome d'un virus rougeole doit être divisible par 6 (Calain & Roux, 1993, J. Virol., 67 : 4822-4830 ; Schneider et al., 1997, Virology, 227 : 314-322). L'insert a été séquencé à l'aide d'un kit BigDye Terminator v1.1 (Applied Biosystems) et d'un séquenceur automatique ABI377.

Afin d'exprimer une forme soluble et sécrétée de la S du SRAS-CoV, un plasmide contenant l'ADNc du polypeptide Ssol correspondant à l'ectodomaine (aa 1-1193) de la S du SRAS-CoV fusionné en son extrémité C-ter à la séquence d'une étiquette FLAG (DYKDDDDK) via un linker BspE1 codant pour le dipeptide SG a ensuite été obtenu. Pour cela, un fragment d'ADN a été amplifié à l'aide des oligonucléotides 5'-CCATTTCAAC AATTTGGCCG-3' et 5'-ATAGGATCCG CGCGCTCATT ATTTATCGTC GTCATCTTTA TAATC-3' à partir du plasmide pCDNA-Ssol puis inséré dans le plasmide pTOPO-S-MV entre les sites Sal1 et BamH1 pour obtenir le plasmide pTOPO-S-MV-SF. La séquence générée est longue de 3618 nt entre les sites BsiW1 et BssHII et respecte la règle des 6. L'insert a été séquencé comme indiqué ci-dessus.

Les fragments BsiW1-BssHII contenant les ADNc de la protéine S et du polypeptide Ssol ont ensuite été excisés par digestion des plasmides pTOPO-S-MV et pTOPO-S-MV-SF puis sous-clonés entre les sites correspondants du plasmide pTM-MVSchw-ATU2 pour donner les plasmides pTM-MVSchw2-SARS-S et pTM-MVSchw2-SARS-Ssol (figure 40B). Ces deux plasmides ont été déposés à la C.N.C.M. le 1^{er} décembre 2004, sous les numéros I-3326 et I-3327, respectivement.

Les virus rougeole recombinants correspondants aux plasmides pTM-MVSchw2-SARS-S et pTM-MVSchw2-SARS-Ssol ont été obtenus par génétique inverse selon le système reposant sur l'utilisation d'une lignée cellulaire auxiliaire, décrit par Radecke et coil. (1995, Embo J., 14, : 5773-5784) et modifié par Parks et coll. (1999, J. Virol., 73 : 3560-3566). Brièvement, les cellules auxiliaires 293-3-46 sont transfectées selon la méthode au phosphate de calcium par 5 µg des plasmides pTM-MVSchw2-SARS-S ou pTM-MVSchw2-SARS-Ssol et 0,02 µg du plasmide pEMC-La dirigeant l'expression de la polymérase L du MV (don de M.A. Billeter). Après une nuit d'incubation à 37°C, un choc thermique est réalisé pendant 2 heures à 43°C et les cellules transfectées sont transférées sur une monocouche de cellules Vero. Pour chacun des deux plasmides, des syncytia sont apparus après 2 à 3 jours de co-culture et ont été transférés successivement sur des monocouches de cellules Vero à 70% de confluence en boites de pétri de 35 mm puis en flacons de 25 et 75 cm². Quand les syncytia ont atteint 80-90% de confluence, les cellules sont récupérées à l'aide d'un grattoir puis congelées et décongelées une fois. Après une centrifugation à basse vitesse, le surnageant contenant le virus est conservé en aliquot à -80°C. Les titres des virus recombinants MVSchw2-SARS-S et MVSchw2-SARS-Ssol ont été déterminés par dilution limite sur cellules Vero et le titre en dose infectant 50% des cupules (TCID₅₀) calculé selon la méthode de Kârber.

### 3) Caractérisation des virus recombinants

L'expression des transgènes codant la protéine S et le polypeptide Ssol a été recherchée par western blot et immunofluorescence.

Des monocouches de cellules Vero en flacons T-25 ont été infectées à une multiplicité de 0,05 par différents passages des deux virus MVSchw2-SARS-S et MVSchw2-SARS-Ssol et le virus sauvage MWSchw à titre de contrôle. Quand les syncytia ont atteint 80 à 90% de confluence, des extraits cytoplasmiques ont été préparés dans un tampon d'extraction (150mMNaCl, 50 mM Tris-HCl pH 7,2, 1% triton-X-100, 0,1% SDS, 1% DOC) puis dilués en tampon de dépôt selon Laemmli, séparés sur un gel SDS à 8% de polyacrylamide et transférés sur une membrane de PVDF (BioRad). La détection de cette immunoempreinte (« western blot ») a été réalisée à l'aide d'un sérum polyclonal de lapin anti-S (immun-sérum du lapin P11135 : cf exemple 4 ci-dessus) et d'anticorps polyclonaux d'âne dirigés contre les IgG de lapin et couplés à la peroxidase (NA934V, Amersham). Les anticorps fixés ont été révélés par luminescence à l'aide du kit ECL+ (Amersham) et de films d'autoradiographie Hyperfilm MP (Amersham).

Des cellules Vero en monocouches sur lamelles de verre ont été infectées par les deux virus MVSchw2-SARS-S et MVSchw2-SARS-Ssol et le virus sauvage MWSchw à titre de contrôle à des multiplicités d'infection de 0,05. Quand les syncytia ont atteint 90 à 100% (virus MVSchw2-SARS-Ssol) ou 30 à 40% (MVSchw2-SARS-S,MWSchw) de confluence, les cellules ont été fixées dans une solution de PBS-PFA 4%, perméabilisées par une solution de PBS contenant 0,2% de triton puis marquées par des anticorps polyclonaux de lapins hyperimmunisés par des virions purifiés et inactivés du SRAS-CoV et par un conjugué d'anticorps de chèvre anti-IgG(H+L) de lapin couplé au FITC (Jackson).

Comme le montrent les figures 41 et 42, les virus recombinants MVSchw2-SARS-S et MVSchw2-SARS-Ssol dirigent l'expression de la protéine S et du polypeptide Ssol respectivement à des niveaux comparables à ceux que l'on peut observer 8h après infection par le SRAS-CoV. L'expression de ces polypeptides est stable après 3 passages des virus recombinants en culture cellulaire. Ces résultats démontrent que les virus rougeole recombinants sont bien porteurs des transgènes et permettent l'expression de la glycoprotéine du SRAS sous sa forme membranaire (S) ou sous une forme soluble (Ssol). On s'attend à ce que le polypeptide Ssol soit sécrété des cellules infectées par le virus MVSchw2-SARS-Ssol comme c'est le cas lorsque ce même polypeptide est exprimé dans des cellules de mammifères après transfection transitoire des séquences correspondantes (cf exemple 11 ci-dessus).

### 4) Applications

Ayant montré que les virus MVSchw2-SARS-S et MVSchw2-SARS-Ssol permettent l'expression de la S du SRAS-CoV, leur capacité à induire une réponse immunitaire protectrice contre le SRAS-CoV chez la souris CD46^{+/-} IFN-α̅βR^{-/-}, qui est susceptible à l'infection par le MV, est évaluée. La réponse en anticorps des souris immunisées est évaluée par test ELISA contre les antigènes natifs du SRAS-CoV et pour leur capacité à neutraliser l'infectivité du SRAS-CoV *in vitro,* en utilisant les méthodologies décrites ci-dessus. Le pouvoir protecteur de la réponse sera évalué en mesurant la réduction de la charge virale pulmonaire 2 jours après une infection d'épreuve non létale par le SRAS-CoV.

Des virus rougeole recombinants de seconde génération sont construits en substituant les séquences sauvages des gènes S et Sol par des gènes synthétiques optimisés pour l'expression en cellules de mammifères, décrits à l'exemple 15 ci-dessus. Ces virus rougeole recombinants sont susceptibles d'exprimer des quantités plus importantes des antigènes S et Ssol et donc de présenter une immunogénicité accrue.

Alternativement, les gènes sauvages ou synthétiques codant pour la protéine S ou le polypeptide Ssol peuvent être insérés dans le vecteur rougeole MVSchw-ATU3 sous la forme d'une unité de transcription supplémentaire localisée entre les gènes H et L, puis les virus recombinants produits et caractérisés de façon similaire. Cette insertion est susceptible de générer des virus recombinants possédant des caractéristiques (multiplication du virus, niveau d'expression du transgène) différentes et possiblement une immunogénicité améliorée par rapport à ceux obtenus après insertion des transgènes entre les gènes P et N.

Le virus rougeole recombinant MVSchw2-SARS-Ssol peut être utilisé pour la production en quantité et la purification de l'antigène Ssol en vue d'applications diagnostiques et vaccinales.

### Exemple 18 : Autres applications liées à la protéine S

a) Les vecteurs lentiviraux permettant l'expression de la S ou de Ssol (voire de fragments de la S) peuvent constituer un vaccin recombinant contre le SRAS-CoV, pour être utilisé en prophylaxie humaine et vétérinaire. Afin de démontrer la faisabilité d'un tel vaccin, l'immunogénicité des vecteurs lentiviraux recombinants TRIP-SD/SA-S-WPRE et TRIP-SD/SA-Ssol-WPRE est étudiée chez la souris.
b) Des anticorps monoclonaux sont produits à l'aide du polypeptide recombinant Ssol. D'après les résultats présentés à l'exemple 14 ci-dessus, ces anticorps ou du moins la majorité d'entre eux reconnaîtront la forme native de la S du SRAS-CoV et seront susceptibles d'applications diagnostiques et/ou prophylactiques.
c) Un test de sérologie du SRAS est mis au point avec le polypeptide Ssol utilisé comme antigène et la méthodologie du double épitope.

## Revendications

1. Paire d'amorces apte à amplifier un fragment de la séquence du génome d'un coronavirus associé au SRAS ou de son équivalent ADN, **caractérisée en ce qu'**elle est sélectionné dans le groupe constitué par :
- la paire d'amorces n° 1 constituée de,l'amorce sens SEQ ID NO : 60 et de l'amorce anti-sens SEQ ID NO : 61,
- la paire d'amorces n° 2 constituée de l'amorce sens SEQ ID NO : 62 et de l'amorce anti-sens SEQ ID NO : 63, et
- la paire d'amorces constituée des amorces SEQ ID Nos 55 et 56.

2. Sonde apte à détecter la présence du génome d'un coronavirus associé au SRAS ou d'un fragment de celui-ci, **caractérisée en ce qu'**elle est sélectionnée dans le groupe constitué par les séquences SEQ ID NO : 64 à 67.

3. Hybridome produisant un anticorps monoclonal contre la protéine N d'un coronavirus associé au SRAS, **caractérisé en ce qu'**il est choisi parmi les hybridomes suivants :
- l'hybridome produisant l'anticorps monoclonal 87, déposé à la CNCM le 1^{er} décembre 2004 sous le numéro 1-3328,
- l'hybridome produisant l'anticorps monoclonal 86, déposé à la CNCM le 1^{er} décembre 2004 sous le numéro I-3329,
- l'hybridome produisant l'anticorps monoclonal 57, déposé à la CNCM le 1^{er} décembre 2004 sous le numéro I-3330, et
- l'hybridome produisant l'anticorps monoclonal 156, déposé à la CNCM le 1^{er} décembre 2004 sous le numéro I-3331.

4. Anticorps ou fragment d'anticorps monoclonal dirigé contre la protéine N d'un coronavirus associé au SRAS, **caractérisé en ce qu'**il est produit par un hybridome selon la revendication 3.

5. Puce ou filtre, **caractérisée en ce qu'**il comprend un anticorps ou un fragment d'anticorps selon la revendication 4.

6. Test d'immunocapture destiné à détecter un coronavirus associé au SRAS, utilisant un anticorps monoclonal spécifique de la nucléoprotéine virale native (protéine N), **caractérisé en ce que** l'anticorps utilisé pour la capture de la nucléoprotéine virale native est un anticorps monoclonal spécifique de la région centrale et/ou d'un épitope conformationnel.

7. Test d'immunocapture selon la revendication 6, **caractérisé en ce que** l'anticorps utilisé pour la capture de la protéine N est l'anticorps monoclonal Acm87, produit par l'hybridome déposé à la CNCM le 1^{er} décembre 2004 sous le numéro I-3328.

8. Test d'immunocapture selon la revendication 6, **caractérisé en ce que** l'anticorps utilisé pour la capture de la protéine N est l'anticorps monoclonal Acm86, produit par l'hybridome déposé à la CNCM le 1^{er} décembre 2004 sous le numéro I-3329.

9. Test d'immunocapture selon la revendication 6, **caractérisé en ce que** les anticorps monoclonaux Acm86 et Acm87 sont utilisés pour la capture de la protéine N.

10. Test d'immunocapture selon l'une quelconque des revendications 6 à 9, **caractérisé en ce que** l'anticorps utilisé pour la révélation de la protéine N est l'anticorps monoclonal Acm57, produit par l'hybridome déposé à la CNCM le 1^{er} décembre 2004 sous le numéro I-3330, ledit anticorps étant conjugué à une molécule ou une particule révélatrice.

11. Test d'immunocapture selon l'une quelconque des revendications 6 à 9, **caractérisé en ce qu'**une combinaison des anticorps Acm57 et Acm87, conjugués à une molécule ou une particule révélatrice, est utilisée pour la révélation de la protéine N.

12. Réactif de détection d'un coronavirus associé au SRAS, **caractérisé en ce qu'**il est sélectionné dans le groupe constitué par :
(a) une paire d'amorces selon la revendication 1, ou une sonde selon la revendication 2,
(b) un anticorps ou un fragment d'anticorps selon la revendication 4,
(c) une combinaison d'anticorps comprenant les anticorps monoclonaux Acm86 et/ou Acm87, et l'anticorps monoclonal Acm57;
(d) une puce ou un filtre selon la revendication 5.

13. Utilisation d'un produit sélectionné dans le groupe constitué par :
(a) une paire d'amorces selon la revendication 1,
(b) une sonde selon la revendication 2,
(c) un vecteur de clonage et/ou d'expression recombinant, comprenant un insert de séquence SEQ ID NO : 38, ledit vecteur étant compris dans une souche bactérienne déposée sous le n° 1-3048, le 5 juin 2003, auprès de la Collection Nationale de Cultures de Microorganismes, 25 rue du Docteur Roux, 75724 Paris Cedex 15,
(d) un vecteur de clonage et/ou d'expression recombinant, contenant un fragment d'ADNc sélectionné dans le groupe constitué par :
- un fragment d'ADNc codant pour une fusion C-terminale de la protéine N (SEQ ID NO : 37) avec une étiquette polyhistidine, et
- un fragment d'ADNc codant pour une fusion N-terminale de la protéine N (SEQ ID NO : 37) avec une étiquette polyhistidine, ledit vecteur pouvant être compris dans une souche bactérienne qui a été déposée sous le n° 1- 3117, le 23 octobre 2003, auprès de la Collection Nationale de Cultures de Microorganismes, 25 rue du Docteur Roux, 75724 Paris Cedex 15, et
(e) une cellule modifiée par un vecteur tel que défini à l'alinéa (c) ou (d) ci-dessus,
pour la préparation d'un réactif de détection et éventuellement de génotypage d'un coronavirus associé au SRAS.

14. Méthode de détection d'un coronavirus associé au SRAS, à partir d'un échantillon biologique, laquelle méthode est **caractérisée en ce qu'**elle comprend au moins :
(a) l'extraction d'acides nucléiques présents dans ledit échantillon biologique,
(b) l'amplification d'un fragment de l'ORF-N par RT-PCR à l'aide d'une paire d'amorces selon la revendication 1, et
(c) la détection par tout moyen approprié des produits d'amplification obtenus en (b).

15. Méthode selon la revendication 14, **caractérisée en ce que** l'étape (c) de détection est réalisée à l'aide d'au moins une sonde selon la revendication 2.

16. Méthode de détection d'une infection par un coronavirus associé au SRAS, à partir d'un échantillon biologique, par ELISA IgG indirect utilisant la protéine N, laquelle méthode est **caractérisée en ce que** les plaques sont sensibilisées par une solution de protéine N à une concentration comprise entre 0,5 et 4µg/mL, de préférence 2µg/mL, dans un tampon PBS 10mM pH 7,2, rouge de phénol à 0,25mL /L.

17. Méthode de détection d'une infection par un coronavirus associé au SRAS, à partir d'un échantillon biologique, par ELISA double épitope, **caractérisée en ce que** le sérum à tester est mélangé à l'antigène de révélation, ledit mélange étant ensuite mis au contact de l'antigène fixé sur un support solide.

18. Complexe immun formé d'un anticorps ou d'un fragment d'anticorps monoclonal selon la revendication 3, et d'une protéine ou d'un peptide du coronavirus associé au SRAS.

19. Kit ou coffret de détection d'un coronavirus associé au SRAS, **caractérisé en ce qu'**il comprend au moins un réactif sélectionné dans le groupe constitué par :
- une paire d'amorces selon la revendication 1,
- une sonde selon la revendication 2,
- un vecteur de clonage et/ou d'expression recombinant tel que défini dans la revendication 13, paragraphes (c) et (d),
- une cellule modifiée telle que définie dans la revendication 18, paragraphe (e).
